(19) Europäisches Patentamt
European Patent Office
Office européen des brevets

(11) EP 4 194 853 A1

(12) EUROPEAN PATENT APPLICATION

(43) Date of publication:
**14.06.2023 Bulletin 2023/24**

(21) Application number: **22204376.2**

(22) Date of filing: **28.12.2017**

(51) International Patent Classification (IPC):
***G01N 33/50*** *(2006.01)* ***C12M 1/00*** *(2006.01)*
***C12Q 1/68*** *(2018.01)* ***G01N 33/49*** *(2006.01)*
***G01N 33/68*** *(2006.01)* ***C12Q 1/6876*** *(2018.01)*

(52) Cooperative Patent Classification (CPC):
**C12Q 1/6876; C12M 1/00; G01N 33/49; G01N 33/5014; G01N 33/5041; G16B 20/00; G16B 25/10; G16B 40/20;** C12Q 2600/106; C12Q 2600/136; C12Q 2600/142; C12Q 2600/158

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.12.2016 JP 2016256270**
**28.12.2016 JP 2016256278**

(62) Document number(s) of the earlier application(s) in accordance with Art. 76 EPC:
**17886714.9 / 3 598 128**

(71) Applicant: **National Institutes of Biomedical Innovation,**
**Health and Nutrition**
**Ibaraki-shi, Osaka 567-0085 (JP)**

(72) Inventor: **ISHII, Ken**
**Osaka, 567-0085 (JP)**

(74) Representative: **Uexküll & Stolberg**
**Partnerschaft von**
**Patent- und Rechtsanwälten mbB**
**Beselerstraße 4**
**22607 Hamburg (DE)**

Remarks:
•The complete document including Reference Table(s) and the Sequence Listing(s) can be downloaded from the EPO website
•This application was filed on 28-10-2022 as a divisional application to the application mentioned under INID code 62.
•Claims filed after the date of filing of the application / after the date of receipt of the divisional application (Rule 68(4) EPC).

# (54) CHARACTERISTIC ANALYSIS METHOD AND CLASSIFICATION OF PHARMACEUTICAL COMPONENTS BY USING TRANSCRIPTOMES

(57) The present invention provides a novel method for the classification of adjuvants. In one embodiment, the present invention provides a method for generating organ transcriptome profiles for adjuvants, said method comprising: (A) a step for obtaining expression data by performing transcriptome analysis for at least one organ of a target organism by using at least two adjuvants; (B) a step for clustering the adjuvants with respect to the expression data; and (C) a step for generating the organ transcriptome profile for the adjuvants on the basis of the clustering.



Processed by Luminess, 75001 PARIS (FR)

## Description

[Technical Field]

The present invention relates to a feature analysis method and classification of components used in drugs (hereinafter, referred to as "drug component" unless specifically noted otherwise, and refers to a component such as active ingredients, additives, or adjuvants). More specifically, the present invention relates to classification and feature analysis methodologies based on transcriptome analysis of a drug component such as an adjuvant.

[Background Art]

Evaluation of efficacy and safety (toxicity) of a drug component (e.g., active ingredient, additive, adjuvant, or the like) or the drug itself serves a critical role in determining whether the drug is approved and allowed to be distributed to the market.

Nonclinical trials are proactively conducted for active ingredients with regard to efficacy and safety from the active pharmaceutical ingredient stages. However, additional components (additive) and adjuvants are not proactively tested. Currently, safety and efficacy are empirically tested without a systematic approach.

Adjuvants in particular have been recognized as supplemental components, rather than drawing attention for their own efficacy. The term adjuvant is derived from "adjuvare" which means "help" in Latin. Adjuvant is a collective term for substances (agents) administered with the primary agent such as a vaccine for use in enhancing the effect thereof (e.g., immunogenicity). Research and development of classical adjuvants (i.e., immunoadjuvants) have a long history of about 90 years, but research on the mechanisms of adjuvants themselves was not very active until recently. Recently, research and development thereon is active, triggered by immunological and microbiological research as well as research on natural immunity and dendritic cells. For empirically conducted adjuvant development, scientific approach is about to become possible lately from molecular to organism levels.

[Summary of Invention]

[Solution to Problem]

The present invention has been completed after finding that by clustering results of transcriptome analysis for a plurality of drug components (e.g., active ingredients, additives, or adjuvants), each cluster can be clustered by each feature of the components (e.g., active ingredients, additives, or adjuvants) to systematically classify the drug components. It was also found that known drug components (e.g., active ingredients, additives, or adjuvants) have a typical reference drug component (reference active ingredient for active ingredients, reference additive for additives, or reference adjuvant for adjuvants), such that the present invention also provides a technology that can identify whether novel substances or substances with an unknown specific effect or function (e.g., efficacy of an active ingredient, assistive function of an additive, or adjuvant function) are substances belonging to separate (e.g., 6 types) categories or others.

Therefore, the present invention provides the following.

(Item a1)

A method of generating an organ transcriptome profile of an adjuvant, the method comprising: (A) obtaining expression data by performing transcriptome analysis on at least one organ of a target organism using two or more adjuvants; (B) clustering the adjuvants with respect to the expression data; and (C) generating a transcriptome profile of the organ of the adjuvants based on the clustering.

(Item a2)

The method of item a1, wherein the transcriptome analysis comprises administering the adjuvants to the target organism and comparing a transcriptome in the organ at a certain time after administration with a transcriptome in the organ before administration of the adjuvants, and identifying a set of differentially expressed genes (DEGs) as a result of the comparison.

(Item a3)

The method of item a2, comprising integrating the set of DEGs in two or more adjuvants to generate a set of differentially expressed genes (DEGs) in a common manner.

(Item a4)

The method of item a3, comprising identifying a gene whose expression has changed beyond a predetermined threshold value as a result of the comparison, and selecting a differentially expressed gene in a common manner among identified genes to generate a set of significant DEGs.

(Item a5)

The method of item a4, wherein the predetermined threshold value is identified by a difference in a predetermined multiple and predetermined statistical significance (p value).

(Item a6)

The method of any one of items a2 to a5, comprising performing the transcriptome analysis for at least two or more organs to identify a set of differentially expressed genes only in a specific organ and using the set as the organ specific gene set.

(Item a7)

The method of any one of items a1 to a6, wherein the transcriptome analysis is performed on a transcriptome in at least one organ selected from the group consisting of a liver, a spleen, and a lymph node.

(Item a8)

The method of any one of items a1 to a7, wherein a number of the adjuvants is a number that enables statistically significant clustering analysis.

(Item a9)

The method of any one of items a1 to a8, comprising providing one or more gene markers unique to a specific adjuvant or an adjuvant cluster and a specific organ in the profile as an adjuvant evaluation marker.

(Item a10)

The method of any one of items a1 to a9, further comprising analyzing a biological indicator to correlate the adjuvants with a cluster.

(Item a11)

The method of item a10, wherein the biological indicator comprises at least one indicator selected from the group consisting of a wounding, cell death, apoptosis, NFκB signaling pathway, inflammatory response, TNF signaling pathway, cytokines, migration, chemokine, chemotaxis, stress, defense response, immune response, innate immune response, adaptive immune response, interferons, and interleukins.

(Item a12)

The method of item a10, wherein the biological indicator comprises a hematological indicator.

(Item a13)

The method of item a12, wherein the hematological indicator comprises at least one selected from the group consisting of white blood cells (WBC), lymphocytes (LYM), monocytes (MON), granulocytes (GRA), relative (%) content of lymphocytes (LY%), relative (%) content of monocytes (MO%), relative (%) content of granulocytes (GR%), red blood cells (RBC), hemoglobins (Hb, HGB), hematocrits (HCT), mean corpuscular volume (MCV), mean corpuscular hemoglobins (MCH), mean corpuscular hemoglobin concentration (MCHC), red blood cell distribution width (RDW), platelets (PLT), platelet concentration (PCT), mean platelet volume (MPV), and platelet distribution width (PDW).

(Item a14)

The method of item a10, wherein the biological indicator comprises a cytokine profile.

(Item a15)

A program for implementing a method of generating an organ transcriptome profile of an adjuvant on a computer, the method comprising: (A) obtaining expression data by performing transcriptome analysis on at least one organ of a target organism using two or more adjuvants; (B) clustering the adjuvants with respect to the expression data; and (C) generating a transcriptome profile of the organ of the adjuvants based on the clustering.

(Item a15A)

The program of item a15, further comprising a feature of any one of items a1 to a14.

(Item a16)

A recording medium storing a program for implementing a method of generating an organ transcriptome profile of an adjuvant on a computer, the method comprising: (A) obtaining expression data by performing transcriptome analysis on at least one organ of a target organism using two or more adjuvants; (B) clustering the adjuvants with respect to the expression data; and (C) generating a transcriptome profile of the organ of the adjuvants based on the clustering.

(Item a16A)

The recording medium of item a16, further comprising a feature of any one of items a1 to a14.

(Item a17)

A system for generating an organ transcriptome profile of an adjuvant, the system comprising: (A) an expression data acquiring unit for obtaining or inputting expression data by performing transcriptome analysis on at least one organ of a target organism using two or more adjuvants; (B) a clustering computing unit for clustering the adjuvants with respect to the expression data; and (C) a profiling unit for generating a transcriptome profile of the organ of the adjuvants based on the clustering.

(Item a17A)

The system of item a17, further comprising a feature of any one of items a1 to a14.

(Item a18)

A method of providing feature information of an adjuvant, the method comprising: (a) providing a candidate adjuvant in at least one organ of a target organism; (b) providing a reference adjuvant set with a known function; (c) obtaining gene expression data by conducting transcriptome analysis on the candidate adjuvant and the reference adjuvant set to cluster the gene expression data; and (d) providing a feature of a member of the reference adjuvant set belonging to the same cluster as that of the candidate adjuvant as a feature of the candidate adjuvant.

(Item a19)

The method of item a18, further comprising a feature of any one of items a1 to a14.

(Item a20)

A program for implementing a method of providing feature information of an adjuvant on a computer, the method comprising: (a) providing a candidate adjuvant in at least one organ of a target organism; (b) providing a reference adjuvant set with a known function; (c) obtaining gene expression data by performing transcriptome analysis on the candidate adjuvant and the reference adjuvant set to cluster the gene expression data; and (d) providing a feature of a member of the reference adjuvant set belonging to the same cluster as that of the candidate adjuvant as a feature of the candidate adjuvant.

(Item a20A)

The program of item a19, further comprising a feature of any one of items a1 to a14.

(Item a21)

A recording medium for storing a program for implementing a method of providing feature information of an adjuvant on a computer, the method comprising: (a) providing a candidate adjuvant in at least one organ of a target organism; (b) providing a reference adjuvant set with a known function; (c) obtaining gene expression data by performing transcriptome analysis on the candidate adjuvant and the reference adjuvant set to cluster the gene expression data; and (d) providing a feature of a member of the reference adjuvant set belonging to the same cluster as that of the candidate adjuvant as a feature of the candidate adjuvant.

(Item a21A)

The recording medium of item a20, further comprising a feature of any one of items a1 to a14.

(Item a22)

A system for providing feature information of an adjuvant, the system comprising: (a) a candidate adjuvant providing unit for providing a candidate adjuvant; (b) a reference adjuvant providing unit for providing a reference adjuvant set with a known function; (c) a transcriptome clustering analysis unit for obtaining gene expression data by performing transcriptome analysis on the candidate adjuvant and the reference adjuvant set to cluster the gene expression data; and (d) a feature analysis unit for providing a feature of a member of the reference adjuvant set belonging to the same cluster as that of the candidate adjuvant as a feature of the candidate adjuvant.

(Item a22A)

The system of item a22, further comprising a feature of any one of items a1 to a14.

(Item a23)

A method of controlling quality of an adjuvant by using the method of item a1 to item a14 or item a18 or item a19, the program of item a15, 15A, item a20, or item a20A, the recording medium of item a16, item a16A, item a21, or item a21A, or the system of item a, item a17, item a17A, item a22, or item a22A.

(Item a24)

A method of testing safety of an adjuvant by using the method of item a1 to item a14 or item a18 or item a19, the program of item a15, 15A, item a20, or item a20A, the recording medium of item a16, item a16A, item a21, or item a21A, or the system of item a, item a17, item a17A, item a22, or item a22A.

(Item a25)

A method of determining an effect of an adjuvant by using the method of item a1 to item a14 or item a18 or item a19, the program of item a15, 15A, item a20, or item a20A, the recording medium of item a16, item a16A, item a21, or item a21A, or the system of item a, item a17, item a17A, item a22, or item a22A.

<Classification method>

(Item b1)

A method of classifying an adjuvant comprising classifying an adjuvant based on transcriptome clustering.

(Item b2)

The method of item b1, wherein the classification further comprises classification by at least one feature selected from the group consisting of classification based on a host response, classification based on a mechanism, classification

by application based on a mechanism or cells (liver, lymph node, or spleen), and module classification.

(Item b3)

The method of item b1 or b2, wherein the classification comprises at least one classification selected from the group consisting of G1 to G6:

(1) G1 (interferon signaling);
(2) G2 (metabolism of lipids and lipoproteins);
(3) G3 (response to stress);
(4) G4 (response to wounding);
(5) G5 (phosphate-containing compound metabolic process); and
(6) G6 (phagosome).

(Item b4)

The method of item b3,

wherein the classification of G1 to G6 is performed by comparison with transcriptome clustering of a reference adjuvant,
wherein

a reference adjuvant of G1 is a STING ligand,
a reference adjuvant of G2 is a cyclodextrin,
a reference adjuvant of G3 is an immune reactive peptide,
a reference adjuvant of G4 is a TLR2 ligand,
a reference adjuvant of G5 is a CpG oligonucleotide,

and/or
a reference adjuvant of G6 is a squalene oil-in-water emulsion adjuvant.

(Item b5)

The method of item b3 or b4,

wherein the classification of G1 to G6 is performed by comparison with transcriptome clustering of a reference adjuvant,
wherein reference adjuvant of G1 is selected from the group consisting of cdiGMP, cGAMP, DMXAA, PolyIC, and R848,
wherein a reference adjuvant of G2 is bCD (β cyclodextrin),
wherein a reference adjuvant of G3 is FK565,
wherein a reference adjuvant of G4 is MALP2s,
wherein a reference adjuvant of G5 is selected from the group consisting of D35, K3, and K3SPG, and/or
wherein a reference adjuvant of G6 is AddaVax.

(Item b6)

The method of any one of items b3 to b5,

wherein the classification of G1 to G6 is performed based on an expression profile of a gene (identification marker gene; DEG) with a significant difference in expression in transcriptome analysis,
wherein a DEG of the G1 comprises at least one selected from the group consisting of Gm14446, Pml, H2-T22, Ifit1, Irf7, Isg15, Stat1, Fcgr1, Oasia, Oas2, Trim12a, Trim12c, Uba7, and Ube216,
wherein a DEG of the G2 comprises at least one selected from the group consisting of Elovl6, Gpam, Hsd3b7, Acer2, Acox1, Tbl1xr1, Alox5ap, and Ggt5,
wherein a DEG of the G3 comprises at least one selected from the group consisting of Bbc3, Pdk4, Cd55, Cd93, Clec4e, Corola, and Traf3, Trem3, C5ar1, Clec4n, Ier3, Il1r1, Plek, Tbx3, and Trem1,
wherein a DEG of the G4 comprises at least one selected from the group consisting of Ccl3, Myof, Papss2, Slc7a11,

and Tnfrsf1b,
wherein a DEG of the G5 comprises at least one selected from the group consisting of Ak3, Insm1, Nek1, Pik3r2, and Ttn, and
wherein a DEG of the G6 comprises at least one selected from the group consisting of Atp6v0d2, Atp6v1c1, and Clec7a.

(Item b7)

A method of classifying an adjuvant, the method comprising:

(a) providing a candidate adjuvant in at least one organ of a target organism;
(b) providing a reference adjuvant set classified to at least one selected from the group consisting of G1 to G6 of any one of items b3 to b6;
(c) obtaining gene expression data by performing transcriptome analysis on the candidate adjuvant and the reference adjuvant set to cluster the gene expression data; and
(d) determining that the candidate adjuvant belongs to the same group if a cluster to which the candidate adjuvant belongs is classified to the same cluster as at least one in groups G1 to G6, and determining as impossible to classify if the cluster does not belong to any cluster.

(Item b8)

A method of manufacturing an adjuvant composition having desirable function, comprising:

(A) providing an adjuvant candidate,
(B) selecting an adjuvant candidate having a transcriptome expression pattern corresponding to a desirable function, and
(C) manufacturing an adjuvant composition using a selected adjuvant candidate.

(Item b9)

The method of item b8, wherein the desirable function comprises any one or more of G1 to G6 of any one of items b3 to b6.

(Item b10)

An adjuvant composition for exerting a desirable function, comprising an adjuvant exerting the desirable function, wherein the desirable function comprises any one or more of G1 to G6 of any one of items b3 to b6.

(Item b11)

A method of controlling quality of an adjuvant by using the method of any one of items b1 to b7.

(Item b12)

A method of testing safety of an adjuvant by using the method of any one of items b1 to b7.

(Item b13)

A method of determining an effect of an adjuvant by using the method of any one of items b1 to b7.

(Item b14)

A program for implementing an adjuvant classification method comprising classifying an adjuvant based on transcriptome clustering on a computer.

(Item b14A)

The program of item b14, wherein the transcriptome clustering further comprises one or more features of any

one of items b2 to b7.

(Item b15)

A recording medium storing a program for implementing an adjuvant classification method comprising classifying an adjuvant based on transcriptome clustering on a computer.

(Item b15A)

The recording medium of item b15, wherein the transcriptome clustering further comprises one or more features of any one of items b2 to b7.

(Item b16)

A system for classifying an adjuvant based on transcriptome clustering, comprising a classification unit for classifying an adjuvant.

(Item b16A)

The system of item b16, wherein the transcriptome clustering further comprises one or more features of any one of items b2 to b7.

(Item b17)

A program for implementing an adjuvant classification method comprising classifying an adjuvant on a computer, the method comprising:

(a) providing a candidate adjuvant in at least one organ of a target organism;
(b) providing a reference adjuvant set classified to at least one selected from the group consisting of G1 to G6 of any one of items b3 to b6;
(c) obtaining gene expression data by performing transcriptome analysis on the candidate adjuvant and the reference adjuvant set to cluster the gene expression data; and
(d) determining that the candidate adjuvant belongs to the same group if a cluster to which the candidate adjuvant belongs is classified to the same cluster as at least one in groups G1 to G6, and determining as impossible to classify if the cluster does not belong to any cluster.

(Item b17A)

The program of item b17, further comprising one or more features of any one of items b2 to b7.

(Item b18)

A recording medium storing a program for implementing an adjuvant classification method on a computer, the method comprising:

(a) providing a candidate adjuvant in at least one organ of a target organism;
(b) providing a reference adjuvant set classified to at least one selected from the group consisting of G1 to G6 of any one of items b3 to b6;
(c) obtaining gene expression data by performing transcriptome analysis on the candidate adjuvant and the reference adjuvant set to cluster the gene expression data; and
(d) determining that the candidate adjuvant belongs to the same group if a cluster to which the candidate adjuvant belongs is classified to the same cluster as at least one in groups G1 to G6, and determining as impossible to classify if the cluster does not belong to any cluster.

(Item b18A)

The recording medium of item b18, further comprising one or more features of any one of items b2 to b7.

(Item b19)

A system for classifying an adjuvant, the system comprising:

(a) a candidate adjuvant providing unit for providing a candidate adjuvant in at least one organ of a target organism;
(b) a reference adjuvant storing unit for providing a reference adjuvant set classified to at least one selected from the group consisting of G1 to G6 of any one of items b3 to b6;
(c) a transcriptome clustering analysis unit for obtaining gene expression data by performing transcriptome analysis on the candidate adjuvant and the reference adjuvant set to cluster the gene expression data; and
(d) a determination unit for determining that the candidate adjuvant belongs to the same group if a cluster to which the candidate adjuvant belongs is classified to the same cluster as at least one in groups G1 to G6, and determining as impossible to classify if the cluster does not belong to any cluster.

(Item b19A)

The system of item b19, further comprising one or more features of any one of items b2 to b7.

(Item b20)

A gene analysis panel for use in classification of an adjuvant to G1 to G6 of any one of items b3 to b6 and/or to others, the gene analysis panel comprising means for detecting at least one DEG selected from the group consisting of a DEG of G1, a DEG of G2, a DEG of G3, a DEG of G4, a DEG of G5, and a DEG of G6,

wherein the DEG of G1 comprises at least one selected from the group consisting of Gm14446, Pml, H2-T22, Ifit1, Irf7, Isg15, Stat1, Fcgr1, Oasia, Oas2, Trim12a, Trim12c, Uba7, and Ube216,
wherein the DEG of G2 comprises at least one selected from the group consisting of Elovl6, Gpam, Hsd3b7, Acer2, Acox1, Tbl1xr1, Alox5ap, and Ggt5,
wherein the DEG of G3 comprises at least one selected from the group consisting of Bbc3, Pdk4, Cd55, Cd93, Clec4e, Corola, and Traf3, Trem3, C5ar1, Clec4n, Ier3, Il1r1, Plek, Tbx3, and Trem1,
wherein the DEG of G4 comprises at least one selected from the group consisting of Ccl3, Myof, Papss2, Slc7a11, and Tnfrsf1b,
wherein the DEG of G5 comprises at least one selected from the group consisting of Ak3, Insm1, Nek1, Pik3r2, and Ttn, and
wherein the DEG of G6 comprises at least one selected from the group consisting of Atp6v0d2, Atp6v1c1, and Clec7a.

(Item b21)

The gene analysis panel of item b20, wherein the gene analysis panel comprises means for detecting at least a DEG of G1, means for detecting at least a DEG of G2, means for detecting at least a DEG of G3, means for detecting at least a DEG of G4, means for detecting at least a DEG of G5, and means for detecting at least a DEG of G6.

<Adjuvant of "adjuvant">

(Item bX1)

A composition for eliciting or enhancing adjuvanticity of an antigen, comprising δ inulin (β-D-[2→1]poly(fructo-furanosyl)α-D-glucose) or a functional equivalent thereof.

(Item bX2)

The composition of item bX1, wherein the equivalent has a transcriptome expression profile equivalent to δ inulin.

<Dendritic cell activation>

(Item bA1)

A composition for activating a dendritic cell, comprising δ inulin or a functional equivalent thereof.

(Item bA2)

The composition of item bA1, wherein the activation is performed in the presence of a macrophage.

(Item bA3)

The composition of item bA1 or bA2 comprising δ inulin or a functional equivalent thereof, wherein the composition is administered with an enhancer of a macrophage.

<Th orientation>

(Item bB1)

A composition for enhancing a Th1 response of a Th1 type antigen and a Th2 response of a Th2 type antigen, comprising δ inulin or a functional equivalent thereof.

<TNFα>

(Item bC1)

An adjuvant composition comprising δ inulin or a functional equivalent thereof, wherein the composition is administered while TNFα is normal or enhanced.

<Same adjuvant/adjuvant determination method + manufacturing method>

(Item bD1)

A method of determining whether a candidate adjuvant elicits or enhances adjuvanticity of an antigen, the method comprising: (a) providing a candidate adjuvant; (b) providing δ inulin or a functional equivalent thereof as an evaluation reference adjuvant; (c) obtaining gene expression data by performing transcriptome analysis on the candidate adjuvant and the evaluation reference adjuvant to cluster the gene expression data; and (d) determining the candidate adjuvant as eliciting or enhancing adjuvanticity of an antigen if the candidate adjuvant is determined to belong to the same cluster as the evaluation reference adjuvant.

(Item bE1)

A method of manufacturing a composition comprising an adjuvant that elicits or enhances adjuvanticity of an antigen, the method comprising: (a) providing one or more candidate adjuvants; (b) providing δ inulin or a functional equivalent thereof as an evaluation reference adjuvant; (c) obtaining gene expression data by performing transcriptome analysis on the candidate adjuvant and the evaluation reference adjuvant to cluster the gene expression data; (d) if there is an adjuvant belonging to the same cluster as the evaluation reference adjuvant among the candidate adjuvants, selecting the adjuvant as an adjuvant that elicits or enhances adjuvanticity of an antigen, and if not, repeating (a) to (c); and (e) manufacturing a composition comprising the adjuvant that elicits or enhances adjuvanticity of an antigen obtained in (d).

(Item c1)

A method for classifying a drug component comprising classifying a drug component based on transcriptome clustering.

(Item c2)

The method of item c1, wherein the step of classifying comprises a) generating a reference component based on the transcriptome clustering; and b) classifying a candidate drug component based on the reference component.

(Item c3)

The method of item c1 or c2, wherein the drug component is selected from the group consisting of an active

ingredient, an additive, and an adjuvant.

(Item c4)

The method of any one of items c1 to c3, wherein the drug component is an adjuvant.

(Item c5)

The method of any one of items c1 to c4, wherein the classification further comprises classification by at least one feature selected from the group consisting of classification based on a host response, classification based on a mechanism, classification by application based on a mechanism or cells (liver, lymph node, or spleen), and module classification.

(Item c6)

The method of any one of items c1 to c5, wherein the classification comprises at least one classification selected from the group consisting of G1 to G6:

(1) G1 (interferon signaling);
(2) G2 (metabolism of lipids and lipoproteins);
(3) G3 (response to stress);
(4) G4 (response to wounding);
(5) G5 (phosphate-containing compound metabolic process); and
(6) G6 (phagosome).

(Item c7)

The method of item c6,

wherein the drug component is an adjuvant, and the classification of G1 to G6 is performed by comparison with transcriptome clustering of a reference drug component,
wherein a reference drug component of G1 is a STING ligand,
wherein a reference drug component of G2 is a cyclodextrin,
wherein a reference drug component of G3 is an immune reactive peptide,
wherein a reference adjuvant of G4 is a TLR2 ligand,
wherein a reference drug component of G5 is a CpG oligonucleotide, and/or
wherein a reference drug component of G6 is a squalene oil-in-water emulsion adjuvant.

(Item c8)

The method of item c6 or c7,

wherein the classification of G1 to G6 is performed by comparison with transcriptome clustering of a reference drug component,
wherein a reference component of G1 is selected from the group consisting of cdiGMP, cGAMP, DMXAA, PolyIC, and R848,
wherein a reference drug component of G2 is bCD (β cyclodextrin),
wherein a reference drug component of G3 is FK565,
wherein a reference drug component of G4 is MALP2s,
wherein a reference drug component of G5 is selected from the group consisting of D35, K3, and K3SPG, and/or
wherein a reference drug component of G6 is AddaVax.

(Item c9)

The method of any one of items c6 to c8,

wherein the classification of G1 to G6 is performed based on an expression profile of a gene (identification marker gene; DEG) with a significant difference in expression in transcriptome analysis,

wherein a DEG of the G1 comprises at least one selected from the group consisting of Gm14446, Pml, H2-T22, Ifit1, Irf7, Isg15, Stat1, Fcgr1, Oasia, Oas2, Trim12a, Trim12c, Uba7, and Ube216,
wherein a DEG of the G2 comprises at least one selected from the group consisting of Elovl6, Gpam, Hsd3b7, Acer2, Acox1, Tbl1xr1, Alox5ap, and Ggt5,
wherein a DEG of the G3 comprises at least one selected from the group consisting of Bbc3, Pdk4, Cd55, Cd93, Clec4e, Corola, and Traf3, Trem3, C5ar1, Clec4n, Ier3, Il1r1, Plek, Tbx3, and Trem1,
wherein a DEG of the G4 comprises at least one selected from the group consisting of Ccl3, Myof, Papss2, Slc7a11, and Tnfrsf1b,
wherein a DEG of the G5 comprises at least one selected from the group consisting of Ak3, Insm1, Nek1, Pik3r2, and Ttn, and
wherein a DEG of the G6 comprises at least one selected from the group consisting of Atp6v0d2, Atp6v1c1, and Clec7a.

(Item c10)

A method of classifying a drug component, the method comprising:

(a) providing a candidate drug component;
(b) providing a reference drug component set;
(c) obtaining gene expression data by performing transcriptome analysis on the candidate drug component and the reference drug component set to cluster the gene expression data; and
(d) determining that the candidate drug component belongs to the same group if a cluster to which the candidate adjuvant belongs is classified to the same cluster as at least one in the reference drug component set, and determining as impossible to classify if the cluster does not belong to any cluster.

(Item c11)

The method of classifying a drug component of item c10, the method comprising:

(a) providing a candidate drug component in at least one organ of a target organism;
(b) providing a reference drug component set classified to at least one selected from the group consisting of G1 to G6 of any one of items c6 to c8;
(c) obtaining gene expression data by performing transcriptome analysis on the candidate drug component and the reference drug component set to cluster the gene expression data; and
(d) determining that the candidate drug component belongs to the same group if a cluster to which the candidate drug component belongs is classified to the same cluster as at least one in groups G1 to G6, and determining as impossible to classify if the cluster does not belong to any cluster.

(Item c12)

A method of manufacturing a composition having a desirable function, comprising:

(A) providing a candidate drug component,
(B) selecting a candidate drug component having a transcriptome expression pattern corresponding to a desirable function, and
(C) manufacturing a composition using a selected candidate drug component.

(Item c13)

A method of screening for a composition having a desirable function, comprising;

(A) providing a candidate drug component; and
(B) selecting a candidate drug component having a transcriptome expression pattern corresponding to a desirable function.

(Item c14)

The method of item c12 or c13, wherein the desirable function comprises any one or more of G1 to G6 of any

one of items c6 to c8.

(Item c15)

A composition for exerting a desirable function, comprising a drug component exerting the desirable function, wherein the desirable function comprises one or more classifications specified by the method of any one of items c1 to c11.

(Item c16)

The composition of item c15 for exerting a desirable function, comprising a drug component exerting the desirable function, wherein the desirable function comprises any one or more of G1 to G6 of any one of items c6 to c8.

(Item c17)

A method of controlling quality of a drug component by using the method of any one of item c1 to c11.

(Item c18)

A method of testing safety of a drug component by using the method of any one of items c1 to c11.

(Item c19)

A method of providing a toxicity bottleneck gene, comprising:

identifying a candidate of a toxicity bottleneck gene by using the method of any one of items c1 to c11;
making a knockout animal by knocking out the toxicity gene in another animal species; and
determining whether toxicity is reduced or eliminated in the knockout animal to select a gene with a reduction or elimination as a toxicity bottleneck gene.

(Item c20)

A method of determining toxicity of an agent, comprising:

determining whether activation of gene expression is observed for at least one of toxicity bottleneck genes for a candidate drug component such as an adjuvant; and
determining a candidate drug component observed to have the activation as having toxicity.

(Item c21)

A method of determining an effect of a drug component by using the method of any one of items c1 to c11.

(Item c22)

A method of providing an efficacy bottleneck gene, comprising:

identifying an efficacy determination gene by using the method of any one of items c1 to c11;
making a knockout animal by knocking out the toxicity gene in another animal species; and
determining whether efficacy is reduced or eliminated in the knockout animal to select a gene with a reduction or elimination as an efficacy bottleneck gene.

(Item c23)

A method of determining efficacy of an agent, comprising;

determining whether activation of gene expression is observed for at least one of efficacy bottleneck genes for a candidate drug component such as an adjuvant; and
determining a candidate drug component observed to have the activation as having efficacy.

(Item c24)

A program for implementing a drug component classification method comprising classifying a drug component based on transcriptome clustering on a computer.

(Item c25)

A recording medium storing a program for implementing a drug component classification method comprising classifying a drug component based on transcriptome clustering on a computer.

(Item c26)

A system for classifying a drug component based on transcriptome clustering, comprising a classification unit for classifying a drug component.

(Item c27)

A program for implementing a method of classifying a drug component on a computer, the method comprising:

(a) providing a candidate drug component in at least one organ of a target organism;
(b) calculating a reference drug component set;
(c) obtaining gene expression data by performing transcriptome analysis on the candidate drug component and the reference drug component set to cluster the gene expression data; and
(d) determining that the candidate drug component belongs to the same group if a cluster to which the candidate drug component belongs is classified to the same cluster as at least one in a reference drug component set, and determining as impossible to classify if the cluster does not belong to any cluster.

(Item c28)

The program of item c27 for implementing a method of classifying a drug component on a computer, the method comprising:

(a) providing a candidate drug component in at least one organ of a target organism;
(b) providing a reference drug component set classified to at least one selected from the group consisting of G1 to G6 of any one of items c6 to c8;
(c) obtaining gene expression data by performing transcriptome analysis on the candidate drug component and the reference drug component set to cluster the gene expression data; and
(d) determining that the candidate drug component belongs to the same group if a cluster to which the candidate drug component belongs is classified to the same cluster as at least one in groups G1 to G6, and determining as impossible to classify if the cluster does not belong to any cluster.

(Item c29)

A recording medium storing a program for implementing a method of classifying a drug component on a computer, the method comprising:

(a) providing a candidate drug component in at least one organ of a target organism;
(b) calculating a reference drug component set;
(c) obtaining gene expression data by performing transcriptome analysis on the candidate drug component and the reference drug component set to cluster the gene expression data; and
(d) determining that the candidate drug component belongs to the same group if a cluster to which the candidate drug component belongs is classified to the same cluster as at least one in a reference drug component set, and determining as impossible to classify if the cluster does not belong to any cluster.

(Item c30)

The recording medium of item c29 storing a program for implementing a method of classifying a drug component on a computer, the method comprising:

(a) providing a candidate drug component in at least one organ of a target organism;
(b) providing a reference drug component set classified to at least one selected from the group consisting of G1 to G6 of any one of items c6 to c8;
(c) obtaining gene expression data by performing transcriptome analysis on the candidate drug component and the reference drug component set to cluster the gene expression data; and
(d) determining that the candidate drug component belongs to the same group if a cluster to which the candidate drug component belongs is classified to the same cluster as at least one in groups G1 to G6, and determining as impossible to classify if the cluster does not belong to any cluster.

(Item c31)

A system for classifying a drug component, the system comprising:

(a) a candidate drug component providing unit for providing a candidate drug component in at least one organ of a target organism;
(b) a reference drug component calculating unit for calculating a reference drug component set;
(c) a transcriptome clustering analysis unit for obtaining gene expression data by performing transcriptome analysis on the candidate drug component and the reference drug component set to cluster the gene expression data; and
(d) a determination unit for determining that the candidate drug component belongs to the same group if a cluster to which the candidate drug component belongs is classified to the same cluster as at least one in a reference drug component set, and determining as impossible to classify if the cluster does not belong to any cluster.

(Item c32)

The system of item c31 for classifying a drug component, the system comprising:

(a) a candidate drug component providing unit for providing a candidate drug component in at least one organ of a target organism;
(b) a reference drug component storing unit for providing a reference drug component set classified to at least one selected from the group consisting of G1 to G6 of any one of items c6 to c8;
(c) a transcriptome clustering analysis unit for obtaining gene expression data by performing transcriptome analysis on the candidate drug component and the reference drug component set to cluster the gene expression data; and
(d) determination unit for determining that the candidate drug component belongs to the same group if a cluster to which the candidate drug component belongs is classified to the same cluster as at least one in groups G1 to G6, and determining as impossible to classify if the candidate drug cluster does not belong to any group.

(Item c33)

A gene analysis panel for using a drug component in classification specified by the method of any one of items c1 to c11.

(Item c34)

A gene analysis panel for use in classification of an adjuvant to G1 to G6 of any one of items c6 to c8 and/or to others, the gene analysis panel comprising means for detecting at least one DEG selected from the group consisting of a DEG of G1, a DEG of G2, a DEG of G3, a DEG of G4, a DEG of G5, and a DEG of G6,

wherein the DEG of G1 comprises at least one selected from the group consisting of Gm14446, Pml, H2-T22, Ifit1, Irf7, Isg15, Stat1, Fcgr1, Oasia, Oas2, Trim12a, Trim12c, Uba7, and Ube216,
wherein the DEG of G2 comprises at least one selected from the group consisting of Elovl6, Gpam, Hsd3b7, Acer2, Acox1, Tbl1xr1, Alox5ap, and Ggt5,
wherein the DEG of G3 comprises at least one selected from the group consisting of Bbc3, Pdk4, Cd55, Cd93, Clec4e, Corola, and Traf3, Trem3, C5ar1, Clec4n, Ier3, Il1r1, Plek, Tbx3, and Trem1,
wherein the DEG of G4 comprises at least one selected from the group consisting of Ccl3, Myof, Papss2, Slc7a11, and Tnfrsf1b,
wherein the DEG of G5 comprises at least one selected from the group consisting of Ak3, Insm1, Nek1, Pik3r2, and Ttn, and
wherein the DEG of G6 comprises at least one selected from the group consisting of Atp6v0d2, Atp6v1c1, and Clec7a.

(Item c35)

The gene analysis panel of item c34, wherein the gene analysis panel comprises means for detecting at least a DEG of G1, means for detecting at least a DEG of G2, means for detecting at least a DEG of G3, means for detecting at least a DEG of G4, means for detecting at least a DEG of G5, and means for detecting at least a DEG of G6.

(Item c36)

A method of generating an organ transcriptome profile of a drug component, the method comprising:

obtaining expression data by performing transcriptome analysis on at least one organ of a target organism using two or more drug components;
clustering the drug components with respect to the expression data; and
generating a transcriptome profile of the organ of the drug components based on the clustering.

(Item c37)

The method of item c36, wherein the transcriptome analysis comprises administering the drug component to the target organism and comparing a transcriptome in the organ at a certain time after administration with a transcriptome in the organ before administration of the drug component, and identifying a set of differentially expressed genes (DEG) as a result of the comparison.

(Item c38)

The method of item c37, comprising integrating the set of DEGs in two or more drug components to generate a set of DEGs with a same change.

(Item c39)

The method of item c37 or c38, comprising selecting a DEG whose expression has changed beyond a predetermined threshold value among the DEGs with the same change as a result of the comparison to generate a set of significant DEGs.

(Item c40)

The method of item c39, wherein the predetermined threshold value is identified by a difference in a predetermined multiple and predetermined statistical significance (p value).

(Item c41)

The method of any one of items c38 to c41, comprising performing the transcriptome analysis for at least two or more organs to identify a set of differentially expressed genes (DEG) only in a specific organ and using the set as the organ specific DEG set.

(Item c42)

The method of any one of items c36 to c41, wherein the transcriptome analysis is performed on a transcriptome in at least one organ selected from the group consisting of a liver, a spleen, and a lymph node.

(Item c43)

The method of any one of items c36 to c42, wherein a number of types of the drug components is a number that enables statistically significant clustering analysis.

(Item c44)

The method of any one of items c36 to c43, comprising providing one or more gene markers unique to a specific drug component or a drug component cluster and a specific organ in the profile as a drug component evaluation marker.

(Item c45)

The method of any one of items c36 to c44, further comprising analyzing a biological indicator to correlate the drug components with a cluster.

(Item c46)

The method of item c45, wherein the biological indicator comprises at least one indicator selected from the group consisting of a wounding, cell death, apoptosis, NFκB signaling pathway, inflammatory response, TNF signaling pathway, cytokines, migration, chemokine, chemotaxis, stress, defense response, immune response, innate immune response, adaptive immune response, interferons, and interleukins.

(Item c47)

The method of item c46, wherein the biological indicator comprises a hematological indicator.

(Item c48)

The method of item c47, wherein the hematological indicator comprises at least one selected from the group consisting of white blood cells (WBC), lymphocytes (LYM), monocytes (MON), granulocytes (GRA), relative (%) content of lymphocytes (LY%), relative (%) content of monocytes (MO%), relative (%) content of granulocytes (GR%), red blood cells (RBC), hemoglobins (Hb, HGB), hematocrits (HCT), mean corpuscular volume (MCV), mean corpuscular hemoglobins (MCH), mean corpuscular hemoglobin concentration (MCHC), red blood cell distribution width (RDW), platelets (PLT), platelet concentration (PCT), mean platelet volume (MPV), and platelet distribution width (PDW).

(Item c49)

The method of item c45, wherein the biological indicator comprises a cytokine profile.

(Item c50)

A program for implementing a method of generating an organ transcriptome profile of a drug component on a computer, the method comprising:

(A) obtaining expression data by performing transcriptome analysis on at least one organ of a target organism using two or more drug components;
(B) clustering the drug components with respect to the expression data; and
(C) generating a transcriptome profile of the organ of the drug components, based on the clustering.

(Item c51)

A recording medium storing a program for implementing a method of generating an organ transcriptome profile of a drug component on a computer, the method comprising:

(A) obtaining expression data by performing transcriptome analysis on at least one organ of a target organism using two or more drug components;
(B) clustering the drug components with respect to the expression data; and
(C) generating a transcriptome profile of the organ of the drug components based on the clustering.

(Item c52)

A system for generating an organ transcriptome profile of a drug component, the system comprising:

(A) an expression data acquiring unit for obtaining or inputting expression data by performing transcriptome analysis on at least one organ of a target organism using two or more drug components;
(B) a clustering computing unit for clustering the drug components with respect to the expression data; and
(C) a profiling unit for generating a transcriptome profile of the organ of the drug components based on the clustering.

(Item c53)

A method of providing feature information of a drug component, the method comprising:

(a) providing a candidate drug component in at least one organ of a target organism;
(b) providing a reference drug component set with a known function;
(c) obtaining gene expression data by performing transcriptome analysis on the candidate drug component and the reference drug component set to cluster the gene expression data; and
(d) providing a feature of a member of the reference drug component set belonging to the same cluster as that of the candidate drug component as a feature of the candidate drug component.

(Item c54)

The method of item c53, further comprising a feature of any one of items c36 to c49.

(Item c55)

A program for implementing a method of providing feature information of a drug component on a computer, the method comprising:

(a) providing a candidate drug component in at least one organ of a target organism;
(b) providing a reference drug component set with a known function;
(c) obtaining gene expression data by performing transcriptome analysis on the candidate drug component and the reference drug component set to cluster the gene expression data; and
(d) providing a feature of a member of the reference drug component set belonging to the same cluster as that of the candidate drug component as a feature of the candidate drug component.

(Item c56)

A recording medium for storing a program for implementing a method of providing feature information of a drug component on a computer, the method comprising:

(a) providing a candidate drug component in at least one organ of a target organism;
(b) providing a reference drug component set with a known function;
(c) obtaining gene expression data by performing transcriptome analysis on the candidate drug component and the reference drug component set to cluster the gene expression data; and
(d) providing a feature of a member of the reference drug component set belonging to the same cluster as that of the candidate drug component as a feature of the candidate drug component.

(Item c57)

A system for providing feature information of a drug component, the system comprising:

(a) a candidate drug component providing unit for providing a candidate drug component;
(b) a reference drug component providing unit for providing a reference drug component set with a known function;
(c) a transcriptome clustering analysis unit for obtaining gene expression data by performing transcriptome analysis on the candidate drug component and the reference drug component set to cluster the gene expression data; and
(d) a feature analysis unit for providing a feature of a member of the reference drug component set belonging to the same cluster as that of the candidate drug component as a feature of the candidate drug component.

(Item c58)

A method of controlling quality of a drug component by using the method of items c36 to c49 or item c53.

(Item c59)

A method of testing safety of a drug component by using the method of items c36 to c49 or item c53.

(Item c60)

A method of determining an effect of a drug component by using the method of items c36 to c49 or item c53.

The present invention is intended so that one or more of the aforementioned features can be provided not only as the explicitly disclosed combinations, but also as other combinations thereof. Additional embodiments and advantages of the invention are recognized by those skilled in the art by reading and understanding the following detailed description as needed.

[Advantageous Effects of Invention]

The present invention provides a technology that can systematically classify a drug component (active ingredient, additive, adjuvant, or the like), and analyze and accurately predict a function thereof (e.g., detailed properties, safety, efficacy, or the like of an active ingredient, additive, or adjuvant) without detailed experimentation even for a drug component (e.g., active ingredient, additive, or adjuvant) with an unknown function. The present invention also provides a technology that can systematically classify a drug component (active ingredient, additive, adjuvant, or the like), and analyze whether a function is the same as one of the known reference drug components (e.g., reference adjuvants of G1 to G6 of the adjuvants exemplified herein) or others even for a drug component (e.g., active ingredient, additive, or adjuvant) with an unknown function.

[Brief Description of Drawings]

[Figure 1A] Figure **1** shows an adjuvant gene space constituting a significantly differentially expressed gene (sDEG) from each organ. The sDEG for each gene is shown in a Venn diagram. A set unique to the lympho node (LN), liver (LV), and spleen (SP) was analyzed using a TargetMine pathway annotation with a p value. Likewise, a gene set shared by all three organs (LV, SP, and LN) was annotated by pathway analysis with a p value.

[Figure 1B] Figure **1** shows an adjuvant gene space constituting a significantly differentially expressed gene (sDEG) from each organ. The sDEG for each gene is shown in a Venn diagram. A set unique to the lymph node (LN), liver (LV), and spleen (SP) was analyzed using a TargetMine pathway annotation with a p value. Likewise, a gene set shared by all three organs (LV, SP, and LN) was annotated by pathway analysis with a p value.

[Figure 2A] Figure **2A** shows an adjuvant that is consistent among three organs. Adjuvant clusters in liver (LV, top), spleen (SP, left), and lymph node (LN, right) when determined by Ward D2 method in R are shown. See Figure **14** for details of the clusters. The reference adjuvant is indicated by a colored font. The batch effect and weak adjuvant (greater than batch effect but below reference line) are indicated by gray. Adjuvant cluster relationship among the three organs is indicated by a line.

[Figure 2B] Figure **2B** shows an adjuvant that is consistent among three organs. The adjuvant cluster in liver (LV) when determined by Ward D2 method in R is shown. The reference adjuvant is indicated by a colored font. The batch effect and weak adjuvant (greater than batch effect but below reference line) are indicated by gray.

[Figure 2C] Figure **2C** shows an adjuvant that is consistent among three organs. The adjuvant cluster in spleen (SP) when determined by Ward D2 method in R is shown. The reference adjuvant is indicated by a colored font. The batch effect and weak adjuvant (greater than batch effect but below reference line) are indicated by gray.

[Figure 2D] Figure **2D** shows an adjuvant that is consistent among three organs. The adjuvant cluster in lymph node (LN) when determined by Ward D2 method in R is shown. The reference adjuvant is indicated by a colored font. The batch effect and weak adjuvant (greater than batch effect but below reference line) are indicated by gray.

[Figure 2E] Figure **2E** is an expanded view of the left side of the cluster of the mouse liver (LV) in Figure **2B.**

[Figure 2F] Figure **2F** is an expanded view of the right side of the cluster of the mouse liver (LV) in Figure **2B.**

[Figure 2G] Figure **2G** is an expanded view of the left side of the cluster of the mouse spleen (SP) in Figure **2C.**

[Figure 2H] Figure **2H** is an expanded view of the right side of the cluster of the mouse spleen (SP) in Figure **2C.**

[Figure 2I] Figure **2I** is an expanded view of the left side of the cluster of the mouse lymph node (LN) in Figure **2D.**

[Figure 2J] Figure **2J** is an expanded view of the right side of the cluster of the mouse lymph node (LN) in Figure **2D.**

[Figure 2K] Figure **2K** shows an adjuvant that is consistent among three organs in rats. The same tendencies as mice were observed. In the same manner as Figure **2A,** adjuvant clusters in liver (top), spleen (left), and lymph node (right) when determined by Ward D2 method in R are shown.

[Figure 2L] Figure **2L** is an expanded view of the rat liver cluster in Figure **2E.**

[Figure 2M] Figure **2M** is an expanded view of the rat spleen cluster in Figure **2E.**

[Figure 2N] Figure **2N** is an expanded view of the rat lung cluster in Figure **2E.**

[Figure 2O] Figure **2O** is an expanded view of the left side of the rat liver cluster in Figure **2L.**

[Figure 2P] Figure **2P** is an expanded view of the right side of the rat liver cluster in Figure **2L.**

[Figure 2Q] Figure **2Q** is an expanded view of the left side of the rat spleen cluster in Figure **2M.**

[Figure 2R] Figure **2R** is an expanded view of the right side of the rat spleen cluster in Figure **2M.**

[Figure 2S] Figure **2S** is an expanded view of the left side of the rat lung cluster in Figure **2N.**

[Figure 2T] Figure **2T** is an expanded view of the right side of the rat lung cluster in Figure **2N.**

[Figure 3A] Figure **3** shows biological and cytokine annotations of adjuvant groups. Adjuvant group related genes selected by using z-score (Table 17) were annotated to a biological process using TargetMine (a), or cytokine annotation was estimated by upstream analysis using IPA (b). Representative annotations, genes (Table 18), and IPA upstream analysis (Table 19) are shown.

[Figure 3B] Figure **3** shows biological and cytokine annotations of adjuvant groups. Adjuvant group related genes selected by using z-score (Table 17) were annotated to a biological process using TargetMine (a), or cytokine annotation was estimated by upstream analysis using IPA (b). Representative annotations, genes (Table 18), and IPA upstream analysis (Table 19) are shown.

[Figure 4A] Figure **4** shows relative comparison of adjuvants targeting the same receptor in the lymph node. A preferentially induced gene was selected by representing the value of fold changes among adjuvants targeting the same receptor such as 35/K3/K3SPG (Table 20) or cdiGMP/cGAMP/DMXAA (Table 21) as a z-score. The figure shows Venn diagrams (a, d), preferentially upregulated top 10 genes (b, e), and mapping of selected genes to 40 modules (c, f).

[Figure 4B] Figures **4** shows relative comparison of adjuvants targeting the same receptor in the lymph node. A preferentially induced gene was selected by representing the value of fold changes among adjuvants targeting the same receptor such as 35/K3/K3SPG (Table 20) or cdiGMP/cGAMP/DMXAA (Table 21) as a z-score. The figure shows Venn diagrams (a, d), preferentially upregulated top 10 genes (b, e), and mapping of selected genes to 40 modules (c, f). In graph b of Figure **4B,** the left column top row, middle column middle row, and right column bottom row correspond to the relationship between adjuvants and preferentially upregulated top 10 genes.

[Figure 4C] Figures **4** shows relative comparison of adjuvants targeting the same receptor in the lymph node. A preferentially induced gene was selected by representing the value of fold changes among adjuvants targeting the same receptor such as 35/K3/K3SPG (Table 20) or cdiGMP/cGAMP/DMXAA (Table 21) as a z-score. The figure shows Venn diagrams (a, d), preferentially upregulated top 10 genes (b, e), and mapping of selected genes to 40 modules (c, f).

[Figure 4D] Figures **4** shows relative comparison of adjuvants targeting the same receptor in the lymph node. A preferentially induced gene was selected by representing the value of fold changes among adjuvants targeting the same receptor such as 35/K3/K3SPG (Table 20) or cdiGMP/cGAMP/DMXAA (Table 21) as a z-score. The figure shows Venn diagrams (a, d), preferentially upregulated top 10 genes (b, e), and mapping of selected genes to 40 modules (c, f). In graph e of Figure **4B,** the left column top row, middle column middle row, and right column bottom row correspond to the relationship between adjuvants and preferentially upregulated top 10 genes.

[Figure 5A] Figure **5** shows adjuvant induced hematological changes. Hematological change of peripheral blood after adjuvant injection (a). Black solid lines, two gray dotted lines on the outside thereof, and two red dotted lines on the outside thereof indicate the average of mice treated with a buffer control, standard deviation (SD) level for 1 SD, and SD level for 2 SD, respectively. A change in parameter over 1 SD is indicated by a red bar (1 SD, light red; 2 SD, dark red), and other changes in parameter are indicated by a black bar. The number of correlated genes and representative list thereof are shown (b). Correlation plot for the white blood cell (WBC) count in the blood and CXCL9 expression level in the liver (LV) (c). The red sloped line indicates the linearly fitted line. Adjuvants that changed the WBC count more than 1 SD are indicated by a (light) red font. It should be noted that the hematological data for samples from Exp5 (bCD_ID, D35_ID, K3SPG_ID) and Exp10 (DMXAA_ID, MALP2s_ID, MPLA_ID, R848_ID) were obtained by independent experiments. For this reason, these are not physically associated with gene expression data of an organ (indicated by black × in the plot), but still exhibited an excellent correlation (see Figure **7**).

[Figure 5B] Figure **5** shows adjuvant induced hematological changes. Hematological change of peripheral blood after adjuvant injection (a). Black solid lines, two gray dotted lines on the outside thereof, and two red dotted lines on the outside thereof indicate the average of mice treated with a buffer control, standard deviation (SD) level for 1 SD, and SD level for 2 SD, respectively. A change in parameter over 1 SD is indicated by a red bar (1 SD, light red; 2 SD, dark red), and other changes in parameter are indicated by a black bar. The number of correlated genes and representative list thereof are shown (b). Correlation plot for the white blood cell (WBC) count in the blood and CXCL9 expression level in the liver (LV) (c). The red sloped line indicates the linearly fitted line. Adjuvants that changed the WBC count more than 1 SD are indicated by a (light) red font. It should be noted that the hematological data for samples from Exp5 (bCD_ID, D35_ID, K3SPG_ID) and Exp10 (DMXAA_ID, MALP2s_ID, MPLA_ID, R848_ID) were obtained by independent experiments. For this reason, these are not physically associated with gene expression data of an organ (indicated by black × in the plot), but still exhibited an excellent correlation (see

Figure **7**).

[Figure 6] Figure **6** is a schematic diagram showing the configuration for practicing the system of the invention.

[Figure 7] Figure **7** is a volcano plot of samples. The figure shows log2 of the fold change (FC) of gene probe expression (horizontal axis) and the result of a paired t-test (vertical axis) in a volcano plot for each sample (adjuvant, route of administration, organ). A sample with a significant change ((FC > 1.5 or FC < 0.667) and (p value < 0.01) and (customized PA call = 1)) is indicated by a red (upregulated) or blue (downregulated) dot. For MPLA_ID_LN and Pam3CSK4_ID_LN indicated by a red arrow, a large number of probes were upregulated (on average), but gene expression in LN of mice treated with MPLA or Pam3CSK4 varied, so that the p value did not reach < 0.01.

[Figure 8] Figures **8** shows Venn diagrams of upregulated gene probes in individual mice. Mice were treated with the respectively indicated adjuvant. The relative size of the circle indicates how much each adjuvant upregulated a gene probe in individual mice. The overlapping portion of circles indicates a gene probe upregulated in common among mice. While MPLA_ID_LN and Pam3CSK4_ID_LN are underlined, one (green, top right) of the three samples treated with adjuvants of these two types hardly exhibited a response or exhibited only a slight response compared to the other two samples. For MPLA_ID_LN and Pam3CSK4_ID_LN, data for one of the samples was considered unsuitable for analysis (see standard procedure 3 for details), so that results for only two samples are shown. A large overlap of the Venn diagram in the analysis indicates that gene responses among individual mice are consistent in the same adjuvant treatment group.

[Figure 9] Figure **9** shows the correlation between the number of upregulated gene probes and consistency among adjuvant treated mice (overlapping portion in the Venn diagram). The horizontal axis indicates the percentage of probes at an overlapping portion among the total number of gene probes excluding overlaps (overlap between two samples for some of the adjuvants) (see Figure **8**). The vertical axis indicates the number of upregulated (mean FC > 2) gene probes. The red line (sloped line) indicates linear fitting. The gray region indicates the 99% confidence region. The names of adjuvants appearing outside the 99% confidence region are shown. The analysis shows that a potent gene response induced by an adjuvant in each organ is positively correlated with consistency of gene responses among individual mice.

[Figure 10A] Figure **10** shows a biological process annotation for each adjuvant. For each organ (LV, a; SP, b; LN, c), a gene probe set with FC > 2 for each adjuvant was annotated in accordance with the biological annotation with TargetMine. The resulting annotation (annotation p value < 0.05, including selected keyword (e.g., wounding, death, cytokine)) was integrated by totaling the LogP value of annotations comprising a keyword (see Table 12 for details). The heat map (red and green gradation) indicates -LogP. Darker red (lighter than the darkest region, but relatively dark among other regions) indicates a higher scope. Green (darkest region) indicates that there was no annotation reaching a p value of < 0.05. Since intranasal route was used, data (c) for ENDCN in LN is blank.

[Figure 10B] Figure **10** shows a biological process annotation for each adjuvant. For each organ (LV, a; SP, b; LN, c), a gene probe set with FC > 2 for each adjuvant was annotated in accordance with the biological annotation with TargetMine. The resulting annotation (annotation p value < 0.05, including selected keyword (e.g., wounding, death, cytokine)) was integrated by totaling the LogP value of annotations comprising a keyword (see Table 12 for details). The heat map (red and green gradation) indicates -LogP. Darker red (lighter than the darkest region, but relatively dark among other regions) indicates a higher scope. Green (darkest region) indicates that there was no annotation reaching a p value of < 0.05. Since intranasal route was used, data (c) for ENDCN in LN is blank.

[Figure 10C] Figure **10** shows a biological process annotation for each adjuvant. For each organ (LV, a; SP, b; LN, c), a gene probe set with FC > 2 for each adjuvant was annotated in accordance with the biological annotation with TargetMine. The resulting annotation (annotation p value < 0.05, including selected keyword (e.g., wounding, death, cytokine)) was integrated by totaling the LogP value of annotations comprising a keyword (see Table 12 for details). The heat map (red and green gradation) indicates -LogP. Darker red (lighter than the darkest region, but relatively dark among other regions) indicates a higher scope. Green (darkest region) indicates that there was no annotation reaching a p value of < 0.05. Since intranasal route was used, data (c) for ENDCN in LN is blank.

[Figure 11A] Figure **11** shows hierarchical clustering of gene probes and adjuvants. Significantly differentially expressed genes were sequentially clustered with respect to adjuvant (horizontal axis) and gene probe (vertical axis) for LV (a), SP (b), and LN (c). Expression values for fold change of each gene probe are shown as a heat map in a color scale shown in the figure. Gene probes were divided into 40 modules with an annotation associated with the highest score shown. The annotation, number of probes and p values of each module according to TargetMine are shown on the right side.

[Figure 11B] Figure **11** shows hierarchical clustering of gene probes and adjuvants. Significantly differentially expressed genes were sequentially clustered with respect to adjuvant (horizontal axis) and gene probe (vertical axis) for LV (a), SP (b), and LN (c). Expression values for fold change of each gene probe are shown as a heat map in a color scale shown in the figure. Gene probes were divided into 40 modules with an annotation associated with the highest score shown. The annotation, number of probes and p values of each module according to TargetMine are shown on the right side.

[Figure 11C] Figure **11** shows hierarchical clustering of gene probes and adjuvants. Significantly differentially expressed genes were sequentially clustered with respect to adjuvant (horizontal axis) and gene probe (vertical axis) for LV (a), SP (b), and LN (c). Expression values for fold change of each gene probe are shown as a heat map in a color scale shown in the figure. Gene probes were divided into 40 modules with an annotation associated with the highest score shown. The annotation, number of probes and p values of each module according to TargetMine are shown on the right side.

[Figure 12A] Figure **12** shows analysis of a cell population responding to an adjuvant in each organ. A cell population responding to an adjuvant in each organ was predicted with a gene probe satisfying mean FC > 2 for each adjuvant. ID of probes and FC expression values thereof were processed using 10 different immune cell type prediction matrices based on the ImmGen database. The cell type scores and samples were clustered with respect to the vertical and horizontal axes and shown as a heat map of z-score.

[Figure 12B] Figure **12** shows analysis of a cell population responding to an adjuvant in each organ. A cell population responding to an adjuvant in each organ was predicted with a gene probe satisfying mean FC > 2 for each adjuvant. ID of probes and FC expression values thereof were processed using 10 different immune cell type prediction matrices based on the ImmGen database. The cell type scores and samples were clustered with respect to the vertical and horizontal axes and shown as a heat map of z-score.

[Figure 12C] Figure **12** shows analysis of a cell population responding to an adjuvant in each organ. A cell population responding to an adjuvant in each organ was predicted with a gene probe satisfying mean FC > 2 for each adjuvant. ID of probes and FC expression values thereof were processed using 10 different immune cell type prediction matrices based on the ImmGen database. The cell type scores and samples were clustered with respect to the vertical and horizontal axes and shown as a heat map of z-score.

[Figure 13A] Figure **13** shows immune cell type analysis of 40 modules in each organ. 40 modules in each organ (LV, a; SP, b; LN, c) were analyzed with respect to immune cell populations responsive thereto by using the ImmGen database as a reference. The cell populations associated with a module is shown as a heat map.

[Figure 13B] Figure **13** shows immune cell type analysis of 40 modules in each organ. 40 modules in each organ (LV, a; SP, b; LN, c) were analyzed with respect to immune cell populations responsive thereto by using the ImmGen database as a reference. The cell populations associated with a module is shown as a heat map.

[Figure 13C] Figure **13** shows immune cell type analysis of 40 modules in each organ. 40 modules in each organ (LV, a; SP, b; LN, c) were analyzed with respect to immune cell populations responsive thereto by using the ImmGen database as a reference. The cell populations associated with a module are shown as a heat map.

[Figure 14A] Figure **14** shows cluster analysis of adjuvants. Each sample from an adjuvant administered mouse was individually clustered for each organ (LV, a; SP, b; LN, c) by the Ward D2 method of R. Adjuvants were grouped with a height threshold value of 1.0 for LV and SP and a height threshold value of 1.5 for LN. The threshold line is indicated in red. A group member of adjuvants consistent among three organs is indicated with color. The batch effect and weak adjuvant (greater than batch effect but below threshold value line) groups are indicated by gray. D35_ID_x2 and K3_ID_x3 (two samples exhibiting very strong gene response) were clustered to G2LV with other DAMP associated adjuvants such as ALM, bCD, ENDCN, and FCA.

[Figure 14B] Figure **14** shows cluster analysis of adjuvants. Each sample from an adjuvant administered mouse was individually clustered for each organ (LV, a; SP, b; LN, c) by the Ward D2 method of R. Adjuvants were grouped with a height threshold value of 1.0 for LV and SP and a height threshold value of 1.5 for LN. The threshold line is indicated in red. A group member of adjuvants consistent among three organs is indicated with color. The batch effect and weak adjuvant (greater than batch effect but below threshold value line) groups are indicated by gray. D35_ID_x2 and K3_ID_x3 (two samples exhibiting very strong gene response) were clustered to G2LV with other DAMP associated adjuvants such as ALM, bCD, ENDCN, and FCA.

[Figure 14C] Figure **14** shows cluster analysis of adjuvants. Each sample from an adjuvant administered mouse was individually clustered for each organ (LV, a; SP, b; LN, c) by the Ward D2 method of R. Adjuvants were grouped with a height threshold value of 1.0 for LV and SP and a height threshold value of 1.5 for LN. The threshold line is indicated in red. A group member of adjuvants consistent among three organs is indicated with color. The batch effect and weak adjuvant (greater than batch effect but below threshold value line) groups are indicated by gray. D35_ID_x2 and K3_ID_x3 (two samples exhibiting very strong gene response) were clustered to G2LV with other DAMP associated adjuvants such as ALM, bCD, ENDCN, and FCA.

[Figure 14D] Figure **14D** is an expanded view of the left side of the LV cluster in Figure **14A.**

[Figure 14E] Figure **14E** is an expanded view of the right side of the LV cluster in Figure **14A.**

[Figure 14F] Figure **14F** is an expanded view of the left side of the SP cluster in Figure **14B.**

[Figure 14G] Figure **14G** is an expanded view of the right side of the SP cluster in Figure **14B.**

[Figure 14H] Figure **14H** is an expanded view of the left side of the LN cluster in Figure **14C.**

[Figure 14I] Figure **141** is an expanded view of the right side of the LN cluster in Figure **14C.**

[Figure 15A] Figures **15** shows the top 10 genes within the group associated with an adjuvant. Z-score heat map of

adjuvant group associated genes. Adjuvant group associated genes were selected from z-score. A list of top 30 genes was first selected in accordance with the z-score, and then top 10 genes were selected in accordance with the actual gene expression values.

[Figure 15B] Figures **15** shows the top 10 genes within the group associated with an adjuvant. Z-score heat map of adjuvant group associated genes. Adjuvant group associated genes were selected from z-score. A list of top 30 genes was first selected in accordance with the z-score, and then top 10 genes were selected in accordance with the actual gene expression values.

[Figure 15C] Figures **15** shows the top 10 genes within the group associated with an adjuvant. Z-score heat map of adjuvant group associated genes. Adjuvant group associated genes were selected from z-score. A list of top 30 genes was first selected in accordance with the z-score, and then top 10 genes were selected in accordance with the actual gene expression values.

[Figure 16A] Figure **16** shows 40 modules from each organ, and the difference and commonality in the relationship among adjuvant group associated genes. Adjuvant group associated upregulated genes were selected based on the z-score of the gene expression value (Table 17). The selected probes were analyzed with respect to the distribution within 40 modules for each organ. G1 associated genes were distributed to a single interferon associated module (Tables 17 and 18). Genes associated with other groups were distributed broadly and differently to several modules for each organ. G1 in LN (5 types of adjuvants) indicates results of distribution of genes associated with 5 types of adjuvants (cdiGMP, cGAMP, DMXAA, PolyIC, and R848). The bars and numbers indicate the percentage of a gene probe within each group. G4 in LV and G3 and G6 in LN each comprise only one type of adjuvant (MalP2s, FK565, and AddaVax, respectively). These results have limited available data, so that interpretation requires care.

[Figure 16B] Figure **16** shows 40 modules from each organ, and the difference and commonality in the relationship among adjuvant group associated genes. Adjuvant group associated upregulated genes were selected based on the z-score of the gene expression value (Table 17). The selected probes were analyzed with respect to the distribution within 40 modules for each organ. G1 associated genes were distributed to a single interferon associated module (Tables 17 and 18). Genes associated with other groups were distributed broadly and differently to several modules for each organ. G1 in LN (5 types of adjuvants) indicates results of distribution of genes associated with 5 types of adjuvants (cdiGMP, cGAMP, DMXAA, PolyIC, and R848). The bars and numbers indicate the percentage of a gene probe within each group. G4 in LV and G3 and G6 in LN each comprise only one type of adjuvant (MalP2s, FK565, and AddaVax, respectively). These results have limited available data, so that interpretation requires care.

[Figure 16C] Figure **16** shows 40 modules from each organ, and the difference and commonality in the relationship among adjuvant group associated genes. Adjuvant group associated upregulated genes were selected based on the z-score of the gene expression value (Table 17). The selected probes were analyzed with respect to the distribution within 40 modules for each organ. G1 associated genes were distributed to a single interferon associated module (Tables 17 and 18). Genes associated with other groups were distributed broadly and differently to several modules for each organ. G1 in LN (5 types of adjuvants) indicates results of distribution of genes associated with 5 types of adjuvants (cdiGMP, cGAMP, DMXAA, PolyIC, and R848). The bars and numbers indicate the percentage of a gene probe within each group. G4 in LV and G3 and G6 in LN each comprise only one type of adjuvant (MalP2s, FK565, and AddaVax, respectively). These results have limited available data, so that interpretation requires care.

[Figure 17] Figure **17** shows a Venn diagram of genes significantly upregulated with a CpG adjuvant and annotation analysis thereof. Significantly differentially expressed genes were analyzed with a Venn diagram, and the shown gene sets were further analyzed for biological annotation by TargetMine. Shared genes of D35_K3_K3SPG (including 75 gene probes) were strongly associated with the interferon associated biological process.

[Figure 18] Figure **18** shows a Venn diagram of genes significantly upregulated with a sting ligand adjuvant and annotation analysis thereof. Significantly differentially expressed genes were analyzed with a Venn diagram, and the shown gene sets were further analyzed for biological annotation by TargetMine. Shared genes of cdiGMP_cGAMP_DMXAA (including 1491 gene probes) were strongly associated with the interferon associated biological process.

[Figure 19A] Figure **19** shows representative correlation plots between hematological data and gene expression. Representative correlation plots between hematological data and gene expression for Cxcl(a), Il7(b), and S1pr5(c) in the indicated organs are shown. The top panel shows data for white blood cells (WBC). The bottom panel shows lymphocytes (LYM). (a) High correlation was observed in WBC_Cxcl9 of LV. In LN, Cxcl9 was induced by cdiGMP, cGAMP, and DMXAA, but these three types of adjuvants did not induce leukemia. (b) The reduction in I17 in SP was strongly correlated with adjuvant induced lymphocyte deficiency. (c) The reduction of S1pr5 in SP was also strongly correlated with lymphocyte deficiency. Interestingly, the correlation between S1pr5 and LYM was only observed in SP.

[Figure 19B] Figure **19** shows representative correlation plots between hematological data and gene expression. Representative correlation plots between hematological data and gene expression for Cxcl(a), Il7(b), and S1pr5(c) in the indicated organs are shown. The top panel shows data for white blood cells (WBC). The bottom panel shows

lymphocytes (LYM). (a) High correlation was observed in WBC_Cxcl9 of LV. In LN, Cxcl9 was induced by cdiGMP, cGAMP, and DMXAA, but these three types of adjuvants did not induce leukemia. (b) The reduction in I17 in SP was strongly correlated with adjuvant induced lymphocyte deficiency. (c) The reduction of S1pr5 in SP was also strongly correlated with lymphocyte deficiency. Interestingly, the correlation between S1pr5 and LYM was only observed in SP.

[Figure 19C] Figure **19** shows representative correlation plots between hematological data and gene expression. Representative correlation plots between hematological data and gene expression for Cxcl(a), Il7(b), and S1pr5(c) in the indicated organs are shown. The top panel shows data for white blood cells (WBC). The bottom panel shows lymphocytes (LYM). (a) High correlation was observed in WBC_Cxcl9 of LV. In LN, Cxcl9 was induced by cdiGMP, cGAMP, and DMXAA, but these three types of adjuvants did not induce leukemia. (b) The reduction in I17 in SP was strongly correlated with adjuvant induced lymphocyte deficiency. (c) The reduction of S1pr5 in SP was also strongly correlated with lymphocyte deficiency. Interestingly, the correlation between S1pr5 and LYM was only observed in SP.

[Figure 20A] Figure **20** shows cluster analysis of AS04. The data for AS04 and alum (ALM_gsk) control sample was processed and analyzed for each organ (LV, a; SP, b; LN, c). As a whole, the cluster structure was similar to those shown in Figures **2** and **14.** The control alum (ALM_gsk) was below the batch effect threshold value, which was the same in ALM in LV and SP. One of the samples of ALM_gsk in LN exceeded the threshold value of batch effect and was classified to G2 in LN.

[Figure 20B] Figure **20** shows cluster analysis of AS04. The data for AS04 and alum (ALM_gsk) control sample was processed and analyzed for each organ (LV, a; SP, b; LN, c). As a whole, the cluster structure was similar to those shown in Figures **2** and **14.** The control alum (ALM_gsk) was below the batch effect threshold value, which was the same in ALM in LV and SP. One of the samples of ALM_gsk in LN exceeded the threshold value of batch effect and was classified to G2 in LN.

[Figure 20C] Figure **20** shows cluster analysis of AS04. The data for AS04 and alum (ALM_gsk) control sample was processed and analyzed for each organ (LV, a; SP, b; LN, c). As a whole, the cluster structure was similar to those shown in Figures **2** and **14.** The control alum (ALM_gsk) was below the batch effect threshold value, which was the same in ALM in LV and SP. One of the samples of ALM_gsk in LN exceeded the threshold value of batch effect and was classified to G2 in LN.

[Figure 20D] Figure **20D** is an expanded view of the left side of the LV cluster in Figure **20A.**

[Figure 20E] Figure **20E** is an expanded view of the right side of the LV cluster in Figure **20A.**

[Figure 20F] Figure **20F** is an expanded view of the left side of the SP cluster in Figure **20B.**

[Figure 20G] Figure **20G** is an expanded view of the right side of the SP cluster in Figure **20B.**

[Figure 20H] Figure **20H** is an expanded view of the left side of the LN cluster in Figure **20C.**

[Figure 20I] Figure **201** is an expanded view of the right side of the LN cluster in Figure **20C.**

[Figure 21] Figure **21** shows grouping of adjuvants when determined by cluster analysis of a plurality of organs. The data shown in Figures **2, 14,** and **20** are summarized into a table. AS04 was G1 in LN and G2 in LV. G2 was a unique group in SP. The adjuvant cluster structures shown in Figures **2** and **14** did not change overall after adding data for AS04.

[Figure 22A] Figure **22** shows that Advax™ induces a Th2 response when combined with a Th2 type antigen. (A to D) On day 0 and day 14, C57BL/6J mice (n = 3) were immunized by i.m. administering SV and an adjuvant. Serum antigen specific total IgG, IgG1, and IgG2c and total IgE were measured by ELISA on days 14 and 28. (E to G) On day 28, spleen cells were prepared from mice immunized with 15 $\mu$g of SV and adjuvant and stimulated with an MHC class I or II nucleoprotein epitope peptide. After the stimulation, IFN-$\gamma$, IL-13, and IL-17 in the supernatant were measured by ELISA. The results represent three separate experiments. The median value and SEM are shown for each group. Statistical significance is indicated by *P < 0.05, **P < 0.01, ***P < 0.001, †P < 0.05, †††P < 0.001 in Dunnett's multiple comparison test.

[Figure 22B] Figure **22** shows that Advax™ induces a Th2 response when combined with a Th2 type antigen. (A to D) On day 0 and day 14, C57BL/6J mice (n = 3) were immunized by i.m. administering SV and an adjuvant. Serum antigen specific total IgG, IgG1, and IgG2c and total IgE were measured by ELISA on days 14 and 28. (E to G) On day 28, spleen cells were prepared from mice immunized with 15 $\mu$g of SV and adjuvant and stimulated with an MHC class I or II nucleoprotein epitope peptide. After the stimulation, IFN-$\gamma$, IL-13, and IL-17 in the supernatant were measured by ELISA. The results represent three separate experiments. The median value and SEM are shown for each group. Statistical significance is indicated by *P < 0.05, **P < 0.01, ***P < 0.001, †P < 0.05, †††P < 0.001 in Dunnett's multiple comparison test.

[Figure 22C] Figure **22** shows that Advax™ induces a Th2 response when combined with a Th2 type antigen. (A to D) On day 0 and day 14, C57BL/6J mice (n = 3) were immunized by i.m. administering SV and an adjuvant. Serum antigen specific total IgG, IgG1, and IgG2c and total IgE were measured by ELISA on days 14 and 28. (E to G) On day 28, spleen cells were prepared from mice immunized with 15 $\mu$g of SV and adjuvant and stimulated with an

MHC class I or II nucleoprotein epitope peptide. After the stimulation, IFN-γ, IL-13, and IL-17 in the supernatant were measured by ELISA. The results represent three separate experiments. The median value and SEM are shown for each group. Statistical significance is indicated by *P < 0.05, **P < 0.01, ***P < 0.001, †P < 0.05, †††P < 0.001 in Dunnett's multiple comparison test.

[Figure 23A] Figure **23** shows that Advax™ exhibits a Th1 response when combined with a Th1 type antigen. (A to D) On day 0 and day 14, C57BL/6J mice (n = 3) were immunized by i.m. administering WV and an adjuvant. The mice were immunized on day 0 and day 14 using 15 μg of SV with alum. Serum antigen specific total IgG, IgG1, and IgG2c and total IgE titer were measured by ELISA on days 14 and 28. (E to G) On day 28, spleen cells were prepared from mice immunized with 15 μg of WV and adjuvant and stimulated with an MHC class I or II specific nucleoprotein epitope peptide. After the stimulation, IFN-γ, IL-13, and IL-17 in the supernatant were measured by ELISA. The results represent three separate experiments. The median value and SEM are shown for each group. Statistical significance is indicated by *P < 0.05, **P < 0.01, ***P < 0.001, and †P < 0.05 in Dunnett's multiple comparison test.

[Figure 23B] Figure **23** shows that Advax™ exhibits a Th1 response when combined with a Th1 type antigen. (A to D) On day 0 and day 14, C57BL/6J mice (n = 3) were immunized by i.m. administering WV and an adjuvant. The mice were immunized on day 0 and day 14 using 15 μg of SV with alum. Serum antigen specific total IgG, IgG1, and IgG2c and total IgE titer were measured by ELISA on days 14 and 28. (E to G) On day 28, spleen cells were prepared from mice immunized with 15 μg of WV and adjuvant and stimulated with an MHC class I or II specific nucleoprotein epitope peptide. After the stimulation, IFN-γ, IL-13, and IL-17 in the supernatant were measured by ELISA. The results represent three separate experiments. The median value and SEM are shown for each group. Statistical significance is indicated by *P < 0.05, **P < 0.01, ***P < 0.001, and †P < 0.05 in Dunnett's multiple comparison test.

[Figure 23C] Figure **23** shows that Advax™ exhibits a Th1 response when combined with a Th1 type antigen. (A to D) On day 0 and day 14, C57BL/6J mice (n = 3) were immunized by i.m. administering WV and an adjuvant. The mice were immunized on day 0 and day 14 using 15 μg of SV with alum. Serum antigen specific total IgG, IgG1, and IgG2c and total IgE titer were measured by ELISA on days 14 and 28. (E to G) On day 28, spleen cells were prepared from mice immunized with 15 μg of WV and adjuvant and stimulated with an MHC class I or II specific nucleoprotein epitope peptide. After the stimulation, IFN-γ, IL-13, and IL-17 in the supernatant were measured by ELISA. The results represent three separate experiments. The median value and SEM are shown for each group. Statistical significance is indicated by *P < 0.05, **P < 0.01, ***P < 0.001, and †P < 0.05 in Dunnett's multiple comparison test.

[Figure 24] Figure **24** shows that Advax™ does not induce an immune response in $Tlr7^{-/-}$ mice or when combined with a Th0 antigen. On day 0 and day 14, (A) C57BL/6J mice (n = 4 or 5) or (B) $Tlr7^{-/-}$ mice (n = 5) were each immunized by i.m. administering 100 μg of OVA and an adjuvant, or 1.5 μg of WV alone, or 1.5 μg of WV and an adjuvant. Serum antigen specific total IgG titer was measured by ELISA on days 14 and 28. The results represent three separate experiments. The median value and SEM are shown for each group. Statistical significance is indicated by *P < 0.05 and **P < 0.01 in Dunnett's multiple comparison test.

[Figure 25] Figure **25** shows that Advax™ activates DC in vivo, but not in vitro. (A to C) Bone marrow derived DC was stimulated in vitro for 24 hours with 1 mg/ml alum, 1 mg/ml Advax™, or 50 ng/ml LPS, and then CD40 expression on pDC was evaluated. (D to F) 0.67 mg of alum, 1 mg of Advax™, or 50 ng of LPS was injected into the base of the tail of C57BL/6J mice. After 24 hours from the injection, the groin lymph nodes were collected and treated with DNaseI and collagenase. The cells were then stained and analyzed by FACS. The results represent three separate experiments. The results for saline are indicated by the gray region and a line, and results for adjuvant are indicated only by a line without further filling in with color.

[Figure 26A] Figure **26** shows that a macrophage is required for the adjuvant effect of Advax™. Two-photon excitation microscopy on the lymph nodes (A to C) after 1 hour and (D to F) after 24 hours from i.d. administration of Brilliant Violet 421 labeled Advax delta inulin particles. $CD169^+$ and $MARCO^+$ macrophages were stained by i.d. injecting anti-CD169-FITC and anti-MARCO-phycoerythrin antibodies 30 minutes prior to the Advax™ administration. (A, D) Blue (left) indicates Advax™, (B, E) green (middle) indicates $CD169^+$ macrophage, and (C, F) red (right) indicates $MARCO^+$ macrophage. (G, H) Phagocytes in the lymph node were depleted by chlodronate liposome injection on the indicated date (days -2 and -7) and then WV + Advax™ were i.d. administered on day 0 for immunization. Serum antigen specific total IgG or IgG2 titer was measured by ELISA on days 14 and 28. The results represent three separate experiments. The median value and SEM are shown for each group. Statistical significance is indicated by *P < 0.05, **P < 0.01, and ***P < 0.001 in Student's t-test.

[Figure 26B] Figure **26** shows that a macrophage is required for the adjuvant effect of Advax™. Two-photon excitation microscopy on the lymph nodes (A to C) after 1 hour and (D to F) after 24 hours from i.d. administration of Brilliant Violet 421 labeled Advax delta inulin particles. $CD169^+$ and $MARCO^+$ macrophages were stained by i.d. injecting anti-CD169-FITC and anti-MARCO-phycoerythrin antibodies 30 minutes prior to the Advax™ administration. (A, D)

Blue (left) indicates Advax™, (B, E) green (middle) indicates $CD169^+$ macrophage, and (C, F) red (right) indicates $MARCO^+$ macrophage. (G, H) Phagocytes in the lymph node were depleted by chlodronate liposome injection on the indicated date (days -2 and -7) and then WV + Advax™ were i.d. administered on day 0 for immunization. Serum antigen specific total IgG or IgG2 titer was measured by ELISA on days 14 and 28. The results represent three separate experiments. The median value and SEM are shown for each group. Statistical significance is indicated by *P < 0.05, **P < 0.01, and ***P < 0.001 in Student's t-test.

[Figure 27A] Figure **27** shows that Advax™ changes the gene expression of IL-1β, CLR, and TNF-α signaling pathway. The transcriptome of all organs (lung; LG, liver; LV, spleen; SP, kidney; KD, lymph node; LN) after 6 hours of administration of Advax™ alone (i.d., or i.p.) was obtained by Affimetrix Gene Chip (n = 3). (A) shows only the selected gene probes (FC > 2 and PA = 1). (B) An Advax™ responsive cell population was analyzed. The left half of the diagram shows 10 different immune cell types, and the right half shows samples. The ribbons inside the inner circle indicate the cell type score of each sample. The color of the ring in the intermediate layer indicates the cell type or sample. The ring on the outermost layer represents the % of each agent (cell type or sample) when viewed from a competing agent of the total contribution. For example, neutrophils account for about 30% of the cell population in SP. ip. (C) IPA upstream regulator of Advax™ induced genes was analyzed in SP.

[Figure 27B] Figure **27** shows that Advax™ changes the gene expression of IL-1β, CLR, and TNF-α signaling pathway. The transcriptome of all organs (lung; LG, liver; LV, spleen; SP, kidney; KD, lymph node; LN) after 6 hours of administration of Advax™ alone (i.d., or i.p.) was obtained by Affimetrix Gene Chip (n = 3). (A) shows only the selected gene probes (FC > 2 and PA = 1). (B) An Advax™ responsive cell population was analyzed. The left half of the diagram shows 10 different immune cell types, and the right half shows samples. The ribbons inside the inner circle indicate the cell type score of each sample. The color of the ring in the intermediate layer indicates the cell type or sample. The ring on the outermost layer represents the % of each agent (cell type or sample) when viewed from a competing agent of the total contribution. For example, neutrophils account for about 30% of the cell population in SP. ip. (C) IPA upstream regulator of Advax™ induced genes was analyzed in SP.

[Figure 27C] Figure **27** shows that Advax™ changes the gene expression of IL-1β, CLR, and TNF-α signaling pathway. The transcriptome of all organs (lung; LG, liver; LV, spleen; SP, kidney; KD, lymph node; LN) after 6 hours of administration of Advax™ alone (i.d., or i.p.) was obtained by Affimetrix Gene Chip (n = 3). (A) shows only the selected gene probes (FC > 2 and PA = 1). (B) An Advax™ responsive cell population was analyzed. The left half of the diagram shows 10 different immune cell types, and the right half shows samples. The ribbons inside the inner circle indicate the cell type score of each sample. The color of the ring in the intermediate layer indicates the cell type or sample. The ring on the outermost layer represents the % of each agent (cell type or sample) when viewed from a competing agent of the total contribution. For example, neutrophils account for about 30% of the cell population in SP. ip. (C) IPA upstream regulator of Advax™ induced genes was analyzed in SP.

[Figure 27D] Figure **27D** is an expanded view of the explanation of symbols in IPA upstream regulator analysis of Advax™ induced genes in SP of Figure **27C.**

[Figure 27E] Figure **27E** is an expanded view of the correlation diagram according to IPA upstream regulator analysis of Advax™ induced genes in SP of Figure **27C.**

[Figure 28A] Figure **28** shows that TNF-α is required for the adjuvant effect of Advax™. (A) The abdominal cavity macrophage was stimulated for 8 hours with Advax™ or alum, and TNF-α in the supernatant was measured by ELISA. (B) 1 hour after 1 mg of Advax™ or 0.67 mg of alum was i.p. injected, serum and peritoneal lavage fluid were collected, and the TNF-α level therein was measured by ELISA. (C to E) On day 0 and day 14, heterozygous or $Tnfa^{-/-}$ mice (n = 5) were immunized by i.m. administering 1.5 μg of WV and an adjuvant. Serum antigen specific total IgG, IgG1, and IgG2c titers were measured by ELISA on days 14 and 28. The results represent three separate experiments. The median value and SEM are shown for each group. Statistical significance is indicated by *P < 0.05, **P < 0.01, and ***P < 0.001 in Dunnett's multiple comparison test and Student's t-test.

[Figure 28B] Figure **28** shows that TNF-α is required for the adjuvant effect of Advax™. (A) The abdominal cavity macrophage was stimulated for 8 hours with Advax™ or alum, and TNF-α in the supernatant was measured by ELISA. (B) 1 hour after 1 mg of Advax™ or 0.67 mg of alum was i.p. injected, serum and peritoneal lavage fluid were collected, and the TNF-α level therein was measured by ELISA. (C to E) On day 0 and day 14, heterozygous or $Tnfa^{-/-}$ mice (n = 5) were immunized by i.m. administering 1.5 μg of WV and an adjuvant. Serum antigen specific total IgG, IgG1, and IgG2c titers were measured by ELISA on days 14 and 28. The results represent three separate experiments. The median value and SEM are shown for each group. Statistical significance is indicated by *P < 0.05, **P < 0.01, and ***P < 0.001 in Dunnett's multiple comparison test and Student's t-test.

[Figure 29] Figure **29** shows a schematic diagram for creating a "toxic" group and "non-toxic" group based on a toxiogenomic database.

[Figure 30A] Figure **30A** shows that probes (genes) selected for a necrosis prediction model were concentrated into 5 pathways associated with immune response (1) and metabolism (4) .

[Figure 30B] Figure **30B** calculated the "toxicity" score of each adjuvant by comparing gene variation patterns at 6

hours and 24 hours after administration of each adjuvant with the "toxic" gene pattern and "nontoxic" gene pattern after 6 and 24 hours in the toxiogenomic database. The top part of the figure shows adjuvants exhibiting a high toxicity score in the comparative result after 6 hours (left) and after 24 hours (right). The bottom part shows the toxicity score of each adjuvant in the comparative result after 24 hours.

[Figure 31] Figure **31** shows the ALT activity at 6 hours (top) and 24 hours (bottom) after actually administrating each adjuvant.

[Figure 32] Figure **32** shows results of staining liver collected on day 1, day 2, day 3, and day 5 of intraperitoneal administration of FK565 to mice with hematoxylin and eosin and performing histological analysis. The arrow indicates liver damage. The scale bar indicates 100 um.

[Figure 33] Figure **33** shows results of staining a liver collected after intraperitoneally administering FK565 into mice with TUNEL and checking for apoptosis. The left side shows results of a control PBS administration, and the right side shows results of FK565 administration.

[Figure 34] Blood was collected on day 1 and day 2 after 3 hours from intraperitoneally administering PBS, FK565 (1 μg/kg, 10 μg/kg, or 100 μg/kg), or LPS (1 mg/kg) to mice. Figure **34** shows results of biochemical analysis on the serum level of aspartate transaminase (AST) and alanine transaminase (ALT) by using the blood.

[Figure 35] Figure **35** shows gene clusters including Osmr obtained as a result of analysis the database.

[Figure 36] The top row shows a change in expression (fold change) of gene Y in the liver after 6 hours from administering each adjuvant to rats. The bottom row shows results of staining liver collected by administering FK565 to wild-type (left) or Osmr knockout (right) mouse with TUNEL.

[Figure 37] Blood was collected one day after intraperitoneally administering PBS, FK565 (1 μg/kg, 10 μg/kg, or 100 μg/kg), or LPS (1 mg/kg) to wild-type or Osmr knockout mice. Figure **37** shows results of biochemical analysis on the serum level of aspartate transaminase (AST) and alanine transaminase (ALT) by using the blood.

[Figure 38] Figure **38** shows a schematic diagram for creating an adjuvanticity prediction model.

[Figure 39] Figure **39** shows that probes (genes) selected for an adjuvanticity prediction model are concentrated into 42 pathways associated with cell death (4), immune response (2), and metabolism (36). The figure shows a Venn diagram for genes constituting pathways associated with cell death (4) and immune response (2).

[Figure 40] Figure **40** shows scores for drug, immunostimulant, LPS, and TNF calculated by an adjuvanticity prediction model (top). PO indicates oral administration, IV indicates intravenous administration, and IP indicates intraperitoneal administration. The ROC curve of the adjuvanticity prediction model is shown (bottom).

[Figure 41] Ovalbumin as well as alum, CpGk3 or 5 types of drugs (ACAP, BOR, CHX, COL, PHA) were intradermally administered to mice on day 0 and day 14 with 2 to 3 doses, and blood and spleens were collected on day 21. The results of measuring anti-ovalbumin (ova) antibody titer (IgG1, IgG2, and total IgG) on day 21 are shown. The Y axis indicates the increase in antibody titer on the scale of log10. The number within the parenthesis indicates the dosage (μg/dose/mouse). Ovalbumin was administered at 10 μg/dose/mouse.

[Figure 42] Spleen cells were stimulated by adding ovalbumin (ova) 257-264 peptide (OVA-MHC1), ova 323-339 peptide (OVA-MHC2), or ova protein (OVA-whole) in addition to each drug in vitro, or treated without additional stimulation. The supernatant was collected. The results of measuring Th1 (IL-2 and IFN-γ) cytokines in the supernatant by ELISA are shown. The number within the parenthesis indicates the dosage (μg/dose/mouse). Ovalbumin was administered at 10 μg/dose/mouse.

[Figure 43] Spleen cells were stimulated by adding ovalbumin (ova) 257-264 peptide (OVA-MHC1), ova 323-339 peptide (OVA-MHC2), or ova protein (OVA-whole) in addition to each drug in vitro, or treated without additional stimulation. The supernatant was collected. The results of measuring Th2 (IL-4 and IL-5) cytokines in the supernatant by ELISA are shown. The number within the parenthesis indicates the dosage (μg/dose/mouse). Ovalbumin was administered at 10 μg/dose/mouse.

[Figure 44] Figure **44** shows result of administering ovalbumin as well as alum, CpGk3 or 5 types of drugs (ACAP, BOR, CHX, COL, PHA) to mice, and collecting blood after 3 hours (day 0), and performing biochemical analysis on the serum level of aspartate transaminase (AST) and alanine transaminase (ALT). The number within the parenthesis indicates the dosage (μg/dose/mouse). Ovalbumin was administered at 10 μg/dose/mouse.

[Figure 45] Figure **45** shows results of collecting blood after 6 hours and 24 hours from intraperitoneally administering each drug to mice and analyzing miRNA in the circulating blood. The vertical axis indicates -log10 (p value), and the horizontal axis indicates -log2 (miRNA volume).

[Figure 46] Figure **46** shows results of analyzing miRNA in the circulating blood after 6 hours and 24 hours from administering Alum and AS04 to mice. The vertical axis indicates -log10 (p value), and the horizontal axis indicates -log2 (miRNA volume).

[Description of Embodiments]

The present invention is explained hereinafter with the best modes thereof. Throughout the entire specification,

a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. Further, the terms used herein should be understood as being used in the meaning that is commonly used in the art, unless specifically noted otherwise. Therefore, unless defined otherwise, all terminologies and scientific technical terms that are used herein have the same meaning as the general understanding of those skilled in the art to which the present invention pertains. In case of a contradiction, the present specification (including the definitions) takes precedence.

(Classification of drug component)

There are many drug components with known effects that are not systematically classified. For example, immune adjuvants have an important role in improving the potency of a vaccine. A wide range of substances have been reported as functioning as an immune adjuvant, but the mode of action and safety profiles are not fully understood. The present invention has found that the mechanism of action and potential safety profile of drug components can be predicted and classified by performing transcriptome analysis on all organs in animals (e.g., mice) using a large number (21 in the Examples) of different drug components (e.g., adjuvant), creating integrated data for drug component induced responses thereof (adjuvant induced response for adjuvants), and elucidating (extracting) detailed features of the drug components. The present invention has also found that drug components can be divided into specific groups, and a standard drug component (e.g., adjuvant) can be determined for each group. The present invention provides a flexible standardizing method for comprehensively and systematically evaluating any drug component (e.g., adjuvant) and a framework thereof (classification method). The present invention demonstrates, for example, that each drug component can be classified to at least a characteristic adjuvant group named G1 to G6 or another group. Such classification can identify or specifically predict the attribute of a target substance by performing transcriptome analysis on the target substance such as a substance with an unknown drug component function (e.g., adjuvant function) or a novel substance, performing clustering analysis by including transcriptome analysis data on a reference drug component (e.g., reference adjuvant) with confirmed function, and determining reference drug component which is classified to the same cluster as the target substance. The present invention provides a flexible standardizing method for comprehensively and systematically evaluating any drug component (active ingredient, additive, adjuvant, or the like), and a framework thereof.

Adjuvants, which are exemplary classification targets of the invention, have been traditionally used as an additive in a vaccine. Various substances such as oil emulsion, small molecules that are often formulated as nanoparticles or aluminum salt, lipids, and nucleic acids are known to function as an adjuvant with many vaccine antigens (Coffman, R.L., Sher, A. & Seder, R.A. Immunity 33, 492-503 (2010); Reed, S.G., Orr, M.T. & Fox, C.B. Nat Med 19, 1597-1608 (2013) and Desmet, C.J. & Ishii, K.J. Nature reviews. Immunology 12, 479-491 (2012)). The modes of action of adjuvants are categorized empirically into two classes (immunostimulant and antigen delivery system), but are not systematically classified. It is proposed that immunostimulants be further classified into pathogen associated molecular patterns (PAMPs) (Janeway, C.A., Jr. Cold Spring Harbor symposia on quantitative biology 54 Pt 1, 1-13 (1989)) or damage associated molecular patterns (DAMPs) (Matzinger, P. Annu Rev Immunol 12, 991-1045 (1994)) in accordance with the exogenous origin or endogenous origin, which is recognized by a germ line coding pattern recognition receptor, resulting in induction of interferon or pro-inflammatory cytokine secretion (Kawai, T. & Akira, S. Nature immunology 11, 373-384 (2010); Matzinger, P. Annu RevImmunol 12, 991-1045 (1994)). Since many adjuvants are understood as affecting multiple signaling pathways in a recipient, the underlining mechanism of each adjuvant enhancing or directing an immune response in vivo was not classifiable with conventional technologies, but the present invention enables the classification thereof. The present invention can categorize all adjuvants in terms of host responses.

To use a new drug component, establishment of appropriate formulation and GMP production method must be cleared. For example, to use a new adjuvant in a clinical vaccine, multiple steps need to be cleared including establishment of appropriate formulation and GMP production method, and more importantly, the risk and benefit (safety and efficacy) of the adjuvant utilization (Shoenfeld, Y. & Agmon-Levin, N. Journal of autoimmunity 36, 4-8 (2011); Batista-Duharte, A., Lindblad, E.B. & Oviedo-Orta, E. Toxicology letters 203, 97-105 (2011); Zaitseva, M. et al. Vaccine 30, 4859-4865 (2012).; Stassijns, J., Bollaerts, K., Baay, M. & Verstraeten, T. Vaccine 34, 714-722 (2016)). Since the results in animal experiments can be extrapolated to humans to some degree, pre-clinical trial animal research is needed for vaccine development. (Batista-Duharte, A., Lindblad, E.B. & Oviedo-Orta, Toxicology letters 203, 97-105 (2011); Sun, Y., Gruber, M. & Matsumoto, M. Journal of pharmacological and toxicological methods 65, 49-57 (2012)). In fact, careful assessment of biological and immunopathological effects in animals is essential for development of new adjuvanted vaccines (Mastelic, B. et al. Biologicals 41, 115-124 (2013)). Currently approved adjuvanted vaccines have cleared these preclinical toxicology evaluations before human use, and after commercialization their safeties are tracked by side effect investigative monitoring such as pharmacovigilance monitoring. However, a variety of substances with very different physicochemical properties function as adjuvants, which has not been provided with a logical explanation. While there was no simple and straightforward method to evaluate vaccine adjuvant’s mode of action and potential safety concerns

in animal models, the approaches used in this invention, enable this evaluation.

A systems vaccinology approach (Pulendran, B. Proc Natl Acad Sci USA 111, 12300-12306 (2014)) represents a relatively new research approach to vaccine science, in particular for humans, by identifying correlates of protection (Ravindran, R. et al. Science 343, 313-317 (2014); Tsang, J.S. et al. Cell 157, 499-513 (2014); Nakaya, H.I. et al. Immunity 43, 1186-1198 (2015); Sobolev, O. et al. Nature immunology 17, 204-213 (2016)). By investigating gene expression profiles induced early after vaccination, these approaches can predict adaptive immune responses that follow.

The present invention provides a systematic and comprehensive research approach for drug components (e.g., active ingredient, additive, and adjuvant). For example, various analysis methods are studied for vaccine adjuvants and molecular signatures are reported in the adjuvant field, but this has not lead to a systematic solution. Rather, it is reported that various known classification methods are not capable of classification (Olafsdottir, T., Lindqvist, M. & Harandi, A.M. Vaccine 33, 5302-5307 (2015)). Therefore, the present invention systematically and comprehensively classifies drug components (e.g., active ingredient, additive, and adjuvant) to provide reliable prediction means for efficacy and safety of novel drug components (e.g., active ingredient, additive, and adjuvant). Furthermore, the present invention provides reliable prediction means for identifying the function of a substance with unknown or indefinite drug component function (e.g., efficacy of active ingredient, assistive function of additive, or adjuvant function). Furthermore, substances with known function can also be utilized for quality control thereof and as part of safety management, and as part of efficacy and safety verification for a new drug component (e.g., adjuvant).

The inventors obtained gene expression data of approximately 330 microarrays from mouse liver (LV), spleen (SP), and draining inguinal lymph nodes (LN) after administration of a wide range of different adjuvants. An adjuvant induced gene (transcriptome) panel was able to be defined by integrating the data. This is also referred to as "adjuvant gene space" herein. Analyzing the adjuvant induced gene expression data (transcriptome) in this space revealed properties of each adjuvant in vivo. This approach was used to predict the unknown mechanism of action of two relatively new adjuvants. One such prediction matched the results that were independently studied, confirming that the approach of the invention provides correct prediction results (Hayashi, M. et al., Scientific Reports 6: 29165.doi:10.1038/srep29165.). This approach is not limited to adjuvants and is envisioned to be applicable to all drug components such as active ingredients and additives. In other words, the inventors can define a drug component induced gene (transcriptome) panel by obtaining gene expression data after administration of a wide range of different drug components and integrating the data. This is also referred to as "drug component gene space" herein. Analyzing the drug component induced gene expression data (transcriptome) in this space can reveal properties of each drug component in vivo. Such a method can be implemented by using artificial intelligence (AI) that utilizes machine learning or the like.

(Definitions)

The definitions of the terms and/or basic technologies particularly used herein are explained hereinafter as appropriate.

As used herein, "drug component" refers to any component or ingredient that can constitute a drug or pharmaceutical. Examples thereof include active ingredients (those exhibiting efficacy on their own), additives (components that are not expected to have efficacy on their own, but are expected to serve a certain role (e.g., excipient, lubricant, surfactant, or the like) when contained in a drug), adjuvants (those enhancing the efficacy (e.g., ability to elicit immune responses) of the primary drug (e.g., antigen for vaccines or the like)), and the like. Examples of drug components include pharmaceutically acceptable carrier, diluent, excipient, buffer, binding agent, blasting agent, diluent, flavoring agent, lubricant, and the like. A drug component can be an individual substance or a combination of a plurality of substances or agents. A combination of an active ingredient and an additive can include any combination, such as a combination of an adjuvant and an active ingredient.

As used herein, "active ingredient" refers to a component exerting the intended efficacy. One or more components can fall under an active ingredient.

As used herein, "additive" refers to any component not expected to have efficacy, but serves a certain role when contained in a drug such as a pharmaceutically acceptable carrier, diluent, excipient, buffer, binding agent, blasting agent, diluent, flavoring agent, lubricant, and the like. As used herein, β cyclodextrin and the like are encompassed as an additive, but such components can also be found to be effective as an adjuvant. In such a case, those skilled in the art determine whether such a component is an adjuvant or an additive depending on the objective.

As used herein, "adjuvant" refers to a compound that enhances an immune response of a subject to an antigen (e.g., vaccines or the like) when co-administered with the antigen. Adjuvant mediated enhancement of an immune response can be typically evaluated by any method known in the art, including, but not limited to, one or more of (i) increase in the number of antibodies generated in response to immunization with the above adjuvant/antigen combination to the number of antibodies generated in response to immunization with the above antigen alone; (ii) increase in the number of T cells recognizing the antigen or the adjuvant; (iii) increase in the level of one or more type I cytokines; and

(iv) in vivo defense after raw challenge.

As used herein, "transcriptome" is a term referring to the entirety of all transcriptional products (e.g., mRNA, primary transitional product (set of all RNA molecules including mRNA, rRNA, tRNA and other noncoding RNA), and transcripts) in cells (one cell or a population of cells) under a specific condition. A transcriptome, in relation to the present invention, refers to a cell, a population of cells, preferably a population of cancer cells, or a set of all RNA molecules produced in all cells of a given individual at a specific time.

As used herein, "exome" refers to an aggregate of all exons in the human genome, referring to the entire part of the genome of an organism formed by an exon which is the coded portion of the expressed gene. An exome provides a genetic blueprint used in the synthesis of a protein and other functional gene products. This is functionally the most important part of the genome, so that it was considered to be most likely to contribute to the phenotype of an organism.

Any suitable sequencing method can be used in accordance with the present invention for "transcriptome analysis" herein. Next-generation sequencing (NGS) technology is preferred. As used herein, the term "next generation sequencing" or "NGS" refers to all new high-throughput sequencing technologies, which divide the entire genome into small pieces to randomly read nucleic acid templates in parallel along the entire genome, in comparison to "conventional" sequencing known as Sanger chemistry. The NGS technologies (also known as massive parallel sequencing technologies) can deliver nucleic acid information of the full genome, exome, transcriptome (all transcribed sequences of the genome) or methylome (all methylated sequences of the genome) in a very short period, such as 1 to 2 weeks or less, preferably 1 to 7 days or less, or most preferably less than 24 hours, which enables a single cell sequencing approach in principle. Any NGS platform that is commercially available or mentioned in a reference can be used for practicing the present invention. As used herein, "transcriptome expression profile" refers to a profile of the expression status of each gene when performing transcriptome analysis on a certain agent.

As used herein, "transcriptome expression profile equivalent to ..." means that the "transcriptome expression profile" is substantially identical or identical, or substantially similar for a certain objective. Identity of expression profiles can be determined by whether expression profiles are similar for a molecule of a drug component (e.g., adjuvant) or the like or a part thereof. In this regard, whether profiles are similar can be determined by the degree of gene expression of sDEG or the like as defined herein and determined based on the degree of expression, amount, active amount, or the like. Although not wishing to be bound by any theory, in some embodiments of the invention, it is understood that drug components (e.g., adjuvants) belonging to the same cluster by classifying drug components (e.g., adjuvants) based on the similarity have the same feature as the drug components (e.g., adjuvants) in the same category. Therefore, features of novel drug components (e.g., adjuvants) or drug components (e.g., adjuvants) with an unknown function can be analyzed by investigating whether drug components belong to the same drug component cluster (e.g., adjuvant cluster) by using the approach of the invention. To study the similarity herein, a "similarity score" can be used. "Similarity score" refers to a specific numerical value indicating similarity. For example, a suitable score can be used appropriately in accordance with the technique used for calculating the transcriptome expression pattern or the like. A similarity score can be calculated using a regressive approach, neural networking method, machine learning algorithm such as support vector machine or random forest, or the like.

As used herein, "clustering" or "cluster analysis" or "clustering analysis" are interchangeably used, referring to a method of classifying a subject by aggregating subjects that are similar to one another among a population (subjects) of subjects having different properties to create (dividing) a cluster. Each subset after the division is referred to as a cluster. There are several types of division methods. In some cases, each of all subjects of classification is an element of one cluster (referred to as a hard or crisp cluster), and in some cases, a cluster partially belongs to a plurality of clusters simultaneously (referred to as soft or fuzzy cluster). Hard cluster analysis is generally used herein. Examples of typical cluster analysis include hierarchical cluster analysis, non-hierarchical cluster analysis, and the like. Hierarchical cluster is generally used, but analysis is not limited thereto. As used herein, "transcriptome clustering" refers to clustering based on results of transcriptome analysis.

As used herein, "cluster" generally refers to a collection of similar elements of a certain population (e.g., drug component (e.g., adjuvant)) from a distribution of elements in a multidimensional space without external criteria or designation of the number of groups. As used herein, a "cluster" of drug components (e.g., adjuvants) refers to a collection of similar drug components among a large number of drug components (e.g., adjuvants). Drug components (e.g., adjuvants) belonging to the same cluster have the same (e.g., identical or similar) effect (e.g., adjuvant function (e.g., cytokine stimulation or the like)). This can be classified by multivariate analysis. A cluster can be constituted by using various cluster analysis approaches. The cluster of drug components (e.g., adjuvants) provided by the present invention is capable of classification by the function of the drug components (e.g., adjuvants) by showing that a drug component belongs to the cluster. Further, drug components (e.g., adjuvants) belonging to the same cluster after classification can be predicted as having a property that is characteristic to the cluster with high precision and reasonable probability. A reasonable probability can be appropriately set at, for example, 99%, 98%, 97%, 96%, 95%, 90%, 85%, 80%, 75%, 70%, or the like depending on the parameter used in cluster analysis.

As used herein, "identical" or "similar" function is used for results after cluster analysis. Functions, when having

substantially the same degree of activity, are referred to as identical, and when having qualitatively the same activity but different amount for a property, are referred to as "similar". Such a degree of similarity can be appropriately determined such as 99%, 98%, 97%, 96%, 95%, 90%, 85%, 80%, 75%, 70%, or the like. As used herein, "identical cluster" refers to being in the same cluster in cluster analysis. Whether it is possible to be in the same cluster can be determined by similarity.

As used herein, "similarity" refers to the degree of similarity of expression profiles for molecules of drug components such as an active ingredient, additive, or adjuvant or a part thereof. Similarity can be defined by the degree of gene expression of the sDEG defined herein or the like and determined based on the degree of expression, amount, active amount, or the like. Although not wishing to be bound by any theory, in some embodiments of the invention, it is understood that drug components (e.g., active ingredients, additives, or adjuvants) belonging to the same cluster by classifying drug components (e.g., active ingredients, additives, or adjuvants) based on similarity have the same feature as the drug components (e.g., active ingredients, additives, or adjuvants) in the same category. Therefore, features of novel drug components (e.g., active ingredients, additives, or adjuvants) or drug components (e.g., active ingredients, additives, or adjuvants) with an unknown function can be analyzed by investigating whether drug components belong to the same drug component cluster (e.g., adjuvant cluster) by using the approach of the invention. To study the similarity herein, a "similarity score" can be used. "Similarity score" refers to a specific numerical value indicating the similarity. For example, a suitable score can be used appropriately in accordance with the technique used for calculating the transcriptome expression pattern or the like. A similarity score can be calculated using a technology used in artificial intelligence (AI) such as a regressive approach, neural networking method, machine learning algorithm such as support vector machine or random forest, or the like. An example of performing cluster analysis is shown in Figure **20.**

As an important indicator, it is suitable to determine a threshold value so that expression patterns of drug components (e.g., active ingredient, additive, or adjuvant) known to have identical or similar function match well. If statistical significance is prioritized, other threshold values can also be used. Those skilled in the art can appropriately determine a threshold value by referring to the descriptions herein depending on the situation. For example, values with a maximum distance found from cluster analysis using a hierarchical clustering approach (e.g., group average method (average linkage clustering)), nearest neighbor method (NN method), K-NN method, Ward’s method, furthest neighbor method, or centroid method) less than a specific value can be considered as the same cluster. Examples of such a value include, but are not limited to, less than 1, less than 0.95, less than 0.9, less than 0.85, less than 0.8, less than 0.75, less than 0.7, less than 0.65, less than 0.6, less than 0.55, less than 0.5, less than 0.45, less than 0.4, less than 0.35, less than 0.3, less than 0.25, less than 0.2, less than 0.15, less than 0.1, less than 0.05, and the like. Clustering approaches are not limited to hierarchical approaches (e.g., nearest neighbor method). A non-hierarchical approach (e.g., k-means method) can be used. A hierarchical clustering can be preferably used.

Those skilled in the art can appropriately determine the distance (similarity) between elements to be classified in clustering. Examples of distances between elements that are commonly used include Euclidian distance, Mahalanobis distance, or cosine similarity (distance), and the like.

Software for performing hierarchical clustering is not limited. Examples thereof include Java®-based free software, Clustering Calculator (Brzustowski, J.) (http://www2.biology.ualberta.ca/jbrzusto/cluster.php/) .

The following output can be obtained by inputting vector data into such software:

Height of connection position of tree (arbitrary unit);
Topology of tree;
Distance between each node of tree (arbitrary unit)
Bootstrap value of each connection (e.g., 1000 runs). Other outputs can also be optionally obtained.

A tree diagram can be drawn by using a suitable software from such output data (including, but not limited to, Phylip/DRAWTREE format, and (hierarchical trees) using Tree Explorer software, Tamura, K., available at http://www.evolgen.biol.)

In addition to bootstrap values (also called bp), values such as p-value of multiscale bootstrap (Au) can also be outputted. Such values indicate the mathematical stability of clustering. AU is parameter that is often used in sequence analysis or the like, which can be suitable for indicating the stability of a phylogenetic tree.

Hierarchical clustering is classified into divisive and agglomerative clustering. For agglomerative clustering that is typically used, an initial state with N clusters comprising only one subject is first created when data consisting of N subjects are given. From this state, distance d between clusters (C1, C2) is calculated from distance d between subjects x1 and x2 (x1, x2) (dissimilarity), and two clusters with the shortest distance are successively merged. Such merging is repeated until all subjects are merged into a single cluster to obtain a hierarchical structure. The hierarchical structure is displayed as a dendrogram. A dendrogram is a binary tree in which each terminal node represents each subject, and a cluster formed by merging is represented by a non-terminal node. The horizontal axis of a non-terminal node represents the distance between clusters when merged. There are several approaches depending on the difference in the distance

function d (C1, C2) of clusters C1 and C2, including nearest neighbor method or single linkage method; furthest neighbor method or complete linkage method; group average method; Ward's method (Ward's method minimizes the sum of square of distance from each subject to the centroid of the cluster including the subject), and the like. The nearest neighbor method, furthest neighbor method, and group average method can be applied when the distance d (xi, xj) between any subjects are given. The distance after merging clusters can be updated with constant time by the Lance-Williams update formula (G.N.Lance and W.T.Williams, The Computer Journal, vol.9, pp.373-380 (1967)). This can be applied by finding the Euclidean distance between vectors if subjects are described as a numerical vector. For Ward's method, a predetermined formula can be directly applied if subjects are given as numerical vectors. If only the distance between subjects is given, this can be applied by updating the distance using the Lance-Williams update formula. The amount of calculation in a normal case where the distance can be updated with a constant time by the Lance-Williams update formula can be found by $0(N^2 \log N)$. Meanwhile, the distance updating approach described above has a property of reducibility. An algorithm (F.Murtagh, The Computer Journal, vol.26, pp.354-359 (1983)) is known which can calculate in the time $0(N^2)$ by utilizing the property of the nearest neighbor graph. The algorithm can be implemented by using known information in the Olson document (C.F.Olson: Parallel Computing, Vol.21, pp.1313-1325 (1995)) or the like for the amount of calculation on a coloring parallel computer. An example of the hierarchical clustering analysis of the invention is provided in the Examples, such as Figure **14.**

Hierarchical clustering can be performed in each organ for each drug component (e.g., active ingredient, additive, or adjuvant) and for each gene probe (Figure **11).** In such analysis, the ratio of cell population responding to a drug component (e.g., adjuvant) can also be analyzed (Figure **12).** Cells can also be analyzed for each type of immune cells (Figure **13).**

In one embodiment of the invention herein, transcriptome analysis can be performed by administering a target drug component (e.g., active ingredient, additive, or adjuvant) to a target organism and comparing a transcriptome in a target organ at a certain time after administration with a transcriptome in the same or corresponding organ prior to administration of the drug component (e.g., adjuvant), and identifying a set of differentially expressed genes (DEGs) as a result of the comparison.

As used herein, "differentiently expressed gene" or "differentially expressed gene" (DEG) refers to a gene whose expression has changed (e.g., increased, decreased, manifested, or eliminated) as a result of administering a drug component (e.g., active ingredient, additive, or adjuvant) to the target organism and comparing a transcriptome in the organ at a certain time after administration with a transcriptome in the organ prior to administration of the drug component (e.g., active ingredient, additive, or adjuvant). If the change is "significant", the gene is referred to as "significant DEG" or "sDEG". In this regard, significant change generally refers to, but is not limited to, a statistically significant change. If suitable for the objective of the invention, significance can be determined using appropriate criteria. DEG determination methods are exemplified in the Examples. Representative examples include, but are not limited to the following: the change can be defined as a statistically significant change (upregulation or downregulation) satisfying all of the following conditions: a predetermined threshold value when determining a significant DEG is identified by a predetermined difference in multiple and predetermined statistical significance (p value); typically the mean fold change (FC) is > 1.5 of < 0.667, the p value in associated t-test is < 0.01 without multiple testing correction, and customized PA call is 1. Other identification methods can also be used. In a preferred embodiment, a gene whose expression has changed beyond a predetermined threshold value is identified as a result of comparison and differentially expressed genes in the common manner among identified genes are selected to generate a set of significant DEG. Analysis of DEGs can use any approach that can analyze differential expression. The volcano plot used in the Examples is a scatter diagram arranging the statistical effect on the y axis and the biological effect on the x axis in all individuals/property matrices. The only limitation is that only examination of the difference between the levels of qualitative explanatory variables of two levels can be executed. In a volcano plot, the coordinate of the y axis is generally scaled by -log10 (p value) to facilitate reading of the diagram. High values reflect the most significant effect, while low values correspond to a barely significant effect. Since a statistically significant effect is not necessarily interesting in the biological scale, use of a volcano plot can reduce the risk of experiments involving very accurate measurements due to a large number of repetitions possibly providing a lower p value associated with a very small biological difference. Therefore, analysis focusing not only on the p value but also the biological effect can be performed.

When analyzing DEGs, a plurality of samples are generally processed, while response can vary in such a plurality of samples. In such a case, the reaction is represented as a Venn diagram (e.g., Figure **8).** A large overlap thereof can be determined as consistent expression with high universality. The present invention has found that a ratio of overlap in a Venn diagram is correlated with the number of upregulated gene probes. By plotting in this manner, potent gene responses of a drug component induced property (e.g., efficacy of active ingredient, assistive function of additive, and adjuvant function) can be determined by Venn diagram analysis. Figure **17** provides an example of annotation analysis and Venn diagram of genes significantly upregulated with a CpG adjuvant. Likewise, Figure **18** provides an example thereof for cdiGMP.

As used herein, a set of all sDEGs for each organ and drug component is referred to as a "drug component

gene space". As used herein, a set of all sDEGs for each organ and adjuvant is referred to as an "adjuvant gene space". Likewise, this can be referred to as "active ingredient gene space" for active ingredients and "additive gene space" for additives.

As used herein, integration of a set of DEGs for two or more drug components (e.g., active ingredient, additive, or adjuvant) to generate a set of differentially expressed genes (DEG) in the common manner can be referred to as shared DEG set generation. An embodiment herein can perform the transcriptome analysis for at least two or more organs to identify a set of differentially expressed genes only in a specific organ and using the set as the organ specific gene set. Therefore, "organ specific gene set" as used herein refers to a set of differentially expressed genes specifically to a certain organ.

As used herein, "organ" refers to a unit constituting the body of a multicellular organism such as an animal or plant among organisms, which is morphologically distinct from the surroundings and serves a set of functions as a whole. Representative examples include, but are not limited to, liver, spleen, and lymph nodes, as well as other organs such as kidney, lung, adrenal glands, pancreas, and heart.

As used herein, "number enabling statistically significant clustering analysis" is the number related to adjuvants, referring to the number of samples from which a statistically significant difference can be detected upon clustering analysis. Those skilled in the art can appropriately determine the detection power or the like and determine the number based on conventional techniques in the field of statistics.

As used herein, "gene marker" refers to a substance used as an indicator for evaluating the condition or action of a target, referring to a gene related substance when correlating with the expression level of a gene herein. As used herein, "gene marker" can also be referred to as a "marker", unless specifically noted otherwise.

A gene (marker) group associated with a drug component (e.g., active ingredient, additive, or adjuvant) can be represented using, but not limited to, a z-score heat map approach (Figure **15).**

As used herein, "drug component evaluation marker" refers to a gene marker that is unique to or specific to a specific drug component or a drug component cluster and a specific organ. A drug component evaluation marker can be unique to or specific to a plurality of organs or drug components or drug component clusters, but in such a case the specific organs or drug components or drug component clusters can be identified by concurrently using another marker. In regard to such a relationship, a gene with a significant relationship shared among drug component related genes is selected. A drug component group of upregulated genes can be selected based on the z-score of the expression (see Figure **16).**

If a drug component is an adjuvant, a drug component evaluation marker is referred to as an "adjuvant evaluation marker". In other words, "adjuvant evaluation marker" as used herein refers to a gene marker that is unique to or specific to a specific adjuvant or adjuvant cluster and a specific organ. An adjuvant evaluation marker can be unique to or specific to a plurality of organs or adjuvants or adjuvant clusters, but in such a case the specific organs or adjuvants or adjuvant clusters can be identified by concurrently using another marker. In regard to such a relationship, a gene with a significant relationship shared among adjuvant related genes is selected. An adjuvant group of upregulated genes can be selected based on the z-score of the expression (see Figure **16).** This can be referred to as an "active ingredient evaluation marker" if a drug component is an active ingredient, and as an "additive evaluation marker" for additives.

A biological process can be annotated by analyzing transcriptome profile data. Such annotation can be performed using software such as TargetMine. Annotation can be represented by a keyword. Wounding, cell death, apoptosis, NFκB signaling pathway, inflammatory response, TNF signaling pathway, cytokines, migration, chemokine, chemotaxis, stress, defense response, immune response, innate immune response, adaptive immune response, interferons, interleukins, or the like can be used. This can be separated for each organ, route of administration, or the like (see Figure **10).** Examples of annotations for wounding include regulation of response to wounding; response to wounding; and positive regulation of response to wounding. Examples of annotations for cell death include cell death; death; programmed cell death; regulation of cell death; regulation of programmed cell death; positive regulation of programmed cell death; positive regulation of cell death; negative regulation of cell death; and negative regulation of programmed cell death. Examples of annotations for apoptosis include apoptotic process; regulation of apoptotic process; apoptotic signaling pathway; intrinsic apoptotic signaling pathway; positive regulation of apoptotic process; regulation of apoptotic signaling pathway; negative regulation of apoptotic process; and regulation of intrinsic apoptotic signaling pathway. Examples of annotations for NFκB signaling pathway include NF-kappa B signaling pathway; I-kappa B kinase/NF-kappa B signaling; positive regulation of I-kappa B kinase/NF-kappa B signaling; and regulation of I-kappa B kinase/NF-kappa B signaling. Examples of annotations for inflammatory response include inflammatory response; regulation of inflammatory response; positive regulation of inflammatory response; acute inflammatory response; and leukocyte migration involved in inflammatory response. Examples of annotations for TNF signaling pathway include TNF signaling pathway. Examples of annotations for cytokines include response to cytokine; Cytokine-cytokine receptor interaction Endocytosis; cellular response to cytokine stimulus; Cytokine-cytokine receptor interaction; cytokine production; regulation of cytokine production; cytokine-mediated signaling pathway; cytokine biosynthetic process; cytokine metabolic process; positive regulation of cytokine production; negative regulation of cytokine production; regulation of cytokine biosynthetic process;

regulation of tumor necrosis factor superfamily cytokine production; tumor necrosis factor superfamily cytokine production; positive regulation of cytokine biosynthetic process; cytokine secretion; and positive regulation of tumor necrosis factor superfamily cytokine production. Examples of annotations for migration include positive regulation of leukocyte migration; cell migration; leukocyte migration; regulation of leukocyte migration; neutrophil migration; positive regulation of cell migration; granulocyte migration; myeloid leukocyte migration; regulation of cell migration; and lymphocyte migration. Examples of annotations for chemokine include chemokine-mediated signaling pathway; chemokine production; regulation of chemokine production; and positive regulation of chemokine production. Examples of annotations for chemotaxis include cell chemotaxis; chemotaxis; leukocyte chemotaxis; positive regulation of leukocyte chemotaxis; taxis; granulocyte chemotaxis; neutrophil chemotaxis; positive regulation of chemotaxis; regulation of leukocyte chemotaxis; regulation of chemotaxis; and lymphocyte chemotaxis. Examples of annotations for stress include response to stress; regulation of response to stress; and cellular response to stress. Examples of annotations for defense response include defense response; regulation of defense response; positive regulation of defense response; defense response to other organism; defense response to bacterium; defense response to Gram-positive bacterium; defense response to protozoan; defense response to virus; regulation of defense response to virus; regulation of defense response to virus by host; and negative regulation of defense response. Examples of annotations for immune response include immune response; positive regulation of immune response; regulation of immune response; activation of immune response; immune response-activating signal transduction; immune response-regulating signaling pathway; negative regulation of immune response; and production of molecular mediator of immune response. Examples of annotations for innate immune response include innate immune response; regulation of innate immune response; positive regulation of innate immune response; activation of innate immune response; innate immune response-activating signal transduction; and negative regulation of innate immune response. Examples of annotations for adaptive immune response include adaptive immune response based on somatic recombination of immune receptors built from immunoglobulin superfamily domains; adaptive immune response; positive regulation of adaptive immune response; regulation of adaptive immune response; and regulation of adaptive immune response based on somatic recombination of immune receptors built from immunoglobulin superfamily domains. Examples of annotations for interferons include response to interferon-alpha; interferon-alpha production; cellular response to interferon-alpha; positive regulation of interferon-alpha production; regulation of interferon-alpha production; cellular response to interferon-beta; response to interferon-beta; positive regulation of interferon-beta production; regulation of interferon-beta production; interferon-beta production; response to interferon-gamma; cellular response to interferon-gamma; interferon-gamma production; and regulation of interferon-gamma production. Examples of annotations for interleukins include interleukin-6 production; regulation of interleukin-6 production; positive regulation of interleukin-6 production; interleukin-12 production; regulation of interleukin-12 production; and positive regulation of interleukin-12 production. Examples of biological indicators include cytokine profiles. Cytokine profiles include, but are not limited to IFNA2; IFNB1; IFNG; IFNL1; IFNA1/IFNA13; IL15; IL4; IL1RN; IFNK; IFNA4; IL1B; IL12B; TNFSF10; TNF; IFNA10; IFNA21; IFNA5; IFNA7; IFNA14; IFNA6; IFNE; IFNA8; IFNA16; CD40LG; IL6; IL2; IL12A; IL27; OSM; IFNA17; EBI3; IL10; IFNW1; TNFSF11; IL7; and the like.

As analyzed items, a plot of a hematological indicator and gene expression can also be applied (Figure **19).** In this regard, examples of hematological indicators include, but are not limited to, white blood cells (WBC), lymphocytes (LYM), monocytes (MON), granulocytes (GRA), relative (%) content of lymphocytes (LY%), relative (%) content of monocytes (MO%), relative (%) content of granulocytes (GR%), red blood cells (RBC), hemoglobins (Hb, HGB), hematocrits (HCT), mean corpuscular volume (MCV), mean corpuscular hemoglobins (MCH), mean corpuscular hemoglobin concentration (MCHC), red blood cell distribution width (RDW), platelets (PLT), platelet concentration (PCT), mean platelet volume (MPV), platelet distribution width (PDW), and the like.

By using the present invention, drug components (e.g., active ingredients, additives, or adjuvants) can be grouped by cluster analysis (Figure **21).**

Biological activity can also be represented, for example, by an absolute value or relative value of absorbance or the like for data using a binding constant or dissociation constant of an antigen-antibody reaction or binding constant or dissociation constant to an antigen of each antibody when using two or more antibodies in a binding assay or the like, or data from ELISA or the like.

The detecting agent or detecting means used in the analysis of the invention can be any means, as long as a gene or the expression thereof can be detected.

The detecting agent or detecting means of the present invention may be a complex or composite molecule in which another substance (e.g., label or the like) is bound to a portion enabling detection (e.g., antibody or the like). As used herein, "complex" or "composite molecule" refers to any construct comprising two or more portions. For instance, when one portion is a polypeptide, the other portion may be a polypeptide or other substances (e.g., sugar, lipid, nucleic acid, other carbohydrate or the like). As used herein, two or more constituent portions of a complex may be bound by a covalent bond or any other bond (e.g., hydrogen bond, ionic bond, hydrophobic interaction, Van der Waals force, the like). When two or more portions are polypeptides, the complex may be called a chimeric polypeptide. Thus, "complex" as used herein includes molecules formed by linking a plurality of types of molecules such as a polypeptide, polynucleotide,

lipid, sugar, or small molecule.

As used herein, "detection" or "quantification" of polynucleotide or polypeptide or polypeptide expression can be accomplished by using a suitable method including, for example, an immunological measuring method and measurement of mRNAs, including a bond or interaction to a marker detecting agent. However, measurement can be performed with the amount of PCR product in the present invention. Examples of a molecular biological measuring method include northern blot, dot blot, PCR and the like. Examples of an immunological measurement method include ELISA using a microtiter plate, RIA, fluorescent antibody method, luminescence immunoassay (LIA), immunoprecipitation (IP), single radial immunodiffusion (SRID), turbidimetric immunoassay (TIA), western blot, immunohistochemical staining and the like. Further, examples of a quantification method include ELISA, RIA and the like. Quantification may also be performed by a gene analysis method using an array (e.g., DNA array, protein array). DNA arrays are outlined extensively in (Ed. by Shujunsha, Saibo Kogaku Bessatsu "DNA Maikuroarei to Saishin PCR ho" [Cellular engineering, Extra issue, "DNA Microarrays and Latest PCR Methods"]. Protein arrays are discussed in detail in Nat Genet. 2002 Dec; 32 Suppl: 526-32. Examples of a method of analyzing gene expression include, but are not limited to, RT-PCR, RACE, SSCP, immunoprecipitation, two-hybrid system, in vitro translation and the like, in addition to the methods discussed above. Such additional analysis methods are described in, for example, Genomu Kaiseki Jikkenho Nakamura Yusuke Labo Manyuaru [Genome analysis experimental method Yusuke Nakamura Lab Manual], Ed. by Yusuke Nakamura, Yodosha (2002) and the like. The entirety of the descriptions therein is incorporated herein by reference.

As used herein, "means" refers to anything that can be a tool for accomplishing an objective (e.g., detection, diagnosis, therapy). In particular, "means for selective recognition (detection)" as used herein refers to means capable of recognizing (detecting) a certain subject differently from others.

As used herein, "(nucleic acid) primer" refers to a substance required for initiating a reaction of a polymeric compound to be synthesized in a polymer synthesizing enzyme reaction. A synthetic reaction of a nucleic acid molecule can use a nucleic acid molecule (e.g., DNA, RNA or the like) complementary to a portion of a sequence of a polymeric compound to be synthesized. A primer can be used herein as a marker detecting means.

Examples of a nucleic acid molecule generally used as a primer include those having a nucleic acid sequence with a length of at least 8 contiguous nucleotides, which is complementary to a nucleic acid sequence of a gene of interest (e.g., marker of the invention). Such a nucleic acid sequence may be a nucleic acid sequence with a length of preferably at least 9 contiguous nucleotides, more preferably at least 10 contiguous nucleotides, still more preferably at least 11 contiguous nucleotides, at least 12 contiguous nucleotides, at least 13 contiguous nucleotides, at least 14 contiguous nucleotides, at least 15 contiguous nucleotides, at least 16 contiguous nucleotides, at least 17 contiguous nucleotides, at least 18 contiguous nucleotides, at least 19 contiguous nucleotides, at least 20 contiguous nucleotides, at least 25 contiguous nucleotides, at least 30 contiguous nucleotides, at least 40 contiguous nucleotides, or at least 50 contiguous nucleotides. A nucleic acid sequence used as a probe comprises a nucleic acid sequence that is at least 70% homologous, more preferably at least 80% homologous, still more preferably at least 90% homologous, or at least 95% homologous to the aforementioned sequence. A sequence that is suitable as a primer may vary depending on the property of a sequence intended for synthesis (amplification). However, those skilled in the art are capable of designing an appropriate primer in accordance with an intended sequence. Design of such a primer is well known in the art, which may be performed manually or by using a computer program (e.g., LASERGENE, PrimerSelect, or DNAStar).

As used herein, "probe" refers to a substance that can be means for search, which is used in a biological experiment such as in vitro and/or in vivo screening. Examples thereof include, but are not limited to, a nucleic acid molecule comprising a specific base sequence, a peptide comprising a specific amino acid sequence, a specific antibody, a fragment thereof, and the like. As used herein, a probe can be used as marker detecting means.

If a drug component is an adjuvant herein, examples of classification of adjuvant include, but are not limited to G1 (interferon signaling); G2 (metabolism of lipids and lipoproteins); G3 (response to stress); G4 (response to wounding); G5 (phosphate-containing compound metabolic process); G6 (phagosome), and the like (see Figures **2, 14,** and **21).** Such classification was only found by executing the method of generating an organ transcriptome profile of a drug component (e.g., adjuvant) of the invention.

A reference drug product (also referred to as a standard drug component) for classifying drug components can be determined by using the present invention. This is exemplified herein. If a drug component is an adjuvant, a reference adjuvant (standard adjuvant) for adjuvant classification can be identified. For example for the G1 to G6, the reference adjuvant (standard adjuvant) of G1 is selected from the group consisting of cdiGMP, cGAMP, DMXAA, PolyIC, and R848, the reference adjuvant (standard adjuvant) of G2 is bCD, the reference adjuvant (standard adjuvant) of G3 is FK565, the reference adjuvant (standard adjuvant) of G4 is MALP2s, the reference adjuvant (standard adjuvant) of G5 is selected from the group consisting of D35, K3, and K3SPG, and/or the reference adjuvant (standard adjuvant) of G6 is AddaVax. The cdiGMP, cGAMP, DMXAA, PolyIC, and R848 are RNA related adjuvants (STING ligands), and cdiGMP elicits a Th1 reponse and DMXAA elicits a Th2 response. G1 can be deemed as biological function: interferon response (type I, type II), and stress related drug component (e.g., active ingredient, additive, or adjuvant). G2 is a metabolism of lipids and lipoproteins, and bCD is a typical drug component (e.g., active ingredient, additive, or adjuvant) while ALM

also has a similar action, and biological function includes inflammatory cytokine, lipid metabolism, and DAMP (action with host derived dsDNA) action. G3 is a response to stress cluster, and representative drug components (e.g., adjuvant) include FK565, and examples of biological function include T-cell cytokine, NK cell cytokine, stress response, wounding response, PAMP, and the like. G4 is response to wounding, and representative drug components (e.g., adjuvants) include MALP2s, and examples of biological function include TNF response, stress response, wounding response, and PAMP. G5 is a phosphate-containing compound metabolic process, and CpG (D35, K3, K3SPG) as well as TLR9 ligands are representative drug components (e.g., active ingredients, additives, or adjuvants), and examples of biological functions include nucleic acid metabolism and phosphoric acid containing compound metabolism. G6 is phagosome, and AddaVax (MF59) is a representative drug component (e.g., adjuvant), and examples of biological functions include phagosome (phagocytosis), ATP, and the like. If a drug component is an active ingredient, the above term can be referred to as a reference (standard) active ingredient or the like. For an additive, the term can be referred to as a representative additive, reference (standard) additive, or the like.

For G1 (interferon signaling), typically a STING ligand is a reference drug component (e.g., reference active ingredient, reference additive, or reference adjuvant). Typical examples thereof include cdiGMP, cGAMP, DMXAA, PolyIC, and R848, including an RNA related adjuvant (STING ligand). CdiGMP elicits a Th1 response, and DMXAA elicits a Th2 response. G1 can be deemed as biological function: interferon response (type I, type II), and stress related drug component (e.g., active ingredient, additive, or adjuvant). "STING" (stimulator of interferon genes) is an adaptor protein, identified as a membrane protein localized in endoplasmic reticulum, activating TBK1 and IRF3 by stimulation of dsDNA to induce the expression of type I interferon. A STING ligand is a ligand to STING. Interferon is secreted by stimulation of STING. Examples of STING include cdiGMP, cGAMP, DMXAA, PolyIC, R848, 2’3’-cGAMP, and the like. cdiGMP is a cyclic diGMP. cGAMP is cyclic AMP-AMP. DMXAA is 5,6-dimethyxanthenone-4-acetic acid. PolyIC is also referred to as Poly I:C. R848 is resiquimod.

A gene with a significant difference in the expression (significant DEG) in transcriptome analysis of G1 comprises at least one selected from the group consisting of Gm14446, Pml, H2-T22, Ifit1, Irf7, Isg15, Stat1, Fcgr1, Oas1a, Oas2, Trim12a, Trim12c, Uba7, and Ube216.

G2 (metabolism of lipids and lipoproteins) is a lipid and lipoprotein metabolizing property. β cyclodextrin (bCD) is a representative drug component (e.g., representative active ingredient, representative additive, or representative adjuvant) and a reference drug component (e.g., reference active ingredient, reference additive, or reference adjuvant). Meanwhile, ALM (D35, K3 (LV)) also has a similar action. Examples of biological functions include inflammatory cytokine, lipid metabolism and DAMP (action with host derived dsDNA) action. bCD is an abbreviation of β cyclodextrin and a representative example used as an adjuvant.

A gene with a significant difference in the expression (significant DEG) in transcriptome analysis of G2 comprises at least one selected from the group consisting of Elovl6, Gpam, Hsd3b7, Acer2, Acox1, Tbl1xr1, Alox5ap, and Ggt5.

G3 (response to stress) is a stress responsive cluster. Representative adjuvants include FK565 (heptanoyl-γ-D-glutamyl-(L)-meso-diaminopimelyl-(D)-alanine), which is an immune reactive peptide. Examples of biological functions include T cell cytokine, NK cell cytokine, stress response, wounding response, PAMP, and the like.

A gene with a significant difference in the expression (significant DEG) in transcriptome analysis of G3 comprises at least one selected from the group consisting of Bbc3, Pdk4, Cd55, Cd93, Clec4e, Corola, and Traf3, Trem3, C5ar1, Clec4n, Ier3, Il1r1, Plek, Tbx3, and Trem1.

G4 (response to wounding) is a wounding responsive drug component (e.g., active ingredient, additive, or adjuvant), which can be expressed as a toll-like receptor (TLR) 2 ligand. Representative drug components (e.g., adjuvant) include MALP2s (macrophage activating lipopeptide 2). Examples of biological functions include TNF response, stress response, wounding response, and PAMP.

A gene with a significant difference in the expression (significant DEG) in transcriptome analysis of G4 comprises at least one selected from the group consisting of Ccl3, Myof, Papss2, Slc7a11, and Tnfrsflb.

G5 (phosphate-containing compound metabolic process) is a drug component (e.g., active ingredient, additive, or adjuvant) with a phosphate-containing compound metabolic process. CpG (D35, K3, K3SPG, and the like) is a representative drug component (e.g., representative adjuvants) and a reference drug component (e.g., reference adjuvant). TLR9 ligands and the like are also representative examples. Examples of biological functions include nucleic acid metabolism and phosphoric acid containing compound metabolism.

A gene with a significant difference in the expression (significant DEG) in transcriptome analysis of G5 comprises at least one selected from the group consisting of Ak3, Insm1, Nek1, Pik3r2, and Ttn.

G6 (phagosome) is an adjuvant with phagosome. Squalene oil-in-water emulsion adjuvant such as AddaVax and MF59 are representative drug components (e.g., representative active ingredients, representative additives, or representative adjuvants) and reference drug components (e.g., reference active ingredients, reference additives, or reference adjuvants). Examples of biological functions include phagosome (phagocytosis), ATP, and the like.

A gene with a significant difference in the expression (significant DEG) in transcriptome analysis of G6 comprises at least one selected from the group consisting of Atp6v0d2, Atp6v1c1, and Clec7a.

While the drug components described above (e.g., active ingredients, additives, and adjuvants) can be denoted by an abbreviation, the full name thereof (if any), typical source, physical property, receptor (if any), and reference articles were summarized in the following table.

[Table 1-1]

| Abbreviation | Typical source | Full name/ explanation | Physical property | Receptor | Reference article |
|---|---|---|---|---|---|
| AddaVax | InvivoGen (VacciGrade) | Emulsion similar to AddaVax® MF59 | Squalene oil-in-water emulsion adjuvant | Unknown | Calabro S et al., Vaccine, 2013 Jul 18; 31 (33): 3363-9.; Caproni E et al., J Immunol. 2012 April 1; 188 (7): 3088-98. |
| ADX | Vaxine | Advax® | Delta inulin semicrystalline particle | Unknown | Honda-Okubo Y et al., Vaccine. 2012 August 3; 30(36): 5373-81.: Saade F et al., Vaccine. 2013 April 8; 31 (15): 1999-2007 |
| ALM | Brenntag | Aluminum hydroxide gel (ALUM) | Gel suspension | Unknown | |
| bCD | ISP Cavitron W7 HP7 Pharma | Hydroxypropyl-β-cyclodextrin | Cyclodextrin | Unknown | |
| cdiGMP | Yamasa Corporation | Cyclic diguanylate monophosphate | Cyclic dinucleotide | STING | |
| cGAMP | InvivoGen | Cyclic [G(2'5')pA (3',5 ')p] | Cyclic dinucleotide | STING | |
| D35 | GeneDesign | A class CpG ODN (5'-GGT GCA TCG ATG CAG GGG GG-3') | Synthetic oligodeoxyribonucleotide | TLR9 | Verthelyi D, Ishii KJ, Gursel M, Takeshita F, Klinman DM, J Immunol. 2001 Feb 15; 166(4): 2372-7.; Aoshi T et al., J Immunol Res. 2015; 2015: 316364. |
| DMXAA | Sigma-Aldrich | 5,6-dimethyl-9-oxo-9H-xanthene-4-acetate | Synthetic chemical substance | STING | |
| ENDCN | Eurocine Vaccines | Endocine® | Monoolein and oleic acid | Unknown | Falkeborn T et al., PLoS One. 2013 August 8; 8(8); e70527.; Maltais AK et al., Vaccine. 2014 May 30; 32 (26): 3307-15. |
| FCA | Sigma-Aldrich | Complete Freund's adjuvant | Mycobacterium/water-in-oil emulsion | CLR and others (but unknown) | |

[Table 1-2]

| | | | | | |
|---|---|---|---|---|---|
| FK565 | Astellas Pharma | Heptanoyl-γ-D-glutamyl-(L)-meso-diaminopimelyl-(D)-alanine | Synthetic peptide glycan | NOD1 | |
| ISAS1VG | SEPPIC | Incomplete | Water-in-oil emulsion | Unknown | |
| | | Freund's adjuvant | | | |
| K3 | GeneDesign | B class CpG ODN (ATCGACTCTCGAGC GTTCTC) | Synthetic oligodeoxyribonucleotide | TLR9 | Verthelyi D, Ishii KJ, Gursel M, Takeshita F, Klinman DM, J Immunol. 2001 Feb 15; 166(4): 2372-7. |
| K3SPG | K3-sAs40 from GeneDesign was combined with SPG in-house | Complex of K3 CpG ODNs and β glucan (schizophyllan, SPG) | Deoxyribonucleotide/ glucan complex | TLR9 | Kobiyama K et al., Proc Natl Acad Sci USA. 2014 Feb 25; 111(8): 3086-91. |
| MALP2s | Camp ridge Peptide Provided by Tsukasa Seya | Macrophage activating lipopeptide 2 short lipopeptide (Pam2CGNNDE) | Lipoprotein | TLR2/6 | Sawahata R et al., Microbes Infect. 2011 Apr; 13(4): 350-8. |
| MBT | InvivoGen | N-acetyl-muranyl-L-alanyl-D-glutamine-n-butyl-ester (Murabutide) | Synthetic peptide glycan | NOD2 | |
| MPLA | InvivoGen (VacciGrade) | Monophosphoryl lipid A | Less toxic derivative of lipopolysaccharide | TLR4 | |
| Pam3CSK4 | InvivoGen (VacciGrade) | Pam3-Cys-Ser-Lys-Lys-Lys-Lys | Synthetic triacylated lipopeptide | TLR1/2 | |
| PolyIC | Sigma-Aldrich | Polyinosinic-1-polycytidylic acid double stranded (Poly I:C) | Ribonucleotide polymer | TLR3 and MDAS | |
| R848 | Enzo Life Sciences | 4-amino-2-(ethoxymethyl) -a,a-dimethyl-1H-imidazo [4,5-c]quinoline-1-ethanol | Guanosine derivative | TLR7 | |

(continued)

| | | | | | |
|---|---|---|---|---|---|
| sHZ | Nippon Zenyaku Kogyo | Synthetic hemozoin β | Hematin crystal | Unknown | Coban C et al., Cell Host Microbe. 2010 Jan 21; 7(1): 50-61; Onishi M et al., Vaccine. 2014 May 23; 32 (25); 3004-9. |

bCD is an abbreviation of β cyclotextrin and is a representative example used as an adjuvant. bCD can also be an additive.

FK565 is a type of immunoreactive peptide. The chemical name is heptanoyl-γ-D-glutamyl-(L)-meso-diaminopimelyl-(D)-alanine, which is explained in detail in J Antibiot (Tokyo). 1983 Aug; 36(8): 1045-50.

MALP2s is an abbreviation of macrophage-activating lipopeptide-2, a Toll-like receptor (TLR) 2 ligand similar to BCG-CWS. This is more specifically represented as [S-(2,3)-bispalmitoyloxy propyl Cys (P2C)-GNNDESNISFKEK, which is described in detail in Akazawa T. et al., CancerSci 2010; 101: 1596-1603 and the like.

As used herein, "CpG motif" refers to a non-methylated dinucleotide moiety of an oligonucleotide, comprising a cytosine nucleotide and the subsequent guanosine nucleotide. This is used as a representative example of an adjuvant. As a result of the analysis of the invention, CpG motifs were found to belong to G5, i.e., nucleic acid metabolism or phosphoric acid containing compound metabolism. 5-methylcytosine may also be used instead of cytosine. CpG oligonucleotides (CpG ODN) are short (about 20 base pairs) single-stranded synthetic DNA fragments comprising an immunostimulatory CpG motif. A CpG oligonucleotide is a potent agonist of Toll-like receptor 9 (TLR9), which activates dendritic cells (DCs) and B cells to induce type I interferons (IFNs) and inflammatory cytokine production (Hemmi, H., et al. Nature 408, 740-745 (2000); Krieg, A.M. Nature reviews. Drug discovery 5, 471-484 (2006)), and acts as an adjuvant of Th1 humoral and cellular immune responses including cytotoxic T lymphocyte (CTL) responses (Brazolot Millan, C.L., Weeratna, R., Krieg, A.M., Siegrist, C.A. & Davis, H.L. Proceedings of the National Academy of Sciences of the United States of America 95, 15553-15558 (1998).; Chu, R.S., Targoni, O.S., Krieg, A.M., Lehmann, P.V. & Harding, C.V. The Journal of experimental medicine 186, 1623-1631 (1997)). In this regard, CpG ODNs were considered to be a potential immunotherapeutic agent against infections, cancer, asthma, and hay fever (Krieg, A.M. Nature reviews. Drug discovery 5, 471-484 (2006); Klinman, D.M. Nature reviews. Immunology 4, 249-258 (2004)). A CpG oligodeoxynucleotide (CpG ODN) is a single stranded DNA comprising an immunostimulatory non-methylated CpG motif, and is an agonist of TLR9. There are four types of CpG ODNs, i.e., K type (also called B type), D type (also called A type), C type, and P type, each with a different backbone sequence and immunostimulatory properties (Advanced drug delivery reviews 61, 195-204 (2009)).

Four different types of immunostimulatory CpG ODNs (A/D, B/K, C, and P types) have been reported. A/D type ODNs are oligonucleotides characterized by a poly-G motif with a centrally-located palindromic (palindromic structure) CpG-containing sequence of phosphodiester (PO) and a phosphorothioate (PS) bond at the 5' and 3' ends, and by high interferon (IFN)-α production from plasmacytoid dendritic cells (pDC). Types other than the A/D type consist of a PS backbone. B/K type ODN contains multiple nonmethylated CpG motifs, typically with a non-palindromic structure. B/K type CpG primarily induces inflammatory cytokines such as interleukin (IL)-6 or IL-12, but has low IFN-α production. B/K type ODN is readily formulated using saline, some of which is still under clinical trial. Two modified ODNs including D35-dAs40 and D35core-dAs40 were discovered by the inventors, which are similarly immunostimulatory as the original D35 in human PBMC and induces high IFN-α secretion in a dose dependent manner. C type and P type CpG ODNs comprise one and two palindromic structure CpG sequences, respectively. Both are capable of activating B cells like K types and activating pDCs like D types, but C-type CpG ODNs more weakly induce IFN-α production than P type CpG ODNs (Hartmann, G., et al. European journal of immunology 33, 1633-1641 (2003); Marshall, J.D., et al. Journal of leukocyte biology 73, 781-792(2003).; and Samulowitz, U., et al. Oligonucleotides 20, 93-101 (2010)).

AddaVax refers to a squalene based oil-in-water adjuvant. MF59 also have a similar structure. As used herein, "squalene based oil-in-water adjuvant" refers to an adjuvant, which is an emulsion with an oil-in-water structure comprising squalene.

The drug component used (e.g., active ingredient, additive, or adjuvant) herein can be isolated or purified. As used herein, a "purified" substance or biological agent (e.g., protein or nucleic acid such as a gene marker or the like) refers to a biological agent having at least a part of a naturally accompanying agent removed. Therefore, the purity of

a purified biological agent is generally higher than the normal state of the biological agent (i.e., concentrated). The term "purified" as used herein refers to the presence of preferably at least 75 wt%, more preferably at least 85 wt%, still more preferably 95 wt%, and most preferably at least 98 wt% of the same type of biological agent. The substance used in the present invention is preferably a "purified" substance. As used herein, "isolated" refers to removal of at least one of any accompanying agent in a naturally occurring state. For example, removal of a specific genetic sequence from a genomic sequence is also referred to as isolation. Therefore, the gene used herein can be isolated.

As used herein, "subject" refers to a target (e.g., organisms such as humans, or cells, blood, serum, or the like extracted from an organism) subjected to the diagnosis, detection, therapy, or the like of the present invention.

As used herein, "agent" broadly may be any substance or another element (e.g., light, radiation, heat, electricity, and other forms of energy) as long as the intended objective can be achieved. Examples of such substances include, but are not limited to, proteins, polypeptides, oligopeptides, peptides, polynucleotides, oligonucleotides, nucleotides, nucleic acids (including for example DNAs such as cDNAs and genomic DNAs, and RNAs such as mRNAs), polysaccharides, oligosaccharides, lipids, organic small molecules (e.g., hormones, ligands, information transmitting substances, organic small molecules, molecules synthesized by combinatorial chemistry, small molecules that can be used as a medicament (e.g., small molecule ligands and the like) and composite molecules thereof).

As used herein, "therapy" refers to the prevention of exacerbation, preferably maintaining the current condition, more preferably alleviation, and still more preferably elimination of a disease or disorder (e.g., cancer or allergy) in case of such a condition, including being capable of exerting an effect of improving or preventing a patient's disease or one or more symptoms accompanying the disease. Preliminary diagnosis conducted for suitable therapy may be referred to as a "companion therapy", and a diagnostic agent therefor may be referred to as "companion diagnostic agent".

As used herein, "therapeutic agent" broadly refers to all agents that are capable of treating the condition of interest (e.g., diseases such as cancer or allergies). In one embodiment of the invention, "therapeutic agent" may be a pharmaceutical composition comprising an active ingredient and one or more pharmacologically acceptable carriers. A pharmaceutical composition can be manufactured, for example, by mixing an active ingredient with the aforementioned carriers by any method that is known in the technical field of pharmaceuticals. Further, usage form of a therapeutic agent is not limited, as long as it is used for therapy. A therapeutic agent may consist solely of an active ingredient or may be a mixture of an active ingredient and any ingredient. Further, the shape of the the carriers is not particularly limited. For example, the carrier may be a solid or liquid (e.g., buffer). Therapeutic agents for cancer, allergies, or the like include drugs (prophylactic agents) used for the prevention of cancer, allergies, or the like, and suppressants of cancer, allergies, or the like.

As used herein, "prevention" refers to the act of taking a measure against a disease or disorder (e.g., diseases such as cancer or allergy) from being in such a condition, prior to the onset of such a condition. For example, it is possible to use the agent of the invention to perform diagnosis, and use the agent of the invention, as needed, to prevent or take measures to prevent allergies or the like.

As used herein, "prophylactic agent" broadly refers to all agents that are capable of preventing the condition of interest (e.g., disease such as cancer or allergies).

As used herein, "kit" refers to a unit providing portions to be provided (e.g., testing agent, diagnostic agent, therapeutic agent, antibody, label, manual, and the like), generally in two or more separate sections. This form of a kit is preferred when intending to provide a composition that should not be provided in a mixed state and is preferably mixed immediately before use for safety reasons or the like. Such a kit advantageously comprises instructions or a manual preferably describing how the provided portions (e.g., testing agent, diagnostic agent, or therapeutic agent) should be used or how a reagent should be processed. When the kit is used herein as a reagent kit herein, the kit generally comprises an instruction describing how to use a testing agent, diagnostic agent, therapeutic agent, antibody, and the like.

As used herein, "instruction" is a document with an explanation of the method of use of the present invention for a physician or for other users. The instruction describes a detection method of the invention, how to use a diagnostic agent, or a description instructing administration of a medicament or the like. An instruction may also have a description instructing oral administration, or administration to the esophagus (e.g., by injection or the like) as the site of administration. The instruction is prepared in accordance with a format specified by a regulatory authority of the country in which the present invention is practiced (e.g., Ministry of Health, Labour and Welfare in Japan, Food and Drug Administration (FDA) in the U.S., or the like), with an explicit description showing approval by the regulatory authority. The instruction is a so-called "package insert", and is generally provided in, but not limited to, paper media. The instructions may also be provided in a form such as electronic media (e.g., web sites provided on the Internet or emails).

As used herein, "diagnosis" refers to identifying various parameters associated with a disease, disorder, condition or the like in a subject to determine the current or future state of such a disease, disorder, or condition. The condition in the body can be examined by using the method, apparatus, or system of the invention. Such information can be used to select and determine various parameters of a formulation, method, or the like for treatment or prevention to be administered, disease, disorder, or condition in a subject or the like. As used herein, "diagnosis" when narrowly defined refers to diagnosis of the current state, but when broadly defined includes "early diagnosis", "predictive diagnosis",

"prediagnosis", and the like. Since the diagnostic method of the invention in principle can utilize what comes out from a body and can be conducted away from a medical practitioner such as a physician, the present invention is industrially useful. In order to clarify that the method can be conducted away from a medical practitioner such as a physician, the term as used herein may be particularly called "assisting" "predictive diagnosis, prediagnosis or diagnosis".

The procedure for formulation as the drug of the invention or the like is known in the art and is described in the Japanese Pharmacopoeia, US Pharmacopoeia, pharmacopoeia of other countries, and the like. Therefore, those skilled in the art can determine the amount to be used without undue experimentation with the descriptions herein.

As used herein, "program" is used in the general meaning used in the art. A program describes the processing to be performed by a computer in order, and is legally considered a "product". All computers are operated in accordance with a program. Programs are expressed as data in modern computers and stored in a recording medium or a storage device.

As used herein, "recording medium" is a recording medium storing a program for executing the present invention. A recording medium can be anything, as long as a program can be recorded. For example, a recording medium can be, but is not limited to, a ROM or HDD or a magnetic disk that can be stored internally, or an external storage device such as flash memory such as a USB memory.

As used herein, "system" refers to a configuration that executes the method of program of the invention. System fundamentally means a system or organization for executing an objective, wherein a plurality of elements are systematically configured to affect one another. In the field of computers, system refers to the entire configuration such as the hardware, software, OS, and network.

(Description of preferred embodiments)

The preferred embodiments of the invention are described hereinafter. It is understood that the embodiments provided hereinafter are provided to better facilitate the understanding of the present invention, and thus the scope of the invention should not be limited by the following descriptions. Thus, it is apparent that those skilled in the art can refer to the descriptions herein to make appropriate modifications within the scope of the present invention. Those skilled in the art can also appropriately combine any embodiments.

<Transcriptome analysis of drug component>

The present invention provides a method of generating an organ transcriptome profile of a drug component (e.g., active ingredient, additive, or adjuvant). The method comprises: (A) obtaining expression data by performing transcriptome analysis on at least one organ of a target organism using two or more drug components (e.g., active ingredients, additives, or adjuvants); (B) clustering the drug components (e.g., active ingredients, additives, or adjuvants) with respect to the expression data; and (C) generating a transcriptome profile of the organ of the drug components (e.g., active ingredients, additives, or adjuvants) based on the clustering.

The transcriptome analysis used in the method of the invention can be embodied by using any approach. Transcriptome analysis can be transcriptome analysis of an organ of a drug component (e.g., active ingredient, additive, or adjuvant) performing transcriptome analysis of an organ of a target organism without administering the drug component (e.g., active ingredient, additive, or adjuvant), obtaining a control transcriptome, performing transcriptome analysis of the same organ of the target organism after administering a candidate drug component (e.g., active ingredient, additive, or adjuvant), and normalizing as needed using the control transcriptome. The same procedure can also be performed on a second or another subsequent drug component (e.g., active ingredient, additive, or adjuvant). Clustering analysis of each drug component (e.g., active ingredient, additive, or adjuvant) can be performed by using expression data obtained from transcriptome analysis for two or more drug components (e.g., active ingredients, additives, or adjuvants). Each drug component (e.g., active ingredient, additive, or adjuvant) can be analyzed based on cluster information of the drug component (e.g., active ingredient, additive, or adjuvant) obtained based on gene expression data, which is the result of clustering analysis. In particular, a drug component (e.g., active ingredient, additive, or adjuvant) belonging to the same cluster as a standard drug component (e.g., active ingredient, additive, or adjuvant) or reference drug component (reference drug component and standard drug component refer to the same component herein) can be estimated to have the same function as the reference drug component (standard drug component). Generation of a transcriptome profile of the organ of the adjuvant based on the clustering can be embodied by using any approach that is known in the art. For example, a profile can use a dendrogram as in Figure **2** or expressed using a spreadsheet software such as Excel®, but the profile is not limited thereto.

(Functional classification of drug component (e.g., active ingredient, additive, or adjuvant))

In one aspect, the present invention provides a method of classifying an adjuvant comprising classifying a drug

component (e.g., active ingredient, additive, or adjuvant) based on transcriptome clustering. The classification based on transcriptome clustering in the present invention can comprise classifying a target drug component (e.g., active ingredient, additive, or adjuvant) based on a result of transcriptome clustering of a reference drug component (e.g., active ingredient, additive, or adjuvant). There are various examples of classification of a drug component (e.g., active ingredient, additive, or adjuvant) including classification by at least one feature selected from the group consisting of classification based on a host response, classification based on a mechanism, classification by application based on a mechanism or cells (liver, lymph node, or spleen), and module classification.

In a representative example, classification of a drug component (e.g., active ingredient, additive, or adjuvant) provided by the present invention comprises at least one classification selected from the group consisting of (1) G1 (interferon signaing); (2) G2 (metabolism of lipids and lipoproteins); (3) G3 (response to stress); (4) G4 (response to wounding); (5) G5 (phosphate-containing compound metabolic process); and (6) G6 (phagosome). For G1 to G6, the corresponding portions can be classified for adjuvants, but some, albeit a small number, are not classified thereto. They can be deemed as not classified to G1 to G6. Additional transcriptome analysis can be performed for further classification as needed.

In a preferred embodiment of the invention, a target substance that has not been classified can be classified by clustering a result of transcriptome analysis using each reference drug component (e.g., active ingredient, additive, or adjuvant) of G1 to G6 and comparing them. In this regard, the reference drug component of G1 is selected from the group consisting of cdiGMP, cGAMP, DMXAA, PolyIC, and R848, the reference drug component of G2 is bCD, the reference drug component of G3 is FK565, the reference drug component of G4 is MALP2s, the reference drug component of G5 is selected from the group consisting of D35, K3, and K3SPG, and/or the reference drug component of G6 is AddaVax. These reference drug components are representative. Other drug components (e.g., active ingredients, additives, or adjuvants) determined as belonging to G1 to G6 can be used instead.

In one embodiment, classification of G1 to G6 is performed based on an expression profile of a gene (identification marker gene; DEG) with a significant difference in expression in transcriptome analysis. The DEG of G1 comprises at least one selected from the group consisting of Gm14446, Pml, H2-T22, Ifit1, Irf7, Isg15, Stat1, Fcgr1, Oas1a, Oas2, Trim12a, Trim12c, Uba7, and Ube216, the DEG of G2 comprises at least one selected from the group consisting of Elovl6, Gpam, Hsd3b7, Acer2, Acox1, Tbl1xr1, Alox5ap, and Ggt5, the DEG of G3 comprises at least one selected from the group consisting of Bbc3, Pdk4, Cd55, Cd93, Clec4e, Coro1a, and Traf3, Trem3, C5ar1, Clec4n, Ier3, Il1r1, Plek, Tbx3, and Trem1, the DEG of G4 comprises at least one selected from the group consisting of Ccl3, Myof, Papss2, Slc7a11, and Tnfrsf1b, the DEG of G5 comprises at least one selected from the group consisting of Ak3, Insm1, Nek1, Pik3r2, and Ttn, and the DEG of G6 comprises at least one selected from the group consisting of Atp6v0d2, Atp6v1c1, and Clec7a.

These DEGs can be used when identifying an alternative to a reference drug component (e.g., active ingredient, additive, or adjuvant). A drug component (e.g., active ingredient, additive, or adjuvant) with substantially the same pattern of the DEG can be used as a reference drug component (e.g., active ingredient, additive, or adjuvant).

Therefore, in one aspect, the present invention provides a gene analysis panel for use in classification of an adjuvant to G1 to G6 or to others. The gene analysis panel comprises a detecting agent or detecting means for detecting at least one DEG selected from the group consisting of a DEG of G1, a DEG of G2, a DEG of G3, a DEG of G4, a DEG of G5, and a DEG of G6, wherein the DEG of G1 comprises at least one selected from the group consisting of Gm14446, Pml, H2-T22, Ifit1, Irf7, Isg15, Stat1, Fcgr1, Oas1a, Oas2, Trim12a, Trim12c, Uba7, and Ube216, wherein the DEG of G2 comprises at least one selected from the group consisting of Elovl6, Gpam, Hsd3b7, Acer2, Acox1, Tbl1xr1, Alox5ap, and Ggt5, wherein the DEG of G3 comprises at least one selected from the group consisting of Bbc3, Pdk4, Cd55, Cd93, Clec4e, Coro1a, and Traf3, Trem3, C5ar1, Clec4n, Ier3, Il1r1, Plek, Tbx3, and Trem1, wherein the DEG of G4 comprises at least one selected from the group consisting of Ccl3, Myof, Papss2, Slc7a11, and Tnfrsf1b, wherein the DEG of G5 comprises at least one selected from the group consisting of Ak3, Insm1, Nek1, Pik3r2, and Ttn, and wherein the DEG of G6 comprises at least one selected from the group consisting of Atp6v0d2, Atp6v1c1, and Clec7a.

Preferably, the gene analysis panel of the invention comprises a detecting agent or detecting means for detecting at least a DEG of G1, a detecting agent or detecting means for detecting at least a DEG of G2, a detecting agent or detecting means for detecting at least a DEG of G3, a detecting agent or detecting means for detecting at least a DEG of G4, a detecting agent or detecting means for detecting at least a DEG of G5, and a detecting agent or detecting means for detecting at least a DEG of G6.

The detecting agent or detecting means contained in the gene analysis panel of the invention can be any means, as long as a gene can be detected.

In one aspect, the present invention provides a method of classifying a drug component, the method comprising:

(a) providing a candidate drug component;
(b) providing a reference drug component set;
(c) obtaining gene expression data by performing transcriptome analysis on the candidate drug component and the

reference drug component set to cluster the gene expression data; and

(d) determining that the candidate drug component belongs to the same group if a cluster to which the candidate adjuvant belongs is classified to the same cluster as at least one in the reference drug component set, and determining as impossible to classify if the candidate drug component does not belong to any cluster. The drug component can be, for example, an active ingredient, additive, adjuvant, or the like.

In one embodiment, the present invention provides a method of classifying an adjuvant. The method comprises: (a) providing a candidate drug component (candidate adjuvant) in at least one organ of a target organism; (b) providing a reference drug component (reference adjuvant) set classified to at least one selected from the group consisting of G1 to G6; (c) obtaining gene expression data by performing transcriptome analysis on the candidate drug component (candidate adjuvant) and the reference drug component (reference adjuvant) set to cluster the gene expression data; and (d) determining that the candidate drug component (candidate adjuvant) belongs to the same group if a cluster to which the candidate drug component (candidate adjuvant) belongs is classified to the same cluster as at least one in groups G1 to G6, and determining as impossible to classify if the cluster does not belong to any cluster.

In the present invention, (a) providing a candidate drug component (candidate adjuvant) in at least one organ of a target organism can be performed by any approach. For example, a novel substance can be obtained or synthesized, or an already commercially available substance can be obtained and provided as a candidate drug component (e.g., candidate adjuvant). Examples of candidate drug components (e.g., candidate adjuvant) include, but are not limited to, a protein, polypeptide, oligopeptide, peptide, polynucleotide, oligonucleotide, nucleotide, nucleic acid, polysaccharide, oligosaccharide, lipid, liposome, oil-in-water molecule, water-in-oil molecule, organic small molecule (e.g., hormone, ligand, information transmitter, organic small molecule, molecule synthesized by combinatorial chemistry, small molecule that can be used as a pharmaceutical product or additive, or the like) and composite molecule thereof.

In the present invention, (b) providing a reference drug component set (e.g., reference adjuvant set classified to at least one selected from the group consisting of G1 to G6) can be performed by any approach. In this regard, the exemplified features of G1 to G6 are described in other parts herein. While anything can be used as a reference drug component (reference adjuvant), typically a reference drug component (e.g., reference adjuvant) of G1 is selected from the group consisting of cdiGMP, cGAMP, DMXAA, PolyIC, and R848, a reference drug component (e.g., reference adjuvant) of G2 is bCD, a reference drug component (e.g., reference adjuvant) of G3 is FK565, a reference drug component (e.g., reference adjuvant) of G4 is MALP2s, a reference drug component (e.g., reference adjuvant) of G5 is selected from the group consisting of D35, K3, and K3SPG, and/or a reference drug component (e.g., reference adjuvant) of G6 is AddaVax. These drug components (e.g, reference adjuvant) can utilize a commercially available, newly synthesized, or manufactured drug component.

In the present invention, (c) obtaining gene expression data by performing transcriptome analysis on the candidate drug component (e.g., candidate adjuvant) and the reference drug component (e.g., reference adjuvant) set to cluster the gene expression data can be performed by any approach. Transcriptome analysis and clustering can be performed by appropriately combining known approaches in the art.

In one embodiment, the transcriptome analysis performed in the present invention administers the drug components (e.g., adjuvants) to the target organism and compares a transcriptome in the organ after a certain time after administration with a transcriptome in the organ before administration of the drug components (e.g., adjuvants), and identifies a set of differentially expressed genes (DEG) (preferably a gene with a statistically significant change, i.e., significant DEG) as a result of the comparison. The series of operations can be standardized. Such a standardized procedure can be that described for example at http://sysimg.ifrec.osaka-u.ac.jp/adjvdb/. It is understood that technologies known in the art can be appropriately applied in this manner for administration of a drug component (e.g., adjuvant), harvesting an organ, RNA extraction, and GeneChip data acquisition. The procedures can be those complying with the law and guidelines meeting the appropriate standards of the facility, and approved by an appropriate committee of the facility to comply with the regulation and ethical standards of authorities.

Examples of administration of a drug component (e.g., adjuvant) include, but are not limited to, administration to a site with low stimulation such as the base of the tail. The administration method can be intradermal (id) administration to the base of a tail, or intraperitoneal (ip), i.n. (intranasal), or oral administration, or the like. The dosage of a drug component (e.g., adjuvant) is selected to induce an excellent effect (e.g., adjuvant function) without inducing severe reactogenicity in a target animal by referring to information known in the art and information in the Examples. A negative control experiment is conducted using a suitable buffer subject group in addition to the drug component (e.g., adjuvant) administration group.

As needed, a preliminary experiment can be conducted to investigate a differentially expressed gene in an organ after administration of a drug component (e.g., adjuvant) and investigate genes with a dramatic change or genes that return to normal after an appropriate period of time has passed. As shown in the Examples, it was found in an example that it is preferable to see the gene expression at 6 hours after administration. In addition, many return to normal after 24 hours. Thus, a change in gene expression can be preferable at, for example, 1 to 20 hours after administration,

preferably after 3 to 12 hours such as any point after 4 to 8 hours or about 6 hours. Gene expression can be investigated at this time or by using a gene chip (e.g., Affimetrix GeneChip microarray system (Affymetrix)) or the like. A sample for testing with a gene chip can be prepared by, for example, preparing Total RNA with an appropriate kit.

Expression can be analyzed by using any approach known in the art. For example, software that is a part of a system such as Affimetrix GeneChip microarray system (Affymetrix) can be used, software can be self-created, or another program available on the Internet or the like can be used.

In one embodiment, the method of the invention comprises integrating the set of DEGs in two or more drug components (e.g., adjuvants) to generate a set of differentially expressed genes (DEG) in the common manner in transcriptome analysis in the present invention. In this regard, the DEG can be preferably a significant DEG. A significant DEG can be extracted by setting any threshold value. Meanwhile in a specific embodiment, a predetermined threshold value used in the present invention can be identified by a difference in a predetermined multiple and a predetermined statistical significance (p value). For example, the value can be defined as a statistically significant change (upregulation or downregulation) satisfying all of the following conditions: Mean fold change (FC) is > 1.5 or < 0.667; p value of associated t-test is < 0.01 without multiple testing correction; and customized PA call is 1. In this regard, other values can be set for FC, which can be greater than 1 fold and 10 fold or less (the other is an inverse thereof), such as 2-fold, 3-fold, 4-fold, 5-fold, 6-fold, 7-fold, 8-fold, 9-fold (and the other is an inverse thereof), or the like. The set of values are generally in a relationship of being inverses, but a combination that is not in a relationship of inverses can also be used. For p values, a baseline other than < 0.01 can also be used, such as < 0.05, < 0.04, < 0.03, < 0.02, or the like, or < 0.009, < 0.008, < 0.007, < 0.006, < 0.005, < 0.004, < 0.003, < 0.002, < 0.001, or the like.

In one embodiment, the method of the invention comprises identifying a gene whose expression has changed beyond a predetermined threshold value as a result of the comparison before and after administration of a drug component (e.g., adjuvant), and selecting differentially expressed genes in the common manner among identified genes to generate a set of significant DEGs. The predetermined threshold value used in this regard can be any threshold value explained in other parts herein. Genes selected as having the same change in the present invention are used as a set of significant DEGs. Such a set of significant DEGs can be used for classification of drug components (e.g., adjuvants).

In one embodiment, the method of the invention comprises performing the transcriptome analysis for at least two or more organs to identify a set of differentially expressed genes only in a specific organ and using the set as the organ specific gene set. If such an organ specific gene set is used, a drug component (e.g., adjuvant) can be classified by only performing transcriptome analysis in a specific organ. A drug component (e.g., adjuvant) can be classified by utilizing comparison with a reference drug component (e.g., reference adjuvant), a standard drug component (e.g., standard adjuvant) or the like.

In another embodiment, the transcriptome analysis performed in the present invention is performed on a large number of organs, preferably on a transcriptome in at least one organ selected from the group consisting of a liver, a spleen, and a lymph node. Although not wishing to be bound by any theory, this is because these organs exhibit results that enable clear identification of a property of an adjuvant as shown in the Examples, but the present invention is not limited thereto. A drug component other than adjuvant (e.g., active ingredient, additive, or the like) and other organs (e.g., kidney, lung, adrenal glands, pancreas, heart, or the like) can also be selected.

In one embodiment, the number of drug components (e.g., adjuvants) to be analyzed by the present invention is a number that enables statistically significant clustering analysis. Such a number can be identified by using common general knowledge associated with statistics. Identification of the number is not the essence of the present invention.

In one embodiment, the method of the invention comprises providing one or more gene markers unique to a specific drug component (e.g., adjuvant) and a specific organ in the determined profile as a drug component (e.g., adjuvant) evaluation marker. By using the drug component (e.g., adjuvant) of the invention, an assay that was not achievable with conventional technology, e.g., unknown drug component (e.g., adjuvant) or a known drug component (e.g., adjuvant) that has not been analyzed, can be evaluated without a large number of experiments. In the present invention, (d) determining that the candidate drug component (e.g., candidate adjuvant) belongs to the same group if a cluster to which the candidate drug component (e.g., candidate adjuvant) belongs is classified to the same cluster as at least one in groups G1 to G6, and determining as impossible to classify if the cluster does not belong to any cluster, can also be determined by analyzing the cluster explained in (c) in the art. Although not wishing to be bound by any theory, as demonstrated by the Examples, gene unique to a specific organ and a specific drug component (e.g., adjuvant) identified by the present invention can be used in cluster analysis. As a result thereof, a drug component (e.g., adjuvant) can be evaluated by comparison with a reference drug component (e.g., reference adjuvant) or standard drug component (e.g., standard adjuvant).

In one embodiment, the method of the invention further comprises analyzing a biological indicator for a drug component (e.g., adjuvant) and correlating with a cluster. Any indicator can be used as the analyzed biological indicator as long as the indicator enables analysis. A biological indicator is an item that is objectively measured/evaluated as an indicator for a normal process or pathological process, or a pharmacological reaction to therapy. The indicator can be measured with a biomarker or the like. Examples of typical biological indicators include, but are not limited to, at least

one indicator selected from the group consisting of a wounding, cell death, apoptosis, NFκB signaling pathway, inflammatory response, TNF signaling pathway, cytokines, migration, chemokine, chemotaxis, stress, defense response, immune response, innate immune response, adaptive immune response, interferons, and interleukins. A biomarker characterizing a condition or change in a disease or the degree of healing is used as a surrogate marker for checking the efficacy of a new drug in a clinical trial. The blood sugar or cholesterol levels or the like are typical biomarkers as indicators for lifestyle diseases. This also includes not only substances derived from an organism contained in the urine or blood, but also electrocardiogram, blood pressure PET image, bone density, lung function, and SNPs. Various biomarkers related to DNA, RNA, biological protein and the like have been found by the advancement in genomic analysis and proteomic analysis.

In one representative embodiment, the biological indicator comprises a hematological indicator.

Examples of such a hematological indicator include, but are not limited to, white blood cells (WBC), lymphocytes (LYM), monocytes (MON), granulocytes (GRA), relative (%) content of lymphocytes (LY%), relative (%) content of monocytes (MO%), relative (%) content of granulocytes (GR%), red blood cells (RBC), hemoglobins (Hb, HGB), hematocrits (HCT), mean corpuscular volume (MCV), mean corpuscular hemoglobins (MCH), mean corpuscular hemoglobin concentration (MCHC), red blood cell distribution width (RDW), platelets (PLT), platelet concentration (PCT), mean platelet volume (MPV), and platelet distribution width (PDW). One or more, or all of these hematological indicators can be measured.

In one embodiment, the biological indicator analyzed in the present invention comprises a cytokine profile. The term "cytokine profile" refers to the amount of various cytokines and the types of cytokines produced in a patient at a certain time. Cytokines are proteins released by white blood cells, having an immunological effect. Examples of cytokines include, but are not limited to, interferon (such as (γ-interferon), tumor necrosis factor, interleukin (IL) 1, IL-2, IL-4, IL-6, and IL-10. Examples of cytokines of the cardiocirculatory system include, but are not limited to, CCL2 (MCP-1), CCL3 (MIP-1α), CCL4 (MIP-1β), CRP, CSF, CXCL16, Erythropoietin (EPO), FGF, Fractalkine (CXC3L1), G-CSF, GM-CSF, IFNγ, IL-1, IL-2, IL-5, IL-6, IL-8, IL-8 (CXCL8), IL-10, IL-15, IL-18, M-CSF, PDGF, RANTES (CCL5), TNFα, VEGF, and the like. In another aspect, the present invention provides a program for implementing a method of generating an organ transcriptome profile of a drug component (e.g., adjuvant) on a computer. The method of implementing a program comprises: (A) obtaining expression data by performing transcriptome analysis on at least one organ of a target organism using two or more drug components (e.g., adjuvants); (B) clustering the drug components (e.g., adjuvants) with respect to the expression data; and (C) generating a transcriptome profile of the organ of the drug components (e.g., adjuvants) based on the clustering. Each step used therein can be carried out in any embodiment that can be employed in the method of the invention or a combination thereof.

In another aspect, the present invention provides a method of manufacturing a composition having a desirable function. The method comprises: (A) providing a candidate drug component; (B) selecting a candidate drug component having a transcriptome expression pattern corresponding to a desirable function; and (C) manufacturing a composition using a selected candidate drug component. In this regard, (A) and (B) can use any feature of providing a candidate drug component (e.g., adjuvant), transcriptome analysis, clustering, and the like in the method of classifying a drug component (e.g., adjuvant) of the invention described herein.

In the present invention, (C) manufacturing a composition using a selected candidate drug component (e.g., candidate adjuvant) can be carried out using any approach that is known in the art. Such manufacturing of a composition can be accomplished, preferably, by mixing a pharmaceutically acceptable carrier, a diluent, an excipient, and/or an active ingredient (antigen or the like for an adjuvant or vaccine) with the selected candidate drug components (e.g., adjuvants). Excipients can include buffer, binding agent, blasting agent, diluent, flavoring agent, lubricant, and the like.

In a specific embodiment, the desirable function comprises one or more of G1 to G6 in the step of selecting a candidate drug component (e.g., candidate adjuvant) having a transcriptome expression pattern corresponding to a desirable function of the invention.

In another aspect, the present invention provides a composition for exerting a desirable function, comprising a drug component (e.g., adjuvant) exerting the desirable function, wherein the desirable function preferably comprises one or more of G1 to G6. The drug component (e.g., adjuvant) exerting the desirable function contained in the drug component (e.g., adjuvant) contained in the composition of the invention can be a drug component identified by the method of the invention. Preferably, the drug component (e.g., adjuvant) exerting the desirable function contained in the drug component (e.g., adjuvant) contained in the composition of the invention is not a reference drug component (e.g., reference adjuvant), but can be a drug component whose function (G1 to G6 or others) is newly identified.

The present invention also provides a method of controlling quality of a drug component (e.g., active ingredient, additive, or adjuvant) by using the method of classifying a drug component (e.g., active ingredient, additive, or adjuvant) of the invention. Quantity control reviews a result of analysis of transcriptome clustering used in the method of classifying a drug component (e.g., active ingredient, additive, or adjuvant) and determines whether a gene expression pattern that is similar to a reference drug component (e.g., reference active ingredient, reference additive, or reference adjuvant) in a reference animal or the like to make a judgment. If a gene expression pattern that is substantially the same as a

reference drug component (e.g., reference active ingredient, reference additive, or reference adjuvant) of a group to which the target drug component (e.g., active ingredient, additive, or adjuvant) belongs or a substitute thereof is found, the target drug component (e.g., target active ingredient, target additive, or target adjuvant) can be determined as having good quality. If a difference in the gene expression pattern is found, the level of quality can be identified in accordance with the degree thereof.

The present invention provides a method of testing safety of a drug component (e.g., active ingredient, additive, or adjuvant) by using the method of classifying a drug component (e.g., active ingredient, additive, or adjuvant) of the invention. A safety test reviews a result of analyzing transcriptome clustering used in the method of classifying a drug component (e.g., active ingredient, additive, or adjuvant) described herein, and determines whether a gene expression pattern that is similar to a reference drug component (e.g., reference active ingredient, reference additive, or reference adjuvant) is found in a reference animal or the like to make a judgment. If a gene expression pattern that is substantially the same as a reference drug component (e.g., reference active ingredient, reference additive, or reference adjuvant) of a group to which the target drug component (e.g., active ingredient, additive, or adjuvant) belongs or a substitute thereof is found, the target drug component (e.g., target active ingredient, target additive, or target adjuvant) can be determined to be highly safe. If a difference in a gene expression pattern is found, the level of safety can be identified in accordance with the degree thereof.

The present invention also provides a method of testing an effect (efficacy) of a drug component (e.g., active ingredient, additive, or adjuvant) by using the method of classifying a drug component (e.g., active ingredient, additive, or adjuvant) of the invention. An effect test (efficacy test) reviews a result of analyzing transcriptome clustering used in the method of classifying a drug component (e.g., active ingredient, additive, or adjuvant), and determines whether a gene expression pattern that is similar to a reference drug component (e.g., reference active ingredient, reference additive, or reference adjuvant) is found in a reference animal or the like to make a judgment. If a gene expression pattern that is substantially the same as a reference drug component (e.g., reference active ingredient, reference additive, or reference adjuvant) of a group to which the target drug component (e.g., active ingredient, additive, or adjuvant) belongs or a substitute thereof is found, the target drug component (e.g., target active ingredient, target additive, or target adjuvant) can be determined as having the same degree of effect as the drug component (e.g., active ingredient, additive, or adjuvant). If a difference in a gene expression pattern is found, the level of effect can be identified in accordance with the degree thereof.

In one aspect, the present invention can identify a bottleneck gene for efficacy of toxicity (safety).

As used herein, "bottleneck gene" refers to a gene that has a fundamental effect on arriving at a phenomenon (e.g., efficacy is found, or toxicity is found).

For example, if the phenomenon is safety or toxicity, a toxicity bottleneck gene can be identified. A toxicity bottleneck gene refers to a gene that can determine whether a target (e.g., drug component) has or does not have toxicity if a change in the expression of the gene is observed (e.g., change from absence to presence of expression, presence to absence of expression, or increase or decrease of expression). Typically, a toxicity bottleneck gene is examined after a target is administered, and it can be determined that the target is toxic if expression of the gene is observed or increases.

A toxicity bottleneck gene can be identified by using the approach of the invention. For example, a candidate gene of a toxicity bottleneck gene can be determined by performing transcriptome analysis on a substance known as having toxicity using the method of the invention, identifying the pattern thereof, and identifying a gene having a pattern that is at least partially similar to the target substance. For the candidate gene, it is possible to knock out, in another animal species, a corresponding gene in the another animal species to prepare a knockout animal, determine whether toxicity is reduced or eliminated in the other animal species compared to a no knockout animal, and select the gene with reduction or elimination in toxicity as a toxicity bottleneck gene. Reduction or elimination is preferably statistically significant. The present invention also provides a method of providing or identifying such a toxicity bottleneck gene.

In one aspect, the present invention provides a method of determining toxicity of a drug component (e.g., active ingredient, additive, or adjuvant). The method comprises: determining whether a change (e.g., activation) in gene expression is observed for at least one of toxicity bottleneck genes for a candidate drug component such as a candidate adjuvant; and determining a candidate drug component having the change (e.g., activation) observed as having toxicity. In determining toxicity, a combination of a plurality of components or a combination in the final formulation can also be tested in addition to testing on individual drug components. In some cases, a toxicity test for the final combination can be important.

In another example, an efficacy bottleneck gene can be identified if the target phenomenon is efficacy. An efficacy bottleneck gene refers to a gene that can determine whether a target (e.g., drug component) has or does not have efficacy if a change in the expression of the gene is observed (e.g., change from absence to presence of expression, presence to absence of expression, or increase or decrease of expression). Typically, an efficacy bottleneck gene is examined after a target is administered, and it can be determined that the target has efficacy if expression of the gene is observed or increases. At least one efficacy bottleneck gene can be identified, but is provided as a set in some cases.

An efficacy bottleneck gene can be identified by using the approach of the invention. For example, a candidate gene of an efficacy bottleneck gene can be determined by performing transcriptome analysis on a substance known as having efficacy using the method of the invention, identifying the pattern thereof, and identifying a gene having a pattern that is at least partially similar to the target substance. For the candidate gene, it is possible to knock out, in another animal species, a corresponding gene in the another animal species to prepare a knockout animal, determine whether efficacy is increased or expressed in the knockout animal compared to a no-knockout animal, and select the gene with increase or expression as an efficacy bottleneck gene. Increase or expression is preferably statistically significant. The present invention also provides a method of providing or identifying such an efficacy bottleneck gene.

In one aspect, the present invention provides a method of determining efficacy of a drug component (e.g., active ingredient, additive, or adjuvant). The method comprises: determining whether a change (activation) in gene expression is observed for at least one of efficacy bottleneck genes for a candidate drug component such as a candidate adjuvant; and determining a candidate drug component having the change (activation) observed as having efficacy. If efficacy can also be defined for an additive, efficacy can be similarly determined using an efficacy bottleneck gene. Adjuvants are envisioned to be tested with the primary drug (e.g., antigen for a vaccine).

(Computer program, system, and recording medium)

In yet another aspect, the present invention provides a recording medium storing a program for implementing a method of generating an organ transcriptome profile of a drug component (e.g., active ingredient, additive, or adjuvant) on a computer. The method of executing a program stored in the recording medium comprises: (A) obtaining expression data by performing transcriptome analysis on at least one organ of a target organism using two or more drug components (e.g., active ingredients, additives, or adjuvants); (B) clustering the drug components (e.g., active ingredients, additives, or adjuvants) with respect to the expression data; and (C) generating a transcriptome profile of the organ of the drug components (e.g., active ingredients, additives, or adjuvants) based on the clustering. Each step used therein can be carried out in any embodiment that can be employed in the method of the invention or a combination thereof.

In yet another aspect, the present invention provides a system for generating an organ transcriptome profile of a drug component (e.g., active ingredient, additive, or adjuvant). The system comprises: (A) an expression data acquiring unit for obtaining or inputting expression data by performing transcriptome analysis on at least one organ of a target organism using two or more adjuvants (e.g., active ingredients, additives, or adjuvants); (B) a clustering computing unit for clustering the drug components (e.g., active ingredients, additives, or adjuvants) with respect to the expression data; and (C) a profiling unit for generating a transcriptome profile of the organ of the drug components (e.g., active ingredients, additives, or adjuvants) based on the clustering. Each unit of the system of the invention (expression data acquiring unit, clustering computing unit, profiling unit, and the like) can employ any configuration for embodying any embodiment that can be employed in the method of the invention or a combination thereof, and can be implemented in any embodiment.

In this regard, the expression data acquiring unit of the system of the invention is configured to be able to generate data by performing transcriptome analysis, or obtain data as a result thereof, using a drug component (e.g., active ingredient, additive, or adjuvant).

In one aspect, the present invention provides a program for implementing a classification method of a drug component (e.g., active ingredient, additive, or adjuvant) comprising classifying a drug component (e.g., active ingredient, additive, or adjuvant) based on transcriptome clustering on a computer. Each step used therein can be carried out in any embodiment that can be employed in the method of the invention or a combination thereof described herein.

In another aspect, the present invention provides a recording medium storing a program for implementing a classification method for a drug component (e.g., active ingredient, additive, or adjuvant) comprising classifying a drug component (e.g., active ingredient, additive, or adjuvant) based on transcriptome clustering on a computer. Each step used therein can be carried out in any embodiment that can be employed in the method of the invention or a combination thereof described herein.

In another aspect, the present invention provides a system for classifying a drug component (e.g., active ingredient, additive, or adjuvant) comprising a classification unit for classifying a drug component (e.g., active ingredient, additive, or adjuvant), based on transcriptome clustering. Each unit of the system of the invention (classification unit, and the like) can employ any configuration for embodying any embodiment that can be employed in the method of the invention or a combination thereof, and can be implemented in any embodiment.

In this regard, the classification unit of the system of the invention is configured to be able to generating data by performing transcriptome analysis, or obtain a data as a result thereof, using a drug components (e.g., active ingredients, additives, or adjuvants).

The configuration of the system of the invention is now explained by referring to the functional block diagram in Figure **6.** It is understood that the figure shows the invention embodied as a single system, but an invention embodied with a plurality of systems is also within the scope of the present invention. The method embodied by the system can

be written as a program (e.g., program for implementing classification of a drug component (e.g., active ingredient, additive, or adjuvant) on a computer). Such a program can be recorded on a recording medium and embodied as a method.

The system **1000** of the invention is constituted by connecting a RAM **1003,** a ROM, SSD, or HDD or a magnetic disk, an external storage device **1005** such as flash memory such as a USB memory, and an input/output interface (I/F) **1025** to a CPU **1001** built into a computer system via a system bus **1020.** An input device **1009** such as a keyboard or a mouse, an output device **1007** such as a display, and a communication device **1011** such as a modem are each connected to the input/output I/F **1025.** The external storage device **1005** comprises an information database storing unit **1030** and a program storing unit **1040.** Both are certain storage areas secured within the external storage apparatus **1005.**

In such a hardware configuration, various instructions (commands) are inputted via the input device **1009** or commands are received via the communication I/F, communication device **1011,** or the like to call up, deploy, and execute a software program installed on the storage device **1005** on the RAM **1003** by the CPU **1001** to accomplish the function of the invention in cooperation with an OS (operating system). Of course, the present invention can be implemented with a mechanism other than such a cooperating setup.

In the implementation of the present invention, data used as transcriptome clustering, such as expression data obtained as a result of performing transcriptome analysis on at least one organ of a target organism for a drug component (e.g., active ingredient, additive, or adjuvant) or information equivalent thereto (e.g., data obtained by simulation) can be inputted via the input device **1009,** inputted via the communication I/F, communication device **1011,** or the like, or stored in the database storing unit **1030.** The step of obtaining expression data by performing transcriptome analysis on at least one organ of a target organism using two or more drug components (e.g., active ingredients, additives, or adjuvants) and/or implementation of transcriptome clustering for classification can be executed with a program stored in the program storing unit **1040,** or a software program installed in the external storage device **1005** by inputting various instructions (commands) via the input device **1009** or by receiving commands via the communication I/F, communication device **1011,** or the like. As such software for performing transcriptome analysis, software shown in the Examples can be used, but software is not limited thereto. Any software known in the art can be used. Analyzed data can be outputted through the output device **1007** or stored in the external storage device **1005** such as the information database storing unit **1030.** The step of clustering the drug components (e.g., active ingredients, additives, or adjuvants) with respect to the expression data can also be executed with a program stored in the program storing unit **1040,** or a software program installed in the external storage device **1005** by inputting various instructions (commands) via the input device **1009** or by receiving commands via the communication I/F, communication device **1011,** or the like. The created clustering analysis data can be outputted through the output device **1007** or stored in the external storage device **1005** such as the information database storing unit **1030.** The step of generating a transcriptome profile of the organ of the drug component (e.g., active ingredient, additive, or adjuvant) based on clustering can also be executed with a program stored in the program storing unit **1040,** or a software program installed in the external storage device **1005** by inputting various instructions (commands) via the input device **1009** or by receiving commands via the communication I/F, communication device **1011,** or the like. The created transcriptome profile data can be outputted through the output device **1007** or stored in the external storage device **1005** such as the information database storing unit **1030.** The data processing or storage of various features of transcriptome profile data can also be executed with a program stored in the program storing unit **1040,** or a software program installed in the external storage device **1005** by inputting various instructions (commands) via the input device **1009** or by receiving commands via the communication I/F, communication device **1011,** or the like. The profile feature or information can be outputted through the output device **1007** or stored in the external storage device **1005** such as the information database storing unit **1030.**

The data or calculation result or information obtained via the communication device **1011** or the like is written and updated immediately in the database storing unit **1030.** Information attributed to samples subjected to accumulation can be managed with an ID defined in each master table by managing information such as each of the sequences in each input sequence set and each genetic information ID of a reference database in each master table.

The above calculation result can be associated with known information such as various information on drug component (e.g., active ingredient, additive, or adjuvant) or biological information and stored in the database storing unit **1030.** Such association can be performed directly to data available through a network (Internet, Intranet, or the like) or as a link to the network.

A computer program stored in the program storing unit **1040** is configured to use a computer as the above processing system, e.g., a system for performing the process of data provision, transcriptome analysis, expression data analysis, clustering, profiling, and other processing. Each of these functions is an independent computer program, a module thereof, or a routine, which is executed by the CPU **1001** to use a computer as each system or device. It is assumed hereinafter that each function in each system cooperates to constitute each system.

<Method of analyzing drug component with unknown function>

In another aspect, the present invention provides feature information of a drug component (e.g., active ingredient, additive, or adjuvant). The method comprises: (a) providing a candidate drug component (e.g., candidate active ingredient, candidate additive, or candidate adjuvant); (b) providing a reference drug component (e.g., reference active ingredient, reference additive, or reference adjuvant) set with a known function; (c) obtaining gene expression data by performing transcriptome analysis on the candidate drug component (e.g., candidate active ingredient, candidate additive, or candidate adjuvant) and the reference drug component (e.g., reference active ingredient, reference additive, or reference adjuvant) set to cluster the gene expression data; and (d) providing a feature of a member of the reference drug component (e.g., reference active ingredient, reference additive, or reference adjuvant) set belonging to the same cluster as that of the candidate drug component (e.g., candidate active ingredient, candidate additive, or candidate adjuvant) as a feature of the candidate drug component (e.g., candidate active ingredient, candidate additive, or candidate adjuvant). The feature information of a drug component (e.g., active ingredient, additive, or adjuvant) of the invention is provided using the transcriptome analysis technology of a drug component (e.g., active ingredient, additive, or adjuvant) of the invention, which can comprise one or a combination of any features in <Transcriptome analysis of drug component> described herein.

In this regard, the candidate drug component (e.g., candidate active ingredient, candidate additive, or candidate adjuvant) can be a novel substance or a known substance. A property as a conventional drug component (e.g., active ingredient, additive, or adjuvant) can be known or unknown. A candidate drug component (e.g., candidate active ingredient, candidate additive, or candidate adjuvant) is intended to be provided to at least one organ of a target organism. Examples of such an organ include, but are not limited to, liver, spleen, lymph node.

A reference drug component (e.g., reference active ingredient, reference additive, or reference adjuvant) set with a known function can be provided using any adjuvant belonging to G1 to G6 specifically mentioned in <Transcriptome analysis of drug component>, or using a drug component (e.g., active ingredient, additive, or adjuvant) separately identified using an approach described in <Transcriptome analysis of drug component> or a set thereof.

Obtaining gene expression data by performing transcriptome analysis on the candidate drug component (e.g., candidate active ingredient, candidate additive, or candidate adjuvant) and the reference drug component (e.g., reference active ingredient, reference additive, or reference adjuvant) set can use any approach known in the art. For the reference drug component (e.g., reference active ingredient, reference additive, or reference adjuvant) set, already analyzed data can be used or new data can be reacquired. When using already analyzed data, transcriptome analysis of a candidate drug component (e.g., candidate active ingredient, candidate additive, or candidate adjuvant) can be preferably performed under conditions that were used for the already analyzed data (e.g., dosage form, dosage and administration, or the like), but the analysis is not necessarily limited thereto. Gene expression data, when obtained, is clustered. Clustering can use any approach. A method mentioned in <Transcriptome analysis of drug component> described herein can be used.

Once a result of analyzing clustering of gene expression data is obtained after transcriptome analysis, the drug component (e.g., active ingredient, additive, or adjuvant) cluster (group) to which the candidate drug component (e.g., candidate active ingredient, candidate additive, or candidate adjuvant) belongs is determined, and a feature of a member of the reference drug component (e.g., reference active ingredient, reference additive, or reference adjuvant) set belonging to the same cluster as that of the candidate drug component (e.g., candidate active ingredient, candidate additive, or candidate adjuvant) can be provided as a feature of the candidate drug component (e.g., candidate active ingredient, candidate additive, or candidate adjuvant). Information provided by such a method of providing a feature information is highly likely to be a feature that the candidate drug component (e.g., candidate active ingredient, candidate additive, or candidate adjuvant) actually has. The method can be considered very useful for predicting a property of a novel substance or a known substance with an unknown function as a drug component (e.g., active ingredient, additive, or adjuvant).

In one aspect, the present invention provides a program for implementing a method of providing feature information of a drug component (e.g., active ingredient, additive, or adjuvant) on a computer. The method implemented by the program comprises: (a) providing a candidate drug component (e.g., candidate active ingredient, candidate additive, or candidate adjuvant); (b) providing a reference drug component (e.g., reference active ingredient, reference additive, or reference adjuvant) set with a known function; (c) obtaining gene expression data by performing transcriptome analysis on the candidate drug component (e.g., candidate active ingredient, candidate additive, or candidate adjuvant) and the reference drug component (e.g., reference active ingredient, reference additive, or reference adjuvant) set to cluster the gene expression data; and (d) providing a feature of a member of the reference drug component (e.g., reference active ingredient, reference additive, or reference adjuvant) set belonging to the same cluster as that of the candidate drug component (e.g., candidate active ingredient, candidate additive, or candidate adjuvant) as a feature of the candidate drug component (e.g., candidate active ingredient, candidate additive, or candidate adjuvant). The feature information of a drug component (e.g., active ingredient, additive, or adjuvant) of the invention is provided using the transcriptome analysis technology of a drug component (e.g., active ingredient, additive, or adjuvant) of the invention, which can

comprise one or a combination of any features in <Transcriptome analysis of drug component> described herein.

In one aspect, the present invention provides a recording medium for storing a program for implementing a method of providing feature information of a drug component (e.g., active ingredient, additive, or adjuvant) on a computer. The method executed by a program stored in the recording medium comprises: a) providing a candidate drug component (e.g., candidate active ingredient, candidate additive, or candidate adjuvant); (b) providing a reference drug component (e.g., reference active ingredient, reference additive, or reference adjuvant) set with a known function; (c) obtaining gene expression data by performing transcriptome analysis on the candidate drug component (e.g., candidate active ingredient, candidate additive, or candidate adjuvant) and the reference drug component (e.g., reference active ingredient, reference additive, or reference adjuvant) set to cluster the gene expression data; and (d) providing a feature of a member of the reference drug component (e.g., reference active ingredient, reference additive, or reference adjuvant) set belonging to the same cluster as that of the candidate drug component (e.g., candidate active ingredient, candidate additive, or candidate adjuvant) as a feature of the candidate drug component (e.g., candidate active ingredient, candidate additive, or candidate adjuvant). The feature information of a drug component (e.g., active ingredient, additive, or adjuvant) of the invention is provided using the transcriptome analysis technology of a drug component (e.g., active ingredient, additive, or adjuvant) of the invention, which can comprise one or a combination of any features in <Transcriptome analysis of drug component> described herein.

In one aspect, the present invention provides a system for providing feature information of a drug component (e.g., active ingredient, additive, or adjuvant). The system comprises: a) a candidate drug component providing unit for providing a candidate drug component (e.g., candidate active ingredient, candidate additive, or candidate adjuvant); (b) a reference drug component providing unit for providing a reference drug component (e.g., active ingredient, additive, or adjuvant) set with a known function; (c) a transcriptome clustering analysis unit for obtaining gene expression data by performing transcriptome analysis on the candidate drug component (e.g., active ingredient, additive, or adjuvant) and the reference drug component (e.g., active ingredient, additive, or adjuvant) set to cluster the gene expression data; and (d) a feature analysis unit for providing a feature of a member of the reference drug component (e.g., active ingredient, additive, or adjuvant) set belonging to the same cluster as that of the candidate drug component (e.g., active ingredient, additive, or adjuvant) as a feature of the candidate drug component (e.g., active ingredient, additive, or adjuvant). The feature information of a drug component (e.g., active ingredient, additive, or adjuvant) of the invention is provided using the transcriptome analysis technology of a drug component (e.g., active ingredient, additive, or adjuvant) of the invention, which can comprise one or a combination of any features in <Transcriptome analysis of drug component> described herein. Each unit of the system of the invention (candidate drug component providing unit, reference drug component providing unit, transcriptome clustering analysis unit, feature analysis unit, and the like) can employ any configuration for embodying any embodiment that can be employed in the method of the invention or a combination thereof, and can be implemented in any embodiment.

In one embodiment, the candidate drug component providing unit can have any configuration, as long as the unit has a function and arrangement for providing a candidate drug component (e.g., active ingredient, additive, or adjuvant) . The unit can be provided as the same or different structure as the analysis unit or profiling unit. A candidate drug component (e.g., active ingredient, additive, or adjuvant) is intended to be provided to at least one organ of a target organism.

In one embodiment, a reference drug component (e.g., active ingredient, additive, or adjuvant) providing unit provides a reference drug component (e.g., reference active ingredient, reference additive, or reference adjuvant) set with a known function. A reference drug component (e.g., reference active ingredient, reference additive, or reference adjuvant) can be stored in advance in the reference drug component (e.g., reference active ingredient, reference additive, or reference adjuvant) providing unit, or the unit may be configured to receive a reference drug component (e.g., reference active ingredient, reference additive, or reference adjuvant) provided separately from the outside. The candidate drug component providing unit and reference drug component providing unit can be different or the same. If the same, a configuration or a function can be provided so that a candidate drug component (e.g., candidate active ingredient, candidate additive, or candidate adjuvant) is not mixed in with a reference drug component (e.g., reference active ingredient, reference additive, or reference adjuvant).

In one embodiment, a transcriptome clustering analysis unit obtains gene expression data by performing transcriptome analysis on the candidate drug component (e.g., candidate active ingredient, candidate additive, or candidate adjuvant) and the reference drug component (e.g., reference active ingredient, reference additive, or reference adjuvant) set to cluster the gene expression data. For a transcriptome analysis of a candidate drug component (e.g., candidate active ingredient, candidate additive, or candidate adjuvant) and a reference drug component (e.g., reference active ingredient, reference additive, or reference adjuvant), a transcriptome clustering analysis unit itself can comprise all of the function, or the transcriptome clustering analysis unit can be configured to have a function of performing transcriptome analysis on a result after externally obtaining gene expression data and inputting the data.

In one embodiment, a feature analysis unit provides a feature of a member of the reference drug component (e.g., reference active ingredient, reference additive, or reference adjuvant) set belonging to the same cluster as that of

the candidate drug component (e.g., candidate active ingredient, candidate additive, or candidate adjuvant) as a feature of the candidate drug component (e.g., candidate active ingredient, candidate additive, or candidate adjuvant).

Next, a system for providing feature information of a drug component (e.g., active ingredient, additive, or adjuvant) can also perform the same processing as the system for generating an organ transcriptome profile of a drug component (e.g., active ingredient, additive, or adjuvant) (see the functional block diagram in Figure 6).

In another aspect, the present invention provides a program for implementing a method of classifying a drug component (e.g., active ingredient, additive, or adjuvant) on a computer. The method comprises: (a) providing a candidate drug component in at least one organ of a target organism; (b) calculating a reference drug component set; (c) obtaining gene expression data by performing transcriptome analysis on the candidate drug component and the reference drug component set to cluster the gene expression data; and (d) determining that the candidate drug component belongs to the same group if a cluster to which the candidate drug component belongs is classified to the same cluster as at least one in a reference drug component set, and determining as impossible to classify if the cluster does not belong to any cluster. In this method, a drug component can be an active ingredient, additive, adjuvant, or combination thereof. In another aspect, the present invention provides a recording medium storing a program for implementing the above method of classifying a drug component (e.g., active ingredient, additive, or adjuvant) on a computer.

In one embodiment, the present invention provides a program for implementing a method of classifying a drug component (e.g., active ingredient, additive, or adjuvant) on a computer, the method comprising: (a) providing a candidate drug component (e.g., candidate active ingredient, candidate additive, or candidate adjuvant) in at least one organ of a target organism; (b) providing a reference adjuvant set classified to at least one selected from the group consisting of G1 to G6; (c) obtaining gene expression data by performing transcriptome analysis on the candidate drug component (e.g., candidate active ingredient, candidate additive, or candidate adjuvant) and the reference drug component (e.g., reference active ingredient, reference additive, or reference adjuvant) set to cluster the gene expression data; and (d) determining that the candidate drug component (e.g., candidate active ingredient, candidate additive, or candidate adjuvant) belongs to the same group if a cluster to which the candidate drug component (e.g., candidate active ingredient, candidate additive, or candidate adjuvant) belongs is classified to the same cluster as at least one in groups G1 to G6, and determining as impossible to classify if the candidate drug component does not belong to any cluster. Each step used therein can be carried out in any embodiment that can be employed in the method of the invention or a combination thereof. In another aspect, the present invention provides a recording medium storing a program for implementing the above method of classifying a drug component (e.g., active ingredient, additive, or adjuvant) on a computer.

In another embodiment, the present invention provides a program for implementing a method of classifying an adjuvant on a computer and a recording medium storing the program. In this regard, the method comprises: (a) providing a candidate adjuvant in at least one organ of a target organism; (b) providing a reference adjuvant set classified to at least one selected from the group consisting of G1 to G6; (c) obtaining gene expression data by performing transcriptome analysis on the candidate adjuvant and the reference adjuvant set to cluster the gene expression data; and (d) determining that the candidate adjuvant belongs to the same group if a cluster to which the candidate adjuvant belongs is classified to the same cluster as at least one in groups G1 to G6, and determining as impossible to classify if the cluster does not belong to any cluster. Each step used therein can be carried out in any embodiment that can be employed in the method of the invention or a combination thereof.

In another aspect, the present invention provides a system for classifying a drug component (e.g., active ingredient, additive, or adjuvant). The system comprises: (a) a candidate drug component providing unit for providing a candidate adjuvant in at least one organ of a target organism; (b) a reference drug component calculating unit for calculating a reference drug component set; (c) a transcriptome clustering analysis unit for obtaining gene expression data by performing transcriptome analysis on the candidate drug component and the reference drug component set to cluster the gene expression data; and (d) a determination unit for determining that the candidate drug component belongs to the same group if a cluster to which the candidate drug component belongs is classified to the same cluster as at least one in a reference drug component set, and determining as impossible to classify if the cluster does not belong to any cluster. In this system, the drug component can be an active ingredient, additive, adjuvant, or combination thereof.

In one embodiment, the present invention provides a system for classifying a drug component. The system comprises: (a) a candidate drug component providing unit for providing a candidate drug component in at least one organ of a target organism; (b) a reference drug component storing unit for providing a reference drug component set classified to at least one selected from the group consisting of G1 to G6 of the invention; (c) a transcriptome clustering analysis unit for obtaining gene expression data by performing transcriptome analysis on the candidate drug component and the reference drug component set to cluster the gene expression data; and (d) a determination unit for determining that the candidate drug component belongs to the same group if a cluster to which the candidate drug component belongs is classified to the same cluster as at least one in groups G1 to G6, and determining as impossible to classify if the cluster does not belong to any cluster.

In another embodiment, the present invention provides a system for classifying an adjuvant, the system comprising: (a) a candidate adjuvant providing unit for providing a candidate adjuvant in at least one organ of a target

organism; (b) a reference adjuvant storing unit for providing a reference adjuvant set classified to at least one selected from the group consisting of G1 to G6; (c) a transcriptome clustering analysis unit for obtaining gene expression data by performing transcriptome analysis on the candidate adjuvant and the reference adjuvant set to cluster the gene expression data; and (d) a determination unit for determining that the candidate adjuvant belongs to the same group if a cluster to which the candidate adjuvant belongs is classified to the same cluster as at least one in groups G1 to G6, and determining as impossible to classify if the cluster does not belong to any cluster. Each unit of the system of the invention (candidate drug component providing unit, reference adjuvant storing unit, transcriptome clustering analysis unit, determination unit, and the like) can employ any configuration for embodying any embodiment that can be employed in the method of the invention or a combination thereof, and can be implemented in any embodiment.

In this regard, the classification unit of the system of the invention is configured to be able to generating data by performing transcriptome analysis, or obtain data as a result thereof, using a drug component (e.g., active ingredient, additive, or adjuvant).

The configuration of the system of the invention is now explained by referring to the functional block diagram in Figure **6.** It is understood that the figure shows the invention embodied as a single system, but an invention embodied with a plurality of systems is also within the scope of the present invention. The method embodied by the system can be written as a program. Such a program can be recorded on a recording medium and embodied as a method.

The system **1000** of the invention is constituted by connecting a RAM **1003,** a ROM, SSD or HDD or a magnetic disk, an external storage device **1005** such as flash memory such as a USB memory, and an input/output interface (I/F) **1025** to a CPU **1001** built into a computer system via a system bus **1020.** An input device **1009** such as a keyboard or a mouse, an output device **1007** such as a display, and a communication device **1011** such as a modem are each connected to the input/output I/F **1025.** The external storage device **1005** comprises an information database storing unit **1030** and a program storing unit **1040.** Both are certain storage areas secured within the external storage apparatus **1005.**

In such a hardware configuration, various instructions (commands) are inputted via the input device **1009** or commands are received via the communication I/F, communication device **1011,** or the like to call up, deploy, and execute a software program installed on the storage device **1005** on the RAM **1003** by the CPU **1001** to accomplish the function of the invention in cooperation with an OS (operating system). Of course, the present invention can be implemented with a mechanism other than such a cooperating setup.

In the implementation of the present invention, if gene expression data was obtained by transcriptome analysis on a candidate drug component (e.g., candidate active ingredient, candidate additive, or candidate adjuvant) and a reference drug component (e.g., reference active ingredient, reference additive, or reference adjuvant) set with a known function, expression data obtained by transcriptome analysis or information equivalent thereto (e.g., data obtained by simulation) can be inputted via the input device **1009,** inputted via the communication I/F, communication device **1011,** or the like, or stored in the database storing unit **1030.** The step of obtaining gene expression data by performing transcriptome analysis on the candidate drug component (e.g., candidate active ingredient, candidate additive, or candidate adjuvant) and the reference drug component (e.g., reference active ingredient, reference additive, or reference adjuvant) set to cluster the gene expression data can be executed with a program stored in the program storing unit **1040,** or a software program installed in the external storage device **1005** by inputting various instructions (commands) via the input device **1009** or by receiving commands via the communication I/F, communication device **1011,** or the like. As such software for performing transcriptome analysis or expression analysis, software shown in the Examples can be used, but software is not limited thereto. Any software known in the art can be used. Analyzed data can be outputted through the output device **1007** or stored in the external storage device **1005** such as the information database storing unit **1030.** The step of providing a feature of a member of the reference drug component (e.g., reference active ingredient, reference additive, or reference adjuvant) set belonging to the same cluster as that of the candidate drug component (e.g., candidate active ingredient, candidate additive, or candidate adjuvant) as a feature of the candidate drug component (e.g., candidate active ingredient, candidate additive, or candidate adjuvant) can also be executed with a program stored in the program storing unit **1040,** or a software program installed in the external storage device **1005** by inputting various instructions (commands) via the input device **1009** or by receiving commands via the communication I/F, communication device **1011,** or the like. The created data of a feature of a candidate drug component (e.g., candidate active ingredient, candidate additive, or candidate adjuvant) can be outputted through the output device **1007** or stored in the external storage device **1005** such as the information database storing unit **1030.** The data processing or storage of various features of transcriptome profile data can also be executed with a program stored in the program storing unit **1040,** or a software program installed in the external storage device **1005** by inputting various instructions (commands) via the input device **1009** or by receiving commands via the communication I/F, communication device **1011,** or the like. The profile feature or information can be outputted through the output device **1007** or stored in the external storage device **1005** such as the information database storing unit **1030.**

In another implementation of the present invention, data related to a candidate adjuvant provided by the step of providing the candidate drug component (e.g., candidate active ingredient, candidate additive, or candidate adjuvant)

in at least one organ of a target organism can be inputted via the input device **1009,** inputted via the communication I/F, communication device **1011,** or the like, or stored in the database storing unit **1030.** Data for a reference drug component (e.g., reference active ingredient, reference additive, or reference adjuvant) set provided by the step of providing a reference drug component (e.g., reference active ingredient, reference additive, or reference adjuvant) set classified to at least one selected from the group consisting of G1 to G6 can also be similarly stored or inputted. Alternatively, data for a reference drug component (e.g., reference active ingredient, reference additive, or reference adjuvant) set can be called out and used from the database storing unit **1030,** or via the communication I/F, communication device **1011,** or the like. The step of obtaining gene expression data by performing transcriptome analysis on the candidate drug component (e.g., candidate active ingredient, candidate additive, or candidate adjuvant) and the reference drug component (e.g., reference active ingredient, reference additive, or reference adjuvant) to cluster the gene expression data can be executed with a program stored in the program storing unit **1040,** or a software program installed in the external storage device **1005** by inputting various instructions (commands) via the input device **1009** or by receiving commands via the communication I/F, communication device **1011,** or the like. As such software for performing transcriptome analysis and/or clustering, software shown in the Examples can be used, but software is not limited thereto. Any software known in the art can be used. Analyzed data can be outputted through the output device **1007** or stored in the external storage device **1005** such as the information database storing unit **1030.** The step of determining that the candidate drug component (e.g., candidate active ingredient, candidate additive, or candidate adjuvant) belongs to the same group if a cluster to which the candidate drug component (e.g., candidate active ingredient, candidate additive, or candidate adjuvant) belongs is classified to the same cluster as at least one in groups G1 to G6, and determining as impossible to classify if the cluster does not belong to any cluster can also be executed with a program stored in the program storing unit **1040,** or a software program installed in the external storage device **1005** by inputting various instructions (commands) via the input device **1009** or by receiving commands via the communication I/F, communication device **1011,** or the like. The determination data can be outputted through the output device **1007** or stored in the external storage device **1005** such as the information database storing unit **1030.**

The data or calculation result or information obtained via the communication device **1011** or the like is written and updated immediately in the database storing unit **1030.** Information attributed to samples subjected to accumulation can be managed with an ID defined in each master table by managing information such as each of the sequences in each input sequence set and each genetic information ID of a reference database in each master table.

The above calculation result can be associated with known information such as various information on drug component (e.g., active ingredient, additive, or adjuvant) or biological information and stored in the database storing unit **1030.** Such association can be performed directly to data available through a network (Internet, Intranet, or the like) or as a link to the network.

A computer program stored in the program storing unit **1040** is configured to use a computer as the above processing system, e.g., a system for performing the process of data provision, transcriptome analysis, expression data analysis, clustering, profiling, and other processing. Each of these functions is an independent computer program, a module thereof, or a routine, which is executed by the CPU **1001** to use a computer as each system or device. It is assumed hereinafter that each function in each system cooperates to constitute each system.

<Application for quality control, safety test, and effect determination>

In one aspect, the present invention provides a method of controlling quality of a drug component (e.g., active ingredient, additive, or adjuvant) using the method of generating an organ transcriptome profile of a drug component (e.g., active ingredient, additive, or adjuvant) of the invention and/or the method of providing feature information of a drug component (e.g., active ingredient, additive, or adjuvant) of the invention. Quality control of a drug component (e.g., active ingredient, additive, or adjuvant) maintains quality at a certain level or higher by testing in advance whether quality of a drug component (e.g., active ingredient, additive, or adjuvant) in each lot is suitable upon distribution as a pharmaceutical product in particular. An organ transcriptome profile of a drug component (e.g., active ingredient, additive, or adjuvant) can analyze a property of various drug components (e.g., active ingredient, additive, or adjuvant) by using a significant DEG, so that quality can be maintained at a certain level without actually conducting complex tests.

Examples of specific approaches of quality control include, but are not limited to, performing the above analysis on a drug component (e.g., active ingredient, additive, or adjuvant) subjected to quality control to obtain the organ transcriptome profile thereof, and comparing the standard organ transcriptome profile (also referred to as reference transcriptome profile) estimated for the drug component (e.g., active ingredient, additive, or adjuvant) with an organ transcriptome profile of a drug component (e.g., active ingredient, additive, or adjuvant) subjected to quality control, and if there is no significant difference, determining that estimated quality is retained. Alternatively, if there is a significant difference, it is determined that quality standard is not satisfied. When a significant difference is not found, whether a quality standard is satisfied can be determined by further testing.

In another aspect, the present invention provides a method of testing safety of a drug component (e.g., active

ingredient, additive, or adjuvant) by using the method of generating an organ transcriptome profile of a drug component (e.g., active ingredient, additive, or adjuvant) of the invention and/or the method of providing feature information of a drug component (e.g., active ingredient, additive, or adjuvant) of the invention. A novel drug component (e.g., active ingredient, additive, or adjuvant) would require toxicological evaluation. For adjuvants, toxicological evaluation as a formulation comprising a novel adjuvant and an antigen is also required. Even known drug components (e.g., active ingredients, additives, or adjuvants) and antigens require toxicological evaluation depending on the combination thereof. As a part or the entire toxicological evaluation, such an organ transcriptome profile or feature information of a drug component (e.g., active ingredient, additive, or adjuvant) can be utilized. This is useful for extrapolation to safety after human injection.

Examples of specific approaches to determine safety include, but are not limited to, performing the above analysis on a drug component (e.g., active ingredient, additive, or adjuvant) subjected to the determination of safety to obtain an organ transcriptome profile thereof, comparing the standard organ transcriptome profile (also referred to as reference transcriptome profile) estimated for the drug component (e.g., active ingredient, additive, or adjuvant) with an organ transcriptome profile of a drug component (e.g., active ingredient, additive, or adjuvant) subjected to determination of safety, and if there is no significant difference, determining that estimated safety is retained. Alternatively, if there is a significant difference, it is determined that a safety standard is not satisfied. When a significant difference is not found, whether a safety standard is satisfied can be determined by further testing.

In another aspect, the present invention provides a method of determining an effect of a drug component (e.g., active ingredient, additive, or adjuvant) by using the method of generating an organ transcriptome profile of a drug component (e.g., active ingredient, additive, or adjuvant) of the invention and/or the method of providing feature information of a drug component (e.g., active ingredient, additive, or adjuvant) of the invention. A novel drug component (e.g., active ingredient, additive, or adjuvant) would require efficacy evaluation. For adjuvants, efficacy evaluation as a formulation comprising a novel adjuvant and an antigen is also required. Even known adjuvants and antigens require efficacy evaluation depending on the combination thereof. As a part or the entire efficacy evaluation, such an organ transcriptome profile or feature information of a drug component (e.g., active ingredient, additive, or adjuvant) can be utilized. This is useful for extrapolation to efficacy evaluation after human intake.

Examples of specific approaches to determine efficacy evaluation include, but are not limited to, performing the above transcriptome analysis on a drug component (e.g., active ingredient, additive, or adjuvant) subjected to the determination of efficacy to obtain an organ transcriptome profile thereof, comparing the standard organ transcriptome profile (also referred to as reference transcriptome profile) estimated for the drug component (e.g., active ingredient, additive, or adjuvant) with an organ transcriptome profile of a drug component (e.g., active ingredient, additive, or adjuvant) subjected to determination of efficacy, and if there is no significant difference, determining that estimated efficacy is attained. Alternatively, if there is a significant difference, it is determined that an efficacy standard is not satisfied. When a significant difference is not found, whether an efficacy standard is satisfied can be determined by further testing.

In the present invention, intra-database analysis can be performed using toxiogenomic data base (GATE) or the like in addition to an adjuvant database. For example, an adjuvant database is demonstrated to be usable in humans, monkeys, mice, rats, and the like. It is also understood that the database can be used similarly in other animals. Further, a toxiogenomic database is open to the public for humans and rats. Intra-database analysis can be performed using other available databases. They are data for 6 hours and 24 hours with a single agent administration. A gene expression profile (liver, kidney, lymph node, spleen, and the like), hematology (white blood cell, red blood cell, platelet, and the like), biochemical testing (aspartate transaminase (AST), alanine transaminase (ALT), creatinine (CRE), and the like, as well as serum miRNA profiles and the like can also be tested. Databases thereof are also available.

While the present invention is capable of transcriptome based toxicity and efficacy prediction, a prediction model can also be generated for prediction by using machine learning (e.g., support vector machine) instead.

For example, for a toxic group (10) and a non-toxic group (10) in a publicly opened toxiogenomic gate (150), a group from which pathological findings were obtained from four administrations and a group from which a toxicity related feature is not observed are identified. A sample exhibiting an expression pattern similar to the toxic group can be predicted as toxic, and a sample exhibiting a pattern similar to the non-toxic group can be determined as non-toxic. In such a case, a prediction model can be generated by machine learning. Such models and prediction methods based on the models are also within the scope of the invention.

Efficacy can be determined by using an adjuvant database associated with efficacy such as an adjuvant database. Databases for particles, emulsions, DNA/RNA, TLR ligands or the like can be used.

The present invention can be practiced using artificial intelligence (AI) that utilizes machine learning. As used herein, "machine learning" refers to a technology that imparts a computer with an ability to learn without explicit programming. It is a process for improving its own performance by a functional unit acquiring new knowledge/skill, or reconstructing existing knowledge/skill. Most of the labor required for programming details can be reduced by programming a computer to learn from experience. In the field of machine learning, a method of constructing a computer program

that can automatically improve from experience has been debated. The role of data analysis/machine learning is as an underlying technology that is the foundation of intelligent processing, together with the field of algorithms. It is generally used in conjunction with other technologies. Knowledge in the collaborating field (domain knowledge, e.g., medical field) is required. The scope of application thereof includes roles in prediction (collecting data to predict something would occur), searching (finding a notable feature from collected data), and testing/description (studying relationship among various elements in data). Machine learning is based on an indicator indicating the degree of achievement of a goal in the real world. A user of machine learning needs to understand the goal in the real world. When the goal is achieved, an indicator that would improve needs to be formulated. Machine learning can use linear regression, logistic regression, support vector machine, or the like. In addition, the precision of determination of each model can be calculated by cross validation. After ranking, a feature amount can be increased one by one and perform machine learning (linear regression, logistic regression, support vector machine, or the like) and cross validation to calculate the precision of determination of each model. A model with the highest precision can be selected in this manner. In the present invention, any machine learning can be used. As supervised machine learning, linear, logistic, support vector machine (SVM) or the like can be used.

Machine learning uses logical reasoning. There are roughly three types of logical reasoning, i.e., deduction, induction, and abduction. Deduction, under the hypothesis that Socrates is a human and all humans die, reaches a conclusion that Socrates would die, which is a special conclusion. Induction, under the hypothesis that Socrates would die and Socrates is a human, reaches a conclusion that all humans would die, and determines a general rule. Abduction, under a hypothesis that Socrates would die and all humans die, arrives at Socrates is a human, which falls under a hypothesis/explanation. However, it should be noted that how induction generalizes is dependent on the premise, so that this may not be objective.

Impossible has three basic principles, i.e., impossible, very difficult, and unsolved. Further, impossible includes generalization error, no free lunch theorem, and ugly duckling theorem and true model observation is impossible, so that it is not possible to verify. Such an ill-posed problem should be noted.

Feature/attribute in machine learning represents the state of a subject of prediction when viewed from a certain aspect. A feature vector/attribute vector combines features (attribute) describing a subject of prediction in a vector form.

As used herein, "model" or "hypothesis" are used synonymously, which is expressed using mapping describing the relationship of inputted prediction subjects to prediction results, or a mathematical function or Boolean expression of a candidate set thereof. For learning with machine learning, a model considered the best approximation of the true model is selected from a model set by referring to training data.

Examples of models include generation model, identification model, function model, and the like. Models show a difference in the direction of classification model expression of the mapping relationship between the input (subject of prediction) x and output (result of prediction) y. A generation model expresses a conditional distribution of output y given input x. An identification model expresses a joint distribution of input x and output y. The mapping relationship is probabilistic for an identification model and a generation model. A function model has a definitive mapping relationship, expressing a definitive functional relationship between input x and output y. While identification is sometimes considered slightly more precise in an identification model and a generation model, there is basically no difference in view of the no free lunch theorem.

For learning in machine learning, a model considered the best approximation of the true model is selected from a model set by referring to training data. There are various learning methods depending on the "approximation". A typical method is the maximum likelihood estimation, which is a standard of learning that selects a model with the highest probability of producing training data from a probabilistic model set. Maximum likelihood estimation can select a model that best approximates the true model. KL divergence to the true distribution becomes small for greater likelihood. There are various types of estimation that vary by the type of form for finding a parameter or estimated prediction value. Point estimation finds only one value with the highest certainty. Maximum likelihood estimation, MAP estimation and the like use the mode of a distribution or function and are most often used. Meanwhile, interval estimation is often used in the field of statistics in a form of finding a range in which an estimated value falls, where the probability of an estimated value falling in the range is 95%. Distribution estimation is used in Bayesian estimation or the like in combination with a generation model introducing a prior distribution for finding a distribution in which an estimated value falls.

(Adjuvant of adjuvant)

Vaccine adjuvants are very important for initiating, maximizing and extending the immunogenicity of many vaccines and the potency thereof. For over 80 years, aluminum salt (alum) was the only adjuvant conventionally used in humans. In recent years, additional adjuvants such as alum combined with monophosphoryl lipid A and squalene oil emulsion, while limited, have been approved for human use. A suitable adjuvant is ideally selected based on vaccine properties that provide the best pathogen protection, including the immune response type. Unfortunately, the number of adjuvants approved for human use is currently lacking. Thus, precise adjustment in immune response induction is

limited. There is an urgent need for the development of different types of adjuvants.

The most known adjuvant targets a pathogen recognition receptor (PRR) such as TLR, NOD, or inflammasome receptor and induces upregulation of a costimulatory molecule on an antigen presenting cell (APC) and production of inflammatory cytokines and type I interferon (IFN) (Olive, 2012, Expert review of vaccines 11, 237-256). Pathogen associated molecule patterns (PAMPs) contained in a vaccine such as microorganism nucleic acids, glycolipids, or proteins function as an endogenous innate adjuvant and elicit an innate immune response, which then potentiates an adaptive immune response induced by a specific antigen (Desmet, C.J., and Ishii, K.J. (2012). Nature reviews Immunology 12, 479-491). For example, viral RNA in an influenza WV vaccine activates TLR7, which induces a response biased to Th1 against a WV antigen (Koyama, S., Aoshi, T., Tanimoto, T., Kumagai, Y., Kobiyama, K., Tougan, T., Sakurai, K., Coban, C., Horii, T., Akira, S., et al. (2010). Science translational medicine 2, 25ra24.)

However, the mechanism of inducing or enhancing adjuvant activity is not known.

Thus, in another aspect, the present invention is based on a result of studying the immunological feature and mechanism of action of Advax™, a delta inulin which is a microparticle derived from inulin developed as a vaccine adjuvant. In this regard, delta inulin becomes a type 2 adjuvant when combined with a Th2 type antigen, an influenza split vaccine, but exhibits a behavior as a type 1 adjuvant when combined with a Th1 type antigen, influenza inactivated whole virion (WV). Its adjuvant effect to WV of delta inulin is lost in TLR7 knockout mice which lack the built-in RNA adjuvant effect of WV, and showed no adjuvant effect for ovalbumin, a neutral Th0-type antigen. Therefore, unlike other adjuvants, delta inulin potentiates the intrinsic property of co-administered antigen, while its adjuvanticity absolutely requires not only dendritic cells but also phagocytic macrophages and TNF-$\alpha$. These results demonstrated that delta inulin is a unique class of adjuvant which can potentiate the endogenous adjuvant effect of the vaccines through a not yet fully-identified, unique mechanisms of action.

As used herein, the term "inulin" is understood as a simple inactive polysaccharide consisting of $\beta$-D-[2$\rightarrow$1]-polyfructofuranosyl$\alpha$-D-glucose family wherein fructose is bound straight without a side chain and a glucose is bound to the end, and includes not only inulin and $\beta$-D-[2$\rightarrow$1]-polyfructofuranosyl$\alpha$-D-glucose, but also inulin derivatives (includes functional equivalents in some cases), including for example $\beta$-D-[2$\rightarrow$1]-polyfructose which is potentially obtained by enzymatic removal of the terminal glucose from inulin by using an invertase or inulase enzyme that can remove the glucose at the end thereof. Other derivatives included within the scope of the term or functional equivalent are, for example, inulin derivatives with a free hydroxyl group etherified or esterified by a chemical substitution with alkyl, aryl, or acyl group by a known method. While an inulin composition has a known constitution consisting of a simple and neutral polysaccharide, the molecular weight varies, ranging from 16 kilodaltons (kD) or less to greater. Inulin is a reserve carbohydrate of Compositae and is available at low cost from the bulb of dahlia. Inulin has a relatively hydrophobic polyoxyethylene-like backbone. In addition to this rare structure, the non-ionized property enables preparation of very pure inulin readily by recrystallization. In nature, inulin consists of fructose with a degree of polymerization (DP) of about 60 or greater and has various solubilities, properties, and the like.

While the molecular composition of inulin is well known, the reported solubily varies. Currently at least 5 types of inulin are known, i.e., alpha inulin (aIN; see Phelps, CF. The physical properties of inulin solutions. Biochem J95: 41-47(1965)), beta inulin (bIN; see Phelps, CF. The physical properties of inulin solutions. Biochem J95: 41-47(1965)), gamma inulin (gIN), delta inulin (dIN, also known as deltin), and epsilon inulin (eIN). aIN to dIN are characterized by different dissolution rates in an aqueous medium, i.e., from a form that rapidly dissolves at 23°C (beta inulin; $\beta_{23°}$ inulin) through a form that is soluble with a half-life of 8 minutes at 37°C (alpha inulin; $\alpha_{37}{}^{8}$ inulin), and a form that is substantially insoluble at 37°C (gamma inulin) and a form that is substantially insoluble at 50°C (delta inulin) (for gamma inulin, see WO87/02679 and Cooper, P.D. and Carter, M., 1986 and Cooper, P.D. and Steele, E.J., 1988). As discussed below, eIN was identified as having a different property from aIN to dIN. For delta inulin, an adjuvant product sold as Advax™ is known. Delta inulin is insoluble to water at 50°C. Delta inulin is disclosed in WO 2006/024100, the content of which is incorporated herein by reference. Delta inulin is soluble only when heated to 70 to 80°C in a concentrated solution (e.g., 50 mg/ml). The 50% OD700 thermal transition point in a non-concentrated solution is 53 to 58°C. Delta inulin can be readily prepared by heating a concentrated gamma inulin solution to 55°C or higher. Alpha inulin (aIN) is obtained by precipitation from water. Beta inulin is obtained by precipitation from ethanol. Epsilon inulin preferably has a 50% $OD_{700}$ thermal transition point of a diluted suspension (< 0.5 mg/ml) in the range of about 58°C to about 80°C, and has a low solubility to a water solvent at 59°C or lower, more preferably 75°C or lower. A single molecule of an eIN particle has a molecular weight in the range from about 5 to about 50 kilodaltons (kD). The degree of polymerization (DP) of a single molecule of an eIN particle is high in many cases (i.e., degree of polymerization of fructose of 25 or greater, preferably 35 or greater). eIN exhibits a lower solubility to dimethyl sulfoxide (solvent known to neutralize a hydrogen bond) compared to each of aIN, bIN, gIN, and dIN.

Gamma inulin is substantially insoluble to water at 37°C, but is soluble to a concentrated solution (e.g., 50 mg/ml) only at a temperature of 45°C or higher, as in $\alpha$ and $\beta$ polymorphic forms. Delta inulin is particulate and has a sharp melting point, i.e., 50% OD700 thermal transition point (dissolution phase transition of non-concentrated solution) of 47 $\pm$ 1°C. Delta inulin is insoluble to water at 50°C, and soluble to a concentrated solution (e.g., 50 mg/ml) only when

heated to 70 to 80°C as described in WO 2006/024100. dIN is characterized by a 50% OD700 thermal transition point in a non-concentrated solution of 53 to 58°C. dIN can be readily prepared by heating a concentrated gIN solution to 55°C or higher. Delta inulin (dIN) and gamma inulin (gIN) are insoluble at 37°C. It is already known that even if introduced into an organism such as a human with this temperature, particulate forms thereof can be maintained, and they are immunologically active and effective especially as an adjuvant of a vaccine, alone or with an antigen binding carrier material such as aluminum hydroxide (Cooper, PD and EJ Steele, 1991, Cooper, PD et al., 1991a, Cooper, PD et al., 1991b. WO90/01949 and WO2006/024100). Episilon inulin is also immunologically active and can have the same or higher immunological activity relative to gamma inulin and delta inulin. Epsilon inulin is the most thermostable among the five inulin polymorphic forms. A suspension of the particles thereof remains insoluble even at temperatures at which other polymorphic forms dissolve. Epsilon inulin is the most thermostably advantageous even when heated to 85°C. When used as an adjuvant requiring thermostability, epsilon inulin particles can be stable at high temperatures.

As used herein, "$\delta$ inulin ($\beta$-D-[2$\rightarrow$1]poly(fructo-furanosyl)$\alpha$-D-glucose" is a substance constituting an adjuvant. An adjuvant consisting of microparticles constituted thereby is typically available as an adjuvant product known as Advax™. Advax™, which is a delta inulin adjuvant, is a microparticulate adjuvant. The microparticles thereof are derived from microparticles of polyfructo furanosyl-d-glucose (delta inulin). It is shown that this improves the immunogenicity and potency of various vaccines including vaccines to influenza, hepatitis B, Japanese encephalitis, West Niles virus, HIV, Bacillus anthracis, and Listeria (Dolter et al., 2011; Feinen et al., 2014; Honda-Okubo et al., 2012; Larena et al., 2013; Petrovsky et al., 2013; Rodriguez-Del Rio et al., 2015; Saade et al., 2013). A vaccine comprising the Advax™ adjuvant such as a vaccine for hepatitis B, influenza, and allergy due to an insect bite has been evaluated in a human clinical trial (Gordon et al., 2014; Heddle et al., 2013; Nolan et al., 2008).

While excellent immunogenicity and tolerance have been demonstrated in these clinical trials, the mechanism of action of Advax™ is still unknown. In the present invention, various types of vaccine antigens were used to test the adjuvant effect of delta inulin. Unexpectedly, delta inulin did not bias an immune response to a co-administered antigen, unlike TLR agonists. Interestingly, delta inulin instead enhanced the immune bias of the vaccine antigen itself. This suggests that delta inulin functions in a new form of "adjuvant of adjuvant" and has action to amplify the innate adjuvant activity of the antigen itself.

As used herein, "elicit or enhance adjuvantivity of antigen" means that for an antigen, antigen's own adjuvantivity is elicited (e.g., generated when absent) or enhanced (e.g., increases an already present activity).

As used herein, "activate dendritic cells" refers to dendritic cells reaching a state where they can exert the innate function thereof or increasing the degree of the state. Examples thereof include elevating the expression of co-stimulatory molecules and presenting an antigen to naive T cells that have never encountered the antigen to give or enhance the function to activate naive T cells, and the like. Dendritic cells that have never encountered a foreign object are called immature dendritic cells, which are significantly different from activated dendritic cells, including the expression of cell surface molecules and the like. While immature dendritic cells are highly phagocytic, expression levels of MHC class II molecules and co-stimulatory molecules such as CD80, CD86, and CD40 are low. Dendritic cells intake antigens even without an onset of infection or the like, but are not able to active naive T cells due to the lower expression of MHC class II or co-stimulatory molecules. Upon bacterial or viral infection, dramatic change is induced in dendritic cells. Dendritic cells that are activated and mature due to various stimulations with the infection would express a large amount of MHC class II presenting an antigen peptide from bacteria or virus and have increased expression of co-stimulatory molecules, and migrate to the T cell region of their lymph node through the lymph duct in a chemokine receptor CCR7 dependent manner. An antigen is presented to naive T cells in the T cell region of the lymph node, and various cytokines are simultaneously released to induce differentiation from naive T cells to effector T cells. It is understood that the following three types of signals are involved in activation of dendritic cells upon an infection. The first activating signal is from cytokines such as TNF$\alpha$ that release neutrophils, macrophages, or the like which have infiltrated the infected site, second activating signal is from a dead cell derived component from neutrophils, macrophages, or the like that have died upon the infection, and third activating signal is from Toll-like receptors (TLR) (see the section of macrophage in part 7 for details) that recognize a component from bacteria or virus (e.g., lippolysaccharide from gram negative bacteria, or the like). Dendritic cells remain at a site of infection for several hours, take in antigens sufficiently and become activated, then migrate their lymph node through the lymph duct, activate naive T cells, and end their life in about a week. New dendritic cells are supplied from the bone marrow to the infected site where dendritic cells are no longer moving. As long as the infection persists, the step of activation of dendritic cells at the focus of infection $\rightarrow$ migration to their lymph node is repeated.

As used herein, "in the presence of macrophage" refers to any environment where an innate or exogenous macrophage is present.

As used herein, "macrophage enhancer" refers to any agent that imparts or enhances the function or activity of a macrophage. Examples of macrophage enhancers include picolinic acid, crystalline silica, conventional adjuvant such as aluminum salt, and the like.

As used herein, "Th1 type antigen" refers to an antigen associated with Th1 cells, preferably any antigen that

elicits or enhances a Th1 immune response. As explained below, Th1 type antigen especially refers to antigens that enhance cellular immunity (engulfs pathogen cells).

As used herein, "Th2 type antigen" refers to an antigen associated with Th2 cells, preferably any antigen that elicits or enhances a Th2 immune response. As explained below, Th2 type antigen especially refers to antigens that enhance humoral immunity (neutralizes toxin of pathogens).

Although the present invention will be explained while presuming Th1 cells and Th2 cells as having any property or function known in the art, the properties and functions of Th1 and Th2 cells that are especially noteworthy herein are further explained hereinafter. Lymphocytes are divided into T cells and B cells that produce an antibody (immunoglobulin). T cells are further divided into helper T cells (CD4 antigen positive) that regulate immune reactions with presentation of an antigen from a monocyte/macrophage and killer T cells (CD8 antigen positive) that kills viral infection cells or the like. Helper T cells are divided into Th1 cells (type 1 T helper cells) and Th2 cells (type 2 T helper cells). Whether antigen presenting cells produce interleukin (IL)-12 or prostaglandin (PG) E2 determines which of Th1 cells (responsible for cellular immunity) and Th2 cells (responsible for humoral immunity) are dominant.

IL-12 secreted by an antigen presenting cell macrophage when presenting an antigen to a T cell differentiates Th0 cells (naive Th cells) to Th1 cells. Th1 cells produce IL-2, interferon (IFN)-$\gamma$ (suppress production of IgE antibody), tumor necrosis factor (TNF)-$\alpha$, TNF-$\beta$, granulocyte-macrophage colony-stimulating factor (GM-CSF), and IL-3 to increase the activity of phagocytes such as monocytes and are involved in cellular immunity (tuberculin reaction or the like). IFN-$\gamma$ produced by Th1 cells promotes differentiation of Th0 cells into Th1 cells. PGE2 secreted by a macrophage when presenting an antigen to T cells induces differentiation of Th0 cells into Th2 cells. Th2 cells produce IL-3, IL-4 (cytokine increasing the production of immunoglobulin (Ig) E antibodies; also produced from mastocytes and natural killer (NK) T cells), IL-5, IL-6, IL-10, and IL-13, and are involved in humoral immunity (antibody production or the like). IL-10 suppresses the production of IL-12 and the production of IFN-$\gamma$ from Th1 cells. IL-4 or IL-6 produced by Th2 cells promotes differentiation of Th0 cells into Th2 cells. For differentiation of Th0 cells into Th2 cells, PGE2 produced from arachidonic acid is understood to be more important than IL-4. Th2 cells proliferate even with an antigen stimulation from B cells (does not produce IL-12) as an antigen presenting cell.

In an immune response by Th1 cells, cellular immunity results in an inflammatory reaction mainly around mononuclear cells such as lymphocytes and macrophage. For example in an immune response to fungus Cryptococcus, Th1 cells predominantly act to form a strong granuloma to confine the infection locally. Meanwhile, if Th2 cells predominantly act, inflammatory cell infiltration is extremely poor. For example, humoral immunity does not kill intracellular parasites such as Cryptococcus. For this reason, Cryptococcus fills the alveolar space, such that the infection readily spreads hematogenously to result in the onset of meningitis or the like. It is undderstood that IgE antibody production increases so that a subject is more likely to have an allergic disposition when Th2 cells are more predominant than Th1 cells.

Fungal components (Pathogen-associated molecular pattern: PAMP) act on dendritic cells, promote differentiation of Th0 cells into Th1 cells and create a Th1 cell predominant state to improve allergic disposition. This is the basis for the so-called sanitary hypothesis to consider intake of food such as natto or yogurt improving allergic disposition. Type 1 interferon (IFN-$\alpha$ and IFN-$\beta$) is produced in a viral infection. Type 1 interferon acts on T cells to induce production of IFN-$\gamma$ or IL-10.

In a bacterial infection, type 2 interferon IFN-$\gamma$ is produced to induce Th1 cells. In an infection by intracellular parasitic bacteria (tubercle bacillus, salmonella, Listeria, or the like), mainly Th1 cells are induced, phagocytes (macrophage) are activated due to IFN-$\alpha$ produced from Th1 cells, and CD8 positive killer T cells are activated due to IL-2 produced from Th1 cells to kill bacteria or the like.

In an infection by extracellularly proliferating bacteria (gram positive coccus such as Staphylococcus or the like), mainly Th2 cells are induced and antibodies are produced by cytokines produced from Th2 cells to kill bacteria or the like. Th1 cells produce IL-2, activate killer T cells, NK cells, or the like, and activate cellular immunity. Th2 cells produce IL-4, activate B cells via a CD40 ligand (CD40L, gp39), and promote production of type I allergy causing IgE antibodies to activate humoral immunity. Th1 cells also produce IFN-$\gamma$, but IFN-$\gamma$ suppresses the CD40 ligand (CD40L) expression of Th2 cells to suppress IgE antibody production. IL-10 and IL-4 produced by Th2 cells suppress the reaction of Th1 cells.

Antigen presenting cells (dendritic cells) identify whether a pathogen (bacteria or virus), toxin, or the like has infiltrated the body by the TLRs on the surface, and produce, in response, inflammatory cytokine, interferon, or the like. As a result, Th0 cells differentiate into Th1 cells or Th2 cells.

In cellular immunity, macrophage is activated by IFN-$\gamma$ produced by Th1 cells to kill intracellular parasitic bacteria. Further, killer T cells are activated by IL-2 produced by Th1 cells to damage viral infection cells.

In humoral immunity, B cells differentiate and proliferate due to IL-4, IL-5, IL-6, and IL-13 produced by Th2 cells, and antibodies (immunoglobulin) are produced. Antibodies neutralize extrabacterial toxins produced by a pathogen, opsonize extracellular parasitic bacteria, promote engulfment by macrophage, activate the complement system, and dissolve bacteria.

As used herein, "Th1 response" refers to the immune response by Th1 cells described above. In an immune response by Th1 cells, cellular immunity acts to induce an inflammatory reaction mainly around mononuclear cells such as lymphocytes and macrophage as described above in detail. In an immune response to fungal Cryptococcus, Th1 cells predominantly act to form a strong granuloma to confine the infection locally. As used herein, "Th2 response" refers to Th2 cells predominantly acting. As described above in detail, inflammatory cell infiltration is extremely poor. For example, humoral immunity cannot kill intracellular parasites such as Cryptococcus. For this reason, Cryptococcus fills the alveolar space, such that the infection readily spreads hematogenously to result in the onset of meningitis or the like.

As used herein, "normal or enhanced state of TNFα" refers to a state where tumor necrosis factor α (TNFα) is maintained at a normal level in vivo or normal level of TNFα in vivo is replicated. "Enhanced" refers to a state where there is a higher level of TNFα than normal level of TNFα in vivo, or higher TNFα than the normal level in vivo is replicated.

As used herein, "adjuvant of adjuvant" is understood as a concept encompassing imparting adjuvant activity to a substance comprising activity to enhance the adjuvant activity of a compound already known to be an adjuvant and other substances that are lacking or unknown to be adjuvant.

As used herein, "candidate adjuvant" is a type of a candidate drug component, referring to any substance or a combination thereof considered as an adjuvant. A candidate adjuvant can be a TLR independent adjuvant, TLR dependent adjuvant, or the like as described below, but a compound whose function or property is unknown as an adjuvant, other substances, and combinations thereof can also be used. Examples of TLR independent adjuvant include, but are not limited to the following: alum (aluminum phosphate/aluminum hydroxide; inorganic salt exhibiting various adaptations); AS03 (GSK; squalene) (10.68 mg), DL-α-tocopherol (11.86 mg), and polysorbate 80 (4.85 mg), oil-in-water emulsion used in pandemic influenza; MF59 (Novartis; 4 to 5% (w/v) squalene, 0.5% (w/v) Tween 80, 0.5% Span 85, optionally variable amounts of muramul tripeptide phosphatidyl-ethanolamine (MTP-PE)), oil-in-water emulsion used in influenza): Provax (Biogen Idec; squalene + Pluronic L121), an oil in water emulsion); Montanide (Seppic SA; Bioven; Cancervax; mannide oleate and mineral oil), water-in-oil emulsion used in treating malaria and cancer); TiterMax (CytRx; squalene + CRL-8941), water-in-oil emulsion); QS21 (Antigenics; fraction of Quil A), plant-derived composition used in treating melanoma, malaria, HIV, and influenza); Quil A (Statens Serum Institute; purified fraction of Quillaja saponaria), plant-derived composition used in various treatments); ISCOM (CSL; Isconova; saponin + sterol plus + optionally phospholipid), plant-derived composition used in various treatments including influenza); liposomes (Crucell; Nasvax; synthetic phospholipid spheres consisting of lipid), used in treating various disease), and the like.

TLR-dependent adjuvants include, but are not limited to the following: Ampligen (Hemispherx; synthetic specifically configured double-stranded RNA containing regularly occurring regions of mismatching), effected by activation of TLR3 and used as a vaccine against pandemic flu); AS01 (GSK; MPL, liposomes, and QS-21), effected by MPL-activation of TLR4, liposomes provide enhanced antigen delivery to APCs, QS-21 provides enhancement of antigen presentation to APCs and induction of cytotoxic T cells, also used as a vaccine against malaria and tuberculosis.); AS02 (GSK; MPL, o/w emulsion, and QS-21) is effected by MPL-activation of TLR4, the o/w emulsion provides innate inflammatory responses, APC recruitment and activation, enhancement of antigen persistence at injection site, presentation to immune-competent cells, elicitation of different patterns of cytokines, and the QS-21 provides enhancement of antigen presentation to APCs and induction of cytotoxic T cells; this is also used as a vaccine against malaria, tuberculosis, HBV, and HIV; AS04 (GSK; MPL, aluminum hydroxide/aluminum phosphate) is effected by MPL-activation of TLR4, alum provides a depot effect, local inflammation, and increase in antigen uptake by APCs; this is also used as a vaccine for HBV, HPV, HSV, RSV, and EBV.); MPL RC-529 (Dynavax; MPL) is effected by activation of TLR4 and is used as a vaccine against HBV); E6020 (Eisa/Sanofi Pasteur; synthetic phospholipid dimer) is effected by activation of TLR4); TLR-technology (Vaxinnate; antigen and flagellin, effected by activation of TLR5 and used in vaccines against influenza); PF-3512676 (CpG 7909) (Coley/Pfizer/Novartis; immunomodulating synthetic oligonucleotide), effected by activation of TLR9 and used in vaccines against HBV, influenza, malaria, and anthrax.); ISS (Dynavax; short DNA sequences), effected by activation of TLR9 and used in vaccines against HBV and influenza.); IC31 (Intercell; peptide and oligonucleotide), effected by activation of TLR9, formation of an injection site depot, and enhancing of antigen uptake into APCs and used as a vaccine against influenza, tuberculosis, malaria, meningitis, allergy, and cancer indications.); and the like.

As used herein, "evaluation reference adjuvant" is also called "reference adjuvant" or "standard adjuvant", which is a reference drug component and is referred to as an adjuvant with a known function. Such an adjuvant has a property or function determined by a method known in the art. For example, the function of δ inulin (β-D-[2→1]poly(fructo-furanosyl)α-D-glucose) or a function equivalent thereof as an adjuvant is known, so that the present invention can use this as a reference.

As used herein "gene expression data" refers to any expression data of various genes.

<δ inulin which is an adjuvant of "adjuvant">

In one aspect, the present invention provides a composition for eliciting or enhancing adjuvanticity of an antigen comprising δ inulin (β-D-[2→1]poly(fructo-furanosyl)α-D-glucose) or a functional equivalent thereof. In this regard, examples thereof include, but are not limited to, an adjuvant product known as Advax™ of δ inulin (β-D-[2→1]poly(fructo-furanosyl)α-D-glucose) or a functional equivalent thereof.

In one embodiment, an equivalent of δ inulin used herein has a transcriptome expression profile that is equivalent to that of δ inulin. Such a transcriptome expression profile can be used in practice by performing transcriptome analysis and analysis of a gene expression profile. Transcriptome analysis can be practiced by any approach described herein.

<Dendritic cell activation>

In another aspect, the present invention provides a composition for activating a dendritic cell, comprising δ inulin or a functional equivalent thereof. In this regard, activation can be performed, for example, in the presence of a macrophage. Alternatively, the composition comprising δ inulin or a functional equivalent thereof can be administered with a macrophage enhancer. This is because the present invention has found that δ inulin or a functional equivalent thereof activates dendritic cells under conditions where a macrophage is normal or enhanced. Therefore, the present invention can be the basis for activation or an indicator when using δ inulin or a functional equivalent thereof as an adjuvant. For δ inulin or a functional equivalent thereof used herein, any substance explained in the section of <Adjuvant of "adjuvant"> or a combination thereof can be used. Further, an adjuvant having the same dendritic cell activation as δ inulin or a functional equivalent thereof can be identified by using transcriptome analysis explained in <Adjuvant of "adjuvant"> or <Same adjuvant/adjuvant determination method and manufacturing method>.

<Th orientation>

In another aspect, the present invention provides a composition for enhancing a Th1 response of a Th1 type antigen and a Th2 response of a Th2 type antigen, comprising δ inulin or a functional equivalent thereof. For δ inulin or a functional equivalent thereof used herein, any substance explained in the section of <Adjuvant of "adjuvant"> or a combination thereof can be used. Further, an adjuvant having the same Th orientation as δ inulin or a functional equivalent thereof can be identified by using transcriptome analysis explained in <Adjuvant of "adjuvant"> or <Same adjuvant/adjuvant determination method and manufacturing method>.

<Technology based on TNFα KO mice>

In another aspect, the present invention provides an adjuvant composition comprising δ inulin or a functional equivalent thereof, wherein the composition is administered while TNFα is normal or enhanced. For δ inulin or a functional equivalent thereof used herein, any substance explained in the section of <Adjuvant of "adjuvant"> or a combination thereof can be used. Further, an adjuvant having the same property under conditions where TNFα is normal or enhanced as δ inulin or a functional equivalent thereof can be identified by using transcriptome analysis explained in <Adjuvant of "adjuvant"> or <Same adjuvant/adjuvant determination method and manufacturing method>.

<Same adjuvant/adjuvant determination method and manufacturing method>

In one aspect, the present invention provides a method of determining whether a candidate adjuvant elicits or enhances adjuvanticity of an antigen. The method comprises: (a) providing a candidate adjuvant; (b) providing δ inulin or a functional equivalent thereof as an evaluation reference adjuvant; (c) obtaining gene expression data by performing transcriptome analysis on the candidate adjuvant and the evaluation reference adjuvant to cluster the gene expression data; and (d) determining the candidate adjuvant as eliciting or enhancing adjuvanticity of an antigen if the candidate adjuvant is determined to belong to the same cluster as the evaluation reference adjuvant. For δ inulin or a functional equivalent thereof used herein, any substance explained in the section of

<Adjuvant of "adjuvant"> or a combination thereof can be used.

A candidate adjuvant can be provided in any form in the method of the invention. δ inulin or a functional equivalent thereof can also be provided as an evaluation reference adjuvant in any form. For example, delta inulin such as Advax™ can be used as an evaluation reference adjuvant. Advax™ is a crystalline nanoparticles of inulin, which is a biological polymer used in vaccines against hepatitis B (prophylactic and therapeutic), influenza, Bacillus anthracis, Shigella, Japanese encephalitis, rabies, bee toxin, or allergy, and cancer immunotherapy (sold by Vaxine Pty).

In another aspect, the present invention provides a method of manufacturing a composition comprising an adjuvant that elicits or enhances adjuvanticity of an antigen. The method comprises: (a) providing one or more candidate adjuvants; (b) providing δ inulin or a functional equivalent thereof as an evaluation reference adjuvant; (c) obtaining gene expression data by performing transcriptome analysis on the candidate adjuvant and the evaluation reference adjuvant

to cluster the gene expression data; (d) if there is an adjuvant belonging to the same cluster as the evaluation reference adjuvant among the candidate adjuvants, selecting the adjuvant as an adjuvant that elicits or enhances adjuvanticity of an antigen, and if not, repeating (a) to (c); and (e) manufacturing a composition comprising an adjuvant that elicits or enhances adjuvanticity of an antigen obtained in (d). For δ inulin or a functional equivalent thereof used herein, any substance explained in the section of <Adjuvant of "adjuvant"> or a combination thereof can be used.

<Drug using adjuvant of adjuvant>

An adjuvant or "adjuvant of adjuvant" used in the present invention is provided as a pharmaceutical product or pharmaceutical composition.

The composition of the invention can be prepared as an injection, or oral, enteral, transvaginal, transdermal, or transocular agent with a pharmaceutically acceptable carrier, diluent, or excipient. The composition can also be a composition comprising an active ingredient such as a vaccine antigen (including genetically recombinant antigen), antigen peptide, or anti-idiotypic antibody. An additional or alternative active ingredient can be a lymphokine, cytokine, thymocyte simulation factor, macrophage simulating factor, endotoxin, polynucleotide molecule (e.g., encoding a vaccine antigen) or recombinant viral vector, microorganisms (e.g., microorganism extract), or virus (e.g., inactivated or attenuated virus). In fact, the composition of the invention is especially suitable for use when an inactivated or attenuated virus is an active ingredient.

When the present invention is used as an adjuvant composition, preferred target vaccine antigens include some or all of antigens of bacteria, virus, yeast, mold, protozoa, and other microorganisms, human, animal, or plant derived pathogens, pollen, and other allergens, especially toxins (e.g., toxin of honey bees or wasps) and allergens inducing asthma such as house dust mites and dog or cat dandruff.

Particularly preferred vaccine antigens are HA protein of an influenza virus (e.g., inactivated seasonable influenza virus and seasonal H1, H3, or B strain or pandemic H5 strain recombinant HA antigen), influenza nucleoprotein, rotavirus outer capsid protein, human immunodeficiency virus (HIV) antigen such as gp120, RS virus (RSV) surface antigen, human papilloma virus E7 antigen, herpes simplex virus antigen, hepatitis B virus antigen (e.g., HBs antigen), hepatitis C virus (HCV) surface antigen, inactivated Japanese encephalitis, lyssavirus surface antigen (inducing rabies), and other viral antigens, Shigella, Porphyromonas gingivalis (e.g. proteases and adhesin protein), Helicobacter pylori (e.g., urease), Listeria monocytogenes, Mycobacterium tuberculosis (e.g., BCG), Mycobacterium avium (e.g., hsp65), Chlamydia trachomatis, Candida albicans (e.g., the outer membrane proteins), pneumococcus, meningococcus (e.g., class 1 outer membrane protein), Bacillus anthracis (anthrax causing bacteria), Coxiella burnetti (Q fever causing bacteria that can induce a long-term defense response against autoimmune diabetes (i.e. type 1 diabetes)), other microorganism derived antigens, and malaria causing protozoa (especially Plasmodium falciparum and Plasmodium vivax). Other particularly preferred antigens are cancer antigens (i.e., antigen associated with one or more cancers) such as carcinoembryonic antigen (CEA), mucin-1 (MUC-1), epithelial tumor antigen (ETA), abnormal products of p53 and ras, and melanoma antigens (MAGE).

When the composition of the invention is a vaccine antigen, the composition preferably comprises an antigen binding carrier material. An antigen binding carrier material is, for example, one or more of magnesium, calcium, or aluminum phosphate, sulfate, hydroxide (e.g., aluminum hydroxide and/or aluminum sulfate) and other metal salts or precipitates, and/or one or more of protein, lipid, sulfated or phosphorylated polysaccharide (e.g., heparin, dextran, or cellulose derivative) containing organic acid and chitin (poly N-acetylglucosamine), deacetylated derivative thereof, base cellulose derivative, other organic bases, and/or other antigens. An antigen binding carrier material can be particles of any material with poor solubility (aluminum hydroxide (alum) gel or hydrated salt complex thereof). Typically, an antigen binding carrier material does not have a tendency to aggregate, or is treated to avoid aggregation. Most preferably, an antigen binding carrier material is an aluminum hydroxide (alum) gel, aluminum phosphate gel, or calcium phosphate gel.

The present invention can be provided as a kit. As used herein, "kit" refers to a unit providing portions to be provided (e.g., testing agent, diagnostic agent, therapeutic agent, antibody, label, manual, and the like), generally in two or more separate sections. This form of a kit is preferred when intending to provide a composition that should not be provided in a mixed state and is preferably mixed immediately before use for safety reasons or the like. Such a kit advantageously comprises instructions or a manual preferably describing how the provided portions (e.g., testing agent, diagnostic agent, or therapeutic agent) should be used or how a reagent should be processed. When the kit is used herein as a reagent kit herein, the kit generally comprises an instruction describing how to use a testing agent, diagnostic agent, therapeutic agent, antibody, and the like.

In this manner in another aspect of the invention, the present invention is directed to a kit, the kit further comprising: (a) a container comprising the pharmaceutical composition of the invention in a solution form or a lyophilized form, (b) optionally a second container comprising a diluent or a reconstitution solution for the lyophilized formulation, and (c) optionally a manual directed to (i) use of the solution or (ii) reconstitution and/or use of the lyophilized formulation. The kit further has one or more of (iii) buffer, (iv) diluent, (v) filter, (vi) needle, or (v) syringe. The container is preferably

a bottle, vial, syringe, or a test tube or a multi-purpose container. The pharmaceutical composition is preferably lyophilized.

The kit of the invention preferably has a manual for the lyophilized formulation of the invention and reconstitution and/or use thereof in a suitable container. Examples of the suitable container include a bottle, vial (e.g., dual chamber vial), syringe (dual chamber syringe or the like), and test tube. The container can be made of various materials such as glass or plastic. Preferably, the kit and/or container comprises a manual showing the method of reconstitution and/or use on the container or accompanying the container. For example, the label thereof can have an explanation showing that the lyophilized formulation is reconstituted to have the aforementioned peptide concentration. The label can further have an explanation showing that the formulation is useful for, or is for subcutaneous injection. The kit of the invention can have a single container including a formulation of the pharmaceutical composition of the invention with or without other constituent elements (e.g., other compounds or pharmaceutical composition of the other compounds) or have separate containers for each constituent element.

The pharmaceutical composition of the invention is suitable for administering the peptide via any acceptable route such as oral (enteral), transnasal, transocular, subcutaneous, intradermal, intramuscular, intravenous, or transdermal route. Preferably, the administration is subcutaneously administration, and most preferably intradermal administration. Administration can use an infusion pump. Therefore, the medicament of the invention can be provided as a therapeutic or prophylactic method. Such a method for treating or preventing a disease comprises administering an effective amount of the composition, adjuvant, or medicament of the invention to a subject in need thereof with an effective amount of vaccine antigen or the like.

As used herein, "or" is used when "at least one or more" of the listed matters in the sentence can be employed. When explicitly described herein as "within the range" of "two values", the range also includes the two values themselves.

(General technology)

Any molecular biological approaches, biochemical approaches, microbiological approaches, and bioinformatics that is known in the art, well known, or conventional can be used herein.

Reference literatures such as scientific literatures, patents, and patent applications cited herein are incorporated herein by reference to the same extent that the entirety of each document is specifically described.

As described above, the present invention has been described while showing preferred embodiments to facilitate understanding. The present invention is described hereinafter based on Examples. The above descriptions and the following Examples are not provided to limit the present invention, but for the sole purpose of exemplification. Thus, the scope of the present invention is not limited to the embodiments and Examples specifically described herein and is limited only by the scope of claims.

[Examples]

The Examples are described hereinafter. When necessary, all experiments were conducted in compliance with the guidelines approved by the ethics committee of the Osaka University in the following Examples. For reagents, the specific products described in the Examples were used. However, the reagents can be substituted with an equivalent product from another manufacturer (Sigma-Aldrich, Wako Pure Chemical, Nacalai Tesque, R & D Systems, USCN Life Science INC, or the like). PBS used as a control reagent and DMSO were obtained from Nacalai Tesque. Tris-HCl was obtained from Wako Pure Chemical.

(Method)

Standard operation protocol

All procedures adhere to each of the following standard operation protocols (standard procedure: adjuvant administration and organ sampling (standard procedure 1), RNA extraction and GeneChip data acquisition (standard procedure 2), and quality control and final data inclusion to the database (standard procedure 3). Detailed information on these protocols is summarized below. All experiments were conducted under the appropriate laws and guidelines and approved by the National Institutes of Biomedical Innovation, Health and Nutrition.

(Standard procedure 1)

(Adjuvant administration and sampling)

C57BL/6 mice (male, 5-week old, C57BL/6JJc1) were purchased from CLEA Japan and acclimated for at least one week (day -7 to day -10). On day 1 at 10 AM: start administration of buffer or adjuvant solution (finish administration

within 30 minutes).

i.d.: administer a total of 100 μL to the base of the tail (left side 50 μL + right side 50 μL)
*A total of 200 μL of bCD was i.d. administered.
i.p.: administer a total of 200 μL to the lower quadrant of abdomen.
i.n.: subcutaneously inject 50 μL of ketamine/xylazine mixture (90 mg/kg of ketamine and 10 mg/kg of xylazine) for anesthesia. Slowly drip 10 μL of the solution into the nose (5 μL into each nasal cavity) with a P20 PIPETMAN. 4 PM: Start sampling of organ (finish sampling from all mice within 30 minutes)

(Blood sampling)

Take about 200 μL of blood from the retro-orbital venous plexus with a non-heparinized capillary tube and place the blood into a 1.5 mL tube comprising 2 μL of 10% EDTA-2K for hematological testing. Thoroughly mix the sample by gentle tapping. Stored at room temperature until hematological testing.

(Organ sampling)

LN: Expose and remove inguinal lymph nodes (both sides). Remove adipose tissue to reduce adipose tissue contamination as much as possible under a stereoscopic microscope in a 35 mm dish containing about 1 mL of RNAlater. After cleaning, transfer lymph nodes of both sides to a 2.0 mL Eppendorf Protein LoBind tube containing 1 mL of RNAlater.

SP: Expose and remove spleen. Remove adipose tissue and pancreas tissue as much as possible. After cleaning, the divide spleen into three parts with a razor blade. Transfer each part individually to 2.0 mL Eppendorf Protein LoBind tubes containing 1 mL of RNAlater (total of three tubes). LN: Expose and remove the left lobe of a liver. Punch out three parts with a Biopsy Punch (φ 5mm). Transfer each part to 2.0 mL Eppendorf Protein LoBind tubes containing 1 mL of RNAlater (total of three tubes). Place each harvested organ in a tube containing RNAlater. Maintain the tubes at 4°C overnight and store at -80°C until use.

(Hematological cell count)

The hematological cell count was found using VetScan HMII (Abaxis). 50 μl of EDTA-2K blood sample was diluted by adding 250 μl of saline. Measurements are taken with VetScan HMII by following the instruction.

(Standard procedure 2)

(RNA extraction and GeneChip data acquisition)

This protocol was established by Molecular Toxicology, Biological Safety Research Center, National Institute of Health Sciences and Toxicogenomics informatics project (TGP2), National Institutes of Biomedical Innovation, Health and Nutrition and Division of Cellular by reference to the manufacture's manuals.

(1. Homogenization of animal tissues)

*1.1. Reagents and equipments

*1.1.1 Reagents

1) RNeasy® Mini Kit (QIAGEN, cat.# 74106)
2) Buffer RLT*
3) 2-Mercapto Ethanol (β-ME)

1.1.2. Equipments

1) Zirconium beads (diameter of 5 mm, Tosoh, cat.# YTZ-5)
2) Electronic scale

3) Aspirator
4) Mixer Mill MM300 (QIAGEN)
5) Microcentrifuge

*1.2. Preparation of Reagents

*1.2.1. Buffer RLT*

1) Add 10 μL of 2-Mercapto Ethanol per 1 mL Buffer RLT before use

*stored at room temperature for up to 1 month

*1.3. Homogenization of Animal Tissues Procedures

1) Dissolve the samples at room temperature.

Confirm that no crystals or a precipitate are present in RNAlater.
*Generally, this will not affect subsequent RNA purification. However, in rare cases, this may lead to RNA instability.

2) Measure organ weight.

*The weight of samples should be about 30-100 mg.
* The weight of muscle samples should be about 30-50 mg. Excessive muscle sample weight leads to the coagulation of homogenate.

3) Remove the RNAlater reagent by aspiratoration. Any crystals that may have formed should be removed at this time.
4) Place Zirconium beads into the tubes (1 bead per tube) using flame-sterilized tweezers.
5) Add 400 μL of Buffer RLT.

*In the case of muscle samples, add 600 μL of Buffer RLT in order to prevent coagulation of homogenate.

6) Place the tubes on the Mixer Mill adaptor. At the TGP2 laboratory, the tubes are placed only on each side of the Mixer Mill Adaptor. Placing tubes on the center of the Mixer Mill Adaptor may cause incomplete disruption.
7) Disrupt and homogenize organ slice by the Mixer Mill under optimal conditions.

*At the TGP2 laboratory, disruption is carried out for 3 min at 25 Hz at room temperature. After the initial disruption step, the Mixer Mill Adapter should be rotated to ensure that each tube is homogenized equally. The second disruption step is also carried out for 3 min at 25 Hz at room temperature.

8) Remove bubbles arising during homogenization by centrifugation for 1 min at 300 x g at room temperature.
9) Store homogenate at -80°C.

(2. Purification of Total RNA from Animal Tissues (TRI-easy method)

This chapter describes the extraction and purification of total RNA from animal tissue. The "TRI-easy method" combines acid guanidinium-phenol-chloroform (AGPC) extraction and RNeasy technology.

*2.1. Reagents and equipments

*2.1.1. Reagents

1) TRIzol® LS Reagent (Invitrogen, cat.# 10296-028)
2) RNeasy® Mini Kit (QIAGEN, cat.# 74106)

Buffer RLT*
Buffer RW1*
Buffer RPE
RNase-Free water
RNeasy mini spin columns
Collection tubes (1.5 mL)
Collection tubes (2 mL)

3) DNase (QIAGEN, cat.# 79254)

DNase I, RNase-Free (lyophilized)
Buffer RDD
DNase-RNase-Free water

4) 2-Mercapto Ethanol (β-ME)
5) Ethanol
6) Chloroform
7) DEPC-Treated water (Ambion, cat.# 9920)

*2.1.2. Equipments

1) Electronic scale
2) Aspirator
3) Mixer Mill
4) Microcentrifuge (rotor for 2 ml tubes)
5) Multiskan Spectrum (Themo Labsystems)
6) Gene Quant pro (Amersham Pharmacia Biotech)

*2.2 Preparation of Reagents
*2.2.1. Buffer RLT*

1) Add 10 μL of β-ME per 1 mL Buffer RLT before use.

*stored at room temperature for up to 1 month
*2.2.2. Buffer RPE

1) Before use for the first time, add 4 volumes of ethanol.

*stored at room temperature
*2.2.3. 50% ethanol

1) Add 1 volume of DEPC-Treated water to the ethanol.

*Do not add bleach or acidic solutions directly to Buffer RLT, Buffer RW1, and the sample-preparation waste.

*2.2.4. DNase

*1) Dissolve the lyophilized DNase I (1500 Kunitz units) in 560 μL of the DNase-RNase-Free water.
*Thawed aliquots can be stored at 2-8°C for up to 6 weeks.
*For long-term storage, aliquots can be stored at -20°C for up to 9 months.
*Do not vortex the reconstituted DNase I.

*Do not refreeze the aliquots after thawing.

2) Add 10 μl DNase I stock solution (see above) to 70 μL Buffer RDD. Mix by gently inverting the tube, and centrifuge briefly to collect residual liquid from the sides of the tube.

[Table 2]

| reagents | one sample | 25 samples | 50 samples |
|---|---|---|---|
| DNase I | 10 μL | 250 μL | 490 μL |
| Buffer RDD | 70 μL | 1750 μL | 3430 μL |

*DNase I is especially sensitive to physical denaturation. Mixing should only be carried out by gently inverting the tube. Do not vortex.

*2.3. [Optional] calculate the volume of Spike RNA

*2.4. Total RNA purification procedures (TRI-easy method)

1) Dissolve the tissue homogenate at room temperature.
2) Adjust the volume to 150 μL with RLT Buffer to prepare the appropriate concentration of the sample homogenate in Collection tubes (2 mL).
3) Add 3 volume of TRIzol LS Reagent (450 μL) and incubate for 5 minutes at room temperature after mixing by vortexing.
4) Add 1 volume of chloroform (150 μL) and incubate for 2 to 15 minutes at room temperature after shaking vigorously by hand for 30 seconds.
5) Centrifuge at no more than 12000 x g for 15 minutes at room temperature.
6) Carefully separate the upper aqueous phase and transfer into a new 1.5 mL tube.
7) Add 1 volume of 50% ethanol and mix by pipetting.

*Using 50% ethanol (instead of 70% ethanol) may increase RNA yields from liver samples.

8) Transfer the supernatant to an RNeasy mini spin column placed in a 2 mL collection tube.
9) Close the lid gently, and centrifuge for 15 seconds at no more than 8,000 x g at room temperature.
10) Discard the flow-through and set the column again.
11) Add 350 μL of Buffer RW1 to the RNeasy spin column to wash the spin column membrane.
12) Close the lid gently, and centrifuge for 15 seconds at no more than 8,000 x g at room temperature.
13) Discard the flow-through and set the column again.
14) [Optional] Add 80 μL of DNase solution to the column and incubate for 15 minutes at room temperature.
15) Add 350 μL of Buffer RW1 to the column.
16) Close the lid gently, and centrifuge for 15 seconds at no more than 8,000 x g at room temperature to wash the spin column membrane.
17) Place the column in a new collection tube.
18) Add 500 μL of RPE to the RNeasy spin column.
19) Close the lid gently, and centrifuge for 15 seconds at no more than 8,000 x g at room temperature.
20) Discard the flow-through and set the column again.
21) Add 500 μL of RPE to the RNeasy spin column.
22) Close the lid gently, and centrifuge for 2 minutes at no more than 8,000 x g at room temperature.
23) Place the column in a new collection tube.
24) Close the lid gently, and centrifuge for 1 minute at no more than 15,000 x g at room temperature.
25) Place the column in a new 1.5 mL collection tube.
26) Add 40 μL of DNase-RNase-Free water to eluted RNA.
27) Incubate the tubes for 3 minutes at room temperature.
28) Close the lid gently, and centrifuge for 1 minute at no more than 15,000 x g at room temperature.
29) Add the total amount of eluate onto the column to reelute RNA for high RNA yield.
30) Incubate the tubes for 3 minutes at room temperature.
31) Close the lid gently, and centrifuge for 1 minute at no more than 15,000 x g at room temperature.

*When the RNA concentration of eluate is lower than the required concentration, repeat steps 29 to 31.

32) Measure OD260, OD280, OD975, OD900 of the eluate by Multiskan Spectrum. The total RNA is required a higher concentration than 500 ng/μL.
33) [QC] The reading OD values, variability, and OD260/OD280
34) [QC] 28S and 18S ribosomal RNA by electrophoresis

(3. Synthesis of cDNA)

*3.1. Reagents and equipments

*3.1.1. Reagents

1) One-Cycle cDNA Synthesis Kit (Affymetrix, cat.# 900431, store at -20°C)

T7-Oligo(dT) Primer, 50 μM
5X 1st Strand Reaction Mix
DTT, 0.1M
dNTP, 10 mM
SuperScript II, 200 U/μL
5X 2nd Strand Reaction Mix
E.coli DNA Ligase, 10 U/μL
E.coli DNA Polymerase I, 10 U/μL
RNase H, 2 U/μL
T4 DNA Polymerase, 5 U/μL
EDTA, 0.5M (store at room temperature)
RNase-free Water (store at room temperature)

*SuperScript for One-Cycle cDNA kit for use with Affymetrix One-Cycle Assays and/or equivalent components from Invitrogen can be used.
2) Sample Cleanup Module (Affymetrix, cat.# 900371)

cDNA Binding Buffer
cDNA Wash Buffer, 6mL concentrate
cDNA Elution Buffer

3) DEPC-Treated water (Ambion, cat.# 9915G)
4) 0.5M EDTA Disodium Salt (Sigma, cat.# E-7889)
5) Ethanol

*3.1.2. Equipments

1) Sample Cleanup Module (Affymetrix, cat.# 900371) cDNA Cleanup Spin Column

2 mL Collection Tube
1.5 mL microtube, DNase/ RNase/pyrogen free

*Do not use 1.5 mL Collection Tube in the kit, because their lids can break in rare cases.
2) Heat block
3) Microcentrifuge

*3.2. Preparation of reagents

*3.2.1. First-Strand Master Mix

1) Prepare sufficient First-Strand Master Mix in a 1.5 mL tube. See the following table.

2) Mix by flicking the tube and spin down briefly after dissolving the solution.
3) Prepare First-Strand Master Mix immediately before use and place on ice.

[Table 3]

| reagents | one sample | 24 samples | 48 samples |
|---|---|---|---|
| 5×First Strand Buffer | 4 μL | 100 μL | 200 μL |
| 0.1M DTT | 2 μL | 50 μL | 100 μL |
| 10 mM dNTPs mix | 1 μL | 25 μL | 50 μL |
| total | 7 μL | 175 μL | 350 μL |

*3.2.2. Second-Strand Master Mix

1) Prepare sufficient Second-Strand Master Mix in a 15 mL tube. See the following table.
2) Mix by flicking the tube and spin down briefly after dissolving the solution.
3) Mix well by gently flicking the tube and spin down briefly.
4) Prepare Second-Strand Master Mix immediately before use and place on ice.

[Table 4]

| reagents | one sample | 24 samples | 48 samples |
|---|---|---|---|
| DEPC-Treated water | 91 μL | 2,275 μL | 4,550 μL |
| 5×First Strand Buffer | 30 μL | 750 μL | 1,500 μL |
| 10 mM dNTP Mix | 3 μL | 75 μL | 150 μL |
| E.coli DNA Ligase | 1 μL | 25 μL | 50 μL |
| E.coli DNA Polymerase I | 4 μL | 100 μL | 200 μL |
| E.coli DNA RNase H | 1 μL | 25 μL | 50 μL |
| total | 130 μL | 3,250 μL | 6,500 μL |

*3.2.3. cDNA Wash Buffer

1) Add 24 mL of ethanol to obtain a working solution and checkmark the box to avoid confusion.

*Store at room temperature.
*When the entire amount of buffer is not used within a month, prepare the required amount of buffer in DNase/RNase free tube.

*3.3. cDNA synthesis procedures

*3.3.1. First Strand cDNA synthesis

1) Prepare 5 pg/10 μL of RNA samples using RNase-free water.
2) Mix well by flicking the tube and spin down briefly after dissolving RNA samples stored at -80°C.
3) Add 2 μL of 50 μM T7-Oligo(dT) Primer. Mix well by flicking the tube and spin down briefly.
4) Incubate the tubes for 10 minutes at 70°C.
5) Cool the sample at 4°C for 2 minutes and spin down briefly.
6) Add 7 μL of First-Strand Master Mix to each reaction mixtures for a final volume of 19 μL. Mix thoroughly by flicking the tube and spin down briefly to collect the reaction mixtures at the bottom of the tube.
7) Immediately incubate the tubes at 42°C for 2 minutes.
8) Add 1 μL of SuperScript II to the reaction mixtures. Mix thoroughly by flicking the tube and spin down briefly.
9) Incubate the tubes at 42°C for 1 hour.
10) Cool the samples at 4°C for 2 minutes.

11) Centrifuge the tube briefly (about 5 seconds) to collect the reaction mixtures at the bottom of the tube and immediately proceed to next step.

*3.3.2. Second Strand cDNA synthesis

1) Add 130 μL of Second-Strand Master Mix to each first-strand synthesis sample. Mix well by flicking the tube and spin down briefly.
2) Incubate the tubes at 16°C for 2 hours.
3) Add 2 μL of T4 DNA Polymerase. Mix well by flicking the tube and spin down briefly.
4) Incubate the tubes at 16°C for 5 minutes.
5) Add 10 μL of EDTA, 0.5M. Mix well by vortexing and spin down briefly.

*Double-stranded cDNA sample can be stored at -20°C.

*3.3.3. Cleanup of Double-Stranded cDNA

*All steps of this protocol should be performed at room temperature.
2) Add 600 μL of cDNA Binding Buffer to the double-stranded cDNA sample. Mix by vortexing for 3 minutes and spin down briefly.
*Check that the color of the mixture is yellow (same for color of cDNA Binding Buffer without the cDNA synthesis reaction).
*If the color of the mixture is orange or violet, add 10 μL of 3M sodium acetate, pH 5.0, and mix. Check that the color of the mixture is yellow.
3) Set the cDNA Cleanup Spin Column on a 2 mL Collection Tube
4) Mark the lids of spin column with the sample number to avoid the misidentification of samples.
5) Place 500 μL of the sample in the cDNA Cleanup Spin Column and close the lids of the column. Then centrifuge for 1 minute at 8,000 x g (10,500 rpm) at room temperature.
6) After centrifuge, discard flow-through and set again the cDNA Cleanup Spin Column on a 2 mL Collection Tube.
7) Load the remaining mixture (262 μL) in the spin column and close the lids of the column. Then centrifuge for 1 minute at 8,000 x g (10,500 rpm) at room temperature.
8) After centrifuge, discard flow-through and Collection Tube.
9) Transfer the spin column into a new 2 mL Collection Tube.
10) Pipet 700 μL of the cDNA Wash Buffer onto the spin column and close the lids of the column. Then centrifuge for 1 minute at 8,000 x g (10,500 rpm) at room temperature.
11) After centrifuge, discard flow-through and Collection Tube. Then, transfer spin column into a new 2 mL Collection Tube.
12) Open the cap of the spin column and centrifuge for 5 minutes at 10,000 x g (15,000 rpm) at room temperature.
13) After centrifuge, discard flow-through and Collection Tube.
14) Transfer spin column into a 1.5 mL Collection Tube.
15) Pipet 14 μL of cDNA Elution Buffer directly onto the spin column membrane.
16) Incubate for 1 minute at room temperature and centrifuge for 1 minute at 10,000 x g (15,000 rpm) to elute.

*The average volume of eluate is 12 μL.

(4. Synthesis of cRNA)

*4.1. Reagents and equipments

*4.1.1. Reagents

1) IVT labeling Kit (Affymetrix, cat.# 900449, store at - 20°C)

10X IVT Labeling Buffer
IVT Labeling Enzyme Mix
IVT Labeling NTP Mix
3'-Labeling Control (0.5 ug/uL)
RNase-free Water

2) GeneChip Sample Cleanup Module (Affymetrix, cat.# 900371)

IVT cRNA Binding Buffer
IVT cRNA Wash Buffer, 5 mL concentrate
RNase-free Water
5X Fragmentation Buffer
cDNA Binding Buffer
cDNA Wash Buffer, 6 mL concentrate
cDNA Elution Buffer

3) DEPC-Treated water (Ambion, cat.# 9920, store at room temperature)
4) Ethanol

4.1.2. Equipments

1) GeneChip Sample Cleanup Module (Affymetrix, cat.# 900371)

IVT cRNA Cleanup Spin Columns
1.5 mL Collection Tubes (for elution)
2 mL Collection Tubes
1.5 mL microtube, DNase/ RNase/pyrogen free

*Be careful not to confuse cRNA Cleanup Spin Columns with cDNA Cleanup Spin Columns.
2) Heat block
3) Microcentrifuge

*4.2. Preparation of Reagents

*4.2.1. IVT Reaction Mix

1) Prepare sufficient IVT Reaction Mix in a 1.5 mL tube. See the following table.
2) Mix by flicking the tube and spin down briefly after dissolving each solution.

*Prepare First-Strand Master Mix immediately before use and place on ice.

[Table 5]

| reagents | one sample | 24 samples | 48 samples |
|---|---|---|---|
| RNase-free Water | 10 μL | 260 μL | 500 μL |
| 10X IVT Labeling Buffer | 4 μL | 104 μL | 200 μL |
| IVT Labeling NTP Mix | 12 μL | 312 μL | 600 μL |
| IVT Labeling Enzyme Mix | 4 μL | 104 μL | 200 μL |
| total | 30μL | 780 μL | 1500 μL |

*4.2.2. IVT cRNA Wash Buffer (store at room temperature) 1) Add 20 mL of ethanol to obtain a working solution, and checkmark the box on the bottle label to avoid confusion.

*If necessary, dissolve a precipitate by warming the precipitate in a water bath at 30°C, and then place the buffer at room temperature.

*4.2.3. 80% Ethanol (store at room temperature) 1) Mix ethanol and DEPC-Treated water in a ratio of 4: 1, in an RNase/DNase free tube.

*4.3. IVT reaction procedure

*4.3.1. IVT reaction

1) Prepare 30 μL of IVT Reaction Mix in a 1.5 mL tube.
2) Add 10 μL of the double-stranded cDNA sample. Mix by flicking the tube and spin down briefly after dissolving each solution.
3) Incubate the tubes for 16 hours at 37°C with mixing at 300 rpm.
4) Store labeled cRNA at -80°C if not purifying immediately

*4.3.2. cRNA clean-up

1) Add 60 μL of RNase-free Water to the samples (40 μL) by vortexing for 3 seconds.
2) Add 350 μL IVT cRNA Binding Buffer to the mixture by vortexing for 3 seconds.

    *Exchange the tip if there is a possibility of contamination.

3) Add 250 μL 100% ethanol to the mixture, and mix well by pipetting.

    *Exchange the tip if there is a possibility of contamination.

4) Place the mixture (700 μL) on the IVT cRNA Cleanup Spin Column sitting in a 2 mL Collection Tube.
5) Centrifuge for 15 seconds at 8,000 x g (10,500 rpm).
6) Discard the flow-through and Collection Tube.
7) Transfer the spin column in a new 2 mL Collection Tube.
8) Pipet 500 μL IVT cRNA Wash Buffer onto the spin column.

    *Exchange the tip if there is a possibility of contamination.

9) Centrifuge for 15 seconds at 8,000 x g (10,500 rpm) and discard flow-through.
10) Pipet 500 μL 80% (v/v) ethanol onto the spin column. *Exchange the tip if there is a possibility of contamination.
11) Centrifuge for 15 seconds at 8,000 x g (10,500 rpm) and discard flow-through.
12) With open caps, centrifuge for 5 minutes at 8,000 x g (10,500 rpm) and discard flow-through and Collection Tube.
13) Transfer spin column into a new 1.5 mL Collection Tube, and pipet 11 μL of RNase-free Water directly onto the spin column membrane.

    *Exchange the tip if there is a possibility of contamination.

14) With closed caps, centrifuge for 1 minute 10,000 x g (15,000 rpm) to elute.
15) Pipet 10 μL of RNase-free Water directly onto the spin column membrane.

    *Exchange the tip if there is a possibility of contamination.

16) With closed caps, centrifuge for 1 minute 10,000 x g (15,000 rpm) to elute.
17) Determine the RNA concentration by measurement of O.D.

*4.3.3. Fragmentation of cRNA

1) Calculate the amount of cRNA and RNase-free water to adjust cRNA concentration to 20 μg/μL.

*In the case of whole blood sample, cRNA concentration is 10 μg/μL
*In TGP, in order to exclude the carry-over of total RNA, calculate the amount of cRNA according to the formula described below.

$$[\text{amount of cRNA}] = \text{RNAm} - \text{totalRNAi} * Y$$

RNAm: apparent amount of cRNA measured after IVT reaction
total RNAi[1)]: amount of total RNA of starting sample
Y: [amount of cDNA solution used in IVT reaction][2)] / [amount of cDNA solution][3)]

1) 5 μg in TGP, [2)] 10 μL in TGP, [3)] 12 μL in TGP

2) Dispense RNase-free Water and cRNA calculated in step 1) into 1.5 mL microtube.
3) Mark the sample number on the lid of the tube.

*Exchange the tip if there is a possibility of contamination.
*In TGP, barcode labels were placed on the residual cRNA samples and stored at -80°C.

4) Add 8 μL of the 5x fragmentation buffer.

*Exchange the tip if there is a possibility of contamination.

5) Mix by tapping the tube and spin down briefly.
6) For the quantification of the cRNA (before reaction), dispense 1 μL of these reaction mixtures into the 8-strip PCR reaction tubes for electrophoresis.
7) Incubate the tubes for 35 minutes at 94°C.
8) For the quantification of the cRNA (after reaction), dispense 1 μL of these fragmented reaction mixtures into the 8-strip PCR reaction tubes for electrophoresis.
9) [QC] Fragmentation pattern of cRNA by electrophoresis on a denaturing agarose

*Generally do not store fragmented cRNA samples. If necessary, store at -80°C.

*4.3.4. Brief quality check of the cRNA and their fragmentation

1) Criterion of the quality check of the cRNA

CRNA electrophoresis pattern does not look smeared entirely, bands around 1k bp are strongly stained (Lane 1). CRNA electrophoresis pattern does not look smeared entirely, bands around 1k bp are not strongly stained (Lane 3) .
The size distribution of cRNA is under 1k bp (Lane 7) . These samples are not suitable for the subsequent reactions, because they often result in showing high backgrounds.

2) Examination of the fragmented cRNA

*The size distribution of fragmented cRNA displays 35-200 bp, which is at the tip of gel electrophoresis (Lanes 2 and 4) .

(5. Hybridization)

*5.1. Reagents and equipments

*5.1.1. Reagents

1) GeneChip Eukaryotic Hybridization Control kit (Affymetrix, cat.#900454 or #900457)

20x Eukaryotic Hybridization Control
Control Oligonucleotide B2
*Control Oligonucleotide B2 (150 μL) were dispended into three tubes and stored at -20°C.

2) MES-free acid monohydrate (Sigma, cat.# M5287, 250g, stored at room temperature)
3) MES Sodium Salt (Sigma, cat.# M5057, 100g, stored at room temperature)
4) 5M NaCl (Ambion, cat.# 9760, 100 mL, stored at room temperature)
5) 0.5M EDTA (Sigma, cat.# E7889, 100mL, stored at room temperature)
6) 10% Tween20 (Surfact-Amps 20, PIERCE, cat.# 9005-64-5, 10 mL, stored at room temperature for up to 1 year)
7) 10 mg/mL Herring Sperm DNA (Promega, cat.# D1811, stored at -20°C for up to 1 year or at 4°C for up to 1 month)
8) 50 mg/mL Bovine Serum Albumin (Invitrogen, cat.# 15561-020, 3 mL, stored at -20°C for up to 1 year or at 4°C for up to 1 month)
9) DEPC-Treated water (Ambion, cat.# 9920, stored at room temperature))
10) 20x SSPE Buffer (Invitrogen, cat.# 15591-043, 1 L, stored at room temperature)

*5.1.2. Equipments

1) 50 mL tube, DNase/RNase/pyrogen free
2) 15 mL tube, DNase/RNase/pyrogen free
3) 1.5 mL microtube, DNase/RNase/pyrogen free
4) Bottle Top Vacuum Filter (Iwaki, cat.# 8024-045)
5) Storage Bottle (Iwaki, cat.# 8930-001)
6) Tough-Spots (Toho, 1/2 inch diameter, white, T-SPOTS-50)
7) Gene Chip Hybridization Oven 640 (Affymetrix)
8) Cool Incubator (Mitsubishi Electronic Engineering, CN-2 5A)

*5.2. Preparation of Reagents

5.2.1. 12x MES stock Buffer (protect from light, stored at 4°C for up to 3 months)

1) Mix and adjust volume to 1,000 mL. The pH should be between 6.5 and 6.7.
2) Filter through a 0.22 μM filter.
*This manipulation is for degassing and removal of dust rather than for sterilization.
*Do not autoclave MES buffer.
*If MES buffer turns yellow, do not use them.

[Table 6]

| reagents | 1000 mL |
|---|---|
| MES-free acid monohydrate | 70.4 g |
| MES Sodium Salt | 193.3 g |
| DEPC-Treated water | 800 mL |

*5.2.2. 2x Hybridization Buffer (protect from light, stored at 4°C for up to 1 month)
Final 1x concentration buffer is 100 mM MES, 1 M [Na+], 20 mM EDTA, 0.01% Tween-20.
1) Prepare in 50 mL tube, and mix by inverting.

[Table 7]

| reagents | 50 mL |
|---|---|
| 12x MES Stock Buffer | 8.3 mL |
| 5 M NaCl | 17.7 mL |
| 0.5 M EDTA | 4.0 mL |
| 10% Tween20 | 0.1 mL |
| DEPC-Treated water | 19.9 mL |
| Total | 50 mL |

*5.2.3. 1x Hybridization Buffer (protect from light, stored at 4°C for up to 1 month)

1) Prepare in 50 mL tube, and mix by inverting.

[Table 8]

| reagents | 50 mL |
|---|---|
| 2x Hybridization Buffer | 25 mL |
| DEPC-Treated water | 25 mL |
| Total | 50 mL |

*5.2.4. Wash A solution (Non-Stringent Wash Buffer, stored at room temperature for up to 1 month)

1) Prepare in beaker using measuring cylinder, and mix well by a stirrer.
2) Transfer the prepared Wash A solution to a clean dedicated bottle.

*This manipulation is for degassing and removal of dust rather than for sterilization.

[Table 9]

| reagents | 1000 mL |
|---|---|
| 20x SSPE | 300 mL |
| 10% Tween-20 | 1 mL |
| DEPC-Treated water | 699 mL |
| total | 1000 mL |

*4.1. Hybridization

1) Equilibrate GeneChip to room temperature 30 minutes before use to prevent dew condensation and cracking of the rubber septa.

*Check the GeneChip (glass surface scratch etc.)

2) Transfer 30 μL of each fragmented and labeled cRNA samples to the 1.5 mL microtubes.
3) Prepare sufficient Hybridization Mix in a 15 mL tube. See the following.

[Table 10]

| reagents | one sample | 24 samples | 48 samples |
|---|---|---|---|
| Control Oligonucleotide B2 | 5 μL | 125 μL | 245 μL |
| 20x Eukaryotic Hybrydization Control (heated to 65°C for 5 minutes) | 15 μL | 375 μL | 735 μL |
| 10 mg/mL Herring Sperm DNA | 3 μL | 75 μL | 147 μL |
| 50 mg/mL Bovine Serum Albumin | 3 μL | 75 μL | 147 μL |
| 2x Hybridization Buffer | 150 μL | 3.75 mL | 7.35 mL |
| DMSO | 30 μL | 0.75 mL | 1.47 mL |
| DEPC-Treated water | 64 μL | 1.6 mL | 3.136 mL |
| total | 270 μL | 6.75 mL | 13.23 mL |

4) Add 270 μL of the Hybridization Mix to the fragmented and labeled cRNA samples. Mix by flicking the tube and spin down briefly.

*Exchange the tip if there is a possibility of contamination.

5) Incubate the tubes for 5 minutes at 99°C with heat block.
6) Incubate the tubes for 5 minutes at 45°C with heat block.

7) Centrifuge for 5 minutes at 10,000 x g (15,000 rpm) at room temperature to collect any insoluble material from the hybridization cocktail.
8) While centrifuging the hybridization cocktail, wet the array with 200 μL of 1x Hybridization Mix.

*Vent the GeneChip through the septa in the top right by inserting a clean, unused RAININ pipette tip.
*Fill 1x Hybridization Mix (pre-hybridization buffer) through the septa in the bottom left with a clean, unused RAININ pipette tip.

9) Incubate the GeneChip for 10 minutes at 45°C with a 60 rpm rotation using Hybridization Oven (pre-hybridization).
10) Check for a buffer leak from the GeneChip.
11) Vent the array with a clean, unused RAININ pipette tip and remove the 1x Hybridization Mix from the GeneChip with a clean, unused RAININ pipette tip.
12) Refill 200 μL of the clarified hybridization cocktail, avoiding any insoluble matter at the bottom of the tube with a clean, unused RAININ pipette tip.
13) Place 1/2 inch Tough Spots on the septa to prevent leakage.
14) Store microtubes after taking out the supernatants (appx. 100 μL of hybridization cocktail remain) at -80°C.

*In TGP, place the sample ID barcode on the tube.
*Do not touch the glass surface of the GeneChip.

15) Place GeneChip into the Hybridization Oven and incubate for 18 hours at 45°C with a 60 rpm rotation (hybridization).

*To avoid applying stress on the motor, load probe arrays in a balanced configuration around the axis.

16) After hybridization, remove the hybridization cocktail from the GeneChip with a clean, unused RAININ pipette tip.

*In TGP, this cocktail is stored at -80°C as the hybridization cocktail for rehybridization, in addition to the remaining hybridization cocktail.

17) Fill the GeneChip completely with the appropriate volume of Wash Buffer A (appx. 260 μL)

*In TGP, place the GeneChip in a cool incubator until the next step.

(Standard protocol 3)

(Quality control (QC) and final data inclusion to the database)

All gene expression in Adjuvant Database Project passed quality control (QC). Data acquisition was performed in-house with GeneChip® Scanner 3000 7G (Affymetrix). The acquired data was further analyzed for the background signal, the corner signal, the number of the presence/absence calls, and the expression values of housekeeping genes. Intra- and inter-group reproducibilities were then checked by scatter plot analysis. X-axis and Y-axis indicate the gene expression value of each gene from two different samples of the same (intra-group) or different (inter-group) adjuvant treated mouse groups. Red dots indicate genes exhibiting high coefficient of correlation (CV).

(Representative QC examples)

The following are 5 types of representative QC examples observed in this project.

1) Failed QC examples with unknown reason (re-assayed with spare samples)

[Chemical Formula 1]

a) failed the QC

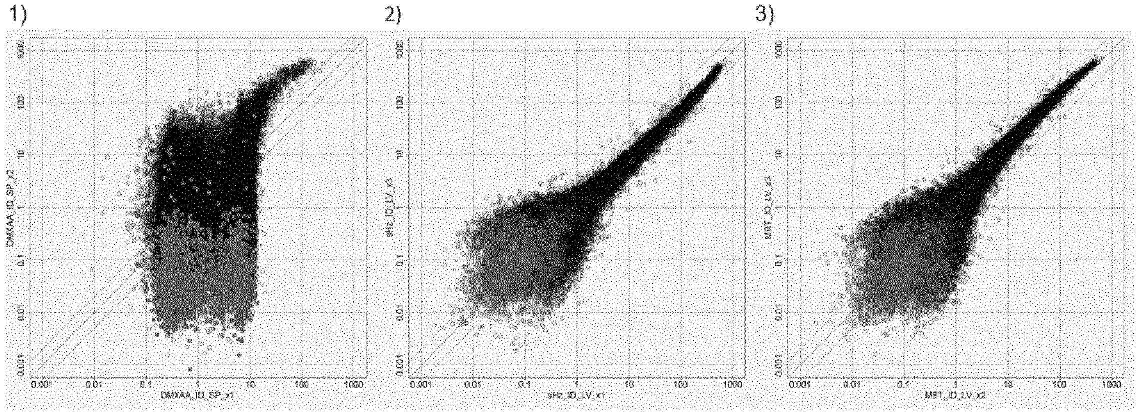


DMXAA_ID_SP_x1 (a-1) exhibited a severely distorted scatter plot for unknown reasons. sHz_ID_LV_x3 (a-2) and MBT_ID_LV_x3 (a-3) exhibited an unusually curved scatter plot for another sample treated with the same adjuvant from the same organ. Other (spare) tissue fragments, which were simultaneously sampled from the same sample but were stored separately as spares, were used for re-assay. If data from the spare fragment also exhibited a distorted scatter plot, the sample was excluded from subsequent analysis. In these three cases, re-assay data for DMXAA_ID_SP_x1 sHz_ID_LV_x3, and MBT_ID_LV_x3 passed QC and were used for subsequent analysis.

2) Failed QC sample with severe contamination of other tissues (excluded from subsequent analysis)

The scatter plot between ADX_ID_LN_c1 and ADX_ID_LN_c3 exhibited one sided placement. CV analysis revealed that the ADX_ID_LN_c1 sample was heavily contaminated with adipose tissue surrounding the inguinal lymph nodes. Even with a CV filter, adipose tissue derived genes could not be eliminated (indicated by black dots broadly distributed from the center line). Therefore, these samples were completely excluded from subsequent analysis to avoid the significant effect due to such contamination.

3) Passed QC with unavoidable weak contamination by other tissues

ADX_IP_SP_c2 (c-1) and cdiGMP_ID_SP_c1 (c-2) exhibited contamination by pancreas on a normal scatter plot in spleen (e-2). Since the pancreas and the spleen are proximately located, it was technically challenging to completely remove pancreatic tissues from the spleen even with careful cleaning. For this reason, a CV filter was applied to remove the contamination derived genes (generally exhibiting high CV) by a data processing method.

4) Passed QC with different magnitude of host responses

While samples exhibiting a broad distribution (d-1, 2) was able to be detected due to individual differences among mice, this reflects the difference in the magnitude of host responses after adjuvant administration.

5) Passed QC with no problem

(Synthetic virtual control)

To obtain at least three control samples for statistical analysis on the samples in experiment 3, "03PBS_LN_c1" was replaced with synthetic virtual control "03PBS_LN_c1v". The synthetic virtual control "03PBS_LN_clv" was created using the average of individual gene expression values among pooled control samples from a total of 10 experiments. For each probe set, average values were calculated after removing probes with the highest and lowest values from the pooled control samples.

(Sample exclusion based on further evaluation of post QC dataset)

Two other samples K3SPG_IP_LV _x2 and K3SPG_IP_SP_x2 (both samples from the #2 mouse in Experiment 2) were removed for exhibiting gene responses that were too strong compared to other two mice (not shown). For ADX_ID_LN, K3SPG_IP_LV, and K3SPG_IP_SP, only two treated samples were used for subsequent analysis. Other

samples were analyzed with three samples.

(CV filtering)

During QC analysis on GeneChip data, some samples contained weak but a substantial level of contamination from genes derived from other tissues (for example, red in the above QC scatter plots). It was technically difficult to completely remove the micro-contamination (QC example 3) by sampling organs after careful cleaning. Therefore, a coefficient of variation (CV) filter was developed to reduce microcontamination derived genes from the target organ gene analysis. Each gene probe's baseline variations were calculated using GeneChip data from a total of 33 buffer injected control mice (not shown). The origin of high CV gene probes in each organ was further analyzed by utilizing the Gene Expression Barcode 3.0 (http://barcode.luhs.org/) database. Analysis with the Barcode 3.0 revealed that these high CV gene probes were mainly from the neuronal tissue in LV (not shown), pancreas tissue in SP (not shown), and adipose tissues in LN (not shown). To remove the effect of these microcontaminations, the data was filtered with CV value < 1, and the filtered data (43200 out of 45037 probes) were used for further analysis.

(Administration of adjuvant and sampling)

Detailed information on adjuvants used in this Example is further described. C57BL/6 mice (male, 5-week old, C57BL/6JJc1) were purchased from CLEA Japan and acclimated for at least one week. Each adjuvant was prepared as described in standard procedure 1. The dose/volume (Table 11) indicated for each adjuvant was administered (intradermally; i.d.) mainly to the base of the tail of the mice (n = 3 for each group). Five types of adjuvants (ADX, ALM, bCD, K3, and K3SPG) were i.p. administered. ENDCN was i.n. (intranasally) administered. Since a genetic profile with very large variability and low reproducibility was obtained in a preliminary experiment from intramuscular administration, this Example did not use intramuscular administration. However, intramuscular injection can also be used if conditions are adjusted. The dose of each adjuvant was selected to induce excellent adjuvant function without severe reactogenicity in the mice based on the inventors' experiment (ALM, K3 and K3SPG, bCD, cdiGMP and cGAMP, D35, DMXAA, FK565, sHZ), previous report (AddaVax, ADX, ENDCN, PolyIC, Pam3CSK4, MPLA, MALP2s, R848), or the following common protocol (1:1 mixture of FCA and ISA51VG) (details described below).

While the selected dose of MBT was the same as the dose of FK565, experiment was not conducted in this regard. This was determined based on preliminary data (data not shown) for FK565 and other NOD ligands. Among the total of 21 different types of adjuvants, 3 to 5 different types of adjuvants were used in one experiment, and a suitable control buffer group was used (for most adjuvants, PBS; for ENDCN, Tris-HCl; and for DMXAA, MALP2s, MPLA, and R848, 5% DM standard procedure BS). In this study, a total of 10 independent experiments were conducted (see standard procedure 1, e.g., Table 11).

[Table 11]

| Exp. No. | Test substance (abbreviation) | Route | Time (hrs) | Dose (/body) | Conc. | Dose volume (/body) | # of mice | Group No. | LV | SP | LN | Kit |
|---|---|---|---|---|---|---|---|---|---|---|---|---|
| Exp. 1 | PBS | i.p. | 6 | 0 mg | 0 mg/ mL | 0.1 mL | 3 | 1 | ✓ | ✓ | | E |
| | ADX | i.p. | 6 | 1 mg | 10 mg/ mL | 0.1 mL | 3 | 2 | ✓ | ✓ | | E |
| Exp. 2 | PBS | i.p. | 6 | 0 mg | 0 mg/ mL | 0.1 mL | 3 | 1 | ✓ | ✓ | | E |
| | ALM | i.p. | 6 | 1 mg | 10 mg/ mL | 0.1 mL | 3 | 2 | ✓ | ✓ | | E |
| | K3 | i.p. | 6 | 0.01 mg | 0.1 mg/ mL | 0.1 mL | 3 | 3 | ✓ | ✓ | | E |
| | K3SPG | i.p. | 6 | 0.01 mg | 0.1 mg/ mL | 0.1 mL | 3 | 4 | ✓*1 | ✓*1 | | E |
| Exp. 3 | PBS | i.d. | 6 | 0 mg | 0 mg/ mL | 0.1 mL | 3 | 1 | ✓ | ✓ | ✓*2 | E |
| | ADX | i.d. | 6 | 1 mg | 10 mg/ mL | 0.1 mL | 3 | 2 | ✓ | ✓ | ✓*3 | E |
| | ALM | i.d. | 6 | 1 mg | 10 mg/ mL | 0.1 mL | 3 | 3 | ✓ | ✓ | ✓ | E |
| | K3 | i.d. | 6 | 0.01 mg | 0.1 mg/ mL | 0.1 mL | 3 | 4 | ✓ | ✓ | ✓ | E |
| Exp. 4 | PBS | i.p. | 6 | | 0% | 0.2 mL | 3 | 1 | ✓ | ✓ | | E |
| | bCD | i.p. | 6 | | 30% | 0.2 mL | 3 | 2 | ✓ | ✓ | | E |
| | Tris-HCl | i.n. | 6 | | 0% | 0.005 mL | 3 | 3 | ✓ | ✓ | | E |
| | ENDCN | i.n. | 6 | | 2% | 0.005 mL | 3 | 4 | ✓ | ✓ | | E |
| Exp. 5*4 | PBS | i.d. | 6 | 0 mg | 0 mg/ mL | 0.1 mL | 3 | 1 | ✓ | ✓ | ✓ | E |
| | D35 | i.d. | 6 | 0.01 mg | 0.1 mg/ mL | 0.1 mL | 3 | 2 | ✓ | ✓ | ✓ | E |
| | K3SPG | i.d. | 6 | 0.01 mg | 0.1 mg/ mL | 0.1 mL | 3 | 3 | ✓ | ✓ | ✓ | E |
| | bCD | i.d. | 6 | | 30% | 0.2 mL | 3 | 4 | ✓ | ✓ | ✓ | E |
| Exp. 6 | PBS | i.d. | 6 | | 0% | 0.1 mL | 3 | 1 | ✓ | ✓ | ✓ | E |
| | FCA | i.d. | 6 | | 50% | 0.1 mL | 3 | 4 | ✓ | ✓ | ✓ | E |
| Exp. 7 | PBS | i.d. | 6 | 0 mg | 0 mg/ mL | 0.1 mL | 3 | 1 | ✓ | ✓ | ✓ | P |
| | sHZ | i.d. | 6 | 0.2 mg | 2 mg/ mL | 0.1 mL | 3 | 2 | ✓ | ✓ | ✓ | P |
| | FK565 | i.d. | 6 | 0.01 mg | 0.1 mg/ mL | 0.1 mL | 3 | 3 | ✓ | ✓ | ✓ | P |
| | MBT | i.d. | 6 | 0.01 mg | 0.1 mg/ mL | 0.1 mL | 3 | 4 | ✓ | ✓ | ✓ | P |
| Exp. 8 | PBS | i.d. | 6 | 0 mg | 0 mg/ mL | 0.1 mL | 3 | 1 | ✓ | ✓ | ✓ | P |
| | PolyIC | i.d. | 6 | 0.1 mg | 1 mg/ mL | 0.1 mL | 3 | 2 | ✓ | ✓ | ✓ | P |
| | Pam3CSK4 | i.d. | 6 | 0.01 mg | 0.1 mg/ mL | 0.1 mL | 3 | 3 | ✓ | ✓ | ✓ | P |
| | cdiGMP | i.d. | 6 | 0.01 mg | 0.1 mg/ mL | 0.1 mL | 3 | 4 | ✓ | ✓ | ✓ | P |
| Exp. 9 | PBS | i.d. | 6 | | 0 | 0.1 mL | 3 | 1 | ✓ | ✓ | ✓ | P |
| | Addavax | i.d. | 6 | | 50% | 0.1 mL | 3 | 2 | ✓ | ✓ | ✓ | P |
| | ISA51VG | i.d. | 6 | | 50% | 0.1 mL | 3 | 3 | ✓ | ✓ | ✓ | P |
| | cGAMP | i.d. | 6 | 0.01 mg | 0.1 mg/ mL | 0.1 mL | 3 | 4 | ✓ | ✓ | ✓ | P |
| Exp. 10*4 | PBS DMSO5% | i.d. | 6 | 0 mg | 0 mg/ mL | 0.1 mL | 3 | 1 | ✓ | ✓ | ✓ | P |
| | MPLA | i.d. | 6 | 0.01 mg | 0.1 mg/ mL | 0.1 mL | 3 | 2 | ✓ | ✓ | ✓ | P |
| | MALP2s | i.d. | 6 | 0.01 mg | 0.1 mg/ mL | 0.1 mL | 3 | 3 | ✓ | ✓ | ✓ | P |
| | R848 | i.d. | 6 | 0.1 mg | 1 mg/ mL | 0.1 mL | 3 | 4 | ✓ | ✓ | ✓ | P |
| | DMXAA | i.d. | 6 | 0.1 mg | 1 mg/ mL | 0.1 mL | 3 | 5 | ✓ | ✓ | ✓ | P |

In a preliminary experiment, FCA and ALM were tested at different points up to 72 hours. In the tested dose range, changes in gene expression reached a peak at the 6 hour mark in many cases, and majority of changes subsided at the 24 hours mark. With a preliminary experiment, changes in gene expression in an organ was studied after adjuvant administration, consistently at 6 hours after administration, revealing that the changes generally returned to a normal level in mice and rats after 24 hours. For this reason, the 6 hours mark after administration was chosen for sampling. At 6 hours after adjuvant administration, LV, SP, and LN (both sides) were removed to study the gene expression thereof by using Affimetrix Genechip microarray system (Affymetrix). The sampled organs were each placed in a tube containing RNAlater. The tube was maintained at 4°C overnight, and stored at -80°C until use. Blood was also collected after 6 hours from adjuvant administration for general hematological testing. About 200 μL of blood was taken from the retro-orbital venous plexus with a non-heparinized capillary tube and placed into a 1.5 mL tube comprising 2 μL of 10% EDTA-2K for hematological testing. The hematological cell count was found using VetScan HMII (Abaxis). 50 μl of EDTA-2K blood sample was diluted by adding 250 μl of saline. Measurements were taken with VetScan HMII by following the instruction.

(Data acquisition and quality control)

Microarray data was acquired in accordance with Igarashi et al (Igarashi, Y. et al. Open TG-GATEs: a large-scale toxicogenomics database. Nucleic acids research 43, D921-927 (2015)). Briefly, RLT buffer (Qiagen GmbH., Germany) was added to a tissue block stored in RNAlater, and homogenized by shaking with 5 mm diameter zirconium beads (ASONE Corporation, Japan) for 5 minutes at 20 Hz in a Mixer Mill 300 (Qiagen, Germany). Total RNA was isolated from LV, SP, and LN by using Trizol LS (Life Technologies, CA) and RNeasy Mini Kit (Qiagen) in accordance with the instruction of the manufacturer. GeneChip® 3'IVT Express Reagent Kit or GeneChip® 3'IVT PLUS Reagent Kit was used to prepare a sample. The gene expression profile was determined using GeneChip® Mouse Genome 2.0 Array (Affymetrix, CA) in accordance with the instruction of the manufacturer. The washing step and the staining step were

carried out using GeneChip® Hybridization, Wash, and Stain kit on a fluidics station 450 (Affymetrix). The GeneChip® array was scanned with GeneChip® Scanner 3000 7G (Affymetrix). The resulting digital image files (DAT file) were converted to CEL files using Affymetrix® GeneChip® Command Console® software (standard procedure 2).

Ultimately, 330 (99 controls and 231 treatment groups) GeneChip data files and hematological parameters were obtained from 10 separately conducted experiments (Table 11). Notably, one PBS control and one ADX_ID-treated LN sample in Experiment 3 had to be removed because of the large amount of adipose tissue-derived gene expression (see standard procedure 3). Consequently, the ADX_ID-treated LN sample data was excluded and the PBS control sample was replaced with the synthetic virtual control data (standard procedure 3) for further analysis on the Experiment 3 dataset. Furthermore, the K3SPG_IP-treated #2 mouse-derived LV and SP samples were excluded from the analysis, because i.p. K3-SPG injection was not performed properly (standard procedure 3). Any gene probes that exhibited high coefficients of variation in the control buffer-treated mice was also excluded to reduce the overall experimental condition-dependent factors including microcontamination of non-target tissue (standard procedure 3).

(Determination of the presence or absence of gene expression (Customized PA call))

The presence or absence (PA) call in MAS5.0 was customized as follows. The normalized MAS5.0 expression data from each of the two groups of three mice that were treated with a single adjuvant or its appropriate vehicle control were averaged, and the average expression ratio for each group was calculated. In this process, the MASS.0 PA calls (customized PA call) were integrated as follows. When the PA calls from the three control samples were ["P", "P", and "A"], "P" was chosen as their dominant PA calls (over half were "P"). The same strategy was applied to the three adjuvant-treated samples. When the expression ratio of each gene was > 1.0, the customized PA call was determined by the dominant call of the treated group. When the expression ratio was < 1.0, the call was determined by the dominant call of the vehicle-control group. The resultant customized PA call was processed as "P" is "1", and "A" is "0", i.e., when the ratio is greater than 1 and the PA calls of the treated samples were "P," "P," and "A," the customized PA call of this gene set was "1". In the case of a two sample analysis (ADX_ID_LN, K3SPG_IP_LV, and K3SPG_IP_SP), ["P", "A"] was processed as "A".

(Significantly differentially expressed genes (genes with a significant change in expression = sDEGs or significant SEGs))

sDEGs were defined as statistically significant changes (up- or down-regulation) satisfying all of the following conditions: an average fold change (FC) of > 1.5 or < 0.667, an associated t-test p-value of < 0.01 with no multiple test corrections, and a customized PA call of 1.

(Biological themes enrichment analysis with TargetMine)

A defined sets of genes were selected according to specified criteria (e.g., FC, PA-call, threshold values). Their functional enrichment values were then obtained from TargetMine (Chen, Y.A., Tripathi, L.P. & Mizuguchi, K. PloS one 6, e17844 (2011)) (http://targetmine.mizuguchilab.org/) using the API interface. The following resources were used throughout the analysis via TargetMine Interface (GO, GOSlim, Integrated Pathway, KEGG, Reactome, and the NCI pathway). The Holm-Bonferroni method was used for multiple testing corrections.

(Biological annotation analysis of individual adjuvants using whole organ transcriptomics (Figure **10** and Table 12))

[Table 12-1]

| Group | Probe Index | Gene Symbol | Modules | (10)R848.ID.LV.x3 | (10)R848.ID.LV.x1 | (10)R848.ID.LV.x2 | (8)Poly_IC.ID.LV.x3 | (8)Poly_IC.ID.LV.x1 | (8)Poly_IC.ID.LV.x2 | (10)DMXAA.ID.LV.x3 | (10)DMXAA.ID.LV.x1 | (10)DMXAA.ID.LV.x2 | (9)cGAMP.ID.LV.x3 | (9)cGAMP.ID.LV.x1 | (9)cGAMP.ID.LV.x2 | (8)cdiGMP.ID.LV.x1 | (8)cdiGMP.ID.LV.x2 | (8)cdiGMP.ID.LV.x3 | (9)AddaVax.ID.LV.x2 | (9)AddaVax.ID.LV.x1 | (9)AddaVax.ID.LV.x3 | (9)ISA51VG.ID.LV.x1 | (9)ISA51VG.ID.LV.x2 | (9)ISA51VG.ID.LV.x3 | (3)K3.ID.LV.x1 | (3)ADX.ID.LV.x1 | (3)K3.ID.LV.x2 | (3)ALM.ID.LV.x2 | (3)ALM.ID.LV.x1 | (3)ALM.ID.LV.x3 | (10)MPLA.ID.LV.x2 |
|---|---|---|---|---|---|---|---|---|---|---|---|---|---|---|---|---|---|---|---|---|---|---|---|---|---|---|---|---|---|---|---|
| G1 | 1449025_at | Ifit3 | 20 | 1.49 | 2.19 | 1.76 | 1.95 | 3.24 | 3.25 | 0.74 | 1.39 | 1.3 | 2.06 | 2.13 | 1.95 | 1.7 | 1.75 | 2.34 | -0.5 | -0.5 | -0.5 | -0.5 | -0.5 | -0.5 | -0.5 | -0.5 | -0.5 | -0.5 | -0.5 | -0.5 | -0.5 |
| G1 | 1456890_at | Ddx58 | 20 | 0.95 | 2.42 | 1.34 | 1.87 | 3.02 | 2.77 | 1.56 | 1.67 | 1.93 | 2.38 | 2.26 | 2.28 | 1.2 | 1.45 | 2.07 | -0.5 | -0.7 | -0.6 | -0.6 | -0.6 | -0.6 | -0.4 | -0.5 | -0.4 | -0.4 | -0.3 | -0.5 | -0.1 |
| G1 | 1450783_at | Ifit1 | 20 | 0.63 | 1.54 | 1.14 | 1.28 | 2.54 | 2.35 | 1.04 | 2.21 | 1.54 | 2.63 | 2.75 | 2.63 | 2.21 | 2.28 | 2.4 | -0.5 | -0.5 | -0.5 | -0.5 | -0.5 | -0.5 | -0.5 | -0.5 | -0.5 | -0.5 | -0.5 | -0.5 | -0.5 |
| G1 | 1450034_at | Stat1 | 20 | 1.52 | 2.37 | 1.92 | 1.94 | 2.89 | 3.04 | 1.16 | 1.53 | 1.96 | 2.26 | 2.46 | 1.97 | 0.88 | 1.02 | 2.07 | -0.5 | -0.6 | -0.6 | -0.6 | -0.6 | -0.6 | -0.4 | -0.5 | -0.5 | -0.5 | -0.5 | -0.6 | -0.5 |
| G1 | 1436562_at | Ddx58 | 20 | 1.6 | 2.64 | 2.33 | 1.64 | 2.92 | 2.82 | 1.31 | 2.07 | 1.54 | 2.6 | 2.04 | 2.13 | 0.94 | 0.88 | 1.49 | -0.5 | -0.6 | -0.4 | -0.5 | -0.6 | -0.5 | -0.3 | -0.6 | -0.4 | -0.3 | -0.6 | -0.3 | -0.4 |
| G1 | 1437503_a_at | Shisa5 | 20 | 2.49 | 3.09 | 2.43 | 1.52 | 2.49 | 2.66 | 1.72 | 2.12 | 1.62 | 2.09 | 1.57 | 1.63 | 1.05 | 1.12 | 1.32 | -0.6 | -0.7 | -0.7 | -0.6 | -0.8 | -0.7 | -0.5 | -0.5 | -0.3 | -0.5 | -0.6 | -0.3 | -0.3 |
| G1 | 1418825_at | Irgm1 | 20 | 2.02 | 2.87 | 2.12 | 1.84 | 2.78 | 2.84 | 0.79 | 1.39 | 1.33 | 2.14 | 2.25 | 2.09 | 1.33 | 1.29 | 1.81 | -0.5 | -0.6 | -0.6 | -0.6 | -0.7 | -0.6 | -0.5 | -0.6 | -0.6 | -0.6 | -0.5 | -0.5 | -0.5 |
| G1 | 1426906_at | Mndal | 20 | 1.21 | 1.65 | 1.42 | 2.37 | 3.14 | 2.4 | 0.73 | 1.42 | 1.19 | 2.19 | 2.08 | 2.11 | 1.77 | 2.45 | 2.76 | -0.4 | -0.6 | -0.6 | -0.5 | -0.7 | -0.6 | -0.3 | -0.3 | -0.3 | -0.4 | -0.4 | -0.3 | -0.4 |
| G1 | 1450033_a_at | Stat1 | 20 | 1.05 | 1.4 | 1.2 | 1.92 | 2.73 | 2.67 | 1.15 | 1.5 | 1.73 | 2.85 | 3.11 | 2.52 | 1.49 | 1.44 | 2.11 | -0.5 | -0.6 | -0.6 | -0.6 | -0.6 | -0.6 | -0.5 | -0.5 | -0.5 | -0.5 | -0.5 | -0.5 | -0.5 |
| G1 | 1436058_at | Rsad2 | 20 | 0.67 | 1.97 | 1.77 | 1.97 | 3 | 3.3 | 0.44 | 1.56 | 1.25 | 1.75 | 2.08 | 1.89 | 2.39 | 2.41 | 2.39 | -0.5 | -0.5 | -0.5 | -0.5 | -0.5 | -0.5 | -0.5 | -0.5 | -0.5 | -0.4 | -0.4 | -0.5 | -0.5 |
| G1 | 1423986_a_at | Shisa5 | 20 | 2.23 | 2.93 | 2.32 | 1.61 | 2.5 | 2.99 | 1.42 | 2.03 | 1.81 | 2.36 | 1.7 | 1.43 | 0.72 | 1.07 | 1.57 | -0.2 | -0.7 | -0.6 | -0.5 | -0.6 | -0.6 | -0.5 | -0.6 | -0.5 | -0.6 | -0.5 | -0.5 | -0.4 |
| G1 | 1434268_at | Adar | 20 | 1 | 2.77 | 1.8 | 1.28 | 2.82 | 2.17 | 0.92 | 2.15 | 1.52 | 1.8 | 2.22 | 1.71 | 1.7 | 1.83 | 2.95 | -0.3 | -0.7 | -0.7 | -0.8 | -0.9 | -0.7 | -0.6 | -0.6 | -0.5 | -0.6 | -0.5 | -0.6 | -0.3 |
| G1 | 1425405_a_at | Adar | 20 | 0.96 | 1.63 | 1.17 | 1.58 | 3.45 | 2.75 | 0.93 | 2.03 | 1.5 | 2.37 | 2.95 | 2.38 | 1.58 | 1.67 | 1.66 | -0.3 | -0.4 | -0.6 | -0.5 | -0.5 | -0.6 | -0.3 | -0.4 | -0.7 | -0.5 | -0.5 | -0.4 | -0.4 |
| G1 | 1435526_at | Tor1aip2 | 20 | 1.22 | 3.12 | 2.18 | 1.58 | 2.88 | 2.62 | 1.01 | 2.23 | 1.48 | 2.32 | 2.32 | 2.28 | 0.94 | 0.83 | 1.54 | -0.4 | -0.4 | -0.3 | -0.4 | -0.5 | -0.4 | -0.4 | -0.3 | -0.5 | -0.5 | -0.4 | -0.5 | -0.1 |
| G1 | 1431591_s_at | Gm9706///Isg15 | 20 | 1.95 | 2.55 | 2.1 | 2.13 | 3.28 | 3.62 | 0.8 | 1.28 | 1.39 | 1.73 | 2.13 | 1.62 | 1.01 | 0.94 | 2.02 | -0.5 | -0.5 | -0.5 | -0.5 | -0.5 | -0.5 | -0.5 | -0.5 | -0.5 | -0.5 | -0.5 | -0.5 | -0.5 |
| G1 | 1455500_at | Rnf213 | 20 | 0.72 | 1.29 | 1.03 | 1.47 | 2.64 | 3.01 | 0.97 | 1.79 | 1.2 | 2.18 | 2.58 | 2.34 | 2.07 | 2.08 | 3.16 | -0.5 | -0.6 | -0.6 | -0.6 | -0.6 | -0.6 | -0.5 | -0.5 | -0.5 | -0.5 | -0.5 | -0.5 | -0.5 |
| G1 | 1418115_s_at | Tor1aip2 | 20 | 0.54 | 1.53 | 1.08 | 1.13 | 2.38 | 2.68 | 0.98 | 2.45 | 1.52 | 2.71 | 2.9 | 2.68 | 1.81 | 1.67 | 2.42 | -0.5 | -0.9 | -0.6 | -0.7 | -0.8 | -0.5 | -0.3 | -0.4 | -0.3 | -0.4 | -0.3 | -0.4 | -0.4 |
| G1 | 1418116_at | Tor1aip2 | 20 | 0.89 | 1.45 | 1.22 | 0.96 | 2.73 | 2.94 | 1.37 | 2.19 | 1.92 | 2.68 | 2.41 | 2.58 | 1.58 | 1.33 | 2.19 | -0.4 | -0.7 | -0.6 | -0.5 | -0.8 | -0.5 | -0.4 | -0.3 | -0.5 | -0.5 | -0.5 | -0.6 | -0.3 |
| G1 | 1426970_a_at | Uba7 | 20 | 2.27 | 2.64 | 2.42 | 1.71 | 3 | 3.13 | 1.21 | 1.81 | 1.59 | 1.91 | 2.06 | 1.28 | 0.53 | 1.46 | 1.41 | -0.5 | -0.6 | -0.5 | -0.5 | -0.4 | -0.3 | -0.2 | -0.6 | -0.3 | -0.6 | -0.5 | -0.6 | -0.2 |
| G1 | 1421323_a_at | G3bp2 | 20 | 1.04 | 2.13 | 1.21 | 1.31 | 2.83 | 2.85 | 0.71 | 1.32 | 1.22 | 2.43 | 3.19 | 2.6 | 1.74 | 1.5 | 2.34 | -0.5 | -0.6 | -0.6 | -0.6 | -0.5 | -0.6 | -0.4 | -0.4 | -0.5 | -0.3 | -0.6 | -0.6 | -0.6 |
| G1 | 1422005_at | Eif2ak2 | 20 | 1.09 | 2.43 | 1.56 | 1.83 | 2.89 | 2.87 | 0.68 | 1.36 | 1.3 | 2.73 | 3.11 | 2.21 | 1.02 | 1.38 | 1.93 | -0.4 | -0.6 | -0.6 | -0.6 | -0.6 | -0.6 | -0.5 | -0.6 | -0.5 | -0.6 | -0.5 | -0.4 | -0.4 |
| G1 | 1448757_at | Pml | 20 | 0.66 | 1.77 | 1.53 | 2 | 3.58 | 3.3 | 0.83 | 1.62 | 1.35 | 2.49 | 2.13 | 2.02 | 1.41 | 1.57 | 2.16 | -0.4 | -0.5 | -0.5 | -0.3 | -0.5 | -0.4 | -0.4 | -0.5 | -0.6 | -0.2 | -0.4 | -0.4 | -0.3 |
| G1 | 1417244_a_at | Irf7 | 20 | 2.37 | 2.27 | 2.39 | 2.38 | 3.53 | 3.38 | 1 | 1.05 | 1.18 | 1.62 | 1.6 | 1.62 | 0.85 | 1.21 | 1.92 | -0.5 | -0.5 | -0.6 | -0.5 | -0.7 | -0.6 | -0.4 | -0.5 | -0.4 | -0.4 | -0.4 | -0.5 | -0.5 |
| G1 | 1426276_at | Ifih1 | 20 | 2.36 | 3.25 | 2.46 | 2.12 | 3.06 | 3.34 | 0.78 | 1.25 | 1.09 | 1.66 | 1.73 | 1.62 | 0.89 | 1.09 | 1.65 | -0.5 | -0.5 | -0.4 | -0.5 | -0.6 | -0.5 | -0.4 | -0.5 | -0.4 | -0.5 | -0.5 | -0.5 | -0.4 |
| G1 | 1449875_s_at | H2-T10///H2-T22///H2-T9 | 20 | 1.77 | 2.05 | 2.01 | 2.21 | 2.89 | 3.28 | 0.51 | 1.27 | 1.64 | 2.45 | 2.29 | 1.8 | 1.07 | 1.04 | 2.06 | -0.5 | -0.6 | -0.6 | -0.5 | -0.6 | -0.4 | -0.3 | -0.4 | -0.5 | -0.6 | -0.6 | -0.5 | -0.3 |
| G1 | 1437432_a_at | Trim12a | 20 | 1.68 | 2.13 | 1.94 | 2.9 | 3.39 | 3.59 | 1.11 | 1.82 | 1.56 | 1.61 | 2.07 | 1.46 | 0.73 | 0.83 | 1.46 | -0.5 | -0.5 | -0.5 | -0.5 | -0.6 | -0.5 | -0.6 | -0.4 | -0.5 | -0.4 | -0.5 | -0.3 | -0.6 |
| G1 | 1456103_at | Pml | 20 | 1.29 | 3.66 | 1.62 | 2.12 | 3.26 | 2.5 | 0.34 | 2.18 | 1.34 | 1.62 | 1.62 | 1.48 | 1.31 | 1.3 | 2.58 | -0.6 | -0.6 | -0.5 | -0.5 | -0.6 | -0.5 | -0.5 | -0.4 | -0.6 | -0.4 | -0.4 | -0.5 | -0.4 |
| G1 | 1426716_at | Tdrd7 | 20 | 0.94 | 1.79 | 1.21 | 1.44 | 3.28 | 3.24 | 1.09 | 1.6 | 1.74 | 2.5 | 2.18 | 2.34 | 1.47 | 1.15 | 2.22 | -0.3 | -0.5 | -0.5 | -0.4 | -0.5 | -0.5 | -0.3 | -0.2 | -0.6 | -0.4 | -0.6 | -0.4 | -0.3 |

[Table 12-2]

| Group | Probe Index | Gene Symbol | Modules | (10)MPLA.ID.LV.x1 | (10)MPLA.ID.LV.x3 | (2)K3.IP.LV.x2 | (2)K3.IP.LV.x3 | (2)K3SPG.IP.LV.x3 | (2)K3.IP.LV.x1 | (2)K3SPG.IP.LV.x1 | (7)sHz.ID.LV.x2 | (7)MBT.ID.LV.x2 | (7)sHz.ID.LV.x3 | (7)sHz.ID.LV.x1 | (7)MBT.ID.LV.x1 | (7)MBT.ID.LV.x3 | (4)bCD.IP.LV.x1 | (4)bCD.IP.LV.x2 | (4)bCD.IP.LV.x3 | (5)K3SPG.ID.LV.x1 | (5)K3SPG.ID.LV.x2 | (5)K3SPG.ID.LV.x3 | (5)D35.ID.LV.x1 | (5)D35.ID.LV.x3 | (5)D35.ID.LV.x2 | (2)ALM.IP.LV.x2 | (2)ALM.IP.LV.x1 | (2)ALM.IP.LV.x3 | (5)bCD.ID.LV.x3 | (6)FCA.ID.LV.x3 | (4)ENDCN.IN.LV.x2 |
|---|---|---|---|---|---|---|---|---|---|---|---|---|---|---|---|---|---|---|---|---|---|---|---|---|---|---|---|---|---|---|---|
| G1 | 1449025_at | Ifit3 | 20 | -0.5 | -0.5 | -0.5 | -0.5 | -0.5 | -0.5 | -0.5 | -0.5 | -0.5 | -0.5 | -0.5 | -0.5 | -0.5 | -0.5 | -0.5 | -0.5 | -0.5 | -0.5 | -0.5 | -0.5 | -0.5 | -0.5 | -0.5 | -0.5 | -0.5 | -0.5 | -0.5 | -0.5 |
| G1 | 1456890_at | Ddx58 | 20 | -0.2 | -0.4 | -0.3 | -0.4 | -0.4 | -0.4 | -0.3 | -0.5 | -0.6 | -0.8 | -0.3 | -0.4 | -0.5 | -0.6 | -0.4 | -0.3 | -0.6 | -0.4 | -0.4 | -0.3 | -0.4 | -0.3 | -0.6 | -0.6 | -0.8 | -0.6 | -0.8 | -0.5 |
| G1 | 1450783_at | Ifit1 | 20 | -0.5 | -0.5 | -0.5 | -0.5 | -0.5 | -0.5 | -0.4 | -0.5 | -0.5 | -0.5 | -0.5 | -0.5 | -0.4 | -0.5 | -0.5 | -0.5 | -0.5 | -0.5 | -0.5 | -0.5 | -0.4 | -0.4 | -0.5 | -0.5 | -0.5 | -0.5 | -0.5 | -0.5 |
| G1 | 1450034_at | Stat1 | 20 | -0.4 | -0.5 | -0.6 | -0.6 | -0.6 | -0.5 | -0.4 | -0.5 | -0.5 | -0.6 | -0.6 | -0.6 | -0.5 | -0.5 | -0.5 | -0.5 | -0.6 | -0.6 | -0.6 | -0.5 | -0.5 | -0.5 | -0.6 | -0.6 | -0.6 | -0.5 | -0.5 | -0.6 |
| G1 | 1436562_at | Ddx58 | 20 | -0.3 | -0.5 | -0.2 | -0.4 | -0.3 | -0.5 | 0.09 | -0.5 | -0.6 | -0.7 | -0.7 | -0.6 | -0.4 | -0.4 | -0.3 | -0.1 | -0.4 | -0.5 | -0.5 | -0.4 | -0.4 | -0.1 | -0.6 | -0.6 | -0.7 | -0.7 | -0.8 | -0.6 |
| G1 | 1437503_a_at | Shisa5 | 20 | -0.3 | -0.4 | -0.4 | -0.6 | -0.5 | -0.5 | -0.2 | -0.5 | -0.5 | -0.6 | -0.6 | -0.7 | -0.6 | -0.4 | -0.3 | -0.3 | -0.3 | -0.4 | -0.4 | -0.4 | -0.4 | 0.15 | -0.8 | -0.8 | -1.1 | -0.4 | -0.7 | -0.4 |
| G1 | 1418825_at | Irgm1 | 20 | -0.4 | -0.6 | -0.5 | -0.6 | -0.6 | -0.5 | -0.4 | -0.5 | -0.5 | -0.6 | -0.6 | -0.6 | -0.5 | -0.5 | -0.6 | -0.5 | -0.6 | -0.6 | -0.6 | -0.5 | -0.5 | -0.3 | -0.6 | -0.6 | -0.6 | -0.5 | -0.5 | -0.6 |
| G1 | 1426906_at | Mndal | 20 | -0.2 | -0.5 | -0.4 | -0.4 | -0.5 | -0.5 | -0.4 | -0.4 | -0.4 | -0.4 | -0.4 | -0.6 | -0.4 | -0.3 | -0.3 | -0.5 | -0.5 | -0.5 | -0.6 | -0.5 | -0.4 | -0.3 | -0.6 | -0.7 | -0.7 | -0.5 | -0.5 | -0.6 |
| G1 | 1450033_a_at | Stat1 | 20 | -0.5 | -0.6 | -0.5 | -0.5 | -0.6 | -0.6 | -0.4 | -0.5 | -0.4 | -0.5 | -0.6 | -0.5 | -0.4 | -0.5 | -0.6 | -0.5 | -0.5 | -0.5 | -0.6 | -0.5 | -0.5 | -0.4 | -0.5 | -0.6 | -0.6 | -0.6 | -0.5 | -0.5 |
| G1 | 1436058_at | Rsad2 | 20 | -0.5 | -0.5 | -0.5 | -0.5 | -0.5 | -0.5 | -0.4 | -0.5 | -0.5 | -0.5 | -0.5 | -0.5 | -0.5 | -0.5 | -0.4 | -0.4 | -0.5 | -0.5 | -0.5 | -0.5 | -0.5 | -0.5 | -0.5 | -0.5 | -0.5 | -0.5 | -0.5 | -0.5 |
| G1 | 1423986_a_at | Shisa5 | 20 | -0.2 | -0.3 | -0.5 | -0.8 | -0.6 | -0.6 | -0.3 | -0.3 | -0.4 | -0.5 | -0.5 | -0.6 | -0.6 | -0.6 | -0.3 | -0.3 | -0.7 | -0.7 | -0.4 | -0.5 | -0.6 | -0 | -0.8 | -0.8 | -0.9 | -0.6 | -0.7 | -0.3 |
| G1 | 1434268_at | Adar | 20 | -0 | -0.3 | -0.8 | -0.8 | -0.7 | -0.6 | -0.3 | -0.5 | -0.5 | -0.7 | -0.6 | -0.9 | -0.3 | -0.3 | -0.4 | -0.4 | -0.5 | -0.6 | -0.4 | -0.3 | -0.4 | -0.3 | -0.5 | -0.6 | -0.7 | -0.1 | -0.5 | -0.7 |
| G1 | 1425405_a_at | Adar | 20 | -0.2 | -0.6 | -0.5 | -0.2 | -0.5 | -0.5 | -0.2 | -0.4 | -0.2 | -0.5 | -0.5 | -0.6 | -0.4 | -0.5 | -0.4 | -0.3 | -0.3 | -0.3 | -0.5 | -0.1 | -0.3 | -0.3 | -0.6 | -0.7 | -0.9 | -0.4 | -0.7 | -0.8 |
| G1 | 1435526_at | Tor1aip2 | 20 | -0.2 | -0.5 | -0.2 | -0.5 | -0.5 | -0.3 | -0.3 | -0.3 | -0.8 | -0.8 | -0.4 | -0.8 | -0.6 | -0.4 | -0.3 | -0.6 | -0.4 | -0.6 | -0.6 | -0.4 | -0.5 | -0.4 | -0.7 | -0.6 | -0.7 | -0.4 | -0.3 | -0.2 |
| G1 | 1431591_s_at | Gm9706///Isg15 | 20 | -0.5 | -0.5 | -0.5 | -0.5 | -0.5 | -0.5 | -0.5 | -0.5 | -0.5 | -0.5 | -0.5 | -0.5 | -0.5 | -0.5 | -0.5 | -0.5 | -0.5 | -0.5 | -0.5 | -0.5 | -0.5 | -0.5 | -0.5 | -0.5 | -0.5 | -0.5 | -0.5 | -0.5 |
| G1 | 1455500_at | Rnf213 | 20 | -0.4 | -0.5 | -0.4 | -0.5 | -0.6 | -0.5 | -0.3 | -0.5 | -0.4 | -0.5 | -0.6 | -0.6 | -0.4 | -0.5 | -0.5 | -0.5 | -0.5 | -0.5 | -0.5 | -0.4 | -0.4 | -0.4 | -0.6 | -0.6 | -0.7 | -0.5 | -0.5 | -0.6 |
| G1 | 1418115_s_at | Tor1aip2 | 20 | -0.4 | -0.6 | -0.2 | -0.2 | -0.1 | -0.5 | -0.2 | -0.3 | -0.5 | -0.7 | -0.6 | -0.8 | -0.5 | -0.4 | -0.5 | -0.4 | -0.5 | -0.5 | -0.7 | -0.4 | -0.6 | -0.1 | -0.5 | -0.6 | -0.6 | -0.2 | -0.5 | -0.5 |
| G1 | 1418116_at | Tor1aip2 | 20 | -0 | -0.2 | -0 | -0.2 | -0.1 | -0.4 | 0.11 | -0.3 | -0.5 | -0.9 | -0.7 | -0.9 | -0.6 | -0.3 | -0.3 | -0.5 | -0.4 | -0.6 | -0.6 | -0.4 | -0.5 | -0.2 | -0.5 | -0.6 | -0.7 | -0.4 | -0.5 | -0.6 |
| G1 | 1426970_a_at | Uba7 | 20 | -0.1 | -0.6 | -0.4 | -0.3 | -0.6 | -0.5 | -0.3 | -0.7 | -0.5 | -0.4 | -0.6 | -0.7 | -0.6 | -0.5 | -0.5 | -0.4 | -0.6 | -0.8 | -0.6 | -0.8 | -0.6 | -0.5 | -0.4 | -0.7 | -0.8 | -0.6 | -0.5 | -0.6 |
| G1 | 1421323_a_at | G3bp2 | 20 | -0.3 | -0.5 | -0.4 | -0.4 | -0.4 | -0.6 | -0.6 | -0.3 | -0.6 | -0.7 | -0.7 | -0.9 | -0.5 | -0.4 | -0.3 | -0.6 | -0.4 | -0.8 | -0.8 | -0.5 | -0.4 | -0.4 | -0.3 | -0.3 | -0.6 | -0.3 | -0.2 | -0.3 |
| G1 | 1422005_at | Eif2ak2 | 20 | -0.3 | -0.4 | -0.7 | -0.5 | -0.6 | -0.6 | -0.5 | -0.3 | -0.6 | -0.7 | -0.5 | -0.5 | -0.3 | -0.5 | -0.4 | -0.5 | -0.6 | -0.6 | -0.6 | -0.5 | -0.4 | -0.4 | -0.6 | -0.6 | -0.6 | -0.4 | -0.5 | -0.6 |
| G1 | 1448757_at | Pml | 20 | -0.4 | -0.4 | -0.3 | -0.6 | -0.4 | -0.5 | -0.3 | -0.4 | -0.4 | -0.2 | -0.5 | -0.6 | -0.4 | -0.3 | -0.3 | -0.4 | -0.5 | -0.6 | -0.5 | -0.7 | -0.4 | -0.3 | -0.7 | -0.6 | -0.8 | -0.6 | -0.4 | -0.7 |
| G1 | 1417244_a_at | Irf7 | 20 | -0.4 | -0.5 | -0.6 | -0.5 | -0.6 | -0.5 | -0.3 | -0.5 | -0.3 | -0.4 | -0.4 | -0.4 | -0.4 | -0.6 | -0.7 | -0.5 | -0.5 | -0.4 | -0.3 | -0.3 | -0.2 | -0.3 | -0.7 | -0.7 | -0.6 | -0.4 | -0.6 | -0.6 |
| G1 | 1426276_at | Ifih1 | 20 | -0.4 | -0.4 | -0.3 | -0.5 | -0.4 | -0.4 | -0.3 | -0.5 | -0.5 | -0.5 | -0.5 | -0.5 | -0.5 | -0.4 | -0.5 | -0.4 | -0.5 | -0.6 | -0.5 | -0.5 | -0.5 | -0.3 | -0.5 | -0.5 | -0.6 | -0.5 | -0.5 | -0.5 |
| G1 | 1449875_s_at | H2-T10///H2-T22///H2-T9 | 20 | -0.3 | -0.4 | -0.2 | -0.4 | -0.3 | -0.2 | -0.1 | -0.3 | -0.4 | -0.6 | -0.4 | -0.6 | -0.4 | -0.3 | -0.4 | -0.3 | -0.3 | -0.6 | -0.6 | -0.4 | -0.3 | -0.2 | -0.8 | -0.8 | -0.8 | -0.6 | -0.8 | -0.4 |
| G1 | 1437432_a_at | Trim12a | 20 | -0.4 | -0.5 | -0.5 | -0.5 | -0.5 | -0.5 | -0.4 | -0.4 | -0.4 | -0.4 | -0.5 | -0.6 | -0.4 | -0.5 | -0.6 | -0.6 | -0.5 | -0.4 | -0.6 | -0.4 | -0.5 | -0.4 | -0.5 | -0.6 | -0.6 | -0.5 | -0.5 | -0.6 |
| G1 | 1456103_at | Pml | 20 | -0.3 | -0.3 | -0.3 | -0.6 | -0.4 | -0.5 | -0.4 | -0.4 | -0.5 | -0.4 | -0.5 | -0.5 | -0.4 | -0.5 | -0.4 | -0.6 | -0.3 | -0.5 | -0.3 | -0.4 | -0.3 | -0.3 | -0.6 | -0.7 | -0.7 | -0.6 | -0.4 | -0.7 |
| G1 | 1426716_at | Tdrd7 | 20 | -0.3 | -0.4 | -0.1 | -0.3 | -0.2 | -0.2 | 0.12 | -0.2 | -0.3 | -0.4 | -0.3 | -0.7 | -0.2 | -0.3 | -0.1 | -0.3 | -0.4 | -0.3 | -0.2 | -0.4 | -0.4 | -0.1 | -0.9 | -0.8 | -0.9 | -0.7 | -0.6 | -0.9 |

[Table 12-3]

| Group | Probe Index | Gene Symbol | Modules | (5)bCD.ID.LV.x2 | (5)bCD.ID.LV.x1 | (4)ENDCN.IN.LV.x1 | (4)ENDCN.IN.LV.x3 | (3)K3.ID.LV.x3 | (6)FCA.ID.LV.x1 | (6)FCA.ID.LV.x2 | (10)MALP2s.ID.LV.x2 | (10)MALP2s.ID.LV.x1 | (10)MALP2s.ID.LV.x3 | (1)ADX.IP.LV.x1 | (1)ADX.IP.LV.x2 | (1)ADX.IP.LV.x3 | (3)ADX.ID.LV.x2 | (3)ADX.ID.LV.x3 | (7)FK565.ID.LV.x3 | (7)FK565.ID.LV.x1 | (7)FK565.ID.LV.x2 | (8)Pam3CSK4.ID.LV.x3 | (8)Pam3CSK4.ID.LV.x1 | (8)Pam3CSK4.ID.LV.x2 | Group | Group Z ave |
|---|---|---|---|---|---|---|---|---|---|---|---|---|---|---|---|---|---|---|---|---|---|---|---|---|---|---|
| G1 | 1449025_at | Ifit3 | 20 | -0.5 | -0.5 | -0.5 | -0.5 | -0.5 | -0.5 | -0.5 | -0.5 | -0.4 | -0.5 | -0.5 | -0.5 | -0.5 | -0.5 | -0.4 | -0.4 | -0.4 | -0.4 | -0.4 | -0.5 | -0.5 | G1 | 1.95 |
| G1 | 1456890_at | Ddx58 | 20 | -0.7 | -0.7 | -0.4 | -0.7 | -0.6 | -0.5 | -0.9 | -0.5 | -0.4 | -0.5 | -0.4 | -0.4 | -0.4 | -0.2 | -0.4 | -0.5 | -0.5 | -0.1 | -0.4 | -0.4 | -0.4 | G1 | 1.95 |
| G1 | 1450783_at | Ifit1 | 20 | -0.5 | -0.5 | -0.5 | -0.5 | -0.5 | -0.5 | -0.5 | -0.5 | -0.5 | -0.5 | -0.5 | -0.5 | -0.5 | -0.5 | -0.4 | -0.4 | -0.4 | -0.4 | -0.4 | -0.5 | -0.4 | G1 | 1.94 |
| G1 | 1450034_at | Stat1 | 20 | -0.6 | -0.6 | -0.5 | -0.6 | -0.5 | -0.4 | -0.5 | -0.1 | 0.19 | 0.05 | -0.3 | -0.4 | -0.4 | -0.5 | -0.4 | -0.1 | -0.1 | -0 | -0.5 | -0.5 | -0.4 | G1 | 1.93 |
| G1 | 1436562_at | Ddx58 | 20 | -0.7 | -0.7 | -0.6 | -0.4 | -0.5 | -0.6 | -0.7 | -0.5 | -0.4 | -0.5 | -0.4 | -0.4 | -0.5 | -0.6 | -0.4 | -0.2 | -0.3 | -0.2 | -0.5 | -0.6 | -0.5 | G1 | 1.93 |
| G1 | 1437503_a_at | Shisa5 | 20 | -0.7 | -0.5 | -0.5 | -0.5 | -0.5 | -0.7 | -0.7 | -0.3 | -0.1 | -0.4 | -0.1 | -0.3 | -0.4 | -0.6 | -0.3 | -0.2 | -0.3 | -0.1 | -0.4 | -0.6 | -0.4 | G1 | 1.93 |
| G1 | 1418825_at | Irgm1 | 20 | -0.7 | -0.6 | -0.6 | -0.6 | -0.5 | -0.4 | -0.5 | -0.3 | 0.35 | -0 | -0.4 | -0.4 | -0.5 | -0.5 | -0.4 | 0.07 | 0.13 | 0.08 | -0.3 | -0.4 | -0.3 | G1 | 1.93 |
| G1 | 1426906_at | Mndal | 20 | -0.6 | -0.6 | -0.4 | -0.4 | -0.5 | -0.5 | -0.6 | -0.4 | 0.4 | 0 | -0.6 | -0.7 | -0.7 | -0.7 | -0.6 | -0.4 | -0.5 | -0.5 | -0.6 | -0.6 | -0.4 | G1 | 1.93 |
| G1 | 1450033_a_at | Stat1 | 20 | -0.6 | -0.6 | -0.5 | -0.5 | -0.5 | -0.5 | -0.5 | -0.2 | -0 | -0 | -0.3 | -0.4 | -0.4 | -0.5 | -0.4 | -0.1 | -0 | 0 | -0.4 | -0.5 | -0.3 | G1 | 1.92 |
| G1 | 1436058_at | Rsad2 | 20 | -0.5 | -0.5 | -0.5 | -0.5 | -0.5 | -0.5 | -0.5 | -0.4 | -0.4 | -0.4 | -0.4 | -0.5 | -0.5 | -0.5 | -0.5 | -0.4 | -0.4 | -0.4 | -0.4 | -0.5 | -0.4 | G1 | 1.92 |
| G1 | 1423986_a_at | Shisa5 | 20 | -0.7 | -0.8 | -0.3 | -0.4 | -0.5 | -0.7 | -0.4 | -0.2 | -0 | -0.2 | -0.3 | -0.4 | -0.4 | -0.6 | -0.3 | -0.1 | 0.01 | 0.18 | -0.5 | -0.5 | -0.3 | G1 | 1.91 |
| G1 | 1434268_at | Adar | 20 | -0.5 | -0.3 | -0.7 | -0.9 | -0.1 | -0.5 | -0.5 | 0.04 | 0.15 | 0.2 | -0.5 | -0.6 | -0.7 | -0.7 | -0.5 | -0.1 | -0.1 | -0.1 | -0.2 | -0.4 | -0.4 | G1 | 1.91 |
| G1 | 1425405_a_at | Adar | 20 | -0.6 | -0.7 | -0.5 | -0.4 | -0.5 | -0.7 | -0.6 | -0.5 | -0.7 | -0.7 | -0.4 | -0.2 | -0.6 | -0.3 | -0.3 | -0.4 | -0.5 | -0.4 | -0.3 | -0.5 | -0.2 | G1 | 1.91 |
| G1 | 1435526_at | Tor1aip2 | 20 | -0.7 | -0.4 | -0.1 | -0.4 | -0.1 | -0.2 | -0.3 | -0.4 | -0.3 | -0.5 | -0.4 | -0.4 | -0.6 | -0.6 | -0.5 | -0.8 | -0.9 | -0.9 | -0.5 | -0.4 | -0.4 | G1 | 1.9 |
| G1 | 1431591_s_at | Gm9706///Isg15 | 20 | -0.5 | -0.5 | -0.5 | -0.5 | -0.5 | -0.5 | -0.5 | -0.4 | -0.3 | -0.3 | -0.5 | -0.5 | -0.5 | -0.5 | -0.4 | -0.1 | -0.1 | -0.2 | -0.4 | -0.5 | -0.5 | G1 | 1.9 |
| G1 | 1455500_at | Rnf213 | 20 | -0.6 | -0.6 | -0.5 | -0.6 | -0.5 | -0.5 | -0.5 | -0.4 | -0.2 | -0.4 | -0.3 | -0.4 | -0.5 | -0.4 | -0.3 | 0.02 | 0.06 | 0.03 | -0.2 | -0.3 | -0.3 | G1 | 1.9 |
| G1 | 1418115_s_at | Tor1aip2 | 20 | -0.7 | -0.6 | -0.3 | -0.4 | -0.1 | -0.1 | -0.3 | -0.5 | -0.5 | -0.5 | -0.4 | -0.5 | -0.7 | -0.6 | -0.4 | -0.6 | -0.5 | -0.5 | -0.3 | -0.4 | -0.3 | G1 | 1.9 |
| G1 | 1418116_at | Tor1aip2 | 20 | -0.8 | -0.6 | -0 | -0.3 | -0.1 | -0.1 | -0.3 | -0.3 | -0.5 | -0.7 | -0.3 | -0.6 | -0.6 | -0.9 | -0.6 | -0.7 | -0.9 | -0.7 | -0.3 | -0.4 | -0.3 | G1 | 1.9 |
| G1 | 1426970_a_at | Uba7 | 20 | -0.7 | -0.6 | -0.5 | -0.5 | -0.4 | -0.4 | -0.6 | -0 | 0.6 | 0.08 | -0.2 | -0.6 | -0.3 | -0.5 | -0.2 | -0.4 | -0.5 | -0.3 | -0.4 | -0.6 | -0.6 | G1 | 1.9 |
| G1 | 1421323_a_at | G3bp2 | 20 | -0.4 | -0.3 | -0.4 | -0.4 | -0.5 | -0.2 | -0.5 | -0.4 | 0.05 | -0.2 | -0.4 | -0.6 | -0.4 | -0.7 | -0.6 | -0.3 | -0.3 | -0.2 | -0.6 | -0.4 | -0.4 | G1 | 1.89 |
| G1 | 1422005_at | Eif2ak2 | 20 | -0.6 | -0.5 | -0.5 | -0.5 | -0.5 | -0.4 | -0.6 | -0.3 | 0.07 | -0.4 | -0.3 | -0.4 | -0.5 | -0.6 | -0.5 | 0.05 | 0.09 | -0.1 | -0.5 | -0.5 | -0.3 | G1 | 1.89 |
| G1 | 1448757_at | Pml | 20 | -0.7 | -0.7 | -0.6 | -0.4 | -0.5 | -0.1 | -0.4 | -0.6 | -0.4 | -0.4 | -0.4 | -0.4 | -0.4 | -0.7 | -0.4 | -0.5 | -0.6 | -0.8 | -0.2 | -0.2 | -0.4 | G1 | 1.89 |
| G1 | 1417244_a_at | Irf7 | 20 | -0.4 | -0.5 | -0.6 | -0.7 | -0.4 | -0.5 | -0.5 | -0.4 | -0.3 | -0.3 | -0.4 | -0.5 | -0.4 | -0.3 | -0.4 | -0.3 | -0.3 | -0.4 | -0.4 | -0.6 | -0.3 | G1 | 1.89 |
| G1 | 1426276_at | Ifih1 | 20 | -0.6 | -0.6 | -0.4 | -0.4 | -0.5 | -0.5 | -0.6 | -0.5 | -0.3 | -0.4 | -0.4 | -0.5 | -0.5 | -0.5 | -0.4 | -0.3 | -0.3 | -0.3 | -0.4 | -0.5 | -0.4 | G1 | 1.89 |
| G1 | 1449875_s_at | H2-T10///H2-T22///H2-T9 | 20 | -0.8 | -0.6 | -0.4 | -0.5 | -0.7 | -0.7 | -0.7 | -0.6 | -0.3 | -0.5 | -0.4 | -0.5 | -0.4 | -0.7 | -0.3 | 0.21 | 0.04 | 0.29 | -0.5 | -0.6 | -0.5 | G1 | 1.89 |
| G1 | 1437432_a_at | Trim12a | 20 | -0.5 | -0.5 | -0.6 | -0.5 | -0.4 | -0.5 | -0.4 | -0.6 | -0.3 | -0.4 | -0.4 | -0.4 | -0.5 | -0.5 | -0.2 | 0.1 | -0.1 | -0.2 | -0.3 | -0.4 | -0.4 | G1 | 1.88 |
| G1 | 1456103_at | Pml | 20 | -0.6 | -0.5 | -0.6 | -0.5 | -0.6 | -0.4 | -0.4 | -0.3 | 0.04 | -0.2 | -0.4 | -0.5 | -0.5 | -0.4 | -0.3 | -0.5 | -0.5 | -0.5 | -0.2 | -0.5 | -0.5 | G1 | 1.88 |
| G1 | 1426716_at | Tdrd7 | 20 | -1 | -0.9 | -0.4 | -0.3 | -0.6 | -0.4 | -0.6 | -0.9 | -0.9 | -0.9 | -0.4 | -0.5 | -0.7 | -0.7 | -0.5 | -0.5 | -0.4 | -0.5 | -0.1 | -0.4 | -0.2 | G1 | 1.88 |

Whole organ transcriptome data for each organ from each mouse were analyzed as follows to obtain biological themes for individual samples from each adjuvant-administered group of three mice. For a particular experimental condition (e.g., DMXAA, LV, i.d.), eij is the expression value of the ith gene in the jth sample (where j = 1, 2, 3) and ei(C) is the mean expression value of the corresponding control samples. A set of Pj genes was first defined, where eij/ei(c)>2.0. Biological themes enriched within Pj were then identified using TargetMine (Chen, Y.A. et al., PloS one 6, e17844 (2011)) (http://targetmine.mizuguchilab.org/). Tj denote the resulting list of biological themes, with associated p-values from Fischer's exact test.

Next, the biological themes called by all the individual samples in the group were scored as follows. Having obtained three lists (T1, T2 and T3) for a given experimental condition, a consensus list T of themes was created as T = {(t1, p1), (t2, p2), ...}, where ti is a biological theme that appears in all of T1, T2 and T3, and pi is the smallest of the associated p-values. Only the themes with the p-value of < 0.05 were included in T.

To summarize the biological themes in T further, a list of terms considered to be associated with adjuvant activity in general was separately prepared. AT = {at1, at2, ...}, where ati is a pre-selected adjuvant-associated term (e.g., "stress" or "cytokine"). Finally, an enrichment score S for an adjuvant-associated term ati was defined as: S(ati) = Σ -log(pj), where the name of tj in T includes a term ati as a substring and pj is the associated p-value.

This scoring scheme summarizes the relative enrichment of pre-selected adjuvant-associated terms in genes responding to each adjuvant in each organ.

(Hierarchical clustering)

The union of all sDEGs for adjuvants and each organ was defined as "adjuvant gene space". Hierarchical clustering of adjuvants and genes in the adjuvant gene space was performed by the hclust function of the R package, with the 1-Pearson correlation coefficient as the distance measure and the Ward D2 algorithm. The resultant gene cluster, defined here as "gene module" (labeled $Mi^x$, where i is the module number and x is the organ), assumes that genes within a module behave similarly upon administration of a given adjuvant. The 21 adjuvants used herein were also hierarchically clustered, and grouped as $Gj^y$, where (j) is the group number and (y) is the organ.

(Cell population profiling based on the ImmGen database)

The ImmGen database (Heng, T.S. et al., Nature immunology 9, 1091-1094 (2008)) (http://www.immgen.org/) provides the gene expression profiles of various immune cell types under steady-state conditions. Their expression profiles were used to estimate the origin of the cell type from which the genes were derived. Initially, from the ImmGen expression profile, each gene (i) was weighted in all ten immune cell types (j).

[Numeral 1]

$$w_{ij} = \frac{e_{ij}}{\sum_{j=1}^{10} e_{ij}}$$

Here, it is assumed that the weight of a cell type (e.g., neutrophil) for a given gene depends only on the expression level of that gene in that particular cell type, independent of its expression level in other cell types (e.g., macrophage, B cell). Subsequently, for the expression profile of each adjuvant, genes were selected based on their fold changes, p-value cutoffs, and PA call thresholds. The cell type contribution in each sample (k) for cell type (j) is calculated as:

[Numeral 2]

$$S_{jk} = \frac{s_{ik} w_{ij}}{G}$$

where (s) is the gene's differential expression and G is the total number of genes that satisfy the given cutoff.

(Z-score-based differential gene expression analysis)

Each sample's FC value was converted into a z-score on a gene basis by using the entire data set for each organ. The Z-score of the triplicates of interest were summed for each adjuvant. For example, the three sample z-score in the LV sample following cdiGMP administration was summed on a gene-by-gene basis, after which the summed scores of > 3 genes were selected as relatively preferentially expressed genes in the LV after cdiGMP administration. The following threshold was applied for selections. In the LV, $G1^{LV}$-associated genes were selected with cdiGMP(z>3)

& cGAMP(z>3) & DMXAA(z>3) & PolyIC (z>3) & R848 (z>3) . G2$^{LV}$: ALM_IP(z>1) & bCD(z>1) & ENDCN(z>1) & FCA(z>1). G3$^{LV}$: ADX (z>1) & Pam3CSK4 (z>1) & FK565 (z>1). G4$^{LV}$: MALP2s (z>3) . In the SP, G1$^{SP}$: cdiGMP (z>3) & cGAMP(z>3) & DMXAA(z>3) & PolyIC(z>3) & R848 (z>3). G2$^{SP}$: ENDCN_x2 + ENDCN_x3(z>2) & ALM_IP(z>3) & bCD(z>3). G3$^{SP}$: FCA(z>3) & FK565 (z>3) & Pam3CSK4 (z>3). G4$^{SP}$: ADX_ID_x2 + ADX_ID_x3 (z>2) & ADX_IP(z>3) & MALP2s(z>3). In the LN, G1$^{LN}$(5adj): cdiGMP(z>3) & cGAMP(z>3) & DMXAA(z>3) & PolyIC(z>3) & R848 (z>3). G1$^{LN}$(8adj): cdiGMP(z>0) & cGAMP(z>0) & DMXAA(z>0) & PolyIC(z>0) & R848 (z>0) & MALP2s (z>575 0) & MPLA_x1 + MPLA_x3(z>0) & Pam3CSK4_x2 + Pam3CSK4_x3(z>0) . G2$^{LN}$: bCD(z>3) & FCA(z>3). G3$^{LN}$: FK565(z>3). G5$^{LN}$: K3(z>1.5) & K3SPG_x1 + K3SPG_x2 (z>1) & D35_x1 + D35_x3(z>1). G6$^{LN}$: AddaVax(z>3) .

Hematological data and gene expression correlation analysis

Pearson's correlation analysis was applied to the hematological data and to the gene expression changes in each organ. The data used for the analysis were selected using the following criteria. The genes should be cPA=1 and the hematology data should be > 1 standard deviation (1 SD) from the mean of the pooled control. The resultant hematological parameter pairs and genes were further selected by Welch's t-test. The pairs with p-values of < 0.05 and with correlation coefficient absolute values of < 0.8 and with an absolute value of the slope (incline) of < 1.2 were selected. The hematological and gene data derived from Exp. 5 and Exp. 10 were obtained in separate experiments, so these unlinked data were not used in this analysis. Importantly, when these unlinked sample data were plotted as test samples, these data were also a good fit with the correlation lines.

Table 13 Adjuvants and control used in this Example

[Table 13]

| | Name | Full Name/Description | Physical property | Receptor |
|---|---|---|---|---|
| 1 | AddaVax | similar to MF59 | squalene oil-in-water emulsion | Unknown |
| 2 | ADX | Advax™ | semi-crystalline particles of delta inulin | Unknown |
| 3 | ALM | Aluminium hydroxide gel | gel suspension | Unknown |
| 4 | bCD | Hydroxypropyl-beta-cyclodextrin | cyclodexetrin | Unknown |
| 5 | cdiGMP | Cyclic diguanylate monophosphate | cyclic di-nucleotide | STING |
| 6 | cGAMP | Cyclic [G(2',5')pA(3',5')p] | cyclic di-nucleotide | STING |
| 7 | D35 | Type A CpG ODNs | syntheitc oligodeoxyribonucleotide | TLR9 |
| 8 | DMXAA | 5,6-dimethylxanthenone-4-acetic acid | Synthetic chemicals | STING |
| 9 | ENDCN | Endocine™ | Mono-olein and oleic acid | Unknown |
| 10 | FCA | Complete Freund's Adjuvant | Mycobacteriumlwater-in-oil emulsion | CLR and unkown |
| 11 | FK565 | heptanoyl-r-D-glutamyl-(L)-meso-diaminopimelyl-(D)-alanine | Synthetic peptidoglycans | NOD1 |
| 12 | ISA51VG | Incomplete Freund's Adjuvant | water-in-oil emulsion | Unknown |
| 13 | K3 | Type B CpG ODNs | syntheitc oligodeoxyribonucleotide | TLR9 |
| 14 | K3SPG | Complex of K3 CpG ODNs and bata-glucan (SPG) | deoxyribonucleotide/ glucan complex | TLR9 |
| 15 | MALP2s | macrophage-activating lipopeptide-2 short lipopeptide | lipopeptide | TLR2/6 |
| 16 | MBT | N-Acetyl-muramyl-L- Alanyl-D-Glutamin-n-butyl-ester | Synthetic peptidoglycans | NOD2 |
| 17 | MPLA | Monophosphoryl lipid A | low-toxicity derivative of lipopolysaccharide | TLR4 |
| 18 | Pam3CSK4 | Pam3-Cys-Ser-Lys-Lys-Lys-Lys | synthetic triacylated lipopeptide | TLR1/2 |
| 19 | PolyIC | Polyinosine-polycytidylic acid | double stranded ribonucleotide polymer | TLR3 and MDA5 |
| 20 | R848 | imidazoquinoline compound | guanosine derivative | TLR7 |

(continued)

| | Name | Full Name/Description | Physical property | Receptor |
|---|---|---|---|---|
| 21 | sHZ | synthetic hemozoin | beta hematin crystals | Unknown |

Next, the adjuvants and the dosages used in this Example are shown.

[Table 14-1]

| Adjuvants and dosages thereof used in each experiment | | | | | | | |
|---|---|---|---|---|---|---|---|
| **Exp. No.** | **Test substance** | **Route** | **Dose** | **Conc.** | **Dose volume** | **Group No.** | **Kit** |
| Exp. 1 | PBS | i.p. | 0 mg | 0 mg/ mL | 0.1 mL | 1 | E |
| | ADX | i.p. | 1 mq | 10 mq/ mL | 0.1 mL | 2 | E |
| | | | | | | | |
| Exp. 2 | PBS | i.p. | 0 mq | 0 mq/ mL | 0.1 mL | 1 | E |
| | ALM | i.p. | 1 mq | 10 mg/ mL | 0.1 mL | 2 | E |
| | K3 | i.p. | | 0.1 mg/ mL | 0.1 mL | 3 | E |
| | K3SPG | i.p. | | 0.1 mg/ mL | 0.1 mL | 4 | E |
| | | | | | | | |
| Exp. 3 | PBS | i.d. | 0 mg | 0 mg/ mL | 0.1 mL | 1 | E |
| | ADX | i.d. | 1 mg | 10 mg/ mL | 0.1 mL | 2 | E |
| | ALM | i.d. | 1 mq | 10 mq/ mL | 0.1 mL | 3 | E |
| | K3 | i.d. | 0.01 mq | 0.1 mg/ mL | 0.1 mL | 4 | E |
| | | | | | | | |
| Exp. 4 | PBS | i.p. | | 0% | 0.2 mL | 1 | E |
| | bCD | i.p. | | 30% | 0.2 mL | 2 | E |
| | Tris-HCl | i.n. | | 0% | 0.005 mL | 3 | E |
| | ENDCN | i.n. | | 2% | 0.005 mL | 4 | E |
| | | | | | | | |
| Exp. 3 | PBS | i.d. | 0 mq | 0 mq/ mL | 0.1 mL | 1 | E |
| | D35 | i.d. | 0.01 mq | 0.1 mg/ mL | 0.1 mL | 2 | E |
| | K3SPG | i.d. | 0.01 mq | 0.1 mg/ mL | 0.1 mL | 3 | E |
| | bCD | i.d. | | 30% | 0.2 mL | 4 | E |
| | | | | | | | |
| Exp. 6 | PBS | i.d. | | 0% | 0.1 mL | 1 | E |
| | FCA | i.d. | | 50% | 0.1 mL | 4 | E |
| | | | | | | | |
| Exp. 7 | PBS | i.d. | 0 mq | 0 mq/ mL | 0.1 mL | 1 | P |
| | sHZ | i.d. | 0.2 mq | 2 mq/ mL | 0.1 mL | 2 | P |

[Table 14-2]

| | | | | | | | |
|---|---|---|---|---|---|---|---|
| | FK565 | i.d. | 0.01 mg | 0.1 mg/ mL | 0.1 mL | 3 | P |

(continued)

| | | | | | | | | |
|---|---|---|---|---|---|---|---|---|
| | MBT | i.d. | 0.01 mg | 0.1 mg/ mL | 0.1 mL | 4 | P |
| | | | | | | | |
| Exp. 8 | PBS | i.d. | 0 mq | 0 mg/ mL | 0.1 mL | 1 | P |
| | PolvIC | i.d. | 0.1 mg | 1 mg/ mL | 0.1 mL | 2 | P |
| | Pam3CSK4 | i.d. | 0.01 m g | 0.1 mg/ mL | 0.1 mL | 3 | P |
| | cdiGMP | i.d. | 0.01 mg | 0.1 mg/ mL | 0.1 mL | 4 | P |
| | | | | | | | |
| Exp. 9 | PBS | i.d. | | 0% | 0.1 mL | 1 | P |
| | Addavax | i.d. | | 50% | 0.1 mL | 2 | P |
| | ISA51VG | i.d. | | 50% | 0.1 mL | 3 | P |
| | cGAMP | i.d. | 0.01 m g | 0.1 mg/ mL | 0.1 mL | 4 | P |
| | | | | | | | |
| Exp. 10 | PBS DMSO5% | i.d. | 0 m g | 0 mg/ mL | 0.1 mL | 1 | P |
| | MPLA | i.d. | 0.01 mg | 0.1 mg/ mL | 0.1 mL | 2 | P |
| | MALP2s | i.d. | 0.01 mg | 0.1 mg/ mL | 0.1 mL | 3 | P |
| | R848 | i.d. | 0.1 mg | 1 mg/ mL | 0.1 mL | 4 | P |
| | DMXAA | i.d. | 0.1 mg | 1 mg/ mL | 0.1 mL | 5 | P |

Table 15 Number of gene probes with statistically significant changes after adjuvant administration. The significantly changed genes, which are described as "significantly differentially expressed genes" (sDEGs), are defined by the following criteria: fold change >1.5 (up) or < 0.667 (down) with p-value of < 0.01 and customized PA call = 1. Liver is indicated as LV. Spleen is indicated as SP. Lymph node is indicated as LN. Blanks indicate that the samples were not examined.

[Table 15]

| | | | AddaVax | ADX | ALM | bCD | cdiGMP | cGAMP | D35 | DMXAA | ENDCN* | FCA | FK565 | ISA51VG | K3 | K3SPG | MALP2s | MBT | MPLA | Pam3CSK4 | PolyIC | R848 | sHz |
|---|---|---|---|---|---|---|---|---|---|---|---|---|---|---|---|---|---|---|---|---|---|---|---|
| i.d. i.n.* | LV | Up | 29 | 11 | 17 | 107 | 280 | 841 | 15 | 162 | 100 | 198 | 2094 | 17 | 10 | 19 | 628 | 14 | 9 | 114 | 516 | 702 | 12 |
| | | Down | 18 | 14 | 15 | 315 | 218 | 248 | 36 | 101 | 24 | 139 | 1972 | 27 | 22 | 81 | 1248 | 28 | 24 | 51 | 1122 | 1462 | 8 |
| | SP | Up | 16 | 14 | 13 | 111 | 707 | 680 | 23 | 121 | 14 | 135 | 1672 | 9 | 11 | 12 | 774 | 34 | 9 | 200 | 1360 | 3879 | 26 |
| | | Down | 13 | 13 | 8 | 99 | 261 | 93 | 19 | 48 | 13 | 39 | 624 | 18 | 18 | 59 | 311 | 25 | 22 | 50 | 738 | 1313 | 12 |
| | LN | Up | 61 | 25 | 38 | 89 | 1579 | 1135 | 69 | 1342 | | 640 | 526 | 16 | 53 | 57 | 1895 | 19 | 8 | 23 | 2203 | 3091 | 8 |
| | | Down | 73 | 25 | 16 | 110 | 908 | 256 | 12 | 261 | | 284 | 306 | 12 | 18 | 99 | 718 | 13 | 18 | 27 | 1460 | 1193 | 5 |
| i.p. | LV | Up | | 160 | 198 | 14 | | | | | | | | | 18 | 20 | | | | | | | |
| | | Down | | 88 | 552 | 20 | | | | | | | | | 50 | 33 | | | | | | | |
| | SP | Up | | 346 | 136 | 4 | | | | | | | | | 24 | 20 | | | | | | | |
| | | Down | | 129 | 21 | 5 | | | | | | | | | 10 | 14 | | | | | | | |

Table 16 Identification information of genes mentioned in this Example

[Table 16-1]

| Gene name | Gene ID: | NCBI reference number |
|---|---|---|
| Gm 14446 | 667373 | NM_001101 605.1;NM_001110517.1 |
| Pml | 18854 | NM_001311088.1;NM_008884.5;NM_178087.4 |
| H2-T22 | 15039 | NM_010397.4 |

(continued)

| Gene name | Gene ID: | NCBI reference number |
|---|---|---|
| Ifit1 | 15957 | NM_008331.3 |
| IrF7 | 54123 | NM_001252600.1;NM_001252601.1;NM_016850.3 |
| Isg15 | 1E+08 | NM_015783.3 |
| Stat1 | 20846 | NM_001205313.1:NM_001205314.1:NM_009283.4 |
| Fcgr1 | 14129 | NM_010186.5 |
| Oasla | 246730 | NM_145211.2 |
| Oas2 | 246728 | NM_145227.3 |
| Trim 12a | 76681 | NM_023835.2 |
| Trim 12c | 319236 | NM_001146007.1;NM_175677.4 |
| Uba7 | 74153 | NM_023738.4 |
| Ube2l6 | 56791 | NM_019949.2 |
| Elovl6 | 170439 | NM_130450.2 |
| Gpam | 14732 | NM_008149.3 |
| Hsd3b7 | 101502 | NM_001040684.1;NM_1 33943.2 |
| Acer2 | 230379 | NM_001290541.1;NM_001290543.1;NM_139306.3 |
| Acox1 | 11430 | NM_001271898.1;NM_015729.3 |
| Tbl1xr1 | 81004 | NM_030732.3 |
| Alox5ap | 11690 | NM_001308462.1;NM_009663.2 |
| GgtS | 23887 | NM_011820.4 |
| Bbc3 | 170770 | NM_133234.2 |
| Pdk4 | 27273 | NM_013743.2 |
| Cd55 | 13136 | NM_010016.3 |
| Cd93 | 17064 | NM_010740.3 |
| Clec4e | 56619 | NM_019948.2 |

[Table 16-2]

| | | |
|---|---|---|
| Coro1a | 12721 | NM_001301374.1;NM_009898.3 |
| Traf3 | 22031 | NM_001286122.1;NM_011632.3 |
| Trem3 | 58218 | NM_021407.3 |
| C5ar1 | 12273 | NM_001173550.1;NM_007577.4 |
| Clec4n | 56620 | NM_001190320.1;NM_001190321.1;NM_020001.2 |
| Ier3 | 15937 | NM_133662.2 |
| Il1r1 | 16177 | NM_001123382.1;NM_008362.2 |
| Plek | 56193 | NM_049549.2 |
| Tbx3 | 21386 | NM_011535.3;NM_198052.2 |
| Trem1 | 58217 | NM_021406.5 |
| Ccl3 | 20302 | NM_011337.2 |

(continued)

| | | |
|---|---|---|
| Myof | 226101 | NM_001099634.1;NM_001302140.1 |
| Papss2 | 23972 | NM_001201470.1;NM_011864.3 |
| Slc7a11 | 26570 | NM_011990.2 |
| Tnfrsf1b | 21938 | NM_011610.3 |
| Ak3 | 56248 | NM_021299.1 |
| Insm1 | 53626 | NM_046889.3 |
| Nek1 | 18004 | NM_001293637.1;NM_001293638.1;NM_001293639.1;NM_175089.4 |
| Pik3r2 | 18709 | NM_008841.3 |
| Ttn | 22138 | NM_011652.3;NM_028004.2 |
| Atp6v0d2 | 242341 | NM_175406.3 |
| Atp6v1c1 | 66335 | NM_025494.3 |
| Clec7a | 56644 | NM_001309637.1;NM_020008.3 |

(Results)

Adjuvant gene space - the union of sDEGs characterizes organ responses to adjuvants

A total of 21 adjuvants (Table 1) were administered to mice at the tail base (intradermally; i.d.), intraperitoneally (i.p.) or intranasally (i.n.) Six hours after adjuvant administration, whole organ transcriptomes of the LV, SP (as systemic organs) and LNs (as local lymphoid tissues) were obtained, and a set of significantly differentially expressed genes (sDEGs) for each organ-adjuvant pair was defined (Table 15). Next, the adjuvant-induced gene responses were integrated by combining all the sDEGs on a per-organ basis. The union of sDEGs from the LV, SP, and LNs resulted in a total of 8049, 8449 and 9451 gene probes, respectively (Fig. **1**). The adjuvant gene spaces included genes whose expression was significantly changed by the administration of at least one of the 21 different adjuvants examined in vivo. Overall, 3874 (48% of the LV-induced genes), 2331 (28% of the SP-induced genes), and 2991 (31% of the LNs-induced genes) genes were unique to each organ. Pathway analysis with TargetMine (Chen, Y.A. et al., PloS one 6, e17844 (2011)) showed that these unique genes were related to lipid metabolism, transcription, and the immune system in the LV, SP and LNs, respectively (Fig. **1**). The three organs shared 2299 genes with pathway enrichments related to interferons, cytokines, NF-κB, and TNF signaling (Fig. **1**).

According to the volcano plot data (Figure **S1**), half of the adjuvants (AddaVax, ADX, ALM, D35, ISA51VG, K3, K3SPG, MBT, MPLA, and sHZ) produced modest responses (< 100 sDEGs) when they were administered locally. This is consistent with the fact that the dose levels were chosen to mimic real vaccination situations. bCD, ENDCN, and Pam3CSK4 induced intermediate (100-200 sDEGs) responses. By comparison, cdiGMP, cGAMP, DMXAA, FCA, FK565, MALP2s, PolyIC, and R848 induced strong (> 500 sDEGs) gene responses in at least one organ. These adjuvants tended to elicit larger gene responses in directly draining LNs, and relatively weaker responses in systemic (LV and SP) organs (Table 15 and Figure **S1**). Interestingly, FK565, the synthetic NOD1 ligand, induced greater responses in LV and SP than in LNs after i.d. administration (Table 15 and Figure **S1**). ADX and ALM induced obvious gene expression changes in LV and SP, whereas K3, K3SPG and bCD induced relatively weaker responses (Table 15 and Figure **S1**). MPLA, Pam3CSK4, and other relatively mild adjuvants tended to exhibit varying gene responses among the individual mice and organs (Figures **8** and **9**).

As half of the adjuvants induced only limited numbers of sDEGs, it was difficult to characterize them by regular gene annotation analysis. Therefore, another gene selection criterion (fold change > 2) was also tested for its ability to extract meaningful biological annotations for each adjuvant. In fact, this analysis obtained enough and reasonable in vivo biological annotations from even a practical dose (relatively small amount) of adjuvant (Figure **10**). Almost all the adjuvants induced inflammation, cytokine/chemokine responses, chemotaxis/migration, and stress/defense/immune responses, relatively more strongly in LNs than in LV and SP after i.d. administration (Figure **10**). With the tissue damage-associated annotations (wounding, cell death, apoptosis, NFκB signaling pathway), ADX, D35, K3, K3SPG, MBT, and sHZ did not induce cell death and wounding in LNs, a finding consistent with the good local tolerability profiles of ADX and sHZ. Interferon and interleukin responses against most adjuvants (except AddaVax, ADX, ISA51VG, MBT, and sHZ) in LNs were also detected (Figure **10**). In spite of relatively strong inflammation and stress responses in LNs (Figure

**10**), ALM, K3SPG and MPLA exhibited limited responses in LV and SP, indicating that these adjuvant effects were locally restricted (Figure **10**). In contrast, with the exception of ISA51VG and sHZ, the other adjuvants appeared to cause detectable levels of systemic organ responses even after i.d. local administration (Figure **10**). Intriguingly, MBT, a NOD2 ligand, appeared to induce more gene responses in the LV than in SP and LNs after i.d. administration, a result similar to that for FK565 (Figure **10**). I.p. administration of ADX, ALM, and K3SPG induced relatively stronger inflammation- and stress-associated responses in LV and SP than i.d. administration (Figure **10**). These results indicate that this dataset without p-value selection provides considerable information about adjuvant-induced gene responses, at a level sufficient to retrieve each adjuvant-induced biological response in vivo with substantial reliability, thus supporting the whole organ transcriptome approach.

To obtain more details of the adjuvant gene spaces that clarifies adjuvant induce host responses in three organs, hierarchical clustering of genes and adjuvants was performed (Figure **11**; see also http://sysimg.ifrec.osaka-u.ac.jp/adjvdb/methodologies/adjv_space.html). This analysis found that separating approximately 10,000 gene probes for each organ into a total of 40 clusters (a few hundred genes per cluster) was suitable for obtaining biological annotations for each cluster using TargetMine. Hereafter, to discriminate the gene clusters from the adjuvant clusters discussed below, these 40 clusters for each organ are referred to as "modules" (Figure **11**). Gene Ontology (GO) enrichment analysis of these modules revealed that adjuvant administration induced a broad range of biological processes in each organ, such as immune-related processes and more basic biological cellular processes (Figure **11**). The types of cells that responded to each adjuvant were also identified by comparing the gene expression profiles with those publicly available from the ImmGen database (Heng, T.S. et al., Nature immunology 9, 1091-1094 (2008)) (https://www.immgen.org/), as described in Methods. This analysis revealed that the LV and SP responses were markedly associated with neutrophils and stromal cells (Figure **12**). In LNs, in addition to neutrophils and stromal cells, the responses were associated with macrophages (Figure **12**). Interestingly, one third of the genes in SP clustered in modules 14-19, all of which were related to RNA processing (Figure **11b**), and were strongly associated with B cells (Figure **13b**). In contrast, in LV, almost no association with T cells was observed, a finding consistent with general knowledge of relatively low T cell residency in the LV (Figure **13a**). In LNs, a greater variety of immune cell types were associated with each module. Genes in module 16 of the LNs ($M16^{LN}$), which were upregulated by Pam3CSK4, K3, K3SPG and FCA, were phagosome-related and were correspondingly associated with macrophage populations (Figure **13c**). The same "immune system process" annotation appeared three times and modules (M)$15^{LN}$, $M27^{LN}$ and $M40^{LN}$ were associated with different cell types. $M15^{LN}$ (weakly induced by ALM and K3) was associated with T cells, $M27^{LN}$ (induced by most of the adjuvants) was strongly associated with neutrophils, and $M40^{LN}$ (strongly induced by cdiGMP, cGAMP, DMXAA, PolyIC, and R848) was associated with a broader range of immune cells including dendritic cells, macrophages, neutrophils, and stromal cells (Figure **13c**). These data show that the adjuvant gene space approach can provide multilayered information, including each adjuvant's biological properties and the responses of different cell types to the adjuvants.

Classification of adjuvants into 6 groups within the adjuvant gene space

Adjuvants have been categorized according to their stimulating properties (e.g., PAMPs or DAMPs) or their physicochemical features (e.g., solute, particles, or emulsions). The qualitative differences of these descriptors make it difficult to categorize adjuvants without bias. Therefore, the hierarchical clustering results were utilized to categorize the adjuvants. The cluster analysis showed an interesting and insightful grouping within the adjuvant gene space for each organ. Although each organ had a characteristic gene profile (Figure **1**), rather consistent adjuvant groupings was observed among the LV, SP, and LNs (Figure **2** (A to D) and Figure **14**). In the cluster trees, most of the three replicates of the same adjuvant were closely connected (forming the first and the second dendrogram nodes), indicating that individual mice administered with the same adjuvant exhibited similar gene expression changes (Figure **14**). Some interesting exceptions were D35_ID_x2 and K3_ID_x3 in LN (Figure **14a**). At a higher clustering threshold level (cutoff height = 1.0), four major groups were formed in LV (Figure **14a**), and similarly in SP (Figure **14b**), excluding batch effects (Leek, J.T. et al., Nat Rev Genet 11, 733-739 (2010)). In LNs, the cutoff height of 1.0 gave 10 groups (Figure **14c**), indicating that LNs responded to each adjuvant more strongly and diversely than did LV or the SP (Figure **14**). The cutoff height of 1.5 for LNs gave a total of five groups excluding the batch effect (Figure **14c**). This cutoff setting also gave more comparable groupings to those of LV and SP (Figure **14**).

The majority of the clusters were consistent across the organs. Five adjuvants (cdiGMP, cGAMP, DMXAA, PolyIC, and R848) were fixed to a single cluster in LV, SP, and LNs, which are denoted as group (G) 1 (Figure **2** (**A** to **D**) and Figure **14**). From a similar perspective, it was found that bCD, FK565, and MALP2s served as references for G2, G3, and G4, respectively, because they constantly appeared in a separate cluster (Figure **2** (**A** to **D**) and Figure **14**). In LNs, two additional groups, G5 and G6, were observed (Figure **2** (**A** to **D**) and Figure **14c**).

When similar transcriptome analysis was performed on rats, the results shown in Figures **2K** to **2N** were obtained. The results are nearly the same as the results for mice, Figures **2A** to **2D**, showing that transcriptome analysis is also useful for grouping of adjuvants in rats.

(Each adjuvant group associated with characteristic biological responses)

To characterize further each adjuvant group at the gene level, genes that were preferentially upregulated in each adjuvant group (G1 to G6) compared with the other groups were first identified by converting their expression fold-change values into z-scores (Table 17 (partially excerpted) and Figure **15**).

[Figure 17-1]

| Term (Keywords) | GO annotation | AddaVax.LV.ID | ADX.LV.ID | ALM.LV.ID | bCD.LV.ID | cdiGMP.LV.ID | cGAMP.LV.ID | D35.LV.ID | DMXAA.LV.ID | ENDCN.LV.IN | FCA.LV.ID | FK565.LV.ID | ISA51VG.LV.ID | K3.LV.ID | K3SPG.LV.ID | MALP2s.LV.ID | MBT.LV.ID | MPLA.LV.ID | Pam3CSK4.LV.ID | Poly_IC.LV.ID | R848.LV.ID | sHz.LV.ID |
|---|---|---|---|---|---|---|---|---|---|---|---|---|---|---|---|---|---|---|---|---|---|---|
| wounding | regulation of response to wounding | 0 | 0 | 0 | 6.31 | 10.7 | 3.31 | 0 | 3.49 | 0 | 3.82 | 12.1 | 0 | 0 | 0 | 9.89 | 0 | 0 | 11.1 | 16.7 | 11.1 | 0 |
| wounding | response to wounding | 0 | 0 | 0 | 5.85 | 11.4 | 0 | 0 | 0 | 0 | 5.68 | 12.8 | 0 | 0 | 0 | 10.3 | 0 | 0 | 12.2 | 14.8 | 9.74 | 0 |
| wounding | positive regulation of response to wounding | 0 | 0 | 0 | 0 | 0 | 0 | 0 | 0 | 0 | 0 | 9.45 | 0 | 0 | 0 | 0 | 0 | 0 | 3.54 | 9.8 | 4.53 | 0 |
| cell death | cell death | 5.38 | 5.36 | 0 | 5.8 | 17.7 | 10.2 | 5.7 | 6.04 | 13.7 | 14.9 | 21.7 | 0 | 18.3 | 0 | 17.5 | 0 | 0 | 20.3 | 22.6 | 23.1 | 3.62 |
| cell death | death | 0 | 0 | 0 | 0 | 12.1 | 4.77 | 0 | 0 | 8.85 | 11.7 | 16.4 | 0 | 13.9 | 0 | 12.6 | 0 | 0 | 14.9 | 17.8 | 18.3 | 0 |
| cell death | programmed cell death | 0 | 0 | 0 | 0 | 14 | 6.49 | 0 | 0 | 6.75 | 8.34 | 15.7 | 0 | 14.9 | 0 | 13.3 | 0 | 0 | 15.2 | 18.1 | 19.4 | 0 |
| cell death | regulation of cell death | 0 | 0 | 0 | 0 | 4.48 | 3.82 | 0 | 0 | 9.15 | 5.76 | 14 | 0 | 10.5 | 0 | 9.13 | 0 | 0 | 6.86 | 15.4 | 15.5 | 0 |
| cell death | regulation of programmed cell death | 0 | 0 | 0 | 0 | 5.36 | 4.93 | 0 | 0 | 6.22 | 5.11 | 12.9 | 0 | 10.9 | 0 | 8.86 | 0 | 0 | 6.45 | 15.6 | 15.5 | 0 |
| cell death | positive regulation of programmed cell death | 0 | 0 | 0 | 0 | 0 | 0 | 3.05 | 0 | 3.68 | 0 | 7.1 | 0 | 7.25 | 0 | 0 | 0 | 0 | 0 | 4.25 | 4.66 | 0 |
| cell death | positive regulation of cell death | 0 | 0 | 0 | 0 | 0 | 0 | 0 | 0 | 4.51 | 0 | 7.29 | 0 | 6.67 | 0 | 0 | 0 | 0 | 0 | 3.46 | 4.64 | 0 |
| cell death | negative regulation of cell death | 0 | 0 | 0 | 0 | 0 | 0 | 0 | 0 | 0 | 0 | 0 | 0 | 0 | 0 | 0 | 0 | 0 | 0 | 4.38 | 0 | 0 |
| cell death | negative regulation of programmed cell death | 0 | 0 | 0 | 0 | 0 | 0 | 0 | 0 | 0 | 0 | 0 | 0 | 0 | 0 | 0 | 0 | 0 | 0 | 4.89 | 0 | 0 |
| apoptosis | apoptotic process | 0 | 0 | 0 | 0 | 12.1 | 4.46 | 0 | 0 | 7.32 | 9.08 | 15.2 | 0 | 15.7 | 0 | 12.4 | 0 | 0 | 15 | 15.7 | 18.6 | 0 |
| apoptosis | regulation of apoptotic process | 0 | 0 | 0 | 0 | 4.93 | 4.63 | 0 | 0 | 5.49 | 5.44 | 13 | 0 | 10.1 | 0 | 8.76 | 0 | 0 | 6.84 | 14.8 | 15.5 | 0 |
| apoptosis | apoptotic signaling pathway | 0 | 0 | 0 | 0 | 0 | 0 | 0 | 0 | 3.18 | 4.48 | 19.9 | 0 | 10.3 | 0 | 9.05 | 0 | 0 | 3.68 | 12.4 | 10.6 | 0 |
| apoptosis | intrinsic apoptotic signaling pathway | 0 | 0 | 0 | 0 | 0 | 0 | 0 | 0 | 4.34 | 0 | 13.9 | 0 | 0 | 0 | 5.47 | 0 | 0 | 0 | 7.32 | 5.18 | 0 |
| apoptosis | positive regulation of apoptotic process | 0 | 0 | 0 | 0 | 0 | 0 | 3.18 | 0 | 0 | 0 | 6.58 | 0 | 5.99 | 0 | 0 | 0 | 0 | 0 | 3.59 | 4.1 | 0 |
| apoptosis | regulation of apoptotic signaling pathway | 0 | 0 | 0 | 0 | 0 | 0 | 0 | 0 | 0 | 0 | 13.2 | 0 | 3.19 | 0 | 0 | 0 | 0 | 0 | 7.04 | 5.48 | 0 |
| apoptosis | negative regulation of apoptotic process | 0 | 0 | 0 | 0 | 0 | 0 | 0 | 0 | 0 | 0 | 0 | 0 | 0 | 0 | 0 | 0 | 0 | 0 | 4.79 | 0 | 0 |
| apoptosis | regulation of intrinsic apoptotic signaling pathway | 0 | 0 | 0 | 0 | 0 | 0 | 0 | 0 | 0 | 0 | 8.33 | 0 | 0 | 0 | 0 | 0 | 0 | 0 | 0 | 0 | 0 |
| NF-kappa B signaling pathway | NF-kappa B signaling pathway | 0 | 11.9 | 0 | 6.86 | 15.7 | 4.86 | 5.27 | 3.77 | 0 | 10.8 | 19.8 | 0 | 6.19 | 0 | 8.36 | 6.87 | 0 | 29.6 | 18 | 8.86 | 0 |
| NF-kappa B signaling pathway | I-kappaB kinase/NF-kappaB signaling | 0 | 3.98 | 0 | 4.21 | 13.8 | 0 | 0 | 0 | 0 | 8.18 | 12.9 | 0 | 0 | 0 | 3.13 | 6.14 | 0 | 16.9 | 13.5 | 7.93 | 0 |
| NF-kappa B signaling pathway | positive regulation of I-kappaB kinase/NF-kappaB signaling | 0 | 0 | 0 | 0 | 7.15 | 0 | 0 | 0 | 0 | 3.69 | 3.05 | 0 | 0 | 0 | 0 | 0 | 0 | 0 | 8.57 | 5.3 | 0 |
| NF-kappa B signaling pathway | regulation of I-kappaB kinase/NF-kappaB signaling | 0 | 0 | 0 | 0 | 8.16 | 0 | 0 | 0 | 0 | 0 | 5.82 | 0 | 0 | 0 | 0 | 0 | 0 | 5.45 | 9.04 | 5.28 | 0 |
| inflammatory response | inflammatory response | 7.12 | 12.6 | 0 | 10.9 | 26.1 | 11.5 | 0 | 0 | 0 | 19 | 29.8 | 0 | 0 | 0 | 23.4 | 0 | 0 | 38 | 32.2 | 22.2 | 0 |
| inflammatory response | regulation of inflammatory response | 0 | 0 | 0 | 7.33 | 12.3 | 0 | 0 | 0 | 0 | 5.87 | 11.4 | 0 | 0 | 0 | 9.25 | 0 | 0 | 13.6 | 14.4 | 6.29 | 0 |
| inflammatory response | positive regulation of inflammatory response | 0 | 0 | 0 | 0 | 0 | 0 | 0 | 0 | 0 | 0 | 3.69 | 0 | 0 | 0 | 0 | 0 | 0 | 5.39 | 5.99 | 0 | 0 |
| inflammatory response | acute inflammatory response | 0 | 0 | 0 | 0 | 0 | 0 | 0 | 0 | 0 | 0 | 0 | 0 | 0 | 0 | 0 | 0 | 0 | 5.25 | 0 | 0 | 0 |
| inflammatory response | leukocyte migration involved in inflammatory response | 0 | 0 | 0 | 0 | 0 | 0 | 0 | 0 | 0 | 0 | 0 | 0 | 0 | 0 | 0 | 0 | 0 | 3.41 | 0 | 0 | 0 |
| TNF signaling pathway | TNF signaling pathway | 5.94 | 4.51 | 0 | 10.6 | 31.3 | 27.6 | 12.1 | 10.7 | 8.38 | 14 | 30.9 | 3.76 | 14.5 | 0 | 18.7 | 6.37 | 3.93 | 35.8 | 22.1 | 16.1 | 0 |
| cytokine | response to cytokine | 0 | 0 | 9.11 | 7.77 | 56.3 | 45.9 | 8.12 | 26.5 | 7.7 | 12.8 | 37.8 | 0 | 0 | 0 | 34.9 | 3.82 | 0 | 46.5 | 66.1 | 44.8 | 4.53 |
| cytokine | Cytokine-cytokine receptor interaction\|Endocytosis | 0 | 9.49 | 0 | 7.76 | 33.9 | 24.6 | 5.2 | 15.4 | 0 | 5.33 | 32.6 | 0 | 0 | 0 | 12.8 | 6.94 | 0 | 25.9 | 48.5 | 30.5 | 0 |
| cytokine | cellular response to cytokine stimulus | 0 | 3.74 | 9.43 | 0 | 38.8 | 32.9 | 3.28 | 16.7 | 0 | 3.22 | 23.6 | 0 | 0 | 0 | 24.8 | 0 | 0 | 31.5 | 42.3 | 30.4 | 0 |
| cytokine | Cytokine-cytokine receptor interaction | 0 | 5.61 | 0 | 4.24 | 13.8 | 5.57 | 0 | 0 | 0 | 5.96 | 17.1 | 0 | 0 | 0 | 8.95 | 3.53 | 0 | 13.6 | 12.1 | 11.1 | 0 |
| cytokine | cytokine production | 0 | 5.88 | 0 | 3.82 | 31.3 | 17.4 | 0 | 6.98 | 0 | 10.7 | 30.2 | 0 | 0 | 0 | 20.9 | 0 | 0 | 32.6 | 45.6 | 30.4 | 0 |
| cytokine | regulation of cytokine production | 0 | 5.41 | 0 | 4.06 | 31.6 | 19.1 | 0 | 8.37 | 0 | 5.96 | 27.5 | 0 | 0 | 0 | 15.5 | 0 | 0 | 29.8 | 43.5 | 26.5 | 0 |
| cytokine | cytokine-mediated signaling pathway | 0 | 0 | 7.05 | 0 | 20 | 16.4 | 0 | 0 | 4.84 | 0 | 8.69 | 0 | 0 | 0 | 11.3 | 0 | 0 | 17 | 20.9 | 16.5 | 0 |
| cytokine | cytokine biosynthetic process | 0 | 0 | 0 | 0 | 7.93 | 6.84 | 0 | 0 | 0 | 7.49 | 16.2 | 0 | 0 | 0 | 6.41 | 0 | 0 | 19.7 | 7.3 | 5.44 | 0 |
| cytokine | cytokine metabolic process | 0 | 0 | 0 | 0 | 7.39 | 8.21 | 0 | 0 | 0 | 9.31 | 17 | 0 | 0 | 0 | 7.38 | 0 | 0 | 21.6 | 8.4 | 4.8 | 0 |
| cytokine | positive regulation of cytokine production | 0 | 0 | 0 | 0 | 23.6 | 11.1 | 0 | 7.86 | 0 | 0 | 18.6 | 0 | 0 | 0 | 6.74 | 0 | 0 | 21.5 | 34.3 | 20.3 | 0 |
| cytokine | negative regulation of cytokine production | 0 | 0 | 0 | 0 | 6.2 | 3.21 | 0 | 0 | 0 | 0 | 3.46 | 0 | 0 | 0 | 4.78 | 0 | 0 | 5.39 | 12.6 | 5.01 | 0 |
| cytokine | regulation of cytokine biosynthetic process | 0 | 0 | 0 | 0 | 3.81 | 0 | 0 | 0 | 0 | 4.48 | 14 | 0 | 0 | 0 | 5.33 | 0 | 0 | 17 | 4.18 | 0 | 0 |
| cytokine | regulation of tumor necrosis factor superfamily cytokine production | 0 | 0 | 0 | 0 | 4.03 | 0 | 0 | 0 | 0 | 0 | 7.57 | 0 | 0 | 0 | 4.82 | 0 | 0 | 11.6 | 15 | 5.55 | 0 |
| cytokine | tumor necrosis factor superfamily cytokine production | 0 | 0 | 0 | 0 | 3.79 | 0 | 0 | 0 | 0 | 0 | 7.13 | 0 | 0 | 0 | 4.49 | 0 | 0 | 11.3 | 14.5 | 6.84 | 0 |

[Table 17-2]

| Term (Keywords) | GO annotation | AddaVax.LV.ID | ADX.LV.ID | ALM.LV.ID | bCD.LV.ID | cdiGMP.LV.ID | cGAMP.LV.ID | D35.LV.ID | DMXAA.LV.ID | ENDCN.LV.IN | FCA.LV.ID | FK565.LV.ID | ISA51VG.LV.ID | K3.LV.ID | K3SPG.LV.ID | MALP2s.LV.ID | MBT.LV.ID | MPLA.LV.ID | Pam3CSK4.LV.ID | Poly_IC.LV.ID | R848.LV.ID | sHz.LV.ID |
|---|---|---|---|---|---|---|---|---|---|---|---|---|---|---|---|---|---|---|---|---|---|---|
| cytokine | positive regulation of cytokine biosynthetic process | 0 | 0 | 0 | 0 | 3.62 | 0 | 0 | 0 | 0 | 0 | 10.1 | 0 | 0 | 0 | 0 | 0 | 0 | 14.5 | 3.4 | 0 | 0 |
| cytokine | cytokine secretion | 0 | 0 | 0 | 0 | 0 | 0 | 0 | 0 | 0 | 0 | 0 | 0 | 0 | 0 | 5.26 | 0 | 0 | 0 | 3.66 | 0 | 0 |
| cytokine | positive regulation of tumor necrosis factor superfamily cytokine production | 0 | 0 | 0 | 0 | 0 | 0 | 0 | 0 | 0 | 0 | 0 | 0 | 0 | 0 | 0 | 0 | 0 | 0 | 5.29 | 0 | 0 |
| migration | positive regulation of leukocyte migration | 0 | 12.3 | 0 | 0 | 3.33 | 0 | 0 | 0 | 0 | 0 | 13.6 | 0 | 0 | 0 | 10.3 | 4.41 | 0 | 21.1 | 22.1 | 8.03 | 0 |
| migration | cell migration | 3.44 | 4.19 | 0 | 0 | 0 | 0 | 0 | 0 | 0 | 0 | 8.98 | 0 | 0 | 0 | 0 | 4.69 | 0 | 14.7 | 6.99 | 0 | 0 |
| migration | leukocyte migration | 0 | 8.85 | 0 | 0 | 0 | 0 | 0 | 0 | 0 | 0 | 14.2 | 0 | 0 | 0 | 15.1 | 0 | 0 | 27.6 | 23.4 | 7.89 | 0 |
| migration | regulation of leukocyte migration | 0 | 9.34 | 0 | 0 | 0 | 0 | 0 | 0 | 0 | 0 | 7.86 | 0 | 0 | 0 | 7.84 | 0 | 0 | 15.4 | 18.5 | 5.8 | 0 |
| migration | neutrophil migration | 0 | 0 | 0 | 0 | 0 | 0 | 0 | 0 | 0 | 0 | 7.57 | 0 | 0 | 0 | 3.05 | 0 | 0 | 14 | 9 | 3.14 | 0 |
| migration | positive regulation of cell migration | 0 | 0 | 0 | 0 | 0 | 0 | 0 | 0 | 0 | 0 | 6.55 | 0 | 0 | 0 | 3.39 | 0 | 0 | 11.4 | 9.34 | 3.96 | 0 |
| migration | granulocyte migration | 0 | 0 | 0 | 0 | 0 | 0 | 0 | 0 | 0 | 0 | 6.19 | 0 | 0 | 0 | 0 | 0 | 0 | 14.1 | 8.14 | 3.31 | 0 |
| migration | myeloid leukocyte migration | 0 | 0 | 0 | 0 | 0 | 0 | 0 | 0 | 0 | 0 | 4.49 | 0 | 0 | 0 | 0 | 0 | 0 | 11.1 | 7.59 | 0 | 0 |
| migration | regulation of cell migration | 0 | 0 | 0 | 0 | 0 | 0 | 0 | 0 | 0 | 0 | 0 | 0 | 0 | 0 | 0 | 0 | 0 | 4.46 | 6.05 | 0 | 0 |
| migration | lymphocyte migration | 0 | 0 | 0 | 0 | 0 | 0 | 0 | 0 | 0 | 0 | 0 | 0 | 0 | 0 | 0 | 0 | 0 | 0 | 3.46 | 0 | 0 |
| chemokine | chemokine-mediated signaling pathway | 0 | 3.8 | 0 | 0 | 5.9 | 0 | 0 | 0 | 0 | 0 | 0 | 0 | 0 | 0 | 0 | 0 | 0 | 5.38 | 6.25 | 3.95 | 0 |
| chemokine | chemokine production | 0 | 0 | 0 | 0 | 8.52 | 0 | 0 | 0 | 0 | 0 | 0 | 0 | 0 | 0 | 0 | 0 | 0 | 4.48 | 7.84 | 0 | 0 |
| chemokine | regulation of chemokine production | 0 | 0 | 0 | 0 | 9.64 | 0 | 0 | 0 | 0 | 0 | 0 | 0 | 0 | 0 | 0 | 0 | 0 | 5.3 | 9.13 | 0 | 0 |
| chemokine | positive regulation of chemokine production | 0 | 0 | 0 | 0 | 8.31 | 0 | 0 | 0 | 0 | 0 | 0 | 0 | 0 | 0 | 0 | 0 | 0 | 0 | 9 | 0 | 0 |
| chemotaxis | cell chemotaxis | 7.11 | 13.1 | 0 | 5.73 | 0 | 4.4 | 4.51 | 0 | 5.77 | 5.33 | 16.1 | 0 | 0 | 0 | 11.8 | 10.2 | 3.38 | 31.4 | 20.7 | 11 | 0 |
| chemotaxis | chemotaxis | 6.22 | 4.82 | 0 | 0 | 0 | 0 | 0 | 0 | 0 | 0 | 0 | 0 | 0 | 0 | 3.46 | 5.4 | 0 | 17.6 | 6.87 | 0 | 0 |
| chemotaxis | leukocyte chemotaxis | 0 | 7.8 | 0 | 0 | 0 | 0 | 0 | 0 | 0 | 0 | 15.7 | 0 | 0 | 0 | 10.7 | 0 | 0 | 24.2 | 22 | 8.03 | 0 |
| chemotaxis | positive regulation of leukocyte chemotaxis | 0 | 4.39 | 0 | 0 | 0 | 0 | 0 | 0 | 0 | 0 | 5.48 | 0 | 0 | 0 | 7.96 | 0 | 0 | 13.8 | 13.1 | 3.91 | 0 |
| chemotaxis | taxis | 6.2 | 4.79 | 0 | 0 | 0 | 0 | 0 | 0 | 0 | 0 | 0 | 0 | 0 | 0 | 3.41 | 5.38 | 0 | 17.5 | 6.8 | 0 | 0 |
| chemotaxis | granulocyte chemotaxis | 0 | 0 | 0 | 0 | 0 | 0 | 0 | 0 | 0 | 0 | 5.83 | 0 | 0 | 0 | 3.47 | 0 | 0 | 15.3 | 7.42 | 4.44 | 0 |
| chemotaxis | neutrophil chemotaxis | 0 | 0 | 0 | 0 | 0 | 0 | 0 | 0 | 0 | 0 | 6.49 | 0 | 0 | 0 | 3.65 | 0 | 0 | 14.7 | 7.6 | 3.7 | 0 |
| chemotaxis | positive regulation of chemotaxis | 0 | 0 | 0 | 0 | 0 | 0 | 0 | 0 | 0 | 0 | 0 | 0 | 0 | 0 | 3.06 | 0 | 0 | 10.9 | 8.31 | 3.31 | 0 |
| chemotaxis | regulation of leukocyte chemotaxis | 0 | 3.26 | 0 | 0 | 0 | 0 | 0 | 0 | 0 | 0 | 0 | 0 | 0 | 0 | 5.59 | 0 | 0 | 11.3 | 9.92 | 0 | 0 |
| chemotaxis | regulation of chemotaxis | 0 | 0 | 0 | 0 | 0 | 0 | 0 | 0 | 0 | 0 | 0 | 0 | 0 | 0 | 0 | 0 | 0 | 6.79 | 6.5 | 3.62 | 0 |
| chemotaxis | lymphocyte chemotaxis | 0 | 0 | 0 | 0 | 0 | 0 | 0 | 0 | 0 | 0 | 0 | 0 | 0 | 0 | 0 | 0 | 0 | 0 | 3.15 | 0 | 0 |
| stress | response to stress | 9.84 | 13.2 | 0 | 10.4 | 56.2 | 44.6 | 9.13 | 40.4 | 17.2 | 23.7 | 57.7 | 3.9 | 12.9 | 0 | 41.9 | 3.75 | 0 | 47.4 | 58.8 | 63.8 | 7.7 |
| stress | regulation of response to stress | 0 | 0 | 0 | 0 | 42.9 | 25.3 | 0 | 21 | 0 | 0 | 28.5 | 0 | 0 | 0 | 17.4 | 0 | 0 | 17.9 | 40.8 | 45.6 | 0 |
| stress | cellular response to stress | 0 | 0 | 0 | 0 | 0 | 0 | 0 | 0 | 3.17 | 4.33 | 9.02 | 0 | 0 | 0 | 7.06 | 0 | 0 | 0 | 5.39 | 0 | 0 |
| defense response | defense response | 3.3 | 20.5 | 0 | 8.51 | 80.9 | 62.7 | 5.76 | 48.4 | 0 | 15.3 | 57.8 | 0 | 0 | 0 | 39.7 | 8.88 | 0 | 64.1 | 90.6 | 71.2 | 0 |
| defense response | regulation of defense response | 4.39 | 9.94 | 0 | 6.85 | 56.5 | 37.5 | 0 | 26.8 | 0 | 8.02 | 29.6 | 0 | 0 | 0 | 17.8 | 0 | 0 | 29.2 | 57.5 | 50.3 | 0 |
| defense response | positive regulation of defense response | 0 | 9.54 | 0 | 0 | 35.8 | 24.7 | 0 | 19 | 0 | 5.46 | 24.9 | 0 | 0 | 0 | 11 | 0 | 0 | 19.9 | 41.3 | 33.9 | 0 |
| defense response | defense response to other organism | 0 | 6.37 | 0 | 0 | 44.1 | 37 | 0 | 23.6 | 0 | 0 | 18.4 | 0 | 0 | 0 | 22.2 | 0 | 0 | 19.3 | 48.9 | 46.5 | 0 |
| defense response | defense response to bacterium | 0 | 0 | 0 | 0 | 10.5 | 8.38 | 0 | 0 | 0 | 0 | 5.98 | 0 | 0 | 0 | 7.44 | 0 | 0 | 11.4 | 15.8 | 18.6 | 0 |
| defense response | defense response to Gram-positive bacterium | 0 | 0 | 0 | 0 | 7.18 | 0 | 0 | 0 | 0 | 0 | 6.22 | 0 | 0 | 0 | 3.07 | 0 | 0 | 7.28 | 6.93 | 8.29 | 0 |
| defense response | defense response to protozoan | 0 | 0 | 0 | 0 | 5.31 | 4.37 | 0 | 3.52 | 0 | 0 | 0 | 0 | 0 | 0 | 6.08 | 0 | 0 | 0 | 4.67 | 5.54 | 0 |
| defense response | defense response to virus | 0 | 0 | 0 | 0 | 24.6 | 19.8 | 0 | 15 | 0 | 0 | 0 | 0 | 0 | 0 | 5.29 | 0 | 0 | 0 | 25.8 | 20 | 0 |
| defense response | regulation of defense response to virus | 0 | 0 | 0 | 0 | 0 | 0 | 0 | 0 | 0 | 0 | 0 | 0 | 0 | 0 | 0 | 0 | 0 | 0 | 6.75 | 6.02 | 0 |
| defense response | regulation of defense response to virus by host | 0 | 0 | 0 | 0 | 0 | 0 | 0 | 0 | 0 | 0 | 0 | 0 | 0 | 0 | 0 | 0 | 0 | 0 | 4.62 | 4.72 | 0 |
| defense response | negative regulation of defense response | 0 | 0 | 0 | 0 | 8.05 | 0 | 0 | 0 | 0 | 0 | 0 | 0 | 0 | 0 | 0 | 0 | 0 | 0 | 0 | 0 | 0 |
| immune response | immune response | 0 | 12.2 | 0 | 0 | 52.3 | 42.2 | 0 | 25.5 | 0 | 8.95 | 46.1 | 0 | 0 | 0 | 22.5 | 12.8 | 0 | 45.5 | 79.8 | 54.5 | 0 |
| immune response | positive regulation of immune response | 0 | 0 | 0 | 0 | 24.4 | 15.7 | 0 | 10.2 | 0 | 0 | 20.8 | 0 | 0 | 0 | 7.16 | 0 | 0 | 15.2 | 35.5 | 27.8 | 0 |

[Table 17-3]

| Term (Keywords) | GO annotation | AddaVax.LV.ID | ADX.LV.ID | ALM.LV.ID | bCD.LV.ID | cdiGMP.LV.ID | cGAMP.LV.ID | D35.LV.ID | DMXAA.LV.ID | ENDCN.LV.IN | FCA.LV.ID | FK565.LV.ID | ISA51VG.LV.ID | K3.LV.ID | K3SPG.LV.ID | MALP2s.LV.ID | MBT.LV.ID | MPLA.LV.ID | Pam3CSK4.LV.ID | Poly_IC.LV.ID | R848.LV.ID | sHz.LV.ID |
|---|---|---|---|---|---|---|---|---|---|---|---|---|---|---|---|---|---|---|---|---|---|---|
| immune response | regulation of immune response | 0 | 0 | 0 | 0 | 29.4 | 20.7 | 0 | 12.4 | 0 | 0 | 19.5 | 0 | 0 | 0 | 6.54 | 0 | 0 | 12.2 | 39.5 | 34.2 | 0 |
| immune response | activation of immune response | 0 | 0 | 0 | 0 | 17.9 | 12.6 | 0 | 5.67 | 0 | 0 | 11.9 | 0 | 0 | 0 | 0 | 0 | 0 | 13.2 | 23.2 | 14.6 | 0 |
| immune response | immune response-activating signal transduction | 0 | 0 | 0 | 0 | 19.3 | 14.1 | 0 | 5.98 | 0 | 0 | 9.97 | 0 | 0 | 0 | 0 | 0 | 0 | 13.2 | 20.5 | 12.6 | 0 |
| immune response | immune response-regulating signaling pathway | 0 | 0 | 0 | 0 | 23.5 | 17.6 | 0 | 8.39 | 0 | 0 | 12.1 | 0 | 0 | 0 | 0 | 0 | 0 | 14.3 | 23.7 | 15.1 | 0 |
| immune response | negative regulation of immune response | 0 | 0 | 0 | 0 | 6.54 | 0 | 0 | 0 | 0 | 0 | 0 | 0 | 0 | 0 | 0 | 0 | 0 | 0 | 3.8 | 0 | 0 |
| immune response | production of molecular mediator of immune response | 0 | 0 | 0 | 0 | 0 | 0 | 0 | 0 | 0 | 0 | 0 | 0 | 0 | 0 | 0 | 0 | 0 | 0 | 4.58 | 0 | 0 |
| innate immune response | innate immune response | 0 | 4.13 | 0 | 0 | 40.7 | 31.4 | 0 | 34.9 | 0 | 0 | 33.7 | 0 | 0 | 0 | 13.1 | 6.73 | 0 | 24.9 | 59.5 | 49.6 | 0 |
| innate immune response | regulation of innate immune response | 0 | 3.19 | 0 | 0 | 33.4 | 24.8 | 0 | 19.8 | 0 | 0 | 19.7 | 0 | 0 | 0 | 7.53 | 0 | 0 | 12.9 | 40.7 | 37.9 | 0 |
| innate immune response | positive regulation of innate immune response | 0 | 0 | 0 | 0 | 22.3 | 17.2 | 0 | 12.4 | 0 | 0 | 16.7 | 0 | 0 | 0 | 3.39 | 0 | 0 | 11.3 | 27.7 | 22.9 | 0 |
| innate immune response | activation of innate immune response | 0 | 0 | 0 | 0 | 21.2 | 16.9 | 0 | 9.24 | 0 | 0 | 16.2 | 0 | 0 | 0 | 0 | 0 | 0 | 10.3 | 23.3 | 17.9 | 0 |
| innate immune response | innate immune response-activating signal transduction | 0 | 0 | 0 | 0 | 20.3 | 16.2 | 0 | 7.99 | 0 | 0 | 14.3 | 0 | 0 | 0 | 0 | 0 | 0 | 11.2 | 18.5 | 13.8 | 0 |
| innate immune response | negative regulation of innate immune response | 0 | 0 | 0 | 0 | 10.4 | 0 | 0 | 0 | 0 | 0 | 0 | 0 | 0 | 0 | 0 | 0 | 0 | 0 | 7.74 | 8.91 | 0 |
| adaptive immune response | adaptive immune response based on somatic recombination of immune receptors built from immunoglobulin superfamily domains | 0 | 0 | 0 | 0 | 10.6 | 3.78 | 0 | 0 | 0 | 0 | 8.99 | 0 | 0 | 0 | 0 | 0 | 0 | 10.5 | 11.9 | 4.42 | 0 |
| adaptive immune response | adaptive immune response | 0 | 0 | 0 | 0 | 10 | 0 | 0 | 0 | 0 | 0 | 8.71 | 0 | 0 | 0 | 0 | 0 | 0 | 11.7 | 13 | 6.23 | 0 |
| adaptive immune response | positive regulation of adaptive immune response | 0 | 0 | 0 | 0 | 0 | 0 | 0 | 0 | 0 | 0 | 3.56 | 0 | 0 | 0 | 0 | 0 | 0 | 0 | 7.16 | 4.06 | 0 |
| adaptive immune response | regulation of adaptive immune response | 0 | 0 | 0 | 0 | 8.01 | 0 | 0 | 0 | 0 | 0 | 0 | 0 | 0 | 0 | 0 | 0 | 0 | 0 | 6.84 | 0 | 0 |
| adaptive immune response | regulation of adaptive immune response based on somatic recombination of immune receptors built from immunoglobulin superfamily domains | 0 | 0 | 0 | 0 | 3.64 | 0 | 0 | 0 | 0 | 0 | 0 | 0 | 0 | 0 | 0 | 0 | 0 | 0 | 0 | 0 | 0 |
| IFN alpha | response to interferon-alpha | 0 | 0 | 0 | 0 | 12.6 | 14.8 | 0 | 11.3 | 0 | 0 | 4.54 | 0 | 0 | 0 | 0 | 0 | 0 | 0 | 13.3 | 10.2 | 0 |
| IFN alpha | interferon-alpha production | 0 | 0 | 0 | 0 | 7.69 | 3.79 | 0 | 6.81 | 0 | 0 | 0 | 0 | 0 | 0 | 0 | 0 | 0 | 0 | 4.7 | 3.15 | 0 |
| IFN alpha | cellular response to interferon-alpha | 0 | 0 | 0 | 0 | 7.42 | 6.72 | 0 | 9.3 | 0 | 0 | 0 | 0 | 0 | 0 | 0 | 0 | 0 | 0 | 4.95 | 0 | 0 |
| IFN alpha | positive regulation of interferon-alpha production | 0 | 0 | 0 | 0 | 8.97 | 4.81 | 0 | 7.85 | 0 | 0 | 0 | 0 | 0 | 0 | 0 | 0 | 0 | 0 | 5.95 | 0 | 0 |
| IFN alpha | regulation of interferon-alpha production | 0 | 0 | 0 | 0 | 8.3 | 4.28 | 0 | 7.3 | 0 | 0 | 0 | 0 | 0 | 0 | 0 | 0 | 0 | 0 | 5.3 | 0 | 0 |
| IFN beta | cellular response to interferon-beta | 0 | 0 | 0 | 0 | 23.1 | 17.6 | 0 | 16.7 | 0 | 0 | 9.65 | 0 | 0 | 0 | 15.2 | 0 | 0 | 7.13 | 17.2 | 17.9 | 0 |
| IFN beta | response to interferon-beta | 0 | 0 | 0 | 0 | 26.5 | 23.5 | 0 | 17.9 | 0 | 0 | 17.3 | 0 | 0 | 0 | 23.8 | 0 | 0 | 11.6 | 23 | 23.9 | 0 |
| IFN beta | positive regulation of interferon-beta production | 0 | 0 | 0 | 0 | 13.2 | 9.2 | 0 | 5.66 | 0 | 0 | 0 | 0 | 0 | 0 | 0 | 0 | 0 | 0 | 8.94 | 6.66 | 0 |
| IFN beta | regulation of interferon-beta production | 0 | 0 | 0 | 0 | 9.22 | 5.76 | 0 | 3.09 | 0 | 0 | 0 | 0 | 0 | 0 | 0 | 0 | 0 | 0 | 7.54 | 5.68 | 0 |
| IFN beta | interferon-beta production | 0 | 0 | 0 | 0 | 8.61 | 5.22 | 0 | 0 | 0 | 0 | 0 | 0 | 0 | 0 | 0 | 0 | 0 | 0 | 9.35 | 7.51 | 0 |
| IFN gamma | response to interferon-gamma | 0 | 0 | 0 | 0 | 10.8 | 10 | 0 | 10.3 | 0 | 0 | 11 | 0 | 0 | 0 | 13.3 | 0 | 0 | 3.17 | 22.3 | 15.7 | 0 |
| IFN gamma | cellular response to interferon-gamma | 0 | 0 | 0 | 0 | 10.1 | 7.44 | 0 | 7.37 | 0 | 0 | 0 | 0 | 0 | 0 | 8.03 | 0 | 0 | 0 | 10.5 | 11.2 | 0 |
| IFN gamma | interferon-gamma production | 0 | 0 | 0 | 0 | 5.35 | 0 | 0 | 0 | 0 | 0 | 0 | 0 | 0 | 0 | 0 | 0 | 0 | 0 | 5.43 | 0 | 0 |
| IFN gamma | regulation of interferon-gamma production | 0 | 0 | 0 | 0 | 0 | 0 | 0 | 0 | 0 | 0 | 0 | 0 | 0 | 0 | 0 | 0 | 0 | 0 | 5.32 | 0 | 0 |
| interleukin-6 | interleukin-6 production | 0 | 0 | 0 | 0 | 7.8 | 0 | 0 | 0 | 0 | 0 | 3.6 | 0 | 0 | 0 | 5.33 | 0 | 0 | 7.44 | 9.62 | 7.8 | 0 |
| interleukin-6 | regulation of interleukin-6 production | 0 | 0 | 0 | 0 | 8.27 | 0 | 0 | 0 | 0 | 0 | 4.17 | 0 | 0 | 0 | 5.87 | 0 | 0 | 7.82 | 10.2 | 6.67 | 0 |
| interleukin-6 | positive regulation of interleukin-6 production | 0 | 0 | 0 | 0 | 0 | 0 | 0 | 0 | 0 | 0 | 0 | 0 | 0 | 0 | 0 | 0 | 0 | 6.14 | 5.06 | 4.53 | 0 |
| interleukin-12 | interleukin-12 production | 0 | 0 | 0 | 0 | 6.51 | 0 | 0 | 0 | 0 | 0 | 8.76 | 0 | 0 | 0 | 0 | 0 | 0 | 9.58 | 6.82 | 0 | 0 |
| interleukin-12 | regulation of interleukin-12 production | 0 | 0 | 0 | 0 | 6.99 | 0 | 0 | 0 | 0 | 0 | 9.49 | 0 | 0 | 0 | 0 | 0 | 0 | 10.1 | 7.39 | 0 | 0 |
| interleukin-12 | positive regulation of interleukin-12 production | 0 | 0 | 0 | 0 | 0 | 0 | 0 | 0 | 0 | 0 | 3.24 | 0 | 0 | 0 | 0 | 0 | 0 | 0 | 4.28 | 0 | 0 |

In the 40 modules of the adjuvant gene space, high z-scored genes in G1 from LV and SP were almost exclusively found in the modules of M20$^{LV}$ (annotated as "response to biotic stimulus") and M23$^{SP}$ (annotated as "response to virus"), respectively (Figures **15** and **16**). Similarly, in LNs, the genes preferentially upregulated in the 5 reference adjuvants of G1 (cdiGMP, cGAMP, DMXAA, PolyIC, and R848) were found in the M40$^{LN}$ module (annotated as "immune system process") (Figures **15** and **16**). The associated genes from the other groups (G2, G3, G4, G5, and G6) were distributed in several modules and clearly differed from the G1-associated modules (Figure **16**). TargetMine (Table 18)

and Ingenuity Pathways Analysis™ (IPA) upstream cytokine (Table 19) analyses provided further details of the adjuvant group-associated biological features.

[Table 18-1]

| Organ | group | Term | Pvalue | Genelist |
|---|---|---|---|---|
| LV | G1 | Interferon Signaling | 1E-08 | Uba7,Irf7,Irf9,Stat1,Trim12a,Trim12c,Trim30d,Pml,Oas1c,Ifit1 |
| LV | G1 | Interferon gamma signaling | 6.4E-07 | Irf7,Irf9,Stat1,Trim12a,Trim12c,Trim30d,Pml,Oas1c |
| LV | G1 | response to interferon-beta | 1.7E-06 | Iigp1,Xaf1,Stat1,Igtp,Ifit3,Ifit1 |
| LV | G1 | Influenza A | 2E-06 | Irf7,Irf9,Eif2ak2,Stat1,Adar,Ifih1,Ddx58,Pml,Rsad2 |
| LV | G1 | response to cytokine | 3.1E-06 | Gbp7,Iigp1,Xaf1,Irf7,Eif2ak2,Stat1,Adar,Parp9,Igtp,Pml,Ifit3,Ifit1 |
| LV | G1 | Measles | 8.6E-06 | Ccnd2,Irf7,Irf9,Eif2ak2,Stat1,Adar,Ifih1,Ddx58 |
| LV | G1 | Herpes simplex infection | 1.3E-05 | Irf7,Irf9,Eif2ak2,Stat1,Ifih1,H2-T10,Ddx58,Pml,Ifit1 |
| LV | G1 | cellular response to interferon-beta | 4.6E-05 | Iigp1,Stat1,Igtp,Ifit3,Ifit1 |
| LV | G1 | response to other organism | 6.6E-05 | Gbp7,Iigp1,Eif2ak2,Stat1,Adar,Ifih1,Ddx58,Dhx58,Cd47,Pml,Ifit3,Rsad2,Ifit1 |
| LV | G1 | response to stress | 9.4E-05 | Gbp7,Ascc3,Iigp1,Pgap2,Irf7,Zfyve26,Eif2ak2,Shisa5,Stat1,Adar,Trim12c,Ifih1,Irgm1,Ddx58,Parp9,Dhx58,Cd47,Pml,Rsad2 |
| LV | G1 | response to virus | 0.0003 | Eif2ak2,Adar,Ifih1,Ddx58,Dhx58,Pml,Ifit3,Rsad2,Ifit1 |
| LV | G1 | innate immune response | 0.00037 | Gbp7,Irf7,Stat1,Adar,Trim12c,Ifih1,Ddx58,Parp9,Dhx58,Pml |
| LV | G1 | cellular response to cytokine stimulus | 0.00108 | Gbp7,Iigp1,Irf7,Stat1,Adar,Igtp,Pml,Ifit3,Ifit1 |
| LV | G1 | defense response | 0.0013 | Gbp7,Iigp1,Irf7,Stat1,Adar,Trim12c,Ifih1,Irgm1,Ddx58,Parp9,Dhx58,Cd47,Pml,Rsad2 |
| LV | G1 | symbiosis, encompassing mutualism through parasitism | 0.00239 | Gbp7,Eif2ak2,Stat1,Adar,Pml,Rsad2 |
| LV | G1 | Hepatitis C | 0.00472 | Irf7,Irf9,Eif2ak2,Stat1,Ddx58,Ifit1 |
| LV | G1 | type I interferon production | 0.00553 | Irf7,Irf9,Ifih1,Ddx58,Dhx58 |
| LV | G1 | regulation of innate immune response | 0.00809 | Irf7,Adar,Trim12c,Ddx58,Parp9,Dhx58,Rsad2 |
| LV | G1 | Immune System | 0.00897 | Uba7,Irf7,Irf9,Stat1,Trim12a,Trim12c,Ifih1,Trim30d,Ddx58,Mndal,Pml,Oas1c,Ifit1 |
| LV | G1 | Innate Immune System\|Adaptive Immune System\|Hemostasis\|Pathways in cancer\|Developmental Biology\|PI3K-Akt signaling pathway\|Signaling by Rho GTPases | 0.0118 | Ccnd2,Uba7,Irf7,Irf9,Eif2ak2,Stat1,Trim12a,Adar,Trim12c,Ifih1,H2-T10,Trim30d,Irgm1,Ddx58,Lama3,Dhx58,Cd47,Mndal,Igtp,Pml,Oas1c,Rsad2,Ifit1 |
| LV | G1 | TRAF3-dependent IRF activation pathway | 0.01547 | Irf7,Ifih1,Ddx58 |
| LV | G1 | immune system process | 0.01883 | Gbp7,Irf7,Eif2ak2,Stat1,Adar,Trim12c,Ifih1,Ddx58,Parp9,Dhx58,Cd47,Pml,Rsad2 |
| LV | G1 | positive regulation of type I interferon production | 0.02291 | Irf7,Ifih1,Ddx58,Dhx58 |
| LV | G1 | multi-organism cellular process | 0.02396 | Eif2ak2,Stat1,Adar,Ddx58,Dhx58,Pml,Rsad2 |
| LV | G1 | negative regulation of viral process | 0.03055 | Eif2ak2,Stat1,Adar,Pml,Rsad2 |
| LV | G1 | negative regulation of multi-organism process | 0.03779 | Eif2ak2,Stat1,Adar,Dhx58,Pml,Rsad2 |
| LV | G1 | regulation of defense response | 0.05868 | Irf7,Adar,Trim12c,Ddx58,Parp9,Dhx58,Cd47,Pml,Rsad2 |
| LV | G1 | immune response | 0.06777 | Gbp7,Irf7,Stat1,Adar,Trim12c,Ifih1,Ddx58,Parp9,Dhx58,Pml,Rsad2 |
| LV | G1 | pattern recognition receptor signaling pathway | 0.08153 | Irf7,Trim12c,Ddx58,Dhx58,Rsad2 |
| LV | G1 | Antiviral mechanism by IFN-stimulated genes | 0.10767 | Uba7,Stat1,Ifit1 |
| LV | G1 | response to interferon-alpha | 0.11458 | Eif2ak2,Adar,Ifit1 |
| LV | G1 | multi-organism process | 0.23618 | Gbp7,Iigp1,Eif2ak2,Stat1,Adar,Ifih1,Ddx58,Dhx58,Cd47,Tdrd7,Pml,Ifit3,Rsad2,Ifit1 |
| LV | G1 | positive regulation of defense response | 0.27807 | Irf7,Trim12c,Ddx58,Dhx58,Cd47,Rsad2 |
| LV | G1 | response to organic substance | 0.57043 | Hap1,Gbp7,Iigp1,Xaf1,Irf7,Eif2ak2,Stat1,Adar,Ddx58,Parp9,Igtp,Pml,Ifit3,Ifit1 |
| LV | G1 | Toxoplasmosis | 0.60505 | Stat1,Irgm1,Lama3,Igtp |
| LV | G1 | regulation of response to stress | 0.63165 | Pgap2,Irf7,Eif2ak2,Adar,Trim12c,Ddx58,Parp9,Dhx58,Cd47,Pml,Rsad2 |

[Table 18-2]

| Organ | group | Term | Pvalue | Genelist |
|---|---|---|---|---|
| LV | G1 | regulation of response to stress | 0.63165 | Pgap2,Irf7,Eif2ak2,Adar,Trim12c,Ddx58,Parp9,Dhx58,Cd47,Pml,Rsad2 |
| LV | G1 | negative regulation of viral life cycle | 0.74299 | Eif2ak2,Adar,Pml,Rsad2 |
| LV | G1 | mRNA Editing | 0.75953 | Adar |
| LV | G1 | NF-kB activation through FADD/RIP-1 pathway mediated by caspase-8 and -10 | 0.82806 | Ifih1,Ddx58 |
| LV | G1 | Ubiquitin mediated proteolysis\|Cell cycle | 0.8748 | Ccnd2,Uba7,Shisa5,Pml |
| LV | G1 | defense response to other organism | 0.9983 | Gbp7,Iigp1,Adar,Ddx58,Dhx58,Pml,Rsad2 |
| LV | G2 | cellular protein modification process | 0.25047 | Acer2,Thpo,Mylip,Gan,Nceh1,Plcb1,Tirap,Egfr,Clock,Ibtk,Ppap2a,Map3k3,Nedd4l,Pdgfd,St5,Stk38l,Nlrp12,Gdf10,St3gal4,Braf,Garem,Metap2,Igfbp3,Pofut2,Galnt2,Tbl1xr1,B3galt1,Fbxo31,Rlim |
| LV | G2 | Metabolism of lipids and lipoproteins | 0.25919 | Acer2,Acox1,Crls1,Clock,Ppap2a,Slc25a20,Gpd2,Agpat3,Tbl1xr1,Insig2,Mgll,Lpgat1 |
| LV | G2 | Vesicle-mediated transport | 0.62121 | Cd163,Ap2m1,Gja1,Tgoln1,Saa2,Saa1 |
| LV | G2 | O-linked glycosylation | 0.90895 | St3gal4,Pofut2,Galnt2,Galnt15 |
| LV | G2 | Fatty acid, triacylglycerol, and ketone body metabolism | 0.98433 | Clock,Slc25a20,Gpd2,Agpat3,Tbl1xr1 |
| LV | G3 | Innate Immune System | 0.00399 | Sirpa,Itgb2,Icam2,Traf2,Traf3,Rps6ka5,Clec4e,Kit,Cd300lb |
| LN | G1 | immune system process | 5.1E-07 | Azi2,Pik3ap1,Itgb2,Coro1a,Tmem176a,Traf2,Traf3,Cd55,Clec4e,Kit,Cd300lb,Trem3,Tbx1,Sh3pxd2a |
| LV | G3 | Immune System | 0.06852 | Sirpa,Itgb2,Icam2,Traf2,Traf3,Rps6ka5,Clec4e,Kit,Cd300lb,Dync1li2 |
| LV | G3 | Innate Immune System\|Adaptive Immune System\|Hemostasis\|Pathways in cancer\|Developmental Biology\|PI3K-Akt signaling pathway\|Signaling by Rho GTPases | 0.07202 | Sirpa,Itgb2,Coro1a,Icam2,Iqgap1,Traf2,Traf3,Rps6ka5,Clec4e,Kit,Pfn2,Cd300lb,Plek,Ptgir,Abcc1,Dync1li2,Mapre1 |
| LV | G3 | immune effector process | 0.11451 | Cd93,Coro1a,Traf2,Traf3,Cd55,Clec4e,Kit,Cd300lb,Trem3 |
| LV | G3 | Cytokine-cytokine receptor interaction\|Endocytosis\|Herpes simplex infection | 0.1413 | Itgb2,Coro1a,Icam2,Traf2,Traf3,Cd55,Kit,Dync1li2 |
| LV | G3 | cytoskeleton organization | 0.16195 | Sirpa,Coro1a,Kit,Pfn2,Plek,Spast,Dync1li2,Mapre1 |
| LV | G3 | cell adhesion | 0.29629 | Sirpa,Cd93,Itgb2,Coro1a,Icam2,Clec4e,Kit,Plek |
| LV | G3 | leukocyte mediated immunity | 0.32576 | Coro1a,Traf2,Cd55,Kit,Cd300lb,Trem3 |
| LV | G3 | vesicle-mediated transport | 0.48714 | Sirpa,Pkdcc,Coro1a,Kit,Pfn2,Plek,Spast |
| LV | G3 | cellular localization | 0.70808 | Itgb2,Pkdcc,Coro1a,Os9,Traf2,Ildr2,Clec4e,Slc25a37,Kit,Pfn2,Plek,Trem3,Snx13,Spast,Dync1li2,Mapre1 |
| LV | G4 | Protein processing in endoplasmic reticulum | 3.3E-24 | Rnf185,Sec61b,Hsp90b1,Srp68,Sel1l,Syvn1,Fbxo6,Ssr1,Ssr2,Dnajc5,Mogs,Spcs2,Spcs3,Ssr4,Atf6,Herpud1,Stt3a,Sec24d,Yod1,Rpn1,Derl1,Pdia3,Sar1a,Ube2j1,Vcp,Rrbp1,Vimp,Serp1,Atf6b,Dnajc10,Sec23b,Gm10177,Wfs1,Srp9,Ddost,Hspa5,Pdia6,Sec61a1,Dnajb11,Srp19,Pdia4,Srprb,Preb,Sec31a,Ckap4,Dnajc3,Txndc5,Ufd1l,Edem2,Edem3,Edem1,Srp72,Hyou1,Srpr,Man1a,Sec13,Plaa,Hsp90aa1,Srp54a,Ero1l,Calr,Ube2g2 |
| LV | G4 | Protein processing in endoplasmic reticulum | 3.3E-20 | Rnf185,Sec61b,Hsp90b1,Sel1l,Syvn1,Fbxo6,Ssr1,Ssr2,Dnajc5,Mogs,Ssr4,Atf6,Herpud1,Stt3a,Sec24d,Yod1,Rpn1,Derl1,Pdia3,Sar1a,Ube2j1,Vcp,Rrbp1,Vimp,Atf6b,Dnajc10,Sec23b,Gm10177,Wfs1,Ddost,Hspa5,Pdia6,Sec61a1,Dnajb11,Pdia4,Preb,Sec31a,Ckap4,Dnajc3,Txndc5,Ufd1l,Edem2,Edem3,Edem1,Hyou1,Man1a,Sec13,Plaa,Hsp90aa1,Ero1l,Calr,Ube2g2 |
| LN | G1 | response to stress | 0.8518 | Hopx,Aen,Mybbp1a,Tbl2,Cyp1b1,Selp,Pik3ap1,Bfar,Slamf8,Atg13,Cd28,Cebpg,Il1rn,Arl6ip5,Hck,Ednra,Btg2,Map3k3,B4galt1,Il1r1,Rab12,Ints7,Qsox1,Hsp90b1,Fktn,Errfi1,Cxcl9,Ung,Ctgf,Pdcd10,Sel1l,Actg1,Pnp,Hif1a,Eif4e,Dap,Marveld3,Lrrc8a,Lcn2,Syvn1,Braf,Scly,Creb3,Senp2,Nod1,Apex1,Clec4d,Hilpda,Pfkp,Cd55,Serinc3,Psma1,Atp7a,Adora1,Usp1,Tnfaip6,Irf8,Trib1,Grb2,Rbm18,Atf6,Atf3,Herpud1,Slc11a2,Slc11a1,Exosc4,Lbp,Skil,Polh,Uba5,Apcs,Ap1g1,Yod1,Fgr,Rcan2,Fgb,S100a8,Ppp4c,A2m,Slc35c1,Fen1,Ifitm2,Ifitm1,Derl1,Atg9b,Pdia3,Itpr1,Fcer1g,Lnp,Srebf2,Ufm1,Cln8,H2afx,Vcp,Dab2,Tnfrsf12a,Scfd1,Syk,Hmga2,Vimp,Adam17,Asns,Rraga,Hyal1,Ddx39,Cd44,Myh9,Serp1,Ctla2a,Atf6b,Reg3b,Creb3l2,Dnajc10,F13a1,Plek,Sdf2l1,Gnl1,Wfs1,Supt5,Hipk3,Txnl1,Gas6,Trim27,Il12rb1,Irak2,Iigp1,Sdc4,Itgam,Lig3,Gata6,Ncf1,Grina,Smarcad1,Pld1,Rasgrp1,Tirap,Trp53inp2,Tlr1,Apoa4,Apoa5,Trip12,Gigyf2,Mt2,Mt1,Hnf4a,Adam9,Hspa5,Jun,Pdia6,Nlrp12,Ubxn2b,Cox8a,Pdia4,Wipi1,Ifrd1,Ptpn2,Ptpn1,Ufl1,Casp4,Parp2,Parp3,Il17ra,Il4ra,Ddx1,Thoc1,Zfp830,Fbxo31,Il1a,Nabp1,Eya3,Psen1,Ap5s1,Erp44,Cdip1,Itgb3,Ccl17,Vav1,Ccl19,Wdr45b,Atg16l2,Eprs,Dnajc3,Trem3,Socs3,Txndc5,Rps6ka3,Tnfrsf1b,Tnfrsf1a,Cpeb2,Rint1,Tmem173,Rab1,Ift20,Hyou1,Cdkn1a,Gadd45b,Gadd45g,Sbno2,Sod2,Snap23,Pik3c2a,Coro1a,Pycard,Map1lc3b,Trp63,Fn1,Clock,Alox12,Chmp4b,Ccr1,Txlna,Plaa,Clec4n,Seh1l,Cr1l,Fabp4,Hsp90aa1,Ero1l,Fcgr3,Rab33b,Psmd14,Scara5,Calr,Polr3d,Nfkbiz,Prkce,Acot11,Crnkl1,Irg1,Myo1f,S100a9 |

[Table 18-3]

| Organ | group | Term | Pvalue | Genelist |
|---|---|---|---|---|
| LV | G4 | response to endoplasmic reticulum stress | 9.9E-12 | Selk,Tbl2,Bfar,Sel1l,Syvn1,Creb3,Serinc3,Atf6,Atf3,Herpud1,Uba5,Yod1,Derl1,Pdia3,Itpr1,Ufm1,Vimp,Serp1,Atf6b,Creb3l2,Dnajc10,Sdf2l1,Wfs1,Grina,Hspa5,Jun,Pdia6,Pdia4,Ptpn2,Ptpn1,Ufl1,Casp4,Erp44,Dnajc3,Txndc5,Hyou1,Ero1l |
| LV | G4 | macromolecule metabolic process | 6.9E-08 | Lars,Rad1,Selk,Mybbp1a,Nampt,Cyp1b1,Armcx3,Nol8,Bfar,Pdgfc,Mbd1,Sec61b,Prkag2,Snx6,Utp6,Dhx30,Garem,Mlx,Cd28,Cebpg,Timp1,Cebpd,Ddx52,Mettl1,Mettl3,Ezr,Hnrnpa2b1,Senp6,Hnrnpab,Polr1e,Pim3,Arl6ip5,Metap2,Hck,Etf1,Btg2,Ntmt1,Dhx40,Anapc4,Wdr77,Mvp,Ppap2a,Map3k3,Krtcap2,B4galt1,Pole4,Bmper,Ficd,Tcf25,Rab12,Ints7,Ints5,Rpf2,Qsox1,Hsp90b1,Plcl1,Fktn,Pus3,Errfi1,Ung,Ctgf,Nktr,Epm2aip1,Pdcd10,Trnt1,Mphosph8,Klf17,Pnp,Hif1a,Mphosph6,Smarce1,Eif4e,Dap,Marveld3,Sf3b4,Eif3d,Syvn1,Prg4,Srsf2,Braf,Timp3,Creb3,Senp2,Efna1,Ell2,Nod1,Fbxo6,Ctif,Gmeb1,Ibtk,Apex1,Ube2s,Trim39,Csnk1a1,Pcyox1,Npm3,Mmp8,Tasp1,Mmp13,Pcid2,Ddx21,Kdm6a,Cwc27,Rlim,Dhx38,Cd55,Dok2,Psma1,Atp7a,Usp8,Usp1,Tnfaip1,Eogt,Aak1,Recql,Mrpl51,Mbnl2,Ell,Tbp,Irf8,Trib1,Dhx35,Grb2,Cebpz,Atf6,Ints2,Atf3,Herpud1,Slc11a2,Gzma,Slc11a1,Mthfr,Tpp2,Mmp9,Exosc1,Exosc4,Cad,Lbp,Serpina3k,Serpina3m,Serpina3n,Serpina3g,Snip1,Skil,Stt3a,Lpin1,Ddx50,Polh,Gtf2f1,Nup98,Uba5,Apcs,Jmjd6,Lpar1,Foxk2,Yod1,Fgr,Arf4,Camkk2,Ube2f,Fgb,Rpn1,Tgm2,Uspl1,S100a8,Tmsb4x,Ppp4c,A2m,Egf,Eif4g2,Fkbp11,Stag1,Nedd4l,C1galt1,Fen1,Wdr61,Ebna1bp2,Trps1,Derl1,Spin1,Iars,Traf7,Pdia3,Acer2,Dpy19l1,Saa3,Rars,Usp14,Tbl1xr1,Srebf2,Ufm1,Suco,Cul5,Cln8,Trappc2,H2afx,Ube2j1,Vcp,Scnm1,Mcm2,Dab2,Dusp2,Asb4,Syk,St6gal1,Hmga2,Vimp,Adam17,Srgn,Hyal1,Eif4a3,Ddx39,Cd44,Myh9,Serp1,Neurl3,Ctla2a,Ctla2b,Prmt1,Dzip3,Prdm1,Atf6b,Iqgap1,Pcsk5,Creb3l2,Wdr12,Dnajc10,F13a1,Nelfa,Rbm39,Sox9,Nus1,Sdf2l1,Odc1,Wfs1,Supt5,Supt6,Hipk3,Tmem165,Helb,Ngp,Txnl1,Gas6,Trim27,Rac2,Ppp2r1a,Il12rb1,Crem,Paip2b,Irak2,Hist1h3b,Gemin8,Ngdn,Lyve1,Cdc73,Parn,Lig3,Gata6,Exog,Tspyl2,Smarcad1,Dis3,Gfpt1,Rasgrp1,Nop58,Tirap,Trp53inp2,Fkbp5,Tlr1,Med17,Apoa4,Apoa5,Trip12,Hars,Zfp869,Ddost,Ick,Gigyf2,Hnf4a,Adam9,Zfp160,Tra2b,Hspa5,Atf7ip,Csgalnact2,Golph3,Jun,Pdia6,Dnajb11,Nlrp12,Nudt21,Papola,Flot1,Crp,Ubxn2b,Denr,Cars2,Capn10,Farsb,Taf2,Nup62,Vars,Pdia4,Wipi1,Ifrd1,Crtc2,Secisbp2,Ptpn2,Ptpn1,Ap1ar,Abca1,Ufl1,Ddx49,Casp4,Znrf1,Plaur,Nceh1,Preb,Parp2,Parp3,Mppe1,Zbtb16,Zbtb21,Med25,Eif2b4,Ddx1,Thoc2,Thoc1,Elp3,Inhbe,B3galt6,B3galt1,Rnps1,Tfb2m,Cdk7,Dph5,Exosc10,Zfp830,Brd8,Fbxo31,Il1a,Alg12,Rps9,Nabp1,Kars,Stk17b,Son,Pggt1b,Tiparp,Eya3,Slc9a3r1,Prkrip1,Sec22b,Psen1,Ap5s1,Erp44,Exosc3,Hltf,Zfand2a,Cd38,Piga,Itgb3,Itgb2,Arid5a,Ccl19,Khsrp,Gan,Gtf2a2,Nfia,Wdr45b,Atg16l2,Trmt61a,Kctd10,Eprs,D19Bwg1357e,St3gal3,Dnajc3,Trim68,Rcor1,Trem3,Socs3,Dr1,Camk2b,Polr1a,Ubxn4,Trim3,Txndc5,Rps6ka3,Tnfrsf1b,Tnfrsf1a,Nek7,Cpeb2,Edem3,Trib2,Edem1,Tmem173,Rab1,Cdkn1a,Taf4b,Nbas,Gadd45b,Gadd45g,Ppp1r11,Magoh,Sbno2,Sod2,Msn,Pik3c2a,Nol9,Vegfc,Pycard,Trp63,Fn1,Stx5a,Clock,Bbs4,B3galnt2,St3gal5,St3gal4,Pomgnt1,Chmp4b,Ppm1l,Ccr1,Cr1l,Fabp4,Hsp90aa1,Ero1l,Srsf1,Fcgr3,Srsf3,Psmd14,Scara5,Zfp27,Serpina10,Calr,Pgm3,Rusc1,Nop56,Pofut2,Polr3k,Galnt2,Tbrg1,Taf1,Pspc1,Nfkbiz,Trmt10a,Enc1,Srrt,Josd2,Lats2,Prkch,Ears2,Prkce,Klk1b24,Crnkl1,Irg1,Rbm42,Larp7,Anapc16,Il6st,Ikbkap,Nif3l1,S100a9,Ube2g2 |
| LV | G4 | cellular macromolecule metabolic process | 7.5E-08 | Lars,Rad1,Selk,Mybbp1a,Nampt,Cyp1b1,Armcx3,Nol8,Bfar,Pdgfc,Mbd1,Sec61b,Prkag2,Snx6,Utp6,Dhx30,Garem,Mlx,Cd28,Cebpg,Timp1,Cebpd,Ddx52,Mettl1,Mettl3,Ezr,Hnrnpa2b1,Senp6,Hnrnpab,Polr1e,Pim3,Arl6ip5,Metap2,Hck,Etf1,Btg2,Ntmt1,Dhx40,Anapc4,Wdr77,Mvp,Ppap2a,Map3k3,Krtcap2,B4galt1,Pole4,Bmper,Ficd,Tcf25,Rab12,Ints7,Ints5,Rpf2,Qsox1,Hsp90b1,Plcl1,Fktn,Pus3,Errfi1,Ung,Ctgf,Nktr,Epm2aip1,Pdcd10,Trnt1,Mphosph8,Klf17,Pnp,Hif1a,Mphosph6,Smarce1,Eif4e,Dap,Marveld3,Sf3b4,Eif3d,Syvn1,Srsf2,Braf,Timp3,Creb3,Senp2,Efna1,Ell2,Nod1,Fbxo6,Ctif,Gmeb1,Ibtk,Apex1,Ube2s,Trim39,Csnk1a1,Pcyox1,Npm3,Tasp1,Mmp13,Pcid2,Ddx21,Cwc27,Rlim,Dhx38,Dok2,Psma1,Atp7a,Usp8,Usp1,Tnfaip1,Eogt,Aak1,Recql,Mrpl51,Mbnl2,Ell,Tbp,Irf8,Trib1,Dhx35,Grb2,Cebpz,Atf6,Ints2,Atf3,Herpud1,Slc11a2,Gzma,Slc11a1,Mthfr,Mmp9,Exosc1,Exosc4,Cad,Serpina3k,Serpina3m,Serpina3n,Serpina3g,Snip1,Skil,Stt3a,Lpin1,Ddx50,Polh,Gtf2f1,Nup98,Uba5,Apcs,Jmjd6,Lpar1,Foxk2,Yod1,Fgr,Arf4,Camkk2,Ube2f,Fgb,Rpn1,Tgm2,Uspl1,S100a8,Tmsb4x,Ppp4c,A2m,Egf,Eif4g2,Fkbp11,Stag1,Nedd4l,C1galt1,Fen1,Wdr61,Ebna1bp2,Trps1,Derl1,Spin1,Iars,Traf7,Pdia3,Acer2,Dpy19l1,Saa3,Rars,Usp14,Tbl1xr1,Srebf2,Ufm1,Cul5,Cln8,Trappc2,H2afx,Ube2j1,Vcp,Scnm1,Mcm2,Dab2,Dusp2,Asb4,Syk,St6gal1,Hmga2,Vimp,Adam17,Eif4a3,Ddx39,Cd44,Myh9,Serp1,Neurl3,Ctla2a,Ctla2b,Prmt1,Dzip3,Prdm1,Atf6b,Iqgap1,Pcsk5,Creb3l2,Wdr12,Dnajc10,F13a1,Nelfa,Rbm39,Sox9,Nus1,Sdf2l1,Wfs1,Supt5,Supt6,Hipk3,Tmem165,Helb,Ngp,Txnl1,Gas6,Trim27,Rac2,Ppp2r1a,Il12rb1,Crem,Paip2b,Irak2,Hist1h3b,Gemin8,Ngdn,Cdc73,Parn,Lig3,Gata6,Exog,Tspyl2,Smarcad1,Dis3,Rasgrp1,Nop58,Tirap,Trp53inp2,Fkbp5,Tlr1,Med17,Trip12,Hars,Zfp869,Ddost,Ick,Gigyf2,Hnf4a,Adam9,Zfp160,Tra2b,Hspa5,Atf7ip,Csgalnact2,Golph3,Jun,Pdia6,Dnajb11,Nlrp12,Nudt21,Papola,Flot1,Ubxn2b,Denr,Cars2,Farsb,Taf2,Nup62,Vars,Pdia4,Wipi1,Ifrd1,Crtc2,Secisbp2,Ptpn2,Ptpn1,Ap1ar,Abca1,Ufl1,Ddx49,Znrf1,Plaur,Nceh1,Preb,Parp2,Parp3,Mppe1,Zbtb16,Zbtb21,Med25,Eif2b4,Ddx1,Thoc1,Elp3,Inhbe,B3galt1,Rnps1,Tfb2m,Cdk7,Dph5,Exosc10,Zfp830,Brd8,Fbxo31,Il1a,Alg12,Rps9,Nabp1,Kars,Stk17b,Son,Pggt1b,Tiparp,Eya3,Slc9a3r1,Prkrip1,Psen1,Ap5s1,Erp44,Exosc3,Hltf,Zfand2a,Cd38,Piga,Itgb3,Itgb2,Arid5a,Ccl19,Khsrp,Gan,Gtf2a2,Nfia,Wdr45b,Atg16l2,Trmt61a,Kctd10,Eprs,D19Bwg1357e,St3gal3,Dnajc3,Trim68,Rcor1,Socs3,Dr1,Camk2b,Polr1a,Ubxn4,Trim3,Txndc5,Rps6ka3,Tnfrsf1b,Tnfrsf1a,Nek7,Cpeb2,Edem3,Trib2,Edem1,Tmem173,Rab1,Cdkn1a,Taf4b,Nbas,Gadd45b,Gadd45g,Ppp1r11,Magoh,Sbno2,Sod2,Pik3c2a,Nol9,Vegfc,Pycard,Trp63,Fn1,Clock,B3galnt2,St3gal4,Pomgnt1,Chmp4b,Ppm1l,Ccr1,Cr1l,Fabp4,Hsp90aa1,Ero1l,Srsf1,Srsf3,Psmd14,Zfp27,Serpina10,Calr,Pgm3,Rusc1,Nop56,Pofut2,Polr3k,Galnt2,Tbrg1,Taf1,Pspc1,Nfkbiz,Trmt10a,Enc1,Srrt,Josd2,Lats2,Prkch,Ears2,Prkce,Crnkl1,Irg1,Rbm42,Larp7,Anapc16,Il6st,Ikbkap,Nif3l1,S100a9,Ube2g2 |
| LV | G4 | cellular response to stress | 1.4E-07 | Rad1,Selk,Mybbp1a,Tbl2,Cyp1b1,Bfar,Atg13,Cebpg,Arl6ip5,Btg2,Map3k3,Rab12,Ints7,Qsox1,Fktn,Errfi1,Ung,Ctgf,Pdcd10,Sel1l,Pnp,Hif1a,Dap,Marveld3,Syvn1,Braf,Scly,Creb3,Senp2,Nod1,Apex1,Pfkp,Serinc3,Atp7a,Usp1,Trib1,Grb2,Atf6,Atf3,Herpud1,Slc11a2,Skil,Polh,Uba5,Yod1,Ppp4c,Slc35c1,Fen1,Derl1,Atg9b,Pdia3,Itpr1,Srebf2,Ufm1,H2afx,Vcp,Dab2,Scfd1,Syk,Hmga2,Vimp,Asns,Rraga,Ddx39,Cd44,Serp1,Atf6b,Creb3l2,Dnajc10,Sdf2l1,Gnl1,Wfs1,Supt5,Hipk3,Txnl1,Gas6,Lig3,Gata6,Grina,Smarcad1,Rasgrp1,Tirap,Trp53inp2,Apoa4,Apoa5,Trip12,Gigyf2,Hspa5,Jun,Pdia6,Ubxn2b,Cox8a,Pdia4,Wipi1,Ptpn2,Ptpn1,Ufl1,Casp4,Parp2,Parp3,Ddx1,Thoc1,Zfp830,Fbxo31,Il1a,Nabp1,Eya3,Psen1,Ap5s1,Erp44,Cdip1,Ccl19,Wdr45b,Atg16l2,Dnajc3,Txndc5,Tnfrsf1b,Tnfrsf1a,Cpeb2,Rint1,Rab1,Ift20,Hyou1,Cdkn1a,Gadd45b,Gadd45g,Sod2,Pycard,Map1lc3b,Trp63,Clock,Chmp4b,Txlna,Ero1l,Rab33b,Psmd14,Scara5,Calr,Prkce,Crnkl1 |
| LV | G4 | Asparagine N-linked glycosylation | 3.8E-07 | B4galt1,Mvd,Mcfd2,Mogs,Nans,Sec24d,Pdia3,St6gal1,Gfpt1,Preb,Alg12,Sec31a,Gne,St3gal3,Edem2,Edem3,Edem1,Gmppa,Man1a,Sec13,Dhdds,St3gal5,St3gal4,Uap1,Calr,Pgm3,Gmppb |
| LV | G4 | Metabolism of proteins | 4.3E-07 | Sec61b,Etf1,Pigm,B4galt1,Mvd,Srp68,Eif4e,Vbp1,Eif3d,Slc30a5,Timm21,Senp2,Fbxo6,Mcfd2,Ssr1,Ssr2,Mogs,Nans,Spcs2,Spcs3,Ssr4,Atf6,Nup98,Sec24d,Gspt2,Rpn1,Cct3,Stag1,C1galt1,Pdia3,Ranbp2,St6gal1,Serp1,Slc30a7,Nupl2,Gas6,Srp9,Gfpt1,Ddost,Kif5b,Sec61a1,Nupl1,Srp19,Nup62,Srprb,Plaur,Preb,Eif2b4,Inhbe,Dph5,Alg12,Rps9,Sec31a,Piga,Gne,St3gal3,Edem2,Edem3,Edem1,Srp72,Gmppa,Pfdn4,Srpr,Man1a,Sec13,Dhdds,St3gal5,St3gal4,Seh1l,Srp54a,Ero1l,Uap1,Calr,Pgm3,Nop56,Pofut2,Galnt2,Gmppb,Klk1b24 |

[Table 18-4]

| Organ | group | Term | Pvalue | Genelist |
|---|---|---|---|---|
| LV | G4 | metabolic process | 5.3E-07 | Cers6,Lars,Sdsl,Rad1,Selk,Mybbp1a,Nampt,Cyp1b1,Armcx3,Abcc1,Nol8,Bfar,Slamf8,Pdgfc,Mbd1,Sec61b,Prkag2,Snx6,Atg13,Utp6,2700097O09Rik,Dhx30,Garem,Tat,Mlx,Cd28,Cebpg,Timp1,Cebpd,Ddx52,Mettl1,Mettl3,Ezr,Rinl,Hnrnpa2b1,Senp6,Hnrnpab,Polr1e,Il1rn,Pim3,Arl6ip5,Metap2,Cog6,Hck,Etf1,Ednra,Btg2,Ntmt1,Dhx40,Anapc4,Wdr77,Mvp,Agk,Adcy1,Ppap2a,Map3k3,Krtcap2,B4galt1,Pole4,Mvd,Bmper,Ficd,Tcf25,Ldha,Rab12,Ints7,Ints5,Rpf2,Qsox1,Hsp90b1,Plcl1,Atp6v0a1,Fktn,Pus3,Errfi1,Cxcl9,Ung,Ctgf,Nktr,Epm2aip1,Pdcd10,Trnt1,Mphosph8,Klf17,Gpt2,Pnp,Hif1a,Mphosph6,Smarce1,Eif4e,Dap,Marveld3,Agpat9,Sf3b4,Eif3d,Syvn1,Prg4,Srsf2,Braf,Scly,Timp3,Creb3,Senp2,Efna1,Rabl3,Ell2,Nod1,Fbxo6,Ctif,Mcfd2,Gmeb1,Cyp7b1,Ibtk,Lrrc16a,Apex1,Ube2s,Trim39,Csnk1a1,Pcyox1,Npm3,Mmp8,Tasp1,Aldh18a1,Mmp13,Pcid2,Ddx21,Pfkp,Kdm6a,Cwc27,Gls,Rlim,B4galnt4,Dhx38,B4galnt1,Cd55,Dok2,Psma1,Atp7a,Ubiad1,Usp8,Entpd7,Usp1,Tnfaip1,Eogt,Aak1,Recql,Mrpl51,Acpp,Mbnl2,Golt1b,Ell,Tbp,Irf8,Trib1,Dhx35,Grb2,Cebpz,Atf6,Ints2,Atf3,Herpud1,Slc11a2,Gzma,Slc11a1,Mthfr,Tpp2,Mmp9,Exosc1,Exosc4,Cad,Lbp,Serpina3k,Serpina3m,Serpina3n,Serpina3g,Rbm26,Snip1,Skil,Stt3a,Lpin1,Ddx50,Polh,Dram1,Gtf2f1,Nup98,Kalrn,Uba5,Apcs,Jmjd6,Lpar1,Foxk2,Yod1,Fgr,Arf4,Camkk2,Ube2f,Rcan2,Fgb,Rpn1,Tgm2,Uspl1,S100a8,Tmsb4x,Ppp4c,Acot9,A2m,Egf,Eif4g2,Slc35c1,Fkbp15,Fkbp11,Stag1,Fgl1,Nedd4l,C1galt1,Fen1,Wdr61,Ebna1bp2,Pkm,Trps1,Derl1,Spin1,Atg9b,Iars,Traf7,Pdia3,Acer2,Dpy19l1,Saa3,Rab35,Rars,Hdc,Usp14,Tbl1xr1,Rrp1b,Pfkfb2,Srebf2,Ufm1,Suco,Far2,Cul5,Ranbp2,Cln8,Trappc2,H2afx,Ube2j1,Vcp,Scnm1,Mcm2,Dab2,Sf1,Dusp2,Scfd1,Asb4,Syk,St6gal1,Hmga2,Ppip5k1,Vimp,Adam17,Srgn,Asns,Rraga,Hyal1,Eif4a3,Ddx39,Cd44,Myh9,Serp1,Neurl3,Ctla2a,Ctla2b,Prmt1,Dzip3,Cyp51,Prdm1,Atf6b,Iqgap1,Rab5b,Pcsk5,Creb3l2,Wdr12,Dnajc10,F13a1,Tbc1d15,Plek,Steap4,Idi1,Nelfa,Rbm39,Sox9,Nus1,Sdf2l1,Odc1,Papss1,Gnl2,Gnl1,Wfs1,Supt5,Supt6,Hipk3,Tmem165,Helb,Ngp,Txnl1,Gas6,Trim27,Rac2,Zdhhc13,Ppp2r1a,Il12rb1,Crem,Paip2b,Irak2,Dynll1,Iigp1,Hist1h3b,Gemin8,Ngdn,Lyve1,Cdc73,Parn,Gm21540,Lig3,Gata6,Exog,Ncf1,Tspyl2,Srm,Smarcad1,Dis3,Gfpt1,Pld1,Tha1,Rasgrp1,Nop58,Tirap,Trp53inp2,Fkbp5,Tlr1,Med17,Apoa4,Apoa5,Trip12,Hars,Zfp869,Ddost,Ick,Gigyf2,Mt2,Kif5b,Mt1,Hnf4a,Adam9,Zfp160,Nnmt,Tra2b,Hspa5,Atf7ip,Csgalnact2,Golph3,Jun,Pdia6,Dnajb11,Nlrp12,Nudt21,Papola,Ralgapa2,Flot1,Crp,Ubxn2b,Denr,Cars2,Capn10,Cox8a,Farsb,Kif21a,Taf2,Ctps,Nup62,Vars,Pdia4,Wipi1,Ppapdc1b,Rab18,Ifrd1,Crtc2,Secisbp2,Ptpn2,Ptpn1,Ap1ar,Abca1,Ufl1,Ddx49,Casp4,Znrf1,Plaur,Nceh1,Preb,Parp2,Parp3,Mppe1,Zbtb16,Zbtb21,Med25,Eif2b4,Ppip5k2,Ddx1,Thoc2,Thoc1,Elp3,Inhbe,B3galt6,B3galt1,Rnps1,Tfb2m,Cdk7,Dph5,Exosc10,Zfp830,Brd8,Fbxo31,Il1a,Alg12,Rps9,Nabp1,Kars,Cda,Stk17b,Son,Pggt1b,Tiparp,Eya3,Slc9a3r1,Prkrip1,Sec22b,Psen1,Ap5s1,Erp44,Exosc3,Hltf,Zfand2a,Cd38,Piga,Itgb3,Itgb2,Arid5a,Vav1,Ccl19,Khsrp,Gan,Gtf2a2,Nfia,Wdr45b,Atg16l2,Trmt61a,Gne,Kctd10,Eprs,D19Bwg1357e,S100a10,St3gal3,Dnajc3,Trim68,Rcor1,Trem3,Socs3,Dr1,Camk2b,Polr1a,Ubxn4,Trim3,Txndc5,Rps6ka3,Tnfrsf1b,Tnfrsf1a,Nek7,Cpeb2,Edem3,Trib2,Edem1,Tmem173,Rab1,Ift20,Cdkn1a,Taf4b,Nbas,Hk2,Gadd45b,Sds,Gadd45g,Ppp1r11,Gale,Magoh,Sbno2,Sod2,Msn,Pik3c2a,Nol9,Etnk2,Vegfc,Pycard,Map1lc3b,Adk,Trp63,Fn1,Stx5a,Clock,Bbs4,Dennd1b,Alox12,B3galnt2,St3gal5,St3gal4,Pomgnt1,Chmp4b,Ppm1l,Ccr1,Txlna,Plaa,Gnai2,Cr1l,Fabp4,Fabp5,Hsp90aa1,Ero1l,Lss,Srsf1,Fcgr3,Srsf3,Rab33b,Psmd14,Scara5,Zfp27,Serpina10,Calr,Pgm3,Rusc1,Nop56,Pofut2,Polr3k,Galnt2,Tbrg1,Taf1,Pspc1,Atl1,Nfkbiz,Trmt10a,Enc1,Srrt,Josd2,Lats2,Prkch,Ears2,Prkce,Acot11,Klk1b24,Crnkl1,Irg1,Rbm42,Larp7,Anapc16,Il6st,Rapgef5,Ikbkap,Nif3l1,Arfgap1,S100a9,Ube2g2 |
| LV | G4 | vesicle-mediated transport | 6.3E-07 | Yipf5,Golga5,Snx6,Ezr,Rinl,Stx6,Cog6,Hck,B4galt1,Rab12,Stx12,Stx18,Arcn1,Uso1,Krt18,Braf,Csnk1a1,Gosr2,Aak1,Marco,Irf8,Grb2,Blzf1,Slc11a1,Pdzd11,Lbp,Cd14,Jmjd6,Ap1g1,Fgr,Fgb,Tgm2,Tmed9,Ap2a2,Sar1a,Vamp4,Rab35,Fcer1g,Dab2,Scfd1,Syk,Ap4e1,Copb2,Myh9,Zw10,Rab5b,Creb3l2,Plek,Chic2,Gas6,Trim27,Rac2,Mfsd2a,Golga4,Mon1b,Sdc4,Rasgrp1,Golph3,Flot1,Wipi1,Ptpn1,Ap1ar,Abca1,Mppe1,Il4ra,Bet1l,Ap3d1,Syt12,Sec22b,Hook1,Psen1,Vav1,Ccl19,Snx10,S100a10,Cltb,Txndc5,Rint1,Rab1,C2cd5,Snap23,Sec13,Pik3c2a,Coro1a,Pycard,Stx5a,Chmp4b,Ccr1,Fcgr3,Rab33b,Scara5,Calr,Myo1f,Arfgap1 |
| LV | G4 | primary metabolic process | 8.7E-07 | Cers6,Lars,Sdsl,Rad1,Selk,Mybbp1a,Nampt,Cyp1b1,Armcx3,Nol8,Bfar,Pdgfc,Mbd1,Sec61b,Prkag2,Snx6,Utp6,Dhx30,Garem,Tat,Mlx,Cd28,Cebpg,Timp1,Cebpd,Ddx52,Mettl1,Mettl3,Hnrnpa2b1,Senp6,Hnrnpab,Polr1e,Il1rn,Pim3,Arl6ip5,Metap2,Cog6,Hck,Etf1,Ednra,Btg2,Ntmt1,Dhx40,Anapc4,Wdr77,Mvp,Agk,Adcy1,Ppap2a,Map3k3,Krtcap2,B4galt1,Pole4,Mvd,Bmper,Ficd,Tcf25,Rab12,Ints7,Ints5,Rpf2,Qsox1,Hsp90b1,Plcl1,Atp6v0a1,Fktn,Pus3,Errfi1,Cxcl9,Ung,Ctgf,Nktr,Epm2aip1,Pdcd10,Trnt1,Mphosph8,Klf17,Gpt2,Pnp,Hif1a,Mphosph6,Smarce1,Eif4e,Dap,Marveld3,Agpat9,Sf3b4,Eif3d,Syvn1,Prg4,Srsf2,Braf,Scly,Timp3,Creb3,Senp2,Efna1,Ell2,Nod1,Fbxo6,Ctif,Gmeb1,Cyp7b1,Ibtk,Apex1,Ube2s,Trim39,Csnk1a1,Pcyox1,Npm3,Mmp8,Tasp1,Aldh18a1,Mmp13,Pcid2,Ddx21,Pfkp,Cwc27,Gls,Rlim,Dhx38,B4galnt1,Cd55,Dok2,Psma1,Atp7a,Usp8,Entpd7,Usp1,Tnfaip1,Eogt,Aak1,Recql,Mrpl51,Acpp,Mbnl2,Golt1b,Ell,Tbp,Irf8,Trib1,Dhx35,Grb2,Cebpz,Atf6,Ints2,Atf3,Herpud1,Slc11a2,Gzma,Slc11a1,Mthfr,Tpp2,Mmp9,Exosc1,Exosc4,Cad,Lbp,Serpina3k,Serpina3m,Serpina3n,Serpina3g,Snip1,Skil,Stt3a,Lpin1,Ddx50,Polh,Gtf2f1,Nup98,Uba5,Apcs,Jmjd6,Lpar1,Foxk2,Yod1,Fgr,Arf4,Camkk2,Ube2f,Fgb,Rpn1,Tgm2,Uspl1,S100a8,Tmsb4x,Ppp4c,A2m,Egf,Eif4g2,Slc35c1,Fkbp11,Stag1,Fgl1,Nedd4l,C1galt1,Fen1,Wdr61,Ebna1bp2,Pkm,Trps1,Derl1,Spin1,Iars,Traf7,Pdia3,Acer2,Dpy19l1,Saa3,Rars,Hdc,Usp14,Tbl1xr1,Pfkfb2,Srebf2,Ufm1,Cul5,Ranbp2,Cln8,Trappc2,H2afx,Ube2j1,Vcp,Scnm1,Mcm2,Dab2,Sf1,Dusp2,Asb4,Syk,St6gal1,Hmga2,Ppip5k1,Vimp,Adam17,Srgn,Asns,Eif4a3,Ddx39,Cd44,Myh9,Serp1,Neurl3,Ctla2a,Ctla2b,Prmt1,Dzip3,Cyp51,Prdm1,Atf6b,Iqgap1,Pcsk5,Creb3l2,Wdr12,Dnajc10,F13a1,Plek,Idi1,Nelfa,Rbm39,Sox9,Nus1,Sdf2l1,Odc1,Papss1,Wfs1,Supt5,Supt6,Hipk3,Tmem165,Helb,Ngp,Txnl1,Gas6,Trim27,Rac2,Ppp2r1a,Il12rb1,Crem,Paip2b,Irak2,Hist1h3b,Gemin8,Ngdn,Cdc73,Parn,Lig3,Gata6,Exog,Ncf1,Tspyl2,Smarcad1,Dis3,Pld1,Tha1,Rasgrp1,Nop58,Tirap,Trp53inp2,Fkbp5,Tlr1,Med17,Apoa4,Apoa5,Trip12,Hars,Zfp869,Ddost,Ick,Gigyf2,Hnf4a,Adam9,Zfp160,Tra2b,Hspa5,Atf7ip,Csgalnact2,Golph3,Jun,Pdia6,Dnajb11,Nlrp12,Nudt21,Papola,Ubxn2b,Denr,Cars2,Capn10,Farsb,Taf2,Ctps,Nup62,Vars,Pdia4,Wipi1,Ppapdc1b,Ifrd1,Crtc2,Secisbp2,Ptpn2,Ptpn1,Abca1,Ufl1,Ddx49,Casp4,Znrf1,Plaur,Nceh1,Preb,Parp2,Parp3,Mppe1,Zbtb16,Zbtb21,Med25,Eif2b4,Ppip5k2,Ddx1,Thoc1,Elp3,Inhbe,B3galt1,Rnps1,Tfb2m,Cdk7,Dph5,Exosc10,Zfp830,Brd8,Fbxo31,Il1a,Alg12,Rps9,Nabp1,Kars,Cda,Stk17b,Son,Pggt1b,Tiparp,Eya3,Slc9a3r1,Prkrip1,Sec22b,Psen1,Ap5s1,Erp44,Exosc3,Hltf,Zfand2a,Cd38,Piga,Itgb3,Itgb2,Arid5a,Ccl19,Khsrp,Gan,Gtf2a2,Nfia,Wdr45b,Atg16l2,Trmt61a,Kctd10,Eprs,D19Bwg1357e,St3gal3,Dnajc3,Trim68,Rcor1,Trem3,Socs3,Dr1,Camk2b,Polr1a,Ubxn4,Trim3,Txndc5,Rps6ka3,Tnfrsf1b,Tnfrsf1a,Nek7,Cpeb2,Edem3,Trib2,Edem1,Tmem173,Rab1,Cdkn1a,Taf4b,Nbas,Hk2,Gadd45b,Sds,Gadd45g,Ppp1r11,Gale,Magoh,Sbno2,Sod2,Pik3c2a,Nol9,Etnk2,Vegfc,Pycard,Adk,Trp63,Fn1,Stx5a,Clock,Bbs4,Alox12,B3galnt2,St3gal4,Pomgnt1,Chmp4b,Ppm1l,Ccr1,Cr1l,Fabp4,Fabp5,Hsp90aa1,Ero1l,Lss,Srsf1,Fcgr3,Srsf3,Psmd14,Zfp27,Serpina10,Calr,Pgm3,Rusc1,Nop56,Pofut2,Polr3k,Galnt2,Tbrg1,Taf1,Pspc1,Nfkbiz,Trmt10a,Enc1,Srrt,Josd2,Lats2,Prkch,Ears2,Prkce,Acot11,Klk1b24,Crnkl1,Irg1,Rbm42,Larp7,Anapc16,Il6st,Ikbkap,Nif3l1,S100a9,Ube2g2 |

[Table 18-5]

| Organ | group | Term | Pvalue | Genelist |
|---|---|---|---|---|
| LV | G4 | organic substance metabolic process | 1.1E-06 | Cers6,Lars,Sdsl,Rad1,Selk,Mybbp1a,Nampt,Cyp1b1,Armcx3,Nol8,Bfar,Pdgfc,Mbd1,Sec61b,Prkag2,Snx6,Utp6,Dhx30,Garem,Tat,Mlx,Cd28,Cebpg,Timp1,Cebpd,Ddx52,Mettl1,Mettl3,Ezr,Hnrnpa2b1,Senp6,Hnrnpab,Polr1e,Il1rn,Pim3,Arl6ip5,Metap2,Cog6,Hck,Etf1,Ednra,Btg2,Ntmt1,Dhx40,Anapc4,Wdr77,Mvp,Agk,Adcy1,Ppap2a,Map3k3,Krtcap2,B4galt1,Pole4,Mvd,Bmper,Ficd,Tcf25,Ldha,Rab12,Ints7,Ints5,Rpf2,Qsox1,Hsp90b1,Plcl1,Atp6v0a1,Fktn,Pus3,Errfi1,Cxcl9,Ung,Ctgf,Nktr,Epm2aip1,Pdcd10,Trnt1,Mphosph8,Klf17,Gpt2,Pnp,Hif1a,Mphosph6,Smarce1,Eif4e,Dap,Marveld3,Agpat9,Sf3b4,Eif3d,Syvn1,Prg4,Srsf2,Braf,Scly,Timp3,Creb3,Senp2,Efna1,Ell2,Nod1,Fbxo6,Ctif,Mcfd2,Gmeb1,Cyp7b1,Ibtk,Lrrc16a,Apex1,Ube2s,Trim39,Csnk1a1,Pcyox1,Npm3,Mmp8,Tasp1,Aldh18a1,Mmp13,Pcid2,Ddx21,Pfkp,Kdm6a,Cwc27,Gls,Rlim,Dhx38,B4galnt1,Cd55,Dok2,Psma1,Atp7a,Ubiad1,Usp8,Entpd7,Usp1,Tnfaip1,Eogt,Aak1,Recql,Mrpl51,Acpp,Mbnl2,Golt1b,Ell,Tbp,Irf8,Trib1,Dhx35,Grb2,Cebpz,Atf6,Ints2,Atf3,Herpud1,Slc11a2,Gzma,Slc11a1,Mthfr,Tpp2,Mmp9,Exosc1,Exosc4,Cad,Lbp,Serpina3k,Serpina3m,Serpina3n,Serpina3g,Snip1,Skil,Stt3a,Lpin1,Ddx50,Polh,Gtf2f1,Nup98,Uba5,Apcs,Jmjd6,Lpar1,Foxk2,Yod1,Fgr,Arf4,Camkk2,Ube2f,Fgb,Rpn1,Tgm2,Uspl1,S100a8,Tmsb4x,Ppp4c,Acot9,A2m,Egf,Eif4g2,Slc35c1,Fkbp11,Stag1,Fgl1,Nedd4l,C1galt1,Fen1,Wdr61,Ebna1bp2,Pkm,Trps1,Derl1,Spin1,Iars,Traf7,Pdia3,Acer2,Dpy19l1,Saa3,Rars,Hdc,Usp14,Tbl1xr1,Pfkfb2,Srebf2,Ufm1,Suco,Far2,Cul5,Ranbp2,Cln8,Trappc2,H2afx,Ube2j1,Vcp,Scnm1,Mcm2,Dab2,Sf1,Dusp2,Asb4,Syk,St6gal1,Hmga2,Ppip5k1,Vimp,Adam17,Srgn,Asns,Hyal1,Eif4a3,Ddx39,Cd44,Myh9,Serp1,Neurl3,Ctla2a,Ctla2b,Prmt1,Dzip3,Cyp51,Prdm1,Atf6b,Iqgap1,Pcsk5,Creb3l2,Wdr12,Dnajc10,F13a1,Plek,Idi1,Nelfa,Rbm39,Sox9,Nus1,Sdf2l1,Odc1,Papss1,Wfs1,Supt5,Supt6,Hipk3,Tmem165,Helb,Ngp,Txnl1,Gas6,Trim27,Rac2,Ppp2r1a,Il12rb1,Crem,Paip2b,Irak2,Hist1h3b,Gemin8,Ngdn,Lyve1,Cdc73,Parn,Lig3,Gata6,Exog,Ncf1,Tspyl2,Srm,Smarcad1,Dis3,Gfpt1,Pld1,Tha1,Rasgrp1,Nop58,Tirap,Trp53inp2,Fkbp5,Tlr1,Med17,Apoa4,Apoa5,Trip12,Hars,Zfp869,Ddost,Ick,Gigyf2,Hnf4a,Adam9,Zfp160,Tra2b,Hspa5,Atf7ip,Csgalnact2,Golph3,Jun,Pdia6,Dnajb11,Nlrp12,Nudt21,Papola,Flot1,Crp,Ubxn2b,Denr,Cars2,Capn10,Farsb,Taf2,Ctps,Nup62,Vars,Pdia4,Wipi1,Ppapdc1b,Ifrd1,Crtc2,Secisbp2,Ptpn2,Ptpn1,Ap1ar,Abca1,Ufl1,Ddx49,Casp4,Znrf1,Plaur,Nceh1,Preb,Parp2,Parp3,Mppe1,Zbtb16,Zbtb21,Med25,Eif2b4,Ppip5k2,Ddx1,Thoc2,Thoc1,Elp3,Inhbe,B3galt6,B3galt1,Rnps1,Tfb2m,Cdk7,Dph5,Exosc10,Zfp830,Brd8,Fbxo31,Il1a,Alg12,Rps9,Nabp1,Kars,Cda,Stk17b,Son,Pggt1b,Tiparp,Eya3,Slc9a3r1,Prkrip1,Sec22b,Psen1,Ap5s1,Erp44,Exosc3,Hltf,Zfand2a,Cd38,Piga,Itgb3,Itgb2,Arid5a,Ccl19,Khsrp,Gan,Gtf2a2,Nfia,Wdr45b,Atg16l2,Trmt61a,Gne,Kctd10,Eprs,D19Bwg1357e,St3gal3,Dnajc3,Trim68,Rcor1,Trem3,Socs3,Dr1,Camk2b,Polr1a,Ubxn4,Trim3,Txndc5,Rps6ka3,Tnfrsf1b,Tnfrsf1a,Nek7,Cpeb2,Edem3,Trib2,Edem1,Tmem173,Rab1,Cdkn1a,Taf4b,Nbas,Hk2,Gadd45b,Sds,Gadd45g,Ppp1r11,Gale,Magoh,Sbno2,Sod2,Msn,Pik3c2a,Nol9,Etnk2,Vegfc,Pycard,Adk,Trp63,Fn1,Stx5a,Clock,Bbs4,Alox12,B3galnt2,St3gal5,St3gal4,Pomgnt1,Chmp4b,Ppm1l,Ccr1,Cr1l,Fabp4,Fabp5,Hsp90aa1,Ero1l,Lss,Srsf1,Fcgr3,Srsf3,Psmd14,Scara5,Zfp27,Serpina10,Calr,Pgm3,Rusc1,Nop56,Pofut2,Polr3k,Galnt2,Tbrg1,Taf1,Pspc1,Nfkbiz,Trmt10a,Enc1,Srrt,Josd2,Lats2,Prkch,Ears2,Prkce,Acot11,Klk1b24,Crnkl1,Irg1,Rbm42,Larp7,Anapc16,Il6st,Ikbkap,Nif3l1,S100a9,Ube2g2 |
| LV | G4 | cellular metabolic process | 3.5E-06 | Cers6,Lars,Sdsl,Rad1,Selk,Mybbp1a,Nampt,Cyp1b1,Armcx3,Nol8,Bfar,Slamf8,Pdgfc,Mbd1,Sec61b,Prkag2,Snx6,Atg13,Utp6,Dhx30,Garem,Tat,Mlx,Cd28,Cebpg,Timp1,Cebpd,Ddx52,Mettl1,Mettl3,Ezr,Hnrnpa2b1,Senp6,Hnrnpab,Polr1e,Pim3,Arl6ip5,Metap2,Hck,Etf1,Ednra,Btg2,Ntmt1,Dhx40,Anapc4,Wdr77,Mvp,Agk,Adcy1,Ppap2a,Map3k3,Krtcap2,B4galt1,Pole4,Mvd,Bmper,Ficd,Tcf25,Ldha,Rab12,Ints7,Ints5,Rpf2,Qsox1,Hsp90b1,Plcl1,Atp6v0a1,Fktn,Pus3,Errfi1,Cxcl9,Ung,Ctgf,Nktr,Epm2aip1,Pdcd10,Trnt1,Mphosph8,Klf17,Gpt2,Pnp,Hif1a,Mphosph6,Smarce1,Eif4e,Dap,Marveld3,Agpat9,Sf3b4,Eif3d,Syvn1,Prg4,Srsf2,Braf,Scly,Timp3,Creb3,Senp2,Efna1,Ell2,Nod1,Fbxo6,Ctif,Mcfd2,Gmeb1,Cyp7b1,Ibtk,Lrrc16a,Apex1,Ube2s,Trim39,Csnk1a1,Pcyox1,Npm3,Tasp1,Aldh18a1,Mmp13,Pcid2,Ddx21,Pfkp,Cwc27,Gls,Rlim,Dhx38,B4galnt1,Dok2,Psma1,Atp7a,Ubiad1,Usp8,Entpd7,Usp1,Tnfaip1,Eogt,Aak1,Recql,Mrpl51,Acpp,Mbnl2,Ell,Tbp,Irf8,Trib1,Dhx35,Grb2,Cebpz,Atf6,Ints2,Atf3,Herpud1,Slc11a2,Gzma,Slc11a1,Mthfr,Mmp9,Exosc1,Exosc4,Cad,Lbp,Serpina3k,Serpina3m,Serpina3n,Serpina3g,Rbm26,Snip1,Skil,Stt3a,Lpin1,Ddx50,Polh,Dram1,Gtf2f1,Nup98,Uba5,Apcs,Jmjd6,Lpar1,Foxk2,Yod1,Fgr,Arf4,Camkk2,Ube2f,Fgb,Rpn1,Tgm2,Uspl1,S100a8,Tmsb4x,Ppp4c,Acot9,A2m,Egf,Eif4g2,Slc35c1,Fkbp15,Fkbp11,Stag1,Fgl1,Nedd4l,C1galt1,Fen1,Wdr61,Ebna1bp2,Pkm,Trps1,Derl1,Spin1,Atg9b,Iars,Traf7,Pdia3,Acer2,Dpy19l1,Saa3,Rars,Hdc,Usp14,Tbl1xr1,Rrp1b,Pfkfb2,Srebf2,Ufm1,Far2,Cul5,Ranbp2,Cln8,Trappc2,H2afx,Ube2j1,Vcp,Scnm1,Mcm2,Dab2,Dusp2,Scfd1,Asb4,Syk,St6gal1,Hmga2,Ppip5k1,Vimp,Adam17,Asns,Rraga,Eif4a3,Ddx39,Cd44,Myh9,Serp1,Neurl3,Ctla2a,Ctla2b,Prmt1,Dzip3,Prdm1,Atf6b,Iqgap1,Pcsk5,Creb3l2,Wdr12,Dnajc10,F13a1,Plek,Idi1,Nelfa,Rbm39,Sox9,Nus1,Sdf2l1,Odc1,Papss1,Wfs1,Supt5,Supt6,Hipk3,Tmem165,Helb,Ngp,Txnl1,Gas6,Trim27,Rac2,Ppp2r1a,Il12rb1,Crem,Paip2b,Irak2,Dynll1,Iigp1,Hist1h3b,Gemin8,Ngdn,Cdc73,Parn,Lig3,Gata6,Exog,Ncf1,Tspyl2,Srm,Smarcad1,Dis3,Pld1,Tha1,Rasgrp1,Nop58,Tirap,Trp53inp2,Fkbp5,Tlr1,Med17,Apoa4,Apoa5,Trip12,Hars,Zfp869,Ddost,Ick,Gigyf2,Hnf4a,Adam9,Zfp160,Tra2b,Hspa5,Atf7ip,Csgalnact2,Golph3,Jun,Pdia6,Dnajb11,Nlrp12,Nudt21,Papola,Flot1,Crp,Ubxn2b,Denr,Cars2,Cox8a,Farsb,Taf2,Ctps,Nup62,Vars,Pdia4,Wipi1,Ppapdc1b,Ifrd1,Crtc2,Secisbp2,Ptpn2,Ptpn1,Ap1ar,Abca1,Ufl1,Ddx49,Znrf1,Plaur,Nceh1,Preb,Parp2,Parp3,Mppe1,Zbtb16,Zbtb21,Med25,Eif2b4,Ppip5k2,Ddx1,Thoc1,Elp3,Inhbe,B3galt1,Rnps1,Tfb2m,Cdk7,Dph5,Exosc10,Zfp830,Brd8,Fbxo31,Il1a,Alg12,Rps9,Nabp1,Kars,Cda,Stk17b,Son,Pggt1b,Tiparp,Eya3,Slc9a3r1,Prkrip1,Psen1,Ap5s1,Erp44,Exosc3,Hltf,Zfand2a,Cd38,Piga,Itgb3,Itgb2,Arid5a,Vav1,Ccl19,Khsrp,Gan,Gtf2a2,Nfia,Wdr45b,Atg16l2,Trmt61a,Gne,Kctd10,Eprs,D19Bwg1357e,St3gal3,Dnajc3,Trim68,Rcor1,Socs3,Dr1,Camk2b,Polr1a,Ubxn4,Trim3,Txndc5,Rps6ka3,Tnfrsf1b,Tnfrsf1a,Nek7,Cpeb2,Edem3,Trib2,Edem1,Tmem173,Rab1,Ift20,Cdkn1a,Taf4b,Nbas,Hk2,Gadd45b,Sds,Gadd45g,Ppp1r11,Magoh,Sbno2,Sod2,Pik3c2a,Nol9,Etnk2,Vegfc,Pycard,Map1lc3b,Adk,Trp63,Fn1,Clock,Alox12,B3galnt2,St3gal4,Pomgnt1,Chmp4b,Ppm1l,Ccr1,Txlna,Cr1l,Fabp4,Fabp5,Hsp90aa1,Ero1l,Srsf1,Fcgr3,Srsf3,Rab33b,Psmd14,Zfp27,Serpina10,Calr,Pgm3,Rusc1,Nop56,Pofut2,Polr3k,Galnt2,Tbrg1,Taf1,Pspc1,Nfkbiz,Trmt10a,Enc1,Srrt,Josd2,Lats2,Prkch,Ears2,Prkce,Acot11,Crnkl1,Irg1,Rbm42,Larp7,Anapc16,Il6st,Ikbkap,Nif3l1,S100a9,Ube2g2 |
| LV | G4 | glycosylation | 4.2E-06 | Cog6,Krtcap2,B4galt1,Fktn,Syvn1,Eogt,Stt3a,Arf4,Rpn1,Slc35c1,C1galt1,Acer2,Dpy19l1,Ube2j1,Vcp,St6gal1,Nus1,Tmem165,Ddost,Parp3,B3galt1,Alg12,Tiparp,Psen1,St3gal3,B3galnt2,St3gal4,Pomgnt1,Pgm3,Pofut2,Galnt2,Ube2g2 |
| LV | G4 | protein glycosylation | 6.2E-06 | Krtcap2,B4galt1,Fktn,Syvn1,Eogt,Stt3a,Arf4,Rpn1,C1galt1,Acer2,Dpy19l1,Ube2j1,Vcp,St6gal1,Nus1,Tmem165,Ddost,Parp3,B3galt1,Alg12,Tiparp,Psen1,St3gal3,B3galnt2,St3gal4,Pomgnt1,Pgm3,Pofut2,Galnt2,Ube2g2 |
| LV | G4 | Post-translational protein modification | 7.7E-06 | Pigm,B4galt1,Mvd,Senp2,Mcfd2,Mogs,Eogt,Nans,Stt3a,Nup98,Sec24d,Rpn1,Stag1,C1galt1,Pdia3,Ranbp2,St6gal1,Galnt18,Nupl2,Gas6,Gfpt1,Ddost,Nupl1,Nup62,Plaur,Preb,B3galt1,Dph5,Alg12,Sec31a,Piga,Gne,St3gal3,Edem2,Edem3,Edem1,Gmppa,Man1a,Sec13,Dhdds,St3gal5,St3gal4,Pomgnt1,Seh1l,Uap1,Calr,Pgm3,Pofut2,Galnt2,Gmppb |
| LV | G4 | Protein export | 1.8E-05 | Sec61b,Srp68,Spcs2,Spcs3,Gm10177,Srp9,Hspa5,Sec61a1,Srp19,Srprb,Srp72,Srpr,Srp54a |

[Table 18-6]

| Organ | group | Term | Pvalue | Genelist |
|---|---|---|---|---|
| LV | G4 | cellular protein metabolic process | 1.9E-05 | Lars,Selk,Bfar,Pdgfc,Sec61b,Prkag2,Garem,Timp1,Mettl3,Senp6,Pim3,Arl6ip5,Metap2,Hck,Etf1,Btg2,Ntmt1,Anapc4,Mvp,Ppap2a,Map3k3,Krtcap2,B4galt1,Pole4,Bmper,Ficd,Rab12,Qsox1,Hsp90b1,Plcl1,Fktn,Errfi1,Ctgf,Nktr,Pdcd10,Eif4e,Dap,Marveld3,Eif3d,Syvn1,Braf,Timp3,Senp2,Efna1,Nod1,Fbxo6,Ctif,Ibtk,Ube2s,Trim39,Csnk1a1,Pcyox1,Mmp13,Pcid2,Cwc27,Rlim,Dok2,Psma1,Atp7a,Usp8,Usp1,Tnfaip1,Eogt,Aak1,Mrpl51,Trib1,Grb2,Atf3,Herpud1,Slc11a2,Gzma,Slc11a1,Mthfr,Mmp9,Cad,Serpina3k,Serpina3m,Serpina3n,Serpina3g,Stt3a,Lpin1,Uba5,Jmjd6,Lpar1,Yod1,Fgr,Arf4,Camkk2,Ube2f,Fgb,Rpn1,Tgm2,Uspl1,S100a8,A2m,Egf,Eif4g2,Fkbp11,Nedd4l,C1galt1,Wdr61,Derl1,Iars,Traf7,Pdia3,Acer2,Dpy19l1,Saa3,Rars,Usp14,Tbl1xr1,Ufm1,Cul5,Cln8,Ube2j1,Vcp,Dab2,Dusp2,Asb4,Syk,St6gal1,Hmga2,Vimp,Adam17,Cd44,Myh9,Serp1,Neurl3,Ctla2a,Ctla2b,Prmt1,Dzip3,Iqgap1,Pcsk5,Dnajc10,F13a1,Sox9,Nus1,Sdf2l1,Wfs1,Supt6,Hipk3,Tmem165,Ngp,Txnl1,Gas6,Trim27,Rac2,Ppp2r1a,Il12rb1,Paip2b,Cdc73,Tspyl2,Smarcad1,Rasgrp1,Tirap,Fkbp5,Tlr1,Trip12,Hars,Ddost,Ick,Gigyf2,Adam9,Hspa5,Jun,Pdia6,Nlrp12,Ubxn2b,Denr,Cars2,Farsb,Nup62,Vars,Pdia4,Wipi1,Crtc2,Secisbp2,Ptpn2,Ptpn1,Abca1,Ufl1,Znrf1,Plaur,Nceh1,Parp3,Mppe1,Eif2b4,Elp3,Inhbe,B3galt1,Dph5,Brd8,Fbxo31,Il1a,Alg12,Rps9,Kars,Stk17b,Pggt1b,Tiparp,Eya3,Slc9a3r1,Prkrip1,Psen1,Erp44,Zfand2a,Piga,Itgb3,Itgb2,Arid5a,Ccl19,Gan,Wdr45b,Kctd10,Eprs,D19Bwg1357e,St3gal3,Dnajc3,Trim68,Rcor1,Socs3,Dr1,Camk2b,Ubxn4,Trim3,Txndc5,Rps6ka3,Tnfrsf1b,Tnfrsf1a,Nek7,Cpeb2,Edem3,Trib2,Edem1,Cdkn1a,Gadd45b,Gadd45g,Ppp1r11,Pik3c2a,Vegfc,Pycard,Fn1,Clock,B3galnt2,St3gal4,Pomgnt1,Chmp4b,Ppm1l,Ccr1,Cr1l,Fabp4,Hsp90aa1,Ero1l,Psmd14,Serpina10,Pgm3,Rusc1,Pofut2,Galnt2,Taf1,Enc1,Josd2,Lats2,Prkch,Ears2,Prkce,Anapc16,Il6st,Ikbkap,S100a9,Ube2g2 |
| LV | G4 | Post-translational protein modification | 2.1E-05 | Pigm,B4galt1,Mvd,Senp2,Mcfd2,Mogs,Nans,Nup98,Sec24d,Stag1,C1galt1,Pdia3,Ranbp2,St6gal1,Nupl2,Gas6,Gfpt1,Nupl1,Nup62,Plaur,Preb,Dph5,Alg12,Sec31a,Piga,Gne,St3gal3,Edem2,Edem3,Edem1,Gmppa,Man1a,Sec13,Dhdds,St3gal5,St3gal4,Seh1l,Uap1,Calr,Pgm3,Pofut2,Galnt2,Gmppb |
| LV | G4 | protein metabolic process | 3.1E-05 | Lars,Selk,Bfar,Pdgfc,Sec61b,Prkag2,Garem,Cd28,Cebpg,Timp1,Mettl3,Senp6,Pim3,Arl6ip5,Metap2,Hck,Etf1,Btg2,Ntmt1,Anapc4,Mvp,Ppap2a,Map3k3,Krtcap2,B4galt1,Pole4,Bmper,Ficd,Rab12,Qsox1,Hsp90b1,Plcl1,Fktn,Errfi1,Ctgf,Nktr,Pdcd10,Hif1a,Eif4e,Dap,Marveld3,Eif3d,Syvn1,Prg4,Braf,Timp3,Senp2,Efna1,Nod1,Fbxo6,Ctif,Ibtk,Ube2s,Trim39,Csnk1a1,Pcyox1,Mmp8,Tasp1,Mmp13,Pcid2,Cwc27,Rlim,Cd55,Dok2,Psma1,Atp7a,Usp8,Usp1,Tnfaip1,Eogt,Aak1,Mrpl51,Trib1,Grb2,Atf3,Herpud1,Slc11a2,Gzma,Slc11a1,Mthfr,Tpp2,Mmp9,Cad,Lbp,Serpina3k,Serpina3m,Serpina3n,Serpina3g,Stt3a,Lpin1,Uba5,Apcs,Jmjd6,Lpar1,Yod1,Fgr,Arf4,Camkk2,Ube2f,Fgb,Rpn1,Tgm2,Uspl1,S100a8,A2m,Egf,Eif4g2,Fkbp11,Nedd4l,C1galt1,Wdr61,Derl1,Iars,Traf7,Pdia3,Acer2,Dpy19l1,Saa3,Rars,Usp14,Tbl1xr1,Ufm1,Cul5,Cln8,Ube2j1,Vcp,Dab2,Dusp2,Asb4,Syk,St6gal1,Hmga2,Vimp,Adam17,Srgn,Cd44,Myh9,Serp1,Neurl3,Ctla2a,Ctla2b,Prmt1,Dzip3,Iqgap1,Pcsk5,Dnajc10,F13a1,Sox9,Nus1,Sdf2l1,Odc1,Wfs1,Supt6,Hipk3,Tmem165,Ngp,Txnl1,Gas6,Trim27,Rac2,Ppp2r1a,Il12rb1,Paip2b,Cdc73,Tspyl2,Smarcad1,Rasgrp1,Tirap,Fkbp5,Tlr1,Apoa4,Apoa5,Trip12,Hars,Ddost,Ick,Gigyf2,Adam9,Hspa5,Csgalnact2,Golph3,Jun,Pdia6,Nlrp12,Ubxn2b,Denr,Cars2,Capn10,Farsb,Nup62,Vars,Pdia4,Wipi1,Crtc2,Secisbp2,Ptpn2,Ptpn1,Abca1,Ufl1,Casp4,Znrf1,Plaur,Nceh1,Parp3,Mppe1,Eif2b4,Elp3,Inhbe,B3galt1,Dph5,Brd8,Fbxo31,Il1a,Alg12,Rps9,Kars,Stk17b,Pggt1b,Tiparp,Eya3,Slc9a3r1,Prkrip1,Sec22b,Psen1,Erp44,Zfand2a,Piga,Itgb3,Itgb2,Arid5a,Ccl19,Gan,Wdr45b,Kctd10,Eprs,D19Bwg1357e,St3gal3,Dnajc3,Trim68,Rcor1,Trem3,Socs3,Dr1,Camk2b,Ubxn4,Trim3,Txndc5,Rps6ka3,Tnfrsf1b,Tnfrsf1a,Nek7,Cpeb2,Edem3,Trib2,Edem1,Rab1,Cdkn1a,Gadd45b,Gadd45g,Ppp1r11,Pik3c2a,Vegfc,Pycard,Trp63,Fn1,Stx5a,Clock,B3galnt2,St3gal4,Pomgnt1,Chmp4b,Ppm1l,Ccr1,Cr1l,Fabp4,Hsp90aa1,Ero1l,Fcgr3,Psmd14,Serpina10,Pgm3,Rusc1,Pofut2,Galnt2,Taf1,Enc1,Josd2,Lats2,Prkch,Ears2,Prkce,Klk1b24,Anapc16,Il6st,Ikbkap,S100a9,Ube2g2 |
| LV | G4 | glycoprotein metabolic process | 4.6E-05 | Krtcap2,B4galt1,Fktn,Hif1a,Syvn1,Fbxo6,Atp7a,Eogt,Stt3a,Apcs,Arf4,Rpn1,C1galt1,Acer2,Dpy19l1,Ube2j1,Vcp,St6gal1,Nus1,Tmem165,Ddost,Csgalnact2,Golph3,Parp3,B3galt1,Alg12,Tiparp,Psen1,Erp44,Ccl19,St3gal3,Edem3,Rab1,B3galnt2,St3gal4,Pomgnt1,Pgm3,Pofut2,Galnt2,Ube2g2 |
| LV | G4 | regulation of response to stress | 0.0001 | Selp,Pik3ap1,Bfar,Cd28,Cebpg,Arl6ip5,Map3k3,Il1r1,Rab12,Qsox1,Fktn,Ctgf,Pdcd10,Pnp,Hif1a,Marveld3,Syvn1,Braf,Scly,Creb3,Senp2,Nod1,Apex1,Hilpda,Cd55,Serinc3,Psma1,Adora1,Usp1,Tnfaip6,Rbm18,Herpud1,Lbp,Skil,Ap1g1,Fgr,S100a8,Ppp4c,A2m,Slc35c1,Fcer1g,Dab2,Tnfrsf12a,Scfd1,Syk,Hmga2,Vimp,Cd44,Ctla2a,Plek,Wfs1,Supt5,Hipk3,Il12rb1,Irak2,Grina,Rasgrp1,Tirap,Trip12,Nlrp12,Cox8a,Ptpn2,Ptpn1,Casp4,Il17ra,Thoc1,Il1a,Eya3,Vav1,Ccl19,Dnajc3,Socs3,Rps6ka3,Tnfrsf1b,Tnfrsf1a,Rint1,Tmem173,Ift20,Hyou1,Gadd45b,Gadd45g,Sbno2,Sod2,Pycard,Trp63,Clock,Alox12,Chmp4b,Ccr1,Cr1l,Fabp4,Fcgr3,Rab33b,Scara5,Polr3d,Prkce,Crnkl1,Irg1,Myo1f,S100a9 |
| LV | G4 | Metal ion SLC transporters | 0.00014 | Slc41a1,Slc30a5,Slc39a6,Slc11a2,Slc11a1,Slc39a7,Slc39a10,Slc39a14,Slc30a7,Cp,Slc41a2 |
| LV | G4 | ncRNA metabolic process | 0.00014 | Lars,Nol8,Utp6,Ddx52,Mettl1,Polr1e,Ints7,Ints5,Rpf2,Pus3,Trnt1,Mphosph6,Ell2,Npm3,Ell,Ints2,Exosc4,Ebna1bp2,Spin1,Iars,Rars,Wdr12,Ngdn,Dis3,Nop58,Hars,Cars2,Farsb,Vars,Ddx1,Elp3,Exosc10,Kars,Exosc3,Khsrp,Trmt61a,Nol9,Nop56,Trmt10a,Srrt,Ears2 |
| LV | G4 | ER-associated ubiquitin-dependent protein catabolic process | 0.00023 | Sec61b,Hsp90b1,Syvn1,Fbxo6,Herpud1,Yod1,Derl1,Usp14,Ube2j1,Vcp,Vimp,Dnajc10,Sdf2l1,Wfs1,Ubxn4,Edem1,Ube2g2 |
| LV | G4 | response to unfolded protein | 0.00026 | Tbl2,Bfar,Syvn1,Creb3,Atf3,Herpud1,Yod1,Derl1,Vimp,Serp1,Wfs1,Hspa5,Ptpn2,Ptpn1,Erp44,Dnajc3,Hsp90aa1,Ero1l |
| LV | G4 | glycoprotein biosynthetic process | 0.00028 | Krtcap2,B4galt1,Fktn,Syvn1,Atp7a,Eogt,Stt3a,Arf4,Rpn1,C1galt1,Acer2,Dpy19l1,Ube2j1,Vcp,St6gal1,Nus1,Tmem165,Ddost,Csgalnact2,Golph3,Parp3,B3galt1,Alg12,Tiparp,Psen1,Ccl19,St3gal3,B3galnt2,St3gal4,Pomgnt1,Pgm3,Pofut2,Galnt2,Ube2g2 |
| LV | G4 | carbohydrate metabolic process | 0.00028 | Prkag2,Cog6,Krtcap2,B4galt1,Fktn,Epm2aip1,Hif1a,Syvn1,Pfkp,Eogt,Atf3,Stt3a,Arf4,Rpn1,Egf,Slc35c1,Fgl1,C1galt1,Pkm,Acer2,Dpy19l1,Pfkfb2,Ranbp2,Ube2j1,Vcp,St6gal1,Ppip5k1,Serp1,Plek,Nus1,Tmem165,Ddost,Crtc2,Ptpn2,Parp3,Ppip5k2,B3galt1,Alg12,Tiparp,Psen1,St3gal3,Hk2,Gale,B3galnt2,St3gal4,Pomgnt1,Fabp5,Pgm3,Pofut2,Galnt2,Prkce,Il6st,Ube2g2 |
| LV | G4 | response to topologically incorrect protein | 0.00046 | Tbl2,Bfar,Syvn1,Creb3,Atf3,Herpud1,Yod1,Derl1,Vimp,Serp1,Sdf2l1,Wfs1,Hspa5,Ptpn2,Ptpn1,Erp44,Dnajc3,Hsp90aa1,Ero1l |
| LV | G4 | catabolic process | 0.0006 | Sdsl,Cyp1b1,Bfar,Sec61b,Prkag2,Atg13,Tat,Timp1,Btg2,Rab12,Qsox1,Hsp90b1,Pnp,Hif1a,Dap,Syvn1,Timp3,Fbxo6,Ctif,Ube2s,Trim39,Pcyox1,Mmp13,Pcid2,Pfkp,Gls,Rlim,Psma1,Usp8,Entpd7,Usp1,Tnfaip1,Trib1,Herpud1,Gzma,Mmp9,Exosc4,Lpin1,Dram1,Yod1,S100a8,Egf,Slc35c1,Nedd4l,Fen1,Wdr61,Pkm,Derl1,Atg9b,Hdc,Usp14,Tbl1xr1,Pfkfb2,Cul5,Cln8,Ube2j1,Vcp,Dab2,Scfd1,Vimp,Rraga,Hyal1,Cd44,Ctla2a,Ctla2b,Dnajc10,Sox9,Sdf2l1,Odc1,Wfs1,Supt5,Iigp1,Lyve1,Parn,Exog,Ncf1,Dis3,Tha1,Trp53inp2,Apoa4,Apoa5,Trip12,Adam9,Hspa5,Ubxn2b,Cox8a,Wipi1,Secisbp2,Ptpn1,Ufl1,Znrf1,Rnps1,Exosc10,Fbxo31,Cda,Tiparp,Sec22b,Psen1,Exosc3,Zfand2a,Khsrp,Wdr45b,Atg16l2,Kctd10,Dnajc3,Ubxn4,Tnfrsf1b,Edem3,Trib2,Edem1,Rab1,Ift20,Nbas,Hk2,Sds,Gale,Magoh,Pycard,Map1lc3b,Stx5a,Clock,Chmp4b,Txlna,Rab33b,Psmd14,Taf1,Enc1,Prkce,Crnkl1,S100a9,Ube2g2 |

[Table 18-7]

| Organ | group | Term | Pvalue | Genelist |
|---|---|---|---|---|
| LV | G4 | catabolic process | 0.0006 | Sdsl,Cyp1b1,Bfar,Sec61b,Prkag2,Atg13,Tat,Timp1,Btg2,Rab12,Qsox1,Hsp90b1,Pnp,Hif1a,Dap,Syvn1,Timp3,Fbxo6,Ctif,Ube2s,Trim39,Pcyox1,Mmp13,Pcid2,Pfkp,Gls,Rlim,Psma1,Usp8,Entpd7,Usp1,Tnfaip1,Trib1,Herpud1,Gzma,Mmp9,Exosc4,Lpin1,Dram1,Yod1,S100a8,Egf,Slc35c1,Nedd4l,Fen1,Wdr61,Pkm,Derl1,Atg9b,Hdc,Usp14,Tbl1xr1,Pfkfb2,Cul5,Cln8,Ube2j1,Vcp,Dab2,Scfd1,Vimp,Rraga,Hyal1,Cd44,Ctla2a,Ctla2b,Dnajc10,Sox9,Sdf2l1,Odc1,Wfs1,Supt5,Iigp1,Lyve1,Parn,Exog,Ncf1,Dis3,Tha1,Trp53inp2,Apoa4,Apoa5,Trip12,Adam9,Hspa5,Ubxn2b,Cox8a,Wipi1,Secisbp2,Ptpn1,Ufl1,Znrf1,Rnps1,Exosc10,Fbxo31,Cda,Tiparp,Sec22b,Psen1,Exosc3,Zfand2a,Khsrp,Wdr45b,Atg16l2,Kctd10,Dnajc3,Ubxn4,Tnfrsf1b,Edem3,Trib2,Edem1,Rab1,Ift20,Nbas,Hk2,Sds,Gale,Magoh,Pycard,Map1lc3b,Stx5a,Clock,Chmp4b,Txlna,Rab33b,Psmd14,Taf1,Enc1,Prkce,Crnkl1,S100a9,Ube2g2 |
| LV | G4 | Golgi vesicle transport | 0.00067 | Yipf5,Golga5,Cog6,Stx18,Arcn1,Uso1,Krt18,Gosr2,Blzf1,Ap1g1,Tmed9,Sar1a,Vamp4,Rab35,Copb2,Zw10,Creb3l2,Chic2,Golga4,Golph3,Wipi1,Ap1ar,Mppe1,Bet1l,Sec22b,Rint1,Rab1,Sec13,Stx5a,Rab33b |
| LV | G4 | tRNA metabolic process | 0.00075 | Lars,Mettl1,Pus3,Trnt1,Iars,Rars,Hars,Cars2,Farsb,Vars,Ddx1,Elp3,Kars,Exosc3,Trmt61a,Trmt10a,Ears2 |
| LV | G4 | Gene Expression | 0.0009 | Cnot11,Sec61b,Prkag2,Mettl3,Hnrnpa2b1,Polr1e,Etf1,Wdr77,Srp68,Eif4e,Sf3b4,Eif3d,Gtf2e1,Zfp143,Ssr1,Ssr2,Plrg1,Gls,Dhx38,Alyref,Psma1,Ell,Spcs2,Spcs3,Tbp,Ssr4,Exosc1,Exosc4,Skil,Gtf2f1,Nup98,Gspt2,Snrnp40,Rpn1,Ccar1,Nedd4l,Ranbp2,H2afx,Casc3,Gtf2f2,Nupl2,Nelfa,Supt5,Ppp2r1a,Srp9,Hist1h3b,Ssb,Parn,Dis3,Med15,Med17,Ddost,Hist1h2bp,Hnf4a,Hist1h2bf,Sec61a1,Nudt21,Papola,Nupl1,Cox8a,Srp19,Taf2,Nup62,Srprb,Med25,Eif2b4,Rnps1,Med20,Tfb2m,Cdk7,Rps9,Exosc3,Khsrp,Gtf2a2,Nfia,Polr1a,Srp72,Taf4b,Magoh,Srpr,Seh1l,Hsp90aa1,Srp54a,Srsf1,Srsf3,Psmd14,Polr3d,Polr3k,Taf1 |
| LV | G4 | cellular protein modification process | 0.00107 | Selk,Bfar,Pdgfc,Prkag2,Garem,Senp6,Pim3,Arl6ip5,Metap2,Hck,Etf1,Btg2,Ntmt1,Anapc4,Mvp,Ppap2a,Map3k3,Krtcap2,B4galt1,Pole4,Bmper,Ficd,Plcl1,Fktn,Errfi1,Ctgf,Nktr,Pdcd10,Marveld3,Syvn1,Braf,Senp2,Efna1,Nod1,Ibtk,Ube2s,Trim39,Csnk1a1,Pcid2,Cwc27,Rlim,Dok2,Atp7a,Usp8,Usp1,Tnfaip1,Eogt,Aak1,Trib1,Grb2,Atf3,Herpud1,Slc11a1,Mthfr,Mmp9,Cad,Stt3a,Lpin1,Uba5,Jmjd6,Lpar1,Yod1,Fgr,Arf4,Camkk2,Ube2f,Fgb,Rpn1,Tgm2,Uspl1,S100a8,Egf,Fkbp11,Nedd4l,C1galt1,Wdr61,Traf7,Acer2,Dpy19l1,Saa3,Usp14,Tbl1xr1,Ufm1,Cul5,Ube2j1,Vcp,Dab2,Dusp2,Asb4,Syk,St6gal1,Hmga2,Adam17,Cd44,Neurl3,Prmt1,Dzip3,Iqgap1,Dnajc10,F13a1,Sox9,Nus1,Wfs1,Supt6,Hipk3,Tmem165,Gas6,Trim27,Rac2,Ppp2r1a,Il12rb1,Cdc73,Tspyl2,Smarcad1,Rasgrp1,Tirap,Fkbp5,Tlr1,Trip12,Ddost,Ick,Adam9,Hspa5,Jun,Nlrp12,Nup62,Wipi1,Crtc2,Ptpn2,Ptpn1,Abca1,Ufl1,Znrf1,Plaur,Nceh1,Parp3,Mppe1,Elp3,Inhbe,B3galt1,Dph5,Brd8,Fbxo31,Il1a,Alg12,Stk17b,Pggt1b,Tiparp,Eya3,Slc9a3r1,Prkrip1,Psen1,Piga,Itgb3,Itgb2,Arid5a,Ccl19,Gan,Wdr45b,St3gal3,Dnajc3,Trim68,Rcor1,Socs3,Dr1,Camk2b,Trim3,Rps6ka3,Tnfrsf1a,Nek7,Trib2,Cdkn1a,Gadd45b,Gadd45g,Ppp1r11,Pik3c2a,Vegfc,Pycard,Fn1,Clock,B3galnt2,St3gal4,Pomgnt1,Ppm1l,Ccr1,Fabp4,Psmd14,Pgm3,Rusc1,Pofut2,Galnt2,Taf1,Enc1,Josd2,Lats2,Prkch,Prkce,Anapc16,Il6st,Ikbkap,S100a9,Ube2g2 |
| LV | G4 | transport | 0.00108 | Selk,Mybbp1a,Mfsd7b,Yipf5,Unc50,Sec61b,Golga5,Snx6,Atg13,Mlx,Ezr,Rinl,Hnrnpa2b1,Stx6,Il1rn,Pim3,Arl6ip5,Cog6,Hck,Ednra,B4galt1,Rab12,Srp68,Stx12,Atp6v0a1,Stx18,Cxcl9,Arcn1,Uso1,Hif1a,Slc37a3,Slc37a1,Lrrc8a,Lcn2,Krt18,Braf,Slc30a5,Timm21,Creb3,Rabl3,Slc39a6,Ibtk,Csnk1a1,Slco3a1,Copa,Serinc3,Atp7a,Slc16a10,Adora1,Gosr2,Aak1,Marco,Irf8,Grb2,Blzf1,Slc11a2,Slc11a1,Mmp9,Pdzd11,Lbp,Snip1,Atp11a,Wdr1,Cd14,Jmjd6,Ap1g1,Fgr,Fgb,Tgm2,Cnnm2,S100a8,Tmed9,Egf,Slc35c1,Cct3,Ica1,Ap2a2,Derl1,Atp2a2,Litaf,Sar1a,Itpr1,Yrdc,Ipo4,Vamp4,Rab35,Fcer1g,Zfyve16,Srebf2,Slc4a4,Cln8,Slc16a6,Vcp,Dab2,Actr2,Scfd1,Syk,Hmga2,Vimp,Srgn,Ap4e1,Copb2,Ddx39,Myh9,Serp1,Zw10,Slc39a14,Golph3l,Rab5b,Mmgt1,Creb3l2,Slc30a7,Plek,Steap4,Slc2a3,Nus1,Wfs1,Chic2,Cklf,Supt6,Tmem165,Slc15a2,Gas6,Trim27,Rac2,Zdhhc13,Mfsd2a,Golga4,Dynll1,Mon1b,Msr1,Sdc4,Gm21540,Dst,Ncf1,Slc10a6,Rasgrp1,Slc10a2,Apoa4,Apoa5,Ick,Pcm1,Kif5b,Hnf4a,Adam9,Hspa5,Golph3,Jun,Sec61a1,Nlrp12,Flot1,Crp,Emb,Capn10,Wipi1,Sidt2,Rab18,Crtc2,Ptpn1,Ap1ar,Abca1,Casp4,Slc38a10,Scamp2,Mppe1,Il4ra,Antxr2,Thoc2,Thoc1,Il1a,Bet1l,Ap3d1,Slc38a2,Syt12,Slc9a3r1,Sec22b,Hook1,Psen1,Ap5s1,Itgb3,Vav1,Ccl19,Slc7a6,Slc35a2,Snx10,S100a10,Trem3,Cltb,Camk2b,Txndc5,Rint1,Srp72,Tmem173,Rab1,Ift20,C2cd5,Cdkn1a,Hk2,Snap23,Sec13,Pik3c2a,Coro1a,Sybu,Pycard,Map1lc3b,Stx5a,Clock,Chmp4b,Nemf,Ccr1,Slc35e1,Clec4n,Slc13a3,Slc13a5,Fabp5,Hsp90aa1,Ero1l,Fcgr3,Rab33b,Scara5,Calr,Lcp2,Pofut2,Tbrg1,Atp6v0e,Prkce,Myo1f,Arfgap1,S100a9 |
| LV | G4 | RNA processing | 0.00118 | Nol8,Utp6,Dhx30,Ddx52,Mettl1,Mettl3,Hnrnpa2b1,Btg2,Dhx40,Ints7,Ints5,Rpf2,Pus3,Trnt1,Mphosph6,Sf3b4,Srsf2,Npm3,Dhx38,Mbnl2,Dhx35,Ints2,Exosc1,Exosc4,Snip1,Jmjd6,Ebna1bp2,Scnm1,Eif4a3,Ddx39,Wdr12,Supt5,Supt6,Gemin8,Ngdn,Cdc73,Dis3,Nop58,Tra2b,Nudt21,Papola,Ddx1,Elp3,Rnps1,Exosc10,Son,Exosc3,Trmt61a,Magoh,Nol9,Srsf1,Srsf3,Nop56,Trmt10a,Srrt,Crnkl1,Rbm42 |
| LV | G4 | endoplasmic reticulum unfolded protein response | 0.00134 | Tbl2,Bfar,Creb3,Atf3,Herpud1,Yod1,Derl1,Vimp,Serp1,Wfs1,Hspa5,Ptpn2,Ptpn1,Dnajc3,Ero1l |
| LV | G4 | Vesicle-mediated transport | 0.00151 | Prkag2,Hsp90b1,Arcn1,Copa,Copb1,Marco,Sec24d,Ap1g1,Clint1,Copg1,Dab2,Ap4e1,Copb2,Myh9,Msr1,Tbc1d8b,Ralgapa2,Preb,Sec31a,Tpd52,Cltb,Txndc5,C2cd5,Hyou1,Yipf6,Snap23,Sec13,Pik3c2a,Chmp4b,Hsp90aa1,Scara5,Calr,Arfgap1 |
| LV | G4 | inflammatory response | 0.00172 | Selp,Pik3ap1,Cd28,Il1rn,B4galt1,Il1r1,Cxcl9,Hif1a,Cd55,Psma1,Adora1,Tnfaip6,Slc11a1,Lbp,S100a8,A2m,Fcer1g,Syk,Vimp,Adam17,Hyal1,Cd44,Ctla2a,Itgam,Ncf1,Rasgrp1,Nlrp12,Ptpn2,Casp4,Il17ra,Il1a,Socs3,Tnfrsf1b,Tnfrsf1a,Sbno2,Snap23,Pik3c2a,Pycard,Clock,Ccr1,Plaa,Seh1l,Cr1l,Fabp4,Fcgr3,Nfkbiz,Irg1,S100a9 |
| LV | G4 | response to external stimulus | 0.0019 | Mybbp1a,Tbl2,Selp,Pik3ap1,Slamf8,Atg13,Cd28,Hck,Ldha,Il1r1,Rab12,Qsox1,Ppan,Cxcl9,Dap,Lcn2,Braf,Creb3,Efna1,Nod1,Clec4d,Hilpda,Ddx21,Cd55,Psma1,Adora1,Tnfaip6,Irf8,Trib1,Rbm18,Atf3,Slc11a1,Exosc4,Lbp,Cd14,Ap1g1,Fgr,Vasp,Fgb,S100a8,A2m,Slc35c1,Ifitm2,Ifitm1,Atg9b,Litaf,Saa3,Usp14,Fcer1g,Tbl1xr1,Srebf2,Bag3,Fpr1,Scfd1,Syk,Vimp,Adam17,Asns,Rraga,Hyal1,Ctla2a,Prdm1,Reg3b,Plek,Sox9,Supt5,Cklf,Gas6,Rac2,Il12rb1,Irak2,Iigp1,Cdc73,Itgam,Ncf1,Pld1,Tirap,Trp53inp2,Tlr1,Kif5b,Adam9,Hspa5,Jun,Nlrp12,Flot1,Ubxn2b,Cox8a,Wipi1,Ptpn2,Abca1,Casp4,Il17ra,Il4ra,Ddx1,Ap3d1,Psen1,Itgb3,Itgb2,Ccl17,Vav1,Ccl19,Wdr45b,Atg16l2,Trem3,Socs3,Rps6ka3,Tnfrsf1b,Tnfrsf1a,Tmem173,Rab1,Ift20,Cdkn1a,Sbno2,Pik3c2a,Coro1a,Vegfc,Pycard,Map1lc3b,Clock,Bbs4,Alox12,Chmp4b,Ccr1,Clec4n,Seh1l,Cr1l,Fabp4,Fcgr3,Rab33b,Scara5,Calr,Nfkbib,Prkce,Crnkl1,Irg1,Myo1f,S100a9 |
| LV | G4 | ribosome biogenesis | 0.00196 | Nol8,Utp6,Ddx52,Rpf2,Mphosph6,Npm3,Tsr1,Exosc4,Ebna1bp2,Ipo4,Wdr12,Gnl2,Gnl1,Ngdn,Dis3,Nop58,Brix1,Exosc10,Exosc3,Nol9,Nop56,Eif6 |
| LV | G4 | protein N-linked glycosylation via asparagine | 0.00263 | Krtcap2,Syvn1,Stt3a,Rpn1,Ube2j1,Vcp,St6gal1,Ube2g2 |
| LV | G4 | cellular response to topologically incorrect protein | 0.00267 | Tbl2,Bfar,Creb3,Atf3,Herpud1,Yod1,Derl1,Vimp,Serp1,Sdf2l1,Wfs1,Hspa5,Ptpn2,Ptpn1,Dnajc3,Ero1l |
| LV | G4 | defense response | 0.00317 | Selk,Selp,Pik3ap1,Slamf8,Cd28,Cebpg,Il1rn,Hck,B4galt1,Il1r1,Cxcl9,Hif1a,Eif4e,Lcn2,Creb3,Nod1,Clec4d,Hilpda,Cd55,Psma1,Adora1,Tnfaip6,Irf8,Rbm18,Slc11a1,Exosc4,Lbp,Apcs,Ap1g1,Fgr,Fgb,S100a8,A2m,Slc35c1,Ifitm2,Ifitm1,Fcer1g,Syk,Vimp,Adam17,Hyal1,Cd44,Ctla2a,Reg3b,Trim27,Il12rb1,Irak2,Iigp1,Itgam,Ncf1,Pld1,Rasgrp1,Tirap,Tlr1,Apoa4,Nlrp12,Cox8a,Ptpn2,Casp4,Il17ra,Il4ra,Il1a,Ccl17,Vav1,Eprs,Trem3,Socs3,Rps6ka3,Tnfrsf1b,Tnfrsf1a,Tmem173,Rab1,Sbno2,Snap23,Pik3c2a,Coro1a,Pycard,Clock,Ccr1,Plaa,Clec4n,Seh1l,Cr1l,Fabp4,Fcgr3,Polr3d,Nfkbiz,Prkce,Crnkl1,Irg1,Myo1f,S100a9 |

[Table 18-8]

| Organ | group | Term | Pvalue | Genelist |
|---|---|---|---|---|
| LV | G4 | protein N-linked glycosylation | 0.00792 | Krtcap2,B4galt1,Syvn1,Stt3a,Rpn1,Ube2j1,Vcp,St6gal1,Tmem165,Ddost,Alg12,Pgm3,Ube2g2 |
| LV | G4 | regulation of cellular response to stress | 0.00836 | Bfar,Cebpg,Arl6ip5,Map3k3,Rab12,Qsox1,Fktn,Ctgf,Pdcd10,Pnp,Hif1a,Marveld3,Syvn1,Braf,Scly,Creb3,Senp2,Nod1,Apex1,Serinc3,Usp1,Herpud1,Skil,Ppp4c,Dab2,Scfd1,Syk,Hmga2,Vimp,Cd44,Wfs1,Supt5,Hipk3,Grina,Rasgrp1,Tirap,Trip12,Ptpn2,Ptpn1,Thoc1,Il1a,Eya3,Ccl19,Dnajc3,Rint1,Ift20,Hyou1,Gadd45b,Gadd45g,Sod2,Pycard,Trp63,Chmp4b,Rab33b |
| LV | G4 | negative regulation of response to endoplasmic reticulum stress | 0.0089 | Bfar,Syvn1,Creb3,Herpud1,Vimp,Wfs1,Grina,Ptpn1,Dnajc3,Hyou1 |
| LV | G4 | cellular amino acid metabolic process | 0.01264 | Lars,Sdsl,Tat,Gpt2,Pcyox1,Aldh18a1,Gls,Atp7a,Mthfr,Cad,Iars,Rars,Hdc,Asns,Odc1,Tha1,Hars,Hnf4a,Cars2,Farsb,Vars,Kars,Sds,Ero1l,Ears2 |
| LV | G4 | Synthesis of substrates in N-glycan biosythesis | 0.01361 | Mvd,Nans,St6gal1,Gfpt1,Gne,St3gal3,Gmppa,Dhdds,St3gal5,St3gal4,Uap1,Pgm3,Gmppb |
| LV | G4 | regulation of endoplasmic reticulum stress-induced intrinsic apoptotic signaling pathway | 0.01401 | Syvn1,Creb3,Serinc3,Herpud1,Vimp,Wfs1,Grina,Ptpn2,Ptpn1,Hyou1 |
| LV | G4 | response to wounding | 0.0142 | Selp,Pik3ap1,Cd28,B4galt1,Il1r1,Pdcd10,Actg1,Hif1a,Braf,Cd55,Psma1,Adora1,Tnfaip6,Slc11a1,Lbp,Fgb,S100a8,A2m,Fcer1g,Lnp,Tnfrsf12a,Syk,Vimp,Cd44,Myh9,Ctla2a,F13a1,Plek,Gas6,Sdc4,Hnf4a,Jun,Pdia6,Nlrp12,Ifrd1,Ptpn2,Casp4,Il17ra,Il1a,Itgb3,Socs3,Tnfrsf1b,Tnfrsf1a,Sbno2,Sod2,Pik3c2a,Pycard,Fn1,Clock,Alox12,Cr1l,Fabp4,Fcgr3,Scara5,Prkce,Irg1,S100a9 |
| LV | G4 | macromolecule modification | 0.01561 | Selk,Bfar,Pdgfc,Mbd1,Prkag2,Garem,Mettl1,Mettl3,Senp6,Pim3,Arl6ip5,Metap2,Hck,Etf1,Btg2,Ntmt1,Anapc4,Mvp,Ppap2a,Map3k3,Krtcap2,B4galt1,Pole4,Bmper,Ficd,Plcl1,Fktn,Pus3,Errfi1,Ctgf,Nktr,Pdcd10,Mphosph8,Marveld3,Syvn1,Braf,Senp2,Efna1,Nod1,Ibtk,Apex1,Ube2s,Trim39,Csnk1a1,Cwc27,Rlim,Dok2,Atp7a,Usp8,Usp1,Tnfaip1,Eogt,Aak1,Trib1,Grb2,Atf3,Herpud1,Slc11a1,Mthfr,Mmp9,Exosc4,Cad,Stt3a,Lpin1,Uba5,Jmjd6,Lpar1,Yod1,Fgr,Arf4,Camkk2,Ube2f,Fgb,Rpn1,Tgm2,Uspl1,S100a8,Egf,Fkbp11,Nedd4l,C1galt1,Wdr61,Traf7,Acer2,Dpy19l1,Saa3,Usp14,Tbl1xr1,Ufm1,Cul5,Ube2j1,Vcp,Dab2,Dusp2,Asb4,Syk,St6gal1,Hmga2,Adam17,Cd44,Neurl3,Prmt1,Dzip3,Iqgap1,Dnajc10,F13a1,Sox9,Nus1,Wfs1,Supt6,Hipk3,Tmem165,Gas6,Trim27,Rac2,Ppp2r1a,Il12rb1,Cdc73,Tspyl2,Smarcad1,Rasgrp1,Nop58,Tirap,Fkbp5,Tlr1,Trip12,Ddost,Ick,Adam9,Hspa5,Atf7ip,Jun,Dnajb11,Nlrp12,Nup62,Wipi1,Crtc2,Ptpn2,Ptpn1,Abca1,Ufl1,Znrf1,Plaur,Nceh1,Parp3,Mppe1,Elp3,Inhbe,B3galt1,Dph5,Brd8,Il1a,Alg12,Stk17b,Pggt1b,Tiparp,Eya3,Slc9a3r1,Prkrip1,Psen1,Exosc3,Piga,Itgb3,Itgb2,Arid5a,Ccl19,Gan,Wdr45b,Trmt61a,St3gal3,Dnajc3,Trim68,Rcor1,Socs3,Dr1,Camk2b,Trim3,Rps6ka3,Tnfrsf1a,Nek7,Trib2,Cdkn1a,Gadd45b,Gadd45g,Ppp1r11,Pik3c2a,Vegfc,Pycard,Fn1,Clock,B3galnt2,St3gal5,St3gal4,Pomgnt1,Ppm1l,Ccr1,Fabp4,Psmd14,Pgm3,Rusc1,Nop56,Pofut2,Galnt2,Taf1,Trmt10a,Enc1,Josd2,Lats2,Prkch,Prkce,Anapc16,Il6st,Ikbkap,S100a9,Ube2g2 |
| LV | G4 | cellular response to starvation | 0.01936 | Mybbp1a,Tbl2,Atg13,Rab12,Qsox1,Dap,Atf3,Atg9b,Srebf2,Scfd1,Asns,Rraga,Supt5,Gas6,Trp53inp2,Hspa5,Ubxn2b,Wipi1,Psen1,Wdr45b,Atg16l2,Rab1,Ift20,Map1lc3b,Chmp4b,Rab33b |
| LV | G4 | intrinsic apoptotic signaling pathway in response to endoplasmic reticulum stress | 0.02034 | Selk,Syvn1,Creb3,Serinc3,Herpud1,Itpr1,Vimp,Dnajc10,Wfs1,Grina,Ptpn2,Ptpn1,Casp4,Hyou1,Ero1l |
| LV | G4 | RNA transport\|Processing of Capped Intron-Containing Pre-mRNA | 0.02073 | Mettl3,Hnrnpa2b1,Etf1,Wdr77,Trnt1,Pnn,Eif4e,Sf3b4,Eif3d,Srsf2,Senp2,Plrg1,Gm10094,Dhx38,Alyref,Gtf2f1,Nup98,Gspt2,Snrnp40,Ccar1,Eif4g2,Ranbp2,Eif4a3,Casc3,Gtf2f2,Nupl2,Ppp2r1a,Gemin8,Tra2b,Snrnp27,Nudt21,Papola,Nupl1,Nup62,Eif2b4,Thoc2,Thoc1,Rnps1,Magoh,Sec13,Hbs1l,Seh1l,Srsf1,Srsf3,Crnkl1 |
| LV | G4 | Membrane Trafficking | 0.02312 | Prkag2,Arcn1,Copa,Copb1,Sec24d,Ap1g1,Clint1,Copg1,Dab2,Ap4e1,Copb2,Myh9,Tbc1d8b,Ralgapa2,Preb,Sec31a,Tpd52,Cltb,Txndc5,C2cd5,Yipf6,Snap23,Sec13,Pik3c2a,Chmp4b,Arfgap1 |
| LV | G4 | cell death | 0.02705 | Selk,Mybbp1a,Cyp1b1,Bfar,Cd28,Pim3,Arl6ip5,Hck,Btg2,B4galt1,Hsp90b1,Ctgf,Pdcd10,Pnp,Hif1a,Dap,Lcn2,Krt18,Syvn1,Braf,Creb3,Nod1,Trim39,Dnajc5,Pcid2,Serinc3,Atp7a,Atf3,Herpud1,Slc11a2,Gzma,Mmp9,Skil,Arf4,Fgb,Tgm2,S100a8,Ccar1,Pkm,Trps1,Traf7,Pdia3,Acer2,Itpr1,Fcer1g,Cln8,Bag3,Vcp,Dab2,Tnfrsf12a,Hmga2,Vimp,Adam17,Srgn,Asns,Rraga,Cd44,Qars,Ier3ip1,Dnajc10,Sox9,Sdf2l1,Wfs1,Hipk3,Gas6,Ppp2r1a,Irak2,Tnfrsf22,Gata6,Grina,Mt1,Hspa5,Golph3,Jun,Nlrp12,Capn10,Nup62,Ptpn2,Ptpn1,Casp4,Plaur,Parp2,Zbtb16,Thoc1,Inhbe,Rnps1,Dcun1d3,Il1a,Stk17b,Son,Slc9a3r1,Psen1,Cdip1,Cd38,Ccl19,Dnajc3,Camk2b,Rps6ka3,Tnfrsf1b,Tnfrsf1a,Hyou1,Cdkn1a,Hk2,Gadd45b,Gadd45g,Sod2,Coro1a,Pycard,Trp63,Ivns1abp,Alox12,Chmp4b,Ero1l,Prkch,S100a9 |
| LV | G4 | Golgi organization | 0.03053 | Golga5,Surf4,Stx6,Stx18,Pdcd10,Blzf1,Tmed9,Vamp4,Zw10,Golph3l,Gm21540,Golph3,Ubxn2b,Rab1,Stx5a,Rab33b,Atl2 |
| LV | G4 | Golgi to ER Retrograde Transport | 0.03083 | Arcn1,Copa,Copb1,Copg1,Copb2,Arfgap1 |
| LV | G4 | RNA Polymerase II Transcription | 0.0314 | Gtf2e1,Dhx38,Alyref,Ell,Tbp,Gtf2f1,Gtf2f2,Nelfa,Supt5,Nudt21,Papola,Taf2,Rnps1,Cdk7,Gtf2a2,Taf4b,Magoh,Srsf1,Srsf3,Taf1 |
| LV | G4 | cellular response to external stimulus | 0.03307 | Mybbp1a,Tbl2,Atg13,Ldha,Rab12,Qsox1,Ppan,Dap,Atf3,Atg9b,Srebf2,Bag3,Scfd1,Asns,Rraga,Sox9,Supt5,Gas6,Trp53inp2,Hspa5,Ubxn2b,Wipi1,Psen1,Wdr45b,Atg16l2,Rab1,Ift20,Cdkn1a,Map1lc3b,Chmp4b,Rab33b |
| LV | G4 | intracellular transport | 0.03565 | Mybbp1a,Mfsd7b,Yipf5,Sec61b,Golga5,Snx6,Atg13,Mlx,Stx6,Cog6,Rab12,Srp68,Stx12,Stx18,Cxcl9,Arcn1,Uso1,Hif1a,Krt18,Timm21,Creb3,Rabl3,Ibtk,Gosr2,Blzf1,Slc11a1,Snip1,Ap1g1,Fgr,Tmed9,Egf,Ap2a2,Derl1,Atp2a2,Litaf,Sar1a,Itpr1,Ipo4,Vamp4,Rab35,Fcer1g,Zfyve16,Vcp,Dab2,Actr2,Syk,Vimp,Ap4e1,Copb2,Ddx39,Zw10,Rab5b,Creb3l2,Nus1,Chic2,Supt6,Tmem165,Gas6,Trim27,Rac2,Golga4,Dynll1,Gm21540,Dst,Ncf1,Rasgrp1,Ick,Pcm1,Kif5b,Golph3,Jun,Sec61a1,Nlrp12,Capn10,Wipi1,Rab18,Ptpn1,Ap1ar,Abca1,Mppe1,Il4ra,Thoc2,Thoc1,Bet1l,Ap3d1,Sec22b,Hook1,Psen1,Ap5s1,Ccl19,Rint1,Srp72,Tmem173,Rab1,Ift20,C2cd5,Cdkn1a,Hk2,Snap23,Sec13,Coro1a,Stx5a,Chmp4b,Nemf,Hsp90aa1,Ero1l,Rab33b,Calr,Tbrg1,Prkce |

[Table 18-9]

| Organ | group | Term | Pvalue | Genelist |
|---|---|---|---|---|
| LV | G4 | biosynthetic process | 0.03959 | Cers6,Lars,Selk,Mybbp1a,Nampt,Cyp1b1,Armcx3,Nol8,Pdgfc,Snx6,Mlx,Cd28,Cebpg,Cebpd,Mettl3,Hnrnpa2b1,Hnrnpab,Polr1e,Hck,Etf1,Ednra,Btg2,Wdr77,Agk,Adcy1,Ppap2a,Krtcap2,B4galt1,Mvd,Bmper,Tcf25,Ldha,Atp6v0a1,Fktn,Epm2aip1,Mphosph8,Klf17,Pnp,Hif1a,Smarce1,Eif4e,Dap,Agpat9,Eif3d,Syvn1,Prg4,Creb3,Senp2,Efna1,Ell2,Nod1,Ctif,Gmeb1,Cyp7b1,Npm3,Tasp1,Aldh18a1,Pcid2,Ddx21,Gls,Rlim,B4galnt1,Atp7a,Ubiad1,Tnfaip1,Eogt,Mrpl51,Ell,Tbp,Irf8,Trib1,Cebpz,Atf6,Atf3,Slc11a2,Slc11a1,Mthfr,Cad,Lbp,Snip1,Skil,Stt3a,Lpin1,Polh,Gtf2f1,Nup98,Foxk2,Arf4,Rpn1,Tmsb4x,Egf,Eif4g2,Stag1,C1galt1,Fen1,Wdr61,Trps1,Spin1,Iars,Traf7,Acer2,Dpy19l1,Rars,Hdc,Tbl1xr1,Srebf2,Suco,Far2,Ranbp2,Trappc2,Ube2j1,Vcp,Mcm2,Dab2,Sf1,Syk,St6gal1,Hmga2,Vimp,Asns,Hyal1,Serp1,Prmt1,Cyp51,Prdm1,Atf6b,Pcsk5,Creb3l2,Plek,Idi1,Nelfa,Rbm39,Sox9,Nus1,Odc1,Papss1,Wfs1,Supt5,Supt6,Hipk3,Tmem165,Helb,Gas6,Trim27,Crem,Paip2b,Irak2,Dynll1,Hist1h3b,Cdc73,Lig3,Gata6,Ncf1,Tspyl2,Srm,Pld1,Tha1,Tirap,Trp53inp2,Tlr1,Med17,Apoa4,Apoa5,Hars,Zfp869,Ddost,Gigyf2,Hnf4a,Zfp160,Atf7ip,Csgalnact2,Golph3,Jun,Nlrp12,Denr,Cars2,Farsb,Taf2,Ctps,Nup62,Vars,Wipi1,Ifrd1,Crtc2,Secisbp2,Ptpn2,Abca1,Ufl1,Preb,Parp3,Mppe1,Zbtb16,Zbtb21,Med25,Eif2b4,Thoc1,Elp3,B3galt6,B3galt1,Tfb2m,Cdk7,Dph5,Zfp830,Il1a,Alg12,Rps9,Kars,Tiparp,Psen1,Hltf,Cd38,Piga,Itgb3,Arid5a,Ccl19,Gtf2a2,Nfia,Wdr45b,Atg16l2,Eprs,D19Bwg1357e,Spg20,St3gal3,Dnajc3,Rcor1,Polr1a,Rps6ka3,Tnfrsf1a,Cpeb2,Trib2,Tmem173,Cdkn1a,Taf4b,Sds,Sbno2,Sod2,Pik3c2a,Etnk2,Pycard,Adk,Trp63,Clock,Alox12,B3galnt2,St3gal4,Pomgnt1,Fabp4,Fabp5,Hsp90aa1,Lss,Fcgr3,Zfp27,Calr,Pgm3,Pofut2,Polr3k,Galnt2,Tbrg1,Taf1,Pspc1,Nfkbiz,Enc1,Srrt,Prkch,Ears2,Irg1,Larp7,Ikbkap,Nif3l1,S100a9,Ube2g2 |
| LV | G4 | negative regulation of endoplasmic reticulum stress-induced intrinsic apoptotic signaling pathway | 0.03966 | Syvn1,Creb3,Herpud1,Vimp,Wfs1,Grina,Ptpn1,Hyou1 |
| LV | G4 | Biosynthesis of the N-glycan precursor (dolichol lipid-linked oligosaccharide, LLO) and transfer to a nascent protein | 0.04085 | Mvd,Nans,St6gal1,Gfpt1,Alg12,Gne,St3gal3,Gmppa,Dhdds,St3gal5,St3gal4,Uap1,Pgm3,Gmppb |
| LV | G4 | Transcription | 0.04772 | Polr1e,Gtf2e1,Zfp143,Dhx38,Alyref,Ell,Tbp,Gtf2f1,Gtf2f2,Nelfa,Supt5,Hist1h3b,Ssb,Nudt21,Papola,Taf2,Rnps1,Tfb2m,Cdk7,Gtf2a2,Nfia,Polr1a,Taf4b,Magoh,Srsf1,Srsf3,Polr3d,Polr3k,Taf1 |
| LV | G4 | Aminoacyl-tRNA biosynthesis | 0.05146 | Lars,Scly,Nars,Iars,Rars,Qars,Papss1,Hars,Cars2,Farsb,Vars,Kars,Eprs,Ears2 |
| LV | G4 | single-organism intracellular transport | 0.05511 | Mfsd7b,Yipf5,Sec61b,Golga5,Snx6,Atg13,Stx6,Cog6,Rab12,Srp68,Stx18,Cxcl9,Arcn1,Uso1,Hif1a,Krt18,Timm21,Creb3,Ibtk,Gosr2,Blzf1,Slc11a1,Snip1,Ap1g1,Fgr,Tmed9,Egf,Derl1,Atp2a2,Litaf,Sar1a,Itpr1,Ipo4,Vamp4,Rab35,Fcer1g,Zfyve16,Vcp,Dab2,Syk,Vimp,Copb2,Ddx39,Zw10,Rab5b,Creb3l2,Nus1,Chic2,Supt6,Tmem165,Gas6,Trim27,Rac2,Golga4,Dynll1,Gm21540,Dst,Ncf1,Rasgrp1,Ick,Pcm1,Kif5b,Golph3,Jun,Sec61a1,Nlrp12,Wipi1,Ap1ar,Abca1,Mppe1,Il4ra,Thoc2,Thoc1,Bet1l,Ap3d1,Sec22b,Hook1,Psen1,Ap5s1,Ccl19,Rint1,Srp72,Tmem173,Rab1,Ift20,C2cd5,Cdkn1a,Hk2,Snap23,Sec13,Coro1a,Stx5a,Chmp4b,Hsp90aa1,Ero1l,Rab33b,Calr,Tbrg1,Prkce |
| LV | G4 | intrinsic apoptotic signaling pathway | 0.06020 | Selk,Mybbp1a,Cyp1b1,Arl6ip5,Pdcd10,Pnp,Hif1a,Syvn1,Creb3,Serinc3,Herpud1,Mmp9,Skil,S100a8,Itpr1,Vimp,Cd44,Dnajc10,Wfs1,Grina,Ptpn2,Ptpn1,Casp4,Plaur,Slc9a3r1,Cdip1,Tnfrsf1b,Tnfrsf1a,Hyou1,Cdkn1a,Sod2,Pycard,Trp63,Ivns1abp,Ero1l,S100a9 |
| LV | G4 | Processing of Capped Intron-Containing Pre-mRNA | 0.06182 | Mettl3,Hnrnpa2b1,Eif4e,Sf3b4,Plrg1,Dhx38,Alyref,Gtf2f1,Nup98,Snrnp40,Ccar1,Ranbp2,Gtf2f2,Nupl2,Nudt21,Papola,Nupl1,Nup62,Rnps1,Magoh,Seh1l,Srsf1,Srsf3 |
| LV | G4 | SNARE interactions in vesicular transport | 0.07286 | Stx6,Stx18,Gosr2,Vamp4,Bet1l,Sec22b,Snap23,Stx5a |
| LV | G4 | phagocytosis | 0.073 | Hck,Csnk1a1,Marco,Irf8,Slc11a1,Lbp,Jmjd6,Fgr,Tgm2,Fcer1g,Scfd1,Syk,Myh9,Gas6,Abca1,Vav1,Txndc5,Coro1a,Pycard,Fcgr3,Calr |
| LV | G4 | cellular response to extracellular stimulus | 0.07784 | Mybbp1a,Tbl2,Atg13,Ldha,Rab12,Qsox1,Ppan,Dap,Atf3,Atg9b,Srebf2,Scfd1,Asns,Rraga,Supt5,Gas6,Trp53inp2,Hspa5,Ubxn2b,Wipi1,Psen1,Wdr45b,Atg16l2,Rab1,Ift20,Cdkn1a,Map1lc3b,Chmp4b,Rab33b |
| LV | G4 | Platelet activation, signaling and aggregation | 0.08181 | Selp,Timp1,Grb2,Wdr1,Fgb,Tmsb4x,A2m,Egf,Itpr1,Fcer1g,Syk,Srgn,F13a1,Plek,Csf2rb,Gas6,Rac2,Rasgrp1,Hspa5,Ptpn1,Itgb3,Vav1,Vegfc,Fn1,Gnai2,Lcp2,Prkch,Prkce |
| LV | G4 | ER to Golgi vesicle-mediated transport | 0.08751 | Yipf5,Stx18,Uso1,Gosr2,Sar1a,Rab35,Zw10,Creb3l2,Mppe1,Sec22b,Rint1,Rab1,Sec13,Stx5a |
| LN | G1 | regulation of immune system process | 0.93881 | Pik3ap1,Trim30a,Pik3ap1,Mfsd7b,Cd28,Cebpg,Cebpd,Hck,B4galt1,Cxcl9,Ung,Pnp,Hif1a,Lrrc8a,Lcn2,Prg4,Braf,Creb3,Nod1,Clec4d,Hilpda,Pcid2,Cd55,Psma1,Atp7a,Adora1,Tnfaip1,Irf8,Trib1,Rbm18,Slc11a2,Slc11a1,Exosc4,Lbp,Skil,Apcs,Jmjd6,Ap1g1,Fgr,Fgb,S100a8,A2m,Slc35c1,Wdr61,Ifitm2,Ifitm1,Ap2a2,Gpatch4,Rab35,Fcer1g,Dab2,Syk,Vimp,Adam17,Cd44,Myh9,Ctla2a,Prmt1,Prdm1,Rab5b,H2-D1,Sox9,Cklf,Supt6,Gas6,Trim27,Rac2,Il12rb1,Irak2,Cdc73,Itgal,Itgam,Ncf1,Rasgrp1,Tirap,Tlr1,Apoa4,Ddost,Adam9,Golph3,Jun,Nlrp12,Cox8a,Ctps,Ptpn2,Zbtb16,Il4ra,Thoc1,Ap3d1,Tpd52,Tiparp,Psen1,Exosc3,Cd38,Itgb3,Itgb2,Vav1,Ccl19,Eprs,Snx10,Rcor1,Trem3,Rps6ka3,Tmem173,Cdkn1a,Gadd45g,Sbno2,Sod2,Snap23,Msn,Pik3c2a,Coro1a,Vegfc,Pycard,Ccr1,Txlna,Clec4n,Cd48,Cr1l,Hsp90aa1,Fcgr3,Calr,Pgm3,Polr3d,Lcp2,Galnt2,Clptm1,Prkch,Prkce,Crnkl1,Irg1,Myo1f,Il6st,S100a9 |
| LV | G4 | macromolecule catabolic process | 0.09361 | Bfar,Sec61b,Timp1,Btg2,Rab12,Hsp90b1,Syvn1,Timp3,Fbxo6,Ctif,Ube2s,Trim39,Pcyox1,Rlim,Psma1,Usp8,Usp1,Tnfaip1,Trib1,Herpud1,Gzma,Exosc4,Yod1,Egf,Nedd4l,Fen1,Wdr61,Derl1,Usp14,Tbl1xr1,Cul5,Cln8,Ube2j1,Vcp,Dab2,Vimp,Hyal1,Cd44,Ctla2a,Ctla2b,Dnajc10,Sox9,Sdf2l1,Odc1,Wfs1,Lyve1,Parn,Exog,Dis3,Trip12,Adam9,Hspa5,Ubxn2b,Secisbp2,Ptpn1,Ufl1,Znrf1,Rnps1,Exosc10,Fbxo31,Tiparp,Sec22b,Psen1,Exosc3,Zfand2a,Khsrp,Kctd10,Dnajc3,Ubxn4,Tnfrsf1b,Edem3,Trib2,Edem1,Nbas,Magoh,Stx5a,Clock,Chmp4b,Psmd14,Taf1,Enc1,Ube2g2 |
| LV | G4 | organic substance transport | 0.10013 | Selk,Mfsd7b,Sec61b,Snx6,Atg13,Ezr,Hnrnpa2b1,Stx6,Il1rn,Pim3,Arl6ip5,Ednra,Rab12,Srp68,Stx12,Uso1,Hif1a,Krt18,Braf,Timm21,Creb3,Rabl3,Slco3a1,Serinc3,Gosr2,Blzf1,Slc11a1,Lbp,Snip1,Atp11a,Ap1g1,Fgr,Fgb,S100a8,Egf,Ap2a2,Derl1,Litaf,Ipo4,Vamp4,Rab35,Fcer1g,Zfyve16,Srebf2,Slc4a4,Cln8,Slc16a6,Vcp,Dab2,Scfd1,Syk,Hmga2,Vimp,Srgn,Ap4e1,Ddx39,Myh9,Serp1,Golph3l,Rab5b,Plek,Slc2a3,Nus1,Supt6,Slc15a2,Gas6,Trim27,Rac2,Mfsd2a,Golga4,Msr1,Gm21540,Ncf1,Slc10a6,Rasgrp1,Slc10a2,Apoa4,Apoa5,Pcm1,Hnf4a,Adam9,Hspa5,Golph3,Jun,Sec61a1,Nlrp12,Crp,Emb,Capn10,Sidt2,Rab18,Ptpn1,Abca1,Casp4,Slc38a10,Scamp2,Il4ra,Thoc2,Thoc1,Il1a,Ap3d1,Slc38a2,Slc9a3r1,Psen1,Itgb3,Ccl19,Slc7a6,Slc35a2,Trem3,Srp72,Tmem173,Rab1,Ift20,C2cd5,Cdkn1a,Hk2,Snap23,Sybu,Pycard,Stx5a,Clock,Chmp4b,Slc35e1,Clec4n,Slc13a3,Slc13a5,Fabp5,Hsp90aa1,Fcgr3,Rab33b,Calr,Lcp2,Prkce,S100a9 |

[Table 18-10]

| Organ | group | Term | Pvalue | Genelist |
|---|---|---|---|---|
| LV | G4 | N-glycan trimming in the ER and Calnexin/Calreticulin cycle | 0.1026 | Mogs,Pdia3,Edem2,Edem3,Edem1,Calr |
| LV | G4 | ncRNA processing | 0.14837 | Nol8,Utp6,Ddx52,Mettl1,Ints7,Ints5,Rpf2,Pus3,Trnt1,Mphosph6,Npm3,Ints2,Exosc4,Ebna1bp2,Wdr12,Ngdn,Dis3,Nop58,Ddx1,Elp3,Exosc10,Exosc3,Trmt61a,Nol9,Nop56,Trmt10a,Srrt |
| LV | G4 | Transport of Mature mRNA derived from an Intron-Containing Transcript | 0.15115 | Eif4e,Dhx38,Alyref,Nup98,Ranbp2,Nupl2,Nupl1,Nup62,Rnps1,Magoh,Seh1l,Srsf1,Srsf3 |
| LV | G4 | catabolic process | 0.151821 | Sdsl,Cyp1b1,Bfar,Sec61b,Prkag2,Atg13,Tat,Timp1,Btg2,Rab12,Qsox1,Hsp90b1,Pnp,Hif1a,Dap,Syvn1,Timp3,Fbxo6,Ctif,Ube2s,Trim39,Pcyox1,Mmp13,Pfkp,Gls,Rlim,Psma1,Usp8,Entpd7,Usp1,Tnfaip1,Trib1,Herpud1,Gzma,Mmp9,Exosc4,Lpin1,Dram1,Yod1,S100a8,Egf,Slc35c1,Nedd4l,Fen1,Wdr61,Pkm,Derl1,Atg9b,Hdc,Usp14,Tbl1xr1,Pfkfb2,Cul5,Cln8,Ube2j1,Vcp,Dab2,Scfd1,Vimp,Rraga,Hyal1,Cd44,Ctla2a,Ctla2b,Dnajc10,Sox9,Sdf2l1,Odc1,Wfs1,Supt5,Iigp1,Lyve1,Parn,Exog,Ncf1,Dis3,Tha1,Trp53inp2,Apoa4,Apoa5,Trip12,Adam9,Hspa5,Ubxn2b,Cox8a,Wipi1,Secisbp2,Ptpn1,Ufl1,Znrf1,Rnps1,Exosc10,Fbxo31,Cda,Tiparp,Sec22b,Psen1,Exosc3,Zfand2a,Khsrp,Wdr45b,Atg16l2,Kctd10,Dnajc3,Ubxn4,Tnfrsf1b,Edem3,Trib2,Edem1,Rab1,Ift20,Nbas,Hk2,Sds,Gale,Magoh,Pycard,Map1lc3b,Stx5a,Clock,Chmp4b,Txlna,Rab33b,Psmd14,Taf1,Enc1,Prkce,Crnkl1,S100a9,Ube2g2 |
| LV | G4 | regulation of response to external stimulus | 0.15254 | Selp,Pik3ap1,Cd28,Il1r1,Rab12,Qsox1,Cxcl9,Braf,Creb3,Hilpda,Cd55,Psma1,Adora1,Tnfaip6,Trib1,Rbm18,Lbp,S100a8,A2m,Slc35c1,Usp14,Fcer1g,Scfd1,Syk,Vimp,Adam17,Ctla2a,Prdm1,Plek,Supt5,Gas6,Rac2,Il12rb1,Tirap,Nlrp12,Cox8a,Ptpn2,Casp4,Il17ra,Ccl19,Socs3,Tnfrsf1b,Tnfrsf1a,Tmem173,Ift20,Sbno2,Vegfc,Pycard,Clock,Bbs4,Alox12,Chmp4b,Ccr1,Cr1l,Fabp4,Fcgr3,Rab33b,Scara5,Calr,Crnkl1,Irg1,S100a9 |
| LV | G4 | cellular macromolecule catabolic process | 0.16017 | Bfar,Sec61b,Btg2,Rab12,Hsp90b1,Syvn1,Fbxo6,Ctif,Ube2s,Trim39,Pcyox1,Rlim,Psma1,Usp8,Usp1,Tnfaip1,Trib1,Herpud1,Gzma,Exosc4,Yod1,Egf,Nedd4l,Fen1,Wdr61,Derl1,Usp14,Tbl1xr1,Cul5,Cln8,Ube2j1,Vcp,Dab2,Vimp,Ctla2a,Ctla2b,Dnajc10,Sdf2l1,Wfs1,Parn,Exog,Dis3,Trip12,Hspa5,Ubxn2b,Secisbp2,Ufl1,Znrf1,Rnps1,Exosc10,Fbxo31,Psen1,Exosc3,Zfand2a,Khsrp,Kctd10,Dnajc3,Ubxn4,Edem3,Trib2,Edem1,Nbas,Magoh,Clock,Chmp4b,Psmd14,Taf1,Enc1,Ube2g2 |
| LV | G4 | SLC-mediated transmembrane transport | 0.16306 | Slc41a1,Slc30a5,Slc39a6,Slco3a1,Slc20a1,Slc16a10,Slc11a2,Slc11a1,Pdzd11,Nup98,Slc35c1,Slc39a7,Slc4a4,Ranbp2,Slc39a10,Slc39a14,Slc30a7,Nupl2,Slc2a3,Slc15a2,Slc10a6,Nupl1,Nup62,Cp,Slc38a2,Slc7a6,Slc35a2,Slc41a2,Hk2,Seh1l,Slc13a3,Slc13a5 |
| LV | G4 | autophagy | 0.16436 | Atg13,Rab12,Qsox1,Hif1a,Dap,Pfkp,Dram1,S100a8,Slc35c1,Atg9b,Scfd1,Rraga,Supt5,Iigp1,Trp53inp2,Ubxn2b,Cox8a,Wipi1,Psen1,Wdr45b,Atg16l2,Rab1,Ift20,Pycard,Map1lc3b,Chmp4b,Txlna,Rab33b,Crnkl1,S100a9 |
| LV | G4 | neutrophil chemotaxis | 0.16581 | Lbp,S100a8,Fcer1g,Syk,Cklf,Rac2,Itgam,Tirap,Itgb2,Vav1,Ccl19,Trem3,Fcgr3,S100a9 |
| LV | G4 | carbohydrate derivative biosynthetic process | 0.16801 | Ednra,Adcy1,Krtcap2,B4galt1,Atp6v0a1,Fktn,Pnp,Syvn1,B4galnt1,Atp7a,Eogt,Stt3a,Arf4,Rpn1,Egf,C1galt1,Acer2,Dpy19l1,Ube2j1,Vcp,St6gal1,Hyal1,Nus1,Papss1,Wfs1,Tmem165,Ddost,Csgalnact2,Golph3,Ctps,Abca1,Parp3,Mppe1,B3galt6,B3galt1,Alg12,Tiparp,Psen1,Piga,Ccl19,St3gal3,Adk,B3galnt2,St3gal4,Pomgnt1,Pgm3,Pofut2,Galnt2,Ube2g2 |
| LV | G4 | leukocyte migration | 0.18595 | Selk,Selp,B4galt1,Cxcl9,Creb3,Adora1,Lbp,S100a8,Fcer1g,Syk,Adam17,Cklf,Gas6,Rac2,Itgam,Tirap,Golph3,Nlrp12,Itgb2,Vav1,Ccl19,Trem3,Msn,Coro1a,Vegfc,Pycard,Ccr1,Fcgr3,Calr,S100a9 |
| LV | G4 | regulation of inflammatory response | 0.19888 | Pik3ap1,Cd28,Il1r1,Cd55,Psma1,Adora1,Tnfaip6,Lbp,S100a8,A2m,Fcer1g,Vimp,Ctla2a,Nlrp12,Ptpn2,Casp4,Il17ra,Socs3,Tnfrsf1b,Tnfrsf1a,Sbno2,Pycard,Clock,Cr1l,Fabp4,Fcgr3,Irg1,S100a9 |
| LV | G4 | autophagosome assembly | 0.20309 | Atg13,Atg9b,Scfd1,Trp53inp2,Ubxn2b,Wipi1,Psen1,Wdr45b,Atg16l2,Rab1,Ift20,Map1lc3b,Chmp4b,Rab33b |
| LV | G4 | translation | 0.20313 | Lars,Mettl3,Etf1,Btg2,Eif4e,Eif3d,Ctif,Mrpl51,Eif4g2,Iars,Rars,Serp1,Paip2b,Hars,Gigyf2,Denr,Cars2,Farsb,Vars,Secisbp2,Eif2b4,Rps9,Kars,Eprs,D19Bwg1357e,Dnajc3,Rps6ka3,Cpeb2,Enc1,Ears2,S100a9 |
| LV | G4 | Platelet degranulation | 0.25253 | Selp,Timp1,Wdr1,Fgb,Tmsb4x,A2m,Egf,Srgn,F13a1,Plek,Gas6,Hspa5,Itgb3,Vegfc,Fn1 |
| LV | G4 | single-organism metabolic process | 0.25861 | Cers6,Lars,Sdsl,Rad1,Selk,Mybbp1a,Nampt,Cyp1b1,Prkag2,Atg13,Garem,Tat,Cd28,Cebpg,Ezr,Il1rn,Arl6ip5,Cog6,Ednra,Agk,Adcy1,Ppap2a,Map3k3,Krtcap2,B4galt1,Pole4,Mvd,Bmper,Ldha,Rab12,Qsox1,Atp6v0a1,Fktn,Cxcl9,Ung,Ctgf,Epm2aip1,Pdcd10,Gpt2,Pnp,Hif1a,Marveld3,Agpat9,Syvn1,Braf,Scly,Senp2,Efna1,Nod1,Mcfd2,Cyp7b1,Lrrc16a,Apex1,Pcyox1,Aldh18a1,Mmp13,Pfkp,Gls,B4galnt1,Cd55,Dok2,Atp7a,Ubiad1,Entpd7,Usp1,Eogt,Acpp,Golt1b,Trib1,Grb2,Atf3,Slc11a2,Slc11a1,Mthfr,Mmp9,Cad,Lbp,Snip1,Stt3a,Lpin1,Polh,Jmjd6,Lpar1,Fgr,Arf4,Fgb,Rpn1,Ppp4c,A2m,Egf,Slc35c1,Fgl1,C1galt1,Fen1,Wdr61,Pkm,Atg9b,Iars,Traf7,Acer2,Dpy19l1,Rars,Hdc,Tbl1xr1,Pfkfb2,Suco,Ranbp2,Cln8,H2afx,Ube2j1,Vcp,Dab2,Sf1,Scfd1,Syk,St6gal1,Hmga2,Ppip5k1,Asns,Hyal1,Cd44,Serp1,Prmt1,Cyp51,Plek,Steap4,Idi1,Sox9,Nus1,Sdf2l1,Odc1,Papss1,Wfs1,Supt5,Supt6,Hipk3,Tmem165,Txnl1,Gas6,Rac2,Crem,Cdc73,Lig3,Gata6,Ncf1,Smarcad1,Pld1,Tha1,Rasgrp1,Tirap,Trp53inp2,Apoa4,Apoa5,Trip12,Hars,Zfp869,Ddost,Hnf4a,Adam9,Csgalnact2,Nlrp12,Flot1,Ubxn2b,Cars2,Cox8a,Farsb,Ctps,Nup62,Vars,Wipi1,Ppapdc1b,Crtc2,Ptpn2,Ptpn1,Abca1,Parp2,Parp3,Mppe1,Ppip5k2,Ddx1,Thoc1,Elp3,Inhbe,B3galt6,B3galt1,Tfb2m,Dph5,Zfp830,Brd8,Il1a,Alg12,Nabp1,Kars,Cda,Tiparp,Eya3,Slc9a3r1,Psen1,Ap5s1,Exosc3,Piga,Arid5a,Ccl19,Wdr45b,Atg16l2,Gne,St3gal3,Dnajc3,Rcor1,Dr1,Tnfrsf1a,Trib2,Rab1,Ift20,Hk2,Gadd45b,Sds,Gadd45g,Gale,Sod2,Pik3c2a,Etnk2,Pycard,Map1lc3b,Adk,Clock,Bbs4,Alox12,B3galnt2,St3gal4,Pomgnt1,Chmp4b,Ppm1l,Ccr1,Txlna,Cr1l,Fabp5,Ero1l,Lss,Rab33b,Psmd14,Pgm3,Pofut2,Galnt2,Taf1,Srrt,Ears2,Prkce,Acot11,Crnkl1,Il6st,Ube2g2 |
| LV | G4 | positive regulation of DNA-templated transcription, elongation | 0.2655 | Ell2,Ell,Wdr61,Dab2,Supt5,Supt6,Cdc73,Thoc1 |
| LV | G4 | tRNA aminoacylation for protein translation | 0.27988 | Lars,Iars,Rars,Hars,Cars2,Farsb,Vars,Kars,Ears2 |

[Table 18-11]

| Organ | group | Term | Pvalue | Genelist |
|---|---|---|---|---|
| LV | G4 | cellular nitrogen compound metabolic process | 0.28514 | Cers6,Lars,Rad1,Mybbp1a,Nampt,Cyp1b1,Armcx3,Nol8,Pdgfc,Mbd1,Prkag2,Snx6,Utp6,Dhx30,Mlx,Cd28,Cebpg,Cebpd,Ddx52,Mettl1,Mettl3,Hnrnpa2b1,Hnrnpab,Polr1e,Hck,Ednra,Btg2,Dhx40,Wdr77,Agk,Adcy1,Ppap2a,Bmper,Tcf25,Ints7,Ints5,Rpf2,Atp6v0a1,Pus3,Cxcl9,Ung,Trnt1,Mphosph8,Klf17,Pnp,Hif1a,Mphosph6,Smarce1,Dap,Sf3b4,Srsf2,Creb3,Senp2,Efna1,Ell2,Nod1,Ctif,Gmeb1,Lrrc16a,Apex1,Npm3,Tasp1,Pcid2,Ddx21,Pfkp,Rlim,Dhx38,B4galnt1,Atp7a,Entpd7,Usp1,Tnfaip1,Recql,Acpp,Mbnl2,Ell,Tbp,Irf8,Trib1,Dhx35,Cebpz,Atf6,Ints2,Atf3,Slc11a2,Gzma,Slc11a1,Mthfr,Exosc1,Exosc4,Cad,Snip1,Skil,Lpin1,Ddx50,Polh,Gtf2f1,Nup98,Jmjd6,Foxk2,Arf4,Tmsb4x,Ppp4c,Egf,Stag1,Fen1,Wdr61,Ebna1bp2,Pkm,Trps1,Spin1,Iars,Traf7,Acer2,Rars,Hdc,Tbl1xr1,Pfkfb2,Srebf2,Cln8,Trappc2,H2afx,Vcp,Scnm1,Mcm2,Dab2,Syk,Hmga2,Asns,Eif4a3,Ddx39,Prmt1,Prdm1,Atf6b,Pcsk5,Creb3l2,Wdr12,Nelfa,Rbm39,Sox9,Odc1,Papss1,Wfs1,Supt5,Supt6,Hipk3,Helb,Gas6,Trim27,Crem,Irak2,Dynll1,Hist1h3b,Gemin8,Ngdn,Cdc73,Parn,Lig3,Gata6,Exog,Ncf1,Tspyl2,Srm,Smarcad1,Dis3,Nop58,Tirap,Trp53inp2,Med17,Apoa4,Apoa5,Trip12,Hars,Zfp869,Hnf4a,Zfp160,Tra2b,Hspa5,Atf7ip,Jun,Dnajb11,Nlrp12,Nudt21,Papola,Cars2,Farsb,Taf2,Ctps,Nup62,Vars,Ifrd1,Crtc2,Secisbp2,Abca1,Ufl1,Ddx49,Preb,Parp2,Parp3,Zbtb16,Zbtb21,Med25,Ddx1,Thoc1,Elp3,Rnps1,Tfb2m,Cdk7,Exosc10,Zfp830,Il1a,Nabp1,Kars,Cda,Son,Eya3,Psen1,Ap5s1,Exosc3,Hltf,Cd38,Arid5a,Khsrp,Gtf2a2,Nfia,Atg16l2,Trmt61a,Spg20,Rcor1,Polr1a,Rps6ka3,Tnfrsf1a,Trib2,Tmem173,Cdkn1a,Taf4b,Nbas,Hk2,Magoh,Sbno2,Sod2,Nol9,Pycard,Adk,Trp63,Clock,B3galnt2,Fabp4,Fabp5,Hsp90aa1,Srsf1,Srsf3,Psmd14,Zfp27,Calr,Pgm3,Nop56,Polr3k,Tbrg1,Taf1,Pspc1,Nfkbiz,Trmt10a,Srrt,Prkch,Ears2,Crnkl1,Irg1,Rbm42,Larp7,Ikbkap,Nif3l1 |
| LV | G4 | macroautophagy | 0.29538 | Atg13,Rab12,Qsox1,Atg9b,Scfd1,Supt5,Trp53inp2,Ubxn2b,Wipi1,Psen1,Wdr45b,Atg16l2,Rab1,Ift20,Map1lc3b,Chmp4b,Rab33b |
| LV | G4 | Translation | 0.29607 | Sec61b,Etf1,Srp68,Eif4e,Eif3d,Ssr1,Ssr2,Spcs2,Spcs3,Ssr4,Gspt2,Rpn1,Srp9,Ddost,Sec61a1,Srp19,Srprb,Eif2b4,Rps9,Srp72,Srpr,Srp54a |
| LV | G4 | response to bacterium | 0.32759 | Slamf8,Hck,Cxcl9,Nod1,Clec4d,Irf8,Trib1,Slc11a1,Lbp,Cd14,Fgr,Fgb,Litaf,Fcer1g,Syk,Vimp,Adam17,Prdm1,Reg3b,Irak2,Iigp1,Cdc73,Ncf1,Pld1,Tirap,Tlr1,Adam9,Jun,Abca1,Trem3,Rps6ka3,Tnfrsf1b,Tnfrsf1a,Rab1,Pycard,Seh1l,Nfkbib,Prkce,Irg1,Myo1f |
| LV | G4 | myeloid cell activation involved in immune response | 0.3279 | Lbp,Fgr,Fcer1g,Syk,Rac2,Rasgrp1,Il4ra,Sbno2,Snap23,Pycard,Prkce,Myo1f |
| LV | G4 | leukocyte chemotaxis | 0.33499 | Cxcl9,Creb3,Lbp,S100a8,Fcer1g,Syk,Adam17,Cklf,Gas6,Rac2,Itgam,Tirap,Itgb2,Vav1,Ccl19,Trem3,Coro1a,Vegfc,Ccr1,Fcgr3,Calr,S100a9 |
| LV | G4 | cellular catabolic process | 0.33919 | Sdsl,Cyp1b1,Bfar,Sec61b,Prkag2,Atg13,Tat,Timp1,Btg2,Rab12,Qsox1,Hsp90b1,Pnp,Hif1a,Dap,Syvn1,Timp3,Fbxo6,Ctif,Ube2s,Trim39,Pcyox1,Pfkp,Gls,Rlim,Psma1,Usp8,Entpd7,Usp1,Tnfaip1,Trib1,Herpud1,Gzma,Exosc4,Lpin1,Dram1,Yod1,S100a8,Egf,Slc35c1,Nedd4l,Fen1,Wdr61,Derl1,Atg9b,Hdc,Usp14,Tbl1xr1,Cul5,Cln8,Ube2j1,Vcp,Dab2,Scfd1,Vimp,Rraga,Ctla2a,Ctla2b,Dnajc10,Sdf2l1,Wfs1,Supt5,Iigp1,Parn,Exog,Ncf1,Dis3,Tha1,Trp53inp2,Apoa4,Apoa5,Trip12,Adam9,Hspa5,Ubxn2b,Cox8a,Wipi1,Secisbp2,Ptpn1,Ufl1,Znrf1,Rnps1,Exosc10,Fbxo31,Cda,Psen1,Exosc3,Zfand2a,Khsrp,Wdr45b,Atg16l2,Kctd10,Dnajc3,Ubxn4,Tnfrsf1b,Edem3,Trib2,Edem1,Rab1,Ift20,Nbas,Sds,Magoh,Pycard,Map1lc3b,Clock,Chmp4b,Txlna,Rab33b,Psmd14,Taf1,Enc1,Crnkl1,S100a9,Ube2g2 |
| LV | G4 | mRNA processing | 0.38074 | Mettl3,Hnrnpa2b1,Btg2,Sf3b4,Srsf2,Dhx38,Mbnl2,Jmjd6,Eif4a3,Ddx39,Supt5,Supt6,Gemin8,Cdc73,Tra2b,Nudt21,Papola,Rnps1,Son,Magoh,Srsf1,Crnkl1,Rbm42 |
| LV | G4 | cell adhesion | 0.38488 | Selk,Cyp1b1,Selp,Cd28,Il1rn,B4galt1,Ctgf,Actg1,Pnp,Pnn,Braf,Efna1,Lrrc16a,Icam2,Clec4d,Atp7a,Cytip,Slc11a1,Jmjd6,Hsd17b12,Fgb,Tgm2,S100a8,Acer2,Fcer1g,Pvr,Fndc3b,Dab2,Tnfrsf12a,Syk,Adam17,Hyal1,Col13a1,Hapln4,Cd44,Myh9,Ctla2a,Plek,Sox9,Gas6,Rac2,Il12rb1,Itgal,Itgam,Rasgrp1,Apoa4,Ddost,Adam9,Golph3,Pdia6,Flot1,Ctps,Ptpn2,Ap1ar,Zbtb16,Il4ra,Ap3d1,Psen1,Itgb3,Itgb2,Vav1,Ccl19,S100a10,Cldn14,Rab1,Gadd45g,Coro1a,Vegfc,Pycard,Fn1,Alox12,Cd48,Hsp90aa1,Calr,Clptm1,Prkce,Myo1f,Il6st,S100a9 |
| LV | G4 | macromolecular complex assembly | 0.38547 | Selp,Pdgfc,Senp6,Polr1e,Hck,Rpf2,Atp6v0a1,Eif3d,Timm21,2610034B18Rik,Lrrc16a,Ube2s,Gls,Capza1,Mpp7,Irf8,Grb2,Tsr1,Skil,Nup98,Vasp,Fgb,Pde4dip,Cct3,Foxred1,Arpc1b,Sar1a,Ipo4,Vamp4,Mapre1,Vcp,Mcm2,Dab2,Actr2,Hmga2,Zw10,Plek,Sox9,Trim27,Gemin8,Rasgrp1,Apoa4,Apoa5,Hist1h2bp,Atf7ip,Jun,Nudt21,Denr,Ap1ar,Abca1,Med25,Brix1,Cda,Pdcl,Gtf2a2,S100a10,Arfip2,Trim6,Rab1,Sod2,Snap23,Pik3c2a,Coro1a,Pycard,Trp63,Fn1,Bbs4,Aspm,Chmp4b,Hsp90aa1,Srsf1,Scara5,Calr,Atl2,Atl1,Prkce,Eif6,Crnkl1 |
| LV | G4 | cell activation | 0.39267 | Selk,Selp,Cd28,Cebpg,Bmper,Actg1,Pnp,Lrrc8a,Braf,Clec4d,Pcid2,Atp7a,Slc11a1,Lbp,Jmjd6,Ap1g1,Fgr,Fgb,Fcer1g,Syk,Adam17,Myh9,Ctla2a,Prdm1,Plek,Supt6,Gas6,Rac2,Il12rb1,Itgal,Itgam,Rasgrp1,Tirap,Tlr1,Ddost,Adam9,Jun,Pdia6,Ctps,Ptpn2,Il17ra,Zbtb16,Il4ra,Thoc1,Ap3d1,Tpd52,Psen1,Exosc3,Cd38,Itgb3,Itgb2,Vav1,Ccl19,Camk2b,Cdkn1a,Gadd45g,Sbno2,Snap23,Pik3c2a,Coro1a,Pycard,Alox12,Txlna,Cd48,Hsp90aa1,Fcgr3,Lcp2,Clptm1,Prkce,Myo1f,Il6st |
| LV | G4 | cellular protein modification process | 0.39691 | Selk,Bfar,Pdgfc,Prkag2,Garem,Senp6,Pim3,Arl6ip5,Metap2,Hck,Etf1,Btg2,Ntmt1,Anapc4,Mvp,Ppap2a,Map3k3,Krtcap2,B4galt1,Pole4,Bmper,Ficd,Plcl1,Fktn,Errfi1,Ctgf,Nktr,Pdcd10,Marveld3,Syvn1,Braf,Senp2,Efna1,Nod1,Ibtk,Ube2s,Trim39,Csnk1a1,Cwc27,Rlim,Dok2,Atp7a,Usp8,Usp1,Tnfaip1,Eogt,Aak1,Trib1,Grb2,Atf3,Herpud1,Slc11a1,Mthfr,Mmp9,Cad,Stt3a,Lpin1,Uba5,Jmjd6,Lpar1,Yod1,Fgr,Arf4,Camkk2,Ube2f,Fgb,Rpn1,Tgm2,Uspl1,S100a8,Egf,Fkbp11,Nedd4l,C1galt1,Wdr61,Traf7,Acer2,Dpy19l1,Saa3,Usp14,Tbl1xr1,Ufm1,Cul5,Ube2j1,Vcp,Dab2,Dusp2,Asb4,Syk,St6gal1,Hmga2,Adam17,Cd44,Neurl3,Prmt1,Dzip3,Iqgap1,Dnajc10,F13a1,Sox9,Nus1,Wfs1,Supt6,Hipk3,Tmem165,Gas6,Trim27,Rac2,Ppp2r1a,Il12rb1,Cdc73,Tspyl2,Smarcad1,Rasgrp1,Tirap,Fkbp5,Tlr1,Trip12,Ddost,Ick,Adam9,Hspa5,Jun,Nlrp12,Nup62,Wipi1,Crtc2,Ptpn2,Ptpn1,Abca1,Ufl1,Znrf1,Plaur,Nceh1,Parp3,Mppe1,Elp3,Inhbe,B3galt1,Dph5,Brd8,Il1a,Alg12,Stk17b,Pggt1b,Tiparp,Eya3,Slc9a3r1,Prkrip1,Psen1,Piga,Itgb3,Itgb2,Arid5a,Ccl19,Gan,Wdr45b,St3gal3,Dnajc3,Trim68,Rcor1,Socs3,Dr1,Camk2b,Trim3,Rps6ka3,Tnfrsf1a,Nek7,Trib2,Cdkn1a,Gadd45b,Gadd45g,Ppp1r11,Pik3c2a,Vegfc,Pycard,Fn1,Clock,B3galnt2,St3gal4,Pomgnt1,Ppm1l,Ccr1,Fabp4,Psmd14,Pgm3,Rusc1,Pofut2,Galnt2,Taf1,Enc1,Josd2,Lats2,Prkch,Prkce,Anapc16,Il6st,Ikbkap,S100a9,Ube2g2 |
| LV | G4 | regulation of response to wounding | 0.39813 | Selp,Pik3ap1,Cd28,Il1r1,Braf,Cd55,Psma1,Adora1,Tnfaip6,Lbp,S100a8,A2m,Fcer1g,Tnfrsf12a,Syk,Vimp,Ctla2a,Plek,Nlrp12,Ptpn2,Casp4,Il17ra,Socs3,Tnfrsf1b,Tnfrsf1a,Sbno2,Pycard,Clock,Alox12,Cr1l,Fabp4,Fcgr3,Scara5,Prkce,Irg1,S100a9 |
| LV | G4 | Transport to the Golgi and subsequent modification | 0.40154 | B4galt1,Mcfd2,Sec24d,St6gal1,Preb,Sec31a,Man1a,Sec13,St3gal4 |
| LV | G4 | KSRP destabilizes mRNA | 0.4071 | Exosc1,Exosc4,Parn,Dis3,Exosc3,Khsrp |

[Table 18-12]

| Organ | group | Term | Pvalue | Genelist |
|---|---|---|---|---|
| LV | G4 | RNA Polymerase II Pre-transcription Events | 0.45004 | Gtf2e1,Ell,Tbp,Gtf2f1,Gtf2f2,Nelfa,Supt5,Taf2,Cdk7,Gtf2a2,Taf4b,Taf1 |
| LV | G4 | cellular localization | 0.47989 | Mybbp1a,Mfsd7b,Yipf5,Armcx3,Sec61b,Golga5,Snx6,Tacc2,Atg13,Mlx,Ezr,Stx6,Il1rn,Pim3,Cog6,Tbccd1,B4galt1,Rab12,Srp68,Stx12,Stx18,Cxcl9,Arcn1,Pdcd10,Uso1,Hif1a,Lcn2,Krt18,Braf,Timm21,Creb3,Rabl3,Ibtk,Plrg1,Gosr2,Blzf1,Slc11a1,Pdzd11,Snip1,Ap1g1,Fgr,Arf4,Fgb,Tmed9,Egf,Ica1,Ap2a2,Tacc1,Derl1,Atg9b,Atp2a2,Litaf,Sar1a,Itpr1,Ipo4,Vamp4,Rab35,Fcer1g,Zfyve16,Mapre1,Vcp,Dab2,Actr2,Syk,Hmga2,Vimp,Srgn,Rraga,Ap4e1,Copb2,Ddx39,Myh9,Casc3,Serp1,Zw10,Golph3l,Rab5b,Creb3l2,Plek,Sox9,Nus1,Chic2,Cklf,Supt6,Tmem165,Gas6,Trim27,Rac2,Golga4,Dynll1,Sdc4,Gm21540,Itgal,Dst,Ncf1,Rasgrp1,Ick,Pcm1,Kif5b,Hnf4a,Adam9,Hspa5,Golph3,Jun,Sec61a1,Nlrp12,Ralgapa2,Flot1,Crp,Capn10,Wipi1,Sidt2,Rab18,Ptpn1,Ap1ar,Abca1,Casp4,Parp3,Mppe1,Il4ra,Thoc2,Thoc1,Exosc10,Il1a,Bet1l,Ap3d1,Syt12,Slc9a3r1,Sec22b,Hook1,Psen1,Ap5s1,Exosc3,Itgb2,Ccl19,Wdr45b,Asun,Snx10,S100a10,Trem3,Rint1,Srp72,Tmem173,Rab1,Ift20,C2cd5,Cdkn1a,Hk2,Snap23,Sec13,Coro1a,Sybu,Pycard,Stx5a,Clock,Bbs4,Aspm,Chmp4b,Nemf,Ccr1,Clec4n,Seh1l,Hsp90aa1,Ero1l,Fcgr3,Rab33b,Calr,Lcp2,Tbrg1,Lats2,Prkce,Myo1f |
| LV | G4 | Calnexin/calreticulin cycle | 0.48294 | Pdia3,Edem2,Edem3,Edem1,Calr |
| LV | G4 | RNA degradation | 0.49399 | Cnot11,Btg2,Mphosph6,Eif4e,Pfkp,Exosc1,Exosc4,Wdr61,Ttc37,Parn,Dis3,Exosc10,Exosc3 |
| LV | G4 | Carbohydrate metabolism | 0.51204 | Pcx,B4galt1,Pfkp,Nans,Nup98,Pkm,Pfkfb2,Ranbp2,Hyal1,Cd44,Nupl2,Slc2a3,Papss1,Ppp2r1a,Lyve1,Sdc4,Gfpt1,Csgalnact2,Nupl1,Nup62,B3galt6,Gne,St3gal3,Gmppa,Hk2,Gale,St3gal4,Seh1l,Uap1,Pgm3,Gmppb |
| LV | G4 | RNA transport | 0.51898 | Trnt1,Pnn,Eif4e,Eif3d,Senp2,Gm10094,Alyref,Nup98,Eif4g2,Ranbp2,Eif4a3,Casc3,Nupl2,Gemin8,Nupl1,Nup62,Eif2b4,Thoc2,Thoc1,Rnps1,Magoh,Sec13,Seh1l |
| LV | G4 | Ribosome biogenesis in eukaryotes | 0.52099 | Utp6,Wdr75,Gar1,Riok1,Rbm28,Gnl2,Nop58,Utp14b,Utp14a,Wdr43,Nop56,Eif6 |
| LV | G4 | protein folding | 0.53287 | Qsox1,Fkbp11,Pdia3,Dnajc10,Txnl1,Fkbp5,Pdia6,Pdia4,Erp44,Itgb3,Txndc5,Hsp90aa1,Ero1l |
| LV | G4 | apoptotic signaling pathway | 0.54199 | Selk,Mybbp1a,Cyp1b1,Cd28,Arl6ip5,Pdcd10,Pnp,Hif1a,Dap,Lcn2,Krt18,Syvn1,Creb3,Trim39,Serinc3,Atp7a,Atf3,Herpud1,Mmp9,Skil,Fgb,S100a8,Trps1,Traf7,Pdia3,Itpr1,Bag3,Dab2,Tnfrsf12a,Vimp,Srgn,Cd44,Dnajc10,Wfs1,Ppp2r1a,Tnfrsf22,Grina,Ptpn2,Ptpn1,Casp4,Plaur,Parp2,Il1a,Slc9a3r1,Psen1,Cdip1,Camk2b,Tnfrsf1b,Tnfrsf1a,Hyou1,Cdkn1a,Sod2,Pycard,Trp63,Ivns1abp,Ero1l,S100a9 |
| LV | G4 | Immune System | 0.56558 | Pik3ap1,Sec61b,Cd28,Il1rn,Hck,Ap2b1,Anapc4,Adcy1,Map3k3,Il1r1,Hsp90b1,Actg1,Eif4e,Nod1,Fbxo6,Icam2,Clec4d,Tank,Capza1,Psma1,Irf8,Grb2,Tpp2,Lbp,Cd14,Uba5,Sec24d,Ap1g1,Fgr,Vasp,Egf,Nedd4l,Ifitm2,Ifitm1,Ap2a2,Pdia3,Itpr1,Dapp1,Fcer1g,Pvr,Cul5,Nrg4,Actr2,Asb3,Asb4,Syk,Adam17,Cd44,Dzip3,Csf2rb,H2-D1,Ppp2r1a,Irak2,Dynll1,Ncf1,Rasgrp1,Tirap,Trip12,Ddost,Mt2,Jun,Sec61a1,Crp,Ptpn2,Ptpn1,Zbtb16,Il1a,Sec31a,Itgb2,Vav1,Gan,Trim68,Socs3,Camk2b,Rps6ka3,Tmem173,Cdkn1a,Sec13,Pycard,Clec4n,Cr1l,Hsp90aa1,Fcgr3,Psmd14,Calr,Polr3d,Lcp2,Polr3k,Nfkbib,Sugt1,Prkce,Il6st |
| LV | G4 | symbiosis, encompassing mutualism through parasitism | 0.56648 | Creb3,Cd55,Irf8,Lbp,Apcs,Osbp,Ifitm2,Ifitm1,Vcp,Hmga2,Rraga,Pcsk5,Gas6,Trim27,Ncf1,Tirap,Jun,Thoc2,Thoc1,Itgb3,Atg16l2,Trem3,Rab1,Chmp4b |
| LV | G4 | Zinc transporters | 0.59484 | Slc30a5,Slc39a6,Slc39a7,Slc39a10,Slc39a14,Slc30a7 |
| LV | G4 | cellular component assembly | 0.64457 | Selp,Pdgfc,Atg13,Ezr,Senp6,Polr1e,Hck,Capg,Rpf2,Hsp90b1,Atp6v0a1,Stx18,Ctgf,Ccp110,Pdcd10,Arhgef26,Actg1,Marveld3,Eif3d,Braf,Timm21,2610034B18Rik,Lrrc16a,Ube2s,Gls,Capza1,Mpp7,Atp7a,Tnfaip1,Irf8,Grb2,Tsr1,Skil,Wdr1,Nup98,Lpar1,Vasp,Fgb,Pde4dip,Cct3,Foxred1,Atg9b,Arpc1b,Sar1a,Ipo4,Vamp4,Mapre1,Vcp,Mcm2,Dab2,Actr2,Scfd1,Hmga2,Hyal1,Zw10,Plec,Plek,Sox9,Gas6,Trim27,Rac2,Gemin8,Sdc4,Pld1,Rasgrp1,Trp53inp2,Apoa4,Apoa5,Ick,Hist1h2bp,Pcm1,Atf7ip,Golph3,Jun,Nudt21,Flot1,Ubxn2b,Denr,Wipi1,Intu,Ap1ar,Abca1,Med25,Brix1,Cda,Slc9a3r1,Psen1,Itgb3,Ccl19,Pdcl,Gtf2a2,Wdr45b,Atg16l2,Snx10,S100a10,Arfip2,Trim6,Rab1,Ift20,Sod2,Snap23,Pik3c2a,Coro1a,Pycard,Map1lc3b,Trp63,Fn1,Bbs4,Aspm,Chmp4b,Hsp90aa1,Srsf1,Rab33b,Scara5,Calr,Atl2,Atl1,Prkch,Prkce,Eif6,Crnkl1 |
| LV | G4 | myeloid leukocyte activation | 0.70771 | Slc11a1,Lbp,Jmjd6,Fgr,Fcer1g,Syk,Rac2,Rasgrp1,Tlr1,Adam9,Jun,Il4ra,Psen1,Sbno2,Snap23,Pycard,Fcgr3,Lcp2,Prkce,Myo1f |
| LV | G4 | Translation | 0.71134 | Sec61b,Etf1,Srp68,Eif4e,Eif3d,Ssr1,Ssr2,Spcs2,Spcs3,Ssr4,Gspt2,Rpn1,Casc3,Ppp2r1a,Srp9,Ddost,Sec61a1,Srp19,Srprb,Eif2b4,Rnps1,Rps9,Srp72,Magoh,Srpr,Srp54a |
| LV | G4 | Cholesterol biosynthesis | 0.71943 | Mvd,Cyp51,Idi1,Lbr,Sqle,Lss |
| LV | G4 | cytoplasmic transport | 0.73544 | Mybbp1a,Yipf5,Sec61b,Atg13,Mlx,Rab12,Srp68,Stx18,Cxcl9,Uso1,Krt18,Timm21,Creb3,Ibtk,Gosr2,Blzf1,Slc11a1,Snip1,Ap1g1,Egf,Derl1,Litaf,Sar1a,Itpr1,Ipo4,Vamp4,Rab35,Zfyve16,Vcp,Dab2,Actr2,Syk,Vimp,Ddx39,Zw10,Rab5b,Creb3l2,Chic2,Supt6,Gas6,Rac2,Golga4,Golph3,Jun,Sec61a1,Nlrp12,Wipi1,Ap1ar,Mppe1,Thoc2,Thoc1,Ap3d1,Sec22b,Hook1,Ccl19,Rint1,Srp72,Tmem173,Rab1,Cdkn1a,Sec13,Coro1a,Stx5a,Chmp4b,Nemf,Hsp90aa1,Ero1l,Rab33b,Calr,Prkce |
| LV | G4 | cellular zinc ion homeostasis | 0.76449 | Slc30a5,Slc39a6,Slc39a14,Slc30a7,Mt2,Mt1,Ap3d1 |
| LV | G4 | establishment of localization in cell | 0.82981 | Mybbp1a,Mfsd7b,Yipf5,Sec61b,Golga5,Snx6,Atg13,Mlx,Ezr,Stx6,Il1rn,Pim3,Cog6,B4galt1,Rab12,Srp68,Stx12,Stx18,Cxcl9,Arcn1,Pdcd10,Uso1,Hif1a,Lcn2,Krt18,Braf,Timm21,Creb3,Rabl3,Ibtk,Gosr2,Blzf1,Slc11a1,Pdzd11,Snip1,Ap1g1,Fgr,Fgb,Tmed9,Egf,Ica1,Ap2a2,Derl1,Atp2a2,Litaf,Sar1a,Itpr1,Ipo4,Vamp4,Rab35,Fcer1g,Zfyve16,Vcp,Dab2,Actr2,Syk,Hmga2,Vimp,Srgn,Ap4e1,Copb2,Ddx39,Myh9,Serp1,Zw10,Golph3l,Rab5b,Creb3l2,Plek,Nus1,Chic2,Cklf,Supt6,Tmem165,Gas6,Trim27,Rac2,Golga4,Dynll1,Sdc4,Gm21540,Dst,Ncf1,Rasgrp1,Ick,Pcm1,Kif5b,Hnf4a,Adam9,Golph3,Jun,Sec61a1,Nlrp12,Crp,Capn10,Wipi1,Sidt2,Rab18,Ptpn1,Ap1ar,Abca1,Casp4,Mppe1,Il4ra,Thoc2,Thoc1,Il1a,Bet1l,Ap3d1,Syt12,Sec22b,Hook1,Psen1,Ap5s1,Ccl19,Trem3,Rint1,Srp72,Tmem173,Rab1,Ift20,C2cd5,Cdkn1a,Hk2,Snap23,Sec13,Coro1a,Sybu,Pycard,Stx5a,Clock,Chmp4b,Nemf,Ccr1,Clec4n,Seh1l,Hsp90aa1,Ero1l,Fcgr3,Rab33b,Calr,Lcp2,Tbrg1,Prkce,Myo1f |
| LV | G4 | organic substance catabolic process | 0.84797 | Sdsl,Cyp1b1,Bfar,Sec61b,Prkag2,Tat,Timp1,Btg2,Rab12,Hsp90b1,Pnp,Hif1a,Syvn1,Timp3,Fbxo6,Ctif,Ube2s,Trim39,Pcyox1,Pfkp,Gls,Rlim,Psma1,Usp8,Entpd7,Usp1,Tnfaip1,Trib1,Herpud1,Gzma,Exosc4,Lpin1,Yod1,Egf,Nedd4l,Fen1,Wdr61,Pkm,Derl1,Hdc,Usp14,Tbl1xr1,Pfkfb2,Cul5,Cln8,Ube2j1,Vcp,Dab2,Vimp,Hyal1,Cd44,Ctla2a,Ctla2b,Dnajc10,Sox9,Sdf2l1,Odc1,Wfs1,Lyve1,Parn,Exog,Ncf1,Dis3,Tha1,Apoa4,Apoa5,Trip12,Adam9,Hspa5,Ubxn2b,Secisbp2,Ptpn1,Ufl1,Znrf1,Rnps1,Exosc10,Fbxo31,Cda,Tiparp,Sec22b,Psen1,Exosc3,Zfand2a,Khsrp,Kctd10,Dnajc3,Ubxn4,Tnfrsf1b,Edem3,Trib2,Edem1,Nbas,Hk2,Sds,Gale,Magoh,Stx5a,Clock,Chmp4b,Psmd14,Taf1,Enc1,Prkce,Ube2g2 |
| LV | G4 | retrograde protein transport, ER to cytosol | 0.90417 | Sec61b,Derl1,Vcp,Vimp |
| SP | G1 | Interferon Signaling | 1.4E-08 | Uba7,H2-T24,Ube2l6,Oas2,Trim12a,Trim12c,Oas1a,Fcgr1 |

[Table 18-13]

| Organ | group | Term | Pvalue | Genelist |
|---|---|---|---|---|
| SP | G1 | Interferon gamma signaling | 6.9E-06 | H2-T24,Oas2,Trim12a,Trim12c,Oas1a,Fcgr1 |
| SP | G1 | response to virus | 0.12414 | Oas2,Ifi27l2a,Adar,Samhd1,Oas1a |
| SP | G1 | ISG15-protein conjugation | 0.20952 | Uba7,Ube2l6 |
| SP | G1 | mRNA Editing | 0.39433 | Adar |
| SP | G1 | Herpes simplex infection | 0.39469 | H2-T24,Oas2,Sp100,Oas1a |
| SP | G1 | synaptic growth at neuromuscular junction | 0.43929 | Lrp4,Agrn |
| SP | G1 | response to other organism | 0.71946 | Oas2,Ifi27l2a,Adar,Samhd1,Oas1a,Fcgr1 |
| SP | G2 | Cytokine-cytokine receptor interaction\|Endocytosis\|Herpes simplex infection | 0.02507 | H2-Q10,Cxcr2,Flt3,Lepr,Mrc1,Igf1r,Ctsb,Pdgfra,Maf,Il13ra1,Ccl6,Ccr1,Cxcl12,Fcgr3 |
| SP | G2 | Cytokine-cytokine receptor interaction | 0.03758 | Cxcr2,Flt3,Lepr,Pdgfra,Il13ra1,Ccl6,Ccr1,Cxcl12 |
| SP | G2 | positive regulation of monocyte chemotaxis | 0.23286 | Pla2g7,Ccr1,Cxcl12 |
| SP | G2 | Glutathione metabolism | 0.23841 | Gsr,Gsta3,Ggt5 |
| SP | G2 | cell motility | 0.4525 | Cxcr2,Igf1r,Gcnt2,Pdgfra,Nr2f2,Tgfbr3,Nlrp12,Pla2g7,Ccr1,Cxcl12,Fcgr3 |
| SP | G2 | locomotion | 0.53194 | Cxcr2,Flot1,Igf1r,Gcnt2,Pdgfra,Nr2f2,Tgfbr3,Nlrp12,Pla2g7,Ccr1,Cxcl12,Fcgr3 |
| SP | G2 | immune system process | 0.58417 | Rab27a,H2-Q10,Cxcr2,Flt3,Mrc1,Igf1r,Pdgfra,Tgfbr3,Nlrp12,Pla2g7,Ccr1,Mertk,Mafb,Cxcl12,Fcgr3 |
| SP | G2 | lipid metabolic process | 0.6968 | Lepr,Hsd17b11,Pdgfra,Cyp2d22,Pla2g7,Ggt5,Enpp6,Alox5ap,Galc |
| SP | G2 | sulfur compound metabolic process | 0.71823 | Acpp,Ctns,Gsr,Gsta3 |
| SP | G2 | Lysosome | 0.97588 | Ap1s2,Ctsb,Ctns,Galc |
| LN | G1 | response to stress | 1.9E-05 | Rbbp8,Pik3ap1,Ier3,C5ar1,Clec4n,Alox5,Prkd1,Nlrp12,Plek,Lgr4,Hmox1,Tbx3,S100a8,Il1r1,Clec5a,Trem1,Trem3,Cd44 |
| LV | G3 | immune system process | 0.00642 | Sirpa,Cd93,Clec4n,Nlrp12,Lgr4,Hmox1,Fam20c,S100a8,Clec5a,Cebpd,Trem1,Trem3,Cd44,Pawr |
| SP | G3 | cytokine secretion | 0.00643 | Clec4n,Nlrp12,Lgr4,Clec5a,Trem1,Trem3 |
| SP | G3 | defense response | 0.01338 | Ier3,C5ar1,Clec4n,Alox5,Nlrp12,Hmox1,S100a8,Il1r1,Clec5a,Trem1,Trem3,Cd44 |
| SP | G3 | locomotion | 0.01413 | Dpysl3,C5ar1,Prkd1,Nlrp12,Myo10,Mmp9,Gcnt2,S100a8,Clec5a,Trem1,Trem3 |
| SP | G3 | cell motility | 0.01771 | Dpysl3,C5ar1,Prkd1,Nlrp12,Myo10,Mmp9,Gcnt2,S100a8,Trem1,Trem3 |
| SP | G3 | protein secretion | 0.02786 | Clec4n,Nlrp12,Plek,Lgr4,Clec5a,Trem1,Trem3,Rab15 |
| SP | G3 | osteoblast differentiation | 0.09015 | Id2,Prkd1,Lgr4,Fam20c,Clec5a,Cebpd |
| SP | G3 | secretion | 0.13843 | Clec4n,Nlrp12,Plek,Lgr4,Tbx3,S100a8,Clec5a,Trem1,Trem3,Rab15 |
| SP | G3 | cell death | 0.18627 | Ier3,Prkd1,Nlrp12,Mmp9,Hmox1,Tbx3,S100a8,Clec5a,Cd44,Pawr |
| SP | G3 | neutrophil chemotaxis | 0.21941 | C5ar1,S100a8,Trem1,Trem3 |
| LN | G1 | response to stress | 0.02261 | Rbbp8,Pik3ap1,Ier3,C5ar1,Clec4n,Alox5,Prkd1,Nlrp12,Plek,Hmox1,Tbx3,S100a8,Il1r1,Clec5a,Trem1,Trem3,Cd44 |
| SP | G3 | positive regulation of multicellular organismal process | 0.25448 | Dpysl3,Id2,C5ar1,Clec4n,Prkd1,Plek,Lgr4,Mmp9,Gcnt2,Fam20c,Clec5a,Cebpd |
| SP | G3 | response to wounding | 0.30016 | Dpysl3,Ier3,Nlrp12,Plek,Hmox1,S100a8,Il1r1,Cd44 |
| SP | G3 | secretion by cell | 0.32505 | Clec4n,Nlrp12,Plek,Lgr4,Tbx3,Clec5a,Trem1,Trem3,Rab15 |
| SP | G3 | inflammatory response | 0.34004 | Ier3,Alox5,Nlrp12,Hmox1,S100a8,Il1r1,Cd44 |
| SP | G3 | transport | 0.34214 | Ier3,Clec4n,Prkd1,Nlrp12,Plek,Myo10,Lgr4,Mmp9,Tbx3,S100a8,Clec5a,Slc25a13,Trem1,Trem3,Rab15 |
| SP | G3 | cytokine production | 0.36944 | C5ar1,Clec4n,Nlrp12,Lgr4,Clec5a,Trem1,Trem3,Pawr |
| SP | G3 | Innate Immune System\|Adaptive Immune System\|Hemostasis\|Pathways in cancer\|Developmental Biology\|PI3K-Akt signaling pathway\|Signaling by Rho GTPases | 0.38688 | Dpysl3,Id2,Fosl2,Clec4n,Ppp1r3b,Alox5,Prkd1,Cd244,Plek,Myo10,Mmp9,Hmox1,Tbx3,Il1r1,Clec5a,Cebpd,Trem1,Cd44 |
| SP | G3 | neutrophil extravasation | 0.51755 | Trem1,Trem3 |
| SP | G3 | bundle of His development | 0.51755 | Id2,Tbx3 |
| SP | G3 | cell proliferation | 0.80295 | Id2,C5ar1,Fosl2,Prkd1,Lgr4,Gcnt2,Hmox1,Tbx3,Pawr |
| SP | G3 | cell adhesion | 0.84305 | Mcam,Plek,Myo10,Gcnt2,S100a8,Cd44,Pawr |
| SP | G3 | generation of precursor metabolites and energy | 0.96858 | Ier3,Ppp1r3b,Slc25a13 |
| SP | G4 | Innate Immune System\|Adaptive Immune System\|Hemostasis\|Pathways in cancer\|Developmental Biology\|PI3K-Akt signaling pathway\|Signaling by Rho GTPases | 0.02903 | Cdkn2b,Cxcl2,Socs2,Atp6v0c,Insm1,Dusp5,Scin,Anxa2,Tnfrsf1b,Ccl22,Plek,Mmp14,Ccl3,Sgpl1,Slc7a11,F10,Pdgfrb,Gadd45b,Prok2,Cacnb3 |

[Table 18-14]

| Organ | group | Term | Pvalue | Genelist |
|---|---|---|---|---|
| SP | G4 | locomotion | 0.0655 | Cxcl2,Insm1,Anxa3,Rap2b,Mmp14,Ccl3,Sgpl1,Pdgfrb,Strip2,Prok2 |
| SP | G4 | cell motility | 0.08528 | Cxcl2,Insm1,Anxa3,Rap2b,Mmp14,Ccl3,Sgpl1,Pdgfrb,Strip2 |
| SP | G4 | response to wounding | 0.3771 | Rap2b,Anxa2,Tnfrsf1b,Plek,Ccl3,Slc7a11,Papss2,Myof |
| SP | G4 | blood coagulation | 0.42852 | Rap2b,Anxa2,Plek,Slc7a11,Papss2 |
| SP | G4 | regulation of body fluid levels | 0.52026 | Socs2,Rap2b,Anxa2,Plek,Slc7a11,Papss2 |
| SP | G4 | wound healing | 0.7751 | Rap2b,Anxa2,Plek,Slc7a11,Papss2,Myof |
| SP | G3 | immune system process | 0.00433 | Id2,C5ar1,Anxa3,Samsn1,Scin,Anxa2,Ccl3,Sgpl1,Gpr183,Cacnb3 |
| SP | G4 | Chemokine receptors bind chemokines | 0.8244 | Cxcl2,Ccl22,Ccl3 |
| SP | G4 | cell proliferation | 0.8418 | Cdkn2b,Cxcl2,Insm1,Scin,Tnfrsf1b,Mmp14,Pdgfrb,Gpr183,Prok2 |
| SP | G4 | Cytokine-cytokine receptor interaction | 0.95292 | Cxcl2,Tnfrsf1b,Ccl22,Ccl3,Pdgfrb |
| LN | G1 | cellular nitrogen compound metabolic process | 0.09306 | Hopx,Dnajb6,Aen,Trdmt1,Tlr9,Hck,Scaf11,Raf1,Bcl9,Nabp1,Wdr33,Thrap3,Timeless,Cask,Rad54l2,Smc1a,Cebpd,Tox4,Fkbp8 |
| SP | G3 | response to stress | 0.0005 | Dpysl3,Id2,Tlr9,Hck,Tnfrsf1b,Bcl9,Nabp1,Timeless,Cask,Rnf34,Smc1a,S1pr3 |
| LN | G1 | Immune System | 1.1E-10 | Pik3ap1,Trim30a,Trim30d,Sos1,Irf9,Ctss,Gm14446,Dtx3l,Ifih1,Ifi204,Ifi205,Asb13,Uba7,Lgals9,Psmb9,Psmb8,Hck,Psmb10,Stat2,Stat1,Klrk1,Trim12c,Oas1a,Ywhaz,Trip12,H2-M3,Ifitm3,Raf1,Blnk,Capza2,Keap1,Psme4,Trim21,Cybb,Nod1,Pml,Casp8,Actr2,Tapbp,Phlpp1,Mndal |
| LV | G4 | immune system process | 0.09263 | Selk,Selp,Trim30a,Sos1,Ctsc,Slamf7,Ctss,Adar,Tgtp1,Ifih1,Ifi204,Cxcl10,Psmb9,Psmb8,Hck,Trafd1,Psmb10,Nmi,Stat2,Stat1,Klrk1,Trim12c,Ankrd17,Samd9l,Parp9,Dhx58,Prkx,H2-M3,Ifitm3,Gbp3,Gbp7,Pnp,Blnk,Samhd1,Trim21,Cybb,Nod1,Pml,Casp8,Tapbp,Herc6,Elmod2,Cd47,Dpp4 |
| LN | G1 | Cytokine Signaling in Immune system | 1.1E-06 | Trim30a,Trim30d,Sos1,Irf9,Gm14446,Uba7,Lgals9,Stat2,Stat1,Trim12c,Oas1a,Ywhaz,H2-M3,Raf1,Blnk,Trim21,Nod1,Pml |
| LN | G1 | innate immune response | 5.4E-06 | Pik3ap1,Adar,Ifih1,Hck,Trafd1,Nmi,Stat1,Klrk1,Trim12c,Ankrd17,Parp9,Dhx58,H2-M3,Ifitm3,Gbp3,Gbp7,Samhd1,Trim21,Cybb,Pml |
| SP | G3 | response to stress | 0.25294 | Dpysl3,Id2,Trim30a,Shisa5,Bfar,Ctss,Adar,Dtx3l,Ifih1,Pdk3,Cxcl10,Hck,Nono,Dusp28,Trafd1,Ifi47,Nmi,Stat2,Stat1,Klrk1,Trim12c,Ankrd17,Parp9,Dhx58,Trip12,H2-M3,Ifitm3,Gbp3,Gbp7,Pdcd10,Pnp,Chmp4b,Sh3glb1,Dek,Irgm1,Samhd1,Tlk2,Psme4,Trim21,Cybb,Nod1,Pml,Hmox2,Tapbp,Xrn2,Zfyve26,Elmod2,Cd47,Aida,Dpp4 |
| LN | G1 | Interferon Signaling | 5.9E-05 | Trim30a,Trim30d,Irf9,Gm14446,Uba7,Stat2,Stat1,Trim12c,Oas1a,H2-M3,Trim21,Pml |
| LN | G1 | Adaptive Immune System | 0.00013 | Pik3ap1,Sos1,Ctss,Dtx3l,Asb13,Uba7,Psmb9,Psmb8,Psmb10,Klrk1,Ywhaz,Trip12,H2-M3,Ifitm3,Raf1,Blnk,Keap1,Psme4,Trim21,Cybb,Tapbp,Phlpp1 |
| LN | G1 | defense response | 0.0002 | Pik3ap1,Ctss,Adar,Ifih1,Cxcl10,Hck,Trafd1,Ifi47,Nmi,Stat2,Stat1,Klrk1,Trim12c,Ankrd17,Parp9,Dhx58,H2-M3,Ifitm3,Gbp3,Gbp7,Irgm1,Samhd1,Trim21,Cybb,Nod1,Pml,Tapbp,Elmod2,Cd47,Dpp4 |
| LN | G1 | Class I MHC mediated antigen processing & presentation | 0.00045 | Ctss,Dtx3l,Asb13,Uba7,Psmb9,Psmb8,Psmb10,Trip12,H2-M3,Keap1,Psme4,Trim21,Cybb,Tapbp |
| LN | G1 | immune response | 0.00075 | Pik3ap1,Ctsc,Ctss,Adar,Tgtp1,Ifih1,Cxcl10,Hck,Trafd1,Nmi,Stat1,Klrk1,Trim12c,Ankrd17,Parp9,Dhx58,H2-M3,Ifitm3,Gbp3,Gbp7,Pnp,Blnk,Samhd1,Trim21,Cybb,Pml |
| LN | G1 | Interferon gamma signaling | 0.00191 | Trim30a,Trim30d,Irf9,Stat1,Trim12c,Oas1a,H2-M3,Trim21,Pml |
| LN | G1 | response to other organism | 0.00301 | Adar,Tgtp1,Ifih1,Plscr3,Cxcl10,Hck,Stat2,Stat1,Klrk1,Oas1a,Ankrd17,Dhx58,H2-M3,Ifitm3,Gbp3,Gbp7,Samhd1,Nod1,Pml,Ifit2,Elmod2,Cd47 |
| LN | G1 | Influenza A | 0.00378 | Irf9,Adar,Akt3,Ifih1,Cxcl10,Stat2,Stat1,Oas1a,Raf1,Tnfsf10,Pml |
| LN | G1 | Herpes simplex infection | 0.00465 | Daxx,Irf9,Gm14446,Ifih1,Stat2,Stat1,Oas1a,H2-T10,H2-M3,Pml,Casp8,Sp100 |
| LN | G1 | regulation of innate immune response | 0.00467 | Pik3ap1,Trim30a,Adar,Trafd1,Nmi,Klrk1,Trim12c,Ankrd17,Parp9,Dhx58,H2-M3,Samhd1 |
| LN | G1 | response to interferon-beta | 0.0049 | Ifi205,Stat1,Ifitm3,Gbp3,Xaf1,Pyhin1 |
| LN | G1 | Innate Immune System\|Adaptive Immune System\|Hemostasis\|Pathways in cancer\|Developmental Biology\|PI3K-Akt signaling pathway\|Signaling by Rho GTPases | 0.00504 | Cers6,Daxx,Azi2,Pik3ap1,Trim30a,Trim30d,Sos1,Irf9,Helz2,Ctss,Gm14446,Adar,Cd2ap,Akt3,Dtx3l,Ifih1,Ifi204,Ifi205,Cxcl10,Asb13,Rtn4,Uba7,Lgals9,Psmb9,Psmb8,Hck,Psmb10,Gm11787,Ifi47,Stat2,Stat1,Klrk1,Trim12c,Oas1a,H2-T10,Ywhaz,Dhx58,Trip12,Prkx,H2-M3,Ifitm3,Arhgap30,Raf1,Blnk,Tnfsf10,Capza2,Keap1,Pttg1,Irgm1,Psme4,Trim21,Cybb,Nod1,Pml,Ranbp2,Casp8,Cab39l,Actr2,Tapbp,Max,Rbl1,Phlpp1,Sp100,Cd47,Atp1b3,Mndal |

[Table 18-15]

| Organ | group | Term | Pvalue | Genelist |
|---|---|---|---|---|
| LN | G1 | symbiosis, encompassing mutualism through parasitism | 0.01421 | Trim30a,Adar,Stat1,Ifitm3,Gbp3,Gbp7,Chmp4b,Trim21,Pml,Dpp4 |
| LN | G1 | Antigen processing-Cross presentation | 0.01876 | Ctss,Psmb9,Psmb8,Psmb10,H2-M3,Psme4,Cybb,Tapbp |
| LN | G1 | regulation of response to stress | 0.02164 | Pik3ap1,Trim30a,Bfar,Ctss,Adar,Nono,Trafd1,Nmi,Stat2,Klrk1,Trim12c,Ankrd17,Parp9,Dhx58,Trip12,H2-M3,Pdcd10,Pnp,Chmp4b,Sh3glb1,Dek,Samhd1,Nod1,Pml,Elmod2,Cd47,Aida |
| LN | G3 | response to stress | 0.00102 | Rbm4,Cyp1b1,Shisa5,Bfar,Ctss,Adar,Dtx3l,Ifih1,Pdk3,Cxcl10,Hck,Nono,Dusp28,Trafd1,Ifi47,Nmi,Stat2,Stat1,Klrk1,Trim12c,Ankrd17,Parp9,Dhx58,Trip12,H2-M3,Ifitm3,Gbp3,Gbp7,Pdcd10,Pnp,Chmp4b,Sh3glb1,Dek,Irgm1,Samhd1,Tlk2,Psme4,Trim21,Cybb,Nod1,Pml,Hmox2,Tapbp,Xrn2,Zfyve26,Elmod2,Cd47,Aida,Dpp4 |
| LN | G1 | response to virus | 0.02368 | Adar,Tgtp1,Ifih1,Cxcl10,Stat2,Oas1a,Ankrd17,Dhx58,Ifitm3,Samhd1,Pml,Ifit2,Elmod2 |
| LN | G1 | Innate Immune System | 0.02895 | Sos1,Ctss,Ifih1,Ifi204,Ifi205,Psmb9,Psmb8,Hck,Psmb10,Klrk1,Raf1,Capza2,Psme4,Trim21,Nod1,Casp8,Actr2,Phlpp1,Mndal |
| LN | G1 | Antigen processing: Ubiquitination & Proteasome degradation | 0.03511 | Dtx3l,Asb13,Uba7,Psmb9,Psmb8,Psmb10,Trip12,Keap1,Psme4,Trim21 |
| LN | G1 | proteasomal ubiquitin-independent protein catabolic process | 0.05458 | Psmb9,Psmb8,Psmb10,Keap1,Psme4 |
| LN | G1 | regulation of defense response | 0.08271 | Pik3ap1,Trim30a,Ctss,Adar,Trafd1,Nmi,Stat2,Klrk1,Trim12c,Ankrd17,Parp9,Dhx58,H2-M3,Samhd1,Pml,Elmod2,Cd47 |
| LN | G1 | catabolic process | 0.10447 | Trim30a,Ctsc,Bfar,Ctss,Uba7,Synj1,Psmb9,Psmb8,Psmb10,Mycbp2,Stat2,Cnot6l,Rnf139,Samd9l,Trip12,Pnp,Phyh,Chmp4b,Sh3glb1,Keap1,Samhd1,Tlk2,Psme4,Pml,Casp8,Usp25,Xrn2,Herc6 |
| LN | G1 | defense response to other organism | 0.10585 | Adar,Cxcl10,Hck,Stat2,Klrk1,Ankrd17,Dhx58,H2-M3,Ifitm3,Gbp3,Gbp7,Samhd1,Nod1,Pml,Elmod2 |
| LN | G1 | response to interferon-alpha | 0.16494 | Adar,Ifi204,Ifitm3,Ifit2 |
| LN | G1 | Hepatitis C | 0.18519 | Sos1,Irf9,Gm14446,Akt3,Stat2,Stat1,Oas1a,Raf1 |
| LN | G1 | Measles | 0.19482 | Irf9,Adar,Akt3,Ifih1,Stat2,Stat1,Oas1a,Tnfsf10 |
| LN | G1 | STING mediated induction of host immune responses | 0.20015 | Ifi204,Ifi205,Trim21,Mndal |
| LN | G1 | B cell receptor signaling pathway | 0.2258 | Pik3ap1,Sos1,Akt3,Gm11787,Raf1,Blnk |
| LN | G1 | Signaling by Interleukins | 0.23245 | Sos1,Lgals9,Stat1,Ywhaz,Raf1,Blnk,Nod1 |
| LN | G1 | protein catabolic process | 0.29429 | Trim30a,Ctsc,Bfar,Ctss,Uba7,Psmb9,Psmb8,Psmb10,Mycbp2,Rnf139,Samd9l,Trip12,Chmp4b,Keap1,Tlk2,Psme4,Pml,Casp8,Usp25,Herc6 |
| LN | G1 | macromolecule catabolic process | 0.32226 | Trim30a,Ctsc,Bfar,Ctss,Uba7,Psmb9,Psmb8,Psmb10,Mycbp2,Cnot6l,Rnf139,Samd9l,Trip12,Chmp4b,Sh3glb1,Keap1,Tlk2,Psme4,Pml,Casp8,Usp25,Xrn2,Herc6 |
| LN | G1 | response to cytokine | 0.35197 | Adar,Ifi204,Ifi205,Serpina3g,Cxcl10,Stat1,Parp9,Ifitm3,Gbp3,Gbp7,Keap1,Pml,Ifit2,Casp8,Xaf1,Pyhin1 |
| LN | G1 | proteolysis involved in cellular protein catabolic process | 0.41796 | Ctsc,Bfar,Ctss,Uba7,Psmb9,Psmb8,Psmb10,Rnf139,Trip12,Chmp4b,Keap1,Tlk2,Psme4,Pml,Casp8,Usp25,Herc6 |
| LN | G1 | Ubiquitin mediated proteolysis\|Cell cycle | 0.48008 | Shisa5,Uba7,Ywhaz,Trip12,Keap1,Pttg1,Pml,Casp8,Rbl1 |
| LN | G1 | Chemokine signaling pathway | 0.50409 | Sos1,Akt3,Cxcl10,Hck,Gm11787,Stat2,Stat1,Prkx,Raf1 |
| LN | G1 | Lysosome | 0.54614 | Cers6,Acer3,Ctsc,Ctss,Gns,Scarb2,Laptm4a |
| LN | G1 | ER-Phagosome pathway | 0.59186 | Psmb9,Psmb8,Psmb10,H2-M3,Psme4,Tapbp |
| LN | G1 | Cytokine-cytokine receptor interaction\|Endocytosis\|Herpes simplex infection | 0.65657 | Daxx,Irf9,Ctss,Gm14446,Sgcb,Ifih1,Cxcl10,Stat2,Stat1,Oas1a,H2-T10,H2-M3,Blnk,Tnfsf10,Chmp4b,Sh3glb1,Cybb,Pml,Casp8,Tapbp,Sp100 |
| LN | G1 | Mitotic Anaphase | 0.72336 | Psmb9,Psmb8,Ankle2,Psmb10,Pttg1,Psme4,Ranbp2,Vrk1 |
| LN | G1 | APC/C:Cdc20 mediated degradation of Securin | 0.91491 | Psmb9,Psmb8,Psmb10,Pttg1,Psme4 |
| LN | G1 | multi-organism cellular process | 0.91725 | Trim30a,Adar,Stat1,Ankrd17,Dhx58,Ifitm3,Chmp4b,Trim21,Pml,Dpp4 |
| LN | G1 | cellular response to interferon-beta | 0.9289 | Ifi205,Stat1,Gbp3,Pyhin1 |
| SP | G4 | immune system process | 0.8014 | Cdkn2b,Cxcl2,Sos1,Slamf7,Adar,Ifi204,Cxcl10,Trafd1,Nmi,Stat2,Klrk1,Trim12c,Ankrd17,Parp9,Dhx58,H2-M3,Pnp,Samhd1,Nod1,Pml,Casp8,Elmod2,Cd47,Dpp4 |
| LN | G2 | Metabolism of lipids and lipoproteins | 0.03291 | Hsd3b7,Ptgs1,Scarb1,Elovl6,Pla2g15,Abca1,Acox3,Slc44a2,Pik3cg,Sgms1,Mgll,Gpam |
| LN | G2 | membrane organization | 0.05633 | Zdhhc23,Fcho1,Abca1,Reep1,Rhoq,Chchd3,Rap2a,Vamp4,Scn3b,Dab2,Myo1c,Nsf |
| LN | G2 | plasma membrane organization | 0.05711 | Zdhhc23,Rhoq,Rap2a,Vamp4,Scn3b,Dab2,Nsf |
| LN | G2 | Lysosome | 0.09937 | Ctsb,Tpp1,Cltb,Pla2g15,Sgms1,Ap1s2 |
| LN | G2 | Vesicle-mediated transport | 0.1992 | Scarb1,Cltb,Rhoq,Ap1s2,Dab2,Myo1c |

[Table 18-16]

| Organ | group | Term | Pvalue | Genelist |
|---|---|---|---|---|
| LN | G2 | biosynthetic process | 0.23956 | Dnmt3b,Pcgf6,Nr2f2,Flcn,Tfec,Zfhx3,Ptgs1,Zdhhc23,Pbx2,Lbp,Cux1,Zdhhc9,Scarb1,Gxylt1,Elovl6,Abca1,Hdac4,Rhoq,Sbno2,Trps1,Scd1,Ldb2,Chchd3,Pik3cg,Insr,Sgms1,Esrra,Osr1,Prg4,Nos3,Cdkn2d,Dab2,Nars2,Gpam,Brwd1,Hfe,Cd1d1 |
| LN | G2 | Metabolism of lipids and lipoproteins | 0.36512 | Hsd3b7,Ptgs1,Scarb1,Elovl6,Abca1,Acox3,Slc44a2,Pik3cg,Sgms1,Mgll,Gpam |
| LN | G2 | cell motility | 0.50891 | Gab1,Nr2f2,Flcn,Lbp,Scarb1,Rap2a,Pik3cg,Insr,Matn2,Nos3,Dab2,Mtus1,Eps8,Myo1c |
| LN | G2 | lipopolysaccharide transport | 0.56442 | Lbp,Scarb1 |
| LN | G2 | vesicle-mediated transport | 0.61791 | Lbp,Scarb1,Fcho1,Cltb,Abca1,Trpc2,Vamp4,Ap1s2,Clcn5,Dab2,Map4k2,Nsf |
| LN | G2 | locomotion | 0.81552 | Gab1,Nr2f2,Flcn,Lbp,Scarb1,Ceacam1,Rap2a,Pik3cg,Insr,Matn2,Nos3,Dab2,Mtus1,Eps8,Myo1c |
| LN | G2 | Biosynthesis of unsaturated fatty acids | 0.87766 | Elovl6,Acox3,Scd1 |
| LN | G2 | homeostatic process | 0.96158 | Flcn,Tpp1,Scarb1,Ubash3b,Ceacam1,Abca1,Ldb2,Trpc2,Insr,Scn3b,Cib2,Gpam,Hfe,Ccdc47 |
| LN | G3 | response to stress | 0.20788 | Rbm4,Cyp1b1,Atg4c,Ret,Pten,Gprc5b,Map3k6,Fktn,Pdcd4,Rad1,Serpine2,Glul,Errfi1,Apex1,Tsc22d3,Herc2,Hamp,Birc3,Nfkb2,Prcp,Scamp5,Thbs4,Ddit4,Cdc7,Per2,Bbc3,Hyal1,Pdia6,Pdia4,Acp5,Upf3b,Spred2,Kit,Fads1,Rad51d,Bid,Large,Lyst,Swsap1,Seh1l,Pdk4,Cd160,Mrc1,Ryr1,Nfe2l2,Tcf7l2,Stc2,Adam9,Acadm,Pik3cg,Pdgfra,Wipi1,Dab2,Agtr1a,Il27ra,Rps3,Uchl1,Clec10a,Cd36,Cxcl13,Rps6ka6,Dpep1,Bok,Comt,Bcl2l11,Tnk2,St8sia1,Fabp1,Srsf6,Polr3g,Scara5,Smc6,Ndufs8,Trpv1,Atp1b1,Fancc |
| LN | G3 | organic substance metabolic process | 0.00104 | Sorl1,Pabpn1,Rbm4,Kpna6,Cyp1b1,Hnrnpa1,Atg4c,Smurf2,Cops3,Ret,Pten,Chchd10,Gprc5b,Btg1,Prmt3,Tox2,Map3k6,Lss,Fktn,Pdcd4,Mrpl11,H3f3b,Rad1,Serpine2,Timp4,Ahcyl2,Errfi1,Apex1,Ddx23,Skp2,Tsc22d3,Trim37,Herc2,Klhl2,Rps17,Ndrg2,Gls,Rlim,Hamp,Birc3,Mcf2l,Nfkb2,Prcp,Ip6k2,Zbtb16,Thbs4,Ptgs1,Ddit4,Serpina3m,Serpina3n,Serpina3c,Zfp36l2,Mettl1,Ncor2,Thrb,Cdc7,Msc,Mbd3,Aebp2,Per3,Per2,Trmt1,Bbc3,Scd2,Hyal1,Foxred2,Ttll4,Ttll7,Pdia6,Pdia4,Upf3b,Tbl1xr1,Dis3l2,Fmo2,Tcf4,Rspo1,Spred2,Dusp4,Dusp6,Dusp1,Dapk2,Otud3,Kit,Fads1,Rad51d,Mthfd1,Acer2,Alg8,Alg3,Hoxd8,Bid,Wdr12,Pank1,Nrip1,Large,Lyst,Swsap1,Pdk4,Pik3ip1,Mafb,Mafg,Vars2,Pecr,Sae1,Plce1,Nfe2l2,Tcf7l1,Tcf7l2,Zmiz1,Fkbp5,Cdk4,Mvd,Stc2,Adam9,Acadm,Fcer2a,Pik3cg,Crp,Psmd11,Pdgfra,Vars,Zfp322a,Wipi1,Secisbp2,Ptpn3,Dab2,Agtr1a,Zfp932,Il27ra,Med24,Rps3,Brd7,Uchl1,Suv420h1,Dhx29,Cd36,Pinx1,Abcg2,Npy1r,Ccdc22,Pkig,Rps6ka6,Zfp52,Zfp53,Dpep1,Angpt4,Rprd1a,Bok,Gusb,Galc,Hpgd,Deptor,Dync2h1,Adk,Lrpprc,Comt,Vcam1,Glce,Ptpn22,Bcl2l11,Heatr1,Hagh,Mecr,Aldh1a1,Tnk2,St8sia6,Mmachc,Fabp1,Il7r,Srsf6,Zfp612,Polr3g,Nrp1,Scara5,Smc6,Maf,Adh1,Trpv1,Anapc16,Atp1b1,Fancc,Baz2a,Chst4,Fbl |
| LN | G3 | metabolic process | 0.01424 | Sorl1,Pabpn1,Rbm4,Kpna6,Cyp1b1,Hnrnpa1,Atg4c,Smurf2,Cops3,Evi5,Ret,Pten,Chchd10,Gprc5b,Btg1,Prmt3,Tox2,Map3k6,Lss,Fktn,Pdcd4,Mrpl11,H3f3b,Rad1,Serpine2,Timp4,Ahcyl2,Errfi1,Apex1,Myo7a,Ddx23,Skp2,Tsc22d3,Trim37,Herc2,Klhl2,Rps17,Ndrg2,Gls,Rlim,Hamp,Birc3,Mcf2l,Nfkb2,Prcp,Ip6k2,Zbtb16,Thbs4,Ptgs1,Ddit4,Serpina3m,Serpina3n,Serhl,Serpina3c,Zfp36l2,Mettl1,Ncor2,Thrb,Cdc7,Msc,Mbd3,Aebp2,Per3,Per2,Trmt1,Bbc3,Scd2,Hyal1,Foxred2,Ttll4,Ttll7,Pdia6,Pdia4,Acp5,Upf3b,Tbl1xr1,Dis3l2,Fmo2,Tcf4,Rspo1,Spred2,Dusp4,Dusp6,Dusp1,Dapk2,Otud3,Kit,Fads1,Rad51d,Mthfd1,Acer2,Alg8,Alg3,Hoxd8,Bid,Wdr12,Pank1,Nrip1,Large,Lyst,Swsap1,Pdk4,Pik3ip1,Mafb,Mafg,Vars2,Pecr,Sae1,Plce1,Nfe2l2,Tcf7l1,Tcf7l2,Zmiz1,Fkbp5,Cdk4,Mvd,Stc2,Pde1c,Dnajc24,Adam9,Kif13a,Acadm,Fcer2a,Pik3cg,Crp,Psmd11,Pdgfra,Vars,Zfp322a,Wipi1,Secisbp2,Ptpn3,Dab2,Agtr1a,Zfp932,Il27ra,Med24,Rps3,Brd7,Uchl1,Suv420h1,Dhx29,Vmp1,Tbrg4,Cd36,Pinx1,Abcg2,Npy1r,Cxcl13,Ccdc22,Pkig,Rps6ka6,Zfp52,Zfp53,Dpep1,Angpt4,Rprd1a,Bok,Gusb,Galc,Hpgd,Deptor,Dync2h1,Adk,Lrpprc,Comt,Vcam1,Glce,Ptpn22,Bcl2l11,Heatr1,Hagh,Mecr,Aldh1a1,Tnk2,St8sia6,Mmachc,Fabp1,Il7r,Srsf6,Zfp612,Polr3g,Nrp1,Plxnb1,Scara5,Smc6,Maf,Adh1,Trpv1,Anapc16,Atp1b1,Fancc,Baz2a,Chst4,Fbl |
| LN | G3 | Fatty acid metabolism | 0.01582 | Hsd17b12,Scd2,Fads1,Pecr,Acadm,Fads2,Mecr |
| LN | G3 | Biosynthesis of unsaturated fatty acids | 0.02407 | Hsd17b12,Scd2,Fads1,Pecr,Fads2 |
| LN | G3 | PPAR signaling pathway\|Peroxisome | 0.03946 | Hmgcs2,Hsd17b12,Scd2,Fads1,Pecr,Pex11a,Acadm,Cd36,Fads2,Mecr,Fabp1,Adh1 |
| LN | G3 | primary metabolic process | 0.04541 | Sorl1,Pabpn1,Rbm4,Kpna6,Cyp1b1,Hnrnpa1,Atg4c,Smurf2,Cops3,Ret,Pten,Chchd10,Gprc5b,Btg1,Prmt3,Tox2,Map3k6,Lss,Fktn,Pdcd4,Mrpl11,H3f3b,Rad1,Serpine2,Timp4,Ahcyl2,Errfi1,Apex1,Ddx23,Skp2,Tsc22d3,Trim37,Herc2,Klhl2,Rps17,Ndrg2,Gls,Rlim,Hamp,Birc3,Mcf2l,Nfkb2,Prcp,Ip6k2,Zbtb16,Thbs4,Ptgs1,Ddit4,Serpina3m,Serpina3n,Serpina3c,Zfp36l2,Mettl1,Ncor2,Thrb,Cdc7,Msc,Mbd3,Aebp2,Per3,Per2,Trmt1,Bbc3,Scd2,Foxred2,Ttll4,Ttll7,Pdia6,Pdia4,Upf3b,Tbl1xr1,Dis3l2,Fmo2,Tcf4,Rspo1,Spred2,Dusp4,Dusp6,Dusp1,Dapk2,Otud3,Kit,Fads1,Rad51d,Mthfd1,Acer2,Alg8,Alg3,Hoxd8,Bid,Wdr12,Pank1,Nrip1,Large,Lyst,Swsap1,Pdk4,Pik3ip1,Mafb,Vars2,Pecr,Sae1,Plce1,Nfe2l2,Tcf7l1,Tcf7l2,Zmiz1,Fkbp5,Cdk4,Mvd,Adam9,Acadm,Pik3cg,Psmd11,Pdgfra,Vars,Zfp322a,Wipi1,Secisbp2,Ptpn3,Dab2,Agtr1a,Zfp932,Il27ra,Med24,Rps3,Brd7,Uchl1,Suv420h1,Dhx29,Cd36,Pinx1,Npy1r,Ccdc22,Pkig,Rps6ka6,Zfp52,Zfp53,Dpep1,Angpt4,Rprd1a,Bok,Gusb,Galc,Hpgd,Deptor,Dync2h1,Adk,Lrpprc,Glce,Ptpn22,Bcl2l11,Heatr1,Mecr,Aldh1a1,Tnk2,St8sia6,Fabp1,Srsf6,Zfp612,Polr3g,Nrp1,Smc6,Maf,Adh1,Trpv1,Anapc16,Fancc,Baz2a,Chst4,Fbl |
| LN | G3 | cellular metabolic process | 0.06094 | Sorl1,Pabpn1,Rbm4,Kpna6,Cyp1b1,Hnrnpa1,Atg4c,Smurf2,Cops3,Ret,Pten,Chchd10,Gprc5b,Btg1,Prmt3,Tox2,Map3k6,Fktn,Pdcd4,Mrpl11,H3f3b,Rad1,Serpine2,Timp4,Ahcyl2,Errfi1,Apex1,Ddx23,Skp2,Tsc22d3,Trim37,Herc2,Klhl2,Rps17,Ndrg2,Gls,Rlim,Hamp,Birc3,Mcf2l,Nfkb2,Prcp,Ip6k2,Zbtb16,Thbs4,Ptgs1,Ddit4,Serpina3m,Serpina3n,Serpina3c,Zfp36l2,Mettl1,Ncor2,Thrb,Cdc7,Msc,Mbd3,Aebp2,Per3,Per2,Trmt1,Bbc3,Scd2,Foxred2,Ttll4,Ttll7,Pdia6,Pdia4,Acp5,Upf3b,Tbl1xr1,Dis3l2,Fmo2,Tcf4,Rspo1,Spred2,Dusp4,Dusp6,Dusp1,Dapk2,Otud3,Kit,Fads1,Rad51d,Mthfd1,Acer2,Alg8,Hoxd8,Bid,Wdr12,Pank1,Nrip1,Large,Lyst,Swsap1,Pdk4,Pik3ip1,Mafb,Vars2,Pecr,Sae1,Plce1,Nfe2l2,Tcf7l1,Tcf7l2,Zmiz1,Fkbp5,Cdk4,Mvd,Stc2,Adam9,Acadm,Pik3cg,Crp,Psmd11,Pdgfra,Vars,Zfp322a,Wipi1,Secisbp2,Ptpn3,Dab2,Agtr1a,Zfp932,Il27ra,Med24,Rps3,Brd7,Uchl1,Suv420h1,Dhx29,Vmp1,Tbrg4,Cd36,Pinx1,Abcg2,Ccdc22,Pkig,Rps6ka6,Zfp52,Zfp53,Dpep1,Angpt4,Rprd1a,Bok,Galc,Hpgd,Deptor,Adk,Lrpprc,Comt,Glce,Ptpn22,Bcl2l11,Heatr1,Hagh,Mecr,Aldh1a1,Tnk2,St8sia6,Mmachc,Fabp1,Srsf6,Zfp612,Polr3g,Nrp1,Smc6,Maf,Adh1,Anapc16,Atp1b1,Fancc,Baz2a,Chst4,Fbl |
| LN | G3 | lipid metabolic process | 0.07061 | Cyp1b1,Pten,Lss,Ip6k2,Ptgs1,Per2,Scd2,Tbl1xr1,Kit,Fads1,Acer2,Alg8,Lyst,Pdk4,Pik3ip1,Pecr,Tcf7l2,Mvd,Acadm,Pik3cg,Pdgfra,Agtr1a,Galc,Hpgd,Mecr,Aldh1a1,St8sia6,Adh1,Trpv1 |
| LN | G3 | cellular protein modification process | 0.09189 | Sorl1,Rbm4,Atg4c,Smurf2,Cops3,Ret,Pten,Gprc5b,Btg1,Prmt3,Map3k6,Fktn,Pdcd4,Errfi1,Skp2,Trim37,Herc2,Klhl2,Ndrg2,Rlim,Birc3,Thbs4,Ddit4,Ncor2,Cdc7,Mbd3,Per2,Ttll4,Ttll7,Tbl1xr1,Rspo1,Spred2,Dusp4,Dusp6,Dusp1,Dapk2,Otud3,Kit,Acer2,Alg8,Large,Pdk4,Sae1,Plce1,Nfe2l2,Fkbp5,Cdk4,Adam9,Pik3cg,Pdgfra,Wipi1,Ptpn3,Dab2,Brd7,Uchl1,Suv420h1,Cd36,Ccdc22,Pkig,Rps6ka6,Angpt4,Rprd1a,Deptor,Ptpn22,Tnk2,St8sia6,Nrp1,Anapc16,Baz2a,Chst4,Fbl |

[Table 18-17]

| Organ | group | Term | Pvalue | Genelist |
|---|---|---|---|---|
| LN | G3 | cellular protein metabolic process | 0.19382 | Sorl1,Rbm4,Atg4c,Smurf2,Cops3,Ret,Pten,Gprc5b,Btg1,Prmt3,Map3k6,Fktn,Pdcd4,Mrpl11,Serpine2,Timp4,Errfi1,Skp2,Trim37,Herc2,Klhl2,Rps17,Ndrg2,Rlim,Hamp,Birc3,Thbs4,Ddit4,Serpina3m,Serpina3n,Serpina3c,Ncor2,Cdc7,Mbd3,Per2,Bbc3,Foxred2,Ttll4,Ttll7,Pdia6,Pdia4,Upf3b,Tbl1xr1,Rspo1,Spred2,Dusp4,Dusp6,Dusp1,Dapk2,Otud3,Kit,Acer2,Alg8,Bid,Large,Pdk4,Vars2,Sae1,Plce1,Nfe2l2,Fkbp5,Cdk4,Adam9,Pik3cg,Psmd11,Pdgfra,Vars,Wipi1,Secisbp2,Ptpn3,Dab2,Agtr1a,Brd7,Uchl1,Suv420h1,Cd36,Ccdc22,Pkig,Rps6ka6,Dpep1,Angpt4,Rprd1a,Bok,Deptor,Lrpprc,Ptpn22,Bcl2l11,Tnk2,St8sia6,Fabp1,Nrp1,Anapc16,Baz2a,Chst4,Fbl |
| LN | G3 | cell proliferation | 0.20172 | Kcnh1,Cyp1b1,Pten,Btg1,Fktn,Serpine2,Glul,Ndrg2,Trnp1,Zbtb16,Thbs4,Ptgs1,Ddit4,Ncor2,Cdc7,Per2,Hyal1,Dis3l2,Rspo1,Kit,Vpreb1,Acer2,Bid,Wdr12,Mafg,Btla,Tcf7l2,Zmiz1,Cdk4,Mvd,Cr2,Pdgfra,Agtr1a,Brd7,Uchl1,Cxcr4,Cxcl13,Vcam1,Ptpn22,Tnk2,St8sia1,Il7r,Srsf6,Polr3g,Plxnb1 |
| LV | G4 | response to stress | 4.1E-13 | Rad1,Selk,Atg4c,Ret,Pten,Gprc5b,Map3k6,Fktn,Pdcd4,Rad1,Serpine2,Glul,Errfi1,Apex1,Tsc22d3,Herc2,Hamp,Nfkb2,Prcp,Scamp5,Thbs4,Ddit4,Cdc7,Per2,Bbc3,Hyal1,Pdia6,Pdia4,Acp5,Upf3b,Spred2,Kit,Fads1,Rad51d,Bid,Large,Lyst,Swsap1,Seh1l,Pdk4,Cd160,Mrc1,Ryr1,Nfe2l2,Tcf7l2,Stc2,Adam9,Acadm,Pik3cg,Pdgfra,Wipi1,Dab2,Agtr1a,Il27ra,Rps3,Uchl1,Clec10a,Cd36,Cxcl13,Rps6ka6,Dpep1,Bok,Comt,Bcl2l11,Tnk2,St8sia1,Fabp1,Srsf6,Polr3g,Scara5,Smc6,Ndufs8,Trpv1,Atp1b1,Fancc |
| LN | G3 | homeostatic process | 0.27783 | Gprc5b,Plcg1,Apex1,Tsc22d3,Rps17,Hamp,Prcp,Ncor2,Bbc3,Acp5,Kit,Rad51d,Vpreb1,Mthfd1,Large,Lyst,Pdk4,Mafb,Ryr1,Nfe2l2,Tcf7l2,Stc2,Pdgfra,Ptpn3,Agtr1a,Pinx1,Cxcl13,Ccdc22,Vcam1,Bcl2l11,Il7r,Scara5,Smc6,Trpv1,Atp1b1,Fancc |
| LN | G3 | macromolecule metabolic process | 0.42101 | Sorl1,Pabpn1,Rbm4,Kpna6,Cyp1b1,Hnrnpa1,Atg4c,Smurf2,Cops3,Ret,Pten,Gprc5b,Btg1,Prmt3,Tox2,Map3k6,Fktn,Pdcd4,Mrpl11,H3f3b,Rad1,Serpine2,Timp4,Errfi1,Apex1,Ddx23,Skp2,Tsc22d3,Trim37,Herc2,Klhl2,Rps17,Ndrg2,Rlim,Hamp,Birc3,Mcf2l,Nfkb2,Prcp,Zbtb16,Thbs4,Ddit4,Serpina3m,Serpina3n,Serpina3c,Zfp36l2,Mettl1,Ncor2,Thrb,Cdc7,Msc,Mbd3,Aebp2,Per3,Per2,Trmt1,Bbc3,Hyal1,Foxred2,Ttll4,Ttll7,Pdia6,Pdia4,Upf3b,Tbl1xr1,Dis3l2,Tcf4,Rspo1,Spred2,Dusp4,Dusp6,Dusp1,Dapk2,Otud3,Kit,Rad51d,Acer2,Alg8,Hoxd8,Bid,Wdr12,Nrip1,Large,Swsap1,Pdk4,Mafb,Mafg,Vars2,Sae1,Plce1,Nfe2l2,Tcf7l1,Tcf7l2,Zmiz1,Fkbp5,Cdk4,Stc2,Adam9,Acadm,Fcer2a,Pik3cg,Crp,Psmd11,Pdgfra,Vars,Zfp322a,Wipi1,Secisbp2,Ptpn3,Dab2,Agtr1a,Zfp932,Il27ra,Med24,Rps3,Brd7,Uchl1,Suv420h1,Dhx29,Cd36,Pinx1,Ccdc22,Pkig,Rps6ka6,Zfp52,Zfp53,Dpep1,Angpt4,Rprd1a,Bok,Deptor,Dync2h1,Lrpprc,Glce,Ptpn22,Bcl2l11,Heatr1,Tnk2,St8sia6,Fabp1,Il7r,Srsf6,Zfp612,Polr3g,Nrp1,Scara5,Smc6,Maf,Anapc16,Atp1b1,Fancc,Baz2a,Chst4,Fbl |
| LN | G3 | cell motility | 0.42249 | Sorl1,Cyp1b1,Smurf2,Ret,Pten,Fktn,Plcg1,Tns1,Tmem201,Prcp,Thbs4,Ddit4,Hyal1,Kit,Lyst,Strip2,Adam9,Pik3cg,Pdgfra,Dab2,Shroom2,Elp6,Agtr1a,Cxcr4,Cxcl13,Dpep1,Angpt4,Sema6d,Vcam1,Tnk2,Nrp1,Chst4 |
| LN | G3 | pigmentation | 0.54296 | Myo7a,Kit,Lyst,Kif13a,Shroom2,Bcl2l11 |
| LN | G3 | cellular macromolecule metabolic process | 0.56618 | Sorl1,Pabpn1,Rbm4,Kpna6,Cyp1b1,Hnrnpa1,Atg4c,Smurf2,Cops3,Ret,Pten,Gprc5b,Btg1,Prmt3,Tox2,Map3k6,Fktn,Pdcd4,Mrpl11,H3f3b,Rad1,Serpine2,Timp4,Errfi1,Apex1,Ddx23,Skp2,Tsc22d3,Trim37,Herc2,Klhl2,Rps17,Ndrg2,Rlim,Hamp,Birc3,Mcf2l,Nfkb2,Zbtb16,Thbs4,Ddit4,Serpina3m,Serpina3n,Serpina3c,Zfp36l2,Mettl1,Ncor2,Thrb,Cdc7,Msc,Mbd3,Aebp2,Per3,Per2,Trmt1,Bbc3,Foxred2,Ttll4,Ttll7,Pdia6,Pdia4,Upf3b,Tbl1xr1,Dis3l2,Tcf4,Rspo1,Spred2,Dusp4,Dusp6,Dusp1,Dapk2,Otud3,Kit,Rad51d,Acer2,Alg8,Hoxd8,Bid,Wdr12,Nrip1,Large,Swsap1,Pdk4,Mafb,Vars2,Sae1,Plce1,Nfe2l2,Tcf7l1,Tcf7l2,Zmiz1,Fkbp5,Cdk4,Adam9,Acadm,Pik3cg,Psmd11,Pdgfra,Vars,Zfp322a,Wipi1,Secisbp2,Ptpn3,Dab2,Agtr1a,Zfp932,Il27ra,Med24,Rps3,Brd7,Uchl1,Suv420h1,Dhx29,Cd36,Pinx1,Ccdc22,Pkig,Rps6ka6,Zfp52,Zfp53,Dpep1,Angpt4,Rprd1a,Bok,Deptor,Lrpprc,Glce,Ptpn22,Bcl2l11,Heatr1,Tnk2,St8sia6,Fabp1,Srsf6,Zfp612,Polr3g,Nrp1,Smc6,Maf,Anapc16,Fancc,Baz2a,Chst4,Fbl |
| LN | G3 | single-organism metabolic process | 0.72953 | Sorl1,Rbm4,Cyp1b1,Atg4c,Ret,Pten,Chchd10,Prmt3,Map3k6,Lss,Fktn,Pdcd4,Rad1,Ahcyl2,Apex1,Trim37,Herc2,Ndrg2,Gls,Prcp,Ip6k2,Ptgs1,Ddit4,Ncor2,Cdc7,Mbd3,Per2,Scd2,Hyal1,Foxred2,Tbl1xr1,Fmo2,Rspo1,Spred2,Dusp6,Dusp1,Kit,Fads1,Rad51d,Mthfd1,Acer2,Alg8,Alg3,Pank1,Large,Lyst,Swsap1,Pdk4,Pik3ip1,Vars2,Pecr,Plce1,Nfe2l2,Tcf7l2,Mvd,Adam9,Acadm,Pik3cg,Pdgfra,Vars,Wipi1,Dab2,Agtr1a,Il27ra,Rps3,Brd7,Uchl1,Suv420h1,Tbrg4,Cd36,Pinx1,Abcg2,Npy1r,Rps6ka6,Dpep1,Galc,Hpgd,Adk,Lrpprc,Vcam1,Glce,Mecr,Aldh1a1,St8sia6,Mmachc,Fabp1,Nrp1,Smc6,Adh1,Trpv1,Atp1b1,Fancc,Baz2a,Fbl |
| LN | G3 | embryo development | 0.73052 | Tmie,Cops3,Ret,Stox2,Plcg1,Myo7a,Zbtb16,Ncor2,Mbd3,Hyal1,Dusp4,Dusp6,Dusp1,Kit,Mthfd1,Mafb,Mafg,Nfe2l2,Tcf7l1,Tcf7l2,Zmiz1,Pdgfra,Dab2,Hoxc4,Rps6ka6,Epn2,Dync2h1,Vcam1,Bcl2l11,Aldh1a1,Nrp1 |
| LN | G3 | cellular lipid metabolic process | 0.83984 | Cyp1b1,Pten,Ip6k2,Ptgs1,Per2,Scd2,Tbl1xr1,Kit,Fads1,Acer2,Alg8,Lyst,Pdk4,Pik3ip1,Pecr,Tcf7l2,Mvd,Acadm,Pik3cg,Pdgfra,Agtr1a,Galc,Hpgd,Mecr,Aldh1a1,St8sia6,Adh1 |
| LN | G3 | protein maturation | 0.88005 | Sorl1,Atg4c,Ret,Serpine2,Birc3,Bbc3,Acer2,Bid,Bok,Dync2h1,Bcl2l11 |
| LN | G3 | Ubiquitin mediated proteolysis\|Cell cycle | 0.89005 | Smurf2,Pten,Skp2,Sesn1,Trim37,Herc2,Birc3,Cdc7,Bbc3,Zmat3,Bid,Sae1,Cdk4 |
| LN | G3 | cell adhesion | 0.94449 | Cyp1b1,Ret,Pten,Serpine2,Zbtb16,Thbs4,Zfp36l2,Hsd17b12,Hyal1,Pdia6,Kit,Acer2,Mafb,Btla,Adam9,Pik3cg,Pdgfra,Dab2,Vmp1,Cd36,Cxcr4,Cxcl13,Vcam1,Ptpn22,Bcl2l11,Il7r,Plxnb1,Dtx1,Chst4 |
| LN | G3 | sulfur compound metabolic process | 0.95768 | Ahcyl2,Mthfd1,Pdk4,Nfe2l2,Tcf7l2,Dpep1,Glce,Hagh,Chst4 |
| LN | G5 | cofactor metabolic process | 0.01335 | Cyp4f18,Prkag2,Adck3,Nudt7,Fech,Ppcdc,Entpd5,Ogt,Pfkfb2 |
| LN | G5 | Integrin alphaIIb beta3 signaling | 0.12096 | Rasgrp2,Rasgrp1,Syk,Rapgef4 |
| LN | G5 | cellular protein modification process | 0.18764 | Prkag2,Ssh1,Flcn,Agbl3,Rnf180,Caprin2,Zdhhc20,Sox4,Eogt,Zdhhc9,Cdc25b,Fgf14,Camk2a,Srpk2,Map3k9,Tinf2,Rprd1a,Rasgrp1,Ttn,Stox1,Fech,Entpd5,Ttll5,Nek1,Ogt,Ctdsp2,Nlrc3,Clcf1,Ick,Ttc3,Insm1,Stk38,Map4k2,Ubash3b,Syk,Tnik,Cdon,Man2b1 |
| LN | G5 | phosphate-containing compound metabolic process | 0.26397 | Prkag2,Ssh1,Flcn,Gucy1a3,Caprin2,Adck3,Cdc25b,Nudt7,Fgf14,Camk2a,Adcy7,Srpk2,Map3k9,Rprd1a,Rasgrp1,Ttn,Stox1,Fech,Ppcdc,Entpd5,Nek1,Ogt,Ctdsp2,Pik3r2,Sytl2,Pfkfb2,Clcf1,Ick,Ak3,Insm1,Stk38,Map4k2,Ubash3b,Syk,Tnik,Akap5,Rbl2,Cdon |
| LN | G5 | nucleobase-containing compound catabolic process | 0.31009 | Prkag2,Pan3,Nudt7,Entpd5,Ogt,Ago4,Upf3b |
| LN | G5 | Rap1 signalling | 0.41749 | Rasgrp2,Rasgrp1,Rapgef4 |

[Table 18-18]

| Organ | group | Term | Pvalue | Genelist |
|---|---|---|---|---|
| LN | G5 | Innate Immune System\|Adaptive Immune System\|Hemostasis\|Pathways in cancer\|Developmental Biology\|PI3K-Akt signaling pathway\|Signaling by Rho GTPases | 0.54744 | Plekhg2,Prkag2,Cers4,Ssh1,Flcn,Cldn11,Gucy1a3,Rgs3,Il13ra2,Amph,Thbs2,Agtr1a,Cd28,Cdc25b,Ciita,Fgf14,Camk2a,Adcy7,Scai,S100a10,H2-Ob,Ppp1r3c,Rasgrp2,Rasgrp1,Sike1,Coro1a,Sh3kbp1,Itga8,E2f2,Pik3r2,Pfkfb2,Habp4,Clcf1,Cacna1b,Ak3,Insm1,Map4k2,Syk,Arhgap9,Arhgap4,Atg16l2,Depdc7,Rbl2,Cdon,Atp6v0e2,Rapgef4,Pde10a |
| LN | G5 | generation of precursor metabolites and energy | 0.85945 | Prkag2,Flcn,Ppp1r3c,Entpd5,Ogt,Pfkfb2 |
| LN | G5 | purine ribonucleotide metabolic process | 0.86597 | Prkag2,Flcn,Gucy1a3,Nudt7,Adcy7,Ppcdc,Entpd5,Ogt,Pfkfb2,Ak3,Akap5 |
| LN | G5 | cellular component assembly | 0.90128 | Ssh1,Flcn,Anxa6,Pan3,Cep164,S100a10,Srpk2,Clec2i,Tinf2,Rasgrp1,Ttn,Fech,Hist1h1c,Haus8,Coro1a,Ttll5,Nek1,Rfx3,Ago4,Pik3r2,Ick,Insm1,Tnik,Atg16l2,Cryz |
| LN | G6 | immune system process | 0.00681 | Lyl1,Klf2,Rgcc,C5ar1,Gprc5b,H2-DMb2,Syk,Hhex,Hif1a,Clec4e,Sox4,H2-Ab1,Fgr,Kcnab2,Adora2b,Clec7a,Sirpa,Calr,Dusp3,Lat2,Cd79b,Matk,Tfeb,Inpp4b,Trim59,Meis2,Lgals3,Itgax,Nfe2l2,Hist2h2be,Fcer2a,Itga9,Pla2g2d,Ednrb,Itgb1,Hand2,Cxcl12,Gbf1,Slc40a1,Fam20c,Ceacam1,Rab4a,Egr3,Vegfc,5430435G22Rik,Cd300lb,H2-DMa,Itpkb,Stap1,Myo1f,Mtss1,Jak3 |
| LN | G6 | locomotion | 0.03084 | Rgcc,Hexb,C5ar1,Pfn2,Syk,Hif1a,Mmp12,Top2b,Flrt3,Fgr,Pdgfa,Sh3kbp1,Calr,Ptp4a3,Hyal1,Matk,Trim59,Npc1,Lgals3,Itga9,Ntrk3,Ednrb,Spns3,Itgb1,Hand2,Cxcl12,Zfand5,Gbf1,Ceacam1,Scai,Egr2,Egr3,Vegfc,Vcan,Stap1,Myo1f,Myo18a,Jak3 |
| LN | G6 | transport | 0.04636 | Rbm4,Rgcc,Apoc2,Cltb,Pfn2,Slc36a4,Syk,Stim1,Hhex,Hif1a,Agpat6,Slc39a6,Slc35f6,Soat1,Clec4e,Atp6v1c1,Sox4,Arf2,Eif2ak3,Fgr,Cacna2d4,Abca13,Kcnab2,Scn4a,Adora2b,Itpr3,Clec7a,Sirpa,Calr,Gnpda1,Slc16a5,Arhgap17,Kif1c,Inpp4b,Slc30a1,Slc16a12,Npc1,Lgals3,Msr1,Itgax,Ibtk,Fabp5,Nfe2l2,Pea15a,Tpcn1,Slc35d2,Trim16,Kcnj10,Scrn3,Rab30,Pdzd11,Clec4b1,Sec22c,Ptpn14,Zfand2b,Sgk1,Plin2,Cenpf,Ccdc93,Txndc5,Gbf1,Fam21,Slc40a1,Ceacam1,Fam89b,Rab4a,Egr2,5430435G22Rik,H2-DMa,Exoc8,Stap1,Xpo7,Myo1f,Rapgef4,Myo18a,Jak3 |
| LN | G6 | cell motility | 0.09897 | Rgcc,Hexb,C5ar1,Pfn2,Syk,Hif1a,Mmp12,Top2b,Fgr,Pdgfa,Sh3kbp1,Calr,Ptp4a3,Hyal1,Matk,Lgals3,Itga9,Ntrk3,Ednrb,Itgb1,Hand2,Cxcl12,Zfand5,Gbf1,Scai,Egr3,Vegfc,Vcan,Stap1,Myo1f,Myo18a,Jak3 |
| LN | G6 | Phagosome | 0.10643 | H2-DMb2,Atp6v1c1,Atp6v0d2,H2-Ab1,Atp6v1a,Clec7a,Calr,Msr1,Itgb1,5430435G22Rik,H2-DMa,Tubb4b |
| LN | G6 | vesicle-mediated transport | 0.13082 | Apoc2,Cltb,Pfn2,Syk,Fgr,Adora2b,Clec7a,Sirpa,Calr,Gnpda1,Arhgap17,Kif1c,Npc1,Lgals3,Scrn3,Pdzd11,Sec22c,Ccdc93,Txndc5,Gbf1,Fam21,Rab4a,5430435G22Rik,Exoc8,Stap1,Myo1f,Rapgef4,Myo18a |
| LN | G6 | Protein processing in endoplasmic reticulum | 0.30308 | Ssr1,Eif2ak3,Calr,Nfe2l2,Dnajb12,Preb,Rrbp1,Ckap4,Txndc5,Hyou1 |
| LN | G6 | Lysosome | 0.30308 | Cers4,Hexb,Cltb,Ppap2a,Atp6v0d2,Pla2g15,Hyal1,Npc1,Ctsb,Cd68 |
| LN | G6 | Cytokine-cytokine receptor interaction\|Endocytosis\|Herpes simplex infection | 0.31884 | C5ar1,Cltb,Fgfr3,H2-DMb2,Skp2,Atp6v1c1,Asap1,Atp6v0d2,H2-Ab1,Atp6v1a,Eif2ak3,Pdgfa,Sh3kbp1,Clec7a,Calr,Ccr6,Pip5k1b,Msr1,Fcer2a,Itga9,Itgb1,Ctsb,Mpzl1,Cxcl12,Gbf1,Rab4a,Vegfc,5430435G22Rik,H2-DMa,Tubb4b,Vcan,Jak3 |
| LN | G6 | Steroid biosynthesis | 0.32098 | Dhcr24,Lss,Soat1 |
| LN | G6 | localization | 0.33692 | Rbm4,Rgcc,Apoc2,Dhcr24,Hexb,C5ar1,Cltb,Pfn2,Slc36a4,Syk,Stim1,Hhex,Hif1a,Agpat6,Slc39a6,Slc35f6,Soat1,Clec4e,Mmp12,Top2b,Sox4,Arf2,Eif2ak3,Gpr158,Fgr,Cacna2d4,Abca13,Kcnab2,Scn4a,Pdgfa,Adora2b,Itpr3,Sh3kbp1,Clec7a,Sirpa,Calr,Gnpda1,Slc16a5,Arhgap17,Ptp4a3,Hyal1,Kif1c,Matk,Inpp4b,Slc30a1,Bub3,Slc16a12,Npc1,Lgals3,Msr1,Itgax,Ibtk,Fabp5,Nfe2l2,Pea15a,Tmem106b,Tpcn1,Slc35d2,Trim16,Itga9,Ntrk3,Kcnj10,Scrn3,Rab30,Pdzd11,Ednrb,Clec4b1,Stk11ip,Sec22c,Ptpn14,Zfand2b,Itgb1,Sgk1,Msx1,Plin2,Cenpf,Hand2,Cxcl12,Ccdc93,Zfand5,Txndc5,Gbf1,Fam21,Slc40a1,Ceacam1,Fam89b,Scai,Rab4a,Egr2,Egr3,Smyd3,Vegfc,5430435G22Rik,Tspan10,H2-DMa,Exoc8,Vcan,Stap1,Xpo7,Myo1f,Rapgef4,Myo18a,Jak3 |
| LN | G6 | Antigen activates B Cell Receptor (BCR) leading to generation of second messengers | 0.42632 | Syk,Stim1,Itpr3,Sh3kbp1,Cd79b |
| LN | G6 | lipid metabolic process | 0.52612 | Cers4,Apoc2,Dhcr24,Hexb,Fgfr3,Ppap2a,Syk,Lss,Agpat6,Soat1,Mgll,Stard4,Eif2ak3,Fgr,Pla2g15,Pdgfa,Tbl1xr1,Pip5k1b,Inpp4b,Npc1,Fabp5,Hpgds,Elovl7,Rbl2,Decr2 |
| LN | G6 | regulation of developmental process | 0.66389 | Rbm4,Rgcc,Hexb,C5ar1,Gprc5b,Fgfr3,Syk,Hhex,Runx1t1,Hif1a,Agpat6,Timp2,Vat1,Mgll,Asap1,Flrt3,Sox4,Setx,Ttbk1,Sostdc1,Eif2ak3,Fgr,Pdgfa,Ldb2,Sh3kbp1,Calr,Metrnl,Hyal1,Inpp4b,Meis2,Msr1,Mapk11,Nfe2l2,Trim16,Ntrk3,Ednrb,Brwd1,Frzb,Itgb1,Msx1,Cenpf,Arhgef15,Hand2,Cxcl12,Foxp2,Fam20c,Egr3,Vegfc,5430435G22Rik,H2-DMa,Zfp157,Ankrd6,Itpkb,Odf2,Stap1,Zfpm2,Jak3 |
| LN | G6 | anatomical structure development | 0.79035 | Rbm4,Lyl1,Klf2,Hes6,Endog,Rgcc,Dhcr24,Hexb,C5ar1,Gprc5b,Fgfr3,Syk,Stim1,Hhex,Runx1t1,Pmp22,Hif1a,Agpat6,Timp2,Vat1,Clec4e,Mmp12,Mgll,Dok4,Top2b,Asap1,Flrt3,Sox4,Setx,H2-Ab1,Ttbk1,Sostdc1,Eif2ak3,Fgr,Phf8,Kcnab2,Pdgfa,Ldb2,Sh3kbp1,Tbl1xr1,Sirpa,Calr,Hyal1,Cml1,Matk,Tfeb,Inpp4b,Slc30a1,Zfp191,Edaradd,Meis2,Lgals3,Mapk11,Mapk12,Srpk3,Nfe2l2,Tpra1,Tmem106b,Trim16,Aes,Ntrk3,Pla2g2d,Kcnj10,Ednrb,Brwd1,Frzb,Ptpn14,Itgb1,Ctsb,Msx1,Cenpf,Arhgef15,Hand2,Cxcl12,Zfand5,Foxp2,Slc40a1,Fam20c,Ceacam1,Egr2,Egr3,Smyd3,Vegfc,5430435G22Rik,H2-DMa,Zfp157,Ankrd6,Vcan,Itpkb,Odf2,Stap1,Zfpm2,Mtss1,Jak3 |
| LN | G6 | cell adhesion | 0.89686 | Rgcc,Syk,Pnn,Clec4e,Sox4,H2-Ab1,Clec7a,Sirpa,Calr,Dusp3,Hyal1,Cml1,Lgals3,Itgax,Itga9,Pla2g2d,Dsc2,Itgb1,Cxcl12,Gbf1,Ceacam1,Egr3,Vegfc,H2-DMa,Vcan,Itpkb,Myo1f,Jak3 |
| LN | G6 | Rheumatoid arthritis | 0.96573 | H2-DMb2,Atp6v1c1,Atp6v0d2,H2-Ab1,Atp6v1a,Cxcl12,H2-DMa |

[Table 19-1]

## UpStream_LN G1(8adj)

| Upstream Regulator | Molecule Type | p-value of overlap | Target molecules in dataset |
|---|---|---|---|
| IL6 | cytokine | 1.64E-02 | CEBPD,CP,GLRX,TLR9,TNFRSF1B |
| TNFSF11 | cytokine | 1.69E-02 | GLRX,HCK,TNFRSF1B |
| IFNG | cytokine | 1.78E-02 | CEBPD,CP,HCK,S1PR3,SLC15A3,TLR9,TNFRSF1B |
| IL2 | cytokine | 2.29E-02 | HCK,RAF1,SOS1,TNFRSF1B |

## UpStream_LN G1(5adj)

| Upstream Regulator | Molecule Type | p-value of overlap | Target molecules in dataset |
|---|---|---|---|
| IFNA2 | cytokine | 3.84E-24 | CORO2A,CXCL10,DPP4,HERC6,HLA-E,IFI16,IFI35,IFIH1,IFIT1,IFIT2,IFITM3,IRF9,LY6E,LYN,OAS1,PARP9,PHF11,PML,PTTG1,RBL1,SAMHD1,STAT1,TNFSF10,TREX1,TRIM21,UBA7,XAF1 |
| IFNB1 | cytokine | 4.46E-23 | CASP8,CXCL10,DAXX,DHX58,GBP4,GBP7,Gvin1 (includes others),IFI16,Ifi47,IFIH1,IFIT1,IFIT2,IRF9,Irgm1,NMI,NOD1,NT5C3A,OAS1,PML,SLFN13,STAT1,STAT2,Tgtp1/Tgtp2,TNFSF10,TRIM21,Trim30a/Trim30d,UBA7,XAF1 |
| IFNG | cytokine | 9.98E-21 | BLNK,CASP8,CERS6,CLIC4,CTSC,CTSS,CXCL10,CYBB,DPP4,DTX3L,FGL2,GBP4,GBP7,Gvin1 (includes others),HCK,HERC6,HLA-E,HLA-G,IFI16,IFI35,Ifi47,IFIH1,IFIT1,IFIT2,IFITM3,IRF9,Irgm1,LGALS9B,LY6E,LYN,MAX,Ms4a4b (includes others),NMI,OAS1,PARP9,PML,PSMB10,PSMB8,PSMB9,SAMHD1,Serpina3g (includes others),STAT1,STAT2,TAPBP,Tgtp1/Tgtp2,TNFSF10,TRAFD1,TRIM21,UBE2E1 |
| IFNL1 | cytokine | 3.20E-14 | CXCL10,HERC6,IFI35,IFIH1,IFIT1,IFIT2,IFITM3,IRF9,OAS1,PHF11,PML,PSMB9,STAT1 |
| IFNA1/IFNA13 | cytokine | 2.21E-11 | CXCL10,DHX58,IFIH1,IFIT1,IFIT2,OAS1,STAT1,STAT2,Tgtp1/Tgtp2,UBE2E1 |
| IL15 | cytokine | 1.79E-06 | CXCL10,DPP4,IFI35,IFIT1,Klrk1,LYN,PNP,PSMB10,PSMB8,PSMB9,RBBP8,SOS1,TNFSF10,TRIM5 |
| IL4 | cytokine | 5.49E-06 | BBX,CTSC,CXCL10,DPP4,GBP7,HCK,IFI16,Klrk1,Ms4a4b (includes others),PNP,PSMB10,PSMB8,Serpina3g (includes others),SLAMF7,SMG7,STAT1,STAT2,TAPBP,Tgtp1/Tgtp2,Trim30a/Trim30d |
| IL1RN | cytokine | 6.98E-06 | CTSS,HERC6,IFIH1,IRF9,OAS1,PML,STAT2,TNFSF10 |
| IFNK | cytokine | 3.38E-05 | CXCL10,IFIH1,OAS1,STAT1 |
| IFNA4 | cytokine | 4.03E-05 | CXCL10,IFIH1,IFIT1,IFIT2 |
| IL1B | cytokine | 5.73E-05 | CTSS,CXCL10,CYBB,DPP4,HELZ2,HLA-E,IFI16,Ifi47,IFIT1,LGALS9B,NMI,PSMB10,PSMB8,PSMB9,Slfn2,STAT1,Tgtp1/Tgtp2,TNFSF10,TRAFD1 |
| IL12B | cytokine | 7.88E-05 | CXCL10,IRF9,PIK3AP1,STAT1,STAT2 |
| TNFSF10 | cytokine | 7.99E-05 | CASP8,IFI16,IFIT1,IRF9,STAT1,TNFSF10 |
| TNF | cytokine | 1.53E-04 | CASP8,CD47,CLIC4,CTSC,CTSS,CXCL10,CYBB,DAXX,DPP4,HLA-E,IFI16,IFIH1,IFIT1,LGALS9B,LYN,OAS1,PDK3,PML,PSMB10,PSMB8,PSMB9,Slfn2,STAT1,TAPBP,Tgtp1/Tgtp2,TNFSF10,TRAFD1 |
| IFNA10 | cytokine | 1.83E-04 | CXCL10,IFIH1,IFIT1 |
| IFNA21 | cytokine | 1.83E-04 | CXCL10,IFIH1,IFIT1 |
| IFNA5 | cytokine | 1.83E-04 | CXCL10,IFIH1,IFIT1 |
| IFNA7 | cytokine | 1.83E-04 | CXCL10,IFIH1,IFIT1 |
| IFNA14 | cytokine | 1.83E-04 | CXCL10,IFIH1,IFIT1 |
| IFNA6 | cytokine | 1.83E-04 | CXCL10,IFIH1,IFIT1 |
| IFNE | cytokine | 2.07E-04 | CXCL10,IFIH1,IFIT2,STAT1 |
| IFNA8 | cytokine | 2.27E-04 | CXCL10,IFIH1,IFIT1 |
| IFNA16 | cytokine | 2.27E-04 | CXCL10,IFIH1,IFIT1 |
| CD40LG | cytokine | 3.50E-04 | CTSC,CXCL10,IFIT1,IFIT2,PML,PSMB10,PSMB8,PSMB9,SMG7,STAT1,TNFSF10 |
| IL6 | cytokine | 5.19E-04 | CTSC,CXCL10,CYBB,IFI16,IFIT1,IFIT2,IFITM3,IRF9,PSMB10,PSMB8,PSMB9,PTTG1,SH3GLB1,STAT1,TNFSF10 |
| IL2 | cytokine | 8.16E-04 | CTSC,CXCL10,DPP4,HCK,LY6E,NMI,PNP,PRKX,RAF1,RGPD4 (includes others),SOS1,TNFSF10 |
| IL12A | cytokine | 9.91E-04 | CXCL10,IRF9,STAT1,STAT2 |
| IL27 | cytokine | 3.67E-03 | CXCL10,HLA-E,STAT1,STAT2,TNFSF10 |
| OSM | cytokine | 3.83E-03 | CXCL10,GCA,GNS,IFI35,IRF9,MYCBP2,OAS1,PSMB8,PSMB9,STAT1,TAPBP |
| IFNA17 | cytokine | 5.66E-03 | CXCL10,IFIH1 |
| EBI3 | cytokine | 5.91E-03 | STAT1,STAT2,TNFSF10 |
| IL10 | cytokine | 8.02E-03 | CASP8,CTSS,CXCL10,HLA-G,IFIT2,PSMB9,STAT1,TNFSF10 |
| IFNW1 | cytokine | 9.32E-03 | CXCL10,IFIH1 |
| TNFSF11 | cytokine | 1.65E-02 | HCK,KEAP1,Slfn2,STAT1,VRK1,YWHAZ |
| IL7 | cytokine | 2.49E-02 | BLNK,DPP4,LYN,TNFSF10 |

## UpStream_LN G2

| Upstream Regulator | Molecule Type | p-value of overlap | Target molecules in dataset |
|---|---|---|---|
| IL33 | cytokine | 9.70E-03 | ABCA1,SCARB1,TFEC |
| TNF | cytokine | 1.18E-02 | ABCA1,CIB2,CSF2RB,CTSB,GAB1,GPAM,INSR,LBP,NOS3,PRSS23,PTGS1,PTPN12,RGS4,SCARB1,SCD,SLC12A6 |
| IL10 | cytokine | 1.32E-02 | CDKN2D,CEACAM1,CSF2RB,CTSB,NOS3,PIK3CG |
| IL6 | cytokine | 1.41E-02 | ABCA1,CDKN2D,CEACAM1,CIB2,CSF2RB,HFE,LBP,NOS3,SBNO2 |
| IL5 | cytokine | 1.58E-02 | CEACAM1,CSF2RB,EPS8,GPAM,KIAA1147 |
| OSM | cytokine | 2.63E-02 | ABCA1,GAB1,GLUL,GMPR,LBP,MGLL,RAP2A |
| CRH | cytokine | 4.52E-02 | NOS3,SCARB1 |

[Table 19-2]

### UpStream_LN G3

| Upstream Regulator | Molecule Type | p-value of overlap | Target molecules in dataset |
|---|---|---|---|
| TNF | cytokine | 6.56E-10 | ABCG2,ACADM,AGTR1,AK2,BBC3,BCL2L11,BID,BIRC3,BTG1,C10orf10,Ccl9,CCR6,CD163,CD209,CD36,CDK4,CHST4,CRP,CXCL13,CXCR4,CYP1B1,DUSP1,DUSP6,FABP1,FADS1,FBL,FCER2,GLS,GUSB,Hamp/Hamp2,HPGD,IFNGR2,IL7R,KIT,LSS,MSC,NCOR2,NFE2L2,NFKB2,NRIP1,NRP1,PCP4,PDGFRA,PDIA4,PER2,PEX11A,PPP5C,PTEN,PTGS1,RPS3,SERPINA3,SERPINE2,SMURF2,SND1,TIMP4,TSC22D3,VCAM1,VMP1 |
| IL10 | cytokine | 3.13E-06 | ARAP2,C10orf10,CCR6,CD163,CD209,CXCL13,CXCR4,DUSP1,FCER2,FKBP5,GPRC5B,HPGD,IL7R,ITGAE,MAF,MRC1,PIK3CG,TSC22D3,VCAM1 |
| IL4 | cytokine | 5.79E-06 | Acp5,ALG3,CCR6,CD163,CD209,CD36,CLEC10A,CR2,CXCR4,EVI5,FBL,FCER2,FKBP5,HPGD,IFNGR2,IL27RA,IL7R,ITGAE,KIT,KLHDC2,MAF,MAFB,MRC1,NFKB2,NRP1,PHB,PPP5C,Scd2,VCAM1,Zfp53 |
| IL2 | cytokine | 4.56E-05 | BBC3,BIRC3,CCR6,CD160,CDK4,CXCR4,DAPK2,DUSP4,DUSP6,IFNGR2,IL27RA,IL7R,IP6K2,ITGAE,KIT,MAF,PDCD4,PHB,PINX1,SESN1,SPRED2 |
| IFNG | cytokine | 7.84E-05 | AGTR1,ARAP2,ATP1B1,BBC3,BCL2L11,BID,BTG1,C10orf10,CCR6,CD163,CD209,CD36,CDK4,CLEC10A,CXCR4,CYB561,DUSP1,FAM107A,FCER2,FKBP5,GLS,GLUL,GPR64,GPRC5B,GUSB,IFNGR2,IL7R,LARGE,MRC1,NFKB2,PCDH17,PLCG1,PPP5C,TCF7L2,TIMP4,TSC22D3,VCAM1,ZBTB16 |
| IL6 | cytokine | 2.42E-04 | ABCG2,Acp5,BBC3,BCL2L11,CCR6,CD163,CD209,CD36,CRP,CXCL13,CXCR4,CYP1B1,DUSP1,DUSP6,FCER2,Hamp/Hamp2,HPGD,IL7R,KIT,LARGE,MAF,PHB,SERPINA3,VCAM1 |
| IL13 | cytokine | 3.23E-04 | Acp5,BID,CD163,CD209,CD36,CXCR4,FADS1,FADS2,FCER2,IFNGR2,MAF,MRC1,TIMP4,TNS1,TRPV1,VCAM1 |
| CSF1 | cytokine | 3.32E-04 | Acp5,BCL2L11,CD163,CD209,CDK4,DUSP1,MAFB,MRC1,NFKB2,VCAM1 |
| EPO | cytokine | 6.19E-04 | ABCA13,BCL2L11,BTG1,CD36,CXCR4,FABP1,GLUL,KIT,N4BP2L1,PDCD4,PIK3IP1,SKP2,TSC22D3 |
| IL1B | cytokine | 8.20E-04 | ABCG2,AGTR1,BIRC3,Ccl9,CCR6,CRP,CXCR4,DAB2,DDIT4,DUSP1,ERRFI1,FABP1,FCER2,FKBP5,GUSB,Hamp/Hamp2,NFE2L2,NFKB2,NRP1,PDGFRA,PTGS1,SERPINA3,SESN1,TIMP4,TSC22D3,VCAM1 |
| PRL | cytokine | 1.99E-03 | ABCG2,BOK,CDK4,MAFG,PDGFRA,PDIA4,PDK4,PKIG,SERPINA3,SND1 |
| IL3 | cytokine | 2.38E-03 | BCL2L11,BIRC3,CXCR4,FCER2,KLF9,NRP1,PTPN22,RPL3,RPS17,RPS20,RPS3,SERPINA3,VCAM1 |
| LTB | cytokine | 4.79E-03 | CHST4,CRP,CXCL13 |
| IL15 | cytokine | 5.19E-03 | BBC3,BCL2L11,BTG1,CD160,CDK4,CXCR4,IL7R,KIT,MAF,PDCD4,PDIA4,SORL1,VCAM1 |
| CCL5 | cytokine | 5.27E-03 | CD163,CYP1B1,DUSP1,DUSP6,NCOR2 |
| TNFSF13B | cytokine | 7.37E-03 | BCL2L11,CDK4,CR2,NFKB2 |
| CD40LG | cytokine | 8.69E-03 | ACY1,ALG3,BIRC3,BTG1,CXCL13,CXCR4,CYP1B1,DUSP1,DUSP4,FBL,IL7R,NFKB2,VCAM1 |
| IL17A | cytokine | 1.02E-02 | CD163,CRP,CXCL13,GUSB,MRC1,NRP1,TIMP4,VCAM1 |
| SPP1 | cytokine | 1.34E-02 | ABCG2,Acp5,BCL2L11,MECR,NDRG2,NDUFS8 |
| IL27 | cytokine | 1.44E-02 | C10orf10,CD163,CDK4,HPGD,MAF,MRC1 |
| CSF3 | cytokine | 1.50E-02 | AGTR1,BIRC3,CDK4,CXCR4,DUSP6,KIT,VCAM1 |
| TNFSF11 | cytokine | 1.74E-02 | Acp5,AEBP2,Ccl9,DAB2,DUSP1,MAFB,NFKB2,SIGMAR1,VCAM1 |
| IL1A | cytokine | 1.92E-02 | ALDH1A1,BIRC3,CCR6,Hamp/Hamp2,IFNGR2,KIT,SERPINA3,VCAM1 |
| IL7 | cytokine | 2.03E-02 | BCL2L11,BTLA,CXCR4,IL7R,MAF,SMURF2 |
| CSF2 | cytokine | 2.40E-02 | BBC3,BCL2L11,BID,BIRC3,CD209,CXCR4,DUSP6,ITGAE,MRC1,NFKB2,NRP1,PIK3CG,TCF4 |
| WNT5A | cytokine | 2.64E-02 | CXCR4,DAB2,KIT,PTEN,RET |
| LTA | cytokine | 3.20E-02 | CHST4,CRP,CXCL13 |
| OSM | cytokine | 3.79E-02 | CRP,CXCL13,CYP1B1,GLUL,Hamp/Hamp2,LARGE,MRC1,MSC,PDCD4,PKIG,PTEN,SERPINA3,ST8SIA1,VCAM1 |
| IL11 | cytokine | 4.42E-02 | CRP,SERPINA3,VCAM1 |
| NAMPT | cytokine | 4.65E-02 | ATXN10,NPY1R |

### UpStream_LN G5

| Upstream Regulator | Molecule Type | p-value of overlap | Target molecules in dataset |
|---|---|---|---|
| IL5 | cytokine | 1.67E-02 | AK3,ANXA6,CD55,CIITA,Crisp1/Crisp3,RASGRP2 |
| IK | cytokine | 3.06E-02 | CIITA |
| EBI3 | cytokine | 4.74E-02 | CIITA,HLA-DOB |

### UpStream_LN G6

| Upstream Regulator | Molecule Type | p-value of overlap | Target molecules in dataset |
|---|---|---|---|
| IL4 | cytokine | 6.27E-06 | AUH,BPGM,C5AR1,CAPG,CCR6,CLEC4C,CLEC7A,Cmah,DDAH1,EGR2,FCER2,GNA15,HLA-DMA,ITGAX,ITGB1,JAK3,KCNAB2,LGALS3,LILRB4,LYL1,MATK,MMP12,MSR1,PLIN2,PMP22,SIRPA,SYK,TIMP2,XPO7 |
| IFNG | cytokine | 2.84E-04 | ADORA2B,C5AR1,CCR6,CEACAM1,CLEC4E,CTSB,CXCL12,E2F2,EDNRB,EGR2,EGR3,FABP5,FCER2,GFM1,GPR158,GPR83,GPRC5B,HIF1A,HLA-DMA,HLA-DMB,HLA-DQB1,ITGB1,ITPKB,JAK3,KLF2,LAT2,LGALS3,MMP12,MSR1,PDGFA,PEA15,SLC40A1,SOAT1,SPI1,VEGFC |
| TNF | cytokine | 5.79E-04 | ADAMTS5,ADORA2B,APOC2,C5AR1,CALR,CCR6,CLEC4E,CTSB,CXCL12,DSC2,EDNRB,EGR2,ENTPD5,FABP5,FBXO32,FCER2,FRZB,HEXB,HIF1A,HPGDS,ITGB1,KLF2,LGALS12,LGALS3,LSS,MMP12,MSR1,MTHFD2L,NFE2L2,NME1,PDGFA,PLIN2,PLK2,SGK1,SLC16A5,SNN,SOAT1,SOX4,TIMP2,VEGFC |
| IL6 | cytokine | 7.27E-04 | ADAMTS5,BUB3,C5AR1,CCR6,CD68,CEACAM1,CLEC4E,E2F2,EGR2,FCER2,HIF1A,HLA-DMA,ITGB1,MMP12,MSR1,PDGFA,PLA2G2D,POLD1,PTP4A3,SGK1,SLC40A1,SPI1 |
| IL1A | cytokine | 1.40E-03 | ADAMTS5,ADORA2B,CCR6,CXCL12,FAM89B,ITGB1,MMP12,PDGFA,RBL2,VEGFC |
| IL13 | cytokine | 1.69E-03 | ADORA2B,CHN2,CLEC4E,CLEC7A,CTSB,DHCR24,FCER2,LILRB3,MATK,MMP12,MTSS1,PDGFA,PLA2G2D,TIMP2 |
| IL5 | cytokine | 2.22E-03 | CEACAM1,CKAP4,EGR2,EGR3,ENDOG,FAM65B,HIF1A,KCNAB2,PMP22,RASGRP2,SGK1 |
| IL10 | cytokine | 2.49E-03 | CCR6,CD68,CEACAM1,CLEC7A,CTSB,CXCL12,FCER2,GPRC5B,LILRB4,MSR1,SLC9A3R2,TUBB4B,VCAN |
| CSF2 | cytokine | 3.01E-03 | ADORA2B,C5AR1,CEACAM1,CLEC4C,CLEC7A,EGR2,EGR3,FAM65B,HLA-DQB1,ITGAX,PLA2G15,PMP22,POLD1,SGK1,SPI1 |
| TNFSF11 | cytokine | 4.48E-03 | ADORA2B,AKAP13,ATP6V0D2,CLEC4E,EGR2,FGR,ITPKB,LDB2,PDGFA,POR |
| IL33 | cytokine | 6.77E-03 | ADORA2B,CLEC4E,EGR2,MSR1,PLIN2 |
| CCL11 | cytokine | 7.69E-03 | CXCL12,ITGB1,PDGFA,VEGFC |
| TNFSF12 | cytokine | 9.16E-03 | CD68,CLEC4E,FBXO32,HES6,MMP12 |
| IL3 | cytokine | 1.17E-02 | CALR,CLEC4C,EGR2,EGR3,EMP3,FCER2,ITGAX,LILRB4,SOX4,SPI1,ZFAND5 |
| CCL5 | cytokine | 2.21E-02 | C5AR1,DDAH1,SGK1,TUBB4B |

[Table 19-3]

UpStream_LN G6 (cont.)

| | | | |
|---|---|---|---|
| IL17A | cytokine | 2.39E-02 | CD68,CXCL12,HEXB,KLF2,KLF3,TIMP2,VEGFC |
| EDN1 | cytokine | 2.71E-02 | EDNRB,HAND2,HIF1A,ITGB1,PDGFA,VCAN,VEGFC |
| CSF3 | cytokine | 3.69E-02 | CAPG,CXCL12,EGR2,EGR3,JAK3,SPI1 |
| CSF1 | cytokine | 4.08E-02 | CD68,EGR3,ITGB1,MMP12,MSR1,SPI1 |
| CX3CL1 | cytokine | 4.63E-02 | HIF1A,MSR1 |
| IL1B | cytokine | 4.87E-02 | ADAMTS5,APOC2,CCR6,CTSB,CXCL12,E2F2,FABP5,FCER2,FGFR3,HEXB,HIF1A,HPGDS,ITGB1,ITPKB,MMP12,NFE2L2,TIMP2,VCAN,VEGFC |

UpStream_SP G1

| Upstream Regulator | Molecule Type | p-value of overlap | Target molecules in dataset |
|---|---|---|---|
| IFNA2 | cytokine | 2.68E-09 | IFI16,OAS1,OAS2,OGFR,SAMHD1,UBA7,UBE2L6 |
| IFNG | cytokine | 3.71E-06 | AGRN,FCGR1A,IFI16,OAS1,OAS2,RNF31,SAMHD1,SLFN12L,UBE2L6 |
| IFNB1 | cytokine | 8.79E-06 | IFI16,Ifi27l2a/Ifi27l2b,OAS1,OAS2,UBA7 |
| IFNA1/IFNA13 | cytokine | 2.91E-05 | OAS1,OAS2,UBE2L6 |
| IFNL1 | cytokine | 7.08E-05 | OAS1,OAS2,UBE2L6 |
| IL1RN | cytokine | 7.67E-03 | OAS1,OAS2 |
| IFNK | cytokine | 2.48E-02 | OAS1 |

UpStream_SP G2

| Upstream Regulator | Molecule Type | p-value of overlap | Target molecules in dataset |
|---|---|---|---|
| IL13 | cytokine | 1.37E-09 | ALOX5AP,ARG2,CASP6,Ccl6,CTSB,CXCR2,DMXL2,FCGR2A,FLOT1,IL13RA1,MAF,MRC1 |
| IL10 | cytokine | 1.02E-07 | ALOX5AP,ARG2,Ccl6,CCR1,CTSB,CXCL12,FCGR2A,MAF,MERTK,MRC1 |
| IFNG | cytokine | 1.43E-07 | ALOX5AP,ARG2,CASP6,Ccl6,CCR1,CTSB,CXCL12,FCGR2A,GGT5,GLUL,HLA-A,IGF1R,MERTK,MRC1,PLA2G7,RAB27A,TGFBR3 |
| IL4 | cytokine | 5.22E-07 | ALOX5AP,ARG2,CASP6,Ccl6,CTNS,FCGR2A,IGF1R,IL13RA1,MAF,MAFB,MERTK,MRC1,TGFBI |
| CSF1 | cytokine | 1.60E-04 | FCGR2A,IGF1R,MAFB,MERTK,MRC1 |
| TNF | cytokine | 7.42E-04 | ADAMTS5,ALOX5AP,CASP6,Ccl6,CCR1,CTSB,CXCL12,CXCR2,HLA-A,IGF1R,NLRP12,PDGFRA,SLC43A2 |
| OSM | cytokine | 1.06E-03 | ADAMTS5,CXCL12,GLUL,HLA-A,IL13RA1,MRC1,PYGL |
| CXCL10 | cytokine | 2.64E-03 | Ccl6,CXCR2 |
| IL3 | cytokine | 3.40E-03 | ALOX5AP,Ccl6,FCGR2A,HLA-A,PLA2G7 |
| IL15 | cytokine | 5.02E-03 | CCR1,CXCL12,GGT5,MAF,PYGL |
| IL27 | cytokine | 6.66E-03 | HLA-A,MAF,MRC1 |
| IL1B | cytokine | 1.37E-02 | ACPP,ADAMTS5,CCR1,CTSB,CXCL12,HLA-A,PDGFRA |
| IL6 | cytokine | 1.81E-02 | ADAMTS5,CCR1,HLA-A,MAF,MERTK,RAB27A |
| IL17A | cytokine | 2.00E-02 | CXCL12,MERTK,MRC1 |
| IL33 | cytokine | 2.44E-02 | CXCR2,LEPR |
| PRL | cytokine | 2.69E-02 | CPD,CTSB,PDGFRA |
| IL1A | cytokine | 2.69E-02 | ADAMTS5,CXCL12,NR2F2 |
| MYDGF | cytokine | 2.72E-02 | MAF |
| CXCL1 | cytokine | 2.72E-02 | CXCR2 |
| TNFSF10 | cytokine | 2.92E-02 | HLA-A,IGF1R |
| PPBP | cytokine | 3.31E-02 | CXCR2 |
| TNFSF11 | cytokine | 4.04E-02 | CCR1,IL13RA1,MAFB |
| CXCL2 | cytokine | 4.78E-02 | CXCR2 |

UpStream_SP G3

| Upstream Regulator | Molecule Type | p-value of overlap | Target molecules in dataset |
|---|---|---|---|
| TNF | cytokine | 2.11E-09 | ALOX5,C5AR1,CD44,CEBPD,CH25H,CLEC5A,FOSL2,HMOX1,IER3,IL1R1,MCAM,MMP9,NLRP12,PTPN12,S100A8,TREM1,TREM3 |
| IFNG | cytokine | 4.83E-07 | C5AR1,CD44,CEBPD,CH25H,CLEC5A,HMOX1,IER3,IL1R1,MMP9,PLEK,S100A8,TREM1,TREM3 |
| IL13 | cytokine | 7.80E-06 | CD44,HMOX1,IL1R1,MMP9,S100A8,SLA,SPINT1 |
| CSF2 | cytokine | 2.29E-05 | ALOX5,C5AR1,ID2,IER3,IL1R1,MMP9,TREM1 |
| IL1B | cytokine | 2.97E-05 | CD44,CEBPD,HMOX1,IER3,IL1R1,MMP9,S100A8,TREM1,TREM3 |
| IL6 | cytokine | 4.94E-05 | C5AR1,CD44,CEBPD,HMOX1,ID2,IL1R1,MMP9,STAP2 |
| IL1A | cytokine | 5.74E-05 | CD44,HMOX1,MCAM,MMP9,S100A8 |
| IL10 | cytokine | 5.83E-05 | CD44,HMOX1,IL1R1,MMP9,S100A8,TREM1 |
| CD40LG | cytokine | 1.03E-04 | CD44,FOSL2,ID2,PAWR,PLEK,PTPN12 |
| IL4 | cytokine | 1.14E-04 | ALOX5,C5AR1,CD44,IER3,IL1R1,MMP9,PAWR,S100A8 |
| CCL5 | cytokine | 3.69E-04 | C5AR1,CD44,MMP9 |
| IL19 | cytokine | 5.04E-04 | HMOX1,MMP9 |
| OSM | cytokine | 5.17E-04 | CEBPD,CH25H,HMOX1,ID2,MMP9,S100A8 |
| IL22 | cytokine | 7.16E-04 | HMOX1,MMP9,S100A8 |
| CX3CL1 | cytokine | 9.31E-04 | HMOX1,MMP9 |
| TNFSF11 | cytokine | 1.41E-03 | CD44,HMOX1,IER3,MMP9 |
| TIMP1 | cytokine | 1.48E-03 | CD44,MMP9 |
| IL21 | cytokine | 1.54E-03 | CD244,ID2,MMP9 |
| SPP1 | cytokine | 1.82E-03 | CD44,HMOX1,MMP9 |
| IL18 | cytokine | 2.42E-03 | CD244,CD44,MMP9 |
| IL2 | cytokine | 2.80E-03 | CD244,CD44,IER3,IL1R1,MMP9 |
| CSF3 | cytokine | 4.04E-03 | CD44,HMOX1,MMP9 |
| IL12B | cytokine | 5.07E-03 | CEBPD,S100A8 |
| CXCL11 | cytokine | 5.88E-03 | MMP9 |
| IL17A | cytokine | 6.00E-03 | CEBPD,MMP9,S100A8 |
| IL17B | cytokine | 7.84E-03 | MMP9 |
| CXCL9 | cytokine | 7.84E-03 | MMP9 |
| MIF | cytokine | 8.38E-03 | CD44,MMP9 |
| CXCL8 | cytokine | 8.85E-03 | CD44,MMP9 |

[Table 19-4]

## UpStream_SP G3 (cont.)

| | | | |
|---|---|---|---|
| CXCL6 | cytokine | 9.79E-03 | MMP9 |
| TNFSF12 | cytokine | 1.22E-02 | MMP9,S100A8 |
| CCL18 | cytokine | 1.37E-02 | MMP9 |
| IL5 | cytokine | 1.49E-02 | FOSL2,IER3,MMP9 |
| IFNB1 | cytokine | 1.52E-02 | CH25H,IL1R1,MMP9 |
| Ccl6 | cytokine | 1.76E-02 | MMP9 |
| IL20 | cytokine | 1.95E-02 | MMP9 |
| CD70 | cytokine | 2.14E-02 | CD44 |
| TNFSF14 | cytokine | 3.29E-02 | MMP9 |
| IL15 | cytokine | 3.41E-02 | CD244,CD44,PLEK |
| NAMPT | cytokine | 4.24E-02 | MMP9 |
| FAM3B | cytokine | 4.43E-02 | IER3 |
| CXCL12 | cytokine | 4.69E-02 | CD44,MMP9 |
| CXCL10 | cytokine | 4.80E-02 | MMP9 |
| TNFSF15 | cytokine | 4.80E-02 | MMP9 |

## UpStream_LV G1

| Upstream Regulator | Molecule Type | p-value of overlap | Target molecules in dataset |
|---|---|---|---|
| IFNB1 | cytokine | 1.85E-24 | DDX58,DHX58,EIF2AK2,GBP7,IFI16,IFIH1,IFIT1B,IFIT3,Igtp,IRF7,IRF9,Irgm1,Oas1d (includes others),PML,RSAD2,STAT1,Trim30a/Trim30d,UBA7,XAF1 |
| IFNA2 | cytokine | 2.29E-17 | DDX58,EIF2AK2,IFI16,IFIH1,IFIT3,IRF7,IRF9,PARP9,PML,RSAD2,STAT1,TDRD7,UBA7,XAF1 |
| IFNL1 | cytokine | 5.24E-14 | DDX58,EIF2AK2,IFIH1,IFIT3,IRF9,PML,RSAD2,STAT1,TDRD7 |
| IFNG | cytokine | 4.47E-09 | CCND2,DDX58,EIF2AK2,GBP7,IFI16,IFIH1,IFIT3,Igtp,Iigp1,IRF7,IRF9,Irgm1,PARP9,PML,RSAD2,STAT1 |
| IL1RN | cytokine | 7.99E-09 | DDX58,IFIH1,IFIT3,IRF7,IRF9,PML,RSAD2 |
| IFNA1/IFNA13 | cytokine | 1.30E-07 | DHX58,EIF2AK2,IFIH1,RSAD2,STAT1 |
| IL4 | cytokine | 6.62E-07 | ASCC3,CCND2,GBP7,IFI16,IFIT1B,IFIT3,Iigp1,IRF7,SMG7,STAT1,Trim30a/Trim30d |
| IFNK | cytokine | 1.68E-05 | EIF2AK2,IFIH1,STAT1 |
| TNF | cytokine | 1.96E-05 | CCND2,CD47,DDX58,EIF2AK2,IFI16,IFIH1,IFIT1B,IFIT3,IRF7,LAMA3,PML,STAT1,TDRD7 |
| TNFSF10 | cytokine | 3.92E-05 | EIF2AK2,IFI16,IRF9,STAT1 |
| CD40LG | cytokine | 1.88E-04 | ASCC3,EIF2AK2,IFIT3,PML,SMG7,STAT1 |
| IFNA17 | cytokine | 4.18E-04 | EIF2AK2,IFIH1 |
| CSF1 | cytokine | 4.84E-04 | CCND2,Iigp1,IRF7,STAT1 |
| IL1B | cytokine | 2.13E-03 | IFI16,IFIT1B,IFIT3,IRF7,LAMA3,RSAD2,STAT1 |
| IFNE | cytokine | 2.81E-03 | IFIH1,STAT1 |
| EBI3 | cytokine | 4.36E-03 | CCND2,STAT1 |
| IL12A | cytokine | 6.22E-03 | IRF9,STAT1 |
| IL12B | cytokine | 6.22E-03 | IRF9,STAT1 |
| IL10 | cytokine | 6.97E-03 | CCND2,Igtp,Iigp1,STAT1 |
| IL5 | cytokine | 1.97E-02 | CCND2,GBP7,IRF7 |
| WNT5A | cytokine | 2.25E-02 | LAMA3,STAT1 |
| OSM | cytokine | 2.68E-02 | CCND2,IRF7,IRF9,STAT1 |
| IFNA10 | cytokine | 3.02E-02 | IFIH1 |
| IFNA21 | cytokine | 3.02E-02 | IFIH1 |
| IFNA5 | cytokine | 3.02E-02 | IFIH1 |
| IFNA7 | cytokine | 3.02E-02 | IFIH1 |
| IFNA14 | cytokine | 3.02E-02 | IFIH1 |
| IFNA6 | cytokine | 3.02E-02 | IFIH1 |
| IL27 | cytokine | 3.05E-02 | CCND2,STAT1 |
| IFNA8 | cytokine | 3.23E-02 | IFIH1 |
| IFNA16 | cytokine | 3.23E-02 | IFIH1 |
| IFNW1 | cytokine | 3.86E-02 | IFIH1 |

## UpStream_LV G2

| Upstream Regulator | Molecule Type | p-value of overlap | Target molecules in dataset |
|---|---|---|---|
| IL6 | cytokine | 1.41E-06 | ACOX1,CD163,CXCL2,EGFR,Hamp/Hamp2,ID2,IGFBP3,IL1R1,LRG1,NR1I2,PPRC1,SAA1,SERPINA3,SLC39A14,STAT3,THPO |
| OSM | cytokine | 3.69E-05 | ACOT2,CXCL2,FMO5,Hamp/Hamp2,ID2,MGLL,NEDD4L,PTP4A1,QSOX1,SAA1,SERPINA3,STAT3 |
| FAM3B | cytokine | 2.78E-04 | GJA1,IGFBP3,LDHA |
| TNF | cytokine | 3.73E-04 | ACOX1,BACE1,CASP8,CD163,CXCL2,EGFR,GJA1,GPD2,Hamp/Hamp2,IGFBP3,IL1R1,LDHA,LRG1,MFSD2A,NLRP12,NR1I2,PLIN2,SAA1,SERPINA3,ST3GAL5 |
| IL13 | cytokine | 1.23E-03 | ACOX1,CASP8,CD163,CXCL2,EGFR,IL1R1,PLXNC1,QSOX1 |
| WNT5A | cytokine | 2.98E-03 | CXCL2,EGFR,ID2,STAT3 |
| SPP1 | cytokine | 5.07E-03 | CXCL2,EGFR,FOXRED1,POR |
| IL1A | cytokine | 6.72E-03 | CXCL2,Hamp/Hamp2,LDHA,SAA1,SERPINA3 |
| IL1B | cytokine | 7.23E-03 | CXCL2,GJA1,Hamp/Hamp2,IGFBP3,IL1R1,LDHA,PTP4A1,SAA1,SAA2-SAA4,SERPINA3,STAT3 |
| CCL5 | cytokine | 7.40E-03 | CD163,CXCL2,STAT3 |
| CNTF | cytokine | 1.06E-02 | GJA1,SERPINA3,STAT3 |
| IL22 | cytokine | 1.37E-02 | CXCL2,SAA1,SERPINA3 |
| IFNB1 | cytokine | 1.67E-02 | CASP8,CXCL2,Hamp/Hamp2,IL1R1,SLC39A14 |
| IL17A | cytokine | 2.14E-02 | CD163,CXCL2,HSPB8,STAT3 |
| CCL22 | cytokine | 2.22E-02 | CXCL2 |
| IL21 | cytokine | 2.74E-02 | ID2,STAT3,TIRAP |
| PRL | cytokine | 3.09E-02 | EGFR,ID2,IGFBP3,SERPINA3 |
| IL10 | cytokine | 4.60E-02 | CASP8,CD163,CXCL2,IL1R1,STAT3 |

[Table 19-5]

## UpStream_LV G3

UpStream_LV G3

| Upstream Regulator | Molecule Type | p-value of overlap | Target molecules in dataset |
|---|---|---|---|
| IL15 | cytokine | 5.58E-05 | CD55,CORO1A,ICAM2,KIT,PLEK,TXNRD1 |
| TNF | cytokine | 4.74E-04 | ABCC1,CD55,CLEC4E,ICAM2,ITGB2,KIT,TRAF2,TRAF3,TREM3,TXNRD1 |
| IL4 | cytokine | 3.01E-03 | CD55,ITGB2,KIT,LILRB4,SIRPA,TRAF3 |
| IFNG | cytokine | 7.95E-03 | CD55,CLEC4E,CORO1A,ITGB2,PLEK,TRAF2,TREM3 |
| CCL18 | cytokine | 1.31E-02 | S100A4 |
| IL2 | cytokine | 1.38E-02 | ABCC1,KIT,S100A4,TRAF2 |
| C5 | cytokine | 1.79E-02 | CD55,ITGB2 |
| CCL1 | cytokine | 1.86E-02 | ITGB2 |
| SCGB1A1 | cytokine | 2.96E-02 | ITGB2 |
| CD40LG | cytokine | 3.57E-02 | PLEK,TRAF2,TRAF3 |
| CSF3 | cytokine | 3.76E-02 | ITGB2,KIT |
| IL6 | cytokine | 3.89E-02 | ABCC1,CLEC4E,KIT,TRAF3 |

## UpStream_LV G4

| Upstream Regulator | Molecule Type | p-value of overlap | Target molecules in dataset |
|---|---|---|---|
| IL1B | cytokine | 3.41E-16 | A2M,ACPP,APCS,ARL6IP5,ATF3,ATP2A2,BTG2,CASP4,CCL17,CCL22,CCR1,CD14,CD44,CD55,CDKN1A,CEBPD,CEBPG,CP,CREM,CRP,CSF2RB,CTGF,Cxcl9,CYTIP,DAB2,DR1,EFNA1,EIF4E,ERRFI1,F13A1,FABP5,FGB,FKBP5,FN1,FPR2,GADD45B,GAS6,GNL1,GRN,GTF2F1,HIF1A,HK2,HLA-A,HNF4A,IFI16,IFRD1,IL1A,IL1R1,IL1RN,IRAK2,IRG1,ITGAM,ITGB3,JUN,LBP,LCN2,LDHA,MMP13,MMP8,MMP9,Mt1,Mt2,NAMPT,NFKBIB,NFKBIZ,ODC1,PFKP,PIM3,PLAA,PLD1,PSEN1,PTP4A1,RAC2,RAN,S100A10,S100A8,S100A9,Saa3,SCLY,SDC4,SERPINA3,Sf1,SLC10A2,SLC11A2,SLC20A1,SOCS3,SOD2,SOX9,SYVN1,TBP,TGM2,TIMP1,TIMP3,TNFAIP6,TNFRSF1B,TREM3,UAP1,VASP,VEGFC |
| TNF | cytokine | 2.76E-14 | ABCA1,ABCC1,ADAM17,ADORA1,AIMP1,APCS,ARF4,ARL6IP5,ATF3,ATP2A2,B4GALNT1,B4GALT1,BMPER,BTG2,CALR,CASP4,CCL17,CCL19,CCL22,CCR1,CD14,CD28,CD38,CD44,CD55,CDKN1A,CEBPD,CEBPG,CP,CREB3,CREM,CRP,CSF2RB,CTGF,Cxcl9,CYP1B1,CYP7B1,CYTIP,DUSP2,EFNA1,FABP4,FABP5,FCER1G,FN1,FPR1,FPR2,GADD45B,GADD45G,GLS,GNAI2,GNL1,GOSR2,GRN,HDC,HIF1A,HIPK3,HK2,HLA-A,HNF4A,ICAM2,IDE,IFI16,IL1A,IL1R1,IL1RN,IL4R,IRAK2,IRF8,IRG1,ITGAL,ITGAM,ITGB2,ITGB3,ITPR1,JUN,KLF6,KLK3,LBP,LCN2,LDHA,LITAF,LRG1,LSS,LYVE1,MFSD2A,MMP13,MMP8,MMP9,MSR1,Mt1,Mt2,MVP,MYH9,NAMPT,NCF1,NFKBIB,NFKBIZ,NLRP12,NNMT,NPM3,NUCB2,NUP98,ODC1,PC,PDIA4,PIM3,PLAA,PLAUR,PRDM1,PSEN1,PYCARD,RAPGEF5,RCAN2,S100A8,S100A9,Saa3,SDC4,SEC22B,SELP,SERPINA3,Sf1,SLC11A2,SLC20A1,SOCS3,SOD2,SOX9,SQLE,ST3GAL3,ST3GAL5,ST6GAL1,STEAP4,SYNPO,SYVN1,TGM2,TIFA,TIMP1,TIMP3,TNFAIP6,TNFRSF1A,TNFRSF1B,TPP2,TREM3,VASP,VEGFC,ZNF330 |
| IFNG | cytokine | 3.79E-13 | A2M,ABCA1,ADORA1,ALDH1L1,ARG2,ARL6IP5,ATF3,ATP2A2,CASP4,CCL17,CCL19,CCL22,CCR1,CD14,CD44,CD55,CDKN1A,CEBPD,CERS6,CORO1A,CP,CREM,CSF2RB,CTGF,Cxcl9,CYB561,DRG1,EDNRA,EGF,FABP5,FCER1G,FCGR2A,FKBP5,FN1,FPR2,GAS6,GLS,GNAI2,GNL1,HCK,HIF1A,HK2,HLA-A,HSP90AA1,IDE,IDI1,IFI16,IFITM2,Iigp1,IL12RB1,IL17RA,IL1A,IL1R1,IL1RN,IL4R,IRAK2,IRF8,IRG1,ITGAL,ITGAM,ITGB2,ITGB3,ITPR1,JUN,KARS,KLF6,LCN2,LCP2,MED15,METAP2,MMP13,MMP9,MSR1,Mt1,MYH9,NAMPT,NEURL3,NFKBIB,NFKBIZ,NUP98,ODC1,PIM3,PLAUR,PLD1,PLEK,PRDM1,PTPN1,RAB12,RAC2,S100A10,S100A8,S100A9,Saa3,SBNO2,SCLY,SDC4,SELP,SERP1,Serpina3g (includes others),SLC11A1,SLC11A2,SOCS3,SOD2,SQLE,SREBF2,SRP54,TIMP1,TIMP3,TLR1,TNFAIP6,TNFRSF12A,TNFRSF1A,TNFRSF1B,TP63,TREM3,TRIB2,VEGFC,WARS,Wfdc17,ZBTB16,ZKSCAN1 |
| IL6 | cytokine | 6.89E-11 | A2M,ABCA1,ABCC1,APCS,ATF3,ATP2A2,BTG2,CCR1,CD14,CD44,CD48,CD97,CDKN1A,CEBPD,Chil3/Chil4,CP,CRP,CSF2RB,CTGF,Cxcl9,CYP1B1,CYTIP,FGB,FGL1,FN1,FPR2,GADD45B,GADD45G,GALE,HIF1A,HLA-A,HSPA5,IFI16,IK,IL1R1,IL1RN,IL4R,IL6ST,ITGAM,ITGB3,ITPR1,JUN,KLK3,KRT18,LBP,LCN2,LRG1,MMP13,MMP8,MMP9,MSR1,Mt1,Mt2,NAMPT,NUCB2,Orm1 (includes others),S100A9,Saa3,SBNO2,SENP2,SERPINA3,SLC39A14,SNX10,SOCS3,SOD2,STAP2,TGM2,TIMP1,TLR1,TNFRSF1A,TNFRSF1B,TRAF7,VASP,WARS,ZW10 |
| IL13 | cytokine | 4.30E-10 | ABCA1,ADORA1,ARG2,ATF3,BZW2,CCL17,CCL22,CD14,CD44,CD48,Chil3/Chil4,CLEC4A,CTGF,EZR,F13A1,FABP4,FCGR2A,FLOT1,FPR2,GAS6,GNAI2,HDC,IARS,IL13RA1,IL1R1,IL1RN,KLF6,LILRB3,MMP13,MMP9,MSMO1,PAPSS1,PCM1,PDGFC,PFKP,QSOX1,S100A8,SELP,SEPT11,SLA,SLC16A6,SLC43A3,SOCS3,TGM2,TIMP1,TLR1,TNFRSF1A,TNFRSF1B,TRPS1 |
| IL5 | cytokine | 7.49E-10 | A2M,ASNS,CASP4,CCL22,CCR1,CD55,CDKN1A,CKAP4,CRELD2,CSF2RB,DDX21,DNTTIP2,ELL2,ERO1L,GADD45G,HIF1A,HSP90B1,HSPA5,IDI1,IL4R,ITGAM,LCP2,MANF,MMP9,NABP1,PDIA6,PFKP,PKM,PRDM1,QSOX1,RBM3,RPN1,SDF2L1,Serpina3g (includes others),SNAP23,SPCS2,SUPT6H,TLR1,ZNF25 |
| OSM | cytokine | 8.85E-10 | A2M,ABCA1,ABCC1,ADAM17,ASNS,BRD8,CASP4,CDA,CDKN1A,CEBPD,CRP,CYP1B1,DNAJC3,EXOSC10,FGB,FN1,GADD45G,GFPT1,HIF1A,HIPK3,HK2,HLA-A,HSPA5,IL13RA1,IL4R,IL6ST,ITGAL,JMJD1C,JUN,KIF5B,LBP,LCN2,LITAF,MMP13,MMP8,MMP9,MOAP1,NAMPT,NEDD4L,NOMO1 (includes others),PFKFB2,PGGT1B,PRDM1,PTP4A1,QSOX1,S100A8,S100A9,SEL1L,SELP,SERPINA3,SLC16A6,SLC39A7,SOCS3,SON,TAT,TIMP1,TIMP3,UAP1,ZNF266,ZNF330 |
| IL4 | cytokine | 1.27E-09 | ACOT9,ALOX12,ARG2,B4GALNT1,CAPG,CCDC86,CCL17,CCL22,CCT3,CD14,CD38,CD44,CD55,CDKN1A,Chil3/Chil4,CSF2RB,CTGF,Ctla2a/Ctla2b,Cxcl9,EBNA1BP2,F13A1,FABP4,FCER1G,FCGR2A,FKBP5,FN1,FPR2,GADD45G,GZMA,HCK,HK2,IDE,IFI16,Iigp1,IL12RB1,IL13RA1,IL1A,IL1R1,IL1RN,IL4R,IL6ST,IRF8,ITGAL,ITGB2,ITGB3,JUN,KLF6,KLK3,LGALSL,LIG3,MARCO,MMP9,MSR1,NABP1,NAP1L1,PFKP,PLD1,PNP,PRDM1,PRMT1,RNPS1,S100A10,S100A8,S100A9,Saa3,SDF2L1,SELP,Serpina3g (includes others),Snrpa (includes others),SOCS3,SRM,ST6GAL1,SYK,SYT11,TGM2,TIMP1,TIMP3,TXLNA |
| TNFSF11 | cytokine | 1.71E-09 | CCR1,CD14,CD44,CDKN1A,CLOCK,DAB2,DOK2,FGR,FPR1,GADD45B,HCK,IL13RA1,IL1A,IL1RN,IRF8,IRG1,ITGAL,ITGAM,ITGB3,JUN,MMP9,NFKBIB,NFKBIZ,NME6,PLAUR,PLD1,PRDM1,Saa3,SDC4,SEL1L,SENP2,SLC11A2,SLC20A1,SMARCAD1,SOCS3,SOD2,TNFRSF1B |
| IL3 | cytokine | 1.93E-09 | BTG2,CALR,CAMKK2,CCL19,CD14,CD48,CD97,CDKN1A,CEBPG,CEBPZ,CREM,CSNK1A1,DOK2,FCGR2A,GADD45B,GADD45G,GZMA,HK2,HLA-A,HNF4A,HNRNPA2B1,HSP90B1,HSPA5,IK,ITGAM,ITGB3,JUN,LCN2,Mt1,NARS,NCF1,NIFK,ODC1,RAN,RANBP1,RARS,RBM3,SERPINA3,Serpina3g (includes others),SLC2A3,SOCS3,STT3A,TPD52,VASP |
| IL10 | cytokine | 2.24E-07 | APCS,ARG2,CCL17,CCL19,CCL22,CCR1,CD14,CD28,CD44,CD55,CDKN1A,CR1L,CSF2RB,Cxcl9,FCER1G,FCGR2A,FKBP5,FPR1,GZMA,Iigp1,IL17RA,IL1A,IL1R1,IL1RN,IL4R,IL6ST,ITGAL,JUN,MMP8,MMP9,MSR1,PRDM1,PTPN1,PTPN2,REG3A,S100A8,SNAP23,SOCS3,TIMP1,TNFRSF1A,TNFRSF1B |
| EDN1 | cytokine | 2.89E-07 | ATF6,ATP2A2,CDC25A,CTGF,EDNRA,ERRFI1,EZR,FN1,GRN,HIF1A,HSPA5,ITGAM,ITGB3,JUN,MMP13,MMP9,MSN,NCF1,ODC1,PDIA6,PLAUR,PRKCE,SOCS3,TGM2,TIMP1,TIMP3,TPM3,VEGFC |

[Table 19-6]

## UpStream_LV G4 (cont.)

| | | | |
|---|---|---|---|
| IL17A | cytokine | 1.92E-06 | CCL17,CCL22,CD14,CEBPD,CRP,CTGF,CYP7B1,HSPB8,IL17RA,IL1A,IL1RN,ITPR1,JUN,KLF3,LCN2,MMP13,MMP8,MMP9,NFKBIZ,REG3A,S100A8,Saa3,SELP,SOCS3,TIMP1,VEGFC |
| CTF1 | cytokine | 6.73E-06 | A2M,CEBPD,CRP,IL6ST,LBP,REG3A,SERPINA3,SOCS3,TIMP1 |
| CSF1 | cytokine | 7.91E-06 | CD97,CDKN1A,DOK2,FCER1G,FCGR2A,FN1,HSP90B1,HSPA5,Iigp1,IL1A,IL6ST,ITGAM,ITGB3,JUN,MAP3K3,MARCO,MMP9,MSR1,STX12,STX6,TLR1,TNFRSF1A,TNFRSF1B |
| IL2 | cytokine | 1.45E-05 | ABCC1,CD244,CD28,CD38,CD44,CDC25A,CDKN1A,CSF2RB,CTPS1,Cxcl9,CYTIP,DAP,DDX21,DUSP2,FCER1G,GADD45B,GADD45G,GZMA,HCK,HK2,HSP90B1,HSPA5,IDI1,IL12RB1,IL1A,IL1R1,IL4R,JUN,KLF6,LCP2,MMP9,NIFK,PDGFC,PDIA3,PNP,POLR3K,PRDM1,RGPD4 (includes others),SLC2A3,SNAP23,SOCS3,STK17B,TIMP1,TNFRSF12A,TNFRSF1A,TNFRSF1B,TRIB2 |
| CSF3 | cytokine | 1.71E-05 | CAPG,CD14,CD44,FGB,FPR1,GADD45B,GADD45G,HDC,IK,IL1RN,ITGAM,ITGB2,JUN,LBP,LCN2,MMP8,MMP9,ODC1,PRKCE,SOCS3,TNFRSF1A,TNFRSF1B |
| IL11 | cytokine | 2.49E-05 | A2M,CEBPD,CRP,EZR,FABP4,IL6ST,LBP,MMP13,SERPINA3,SOCS3,TIMP1 |
| CCL5 | cytokine | 3.77E-05 | CCR1,CD44,CD97,CYP1B1,EGF,HDC,MMP9,NAMPT,PLAUR,PLEC,PNP,SQLE,VASP |
| CSF2 | cytokine | 3.81E-05 | ABCA1,CASC3,CCL17,CCR1,CD14,CD28,CD38,CD97,CDKN1A,CSF2RB,Cxcl9,DOK2,FPR2,HDC,IK,IL1A,IL1R1,IL1RN,ITGAM,ITGB3,MARCO,MFSD2A,MMP9,ODC1,PIM3,PSEN1,QSOX1,RBM3,REG3A,RINT1,Saa3,SLC11A2,SLC30A7,SNAP23,SOCS3,SOD2,TGM2,TIFA,TLR1,TNFRSF1A,TNFRSF1B,ZNF25 |
| CD40LG | cytokine | 4.12E-05 | ADAM17,AKAP2,ATF3,CCL17,CCL19,CCL22,CCR1,CD38,CD44,CDKN1A,Chil3/Chil4,CTGF,CYP1B1,CYTIP,DAPP1,DUSP2,HIF1A,HK2,HSP90AA1,IL12RB1,IL13RA1,IL1A,ITGAM,JUN,LIG3,NAMPT,NAP1L1,NFKBIB,PLAUR,PLEK,PRDM1,PTPN1,SELP,SERPINA10,SOD2,TANK,TGM2,TNFAIP6 |
| LIF | cytokine | 1.03E-04 | A2M,CDKN1A,CEBPD,CRP,FGB,FN1,GATA6,HIF1A,HK2,IFI16,IL6ST,JUN,LBP,MMP13,PIM3,PLD1,PTPN1,REG3A,SERPINA3,SNAP23,SOCS3,SOD2,TIMP1 |
| CX3CL1 | cytokine | 6.88E-04 | HIF1A,IL1A,MMP9,MSR1,SELP,TIMP1 |
| TNFSF10 | cytokine | 1.12E-03 | CD14,HLA-A,IFI16,IFITM2,IL1RN,ITGAM,JUN,MMP9,PRKCE,TIMP1,Tnfrsf22/Tnfrsf23,TP63 |
| PRL | cytokine | 1.77E-03 | A2M,CDKN1A,CEBPD,EGF,FN1,HERPUD1,HIPK3,IDE,JUN,LYVE1,MSN,ODC1,PC,PDIA4,PIM3,SERPINA3,SOCS3,TIMP1,Tmsb4x (includes others),TP63,TPM3 |
| TIMP1 | cytokine | 2.51E-03 | CD38,CD44,CDKN1A,MMP9,PLAUR,TIMP3 |
| IL22 | cytokine | 4.25E-03 | CCL17,CCL22,IL1A,LBP,LCN2,MMP9,REG3A,S100A8,S100A9,SERPINA3,SOCS3 |
| FAM3B | cytokine | 4.59E-03 | CASP4,CDKN1A,FN1,LCN2,LDHA |
| LTA | cytokine | 5.34E-03 | APCS,CCL19,CRP,HDC,ITGB3,LYVE1,ODC1 |
| CNTF | cytokine | 6.31E-03 | A2M,CEBPD,CRP,IL1A,IL6ST,LBP,REG3A,SERPINA3,SOCS3,TIMP1 |
| Il3 | cytokine | 6.54E-03 | CD14,CDKN1A,ITGAM,LCN2,PRKCE,SNAP23,SRP9,TNFRSF1A,TNFRSF1B |
| IL15 | cytokine | 7.11E-03 | CCL17,CCL19,CCR1,CD244,CD28,CD38,CD44,CD55,CDKN1A,COPB2,CORO1A,CTGF,GNL2,HNRNPA2B1,ICAM2,IL12RB1,JUN,PDIA3,PDIA4,PFDN4,PLD1,PLEK,PNP,PTP4A1,RAC2,SYNPO,TIMP1,TNFRSF1A,VBP1 |
| IL1A | cytokine | 8.35E-03 | CD44,CDKN1A,HDC,IK,IL1A,IL1RN,ITGB3,JUN,LCN2,LDHA,MMP13,MMP9,NFKBIZ,S100A8,S100A9,Saa3,SERPINA3,SOD2,VEGFC |
| CCL2 | cytokine | 8.62E-03 | ABCA1,HDC,IL1A,ITGAL,LCN2,MMP9,NCF1,SLC11A1,TIMP1 |
| TNFSF12 | cytokine | 8.82E-03 | CCL19,CCR1,CLEC4D,ITGAM,MMP13,MMP9,S100A8,S100A9,TIMP1,TNFRSF12A |
| CXCL12 | cytokine | 1.35E-02 | CD44,FN1,HIPK3,IL1A,ITGAL,ITGB3,JMJD1C,JUN,LCP2,MMP13,MMP9,RPS6KA3,SOCS3,TDG,TNFRSF1B,ZBTB16 |
| IL17B | cytokine | 1.36E-02 | CDKN1A,MMP9 |
| WNT5A | cytokine | 1.53E-02 | CD14,CD38,DAB2,DOK2,FABP4,FN1,IL1A,IRF8,MMP13,SOCS3,TLR1 |
| CXCL5 | cytokine | 3.18E-02 | ITGAL,ITGAM |
| IL17C | cytokine | 3.38E-02 | IL1A,LCN2,PRDM1,S100A8,S100A9 |
| IL18 | cytokine | 3.63E-02 | CD244,CD44,GADD45B,GADD45G,IL12RB1,IL1A,ITGAM,JUN,MMP13,MMP9,NFKBIZ,TIMP1 |
| C5 | cytokine | 4.24E-02 | APCS,ATF3,CD55,CRP,FCGR2A,IL1A,ITGAM,ITGB2,ST6GAL1,VEGFC |
| IL12B | cytokine | 4.86E-02 | CEBPD,IL1A,PIK3AP1,S100A8,S100A9,SOCS3 |
| Ctf2 | cytokine | 4.92E-02 | SOCS3 |

As expected, the G1 adjuvants were characterized by interferon responses (Figures **3a** and **3b**). G2 to G6 adjuvants were associated with inflammatory cytokines such as IL6 and TNF (Figure **3b**). G2 adjuvants had weaker associations with lipid metabolism (Figure **3a**), and IPA analysis suggested oncostatin M and IL10 association (Figure **3b**). It was difficult to retrieve preferential associations for the G3-G5 adjuvants because these groups contained a limited number of adjuvants and organs (Figure **3a**). Although conducted under limited conditions, the analysis suggested that the G3 adjuvants might be associated with T and NK cell cytokines such as IL2, IL4 and IL15 (Figure **3b**). G4 adjuvants had broader profiles than the other adjuvants and contained many cytokines. However, the preferential $M32^{SP}$ module association suggested that G4 might be characterized by TNF responses (Figure **16b**). G5 adjuvants were associated with phosphate-containing compounds with metabolic processes suggestive of nucleotide metabolic processes, a finding consistent with G5 being a CpG nucleic acid adjuvant group (Figure **3a**). G6 adjuvants such as AddaVax, an MF59 equivalent oil adjuvant, were associated with the phagosome (Figure **3a**), and IL1A and IL33 were suggested as the cytokines for them by IPA upstream cytokine analysis (Figure **3b**).

(Adjuvant group analysis predicts the modes of action of ADX, bCD and ENDCN)

The G1 adjuvants (cdiGMP, cGAMP, DMXAA, PolyIC, and R848), which were RNA-related adjuvants or STING ligands, were strongly associated with type I and type II interferon responses, making them clearly distinct from the G2-G6 adjuvants. G2 to G6 adjuvants appear to be associated with inflammatory responses. G2 includes ALM and bCD, which are both expected to work through DAMPs (Marichal, T. et al., Nat Med 17, 996-1002 (2011) and Onishi, M. et al., Journal of immunology 194, 2673-2682 (2015)). Intriguingly, the strongly responding D35 and K3 samples (D35_ID_x2 and K3_ID_x3 in LV), which were mentioned above as clustering exceptions, are indicated in red in Figure **14a**) both clustered in $G2^{LV}$. Hence, it is possible that in certain situations D35 and K3 can mimic the DNA released from damaged cells to induce host immune responses (Marichal, T. et al., Nat Med 17, 996-1002 (2011) and Onishi, M. et al., Journal of immunology 194, 2673-2682 (2015)). G3 and G4 consisted mainly of adjuvants derived from bacterial cell wall-derived PAMPs. G5 consisted of CpG adjuvants. Although G5 adjuvants such as D35, K3, and K3SPG are recognized as good interferon inducers in vitro, the analysis suggested that the CpG adjuvants differ biologically from the typical G1-type

TLR and RLR ligands in vivo. G6 consisted of AddaVax, a MF59 equivalent. MF59's mechanism of action has been extensively examined. It was suggested that ATP (another DAMP) released from muscle acts as a mediator of the adjuvant effect (Vono, M.et al., Proceedings of the National Academy of Sciences of the United States of America 110, 21095-21100 (2013)). The adjuvant clustering results may support this interpretation, as G2 and G6 formed related but separate clusters in the samples from LNs (Figure **14c**).

Taken together, the resulting data strongly suggest that the adjuvants investigated were grouped according to shared similarities in their modes of action. Hence, it is possible to predict the mode of the action of a new adjuvant with knowledge about its grouping. To verify this hypothesis, two adjuvants were selected for testing. ENDCN is a novel lipid-based nasal adjuvant, currently in phase I/II clinical studies as part of an influenza vaccine (Falkeborn, T. et al., PloS one 8, e70527 (2013) and Maltais, A.K. et al., Vaccine 32, 3307-3315 (2014)). ENDCN was categorized in G2, the same group as bCD in both LV and SP. It has been previously showed that the adjuvanticity of bCD is likely to be associated with host cell-derived dsDNA via DAMPs (Onishi, M. et al., Journal of immunology 194, 2673-2682 (2015)). G2 categorization of ENDCN strongly suggested that ENDCN utilizes host-derived factors for its adjuvanticity. This hypothesis was further tested, and detailed immunological analyses of ENDCN in vivo have revealed that host cell releasing RNA and TBK1 are involved in the adjuvanticity (Hayashi, M. et al., Scientific Reports, 6, Article number: 29165 (2016)), further supporting ENDCN as a G2 adjuvant. Another example is ADX (Honda-Okubo, Y. et al., Vaccine 30, 5373-5381 (2012) and Saade, F. et al., Vaccine 31, 1999-2007 (2013)), which is an inulin-based particulate adjuvant with an unknown mode of action. $G3^{LV}/G4^{SP}$ but not G2 clustering suggests that ADX works through unidentified PAMPs receptors, instead of DAMPs mediators (Figures **2** and **14**). More detailed analysis of ADX is currently on going (Hayashi et al. Scientific Reports 6, Article number: 29165 (2016)).

(Adjuvant gene space analysis reveals differences among the same receptor-targeted adjuvants)

D35, K3 and K3SPG all act on TLR9, and cdiGMP, cGAMP, and the DMXAA target STING (Gao, P. et al., Cell 154, 748-762(2013)). Correspondingly, these adjuvants clustered in the same adjuvant group by the method of the invention. All TLR9 ligands were clustered in G5, and all STING ligands were clustered in G1 (Figures **2** and **14**). D35 and K3 have been known to induce overlapping but substantially different biological responses (Steinhagen, F. et al., Journal of Leukocyte Biology 92, 775-785 (2012)). With STING ligands, no direct comparison has been reported yet, but cdiGMP appears to induce Th1 responses (Madhun, A.S. et al., Vaccine 29, 4973-4982 (2011)), whereas DMXAA induces Th2 responses (Tang, C.K. et al., PloS one 8, e60038(2013)). Although the three STING ligands (cdiGMP, cGAMP, and DMXAA) share similar chemical structures, each of them is derived from bacteria, host cells, and synthetic chemicals, respectively (Gao, P. et al., Cell 154, 748-762 (2013)). Simple Venn analysis of the sDEGs from CpG (Figure **17**) and STING ligands (Figure **18**) in LNs only confirmed the well-known fact that these adjuvants induce interferon responses, and their individual differences were difficult to retrieve. However, use of z-scores (Figure **15**) and 40 module mapping (Figure **16**) analysis in the adjuvant gene space enabled identification of more detailed differences among them. D35 was a relatively stronger interferon inducer than K3 or K3SPG in vivo under the experimental conditions (Figures **4a** to **4c** and Table 20).

[Table 20]

| ProbeGenes | Module#(LN) | (5)D35.ID.LN.x2 | (5)D35.ID.LN.x1 | (5)D35.ID.LN.x3 | (3)K3.ID.LN.x1 | (3)K3.ID.LN.x2 | (3)K3.ID.LN.x3 | (5)K3SPG.ID.LN.x3 | (5)K3SPG.ID.LN.x1 | (5)K3SPG.ID.LN.x2 | D35(z sum) | K3(z sum) | K3SPG(z sum) | Preference |
|---|---|---|---|---|---|---|---|---|---|---|---|---|---|---|
| 1421009_at Rsad2 | 37 | -0.05 | 1.735 | 1.521 | -0.63 | -0.36 | 0.231 | -0.57 | -0.99 | -0.89 | 3.209 | -0.76 | -2.45 | D35 |
| 1436058_at Rsad2 | 37 | 0.104 | 1.354 | 1.822 | -0.57 | -0.44 | 0.303 | -0.76 | -0.93 | -0.88 | 3.281 | -0.71 | -2.57 | D35 |
| 1421008_at Rsad2 | 37 | -0.14 | 1.446 | 1.916 | -0.83 | -0.44 | -0.15 | -0.21 | -0.82 | -0.77 | 3.221 | -1.42 | -1.81 | D35 |
| 1418293_at Ifit2 | 40 | -0.13 | 1.614 | 1.531 | -0.65 | -0.28 | 0.445 | -0.5 | -1.12 | -0.9 | 3.02 | -0.49 | -2.53 | D35 |
| 1419603_at Ifi204 | 40 | 1.185 | 1.269 | 1.205 | -1.05 | -0.47 | 0.239 | -0.75 | -1.18 | -0.45 | 3.659 | -1.28 | -2.38 | D35 |
| 1423555_a_at Ifi44 | 40 | 1.105 | 0.679 | 1.768 | -0.79 | -0.51 | -0.13 | -0.11 | -0.64 | -1.37 | 3.551 | -1.43 | -2.12 | D35 |
| 1453196_a_at Oasl2 | 40 | 1.943 | 0.714 | 0.467 | -1.26 | -0.27 | 0.262 | -0.01 | -0.75 | -1.09 | 3.123 | -1.27 | -1.85 | D35 |
| 1440371_at --- | 8 | 1.485 | 1.31 | 0.495 | -0.52 | -0.37 | -0.36 | -0.52 | 0.197 | -1.73 | 3.29 | -1.24 | -2.05 | D35 |
| 1418580_at Rtp4 | 40 | 0.809 | 1.609 | 0.611 | -1.12 | 0.119 | -0 | 0.349 | -1.48 | -0.89 | 3.028 | -1 | -2.03 | D35 |
| 1460069_at Smc6 | 7 | 0.716 | 1.173 | 1.385 | -1.3 | -1.14 | -1.08 | 0.094 | 0.098 | 0.059 | 3.274 | -3.53 | 0.251 | D35 |
| 1418191_at Usp18 | 40 | 0.39 | 0.966 | 1.679 | -0.63 | 0.534 | 0.071 | -0.56 | -1.45 | -0.99 | 3.035 | -0.03 | -3.01 | D35 |
| 1424339_at Oasl1 | 37 | 0.62 | 1.742 | 0.836 | -0.81 | 0.112 | 0.196 | -0.37 | -0.76 | -1.57 | 3.198 | -0.5 | -2.7 | D35 |
| 1457035_at AI607873 | 40 | 0.908 | 1.231 | 1.239 | -0.55 | 0.616 | -0.32 | -1.27 | -0.83 | -1.02 | 3.377 | -0.26 | -3.12 | D35 |
| 1452349_x_at Ifi205///Mnda | 40 | 0.51 | 1.197 | 1.416 | -1.27 | -0.1 | -0.11 | 0.515 | -0.9 | -1.25 | 3.123 | -1.48 | -1.64 | D35 |
| 1422006_at Eif2ak2 | 40 | 1.174 | 1.037 | 1.068 | -1.75 | 0.425 | -0.62 | -0.63 | -0.09 | -0.62 | 3.279 | -1.95 | -1.33 | D35 |
| 1450403_at Stat2 | 40 | 2.023 | 0.499 | 0.867 | -0.99 | 0.138 | -0.21 | -0.75 | -0.62 | -0.95 | 3.389 | -1.07 | -2.32 | D35 |
| 1440475_at AW011738 | 36 | 0.863 | 0.622 | 1.554 | -1.4 | -0.73 | -0.23 | -1.31 | 0.286 | 0.341 | 3.038 | -2.36 | -0.68 | D35 |
| 1443302_at --- | 12 | 1.287 | 0.399 | 1.721 | -1.23 | -0.42 | -1.13 | -0.11 | -0.5 | -0.02 | 3.407 | -2.78 | -0.63 | D35 |
| 1422141_s_at Csprs///Gm15433///Gm2666///Gm7609///LOC100041903///LOC100503923 | 40 | 1.776 | 0.507 | 0.791 | -0.17 | -0.74 | -0.45 | -1.28 | 0.634 | -1.07 | 3.074 | -1.36 | -1.72 | D35 |
| 1453757_at Herc6 | 40 | 0.97 | 1.805 | 0.409 | -0.43 | 0.015 | 0.213 | -0.51 | -1.01 | -1.45 | 3.183 | -0.2 | -2.98 | D35 |
| 1421911_at Stat2 | 40 | 0.612 | 1.757 | 0.768 | -0.53 | -0.24 | 0.338 | -0.53 | -0.44 | -1.74 | 3.137 | -0.43 | -2.71 | D35 |
| 1441344_at Erlin1 | 21 | 1.36 | 0.052 | 1.758 | -1.03 | -0.98 | -0.61 | -0.78 | 0.047 | 0.191 | 3.171 | -2.63 | -0.54 | D35 |
| 1431095_a_at Herc6 | 40 | 1.937 | 0.426 | 0.702 | -0.94 | 0.033 | 0.178 | -0.06 | -1.31 | -0.97 | 3.065 | -0.73 | -2.34 | D35 |
| 1450377_at Thbs1 | 28 | 1.225 | 0.185 | 1.593 | -0.92 | -0.96 | -0.98 | 0.661 | -0.86 | 0.068 | 3.002 | -2.87 | -0.14 | D35 |
| 1456164_at AW011738 | 40 | 1.139 | 0.14 | 2.068 | -0.91 | -0.18 | -0.99 | -0.36 | -0.55 | -0.35 | 3.347 | -2.08 | -1.27 | D35 |
| 1425105_at Rbp3 | 10 | 1.968 | 0.568 | 0.796 | -1.04 | -0.86 | -0.21 | 0.173 | -0.31 | -1.09 | 3.333 | -2.11 | -1.23 | D35 |
| 1432417_a_at Tspan2 | 40 | 1.133 | 1.404 | 1.369 | -0.99 | -0.72 | -0.43 | -0.27 | -0.74 | -0.74 | 3.906 | -2.15 | -1.76 | D35 |
| 1442518_at C030044O21Rik | 14 | 0.953 | 1.161 | 1.432 | -0.25 | 0.036 | -1.37 | -0.52 | -0.25 | -1.2 | 3.546 | -1.58 | -1.97 | D35 |
| 1436472_at Slfn9 | 37 | 1.143 | 1.047 | 1.405 | -0.56 | -0.79 | -0.65 | 0.326 | -0.56 | -1.36 | 3.595 | -2 | -1.59 | D35 |
| 1419486_at Foxc1 | 19 | 1.628 | 0.676 | 1.04 | -0.77 | 0.046 | -0.03 | -1.67 | -0.49 | -0.43 | 3.344 | -0.76 | -2.58 | D35 |
| 1443853_x_at Sfmbt1 | 32 | 1.228 | 1.862 | 0.419 | -0.91 | -0.9 | -0.79 | -0.77 | -0.03 | -0.12 | 3.51 | -2.59 | -0.92 | D35 |
| 1448179_at Usmg5 | 24 | 0.103 | 1.297 | 2.062 | -0.66 | -0.66 | -0.7 | -0.48 | -0.46 | -0.5 | 3.461 | -2.02 | -1.44 | D35 |
| 1451777_at Ddx60 | 40 | 0.83 | 1.215 | 0.975 | -0.5 | 0.042 | 0.618 | -0.61 | -1.82 | -0.75 | 3.02 | 0.163 | -3.18 | D35 |
| 1447241_at --- | 37 | 0.048 | 2.27 | 0.715 | -0.88 | -0.82 | -0.89 | -0.03 | -0.18 | -0.23 | 3.033 | -2.6 | -0.44 | D35 |
| 1416380_at Mov10 | 37 | 1.449 | 0.681 | 1.147 | -1.16 | -1.48 | 0.219 | -0.65 | 0.088 | -0.28 | 3.277 | -2.43 | -0.85 | D35 |
| 1443392_at Trpv1 | 33 | 1.731 | 0.59 | 0.863 | 0.024 | 0.219 | -1.28 | -0.68 | -1.32 | -0.15 | 3.184 | -1.04 | -2.15 | D35 |
| 1455796_x_at Olfm1 | 11 | 0.86 | 0.592 | 1.811 | -0.89 | -0.11 | -0.44 | -0.46 | 0.18 | -1.55 | 3.262 | -1.44 | -1.83 | D35 |
| 1426013_s_at Plekha4 | 37 | 0.54 | 1.48 | 1.208 | -0.2 | 0.205 | -1.53 | -0.18 | -1.23 | -0.3 | 3.228 | -1.53 | -1.7 | D35 |
| 1416039_x_at Cyr61 | 39 | 1.724 | 0.461 | 0.83 | 0.035 | -0.72 | -0.89 | -0.18 | -1.63 | 0.372 | 3.015 | -1.58 | -1.44 | D35 |
| 1453299_a_at Pnp///Pnp2 | 40 | 1.476 | 0.696 | 0.948 | -0.96 | -0.05 | 0.23 | 0.266 | -1.27 | -1.33 | 3.12 | -0.78 | -2.34 | D35 |
| 1424609_a_at Cwc22///Gm4354///Xdh | 40 | 1.178 | 1.294 | 0.958 | -1.21 | -1.2 | -0.36 | -0.96 | 0.293 | 0 | 3.429 | -2.76 | -0.67 | D35 |
| 1424444_a_at 1600014C10Rik | 40 | 0.904 | 0.235 | 1.969 | -0.22 | -0.65 | 0.015 | -0.61 | -1.55 | -0.09 | 3.108 | -0.85 | -2.25 | D35 |
| 1439886_at --- | 40 | 0.159 | 1.149 | 1.843 | -0.46 | 0.3 | -1.03 | -1.2 | -0.09 | -0.67 | 3.151 | -1.19 | -1.96 | D35 |
| 1424607_a_at Gm4354 | 40 | 1.005 | 1.314 | 1.103 | -0.62 | -0.48 | 0.206 | -1.72 | -0.19 | -0.62 | 3.422 | -0.89 | -2.53 | D35 |
| 1423611_at Alpl | 24 | 0.707 | 1.244 | 1.244 | -1.13 | -0 | 0.131 | 0.066 | -1.66 | -0.61 | 3.194 | -1 | -2.2 | D35 |
| 1456346_at --- | 9 | 0.271 | 1.15 | 2.034 | -0.37 | -0.96 | -0.81 | -0.19 | -0.78 | -0.35 | 3.455 | -2.13 | -1.32 | D35 |
| 1420894_at Tgfbr1 | 3 | 0.691 | 0.803 | 1.839 | 0.157 | -1.17 | -1.13 | 0.079 | -0.72 | -0.55 | 3.333 | -2.14 | -1.19 | D35 |
| 1444615_x_at Runx1t1 | 1 | 0.71 | 1.504 | 1.304 | -0.02 | -1.32 | -0.77 | -0.57 | -0.93 | 0.09 | 3.519 | -2.11 | -1.4 | D35 |
| 1458457_at --- | 26 | 0.123 | 1.673 | 1.351 | -0.58 | 0.355 | -0.11 | -1.23 | -1.04 | -0.54 | 3.147 | -0.33 | -2.81 | D35 |

K3 preferentially induced RNA biogenesis, which was enriched in $M26^{LN}$, compared with D35 or K3SPG (Figure **4c**). Similar analysis (Figures **4d** to **4f** and Table 21) revealed that cdiGMP was the strongest interferon inducer, and it was associated with preferential gene mapping in $M37^{LN}$ and $M40^{LN}$ more than cGAMP and DMXAA (Figure **4f**).

[Table 21]

| ProbeGenes | Modules | (8)cdiGMP.ID.LN.x2 | (8)cdiGMP.ID.LN.x1 | (8)cdiGMP.ID.LN.x3 | (9)cGAMP.ID.LN.x1 | (9)cGAMP.ID.LN.x2 | (9)cGAMP.ID.LN.x3 | (10)DMXAA.ID.LN.x2 | (10)DMXAA.ID.LN.x1 | (10)DMXAA.ID.LN.x3 | cdiGMP(z sum) | cGAMP(z sum) | DMXAA(z sum) | preference |
|---|---|---|---|---|---|---|---|---|---|---|---|---|---|---|
| 1421578_at Ccl4 | 37 | 1.402 | 1.22 | 1.368 | -0.76 | -0.67 | -0.72 | -0.64 | -0.64 | -0.57 | 3.99 | -2.14 | -1.85 | cdiGMP |
| 1457404_at Nfkbiz | 37 | 1.371 | 1.232 | 1.386 | -0.73 | -0.57 | -0.63 | -0.75 | -0.72 | -0.59 | 3.989 | -1.92 | -2.06 | cdiGMP |
| 1442556_at --- | 35 | 1.421 | 1.315 | 1.245 | -0.74 | -0.5 | -0.71 | -0.64 | -0.59 | -0.8 | 3.981 | -1.95 | -2.03 | cdiGMP |
| 1421679_a_at Cdkn1a | 37 | 1.429 | 1.421 | 1.13 | -0.55 | -0.71 | -0.72 | -0.71 | -0.62 | -0.66 | 3.98 | -1.99 | -1.99 | cdiGMP |
| 1455197_at Rnd1 | 29 | 1.54 | 1.27 | 1.166 | -0.77 | -0.65 | -0.7 | -0.66 | -0.62 | -0.58 | 3.976 | -2.12 | -1.86 | cdiGMP |
| 1453636_at Pcgf5 | 36 | 1.332 | 1.143 | 1.496 | -0.76 | -0.48 | -0.62 | -0.69 | -0.72 | -0.7 | 3.972 | -1.86 | -2.11 | cdiGMP |
| 1425687_at Cflar | 36 | 1.199 | 1.3 | 1.469 | -0.81 | -0.59 | -0.81 | -0.58 | -0.48 | -0.7 | 3.968 | -2.21 | -1.76 | cdiGMP |
| 1441843_s_at 5230400M03Rik | 35 | 1.442 | 1.354 | 1.171 | -0.71 | -0.47 | -0.54 | -0.84 | -0.73 | -0.68 | 3.967 | -1.71 | -2.25 | cdiGMP |
| 1436387_at C330006P03Rik///Homer1 | 29 | 1.372 | 1.437 | 1.158 | -0.43 | -0.59 | -0.71 | -0.79 | -0.71 | -0.73 | 3.967 | -1.73 | -2.24 | cdiGMP |
| 1423028_at Ifna2 | 29 | 1.076 | 1.31 | 1.58 | -0.68 | -0.59 | -0.65 | -0.69 | -0.68 | -0.67 | 3.966 | -1.92 | -2.04 | cdiGMP |
| 1434350_at Csrnp1 | 37 | 1.353 | 1.305 | 1.308 | -0.59 | -0.54 | -0.85 | -0.83 | -0.72 | -0.45 | 3.966 | -1.97 | -1.99 | cdiGMP |
| 1459144_at --- | 35 | 1.275 | 1.199 | 1.493 | -0.62 | -0.57 | -0.72 | -0.52 | -0.89 | -0.65 | 3.966 | -1.91 | -2.06 | cdiGMP |
| 1458376_at B930025B16Rik | 35 | 1.188 | 1.502 | 1.273 | -0.6 | -0.61 | -0.64 | -0.9 | -0.72 | -0.49 | 3.963 | -1.85 | -2.11 | cdiGMP |
| 1453851_a_at Gadd45g | 37 | 1.368 | 1.383 | 1.21 | -0.46 | -0.65 | -0.85 | -0.84 | -0.5 | -0.66 | 3.961 | -1.96 | -2 | cdiGMP |
| 1418424_at Tnfaip6 | 29 | 1.141 | 1.631 | 1.189 | -0.66 | -0.69 | -0.7 | -0.66 | -0.67 | -0.58 | 3.961 | -2.05 | -1.91 | cdiGMP |
| 1425837_a_at Ccrn4l | 29 | 1.34 | 1.091 | 1.529 | -0.79 | -0.65 | -0.67 | -0.65 | -0.73 | -0.47 | 3.961 | -2.11 | -1.85 | cdiGMP |
| 1421473_at Il1a | 34 | 1.525 | 1.178 | 1.257 | -0.76 | -0.72 | -0.76 | -0.7 | -0.41 | -0.6 | 3.96 | -2.25 | -1.71 | cdiGMP |
| 1422305_at Ifnb1 | 29 | 0.997 | 1.431 | 1.532 | -0.67 | -0.66 | -0.65 | -0.66 | -0.67 | -0.66 | 3.959 | -1.97 | -1.99 | cdiGMP |
| 1427736_a_at Ccrl2 | 36 | 1.328 | 1.075 | 1.554 | -0.48 | -0.63 | -0.62 | -0.75 | -0.75 | -0.72 | 3.958 | -1.74 | -2.22 | cdiGMP |
| 1448325_at Ppp1r15a | 29 | 1.314 | 1.203 | 1.441 | -0.74 | -0.78 | -0.81 | -0.66 | -0.64 | -0.34 | 3.957 | -2.32 | -1.63 | cdiGMP |
| 1450297_at Il6 | 29 | 1.437 | 0.989 | 1.53 | -0.69 | -0.69 | -0.69 | -0.65 | -0.64 | -0.59 | 3.956 | -2.07 | -1.88 | cdiGMP |
| 1451340_at Arid5a | 37 | 1.5 | 1.292 | 1.161 | -0.57 | -0.61 | -0.49 | -0.84 | -0.56 | -0.87 | 3.953 | -1.67 | -2.28 | cdiGMP |
| 1417262_at Ptgs2 | 34 | 1.507 | 1.214 | 1.232 | -0.83 | -0.79 | -0.79 | -0.57 | -0.53 | -0.44 | 3.953 | -2.41 | -1.55 | cdiGMP |
| 1419534_at Olr1 | 29 | 1.484 | 1.094 | 1.375 | -0.81 | -0.75 | -0.79 | -0.53 | -0.46 | -0.6 | 3.953 | -2.35 | -1.6 | cdiGMP |
| 1439981_at Tespa1 | 35 | 1.343 | 1.427 | 1.18 | -0.76 | -0.81 | -0.58 | -0.38 | -0.57 | -0.85 | 3.95 | -2.15 | -1.8 | cdiGMP |
| 1419132_at Tlr2 | 34 | 1.576 | 1.128 | 1.246 | -0.42 | -0.66 | -0.78 | -0.74 | -0.6 | -0.75 | 3.95 | -1.86 | -2.09 | cdiGMP |
| 1433508_at Klf6 | 37 | 1.271 | 1.413 | 1.263 | -0.87 | -0.64 | -0.91 | -0.54 | -0.6 | -0.39 | 3.947 | -2.41 | -1.54 | cdiGMP |
| 1458589_at --- | 37 | 1.137 | 1.339 | 1.469 | -0.76 | -0.65 | -0.95 | -0.61 | -0.43 | -0.55 | 3.945 | -2.36 | -1.59 | cdiGMP |
| 1417263_at Ptgs2 | 34 | 1.51 | 0.965 | 1.47 | -0.73 | -0.73 | -0.71 | -0.64 | -0.59 | -0.53 | 3.945 | -2.18 | -1.77 | cdiGMP |
| 1428027_at LOC100653389 | 26 | 1.302 | 1.534 | 1.106 | -0.51 | -0.4 | -0.81 | -0.7 | -0.78 | -0.74 | 3.943 | -1.72 | -2.22 | cdiGMP |
| 1428735_at Cd69 | 40 | 1.22 | 1.344 | 1.378 | -0.68 | -0.43 | -0.41 | -0.93 | -0.66 | -0.83 | 3.941 | -1.52 | -2.42 | cdiGMP |
| 1457944_at --- | 35 | 1.366 | 1.428 | 1.146 | -0.83 | -0.54 | -0.41 | -0.95 | -0.57 | -0.65 | 3.94 | -1.78 | -2.16 | cdiGMP |
| 1439349_at Sbno2 | 29 | 1.367 | 1.407 | 1.166 | -0.45 | -0.8 | -0.85 | -0.86 | -0.48 | -0.49 | 3.94 | -2.1 | -1.84 | cdiGMP |
| 1448724_at Cish | 37 | 1.476 | 1.357 | 1.106 | -0.69 | -0.91 | -0.79 | -0.48 | -0.65 | -0.42 | 3.94 | -2.38 | -1.56 | cdiGMP |
| 1419508_at Ripk1 | 37 | 1.035 | 1.437 | 1.467 | -0.54 | -0.5 | -0.85 | -0.69 | -0.51 | -0.84 | 3.938 | -1.9 | -2.04 | cdiGMP |
| 1445534_at --- | 35 | 1.345 | 1.515 | 1.078 | -0.35 | -0.71 | -0.87 | -0.61 | -0.67 | -0.73 | 3.938 | -1.93 | -2 | cdiGMP |
| 1444010_at Eif4e | 29 | 1.659 | 1.049 | 1.227 | -0.79 | -0.61 | -0.6 | -0.78 | -0.51 | -0.66 | 3.936 | -1.99 | -1.94 | cdiGMP |
| 1424638_at Cdkn1a | 37 | 1.624 | 1.232 | 1.074 | -0.62 | -0.81 | -0.78 | -0.76 | -0.46 | -0.5 | 3.93 | -2.22 | -1.71 | cdiGMP |
| 1444598_at --- | 35 | 1.559 | 1.318 | 1.053 | -0.84 | -0.43 | -0.67 | -0.49 | -0.84 | -0.67 | 3.93 | -1.93 | -2 | cdiGMP |
| 1455899_x_at Socs3 | 29 | 1.206 | 1.544 | 1.18 | -0.71 | -0.6 | -0.83 | -0.89 | -0.34 | -0.56 | 3.93 | -2.14 | -1.79 | cdiGMP |
| 1439680_at Tnfsf10 | 37 | 1.169 | 1.36 | 1.399 | -0.59 | -0.29 | -0.78 | -0.63 | -0.68 | -0.97 | 3.927 | -1.65 | -2.27 | cdiGMP |
| 1421457_a_at Samsn1 | 29 | 1.437 | 1.192 | 1.298 | -0.39 | -0.59 | -0.9 | -0.87 | -0.4 | -0.77 | 3.927 | -1.88 | -2.04 | cdiGMP |
| 1457888_at --- | 36 | 1.466 | 1.08 | 1.38 | -0.81 | -0.3 | -0.79 | -0.63 | -0.84 | -0.54 | 3.925 | -1.91 | -2.02 | cdiGMP |
| 1419721_at Niacr1 | 27 | 1.493 | 1.403 | 1.03 | -0.43 | -0.74 | -0.87 | -0.54 | -0.49 | -0.84 | 3.925 | -2.05 | -1.88 | cdiGMP |
| 1449311_at Bach1 | 29 | 1.178 | 1.558 | 1.189 | -0.83 | -0.72 | -0.41 | -0.78 | -0.8 | -0.39 | 3.924 | -1.95 | -1.97 | cdiGMP |
| 1443414_at C78513 | 35 | 1.402 | 0.963 | 1.557 | -0.7 | -0.77 | -0.44 | -0.5 | -0.83 | -0.69 | 3.922 | -1.91 | -2.02 | cdiGMP |
| 1449169_at Has2 | 36 | 1.306 | 1.158 | 1.458 | -0.47 | -0.39 | -0.81 | -0.99 | -0.52 | -0.75 | 3.922 | -1.67 | -2.25 | cdiGMP |
| 1422054_a_at Skil | 40 | 1.491 | 1.218 | 1.211 | -0.38 | -0.83 | -1.02 | -0.51 | -0.59 | -0.59 | 3.92 | -2.23 | -1.69 | cdiGMP |
| 1446245_at --- | 35 | 1.239 | 1.22 | 1.46 | -0.6 | -0.31 | -0.67 | -0.56 | -1.04 | -0.75 | 3.919 | -1.58 | -2.34 | cdiGMP |

DMXAA preferentially induced RNA processing and gene expression responses more than cdiGMP or cGAMP (Figure **4f**). Analyses indicated that cdiGMP induced more interferon responses in vivo than the other sting ligands. This may be related to the fact that cdiGMP is produced by pathogenic bacteria (Woodward, J.J. et al., Science 328, 1703-1705 (2010)).

(Systemic gene responses are associated with adjuvant-induced hematological changes)

Many adjuvants affect the numbers of a variety of circulating blood cells. Granulocytosis was the most common event after administrating any of the 21 adjuvants (Figure **5a**), a finding consistent with the fact that neutrophils were the cells that responded most to the adjuvant administration (Figure **12**). Monocytosis was the next most common event, and was clearly induced by bCD, cdiGMP, cGAMP, MPLA, and Pam3CSK4 (Figure **5a**). Lymphopenia was less common but strongly induced by FK565, MALP2s, PolyIC, and R848 (Figure **5a**), and these adjuvants also caused leukopenia (Figure **5a**). Interestingly, FK565 caused granulocytosis (as was observed in a previous study (Tanaka, M. et al., Bioscience, Biotechnology, and Biochemistry 57, 1602-1603 (1993))) and lymphopenia at the same time (Figure **5a**). Next, correlations between these hematological parameters and the gene expression changes in the organs were examined using a linear model (see http://sysimg.ifrec.osaka324u.ac.jp/adjvdb/methodologies/he ma.html for the scheme). A variety of genes were retrieved for each blood cell type in each organ by this approach (Figure **5b** and Table 22). Based on the correlated gene numbers, LVs exhibited the most hematological changes (Figure **5b**). As a representative example, Cxcl9 upregulation in LVs, which is strongly induced by interferons and has been reported as an influenza vaccine safety marker (Mizukami, T. et al., Vaccine 26, 2270-2283 (2008) and Mizukami, T. et al., PloS one 9, e101835 (2014))) was clearly correlated with leukopenia (Figure **19a**). Downregulated examples include Il7 and S1pr5 in SP. These were also correlated with lymphopenia (Figure **5b** and Figures **19b** and **19c**). Contrastingly, no correlation was found for genes involved in monocytosis and granulocytosis (Figure **5b** and Table 22), suggesting that multiple genes are involved in these processes. These results suggest that the number of granulocytes, monocytes, and lymphocytes in the blood is regulated by different mechanisms, and gene changes in the systemic organs may lead to adjuvant-induced leukopenia and lymphopenia, possibly through interferons, Il7, and S1P related mechanisms.

[Table 22]

| Probe_set_id | Gene.Symbol | Gene.Title | pos.bd | coef.sig | incline | ttest | org |
|---|---|---|---|---|---|---|---|
| 1455320_at | Nampt | nicotinamide phosphoribosyltransferase | WBC | -0.9402 | -1.1902 | 0.03066 | LV |
| 1433883_at | Tpm4 | tropomyosin 4 | WBC | -0.8228 | -1.1881 | 0.00937 | LV |
| 1452352_at | Ctla2b | cytotoxic T lymphocyte-associated protein 2 beta | WBC | -0.9913 | -1.1869 | 0.01334 | LV |
| 1434512_x_at | Srsf3 | serine/arginine-rich splicing factor 3 | WBC | -0.9445 | -1.1848 | 0.03175 | LV |
| 1426324_at | H2-D1 /// H2-L | histocompatibility 2, D region locus 1 /// histocompatibility 2, D region locus L | WBC | -0.8181 | -1.1798 | 0.01491 | LV |
| 1416927_at | Trp53inp1 | transformation related protein 53 inducible nuclear protein 1 | WBC | -0.9651 | -1.1756 | 0.04645 | LV |
| 1460197_a_at | Steap4 | STEAP family member 4 | WBC | -0.9749 | -1.1602 | 0.0008 | LV |
| 1415993_at | Sqle | squalene epoxidase | WBC | -0.9363 | -1.1598 | 0.00334 | LV |
| 1439448_x_at | Tmed9 | transmembrane emp24 protein transport domain containing 9 | WBC | -0.9289 | -1.1579 | 0.02781 | LV |
| 1418536_at | H2-Q7 /// H2-Q9 | histocompatibility 2, Q region locus 7 /// histocompatibility 2, Q region locus 9 | WBC | -0.9853 | -1.1574 | 0.04534 | LV |
| 1426708_at | Antxr2 | anthrax toxin receptor 2 | WBC | -0.8664 | -1.1562 | 0.0269 | LV |
| 1428070_at | Syvn1 | synovial apoptosis inhibitor 1, synoviolin | WBC | -0.9785 | -1.1542 | 0.04024 | LV |
| 1416234_at | Lrrc59 | leucine rich repeat containing 59 | WBC | -0.9545 | -1.1527 | 0.02457 | LV |
| 1449111_a_at | Grb2 | growth factor receptor bound protein 2 | WBC | -0.8924 | -1.1491 | 0.03008 | LV |
| 1434372_at | AW112010 | expressed sequence AW112010 | WBC | -0.9301 | -1.1422 | 0.02126 | LV |
| 1431295_a_at | Stx18 | syntaxin 18 | WBC | -0.9956 | -1.1413 | 0.0306 | LV |
| 1431644_a_at | Ica1 | islet cell autoantigen 1 | WBC | -0.9308 | -1.138 | 0.02401 | LV |
| 1437495_at | Mbtps2 /// Yy2 | membrane-bound transcription factor peptidase, site 2 /// Yy2 transcription factor | WBC | -0.9064 | -1.1365 | 0.04603 | LV |
| 1450697_at | Slc30a7 | solute carrier family 30 (zinc transporter), member 7 | WBC | -0.8352 | -1.1314 | 0.0456 | LV |
| 1416250_at | Btg2 | B cell translocation gene 2, anti-proliferative | WBC | -0.9746 | -1.118 | 0.00496 | LV |
| 1455133_s_at | Suco | SUN domain containing ossification factor | WBC | -0.8611 | -1.117 | 0.01699 | LV |
| 1423192_at | Pspc1 | paraspeckle protein 1 | WBC | -0.8284 | -1.1079 | 0.04267 | LV |
| 1455892_x_at | --- | --- | WBC | -0.8722 | -1.1071 | 0.03373 | LV |
| 1428579_at | Fmnl2 | formin-like 2 | WBC | -0.9752 | -1.1023 | 0.0179 | LV |
| 1431146_a_at | Cpne8 | copine VIII | WBC | -0.9789 | -1.0864 | 0.0198 | LV |
| 1431339_a_at | Efhd2 | EF hand domain containing 2 | WBC | -0.8356 | -1.0829 | 0.038 | LV |
| 1416530_a_at | Pnp | purine-nucleoside phosphorylase | WBC | -0.9973 | -1.0796 | 0.0271 | LV |
| 1449328_at | Ly75 | lymphocyte antigen 75 | WBC | -0.9881 | -1.0789 | 0.01909 | LV |
| 1417612_at | Ier5 | immediate early response 5 | WBC | -0.8485 | -1.0647 | 0.01377 | LV |
| 1460351_at | Gm12854 /// Gm5068 /// S100a11 | predicted gene 12854 /// predicted gene 5068 /// S100 calcium binding protein A11 (calgizzarin) | WBC | -0.9532 | -1.0386 | 0.00772 | LV |
| 1433897_at | AI597468 | expressed sequence AI597468 | WBC | -0.9123 | -1.0322 | 0.04206 | LV |
| 1416926_at | Trp53inp1 | transformation related protein 53 inducible nuclear protein 1 | WBC | -0.9777 | -1.0303 | 0.02814 | LV |
| 1448550_at | Lbp | lipopolysaccharide binding protein | WBC | -0.8689 | -1.0246 | 0.01525 | LV |
| 1451335_at | Plac8 | placenta-specific 8 | WBC | -0.8843 | -1.0201 | 0.04035 | LV |
| 1418674_at | Osmr | oncostatin M receptor | WBC | -0.9967 | -1.0195 | 0.00976 | LV |
| 1417190_at | Nampt | nicotinamide phosphoribosyltransferase | WBC | -0.9854 | -1.0187 | 0.02931 | LV |
| 1453128_at | Lyve1 | lymphatic vessel endothelial hyaluronan receptor 1 | WBC | -0.9471 | -1.0159 | 0.01434 | LV |
| 1417303_at | Mvd | mevalonate (diphospho) decarboxylase | WBC | -0.8166 | -1.0028 | 0.02977 | LV |
| 1451776_s_at | Hopx | HOP homeobox | WBC | -0.9922 | -1.0017 | 0.04008 | LV |
| 1429502_at | Hspa13 | heat shock protein 70 family, member 13 | WBC | -0.8317 | -0.9964 | 0.04005 | LV |
| 1427578_a_at | Eif6 | eukaryotic translation initiation factor 6 | WBC | -0.8366 | -0.9859 | 0.04884 | LV |
| 1443609_s_at | Syvn1 | synovial apoptosis inhibitor 1, synoviolin | WBC | -0.9848 | -0.9838 | 0.04002 | LV |
| 1451532_s_at | Steap1 | six transmembrane epithelial antigen of the prostate 1 | WBC | -0.9926 | -0.9769 | 0.02028 | LV |
| 1425106_a_at | LOC101056688 /// Wars | tryptophan--tRNA ligase, cytoplasmic-like /// tryptophanyl-tRNA synthetase | WBC | -1 | -0.9743 | 0.042 | LV |
| 1457035_at | AI607873 | expressed sequence AI607873 | WBC | -0.9246 | -0.9626 | 0.03115 | LV |
| 1429379_at | Lyve1 | lymphatic vessel endothelial hyaluronan receptor 1 | WBC | -0.9865 | -0.9603 | 0.01806 | LV |
| 1448632_at | Psmb10 | proteasome (prosome, macropain) subunit, beta type 10 | WBC | -0.9753 | -0.9536 | 0.02755 | LV |
| 1434813_x_at | LOC101056688 /// Wars | tryptophan--tRNA ligase, cytoplasmic-like /// tryptophanyl-tRNA synthetase | WBC | -0.9831 | -0.9257 | 0.04759 | LV |
| 1433428_x_at | Tgm2 | transglutaminase 2, C polypeptide | WBC | -0.8966 | -0.9232 | 0.01276 | LV |

(AS04 characterization in the adjuvant gene space)

Finally, the flexibility of the adjuvant gene space approach was tested by investigating another test adjuvant, AS04 (Didierlaurent, A.M. et al., Journal of immunology 183, 6186-6197 (2009)). AS04 consists of aluminum hydroxide and 3-O-desacyl-4'-monophosphoryl lipid A, which is utilized in Cervarix, a vaccine against human papillomavirus 16/18. AS04, the buffer control, and alum-only control were administered i.d. to the mice, and their hematological parameters and organ gene expressions were examined (see Experiment 11).
Experiment 11 is the following.

[Table 23]

| Substance used in the test and dosage thereof | | | | | |
|---|---|---|---|---|---|
| Group No. | Test substance | Route of administration | Dose | Concentration | Volume dosage |
| 1 (Control) | AS04Buffer (GSK) | i.d. | 0mg | 150mM NaCl | 100μl |
| 2 | AS04 (GSK) | i.d. | 0.1mg+0.01mg | 1mg/mL of alum, 100μg/mL of MPL | 100μl |
| 3 | Alum (GSK) | i.d. | 0.1mg | 1mg/mL | 100μl |
| *All groups included three mice.<br>*Samples were collected after 6 hours from administration. | | | | | |

(Hematology (associated with Figure **5a**))

The effect of adjuvant administration with respect to the changes in several hematological parameters was investigated. The following parameters were studied: white blood cells (WBC), lymphocytes (LYM), monocytes (MON), granulocytes (GRA), relative (%) content of lymphocytes (LY%), relative (%) content of monocytes (MO%), relative (%) content of granulocytes (GR%), red blood cells (RBC), hemoglobins (Hb, HGB), hematocrits (HCT), mean corpuscular volume (MCV), mean corpuscular hemoglobins (MCH), mean corpuscular hemoglobin concentration (MCHC), red blood cell distribution width (RDW), platelets (PLT), platelet concentration (PCT), mean platelet volume (MPV), and platelet distribution width (PDW). Data was obtained for the items shown in Figure **5**.
The volcano plot (associated with Figure **7**): format is explained. The x and y axes correspond to the log (fold change) and log(p-value), respectively.
Venn diagram (associated with Figure **8**) for three mice: format is explained. Upregulated gene probes are determined from three individual mice. The three individuals are denoted as x1, x2 and x3, respectively.
Analysis using sDEGs (FC > 1.5 or < 0.67, p < 0.01, cPA = 1)

a. The number of sDEGs is the following.

[Table 24]

| | Up | | | Down | | |
|---|---|---|---|---|---|---|
| | **LV** | **SP** | **LN** | **LV** | **SP** | **LN** |
| **ALM_gsk** | 35 | 13 | 10 | 20 | 29 | 20 |
| **AS04** | 88 | 71 | 53 | 33 | 16 | 43 |

b. Gene list of sDEGs

The gene differential expression is represented as log2(fold change). Gene count with non-zero values corresponds to the counts in the table above.

c. Lists of biological process given by sDEGs

The functional analysis is scored with -log(p-value). Thus, a function with a high score suggests that it is more significant than functions with a lower score.

Hematological assessment showed that AS04 increased monocyte and granulocyte levels in the blood which is similar to the MPLA hematological profile (Figure **5a**). After cluster analysis, the AS04-derived individual samples were closely connected to the FCA samples in $G2^{LV}$, the same group as that of bCD, ALM_IP and ENDCN (Figure **20a**). In SP, AS04 formed a new cluster neighboring G2, which again included bCD, ALM_IP, and ENDCN (Figure **20b**). AS04 was categorized in $G1^{LN}$, the group that represents most of the PAMP ligands besides CpG oligodeoxynucleotides. These data suggest that AS04-induced local responses are similar to many of the PAMP ligands categorized in $G1^{LN}$, but relatively mild systemic responses similar to G2 adjuvants were observed, including bCD, ALM_IP and ENDCN (Figure **20**), which is consistent with the fact that AS04 is a combination adjuvant of aluminum hydroxide and 3-O-desacyl-4'-monophosphoryl lipid A. The adjuvant groupings including AS04 are summarized in Figure **21**. Taken together, these results suggest that the adjuvant gene space provides a flexible but reliable and insightful profile of many different adjuvants.

(Discussion)

The results are discussed below. This Example studied a comprehensive organ transcriptome analysis of 21 different adjuvants administered to mice. The results showed that the adjuvant induced gene responses were influenced by many factors including the adjuvant type, the administration route, and the examined organ. Surprisingly, organs such as LV and SP, which are remote from the injection site and also considered as systemic organs, were also useful organs for characterizing the general mode of action of a given adjuvant. The data collected from LVs and SPs augmented or compensated local LN-derived information. Currently, many clinical vaccines are administered intramuscularly and subcutaneously. In addition, mucosal administration via the intranasal route is also practiced. With the nonparenteral administration route, it is sometimes difficult to sample the directly draining lymphoid tissues, and with ENDCN (Falkeborn, T. et al., PloS one 8, e70527 (2013) and Maltais, A.K. et al., Vaccine 32, 3307-3315 (2014)), that proved to be the case. Nevertheless, the transcriptomes of LV and SP revealed ENDCN as a potential DAMP-releasing adjuvant. ENDCN shared some similarity with bCD25 (Onishi, M. et al., Journal of immunology 194, 2673-2682 (2015)) with regard to its mode of action. Furthermore, remote organ transcriptome examination provided the biodistribution of the "adjuvant effect" in vivo (Figure **21**). Although organ transcriptome analysis cannot verify whether the adjuvant itself reached the organ or not, it can confirm that the organ responded to its administration. Consequently, the results indicated that many locally administered adjuvants have the potential to cause gene expression change in systemic organs that are remote from the injection site. However, remote organ gene expression after adjuvant administration does not necessarily imply organ toxicities, and the doses used for mice were generally overdosed for human applications on a per weight basis. Therefore, direct extrapolation of the results to humans also has a limitation. These points need be examined in future studies.

Another important observation with this approach involved the batch effect. It has been reported that many array- and Next Generation Sequencer-based comprehensive gene analysis methods can be disturbed by the batch effect (Leek, J.T. et al., Nat Rev Genet 11, 733-739 (2010)). A batch effect was observed in the data, but it turned out to be beneficial and a useful internal threshold in this approach. The batch effect discriminated whether the organs responded substantially or did not respond to adjuvant administration. If necessary and sufficient amount of microarray data could be obtained, batch effects would simply be reduced to "background noise" such as gene expression fluctuations. However, the 330 microarray data in this study were still not enough to determine the background noise levels without batch effect signatures. In this sense, this approach of examining three different adjuvants in one experiment is a simple and effective method to monitor the batch effect, which worked as a sensitive internal threshold for determining whether the gene expression was adjuvant-specific or not. Of all the adjuvants examined in this study, ALM was one of the least gene response-inducing adjuvants when it was administered locally. Similarly, MPLA, D35, K3, K3SPG and AddaVax acted on local LNs but not in the systemic remote organs, suggesting their local action tendencies (Figure **21**). Other adjuvants induced detectable levels of gene responses in LV and SP (Figure **21**), but, as mentioned above, this does not directly mean the organ toxicities, and further study is required for their interpretations.

To construct the adjuvant gene space, strict data Quality Check (QC) protocol was followed (standard procedure 3). This strict protocol forced discarding of some data and necessitated the creation of a virtual control as a data replacement for QC failed samples. However, concurrently, this strict data processing policy enabled observation of a clear batch effect and, more importantly, "self-organizing" adjuvant clusters, which consisted of the batch effect and host response-based clusters without any tagging or subjective instructions. Furthermore, the addition of AS04 as a test adjuvant to the data analysis did not change the overall adjuvant cluster structures, thereby enabling a relative comparison among the other adjuvants, and confirming the dynamic flexibility for characterizing additional new adjuvants of the

inventor's database.

Taken together, the results suggest that this approach can provide a useful platform for evaluating preclinical and clinical adjuvants in a comprehensive and objective manner. The flexibility of this approach also allows for further improvement in the future by depositing data sets from new adjuvants, additional variations in administration, or by including antigen data. In this study, 21 adjuvants were examined at only 6-hour time-points without antigens. Future studies with other time points and antigens, and in silico integration of the publicly available vaccine adjuvant related immunological signatures (Knudsen, N.P. et al., Scientific reports 6, 19570(2016)) are required to understand adjuvanted vaccine mechanisms in more detail. In parallel, the inventors acquired analogous microarray data in rats to evaluate toxicological aspects of adjuvants (see Example 4). The rat transcriptome data can be integrated directly with the toxicogenomics datasets from open TG-GATEs (Igarashi, Y. et al., Nucleic acids research 43, D921-927 (2015)) (which is also in rats). Furthermore, the inventors collected micro RNA expression profiles from human clinical samples (see Example 4). Integration of all the aforementioned datasets demonstrated that the databases enable more integrated and comprehensive analyses on adjuvant-induced gene expression signatures from different species under different experimental settings.

(Example 2)

This Example carries out a method of classifying substances with an unknown adjuvant function using the method of the invention.

Appropriate candidate substances are provided as the substances.

Adjuvant administration, gene marker expression analysis, clustering, other data analysis and the like are performed in accordance with Example 1.

Candidate substances and reference adjuvants (G1) dciGMP, cGAMP, DMXAA, PolyIC, and R848; (G2) bCD; (G3) FK565; (G4) MALP2s; (G5) D35, K3, and K3SPG; and (G6) AddaVax) are clustered.

(Results)

The transcriptome of candidate substances when administered to murine spleen, liver, or the like and the transcriptome of reference adjuvants of G1 to G6 are compared, and substances classified to the same cluster are each classified to G1 to G6.

(Example 3: Adjuvant of adjuvant)

(Materials and methods)

(Mice)

Six-week-old female C57BL/6J mice were purchased from CLEA Japan. $Tlr7^{-/-}$ or $Il\text{-}1r^{-/-}$ mice were purchased from Oriental BioService and the Jackson Laboratory, respectively. $Card9^{-/-}$ (Hara et al., 2007, Nature immunology 8, 619-629.), $Fcrg^{-/-}$ (Arase et al., 1997, J Exp Med 186, 1957-1963.) or $Dap12^{-/-}$ (Takai et al., 1994, Cell 76, 519-529.) mice were donated by Dr. Hara, Dr. Saito, or Dr. Takai, respectively. $Tnfa^{-/-}$ mice were described previously ((Marichal et al., 2011, Nature medicine 17, 996-1002.). All animal experiments were approved by the Institutional Animal Care and Use Committee, and performed in accordance with institutional guidelines for the National Institute of Biomedical Innovation, Health and Nutrition animal facility.

(Antigens, antibodies, adjuvants and peptides)

Ovalbumin was purchased from Seikagaku-kogyo. SV and inactivated WV derived from A/New Caledonia/20/99 strain were a gift from the Institute of Microbial Chemistry (Osaka, Japan). CpG-ODN (5'-ATCGACTCTCGAGCGTTCTC-3' = SEQ ID NO: 1) was synthesized by GeneDesign (Osaka, Japan). CpG-SPG was prepared as previously reported Kobiyama et al., 2014, Proceedings of the National Academy of Sciences of the United States of America 111, 3086). Alum was purchased from Sigma. Advax™ and hepatitis B surface antigen (HBs) were provided by Vaxine Pty Ltd. LPS was purchased from Sigma. MHC Class I (ASNENMETM = SEQ ID NO: 2) and class II (ARSALILRGSVAHKSCLPACVYGP = SEQ ID NO: 3) epitope peptides of nucleoprotein were synthesized by Operon Biotechnologies. Cytokine ELISA kits for IFN-$\gamma$, IL-13, IL-17, TNF-$\alpha$ and IL-1$\beta$ were purchased from R&D Systems.

(Immunization)

C57BL/6J mice were immunized twice either intramuscularly (i.m.) or i.d. with 2 week intervals (days 0 and 14). For antigen-specific ELISA, blood samples were taken on days 14 and 28. During vaccination and blood collection, mice were anesthetized with ketamine. Alum-adjuvanted antigen was rotated for more than one hour before immunization. Advax™, alum, and CpG-SPG were used for immunization at 1 mg per mouse, 0.67 mg per mouse, and 10 ug per mouse, respectively.

(Antibody titers)

For ELISA, 96-well plates were coated with 1 μg/ml SV in carbonate buffer (pH 9.6) for SV- and WV-vaccinated groups, 10 ug/ml OVA for OVA-vaccinated groups, and 1 μg/ml HB for HB-vaccinated groups. Wells were blocked with PBS containing 1% bovine serum albumin and diluted sera from immunized mice were incubated on the antigen-coated plate. After washing, goat anti-mouse total IgG, IgG1 or IgG2c conjugated with horseradish peroxidase (Southern Biotech) was added and incubated for 1 h at room temperature. After additional washing, the plates were incubated with TMB substrate for 30 min, the reaction was stopped with 1N $H_2SO_4$, and then the absorbance was measured. Antibody titers were calculated. OD of 0.2 was set as the cut-off value for positive samples. The concentration of total IgE in serum was measured by a total IgE ELISA kit (Bethyl).

(Measurement of antigen-specific cytokine responses)

Two weeks after the second immunization, spleens were collected from mice. $1\times10^6$ splenocytes were plated on 96-well plates and stimulated with MHC class I or II epitope peptides of nucleoprotein. Two days after stimulation, IFN-γ, IL-13, and IL-17 in supernatant were measured by ELISA.

(Cytokine production profile)

At several time points after i.p. injection of adjuvant, mice were sacrificed and peritoneal lavage fluids were collected. The cytokines in the fluids were measured by Bio-plex (BioRad).

(Activation of DCs)

For in vitro experiments, bone marrow-derived DCs were generated by cultivation of bone marrow cells in RPMI 1640 supplemented with 10% fetal bovine serum (FBS), 1% Penicillin/Streptomycin solution (Naclai Tesque) and 100 ng/ml of human fms-like tyrosine kinase 3 ligand (Flt3L) (PeproTech) for 7 days, stimulated with 1 mg/ml alum, 1 mg/ml Advax™, or 50 ng/ml LPS (Sigma) for 15 h and then CD40 expression on plasmacytoid DCs (pDCs) was evaluated by FACS. We defined pDC as $CD11c^+$/ $SiglecH^+$ cells.

In vivo experiments were performed as described previously (Kobiyama et al., 2014, Proceedings of the National Academy of Sciences of the United States of America 111, 3086.). Briefly, C57BL/6J mice were injected with 0.67 mg alum, 1 mg Advax™, or 50 ng LPS at the base of tail. Twenty-four hours after the injection, draining lymph nodes were removed, treated with DNaseI and collagenase for 30 min, then stained with anti-mCD11c (N418), mCD8α (56-6.7), mPDCA-1 (JF05-1C2.4.1), mCD40 (3/23) antibodies and 7AAD, and analysed by FACS. pDC was defined as $CD11c^+$/$mPDCA\text{-}1^+$ cells, $CD8\alpha^+$DC as $CD11c^+$/$CD8\alpha^+$ cells, and $CD8\alpha^-$DC as $CD11c^+$/$CD8\alpha^-$/$mPDCA\text{-}1^-$ cells.

(In vitro stimulation of macrophages and GM-DCs)

For macrophage preparation, mice were i.p. injected with 3 ml of 4% (w/v) thioglycolate (Sigma) solution. Four days later, macrophages were collected from the peritoneal cavity and plated on 96-well plates. Macrophages were primed with 50 ng/ml LPS for 18 h, and stimulated with adjuvants for 8 h. IL-1β in supernatants was measured by ELISA. TNF-α in supernatants was measured by ELISA after stimulation with Advax™ or alum without priming by LPS. For GM-DC preparation, mouse bone marrow cells were cultured in RPMI 1640 supplemented with 10% FBS, 1% Penicillin/Streptomycin solution, and 20 ng/ml mouse GM-CSF (PeproTech) for 7 days.

GM-DCs were collected, plated on 96-well plates, primed with 50 ng/ml LPS for 18 h and then stimulated with adjuvants for 8 h. IL-1β in supernatants was measured by ELISA.

(Two photon microscopy analysis)

Biotinylated delta inulin particles (1 mg) were premixed with Brilliant Violet 421 Streptavidin (BioLegend), and

then administered i.d. at the tail base of mice. At 30 min before inguinal LN removal, mice were i.d. administered anti-MARCO-phycoerythrin or anti-CD169-FITC antibodies. Distributions of Advax™ particles in the inguinal lymph nodes were examined by two-photon excitation microscopy (FV1000MPE; Olympus, Tokyo, Japan).

(Clodronate liposome injection)

Clodronate liposome (FormMax) was administered to the base of the tail of mice either 7 or 2 days before immunization. Mice were immunized with WV (1.5 ug) + adjuvant at the base of the tail on days 0 and 14. The day -2 clodronate treatment depleted both macrophages and DCs on day 0. Day -7 treatment depleted macrophages, but DCs were already recovered on day 0. Blood samples were taken on days 14 and 28, and serum antibody titers were measured by ELISA.

(Microarray analysis)

Six hours after administration of adjuvant, the spleen, lung, kidney, and liver were removed (n = 3), and total RNAs were extracted as described previously Onishi et al., 2015, Journal of immunology 194, 2673-2682). After total RNA preparation, the gene expression profiles were obtained using 3' IVT Express Kit and GeneChip Mouse Genome 430 2.0 Array (Affymetrix). The expression values were normalized by the median value of each GeneChip. The resulting digital image files were preprocessed using the Affymetrix Microarray Suite version 5.0 algorithm (MAS5.0). The differential expression was computed as the ratio between the mean of the treated samples and control samples. The presence and absence (PA) call in MAS5.0 was further customized as follows. When the ratio is > 1, the PA call is dependent on treated samples. When the ratio is < 1 or = 1, the call is dependent on control vehicle samples. Dominant calls (over half) were applied to a set of samples (e.g. when the ratio < 1 and PA call of control samples are "P", "P", and "A", then the customized PA call of the set is "1"). The MAS5.0 and PA calls analysis were conducted using the Bioconductor Affy package for R (http://www.bioconductor.org). The p-values for the significance of differentially expressed genes were calculated by using t-test between the normalized treated samples and the normalized vehicle samples. For subsequent analyses, only probes where the fold-change between control and stimulated samples was > 2 were used. Probes that were Absent-flagged, i.e., PA call was "0", were excluded.

(Cell population analysis)

The cell population analysis was carried out as follows. The gene expression profile of various immune cell types from a steady state condition were obtained directly from the ImmGen database (http://www.immgen.org/). These expression profiles were used to estimate the cell type origin of the genes. Each gene (i) in all ten immune cell types (j) was first weighted as:

[Numeral 3]

$$\omega_{ij} = \frac{e_{ij}}{\sum_{j=1}^{10} e_{ij}}$$

It is assumed that the weight of a cell type of a given gene depended only on the expression level of that gene in that particular cell type, independent from the expression in other cell types. Given the expression profile of each adjuvant sample, the genes were determined based on the fold change (FC > 2) and PA-call threshold (PA = 1). Finally, the cell type contribution for sample (k) for cell type (j) was calculated as:

[Numeral 4]

$$S_{jk} = \frac{\sum_i S_{ik}\omega_{ij}}{\sum_i S_i\omega_{ij} + C}$$

where c is the pseudocount. In Figure **6b**, the score is represented as the thickness of the ribbon.

(Upstream regulator analysis in IPA)

Upstream analysis was performed using the IPA Regulator Effects feature. The main purpose was to elucidate the upstream regulatory mechanism and their connection to downstream functional impact. For this analysis, genes with fold change > 2 and PA-call = 1 were selected. Finally reported networks had a P-value < 0.001.

(Statistical analysis)

Statistical significance (P < 0.05) between groups was determined by Dunnett's multiple comparison test or Student t-test.

(Results)

(Advax™ adjuvant enhances Th2 responses when combined with a Th2-type antigen)

To understand the adjuvant effect of Advax™, immune responses in mice immunized with Advax™ plus influenza split vaccine (SV), an antigen previously shown to elicit Th2 immune responses, were first examined (Kistner et al., 2010, PloS one 5, e9349). SV immunization alone elicited IgG1 production (a Th2-type IgG subclass) and at higher immunization doses induced IL-13 (Th2-type cytokine) production, consistent with it being a Th2-inducing antigen (Figures **22A** to **22C**). The addition of Advax™ to SV enhanced IgG1 but not IgG2c antibody production, which is prominent with lower (0.015 and 0.15) antigen dose (Figures **22A** to **22C**). Alum, a typical Th2 type adjuvant commonly used for human vaccination also enhanced IgG1 dominant antibody responses. Advax™, similar to alum, induced Th2 responses to SV antigen. Alum is also known to induce IgE production, potentially increasing the risk of vaccine allergy (Nordvall, 1982, Allergy 37, 259-264). Therefore, IgE-levels in sera were measured after SV immunization with either Advax™ or alum. While alum significantly increased IgE production in a dose dependent manner, Advax™ did not induce IgE production (Figure **22D**), consistent with the observation that Advax™ only magnifies the antibody subtype response induced by the SV alone.

T-cell responses were examined by stimulating splenocytes from immunized mice with MHC class I (CD8 T cell) or class II (CD4 T cell) peptides from the influenza virus nucleoprotein. CD4 T cell IL-13 production was not significantly different in mice immunized with SV + Advax™ versus controls, although Advax™ enhanced the ability of SV to produce IgG1 subclass antibody. Then, alum exhibited significantly increased IL-13 production (Figures **22E** to **22G**). These results demonstrated that Advax™ functions as a Th2-type adjuvant when combined with SV, but Advax™ did not increased IL-13 and IgE compared with alum.

(Advax™ adjuvant enhances Th1 responses when combined with a Th1-type antigen)

Next, immune responses in mice immunized with inactivated whole virion influenza vaccine (WV) plus either Advax™ or alum were examined. This WV contains viral RNA that induces TLR7 activation and thereby acts as an endogenous adjuvant that induces Th1 responses (Koyama et al., 2010, Science translational medicine 2, 25ra24). Advax™ adjuvant only enhanced IgG2c (Th1-type subclass) production with minimal effects on IgG1 production, whereas alum suppressed the WV-induced IgG2c response and significantly increased IgG1 levels (Figures **23A** to **23D**). At the cytokine level, Advax™ enhanced IFN-$\gamma$ (Th1-type cytokine) production by CD4 and CD8 T cells when compared to mice immunized with WV alone. By contrast, alum suppressed IFN-$\gamma$ production, while significantly increasing IL-13 production by CD4 T cells (Figures **23E** to **23G**). These results demonstrated that Advax™ functioned as a Th1-type adjuvant when combined with WV, whereas alum functioned as a Th2-type adjuvant for both SV and WV.

(Advax™ adjuvant effect is shaped by endogenous adjuvant in the vaccine antigen)

It is understood from the above findings that the combination of Advax™ with a Th2-type antigen enhanced Th2 immunity, whereas its combination with a Th1-type antigen enhanced Th1 immunity. This led to a hypothesis that the adjuvant effect of Advax™ might be mediated by an effect on potentiating the inherent adjuvanticity encapsulated within each antigen. By contrast, other adjuvants have a fixed immune bias effect whereby, for example, alum always biases to Th2 responses and CpG-ODN always biases to Th1 responses, regardless of the innate properties of the antigen with which they are co-administered. To test this hypothesis, the adjuvant effect of Advax™ on ovalbumin (OVA) antigen, which is considered a neutral Th0-type antigen, was tested. Advax™ failed to enhance the OVA-specific antibody response, whereas alum, as expected, solely enhanced IgG1 production (Figure **24A**).

It was previously shown that the endogenous built-in adjuvant effect of WV was abolished in Tlr7-deficient mice, establishing the importance of TLR7 signalling in WV immunogenicity (Koyama et al., 2010, Science translational medicine 2, 25ra24.) Finally, the adjuvant effect of Advax™ on WV in Tlr7-deficient mice was tested. Notably, the enhanced WV-antibody response seen with Advax™ in wild type mice was lost in Tlr7-deficient mice, whereas the enhanced IgG1 response induced by alum was preserved in Tlr7-deficient mice (Figure **24B**). Taken together, these findings suggest that Advax™ belongs to a new additional class of adjuvant that functions as an amplifier of endogenous built-in adjuvants contained within vaccine antigens without changing their inherent immune-polarizing properties.

(Advax adjuvant activates dendritic cells in vivo but not in vitro)

Since dendritic cells (DCs) play a central role in adjuvant-induced immune responses, the ability of Advax™ to activate DCs, in vitro and in vivo, was investigated. Mouse bone marrow-derived DCs were stimulated with Advax™, alum or lipopolysaccharide (LPS) for 15 h in vitro and the expression of CD40, an activation marker on DCs, was evaluated by flow cytometry. While LPS expectedly increased CD40 expression on DCs in vitro, neither Advax™ nor alum influenced CD40 expression on DCs (Figures **25A** to **25C**). Next, the ability of Advax™ or alum to activate DC CD40 expression, in vivo, was investigated by measuring CD40 expression on DCs from draining lymph nodes following adjuvant administration. In contrast to the in vitro findings, both Advax™ and alum increased the frequency of activated DCs in draining lymph nodes when administered in vivo (Figures **25D** to **25F**). As alum induces extracellular DNA release from dead cells at the injection site (Marichal et al., 2011, Nature medicine 17, 996-1002.), this extracellular DNA via binding DAMP receptors likely explains how alum induced DC activation in vivo but not in vitro. Therefore, it was asked whether Advax™ might similarly be inducing cell death at the injection site and thereby indirectly activating DCs via DAMP receptors. To test this possibility, cytotoxicity and host DNA/RNA release at the injection site of Advax™ or alum were evaluated in vivo. After the intraperitoneal (i.p.) administration of Advax™ or alum, peritoneal lavage fluids were collected, and the number of dead cells and the concentration of DNA/RNA in these fluids were measured. Whereas alum induced cell death and nucleic acid release as expected, injection of Advax™ induced no cytotoxicity or nucleic acid release, indicating that DAMP signalling pathways are not involved in the ability of Advax™ to induce DC activation and CD40 expression, in vivo.

(Phagocytic macrophages are cellular mediator of the adjuvant effect of Advax™)

Although the immune complex and inactivated influenza virus are captured by $CD169^+$ (also called Siglec-1 or MOMA-1) macrophages in draining lymph node (DLN) to induce humoral immune responses (Gonzalez et al., 2010, Nature immunology 11, 427-434.; Suzuki et al., 2009, J Exp Med 206, 1485-1493.), some particulate adjuvants are efficiently taken up by $MARCO^+$ macrophages (Aoshi et al., 2009, European journal of immunology 39, 417-425.; Kobiyama et al., 2014, Proceedings of the National Academy of Sciences of the United States of America 111, 3086.). Thus, the behaviour of Advax™ in DLN was analyzed in vivo using fluorescent-labelled Advax™ particles. One hour after intradermal (i.d.) administration, a weak Advax™ signal co-localized with $MARCO^+$ macrophages in the DLN, with this co-localization signal being much higher 24 h later. Almost no co-localization of Advax™ with $CD169^+$ macrophages was observed in the DLN (Figures **26A** to **26F**), suggesting i.d. administered Advax™ is taken up by $MARCO^+$ macrophages. Next, to investigate the requirement of Advax™ uptake into macrophages for its adjuvant effect, the different recovery kinetics of macrophages and DCs following clodronate liposome injection was utilized. This depletes both macrophages and DCs completely by day 2 but subsequently, macrophages do not recover for at least 7 days whereas DCs are mostly recovered by this time (Aoshi et al., 2008, Immunity 29, 476-486.; Kobiyama et al., 2014, Proceedings of the National Academy of Sciences of the United States of America 111, 3086.). Interestingly, the adjuvant effect of Advax™ was significantly reduced with clodronate liposome treatment, irrespective of the time point tested (day 2 or 7) (Figures **26G** and **26H**), suggesting Advax™ adjuvanticity is dependent on the presence of macrophages. By contrast, the adjuvanticity of CpG-SPG was significantly reduced on day 2 post-clodronate treatment but not on day 7, indicating that the adjuvanticity of CpG-SPG was mostly dependent on DCs but not macrophages, as previously reported (Kobiyama et al., 2014, Proceedings of the National Academy of Sciences of the United States of America 111, 3086.)

(Advax™ alters the gene expression of IL-1$\beta$-, C-type lectin receptors- and TNF-$\alpha$-related signalling pathways)

To understand the biological properties of Advax™, its effect on cytokine production was investigated. At several time points after i.p. administration of Advax™ or alum, peritoneal lavage fluids were collected, and cytokines in the fluids were analysed. Alum induced the production of various cytokines including interleukin (IL)-5, IL-10, IL-12, tumor necrosis factor (TNF)-$\alpha$, and granulocyte-colony stimulating factor (G-CSF), whereas limited cytokine response such as IL-10, G-CSF, or macrophage inflammatory protein (MIP)-1 was observed by Advax™ administration.

To further explore the predominated biological effect of Advax™ in vivo, gene expression profiles were examined in tissues after local (i.d.) or systemic (i.p.) administration of Advax™. Advax™ administration via i.d. conferred limited gene expression changes, whereas i.p. administration altered gene expression in several tissues (Figure **27A**). Administration via i.p. resulted in differential regulation of genes associated with the acute phase response, inflammation, chemokines, complement/platelet, and C-type lectin receptor (CLRs)-related responses. Furthermore, analysis of the responding cell population revealed Advax™ induced neutrophil and macrophage responses in vivo (Figure **27B**). Additionally, upstream regulator analysis in Ingenuity pathway analysis (IPA) suggested that four upstream regulators: NF-κB (complex), IL-1β, IFN-γ, and TNF-α were affected by i.p. administration of Advax™. This drives the expression of genes that enhance phagocyte adhesion, neutrophil chemotaxis and movement of hematopoietic and natural killer cells (Figure **27C**).

To examine the contribution of these biological factors to the adjuvanticity of Advax™, the ability of Advax™ to induce IL-1β production was first invesigated. Peritoneal macrophages or granulocyte-macrophage colony stimulating factor (GM-CSF)-induced bone marrow-derived DCs (GM-DCs) were stimulated with Advax™ or alum following LPS-priming, and then IL-1β production was examined. However, IL-1β production was not detected in these cells stimulated with Advax™ in vitro, whereas alum significantly induced IL-1β production. Because some particulate adjuvants require NLRP3 inflammasome activation and subsequent IL-1β production for their adjuvanticity (Eisenbarth et al., 2008, Nature 453, 1122-1126.; Kuroda et al., 2013, Int Rev Immunol 32, 209-220.), the effect of the absence of the NLPR3 inflammasome components, Nlrp3, Caspase1, or IL-1r on Advax™ adjuvanticity was examined in vivo. Advax™ conferred significant adjuvant effects in each of these NLPR3 inflammasome deficient mice, indicating the adjuvant effect of Advax™ is independent of the NLPR3 inflammasome/IL-1β signalling pathway. Next, to examine the involvement of CLR-related signalling pathways in Advax™ adjuvanticity, its adjuvant effect was examined in mice lacking the CLRs signalling pathway related genes, Fcrg$^{-/-}$, Card9$^{-/-}$, or Dap12$^{-/-}$. Absence of these genes did not affect the ability of Advax™ to enhance antibody responses, indicating that Advax™'s adjuvant effect is independent of these CLR-related signalling pathways.

(TNF-α is essential for the adjuvant effect of Advax™)

Gene expression analysis also indicated that i.p. administration of Advax™ affected TNF-α-related signalling pathways (Figure **27C**). Thus, the ability of Advax™ to generate TNF-α production was examined. Macrophages were stimulated with Advax™ in vitro and TNF-α in the culture supernatant was measured. Advax™ did not induce TNF-α production, whereas stimulation with alum significantly induced macrophage TNF-α production in vitro (Figure **28A**). However, i.p. injection of Advax™ did result in significantly increased serum TNF-α levels (Figure **28B**), whereas paradoxically i.p. alum did not affect serum TNF-α levels. Because i.p. injection of Advax™ influenced gene expression including TNF-α-signalling pathway-related genes in remote tissues such as the lung and spleen (Figures **27A** and **27B**), TNF-α in the serum might be derived from these tissues. Finally, to examine the role of TNF-α in Advax™'s adjuvant effect, Tnfa$^{-/-}$ mice were immunized with Advax™-adjuvanted WV or hepatitis B surface antigen, and the resulting antibody responses were examined (Figures **28C** to **28E,** respectively). The absence of Tnfa significantly decreased the adjuvant effect of Advax™ on antibody responses, suggesting that intact TNF-α signalling is important for Advax™'s adjuvant effect on antibody responses.

(Example 4: Safety and efficacy model construction)

Next, this Example demonstrates that safety and efficacy databases and models can be constructed using the present invention.

Use of a toxicogenomic database (GATE) in addition to the adjuvant databases shown in Examples 1 to 2 enables transcriptome based toxicity and safety prediction by machine learning or the like.

(Toxicity prediction at 6 hours and 24 hours in rat liver transcriptome)

A "toxic" group" including 10 compounds and a "non-toxic" group including 10 compounds were each created from a toxicogenomic database. The compounds in the "toxic" group were compounds exhibiting pathological findings within 4 administrations. The compounds in the "non-toxic" group were compounds with no toxicity related feature observed (Figure **29**) (these data are available from publicly open TG-GATE (Igarashi, Y. et al., Nucleic acids research 43, D921-927 (2015)).

A pattern of differentially expressed genes by administration of a compound in the "toxic" group and a pattern of differentially expressed genes by administration of a compound in the "non-toxic" group were obtained based on results of transcriptome analysis after 6 hours and 24 hours from administration of the compound in the "toxic" group and the compound in the "non-toxic" group (these data are available from publicly open TG-GATE (Igarashi, Y. et al., Nucleic acids research 43, D921-927 (2015)). Probes (genes) selected for a necrosis prediction model were concentrated into 5 routes associated with immune response (1) and metabolism (4) (Figure **30A**).

The gene variation patterns as of 6 and 24 hours after each adjuvant administration in the adjuvant database

shown in Examples 1 to 2 were compared to the "toxic" gene pattern and "non-toxic" gene pattern as of 6 and 24 hours after administration of the known compounds to calculate the degree of "toxicity" score of each adjuvant (Figure **30B**). See, for example, Igarashi, Y. et al., Nucleic acids research 43, D921-927 (2015) and Uehara, T. et al., Toxicol Appl Pharmacol 2011 Sep 15; 255(3): 297-306 for "toxicity" score calculation methods.

Activity of ALT after 6 hours and 24 hours was measured by actually administering each adjuvant (Figure **31**). AST and ALT activities were measured by an approach known in the art (e.g., colorimetric measurement using a kinetics assay or endpoint assay). The data was commissioned to and measured by LSI Medience.

Toxicity of adjuvant X (FK565) expected to have high toxicity was actually examined in mice.

PBS and FK565 (1 μg/kg, 10 μg/kg, or 100 ug/kg) or LPS (1 mg/kg) were intraperitoneally administered to mice. After 3 hours/6 hours, blood and liver were collected on day 1, day 2, day 3, and day 5. Hematoxylin and eosin staining was applied to the collected liver and histologically analyzed (Figure **32**). Further, the liver was stained with TUNEL to examine apoptosis (Figure **33**). The serum levels of aspartate transaminase (AST) and alanine transaminase (ALT) were biochemically analyzed (Figure **34**).

The suitability of a toxicity prediction model was demonstrated from a ROC curve. Suitability was also demonstrated from PCA analysis of a selected probe for an adjuvanticity prediction model (adjuvant database + toxicogenomic database (GATE)). A heat map of routes centered around Osmr was created (not shown).

A gene cluster centered around Osmr was obtained from analysis of the database described above (Figure **35**). Gene Y (Osmr), which was predicted to have a strong relationship with toxicity, was investigated. The top row of Figure **36** shows a change in expression (fold change) of gene Y in the liver after 6 hours from administering each adjuvant to rats. Osmr knockout mice obtained from Jackson Laboratories were used as the base.

PBS, FK565 (1 μg/kg, 10 μg/kg, or 100 μg/kg), or LPS (1 mg/kg) was intraperitoneally administered to wild-type and Osmr knockout mice. The blood and liver were collected after one day. DRI-CHEM 7000V (Fujifilm, Tokyo, Japan) was used to analyze the serum AST and ALT levels (Figure **37**). Further, the liver was stained with TUNEL to examine apoptosis (bottom row of Figure **36**). AST and ALT activities were measured by an approach known in the art (e.g., colorimetric measurement using a kinetics assay or endpoint assay).

(Examination of liver virulence gene in mice)

For example, a gene suggested to be related to toxicity in rats can be selected as a knockout candidate in mice by referring to the following table (e.g., NOD1 to a NOD1 ligand). In this Example, Osmr was knocked out.

[Table 25]

| | Name | Receptor |
|---|---|---|
| 1 | AddaVax | Unknown |
| 2 | ADX | Unknown |
| 3 | ALM | Unknown |
| 4 | bCD | Unknown |
| 5 | cdiGMP | STING |
| 6 | cGAMP | STING |
| 7 | D35 | TLR9 |
| 8 | DMXAA | STING |
| 9 | ENDCN | Unknown |
| 10 | FCA | CLR and unkown |
| 11 | FK565 | NOD1 |
| 12 | ISA51VG | Unknown |
| | 13 K3 | TLR9 |
| 14 | K3SPG | TLR9 |
| 15 | MALP2s | TLR2/6 |
| 16 | MBT | NOD2 |
| 17 | MPLA | TLR4 |
| 18 | Pam3CSK4 | TLR1/2 |
| 19 | PolyIC | TLR3 and MDA5 |
| 20 | R848 | TLR7 |
| 21 | sHZ | Unknown |

(Prediction of adjuvanticity in rat liver model)

As shown in Figure **38,** a feature of a safe adjuvant is searched from an adjuvant database, and a compound that acts as an adjuvant is searched from a public toxicogenomic database.

Many drugs were suggested as a potential adjuvant by an adjuvanticity prediction model. This was investigated by an experiment using mice.

Probes (gene) selected from an adjuvanticity prediction model were concentrated to 42 routes related to cell death (4), immune response (2), and metabolism (36). In the Venn diagram of genes constituting a route related to cell death (4) and immune response (2), 7 genes were shared therebetween (Figure **39**).

Many drugs, immunostimulants, LPS, and TNF had a high score from an adjuvanticity prediction model (Figure **40,** top). Drugs indicated by colored letters were purchased and then tested in mice. Suitability of the adjuvanticity prediction model was confirmed by a ROC curve (Figure **40,** bottom).

Ovalbumin as well as alum, CpGk3, or five types of drugs (ACAP, BOR, CHX, COL, and PHA) were intradermally administered to mice on day 0 and day 14 at 2 to 3 different doses. Blood and spleen were collected on day 21. The anti-ovalbumin (ova) antibody titers (IgG1 and IgG2) were measured on day 21. Different adjuvant properties of IgG1 and IgG2 titers were observed depending on the drug (Figure **41**). Each drug, ovalbumin, and the like in the above example were obtained from Japan Biochemical or WAKO. In addition to drugs from these suppliers, drugs available from other supplies can also be used.

Furthermore, supernatant was collected after adding ovalbumin (ova), 257-264 peptide (OVA-MHC1), ova 323-339 peptide (OVA-MHC2), or ova protein (OVA-whole) in addition to each drug in vitro and stimulating, or treating without additional stimulation, spleen cells (cells from organ collected from immunized mice). Th1 (IL-2 and IFN-$\gamma$) (Figure **42**)- and Th2 (IL-4 and IL-5) (Figure **43**)-type cytokines in the supernatant were measured by ELISA.

Ovalbumin as well as alum, CpGk3, or five types of drugs (ACAP, BOR, CHX, COL, and PHA) were administered to mice. Blood was collected after three hours (day 0). DRI-CHEM 7000V (Fujifilm, Tokyo, Japan) was used for biochemical analysis on the serum aspartate transaminase (AST) and alanine transaminase (ALT) levels (Figure **44**).

Each drug was intraperitoneally administered to mice. Blood was collected after 6 hours and 24 hours to analyze miRNA in the circulating blood (Figure **45**). The results of analyzing miRNA in the circulating blood after 6 hours and 24 hours from administration of Alum and AS04 are shown (Figure **46**).

(Results)

In view of the above results, it can be understood that drug components such as adjuvants can be classified by the transcriptome analysis of the invention, and efficacy and safety can be tested.

In this Example, the inventors obtained microarray data similar to that of Examples 1 for rats in order to evaluate toxicological properties of adjuvants. Since transcriptome data of rats can be directly incorporated into a toxicogenomic data set of the public TG-GATE (Igarashi, Y. et al., Nucleic acids research 43, D921-927 (2015)) (this is also for rats), analysis was performed based thereon, which demonstrated that toxicity can be analyzed. Furthermore, knockout experiments were conducted with mice based on the findings from transcriptome analysis. It was substantiated to be a gene involved with toxicity. Therefore, it was substantiated that the results in this Example can be demonstrated across species, and the present invention and the findings obtained in the present invention can be utilized in verification of toxicity experiments in animal models. Furthermore, the inventors collected microRNA expression profiles from human clinical samples to analyze miRNA therewith. Integration of all of the aforementioned datasets proved that a more integrated and comprehensive analysis of adjuvant induced gene expression signatures of different species under different experimental conditions can be performed with such databases, and a test based on findings from transcriptome analysis can also be conducted for efficacy.

For toxicity and efficacy, those skilled in the art understand that the same analysis is possible in principle for not only adjuvants but also for all drug components. Especially for toxicity, the same test can be conducted on not only active ingredients, but also additives (excipient, carrier, and the like), and the same test can be conducted on a final formulation of mixture prepared by mixing a plurality of the same or different drug components. Those skilled in the art also understand that the same test using transcriptome analysis is possible for active ingredients based on an indicator of active ingredients.

As described above, the present invention is exemplified by the use of its preferred embodiments. However, it is understood that the scope of the present invention should be interpreted solely based on the Claims. It is also understood that any patent, any patent application, and any references cited herein should be incorporated herein by reference in the same manner as the contents are specifically described herein. The present application claims priority to Japanese Patent Application No. 2016-256270 and Japanese Patent Application No. 2016-256278 filed on December 28, 2016 In Japan. The entire content of these applications is incorporated herein by reference in the same manner as the contents are specifically described herein.

[Industrial Applicability]

Clinical application with high precision including classification of adjuvants is possible for immune related diseases.

[Sequence Listing Free Text]

SEQ ID NO: 1 CpG-ODN (5'-ATCGACTCTCGAGCGTTCTC-3')
SEQ ID NO: 2 MHC class I (ASNENMETM)
SEQ ID NO: 3 MHC class II (ARSALILRGSVAHKSCLPACVYGP)

[Embodiments]

In the following, preferred embodiments of the instant invention are described.

[Embodiment 1]

A method for classifying a drug component comprising classifying a drug component based on transcriptome clustering.

[Embodiment 2]

The method of embodiment 1, wherein the step of classifying comprises a) generating a reference component based on the transcriptome clustering; and b) classifying a candidate drug component based on the reference component.

[Embodiment 3]

The method of embodiment 1 or 2, wherein the drug component is selected from the group consisting of an active ingredient, an additive, and an adjuvant.

[Embodiment 4]

The method of any one of embodiments 1 to 3, wherein the drug component is an adjuvant.

[Embodiment 5]

The method of any one of embodiments 1 to 4, wherein the classification further comprises classification by at least one feature selected from the group consisting of classification based on a host response, classification based on a mechanism, classification by application based on a mechanism or cells (liver, lymph node, or spleen), and module classification.

[Embodiment 6]

The method of any one of embodiments 1 to 5, wherein the classification comprises at least one classification selected from the group consisting of G1 to G6:

(1) G1 (interferon signaling);
(2) G2 (metabolism of lipids and lipoproteins);
(3) G3 (response to stress);
(4) G4 (response to wounding);
(5) G5 (phosphate-containing compound metabolic process); and
(6) G6 (phagosome).

[Embodiment 7]

The method of embodiment 6,

wherein the drug component is an adjuvant, and the classification of G1 to G6 is performed by comparison with transcriptome clustering of a reference drug component,
wherein a reference drug component of G1 is a STING ligand,
wherein a reference drug component of G2 is a cyclodextrin,
wherein a reference drug component of G3 is an immune reactive peptide,
wherein a reference adjuvant of G4 is a TLR2 ligand,
wherein a reference drug component of G5 is a CpG oligonucleotide, and/or
wherein a reference drug component of G6 is a squalene oil-in-water emulsion adjuvant.

[Embodiment 8]

The method of embodiment 6 or 7,

wherein the classification of G1 to G6 is performed by comparison with transcriptome clustering of a reference drug component,
wherein a reference component of G1 is selected from the group consisting of cdiGMP, cGAMP, DMXAA, PolyIC, and R848,
wherein a reference drug component of G2 is bCD (β cyclodextrin),
wherein a reference drug component of G3 is FK565,
wherein a reference drug component of G4 is MALP2s,
wherein a reference drug component of G5 is selected from the group consisting of D35, K3, and K3SPG, and/or
wherein a reference drug component of G6 is AddaVax.

[Embodiment 9]

The method of any one of embodiments 6 to 8,

wherein the classification of G1 to G6 is performed based on an expression profile of a gene (identification marker gene; DEG) with a significant difference in expression in transcriptome analysis,
wherein a DEG of the G1 comprises at least one selected from the group consisting of Gm14446, Pml, H2-T22, Ifit1, Irf7, Isg15, Stat1, Fcgr1, Oas1a, Oas2, Trim12a, Trim12c, Uba7, and Ube216,
wherein a DEG of the G2 comprises at least one selected from the group consisting of Elovl6, Gpam, Hsd3b7, Acer2, Acox1, Tbl1xr1, Alox5ap, and Ggt5,
wherein a DEG of the G3 comprises at least one selected from the group consisting of Bbc3, Pdk4, Cd55, Cd93, Clec4e, Coro1a, and Traf3, Trem3, C5ar1, Clec4n, Ier3, Il1r1, Plek, Tbx3, and Trem1,
wherein a DEG of the G4 comprises at least one selected from the group consisting of Ccl3, Myof, Papss2, Slc7a11, and Tnfrsf1b,
wherein a DEG of the G5 comprises at least one selected from the group consisting of Ak3, Insm1, Nek1, Pik3r2, and Ttn, and
wherein a DEG of the G6 comprises at least one selected from the group consisting of Atp6v0d2, Atp6v1c1, and Clec7a.

[Embodiment 10]

A method of classifying a drug component, the method comprising:

(a) providing a candidate drug component;
(b) providing a reference drug component set;
(c) obtaining gene expression data by performing transcriptome analysis on the candidate drug component and the reference drug component set to cluster the gene expression data; and
(d) determining that the candidate drug component belongs to the same group if a cluster to which the candidate adjuvant belongs is classified to the same cluster as at least one in the reference drug component set, and determining as impossible to classify if the cluster does not belong to any cluster.

[Embodiment 11]

The method of classifying a drug component of embodiment 10, the method comprising:

(a) providing a candidate drug component in at least one organ of a target organism;
(b) providing a reference drug component set classified to at least one selected from the group consisting of G1 to G6 of any one of embodiments 6 to 8;
(c) obtaining gene expression data by performing transcriptome analysis on the candidate drug component and the reference drug component set to cluster the gene expression data; and
(d) determining that the candidate drug component belongs to the same group if a cluster to which the candidate drug component belongs is classified to the same cluster as at least one in groups G1 to G6, and determining as impossible to classify if the cluster does not belong to any cluster.

[Embodiment 12]

A method of manufacturing a composition having a desirable function, comprising:

(A) providing a candidate drug component,
(B) selecting a candidate drug component having a transcriptome expression pattern corresponding to a desirable function, and
(C) manufacturing a composition using a selected candidate drug component.

[Embodiment 13]

A method of screening for a composition having a desirable function, comprising;

(A) providing a candidate drug component; and
(B) selecting a candidate drug component having a transcriptome expression pattern corresponding to a desirable function.

[Embodiment 14]

The method of embodiment 12 or 13, wherein the desirable function comprises any one or more of G1 to G6 of any one of embodiments 6 to 8.

[Embodiment 15]

A composition for exerting a desirable function, comprising a drug component exerting the desirable function, wherein the desirable function comprises one or more classifications specified by the method of any one of embodiments 1 to 11.

[Embodiment 16]

The composition of embodiment 15 for exerting a desirable function, comprising a drug component exerting the desirable function, wherein the desirable function comprises any one or more of G1 to G6 of any one of embodiments 6 to 8.

[Embodiment 17]

A method of controlling quality of a drug component by using the method of any one of embodiment 1 to 11.

[Embodiment 18]

A method of testing safety of a drug component by using the method of any one of embodiments 1 to 11.

[Embodiment 19]

A method of providing a toxicity bottleneck gene, comprising:

identifying a candidate of a toxicity bottleneck gene by using the method of any one of embodiments 1 to 11;
making a knockout animal by knocking out the toxicity gene in another animal species; and
determining whether toxicity is reduced or eliminated in the knockout animal to select a gene with a reduction or

elimination as a toxicity bottleneck gene.

[Embodiment 20]

A method of determining toxicity of an agent, comprising:

determining whether activation of gene expression is observed for at least one of toxicity bottleneck genes for a candidate drug component such as an adjuvant; and
determining a candidate drug component observed to have the activation as having toxicity.

[Embodiment 21]

A method of determining an effect of a drug component by using the method of any one of embodiments 1 to 11.

[Embodiment 22]

A method of providing an efficacy bottleneck gene, comprising:

identifying an efficacy determination gene by using the method of any one of embodiments 1 to 11;
making a knockout animal by knocking out the toxicity gene in another animal species; and
determining whether efficacy is reduced or eliminated in the knockout animal to select a gene with a reduction or elimination as an efficacy bottleneck gene.

[Embodiment 23]

A method of determining efficacy of an agent, comprising;

determining whether activation of gene expression is observed for at least one of efficacy bottleneck genes for a candidate drug component such as an adjuvant; and
determining a candidate drug component observed to have the activation as having efficacy.

[Embodiment 24]

A program for implementing a drug component classification method comprising classifying a drug component based on transcriptome clustering on a computer.

[Embodiment 25]

A recording medium storing a program for implementing a drug component classification method comprising classifying a drug component based on transcriptome clustering on a computer.

[Embodiment 26]

A system for classifying a drug component based on transcriptome clustering, comprising a classification unit for classifying a drug component.

[Embodiment 27]

A program for implementing a method of classifying a drug component on a computer, the method comprising:

(a) providing a candidate drug component in at least one organ of a target organism;
(b) calculating a reference drug component set;
(c) obtaining gene expression data by performing transcriptome analysis on the candidate drug component and the reference drug component set to cluster the gene expression data; and
(d) determining that the candidate drug component belongs to the same group if a cluster to which the candidate drug component belongs is classified to the same cluster as at least one in a reference drug component set, and determining as impossible to classify if the cluster does not belong to any cluster.

[Embodiment 28]

The program of embodiment 27 for implementing a method of classifying a drug component on a computer, the method comprising:

(a) providing a candidate drug component in at least one organ of a target organism;
(b) providing a reference drug component set classified to at least one selected from the group consisting of G1 to G6 of any one of embodiments 6 to 8;
(c) obtaining gene expression data by performing transcriptome analysis on the candidate drug component and the reference drug component set to cluster the gene expression data; and
(d) determining that the candidate drug component belongs to the same group if a cluster to which the candidate drug component belongs is classified to the same cluster as at least one in groups G1 to G6, and determining as impossible to classify if the cluster does not belong to any cluster.

[Embodiment 29]

A recording medium storing a program for implementing a method of classifying a drug component on a computer, the method comprising:

(a) providing a candidate drug component in at least one organ of a target organism;
(b) calculating a reference drug component set;
(c) obtaining gene expression data by performing transcriptome analysis on the candidate drug component and the reference drug component set to cluster the gene expression data; and
(d) determining that the candidate drug component belongs to the same group if a cluster to which the candidate drug component belongs is classified to the same cluster as at least one in a reference drug component set, and determining as impossible to classify if the cluster does not belong to any cluster.

[Embodiment 30]

The recording medium of embodiment 29 storing a program for implementing a method of classifying a drug component on a computer, the method comprising:

(a) providing a candidate drug component in at least one organ of a target organism;
(b) providing a reference drug component set classified to at least one selected from the group consisting of G1 to G6 of any one of embodiments 6 to 8;
(c) obtaining gene expression data by performing transcriptome analysis on the candidate drug component and the reference drug component set to cluster the gene expression data; and
(d) determining that the candidate drug component belongs to the same group if a cluster to which the candidate drug component belongs is classified to the same cluster as at least one in groups G1 to G6, and determining as impossible to classify if the cluster does not belong to any cluster.

[Embodiment 31]

A system for classifying a drug component, the system comprising:

(a) a candidate drug component providing unit for providing a candidate drug component in at least one organ of a target organism;
(b) a reference drug component calculating unit for calculating a reference drug component set;
(c) a transcriptome clustering analysis unit for obtaining gene expression data by performing transcriptome analysis on the candidate drug component and the reference drug component set to cluster the gene expression data; and
(d) a determination unit for determining that the candidate drug component belongs to the same group if a cluster to which the candidate drug component belongs is classified to the same cluster as at least one in a reference drug component set, and determining as impossible to classify if the cluster does not belong to any cluster.

[Embodiment 32]

The system of embodiment 31 for classifying a drug component, the system comprising:

(a) a candidate drug component providing unit for providing a candidate drug component in at least one organ of a target organism;
(b) a reference drug component storing unit for providing a reference drug component set classified to at least one selected from the group consisting of G1 to G6 of any one of embodiments 6 to 8;
(c) a transcriptome clustering analysis unit for obtaining gene expression data by performing transcriptome analysis on the candidate drug component and the reference drug component set to cluster the gene expression data; and
(d) determination unit for determining that the candidate drug component belongs to the same group if a cluster to which the candidate drug component belongs is classified to the same cluster as at least one in groups G1 to G6, and determining as impossible to classify if the candidate drug cluster does not belong to any group.

[Embodiment 33]

A gene analysis panel for using a drug component in classification specified by the method of any one of embodiments 1 to 11.

[Embodiment 34]

A gene analysis panel for use in classification of an adjuvant to G1 to G6 of any one of embodiments 6 to 8 or to others, the gene analysis panel comprising means for detecting at least one DEG selected from the group consisting of a DEG of G1, a DEG of G2, a DEG of G3, a DEG of G4, a DEG of G5, and a DEG of G6,

wherein the DEG of G1 comprises at least one selected from the group consisting of Gm14446, Pml, H2-T22, Ifit1, Irf7, Isg15, Stat1, Fcgr1, Oas1a, Oas2, Trim12a, Trim12c, Uba7, and Ube216,
wherein the DEG of G2 comprises at least one selected from the group consisting of Elovl6, Gpam, Hsd3b7, Acer2, Acox1, Tbl1xr1, Alox5ap, and Ggt5,
wherein the DEG of G3 comprises at least one selected from the group consisting of Bbc3, Pdk4, Cd55, Cd93, Clec4e, Coro1a, and Traf3, Trem3, C5ar1, Clec4n, Ier3, Il1r1, Plek, Tbx3, and Trem1,
wherein the DEG of G4 comprises at least one selected from the group consisting of Ccl3, Myof, Papss2, Slc7a11, and Tnfrsf1b,
wherein the DEG of G5 comprises at least one selected from the group consisting of Ak3, Insm1, Nek1, Pik3r2, and Ttn, and
wherein the DEG of G6 comprises at least one selected from the group consisting of Atp6v0d2, Atp6v1c1, and Clec7a.

[Embodiment 35]

The gene analysis panel of embodiment 34, wherein the gene analysis panel comprises means for detecting at least a DEG of G1, means for detecting at least a DEG of G2, means for detecting at least a DEG of G3, means for detecting at least a DEG of G4, means for detecting at least a DEG of G5, and means for detecting at least a DEG of G6.

[Embodiment 36]

A method of generating an organ transcriptome profile of a drug component, the method comprising:

obtaining expression data by performing transcriptome analysis on at least one organ of a target organism using two or more drug components;
clustering the drug components with respect to the expression data; and
generating a transcriptome profile of the organ of the drug components based on the clustering.

[Embodiment 37]

The method of embodiment 36, wherein the transcriptome analysis comprises administering the drug component to the target organism and comparing a transcriptome in the organ at a certain time after administration with a transcriptome in the organ before administration of the drug component, and identifying a set of differentially expressed genes (DEG) as a result of the comparison.

[Embodiment 38]

The method of embodiment 37, comprising integrating the set of DEGs in two or more drug components to

generate a set of DEGs with a same change.

[Embodiment 39]

The method of embodiment 38, comprising selecting a DEG whose expression has changed beyond a predetermined threshold value among the DEGs with the same change as a result of the comparison to generate a set of significant DEGs.

[Embodiment 40]

The method of embodiment 39, wherein the predetermined threshold value is identified by a difference in a predetermined multiple and predetermined statistical significance (p value).

[Embodiment 41]

The method of any one of embodiments 38 to 41, comprising performing the transcriptome analysis for at least two or more organs to identify a set of differentially expressed genes (DEG) only in a specific organ and using the set as the organ specific DEG set.

[Embodiment 42]

The method of any one of embodiments 36 to 41, wherein the transcriptome analysis is performed on a transcriptome in at least one organ selected from the group consisting of a liver, a spleen, and a lymph node.

[Embodiment 43]

The method of any one of embodiments 36 to 42, wherein a number of types of the drug components is a number that enables statistically significant clustering analysis.

[Embodiment 44]

The method of any one of embodiments 36 to 43, comprising providing one or more gene markers unique to a specific drug component or a drug component cluster and a specific organ in the profile as a drug component evaluation marker.

[Embodiment 45]

The method of any one of embodiments 36 to 44, further comprising analyzing a biological indicator to correlate the drug components with a cluster.

[Embodiment 46]

The method of embodiment 45, wherein the biological indicator comprises at least one indicator selected from the group consisting of a wounding, cell death, apoptosis, NEκB signaling pathway, inflammatory response, TNF signaling pathway, cytokines, migration, chemokine, chemotaxis, stress, defense response, immune response, innate immune response, adaptive immune response, interferons, and interleukins.

[Embodiment 47]

The method of embodiment 46, wherein the biological indicator comprises a hematological indicator.

[Embodiment 48]

The method of embodiment 47, wherein the hematological indicator comprises at least one selected from the group consisting of white blood cells (WBC), lymphocytes (LYM), monocytes (MON), granulocytes (GRA), relative (%) content of lymphocytes (LY%), relative (%) content of monocytes (MO%), relative (%) content of granulocytes (GR%), red blood cells (RBC), hemoglobins (Hb, HGB), hematocrits (HCT), mean corpuscular volume (MCV), mean corpuscular hemoglobins (MCH), mean corpuscular hemoglobin concentration (MCHC), red blood cell distribution width (RDW),

platelets (PLT), platelet concentration (PCT), mean platelet volume (MPV), and platelet distribution width (PDW).

[Embodiment 49]

The method of embodiment 45, wherein the biological indicator comprises a cytokine profile.

[Embodiment 50]

A program for implementing a method of generating an organ transcriptome profile of a drug component on a computer, the method comprising:

(A) obtaining expression data by performing transcriptome analysis on at least one organ of a target organism using two or more drug components;
(B) clustering the drug components with respect to the expression data; and
(C) generating a transcriptome profile of the organ of the drug components, based on the clustering.

[Embodiment 51]

A recording medium storing a program for implementing a method of generating an organ transcriptome profile of a drug component on a computer, the method comprising:

(A) obtaining expression data by performing transcriptome analysis on at least one organ of a target organism using two or more drug components;
(B) clustering the drug components with respect to the expression data; and (C) generating a transcriptome profile of the organ of the drug components based on the clustering.

[Embodiment 52]

A system for generating an organ transcriptome profile of a drug component, the system comprising:

(A) an expression data acquiring unit for obtaining or inputting expression data by performing transcriptome analysis on at least one organ of a target organism using two or more drug components;
(B) a clustering computing unit for clustering the drug components with respect to the expression data; and
(C) a profiling unit for generating a transcriptome profile of the organ of the drug components based on the clustering.

[Embodiment 53]

A method of providing feature information of a drug component, the method comprising:

(a) providing a candidate drug component in at least one organ of a target organism;
(b) providing a reference drug component set with a known function;
(c) obtaining gene expression data by performing transcriptome analysis on the candidate drug component and the reference drug component set to cluster the gene expression data; and
(d) providing a feature of a member of the reference drug component set belonging to the same cluster as that of the candidate drug component as a feature of the candidate drug component.

[Embodiment 54]

The method of embodiment 53, further comprising a feature of any one of embodiments 36 to 49.

[Embodiment 55]

A program for implementing a method of providing feature information of a drug component on a computer, the method comprising:

(a) providing a candidate drug component in at least one organ of a target organism;
(b) providing a reference drug component set with a known function;
(c) obtaining gene expression data by performing transcriptome analysis on the candidate drug component and the

reference drug component set to cluster the gene expression data; and
(d) providing a feature of a member of the reference drug component set belonging to the same cluster as that of the candidate drug component as a feature of the candidate drug component.

[Embodiment 56]

A recording medium for storing a program for implementing a method of providing feature information of a drug component on a computer, the method comprising:

(a) providing a candidate drug component in at least one organ of a target organism;
(b) providing a reference drug component set with a known function;
(c) obtaining gene expression data by performing transcriptome analysis on the candidate drug component and the reference drug component set to cluster the gene expression data; and
(d) providing a feature of a member of the reference drug component set belonging to the same cluster as that of the candidate drug component as a feature of the candidate drug component.

[Embodiment 57]

A system for providing feature information of a drug component, the system comprising:

(a) a candidate drug component providing unit for providing a candidate drug component;
(b) a reference drug component providing unit for providing a reference drug component set with a known function;
(c) a transcriptome clustering analysis unit for obtaining gene expression data by performing transcriptome analysis on the candidate drug component and the reference drug component set to cluster the gene expression data; and
(d) a feature analysis unit for providing a feature of a member of the reference drug component set belonging to the same cluster as that of the candidate drug component as a feature of the candidate drug component.

[Embodiment 58]

A method of controlling quality of a drug component by using the method of embodiments 36 to 49 or embodiment 53.

[Embodiment 59]

A method of testing safety of a drug component by using the method of embodiments 36 to 49 or embodiment 53.

[Embodiment 60]

A method of determining an effect of a drug component by using the method of embodiments 36 to 49 or embodiment 53.

## Claims

**1.** A method of classifying a drug component, the method comprising:

(a) providing a candidate drug component;
(b) obtaining gene expression data by performing transcriptome analysis on the candidate drug component;
(c) clustering the gene expression data by combined use of adjuvant database created by performing transcriptome analysis for efficacy as an adjuvant and toxicity genome data obtained from toxicity database created by performing transcriptome analysis for toxicity of drugs; and
(d) determining that the candidate drug component has efficacy as an adjuvant and/or toxicity similar to drugs having efficacy as an adjuvant and/or toxicity in the databases if a cluster to which the candidate drug component belongs is classified to the same cluster as at least one of the drugs.

**2.** The method of claim 1, wherein the combination of the adjuvant database and the toxicity genome data comprises gene expression data from different species of animals.

3. The method of claim 1 or 2, wherein the combination of the adjuvant database and the toxicity genome data comprises human gene expression data, optionally wherein the method determines efficacy as an adjuvant and/or toxicity in human for the candidate drug component.

4. The method of any one of claims 1 to 3, wherein the toxicity genome data comprises genome data for drug components in toxicity group and drug components in non-toxicity group.

5. The method of any one of claims 1 to 4, wherein the clustering comprises creating a cluster for efficacy as an adjuvant and creating a cluster for toxicity, optionally, the method determines efficacy as an adjuvant and toxicity for the candidate drug component.

6. The method of any one of claims 1 to 5, comprising extracting a characteristic gene from a gene expression profile with machine learning, and/or creating a prediction model which determines efficacy as an adjuvant and/or toxicity with machine learning.

7. The method of claim 6, wherein the machine learning comprises neural networking method, support vector machine, random forest, linear regression, logistic regression, support vector machine or cross validation, optionally wherein the method uses data from the adjuvant database and/or the toxicity database as training data for the machine learning.

8. The method of any one of claims 1 to 7, comprising determining a candidate gene of toxicity bottleneck gene by the transcriptome analysis.

9. The method of claim 8, wherein the candidate gene of toxicity bottleneck gene is a significantly differentially expressed gene.

10. The method of any one of claims 1 to 9, wherein the classification further comprises classification by at least one feature selected from the group consisting of classification based on a host response, classification based on a mechanism, classification by application based on a mechanism or cells (liver, lymph node, or spleen), and module classification.

11. A program for implementing a drug component classification method comprising classifying a drug component based on the method of any one of claims 1 to 10.

12. A recording medium storing a program for implementing a drug component classification method comprising classifying a drug component based on the method of any one of claims 1 to 10.

13. A system for classifying a drug component comprising a classification unit which classifies a drug component based on the method of any one of claims 1 to 10.

14. A computer system for classifying a drug component, the system comprising:

(a) a storing unit for adjuvant database created by performing transcriptome analysis for efficacy as an adjuvant;
(b) a storing unit for toxicity database created by performing transcriptome analysis for toxicity of drugs;
(c) a transcriptome clustering analysis unit for obtaining gene expression data by performing transcriptome analysis on the candidate drug component and clustering the gene expression data by combined use of the adjuvant database and toxicity genome data obtained from the toxicity database; and
(d) a determination unit for determining that the candidate drug component has efficacy as an adjuvant and/or toxicity similar to drugs having efficacy as an adjuvant and/or toxicity in the databases if a cluster to which the candidate drug component belongs is classified to the same cluster as at least one of the drugs.

15. A gene analysis panel comprising means to detect nucleic acids or proteins for use in classification specified by the method of any one of claims 1 to 10.

[FIG.1A]

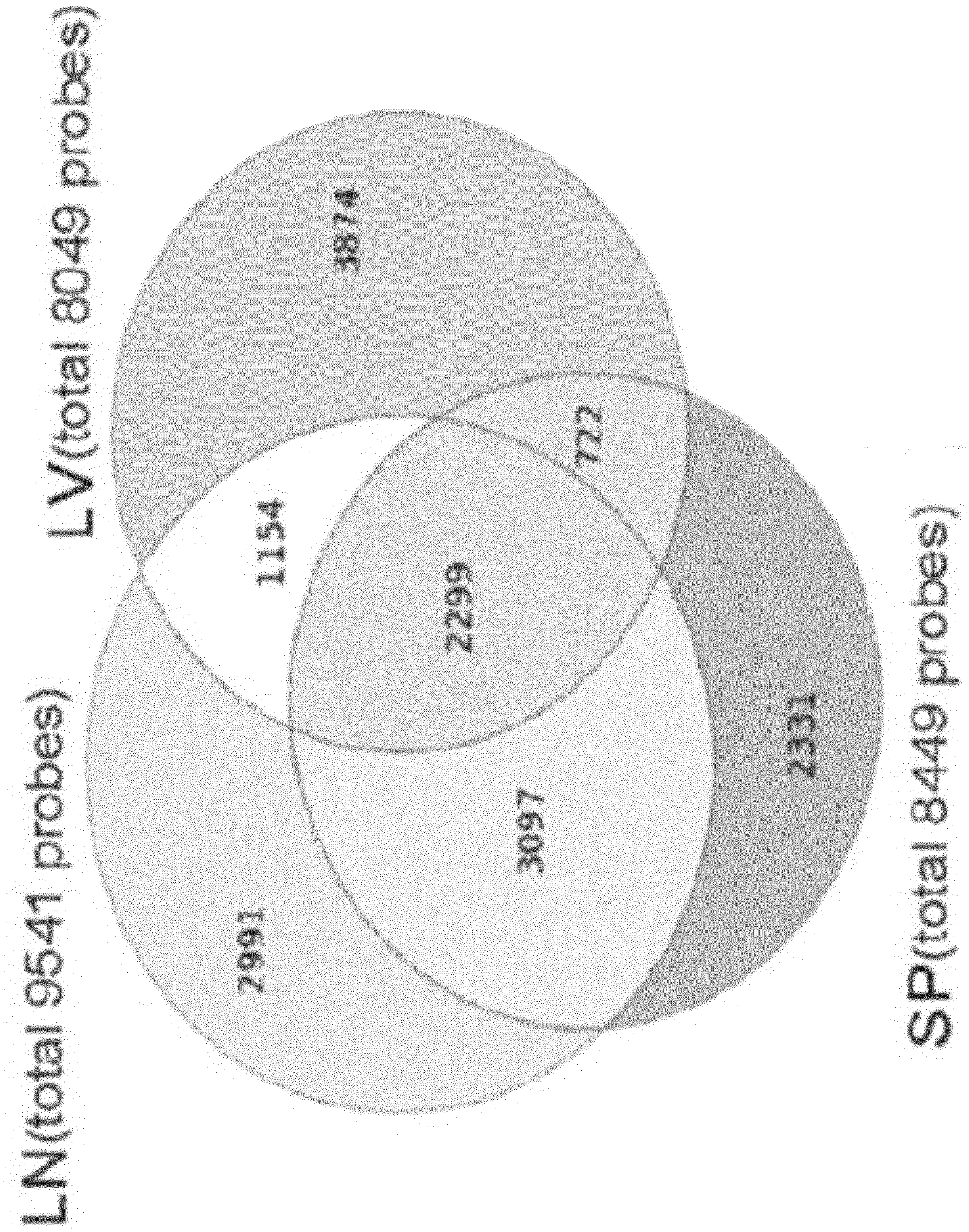
LV(total 8049 probes)
LN(total 9541 probes)
SP(total 8449 probes)
3874
722
1154
2299
2331
3097
2991

[FIG.1B]

LN only (2991 probes)

| | |
|---|---|
| Immune System | 1.99E-05 |
| Jak-STAT signaling pathway | 4.65E-04 |
| Tuberculosis | 7.79E-04 |
| Innate Immune System | 1.25E-03 |
| RIG-I-like receptor signaling pathway | 2.22E-03 |
| C-type lectin receptors (CLRs) | 5.12E-03 |
| VEGF signaling pathway | 1.66E-02 |
| Signaling by Interleukins | 2.05E-02 |
| TCR signaling | 2.26E-02 |
| Measles | 2.87E-02 |
| Hemostasis | 3.17E-02 |

LV only (3874 probes)

| | |
|---|---|
| Metabolism | 1.39E-07 |
| Metabolism of lipids and lipoproteins | 1.42E-07 |
| DNA methylation | 7.67E-05 |
| Metabolic pathways | 1.15E-04 |
| Meiotic synapsis | 1.38E-04 |
| Meiotic Synapsis | 1.38E-04 |
| Meiotic Recombination | 2.18E-04 |
| Meiosis | 2.55E-04 |
| NoRC negatively regulates rRNA expression | 2.99E-04 |
| NoRC negatively regulates rRNA expression | 4.07E-04 |

SP only (2331 probes)

| | |
|---|---|
| Gene Expression | 4.82E-12 |
| Spliceosome | 5.61E-11 |
| Processing of Capped Intron-Containing Pre-mRNA | 1.90E-07 |
| Transcriptional regulation by small RNAs | 2.60E-07 |
| Mitotic Prophase | 4.55E-07 |
| Non-coding RNA Metabolism | 4.83E-06 |
| snRNP Assembly | 4.83E-06 |
| Packaging Of Telomere Ends | 1.86E-05 |
| Condensation of Prophase Chromosomes | 2.10E-05 |
| Deposition of new CENPA-containing nucleosomes at the centromere | 4.17E-05 |

LV&SP&LN (2299 probes)

| | |
|---|---|
| Immune System | 1.04E-16 |
| Herpes simplex infection | 1.91E-16 |
| Interferon Signaling | 3.71E-16 |
| Cytokine Signaling in Immune system | 1.04E-15 |
| Interferon gamma signaling | 2.15E-12 |
| NF-kappa B signaling pathway | 1.70E-10 |
| Influenza A | 4.94E-10 |
| TNF signaling pathway | 3.88E-07 |
| Chemokine receptors bind chemokines | 3.40E-05 |
| Epstein-Barr virus infection | 4.65E-05 |

[FIG.2A]

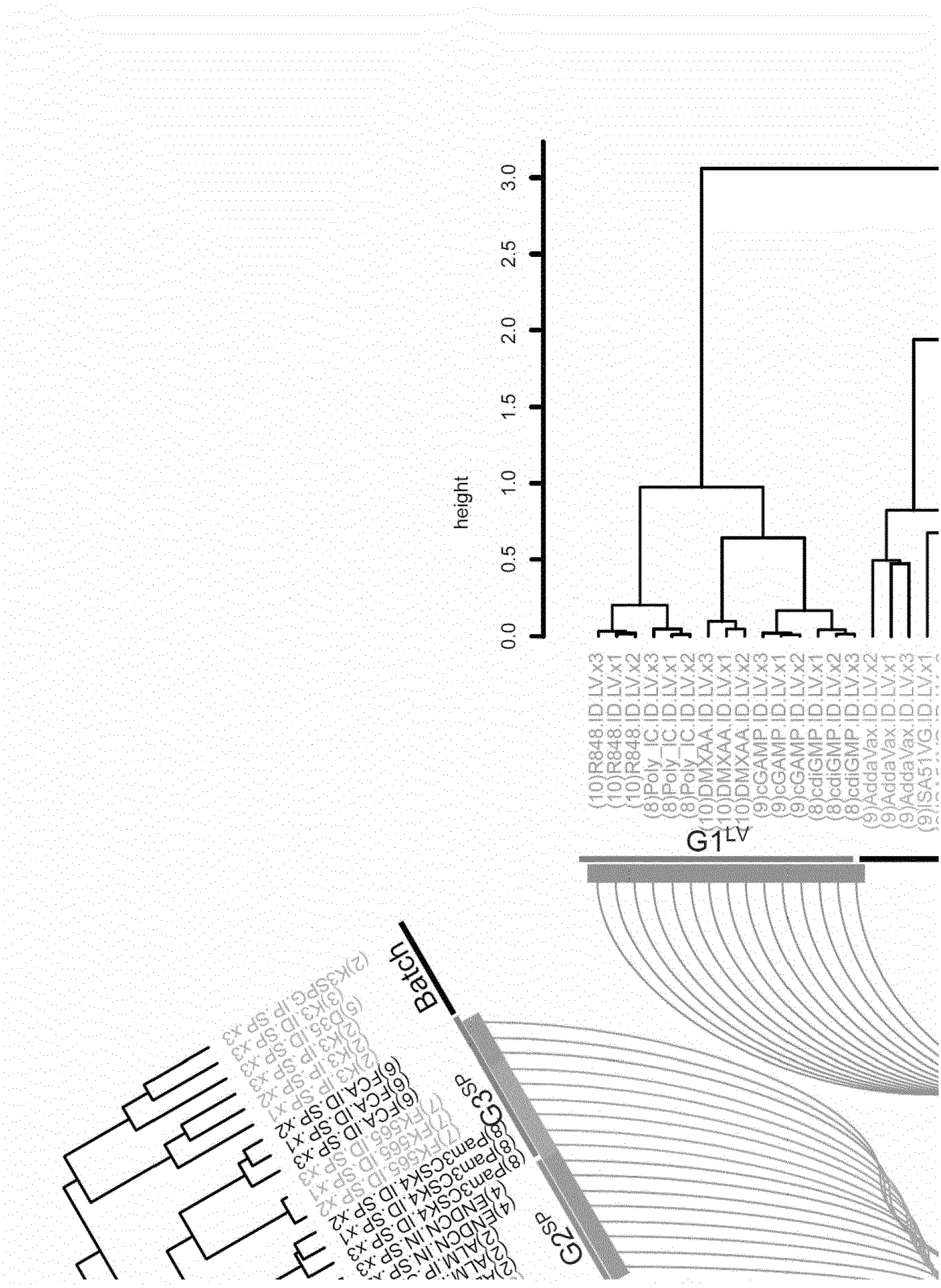

[FIG.2A](**Continue**)

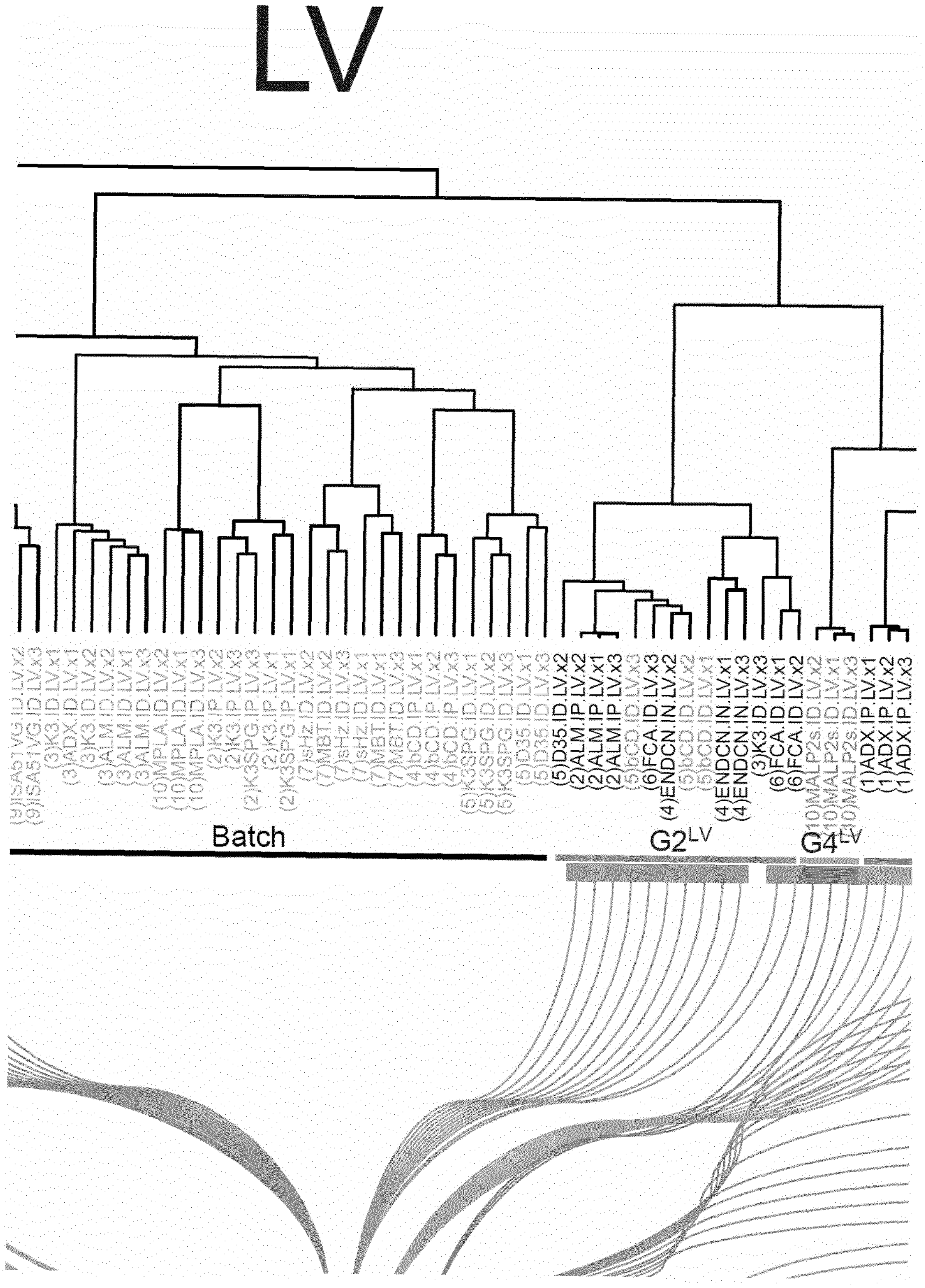

[FIG.2A](**Continue**)

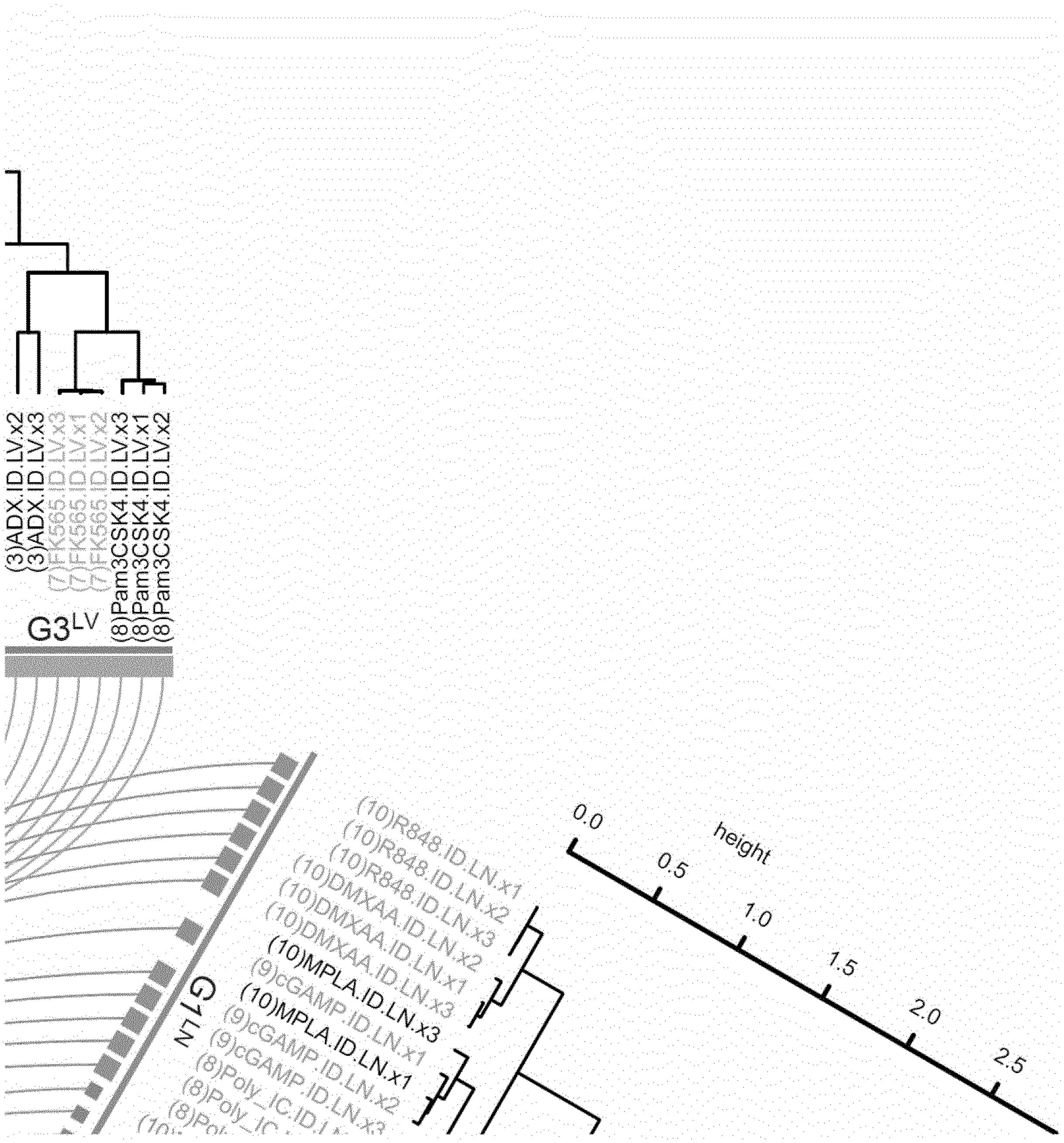

[FIG.2A](**Continue**)

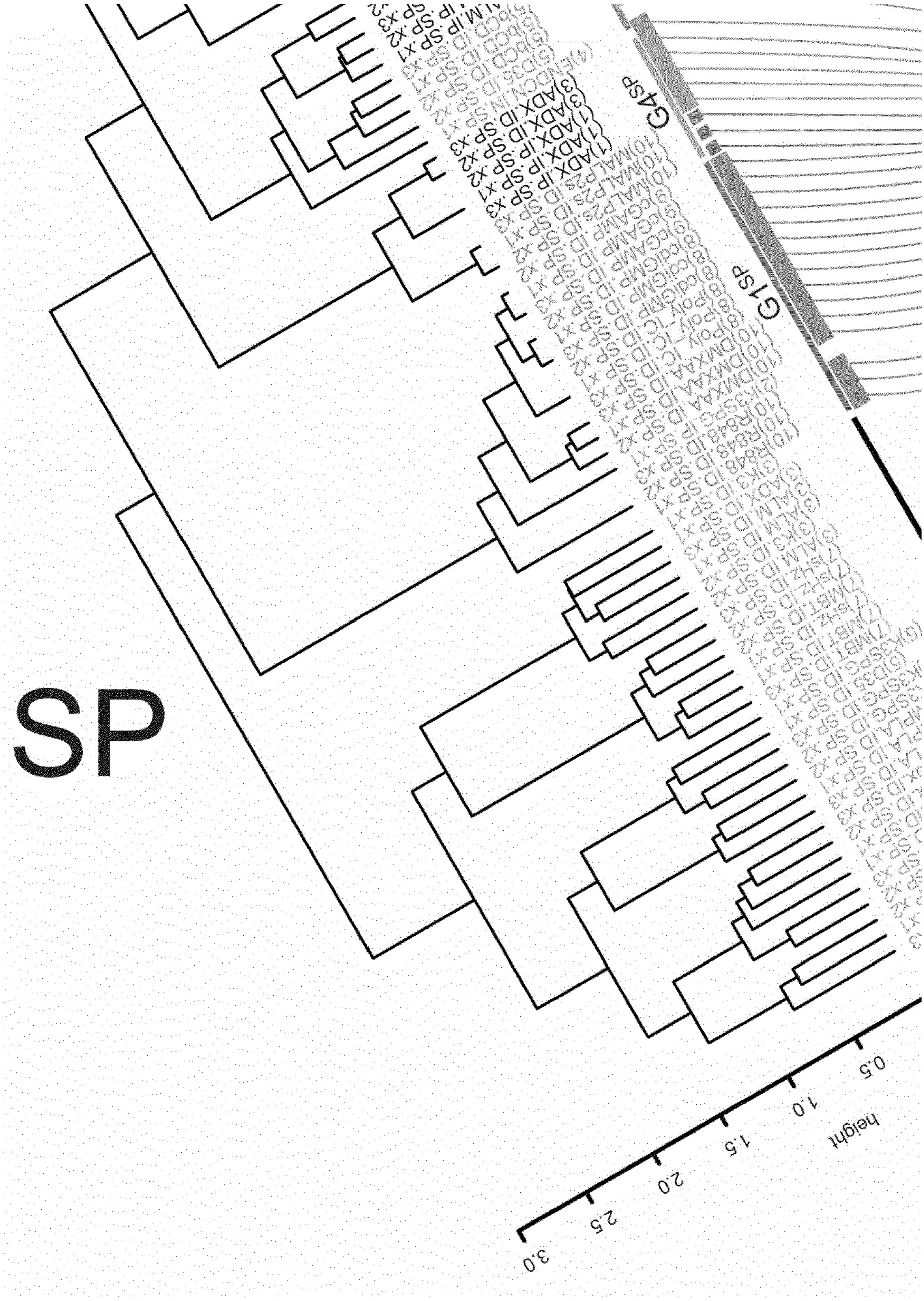

[FIG.2A](**Continue**)

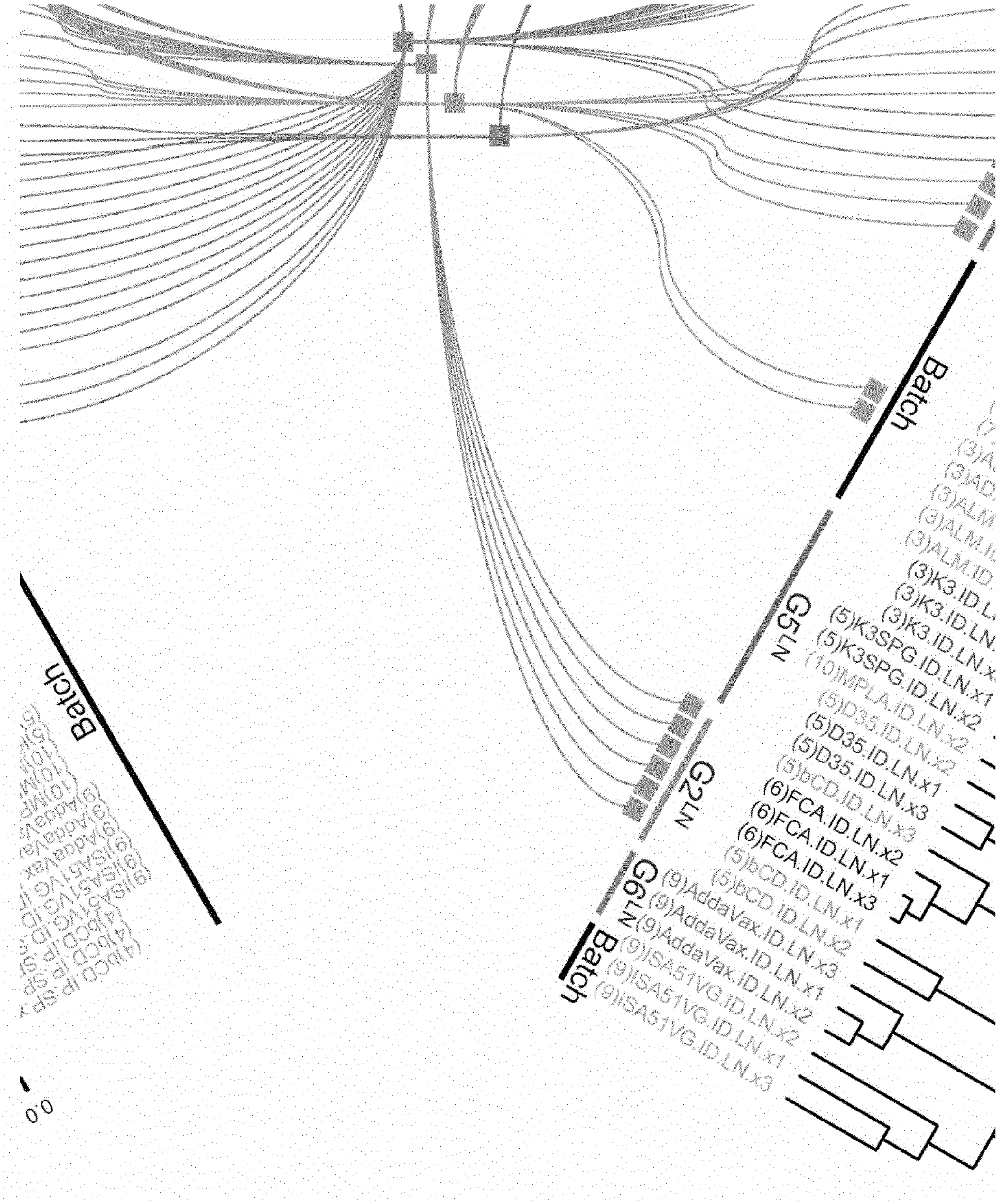

[FIG.2A](**Continue**)

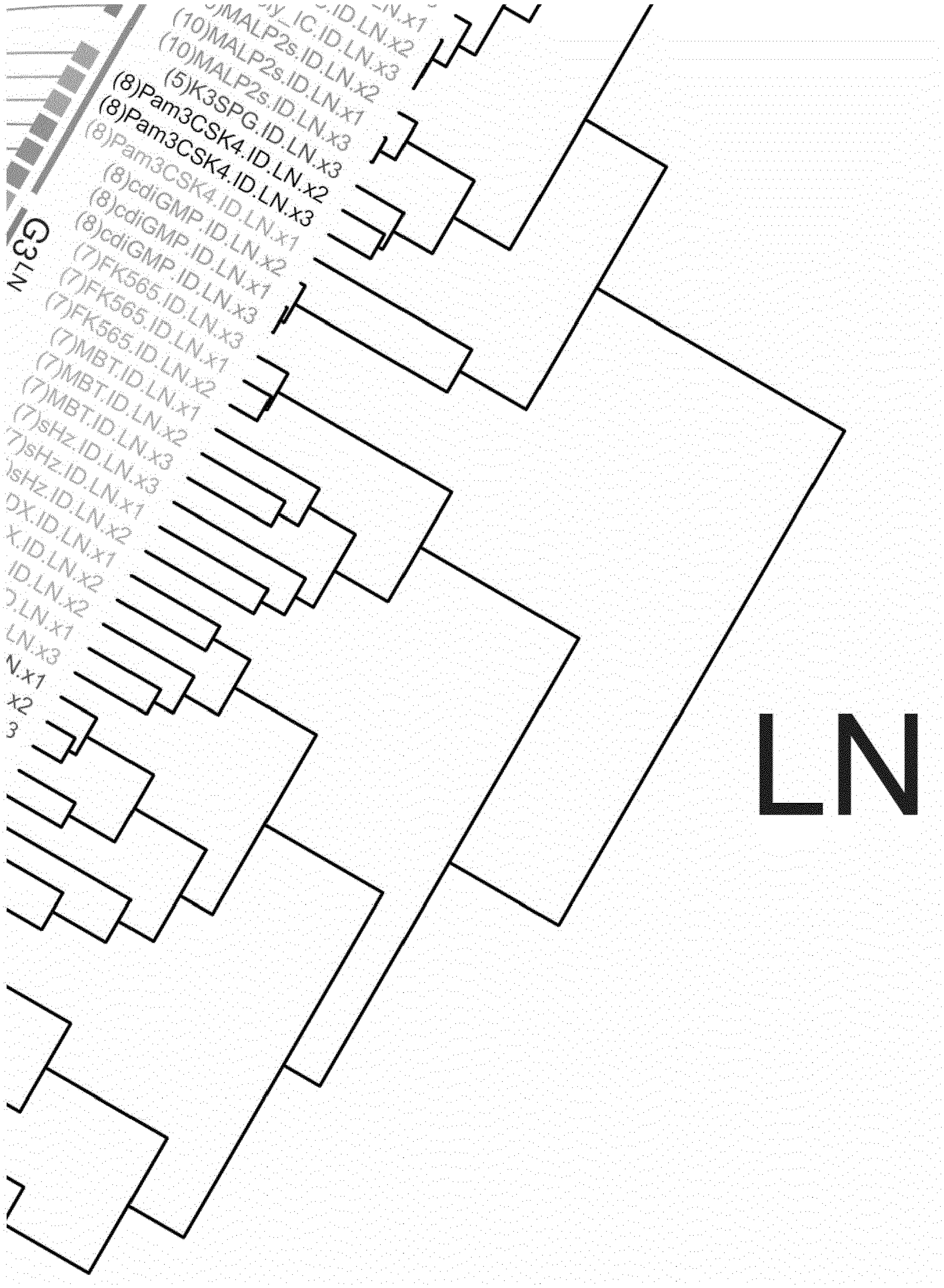

[FIG.2B]

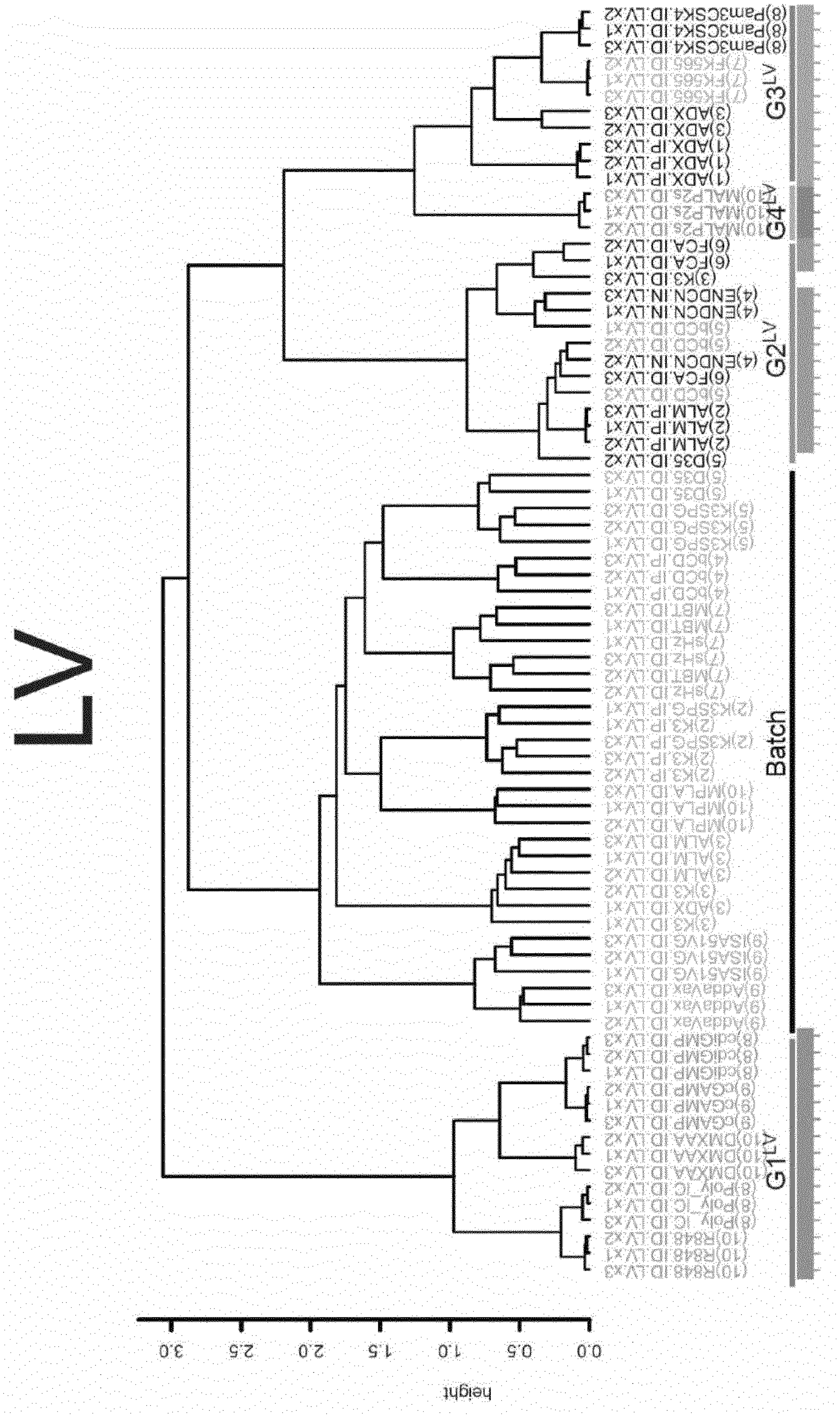

[FIG.2C]

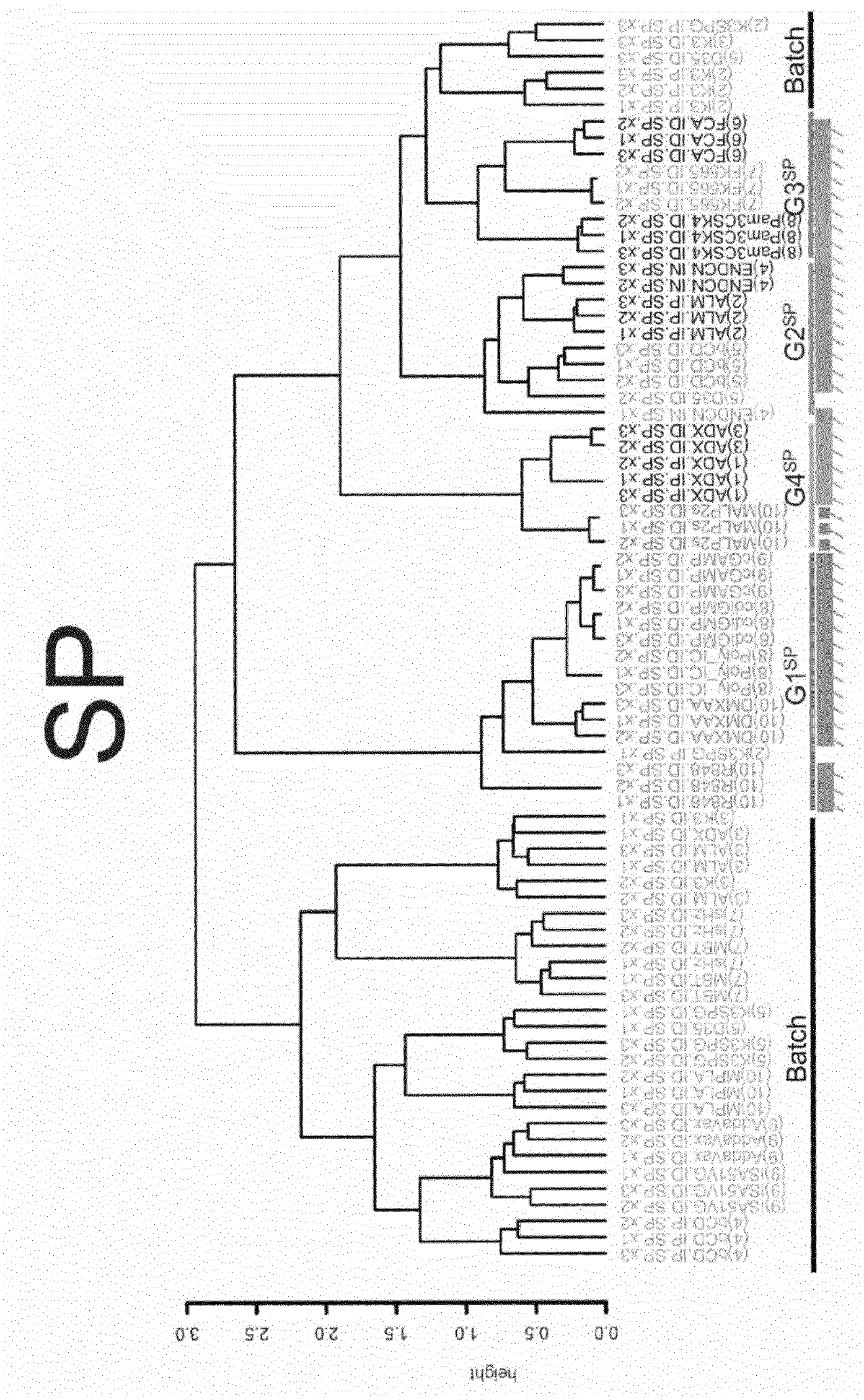
SP
height
0.0
0.5
1.0
1.5
2.0
2.5
3.0
Batch
G3SP
G2SP
G4SP
G1SP
Batch
(2)K3SPG.IP.SP.x3
(3)K3.ID.SP.x3
(5)D35.ID.SP.x3
(2)K3.IP.SP.x3
(2)K3.IP.SP.x2
(2)K3.IP.SP.x1
(6)FCA.ID.SP.x2
(6)FCA.ID.SP.x1
(6)FCA.ID.SP.x3
(7)FK565.ID.SP.x3
(7)FK565.ID.SP.x1
(7)FK565.ID.SP.x2
(8)Pam3CSK4.ID.SP.x2
(8)Pam3CSK4.ID.SP.x1
(8)Pam3CSK4.ID.SP.x3
(4)ENDCN.IN.SP.x3
(4)ENDCN.IN.SP.x2
(2)ALM.IP.SP.x3
(2)ALM.IP.SP.x2
(2)ALM.IP.SP.x1
(5)bCD.ID.SP.x3
(5)bCD.ID.SP.x1
(5)bCD.ID.SP.x2
(5)D35.ID.SP.x2
(4)ENDCN.IN.SP.x1
(3)ADX.ID.SP.x3
(3)ADX.ID.SP.x2
(1)ADX.IP.SP.x2
(1)ADX.IP.SP.x1
(1)ADX.IP.SP.x3
(10)MALP2s.ID.SP.x3
(10)MALP2s.ID.SP.x1
(10)MALP2s.ID.SP.x2
(9)cGAMP.ID.SP.x2
(9)cGAMP.ID.SP.x1
(9)cGAMP.ID.SP.x3
(8)cdiGMP.ID.SP.x2
(8)cdiGMP.ID.SP.x1
(8)cdiGMP.ID.SP.x3
(8)Poly_IC.ID.SP.x2
(8)Poly_IC.ID.SP.x1
(8)Poly_IC.ID.SP.x3
(10)DMXAA.ID.SP.x3
(10)DMXAA.ID.SP.x1
(10)DMXAA.ID.SP.x2
(2)K3SPG.IP.SP.x1
(10)R848.ID.SP.x3
(10)R848.ID.SP.x2
(10)R848.ID.SP.x1
(3)K3.ID.SP.x1
(3)ADX.ID.SP.x1
(3)ALM.ID.SP.x3
(3)ALM.ID.SP.x1
(3)K3.ID.SP.x2
(3)ALM.ID.SP.x2
(7)sHz.ID.SP.x3
(7)sHz.ID.SP.x2
(7)MBT.ID.SP.x2
(7)sHz.ID.SP.x1
(7)MBT.ID.SP.x1
(7)MBT.ID.SP.x3
(5)K3SPG.ID.SP.x1
(5)D35.ID.SP.x1
(5)K3SPG.ID.SP.x3
(5)K3SPG.ID.SP.x2
(10)MPLA.ID.SP.x2
(10)MPLA.ID.SP.x1
(10)MPLA.ID.SP.x3
(9)AddaVax.ID.SP.x3
(9)AddaVax.ID.SP.x2
(9)AddaVax.ID.SP.x1
(9)ISA51VG.ID.SP.x1
(9)ISA51VG.ID.SP.x3
(9)ISA51VG.ID.SP.x2
(4)bCD.IP.SP.x2
(4)bCD.IP.SP.x1
(4)bCD.IP.SP.x3

[FIG.2D]

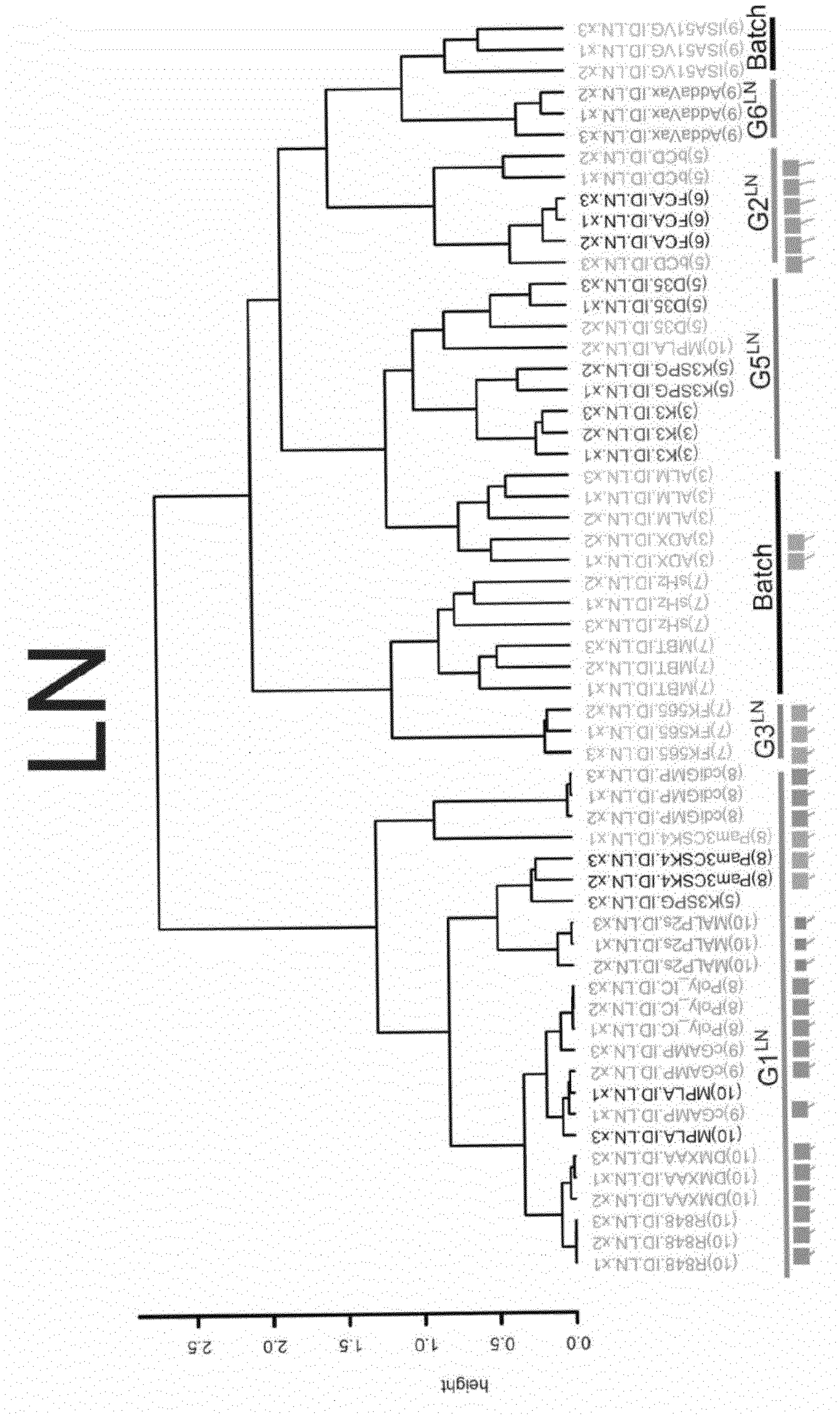
LN
Batch
G6LN
G2LN
G5LN
Batch
G3LN
G1LN
height
0.0
0.5
1.0
1.5
2.0
2.5

[FIG.2E]

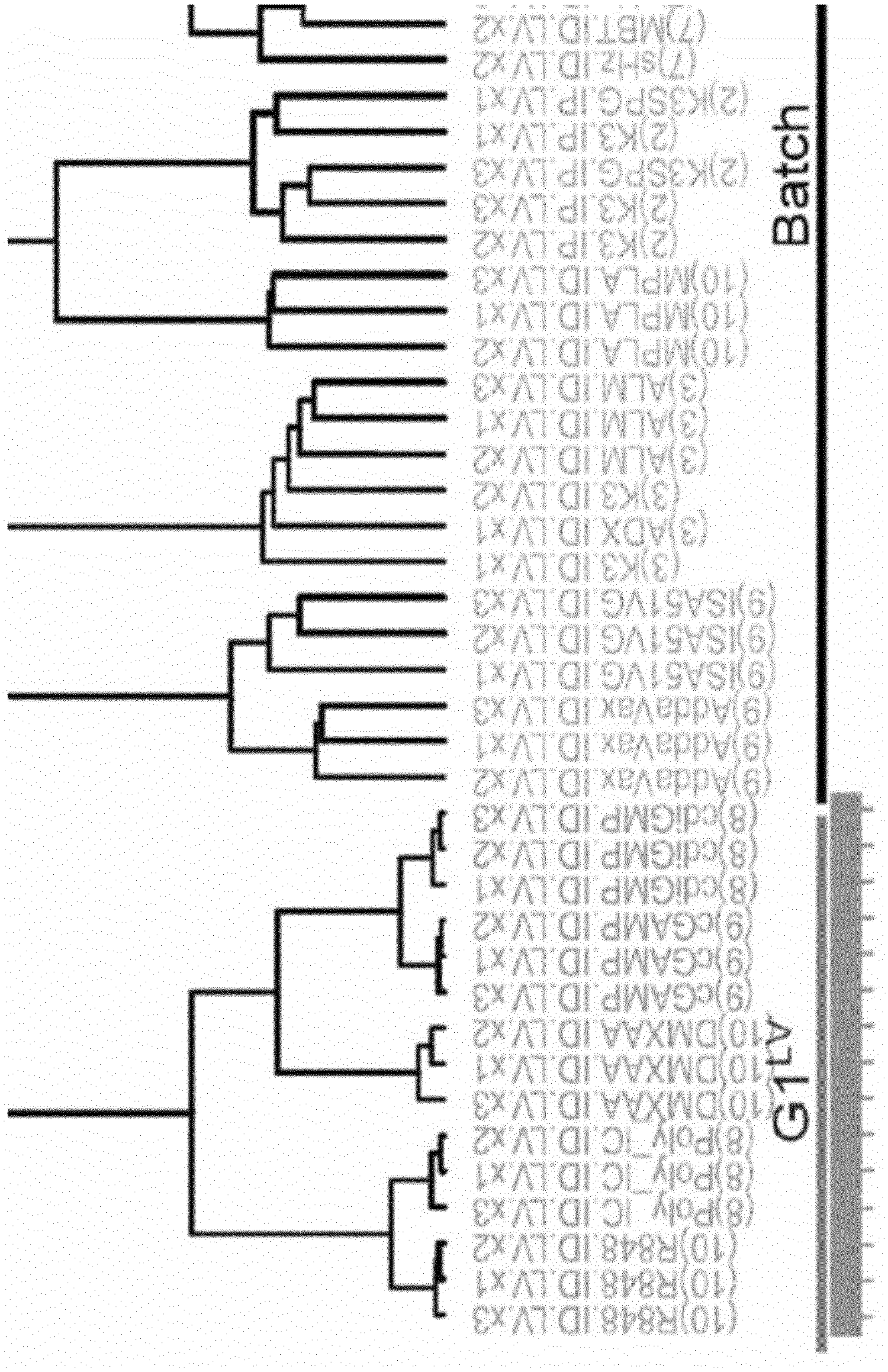
(7)MBT.ID.LV.x2
(7)sHz.ID.LV.x2
(2)K3SPG.IP.LV.x1
(2)K3.IP.LV.x1
(2)K3SPG.IP.LV.x3
(2)K3.IP.LV.x3
(2)K3.IP.LV.x2
(10)MPLA.ID.LV.x3
(10)MPLA.ID.LV.x1
(10)MPLA.ID.LV.x2
(3)ALM.ID.LV.x3
(3)ALM.ID.LV.x1
(3)ALM.ID.LV.x2
(3)K3.ID.LV.x2
(3)ADX.ID.LV.x1
(3)K3.ID.LV.x1
(9)ISA51VG.ID.LV.x3
(9)ISA51VG.ID.LV.x2
(9)ISA51VG.ID.LV.x1
(9)AddaVax.ID.LV.x3
(9)AddaVax.ID.LV.x1
(9)AddaVax.ID.LV.x2
(8)cdiGMP.ID.LV.x3
(8)cdiGMP.ID.LV.x2
(8)cdiGMP.ID.LV.x1
(9)cGAMP.ID.LV.x2
(9)cGAMP.ID.LV.x1
(9)cGAMP.ID.LV.x3
(10)DMXAA.ID.LV.x2
(10)DMXAA.ID.LV.x1
(10)DMXAA.ID.LV.x3
(8)Poly_IC.ID.LV.x2
(8)Poly_IC.ID.LV.x1
(8)Poly_IC.ID.LV.x3
(10)R848.ID.LV.x2
(10)R848.ID.LV.x1
(10)R848.ID.LV.x3
Batch
G1LV

[FIG.2F]

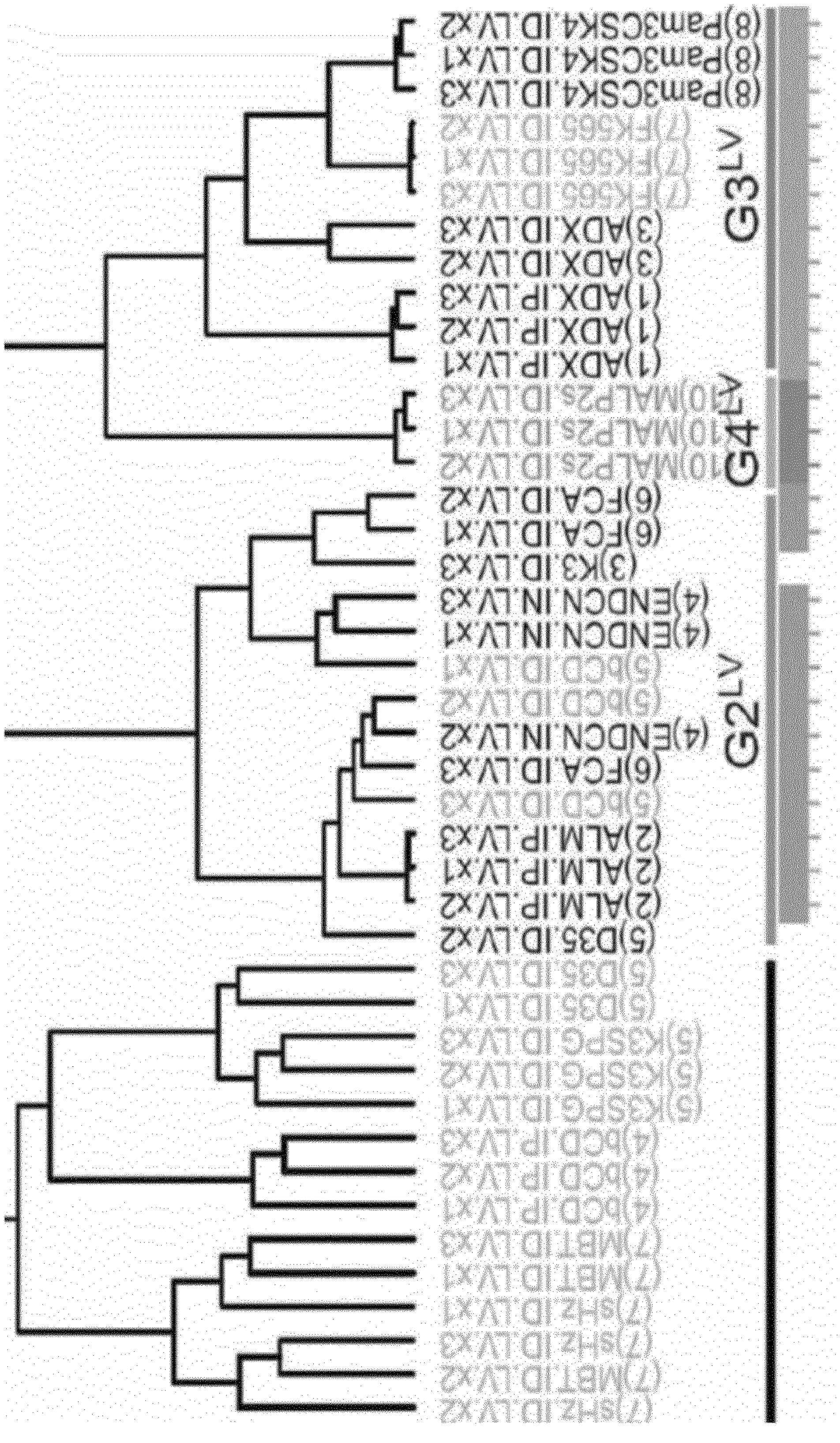
(7)sHz.ID.LV.x2
(7)MBT.ID.LV.x2
(7)sHz.ID.LV.x3
(7)sHz.ID.LV.x1
(7)MBT.ID.LV.x1
(7)MBT.ID.LV.x3
(4)bCD.IP.LV.x1
(4)bCD.IP.LV.x2
(4)bCD.IP.LV.x3
(5)K3SPG.ID.LV.x1
(5)K3SPG.ID.LV.x2
(5)K3SPG.ID.LV.x3
(5)D35.ID.LV.x1
(5)D35.ID.LV.x3
(5)D35.ID.LV.x2
(2)ALM.IP.LV.x2
(2)ALM.IP.LV.x1
(2)ALM.IP.LV.x3
(5)bCD.ID.LV.x3
(6)FCA.ID.LV.x3
(4)ENDCN.IN.LV.x2
(5)bCD.ID.LV.x2
(5)bCD.ID.LV.x1
(4)ENDCN.IN.LV.x1
(4)ENDCN.IN.LV.x3
(3)K3.ID.LV.x3
(6)FCA.ID.LV.x1
(6)FCA.ID.LV.x2
(10)MALP2s.ID.LV.x2
(10)MALP2s.ID.LV.x1
(10)MALP2s.ID.LV.x3
(1)ADX.IP.LV.x1
(1)ADX.IP.LV.x2
(1)ADX.IP.LV.x3
(3)ADX.ID.LV.x2
(3)ADX.ID.LV.x3
(7)FK565.ID.LV.x3
(7)FK565.ID.LV.x1
(7)FK565.ID.LV.x2
(8)Pam3CSK4.ID.LV.x3
(8)Pam3CSK4.ID.LV.x1
(8)Pam3CSK4.ID.LV.x2
G2LV
G4LV
G3LV

[FIG.2G]

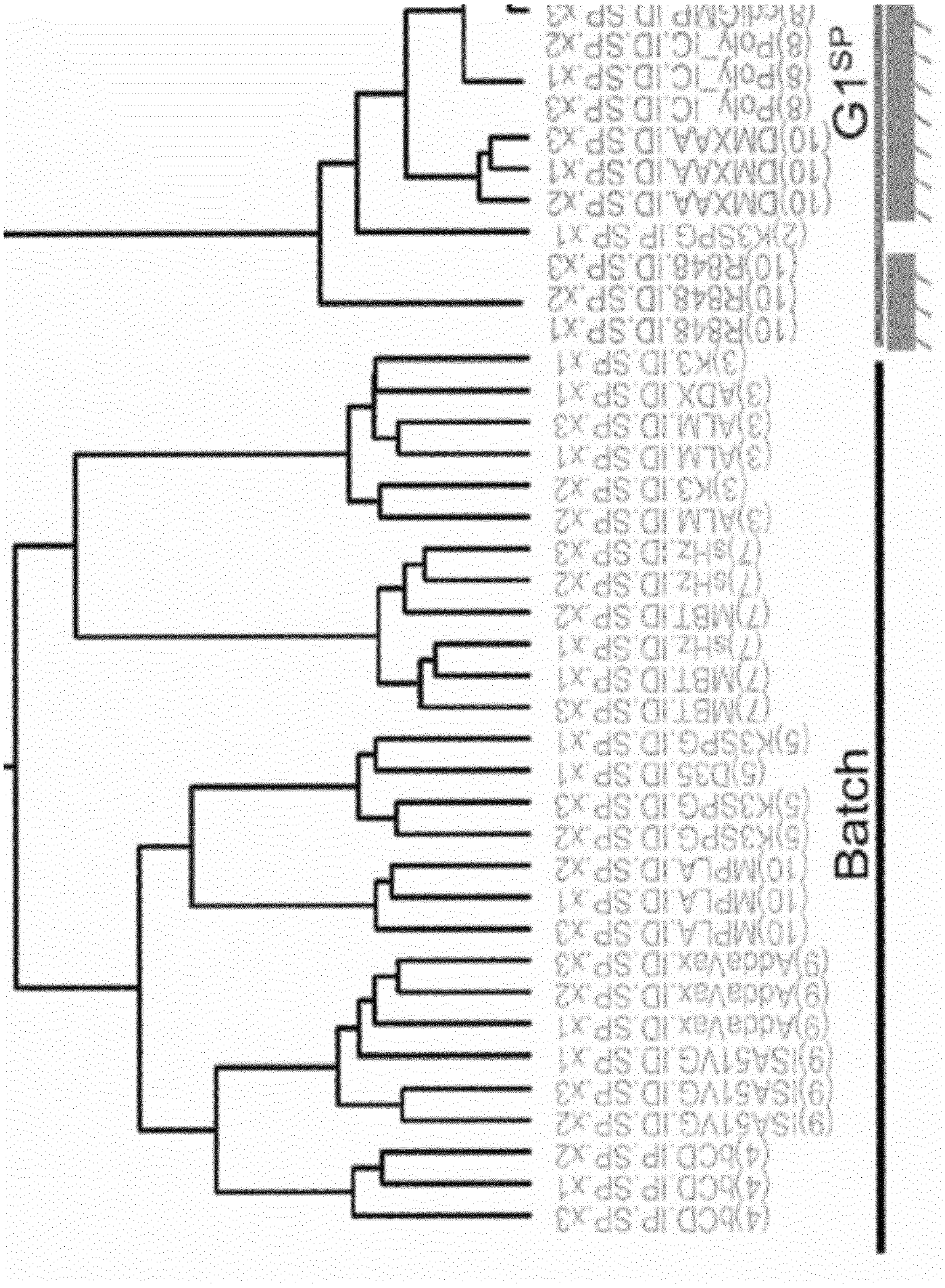
G1SP
Batch
(8)cdiGMP.ID.SP.x3
(8)Poly_IC.ID.SP.x2
(8)Poly_IC.ID.SP.x1
(8)Poly_IC.ID.SP.x3
(10)DMXAA.ID.SP.x3
(10)DMXAA.ID.SP.x1
(10)DMXAA.ID.SP.x2
(2)K3SPG.IP.SP.x1
(10)R848.ID.SP.x3
(10)R848.ID.SP.x2
(10)R848.ID.SP.x1
(3)K3.ID.SP.x1
(3)ADX.ID.SP.x1
(3)ALM.ID.SP.x3
(3)ALM.ID.SP.x1
(3)K3.ID.SP.x2
(3)ALM.ID.SP.x2
(7)sHz.ID.SP.x3
(7)sHz.ID.SP.x2
(7)MBT.ID.SP.x2
(7)sHz.ID.SP.x1
(7)MBT.ID.SP.x1
(7)MBT.ID.SP.x3
(5)K3SPG.ID.SP.x1
(5)D35.ID.SP.x1
(5)K3SPG.ID.SP.x3
(5)K3SPG.ID.SP.x2
(10)MPLA.ID.SP.x2
(10)MPLA.ID.SP.x1
(10)MPLA.ID.SP.x3
(6)AddaVax.ID.SP.x3
(6)AddaVax.ID.SP.x2
(6)AddaVax.ID.SP.x1
(9)ISA51VG.ID.SP.x1
(9)ISA51VG.ID.SP.x3
(9)ISA51VG.ID.SP.x2
(4)bCD.IP.SP.x2
(4)bCD.IP.SP.x1
(4)bCD.IP.SP.x3

[FIG.2H]

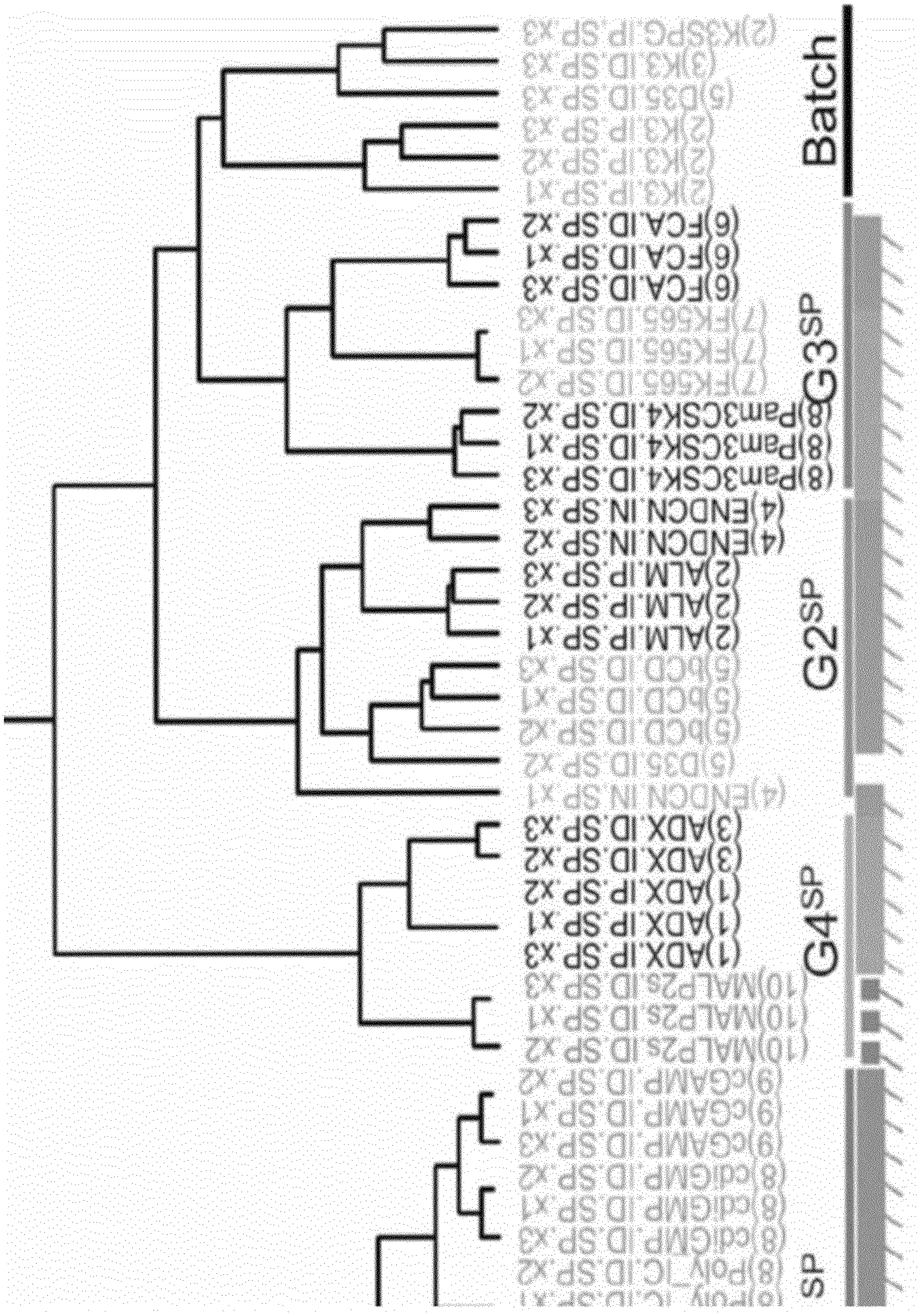

[FIG.2I]

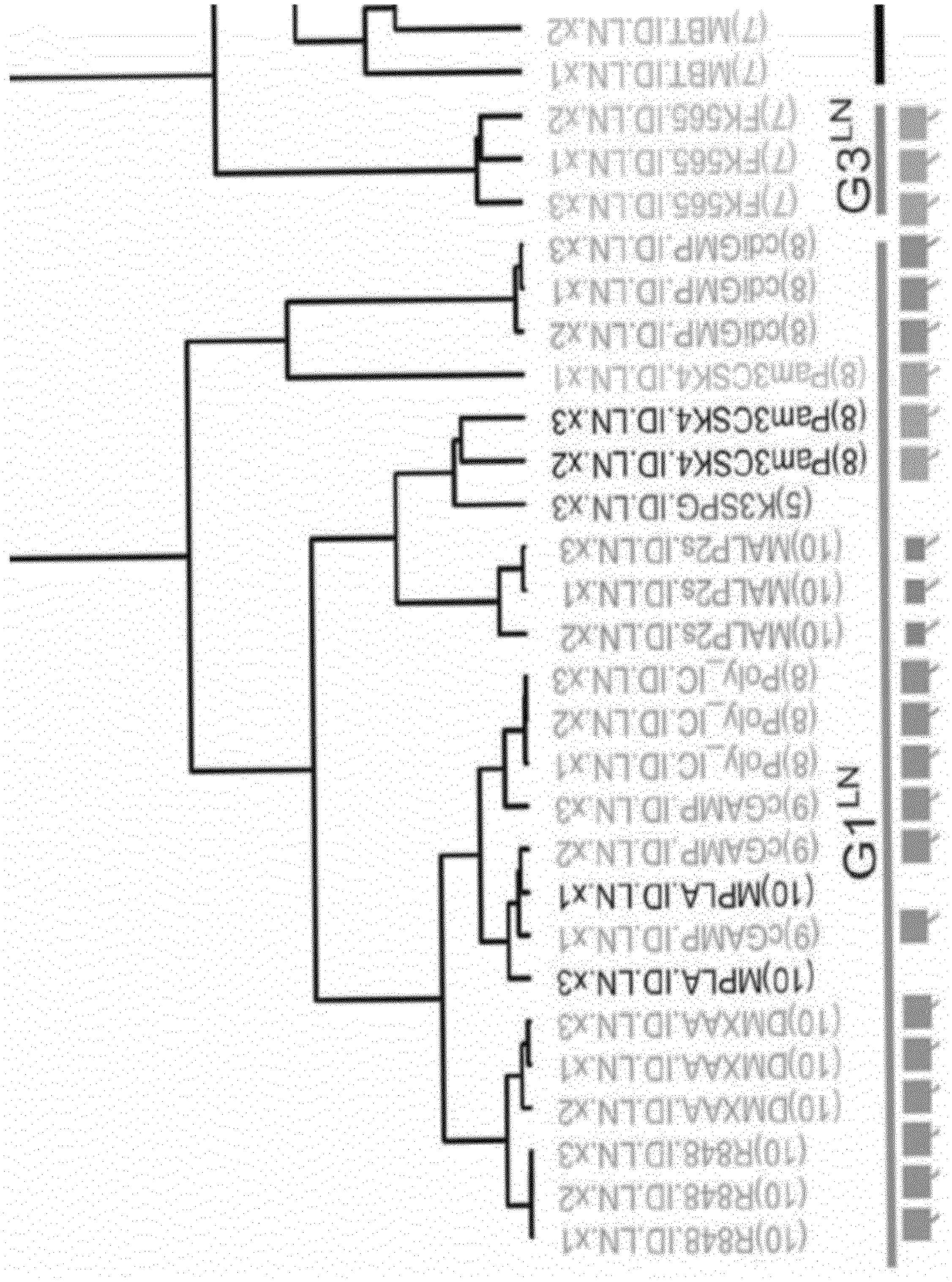
(7)MBT.ID.LN.x2
(7)MBT.ID.LN.x1
(7)FK565.ID.LN.x2
(7)FK565.ID.LN.x1
(7)FK565.ID.LN.x3
G3LN
(8)cdiGMP.ID.LN.x3
(8)cdiGMP.ID.LN.x1
(8)cdiGMP.ID.LN.x2
(8)Pam3CSK4.ID.LN.x1
(8)Pam3CSK4.ID.LN.x3
(8)Pam3CSK4.ID.LN.x2
(5)K3SPG.ID.LN.x3
(10)MALP2s.ID.LN.x3
(10)MALP2s.ID.LN.x1
(10)MALP2s.ID.LN.x2
(8)Poly_IC.ID.LN.x3
(8)Poly_IC.ID.LN.x2
(8)Poly_IC.ID.LN.x1
(9)cGAMP.ID.LN.x3
(9)cGAMP.ID.LN.x2
(10)MPLA.ID.LN.x1
(9)cGAMP.ID.LN.x1
(10)MPLA.ID.LN.x3
(10)DMXAA.ID.LN.x3
(10)DMXAA.ID.LN.x1
(10)DMXAA.ID.LN.x2
(10)R848.ID.LN.x3
(10)R848.ID.LN.x2
(10)R848.ID.LN.x1
G1LN

[FIG.2J]

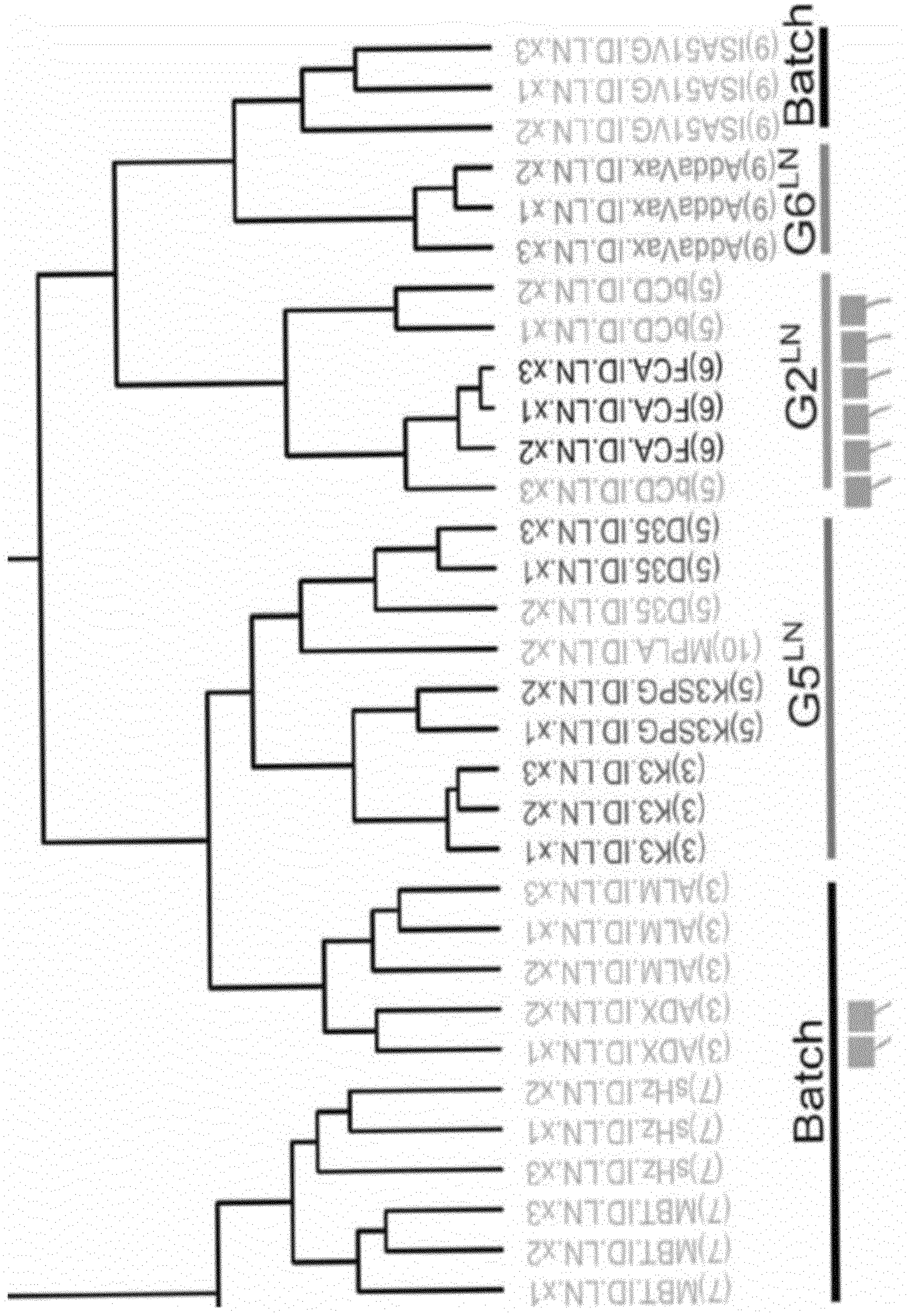
(9)ISA51VG.ID.LN.x3
(9)ISA51VG.ID.LN.x1
(9)ISA51VG.ID.LN.x2
(9)AddaVax.ID.LN.x2
(9)AddaVax.ID.LN.x1
(9)AddaVax.ID.LN.x3
(5)bCD.ID.LN.x2
(5)bCD.ID.LN.x1
(6)FCA.ID.LN.x3
(6)FCA.ID.LN.x1
(6)FCA.ID.LN.x2
(5)bCD.ID.LN.x3
(5)D35.ID.LN.x3
(5)D35.ID.LN.x1
(5)D35.ID.LN.x2
(10)MPLA.ID.LN.x2
(5)K3SPG.ID.LN.x2
(5)K3SPG.ID.LN.x1
(3)K3.ID.LN.x3
(3)K3.ID.LN.x2
(3)K3.ID.LN.x1
(3)ALM.ID.LN.x3
(3)ALM.ID.LN.x1
(3)ALM.ID.LN.x2
(3)ADX.ID.LN.x2
(3)ADX.ID.LN.x1
(7)sHz.ID.LN.x2
(7)sHz.ID.LN.x1
(7)sHz.ID.LN.x3
(7)MBT.ID.LN.x3
(7)MBT.ID.LN.x2
(7)MBT.ID.LN.x1
Batch
G6LN
G2LN
G5LN
Batch

[FIG.2K]

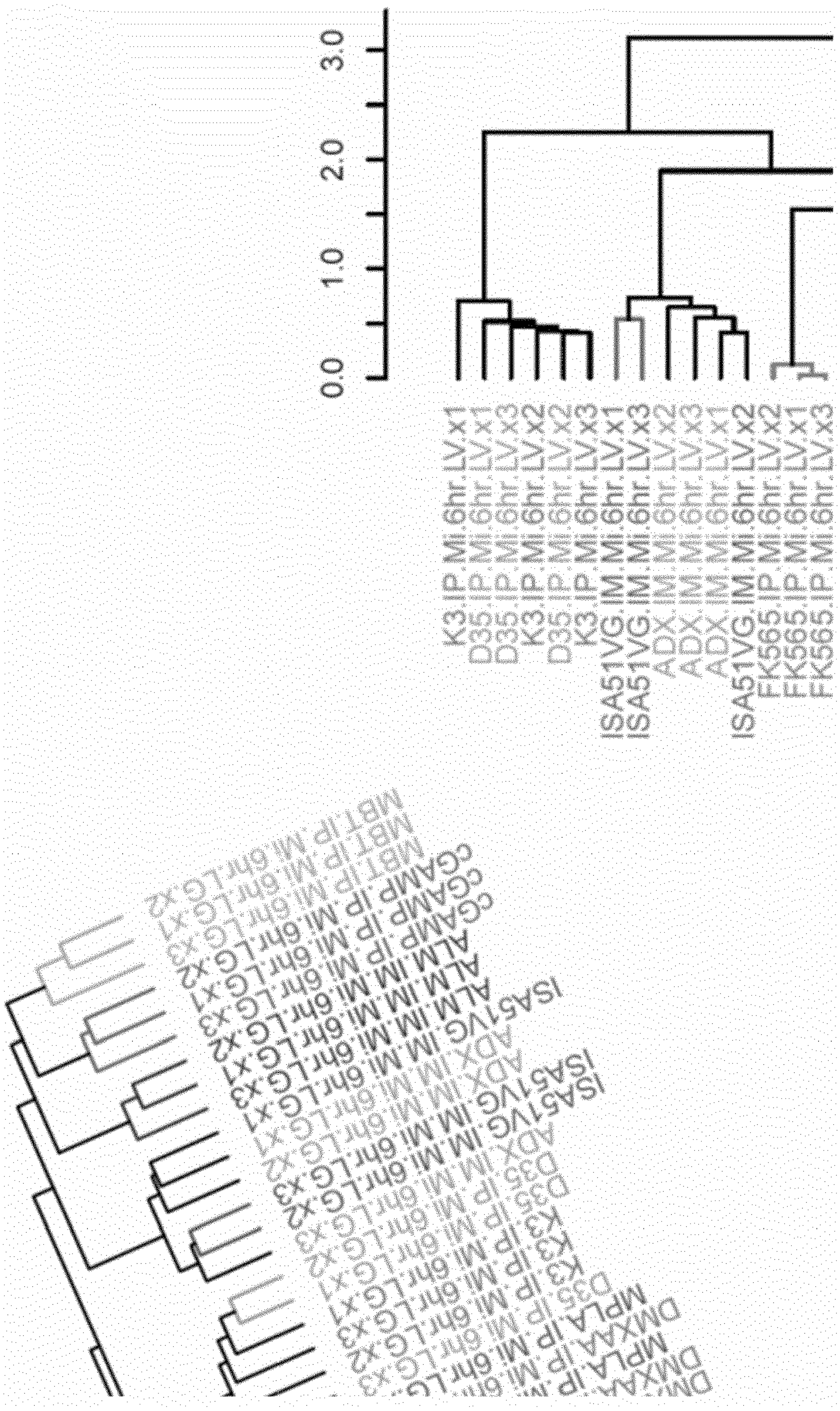

0.0
1.0
2.0
3.0
K3.IP.Mi.6hr.LV.x1
D35.IP.Mi.6hr.LV.x1
D35.IP.Mi.6hr.LV.x3
K3.IP.Mi.6hr.LV.x2
D35.IP.Mi.6hr.LV.x2
K3.IP.Mi.6hr.LV.x3
ISA51VG.IM.Mi.6hr.LV.x1
ISA51VG.IM.Mi.6hr.LV.x3
ADX.IM.Mi.6hr.LV.x2
ADX.IM.Mi.6hr.LV.x3
ADX.IM.Mi.6hr.LV.x1
ISA51VG.IM.Mi.6hr.LV.x2
FK565.IP.Mi.6hr.LV.x2
FK565.IP.Mi.6hr.LV.x1
FK565.IP.Mi.6hr.LV.x3

[FIG. 2K](**Continue**)

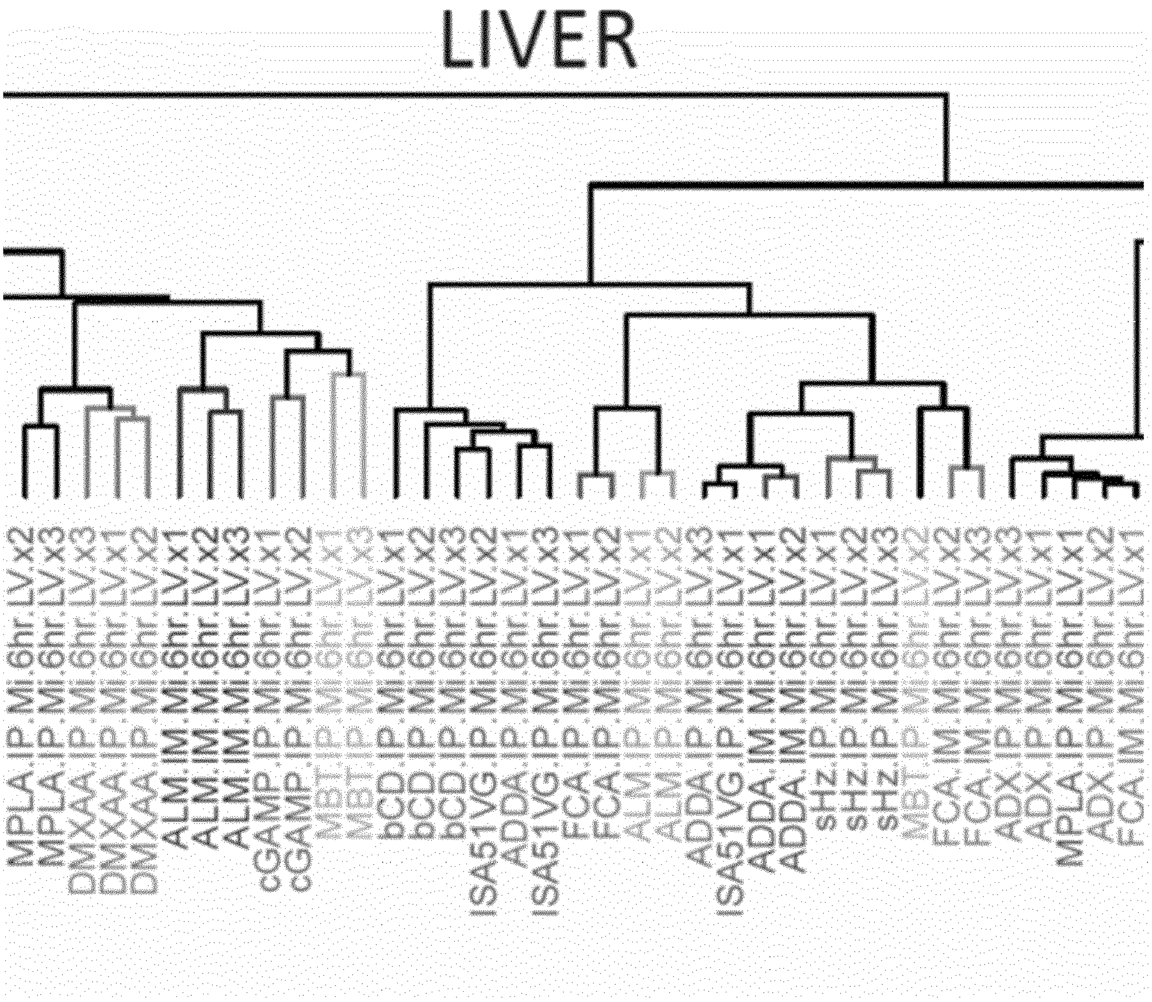

[FIG. 2K](**Continue**)

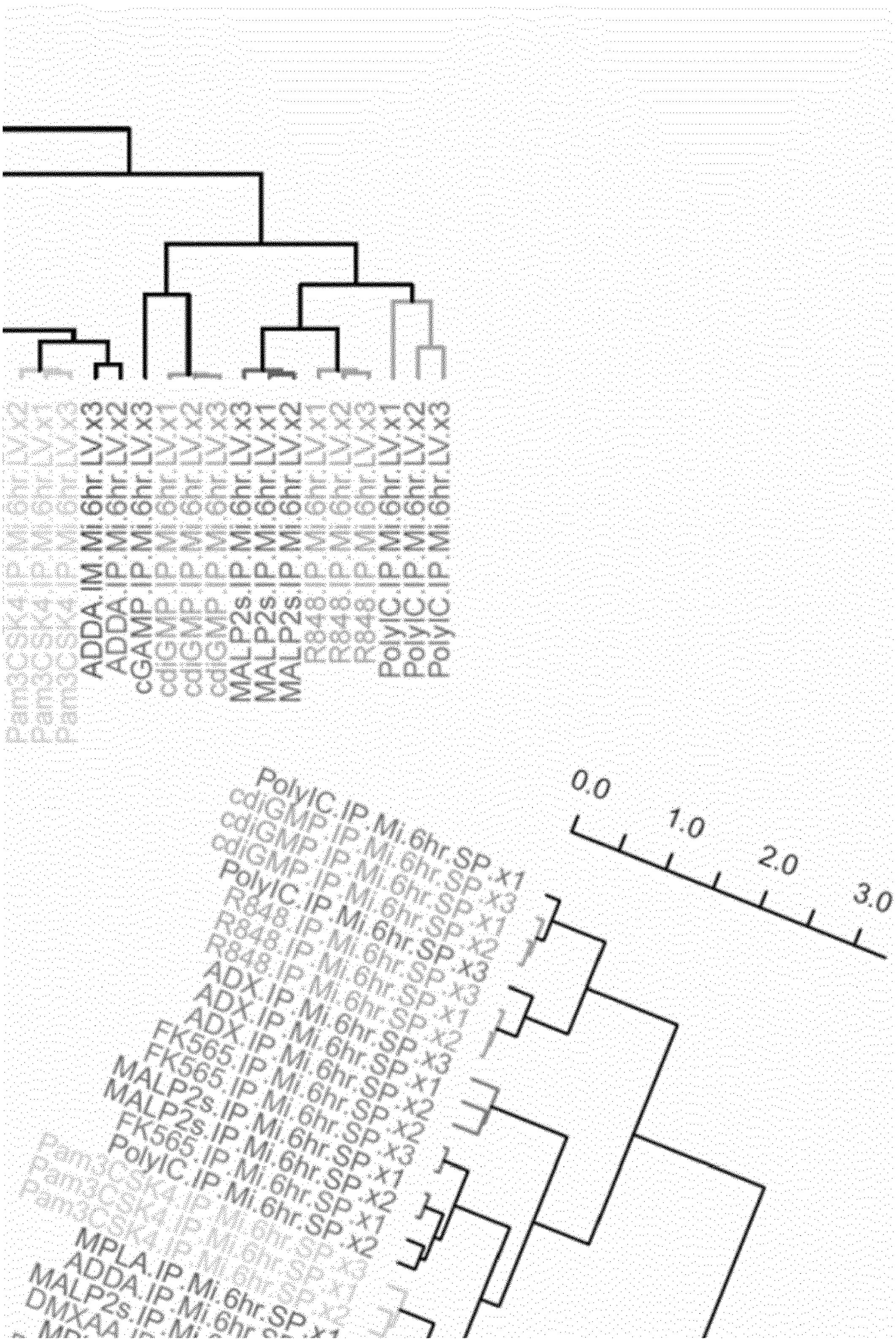

[FIG. 2K](**Continue**)

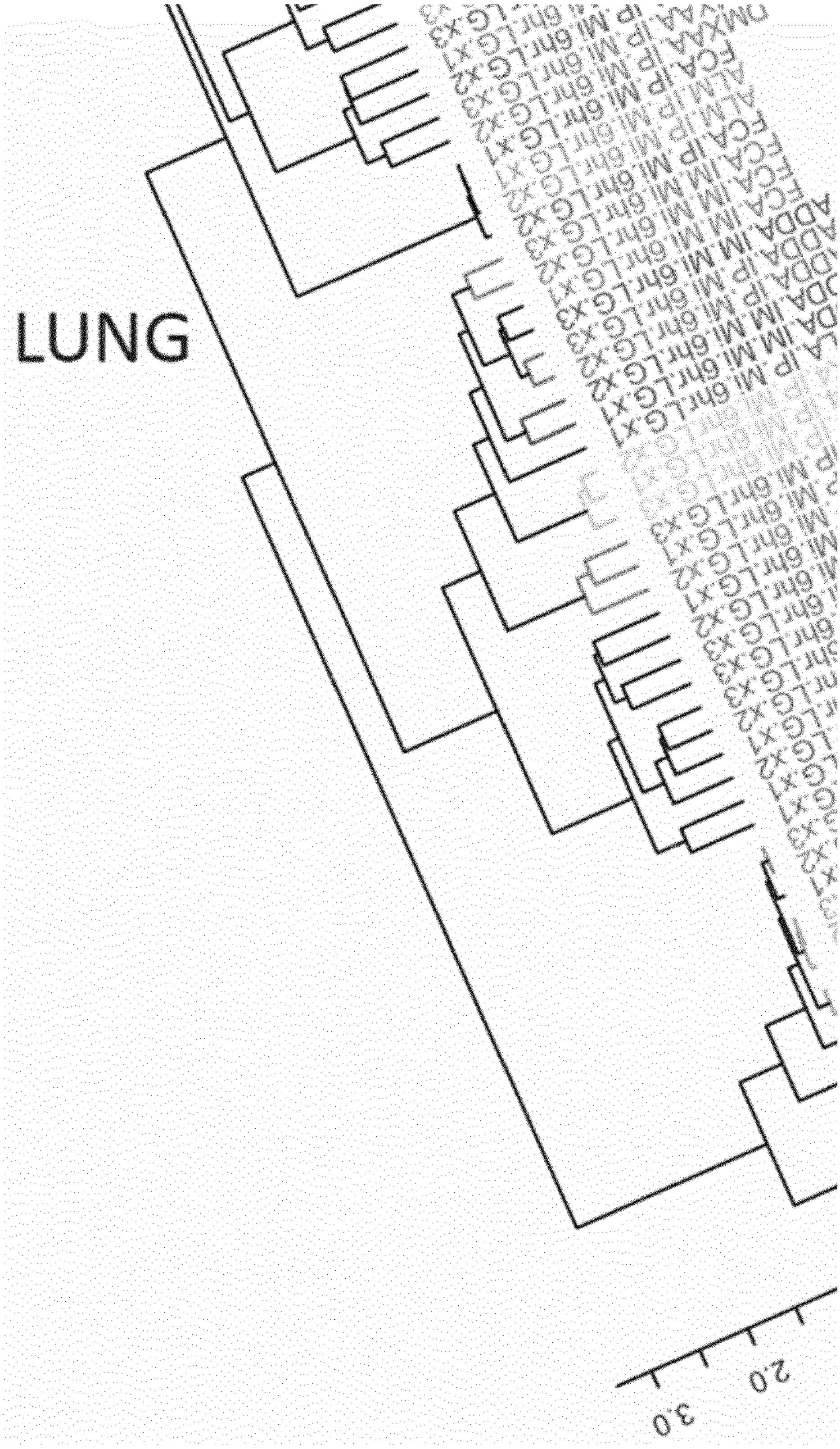

[FIG.2K](**Continue**)

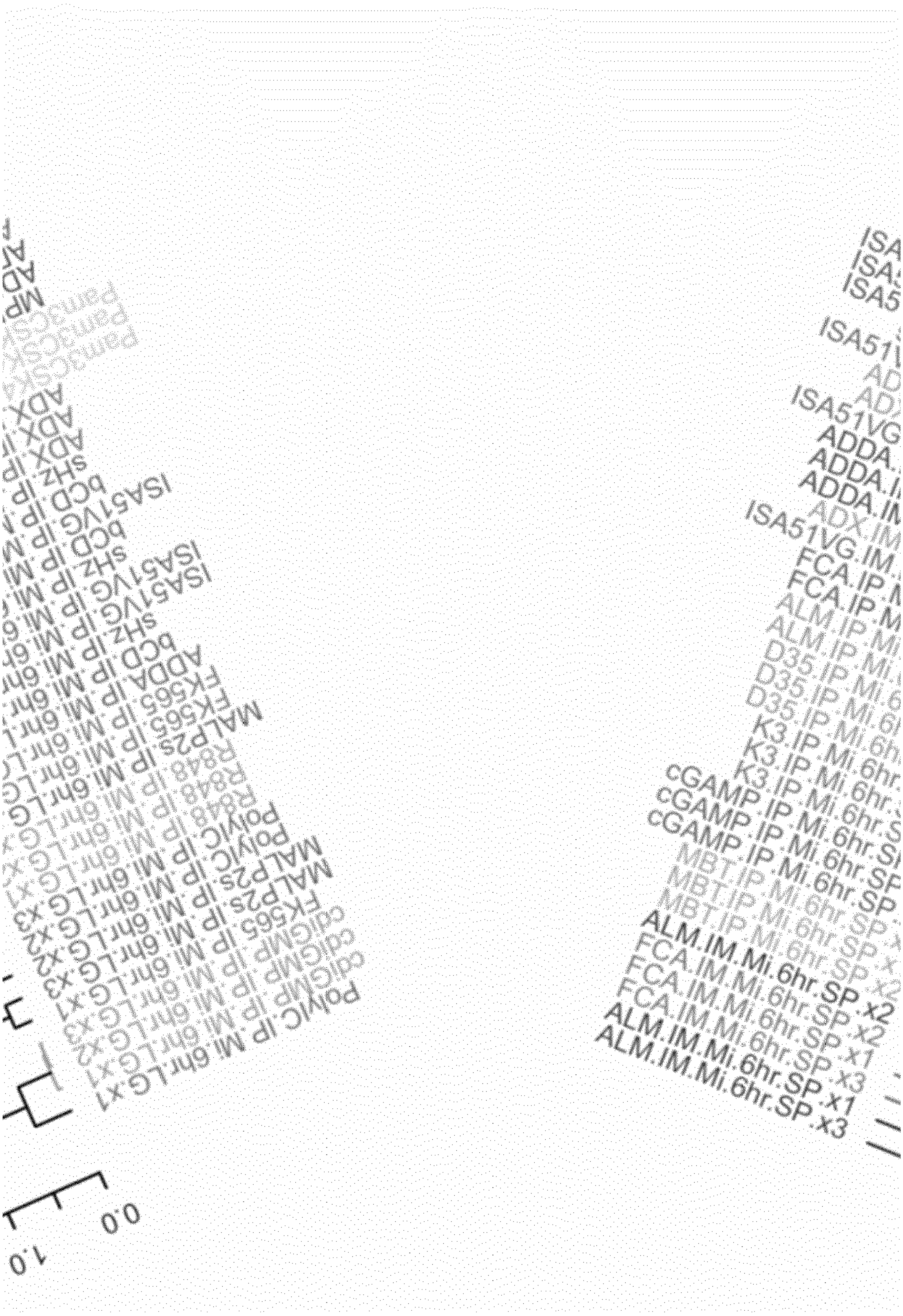

[FIG.2K](**Continue**)

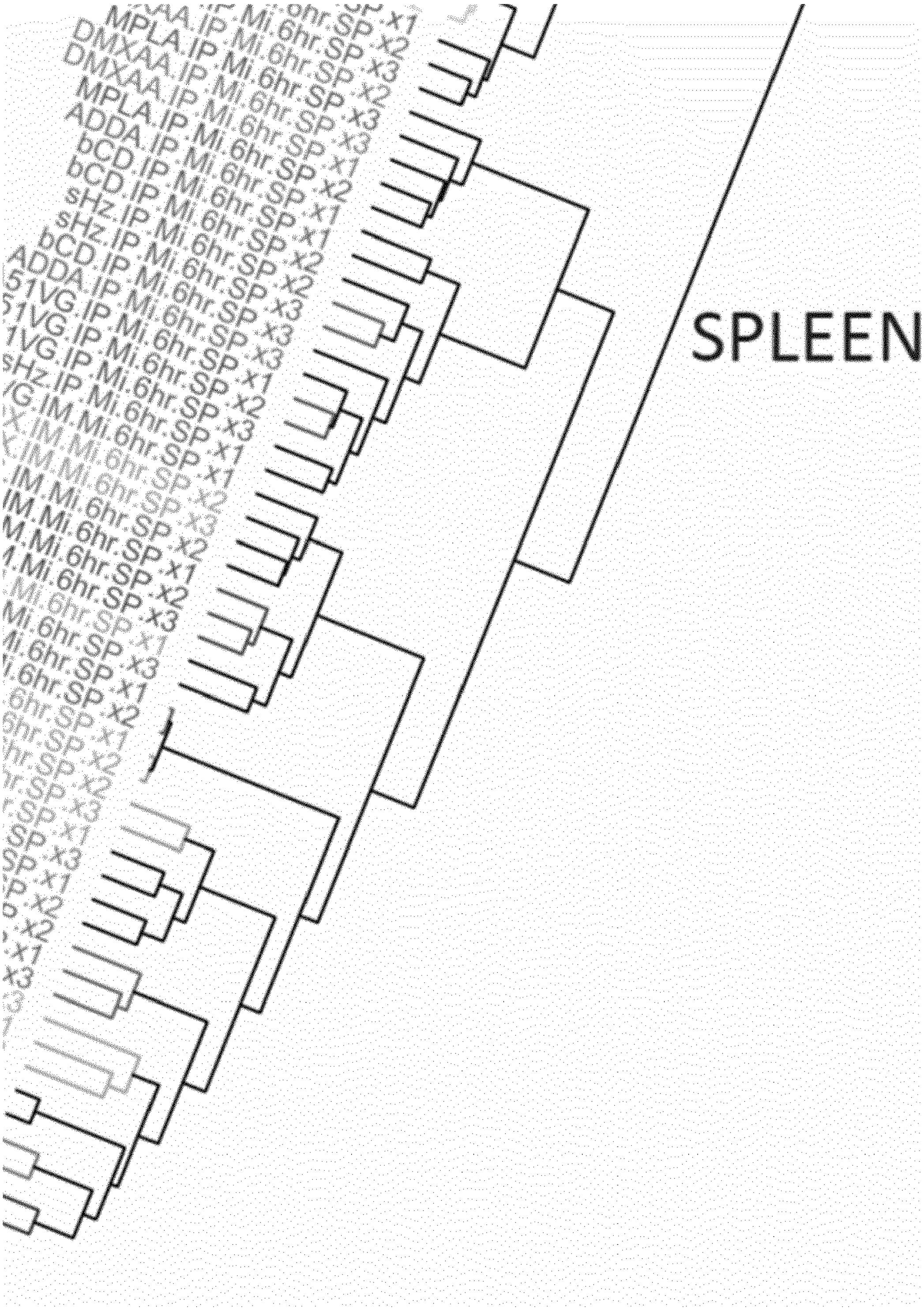

[FIG.2L]

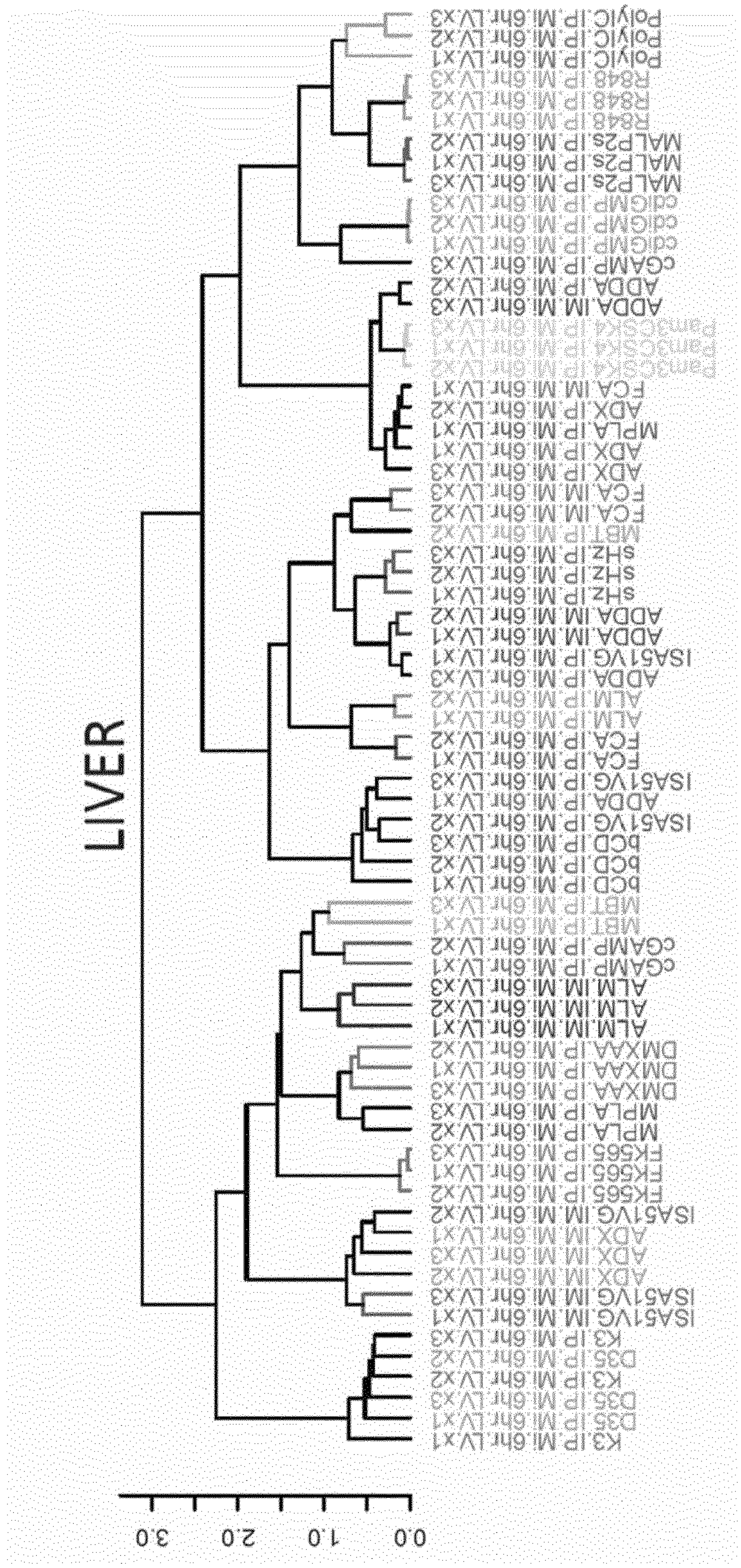
LIVER
PolyIC.IP.Mi.6hr.LV.x3
PolyIC.IP.Mi.6hr.LV.x2
PolyIC.IP.Mi.6hr.LV.x1
R848.IP.Mi.6hr.LV.x3
R848.IP.Mi.6hr.LV.x2
R848.IP.Mi.6hr.LV.x1
MALP2s.IP.Mi.6hr.LV.x2
MALP2s.IP.Mi.6hr.LV.x1
MALP2s.IP.Mi.6hr.LV.x3
cdiGMP.IP.Mi.6hr.LV.x3
cdiGMP.IP.Mi.6hr.LV.x2
cdiGMP.IP.Mi.6hr.LV.x1
cGAMP.IP.Mi.6hr.LV.x3
ADDA.IP.Mi.6hr.LV.x2
ADDA.IM.Mi.6hr.LV.x3
Pam3CSK4.IP.Mi.6hr.LV.x3
Pam3CSK4.IP.Mi.6hr.LV.x1
Pam3CSK4.IP.Mi.6hr.LV.x2
FCA.IM.Mi.6hr.LV.x1
ADX.IP.Mi.6hr.LV.x2
MPLA.IP.Mi.6hr.LV.x1
ADX.IP.Mi.6hr.LV.x1
ADX.IP.Mi.6hr.LV.x3
FCA.IM.Mi.6hr.LV.x3
FCA.IM.Mi.6hr.LV.x2
MBT.IP.Mi.6hr.LV.x2
sHz.IP.Mi.6hr.LV.x3
sHz.IP.Mi.6hr.LV.x2
sHz.IP.Mi.6hr.LV.x1
ADDA.IM.Mi.6hr.LV.x2
ADDA.IM.Mi.6hr.LV.x1
ISA51VG.IP.Mi.6hr.LV.x1
ADDA.IP.Mi.6hr.LV.x3
ALM.IP.Mi.6hr.LV.x2
ALM.IP.Mi.6hr.LV.x1
FCA.IP.Mi.6hr.LV.x2
FCA.IP.Mi.6hr.LV.x1
ISA51VG.IP.Mi.6hr.LV.x3
ADDA.IP.Mi.6hr.LV.x1
ISA51VG.IP.Mi.6hr.LV.x2
bCD.IP.Mi.6hr.LV.x3
bCD.IP.Mi.6hr.LV.x2
bCD.IP.Mi.6hr.LV.x1
MBT.IP.Mi.6hr.LV.x3
MBT.IP.Mi.6hr.LV.x1
cGAMP.IP.Mi.6hr.LV.x2
cGAMP.IP.Mi.6hr.LV.x1
ALM.IM.Mi.6hr.LV.x3
ALM.IM.Mi.6hr.LV.x2
ALM.IM.Mi.6hr.LV.x1
DMXAA.IP.Mi.6hr.LV.x2
DMXAA.IP.Mi.6hr.LV.x1
DMXAA.IP.Mi.6hr.LV.x3
MPLA.IP.Mi.6hr.LV.x3
MPLA.IP.Mi.6hr.LV.x2
FK565.IP.Mi.6hr.LV.x3
FK565.IP.Mi.6hr.LV.x1
FK565.IP.Mi.6hr.LV.x2
ISA51VG.IM.Mi.6hr.LV.x2
ADX.IM.Mi.6hr.LV.x1
ADX.IM.Mi.6hr.LV.x3
ADX.IM.Mi.6hr.LV.x2
ISA51VG.IM.Mi.6hr.LV.x3
ISA51VG.IM.Mi.6hr.LV.x1
K3.IP.Mi.6hr.LV.x3
D35.IP.Mi.6hr.LV.x2
K3.IP.Mi.6hr.LV.x2
D35.IP.Mi.6hr.LV.x3
D35.IP.Mi.6hr.LV.x1
K3.IP.Mi.6hr.LV.x1
0.0
1.0
2.0
3.0

[FIG.2M]

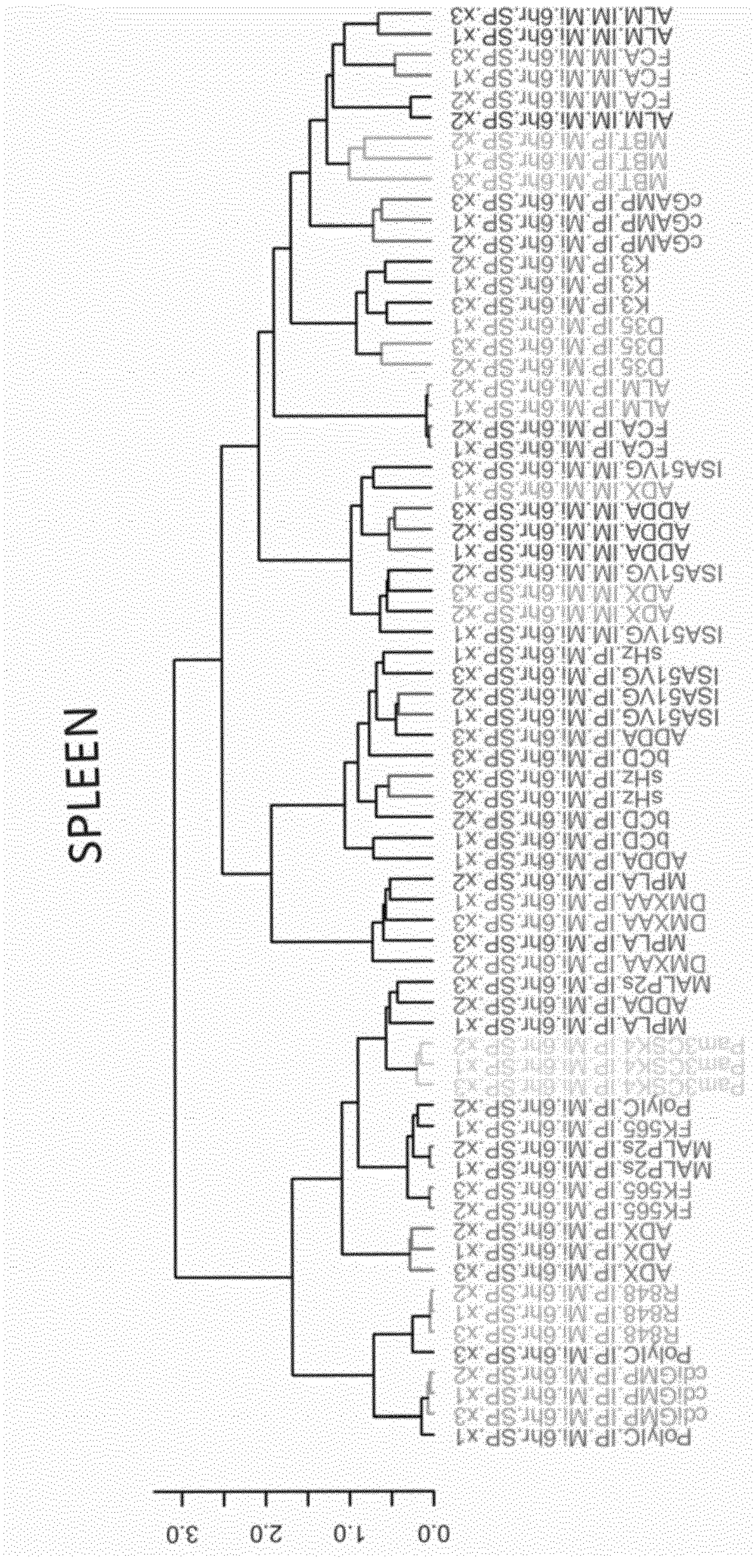
SPLEEN
ALM.IM.Mi.6hr.SP.x3
ALM.IM.Mi.6hr.SP.x1
FCA.IM.Mi.6hr.SP.x3
FCA.IM.Mi.6hr.SP.x1
FCA.IM.Mi.6hr.SP.x2
ALM.IM.Mi.6hr.SP.x2
MBT.IP.Mi.6hr.SP.x2
MBT.IP.Mi.6hr.SP.x1
MBT.IP.Mi.6hr.SP.x3
cGAMP.IP.Mi.6hr.SP.x3
cGAMP.IP.Mi.6hr.SP.x1
cGAMP.IP.Mi.6hr.SP.x2
K3.IP.Mi.6hr.SP.x2
K3.IP.Mi.6hr.SP.x1
K3.IP.Mi.6hr.SP.x3
D35.IP.Mi.6hr.SP.x1
D35.IP.Mi.6hr.SP.x3
D35.IP.Mi.6hr.SP.x2
ALM.IP.Mi.6hr.SP.x2
ALM.IP.Mi.6hr.SP.x1
FCA.IP.Mi.6hr.SP.x2
FCA.IP.Mi.6hr.SP.x1
ISA51VG.IM.Mi.6hr.SP.x3
ADX.IM.Mi.6hr.SP.x1
ADDA.IM.Mi.6hr.SP.x3
ADDA.IM.Mi.6hr.SP.x2
ADDA.IM.Mi.6hr.SP.x1
ISA51VG.IM.Mi.6hr.SP.x2
ADX.IM.Mi.6hr.SP.x3
ADX.IM.Mi.6hr.SP.x2
ISA51VG.IM.Mi.6hr.SP.x1
sHz.IP.Mi.6hr.SP.x1
ISA51VG.IP.Mi.6hr.SP.x3
ISA51VG.IP.Mi.6hr.SP.x2
ISA51VG.IP.Mi.6hr.SP.x1
ADDA.IP.Mi.6hr.SP.x3
bCD.IP.Mi.6hr.SP.x3
sHz.IP.Mi.6hr.SP.x3
sHz.IP.Mi.6hr.SP.x2
bCD.IP.Mi.6hr.SP.x2
bCD.IP.Mi.6hr.SP.x1
ADDA.IP.Mi.6hr.SP.x1
MPLA.IP.Mi.6hr.SP.x2
DMXAA.IP.Mi.6hr.SP.x1
DMXAA.IP.Mi.6hr.SP.x3
MPLA.IP.Mi.6hr.SP.x3
DMXAA.IP.Mi.6hr.SP.x2
MALP2s.IP.Mi.6hr.SP.x3
ADDA.IP.Mi.6hr.SP.x2
MPLA.IP.Mi.6hr.SP.x1
Pam3CSK4.IP.Mi.6hr.SP.x2
Pam3CSK4.IP.Mi.6hr.SP.x1
Pam3CSK4.IP.Mi.6hr.SP.x3
PolyIC.IP.Mi.6hr.SP.x2
FK565.IP.Mi.6hr.SP.x1
MALP2s.IP.Mi.6hr.SP.x2
MALP2s.IP.Mi.6hr.SP.x1
FK565.IP.Mi.6hr.SP.x3
FK565.IP.Mi.6hr.SP.x2
ADX.IP.Mi.6hr.SP.x2
ADX.IP.Mi.6hr.SP.x1
ADX.IP.Mi.6hr.SP.x3
R848.IP.Mi.6hr.SP.x2
R848.IP.Mi.6hr.SP.x1
R848.IP.Mi.6hr.SP.x3
PolyIC.IP.Mi.6hr.SP.x3
cdiGMP.IP.Mi.6hr.SP.x2
cdiGMP.IP.Mi.6hr.SP.x1
cdiGMP.IP.Mi.6hr.SP.x3
PolyIC.IP.Mi.6hr.SP.x1
0.0
1.0
2.0
3.0

[FIG.2N]

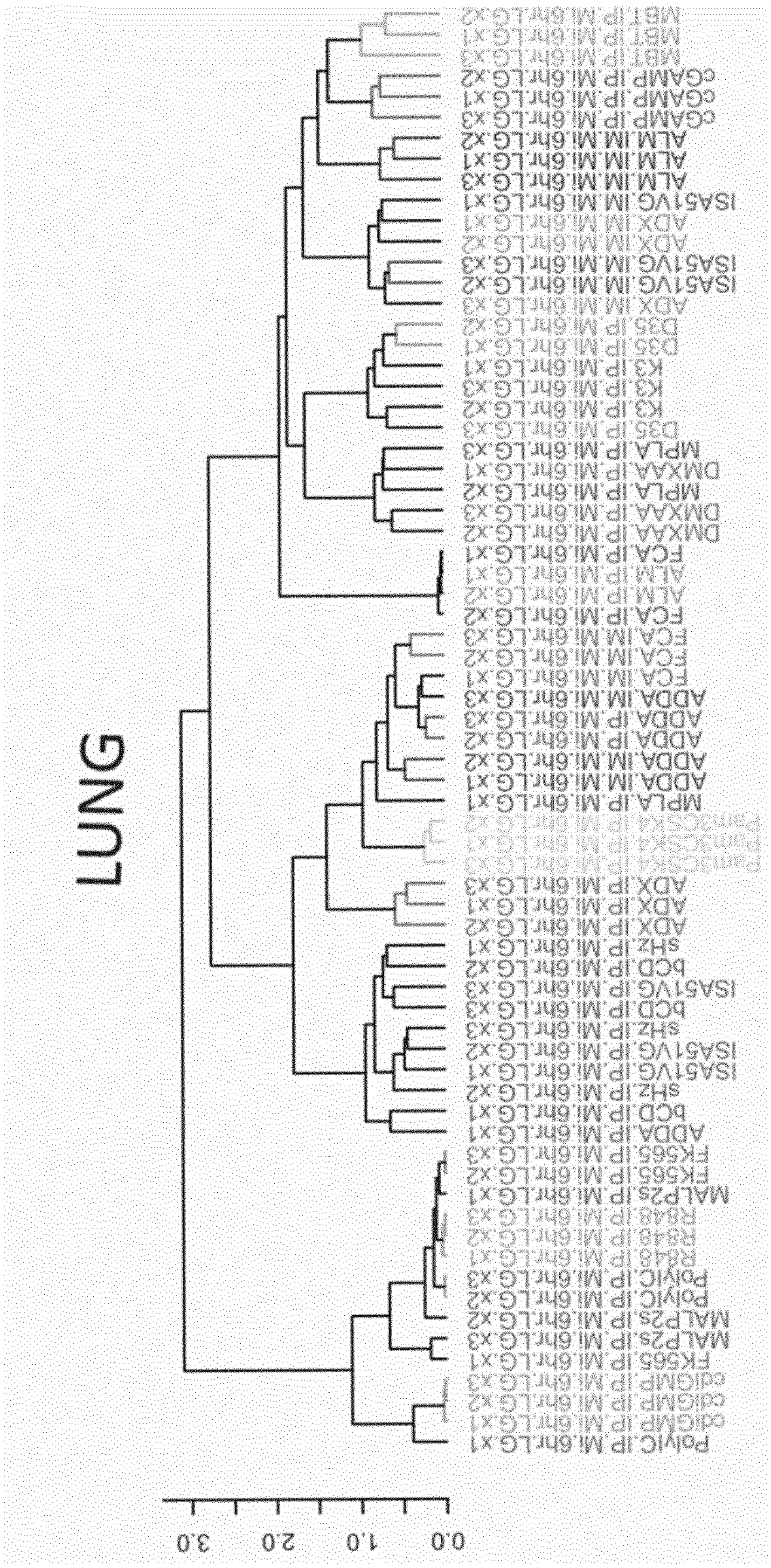
LUNG
MBT.IP.Mi.6hr.LG.x2
MBT.IP.Mi.6hr.LG.x1
MBT.IP.Mi.6hr.LG.x3
cGAMP.IP.Mi.6hr.LG.x2
cGAMP.IP.Mi.6hr.LG.x1
cGAMP.IP.Mi.6hr.LG.x3
ALM.IM.Mi.6hr.LG.x2
ALM.IM.Mi.6hr.LG.x1
ALM.IM.Mi.6hr.LG.x3
ISA51VG.IM.Mi.6hr.LG.x1
ADX.IM.Mi.6hr.LG.x1
ADX.IM.Mi.6hr.LG.x2
ISA51VG.IM.Mi.6hr.LG.x3
ISA51VG.IM.Mi.6hr.LG.x2
ADX.IM.Mi.6hr.LG.x3
D35.IP.Mi.6hr.LG.x2
D35.IP.Mi.6hr.LG.x1
K3.IP.Mi.6hr.LG.x1
K3.IP.Mi.6hr.LG.x3
K3.IP.Mi.6hr.LG.x2
D35.IP.Mi.6hr.LG.x3
MPLA.IP.Mi.6hr.LG.x3
DMXAA.IP.Mi.6hr.LG.x1
MPLA.IP.Mi.6hr.LG.x2
DMXAA.IP.Mi.6hr.LG.x3
DMXAA.IP.Mi.6hr.LG.x2
FCA.IP.Mi.6hr.LG.x1
ALM.IP.Mi.6hr.LG.x1
ALM.IP.Mi.6hr.LG.x2
FCA.IP.Mi.6hr.LG.x2
FCA.IM.Mi.6hr.LG.x3
FCA.IM.Mi.6hr.LG.x2
FCA.IM.Mi.6hr.LG.x1
ADDA.IM.Mi.6hr.LG.x3
ADDA.IP.Mi.6hr.LG.x3
ADDA.IP.Mi.6hr.LG.x2
ADDA.IM.Mi.6hr.LG.x2
ADDA.IM.Mi.6hr.LG.x1
MPLA.IP.Mi.6hr.LG.x1
Pam3CSK4.IP.Mi.6hr.LG.x2
Pam3CSK4.IP.Mi.6hr.LG.x1
Pam3CSK4.IP.Mi.6hr.LG.x3
ADX.IP.Mi.6hr.LG.x3
ADX.IP.Mi.6hr.LG.x1
ADX.IP.Mi.6hr.LG.x2
sHz.IP.Mi.6hr.LG.x1
bCD.IP.Mi.6hr.LG.x2
ISA51VG.IP.Mi.6hr.LG.x3
bCD.IP.Mi.6hr.LG.x3
sHz.IP.Mi.6hr.LG.x3
ISA51VG.IP.Mi.6hr.LG.x2
ISA51VG.IP.Mi.6hr.LG.x1
sHz.IP.Mi.6hr.LG.x2
bCD.IP.Mi.6hr.LG.x1
ADDA.IP.Mi.6hr.LG.x1
FK565.IP.Mi.6hr.LG.x3
FK565.IP.Mi.6hr.LG.x2
MALP2s.IP.Mi.6hr.LG.x1
R848.IP.Mi.6hr.LG.x3
R848.IP.Mi.6hr.LG.x2
R848.IP.Mi.6hr.LG.x1
PolyIC.IP.Mi.6hr.LG.x3
PolyIC.IP.Mi.6hr.LG.x2
MALP2s.IP.Mi.6hr.LG.x2
MALP2s.IP.Mi.6hr.LG.x3
FK565.IP.Mi.6hr.LG.x1
cdiGMP.IP.Mi.6hr.LG.x3
cdiGMP.IP.Mi.6hr.LG.x2
cdiGMP.IP.Mi.6hr.LG.x1
PolyIC.IP.Mi.6hr.LG.x1
0.0
1.0
2.0
3.0

[FIG.2O]

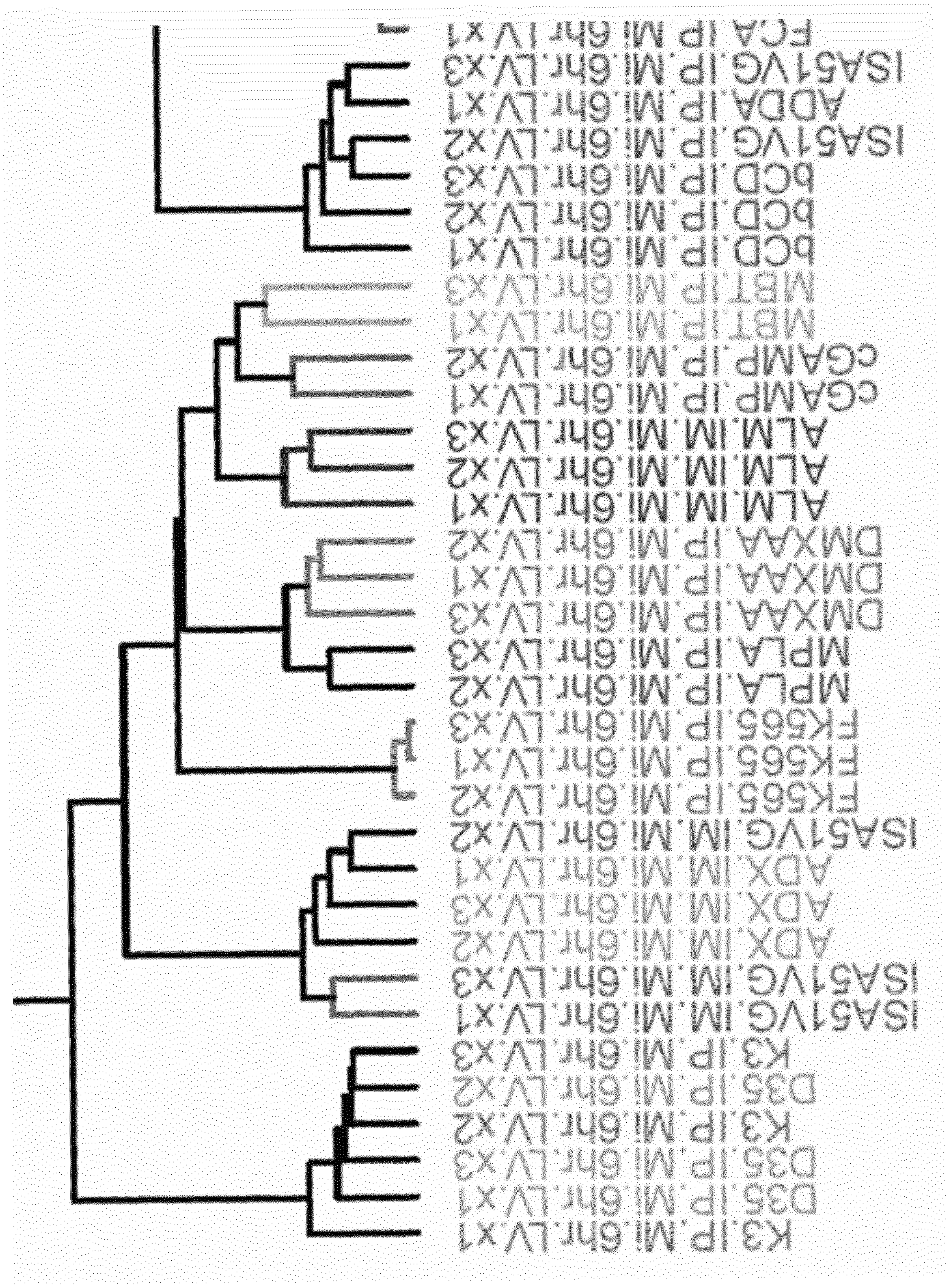
FCA.IP.Mi.6hr.LV.x1
ISA51VG.IP.Mi.6hr.LV.x3
ADDA.IP.Mi.6hr.LV.x1
ISA51VG.IP.Mi.6hr.LV.x2
bCD.IP.Mi.6hr.LV.x3
bCD.IP.Mi.6hr.LV.x2
bCD.IP.Mi.6hr.LV.x1
MBT.IP.Mi.6hr.LV.x3
MBT.IP.Mi.6hr.LV.x1
cGAMP.IP.Mi.6hr.LV.x2
cGAMP.IP.Mi.6hr.LV.x1
ALM.IM.Mi.6hr.LV.x3
ALM.IM.Mi.6hr.LV.x2
ALM.IM.Mi.6hr.LV.x1
DMXAA.IP.Mi.6hr.LV.x2
DMXAA.IP.Mi.6hr.LV.x1
DMXAA.IP.Mi.6hr.LV.x3
MPLA.IP.Mi.6hr.LV.x3
MPLA.IP.Mi.6hr.LV.x2
FK565.IP.Mi.6hr.LV.x3
FK565.IP.Mi.6hr.LV.x1
FK565.IP.Mi.6hr.LV.x2
ISA51VG.IM.Mi.6hr.LV.x2
ADX.IM.Mi.6hr.LV.x1
ADX.IM.Mi.6hr.LV.x3
ADX.IM.Mi.6hr.LV.x2
ISA51VG.IM.Mi.6hr.LV.x3
ISA51VG.IM.Mi.6hr.LV.x1
K3.IP.Mi.6hr.LV.x3
D35.IP.Mi.6hr.LV.x2
K3.IP.Mi.6hr.LV.x2
D35.IP.Mi.6hr.LV.x3
D35.IP.Mi.6hr.LV.x1
K3.IP.Mi.6hr.LV.x1

[FIG.2P]

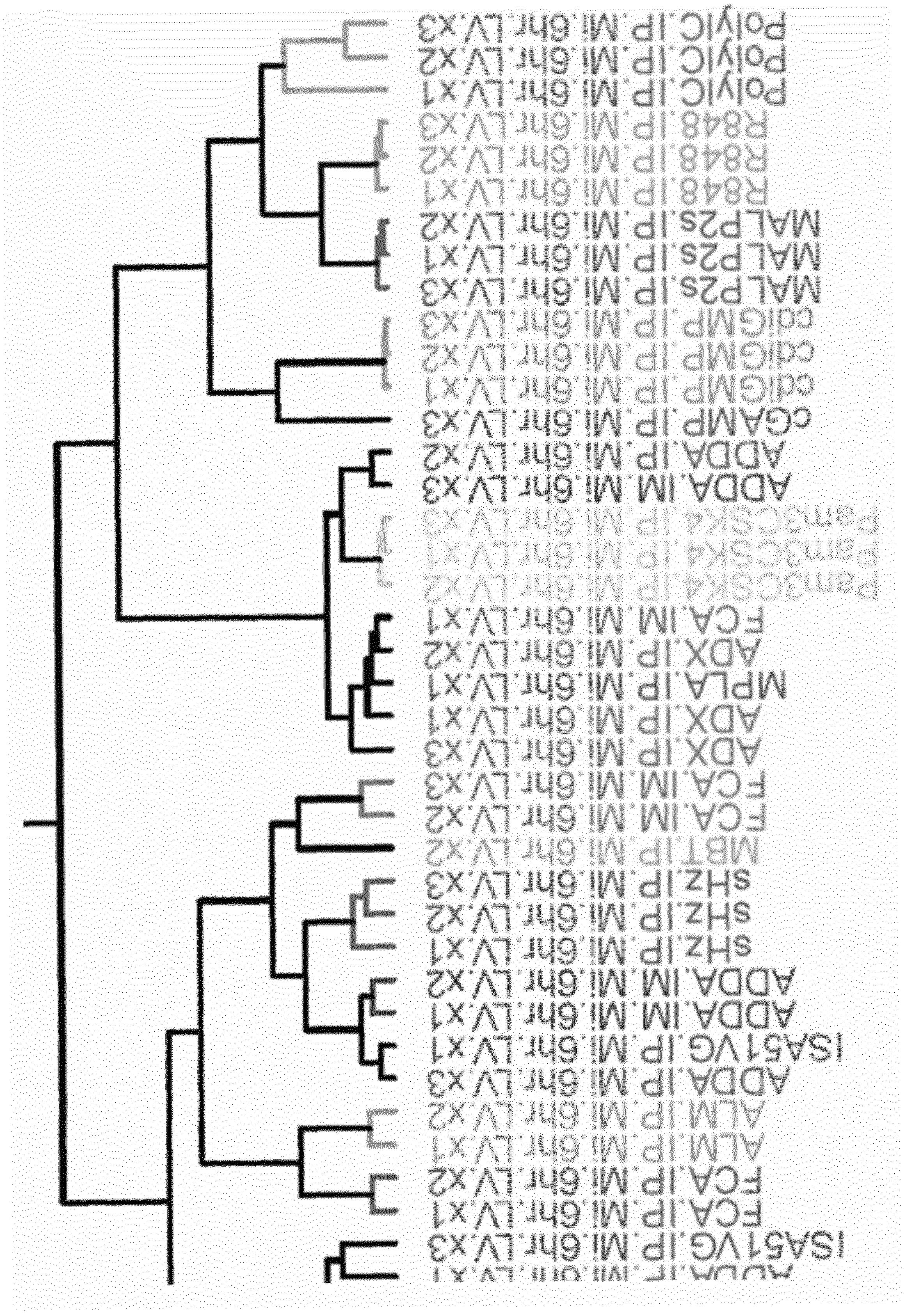
PolyIC.IP.Mi.6hr.LV.x3
PolyIC.IP.Mi.6hr.LV.x2
PolyIC.IP.Mi.6hr.LV.x1
R848.IP.Mi.6hr.LV.x3
R848.IP.Mi.6hr.LV.x2
R848.IP.Mi.6hr.LV.x1
MALP2s.IP.Mi.6hr.LV.x2
MALP2s.IP.Mi.6hr.LV.x1
MALP2s.IP.Mi.6hr.LV.x3
cdiGMP.IP.Mi.6hr.LV.x3
cdiGMP.IP.Mi.6hr.LV.x2
cdiGMP.IP.Mi.6hr.LV.x1
cGAMP.IP.Mi.6hr.LV.x3
ADDA.IP.Mi.6hr.LV.x2
ADDA.IM.Mi.6hr.LV.x3
Pam3CSK4.IP.Mi.6hr.LV.x3
Pam3CSK4.IP.Mi.6hr.LV.x1
Pam3CSK4.IP.Mi.6hr.LV.x2
FCA.IM.Mi.6hr.LV.x1
ADX.IP.Mi.6hr.LV.x2
MPLA.IP.Mi.6hr.LV.x1
ADX.IP.Mi.6hr.LV.x1
ADX.IP.Mi.6hr.LV.x3
FCA.IM.Mi.6hr.LV.x3
FCA.IM.Mi.6hr.LV.x2
MBT.IP.Mi.6hr.LV.x2
sHz.IP.Mi.6hr.LV.x3
sHz.IP.Mi.6hr.LV.x2
sHz.IP.Mi.6hr.LV.x1
ADDA.IM.Mi.6hr.LV.x2
ADDA.IM.Mi.6hr.LV.x1
ISA51VG.IP.Mi.6hr.LV.x1
ADDA.IP.Mi.6hr.LV.x3
ALM.IP.Mi.6hr.LV.x2
ALM.IP.Mi.6hr.LV.x1
FCA.IP.Mi.6hr.LV.x2
FCA.IP.Mi.6hr.LV.x1
ISA51VG.IP.Mi.6hr.LV.x3

[FIG.2Q]

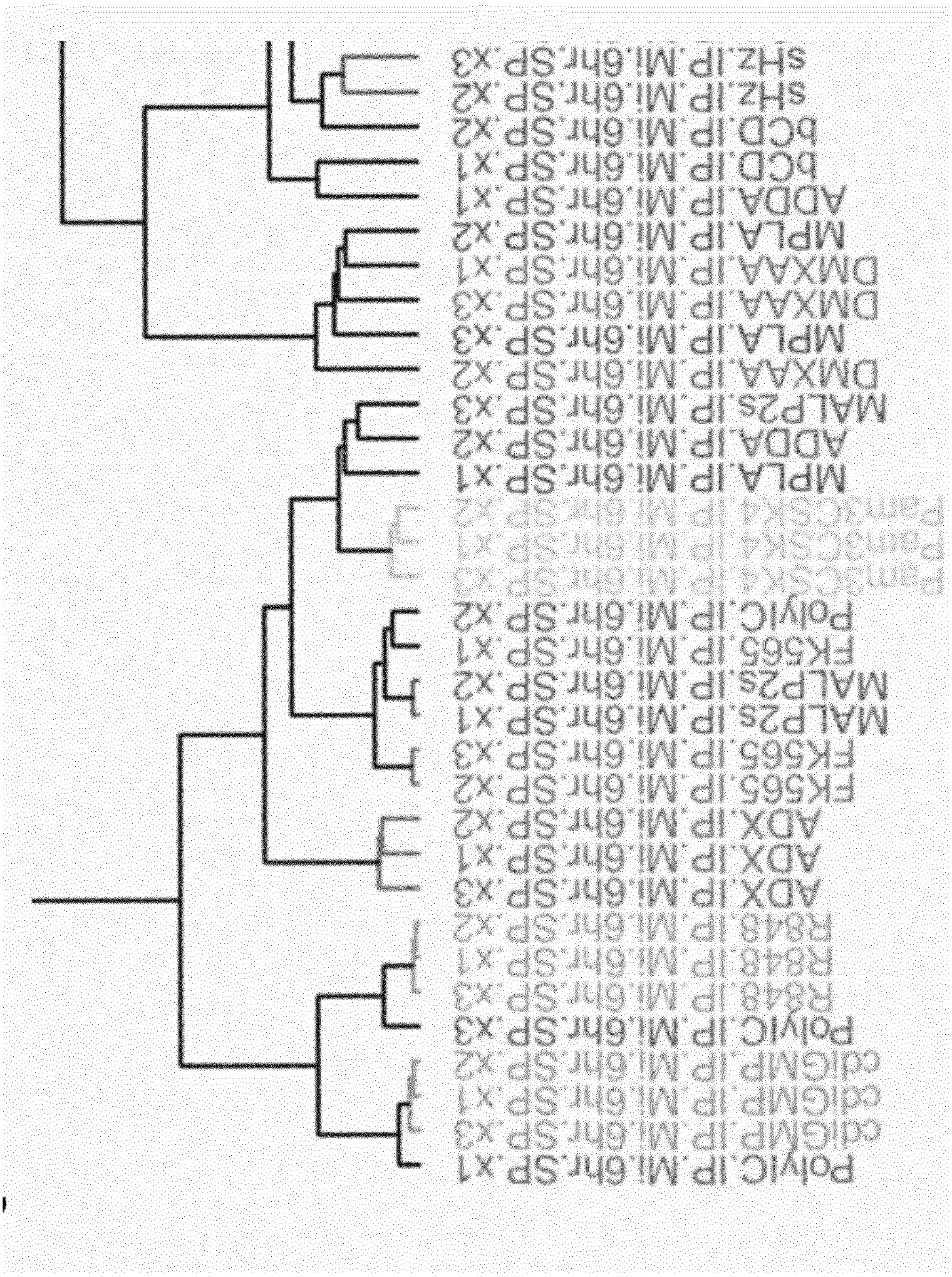
sHz.IP.Mi.6hr.SP.x3
sHz.IP.Mi.6hr.SP.x2
bCD.IP.Mi.6hr.SP.x2
bCD.IP.Mi.6hr.SP.x1
ADDA.IP.Mi.6hr.SP.x1
MPLA.IP.Mi.6hr.SP.x2
DMXAA.IP.Mi.6hr.SP.x1
DMXAA.IP.Mi.6hr.SP.x3
MPLA.IP.Mi.6hr.SP.x3
DMXAA.IP.Mi.6hr.SP.x2
MALP2s.IP.Mi.6hr.SP.x3
ADDA.IP.Mi.6hr.SP.x2
MPLA.IP.Mi.6hr.SP.x1
Pam3CSK4.IP.Mi.6hr.SP.x2
Pam3CSK4.IP.Mi.6hr.SP.x1
Pam3CSK4.IP.Mi.6hr.SP.x3
PolyIC.IP.Mi.6hr.SP.x2
FK565.IP.Mi.6hr.SP.x1
MALP2s.IP.Mi.6hr.SP.x2
MALP2s.IP.Mi.6hr.SP.x1
FK565.IP.Mi.6hr.SP.x3
FK565.IP.Mi.6hr.SP.x2
ADX.IP.Mi.6hr.SP.x2
ADX.IP.Mi.6hr.SP.x1
ADX.IP.Mi.6hr.SP.x3
R848.IP.Mi.6hr.SP.x2
R848.IP.Mi.6hr.SP.x1
R848.IP.Mi.6hr.SP.x3
PolyIC.IP.Mi.6hr.SP.x3
cdiGMP.IP.Mi.6hr.SP.x2
cdiGMP.IP.Mi.6hr.SP.x1
cdiGMP.IP.Mi.6hr.SP.x3
PolyIC.IP.Mi.6hr.SP.x1

[FIG.2R]

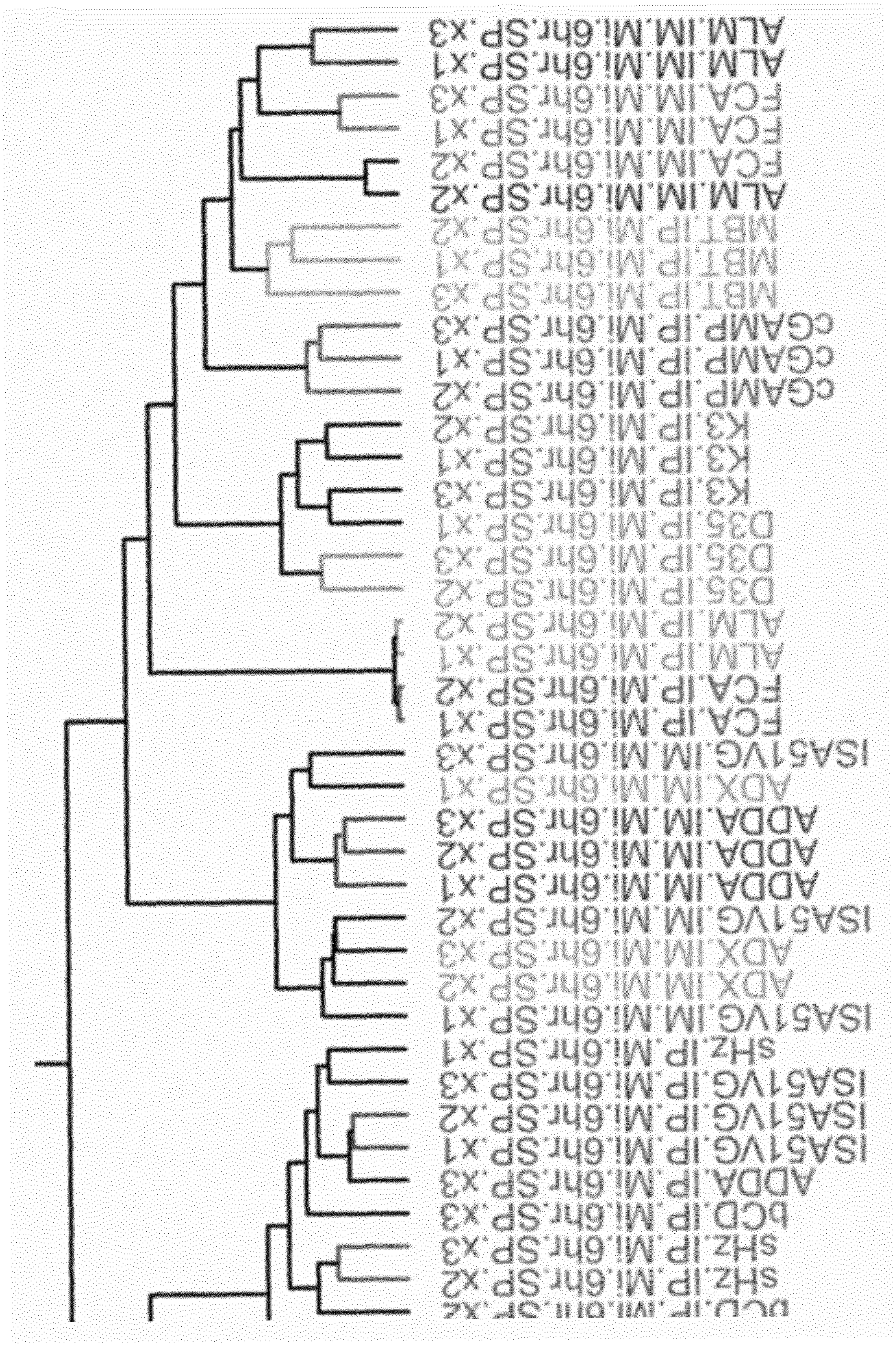
ALM.IM.Mi.6hr.SP.x3
ALM.IM.Mi.6hr.SP.x1
FCA.IM.Mi.6hr.SP.x3
FCA.IM.Mi.6hr.SP.x1
FCA.IM.Mi.6hr.SP.x2
ALM.IM.Mi.6hr.SP.x2
MBT.IP.Mi.6hr.SP.x2
MBT.IP.Mi.6hr.SP.x1
MBT.IP.Mi.6hr.SP.x3
cGAMP.IP.Mi.6hr.SP.x3
cGAMP.IP.Mi.6hr.SP.x1
cGAMP.IP.Mi.6hr.SP.x2
K3.IP.Mi.6hr.SP.x2
K3.IP.Mi.6hr.SP.x1
K3.IP.Mi.6hr.SP.x3
D35.IP.Mi.6hr.SP.x1
D35.IP.Mi.6hr.SP.x3
D35.IP.Mi.6hr.SP.x2
ALM.IP.Mi.6hr.SP.x2
ALM.IP.Mi.6hr.SP.x1
FCA.IP.Mi.6hr.SP.x2
FCA.IP.Mi.6hr.SP.x1
ISA51VG.IM.Mi.6hr.SP.x3
ADX.IM.Mi.6hr.SP.x1
ADDA.IM.Mi.6hr.SP.x3
ADDA.IM.Mi.6hr.SP.x2
ADDA.IM.Mi.6hr.SP.x1
ISA51VG.IM.Mi.6hr.SP.x2
ADX.IM.Mi.6hr.SP.x3
ADX.IM.Mi.6hr.SP.x2
ISA51VG.IM.Mi.6hr.SP.x1
sHz.IP.Mi.6hr.SP.x1
ISA51VG.IP.Mi.6hr.SP.x3
ISA51VG.IP.Mi.6hr.SP.x2
ISA51VG.IP.Mi.6hr.SP.x1
ADDA.IP.Mi.6hr.SP.x3
bCD.IP.Mi.6hr.SP.x3
sHz.IP.Mi.6hr.SP.x3
sHz.IP.Mi.6hr.SP.x2

[FIG.2S]

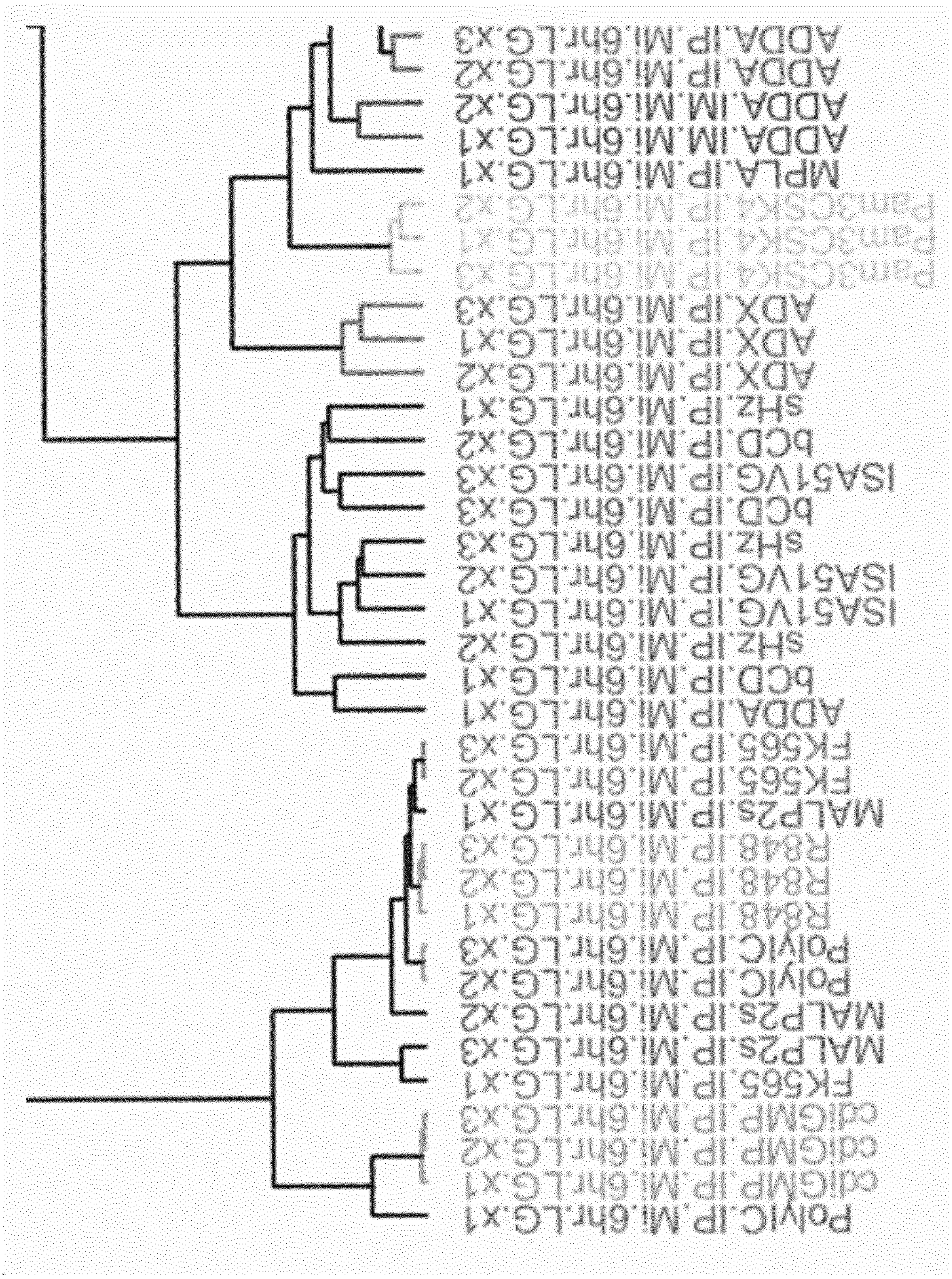
ADDA.IP.Mi.6hr.LG.x3
ADDA.IP.Mi.6hr.LG.x2
ADDA.IM.Mi.6hr.LG.x2
ADDA.IM.Mi.6hr.LG.x1
MPLA.IP.Mi.6hr.LG.x1
Pam3CSK4.IP.Mi.6hr.LG.x2
Pam3CSK4.IP.Mi.6hr.LG.x1
Pam3CSK4.IP.Mi.6hr.LG.x3
ADX.IP.Mi.6hr.LG.x3
ADX.IP.Mi.6hr.LG.x1
ADX.IP.Mi.6hr.LG.x2
sHz.IP.Mi.6hr.LG.x1
bCD.IP.Mi.6hr.LG.x2
ISA51VG.IP.Mi.6hr.LG.x3
bCD.IP.Mi.6hr.LG.x3
sHz.IP.Mi.6hr.LG.x3
ISA51VG.IP.Mi.6hr.LG.x2
ISA51VG.IP.Mi.6hr.LG.x1
sHz.IP.Mi.6hr.LG.x2
bCD.IP.Mi.6hr.LG.x1
ADDA.IP.Mi.6hr.LG.x1
FK565.IP.Mi.6hr.LG.x3
FK565.IP.Mi.6hr.LG.x2
MALP2s.IP.Mi.6hr.LG.x1
R848.IP.Mi.6hr.LG.x3
R848.IP.Mi.6hr.LG.x2
R848.IP.Mi.6hr.LG.x1
PolyIC.IP.Mi.6hr.LG.x3
PolyIC.IP.Mi.6hr.LG.x2
MALP2s.IP.Mi.6hr.LG.x2
MALP2s.IP.Mi.6hr.LG.x3
FK565.IP.Mi.6hr.LG.x1
cdiGMP.IP.Mi.6hr.LG.x3
cdiGMP.IP.Mi.6hr.LG.x2
cdiGMP.IP.Mi.6hr.LG.x1
PolyIC.IP.Mi.6hr.LG.x1

[FIG.2T]

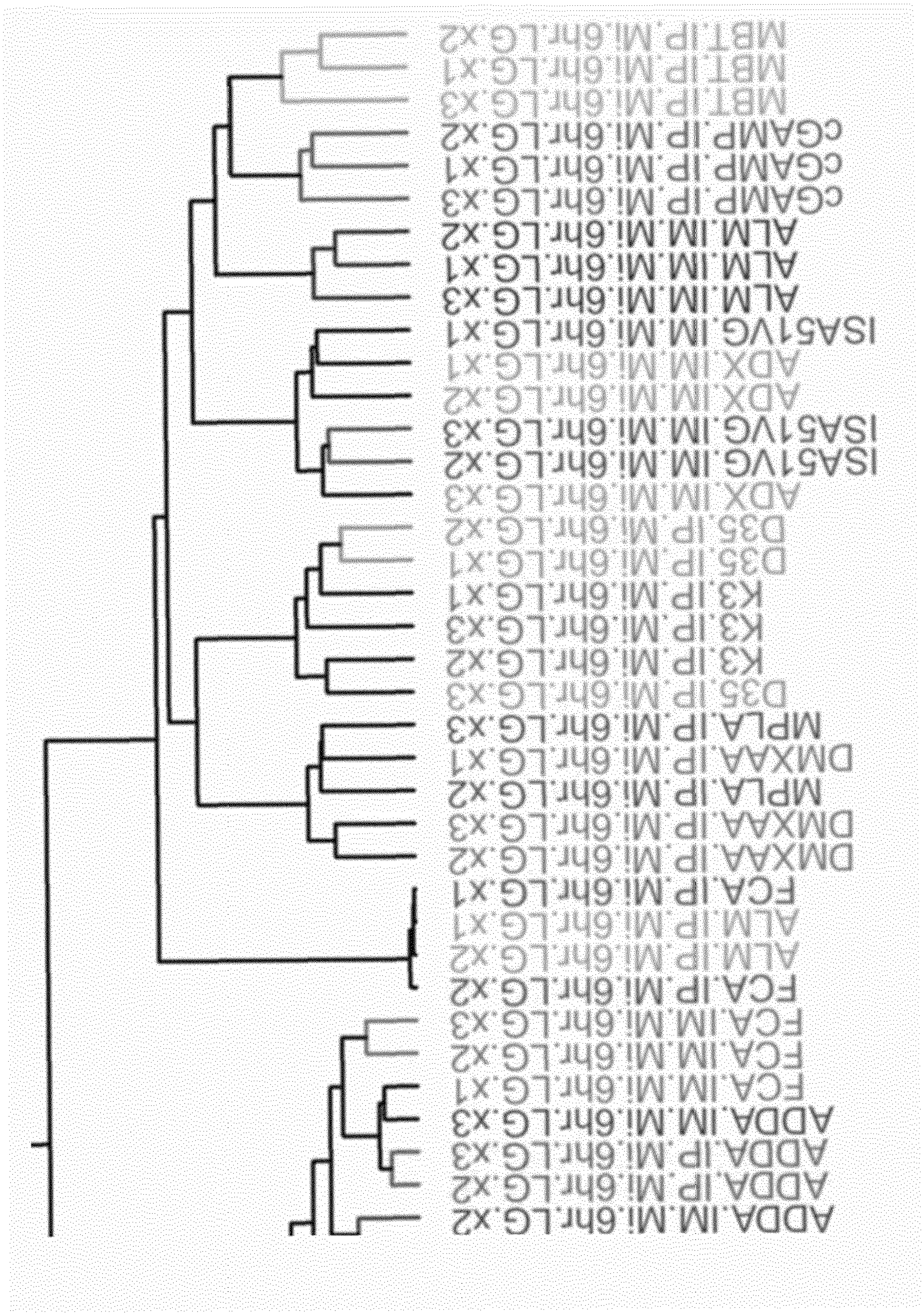
MBT.IP.Mi.6hr.LG.x2
MBT.IP.Mi.6hr.LG.x1
MBT.IP.Mi.6hr.LG.x3
cGAMP.IP.Mi.6hr.LG.x2
cGAMP.IP.Mi.6hr.LG.x1
cGAMP.IP.Mi.6hr.LG.x3
ALM.IM.Mi.6hr.LG.x2
ALM.IM.Mi.6hr.LG.x1
ALM.IM.Mi.6hr.LG.x3
ISA51VG.IM.Mi.6hr.LG.x1
ADX.IM.Mi.6hr.LG.x1
ADX.IM.Mi.6hr.LG.x2
ISA51VG.IM.Mi.6hr.LG.x3
ISA51VG.IM.Mi.6hr.LG.x2
ADX.IM.Mi.6hr.LG.x3
D35.IP.Mi.6hr.LG.x2
D35.IP.Mi.6hr.LG.x1
K3.IP.Mi.6hr.LG.x1
K3.IP.Mi.6hr.LG.x3
K3.IP.Mi.6hr.LG.x2
D35.IP.Mi.6hr.LG.x3
MPLA.IP.Mi.6hr.LG.x3
DMXAA.IP.Mi.6hr.LG.x1
MPLA.IP.Mi.6hr.LG.x2
DMXAA.IP.Mi.6hr.LG.x3
DMXAA.IP.Mi.6hr.LG.x2
FCA.IP.Mi.6hr.LG.x1
ALM.IP.Mi.6hr.LG.x1
ALM.IP.Mi.6hr.LG.x2
FCA.IP.Mi.6hr.LG.x2
FCA.IM.Mi.6hr.LG.x3
FCA.IM.Mi.6hr.LG.x2
FCA.IM.Mi.6hr.LG.x1
ADDA.IM.Mi.6hr.LG.x3
ADDA.IP.Mi.6hr.LG.x3
ADDA.IP.Mi.6hr.LG.x2
ADDA.IM.Mi.6hr.LG.x2

[FIG.3A]

a

| Term | Genes (*group representative genes in the top30 list) | G1 LV cdiGMP, cGAMP, DMXAA, PolyIC, R848 | G1 SP cdiGMP, cGAMP, DMXAA, PolyIC, R848 | G1 LN cdiGMP, cGAMP, DMXAA, PolyIC, R848 | G2 LV ALM_IP, bCD, ENDCN, FCA | G2 SP ALM_IP, bCD, ENDCN | G2 LN bCD, FCA | G3 LV ADX, ADX_IP, Pam3CSK4, FK565 | G3 SP FCA, Pam3CSK4, FK565 | G3 LN FK565 | G4 LV MALP2s | G4 SP ADX, ADX_IP, MALP2s | G5 LN D35, K3, K3SPG | G6 LN AddaVax |
|---|---|---|---|---|---|---|---|---|---|---|---|---|---|---|
| Interferon Signaling | (G1) Gm14446, Pml, H2-T22, Ifit1, Irf7, Isg15, Stat1, Fcgr1, Oas1a, Oas2, Trim12a, Trim12c, Uba7, Ube2l6, | | | | | | | | | | | | | |
| Metabolism of lipids and lipoproteins | (G2) Elovl6, Gpam, Hsd3b7, Acer2, Acox1, Tbl1xr1, Alox5ap, Ggt5 | | | | | | | | | | | | | |
| response to stress | (G3) Bbc3, Pdk4, Cd55, Cd93, Clec4e, Coro1a, Traf3, Trem3, C5ar1, Clec4n, Ier3, Il1r1, Plek, Tbx3, Trem1 | | | | | | | | | | | | | |
| response to wounding | (G4) Col3, Myof, Papss2, Slc7a11, Tnfrsf1b | | | | | | | | | | | | | |
| phosphate-containing compound metabolic process | (G5) Ak3, Insm1, Nek1, Pik3r2, Ttn | | | | | | | | | | | | | |
| Phagosome | (G6) Atp6v0d2, Atp6v1c1, Clec7a | | | | | | | | | | | | | |

[FIG.3B]

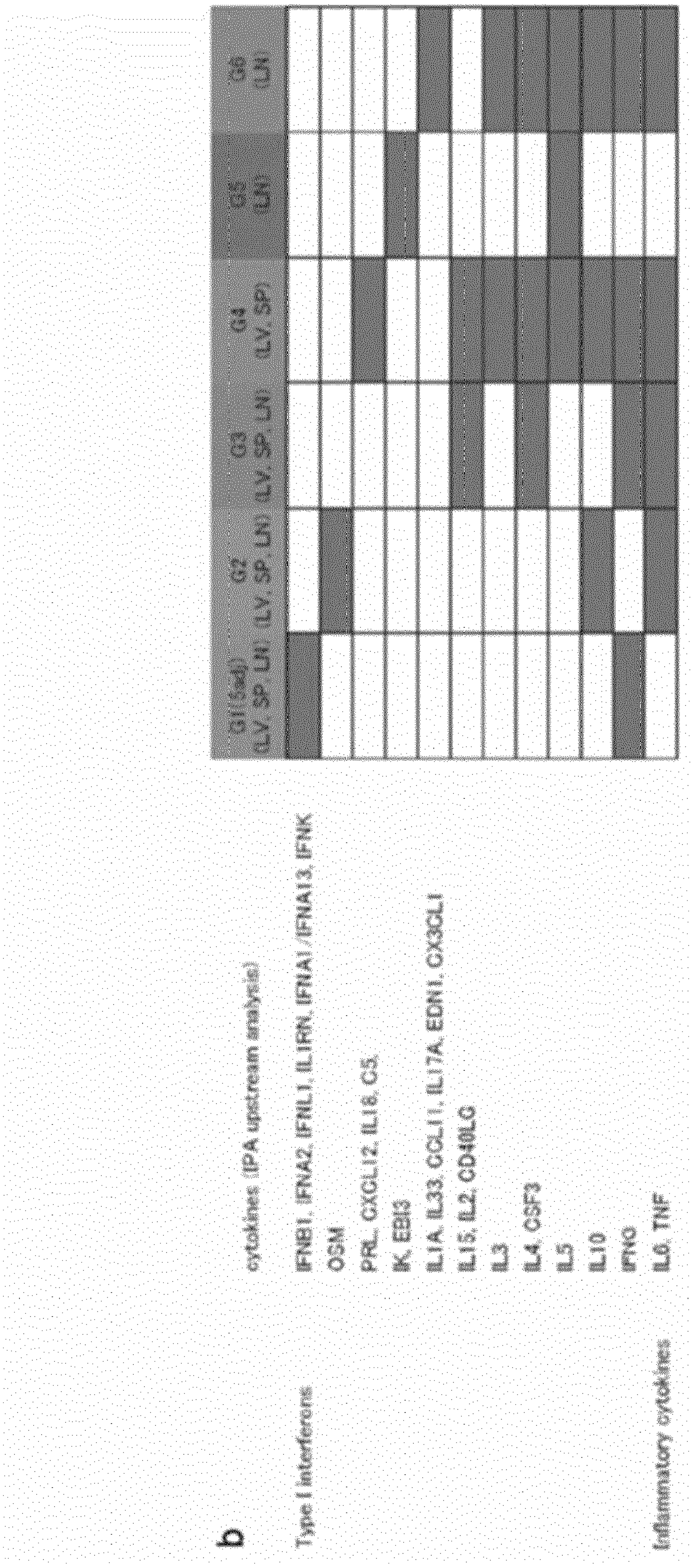
b
cytokines (IPA upstream analysis)
G1(5adj) (LV, SP, LN)
G2 (LV, SP, LN)
G3 (LV, SP, LN)
G4 (LV, SP)
G5 (LN)
G6 (LN)
Type I interferons
IFNB1, IFNA2, IFNL1, IL1RN, IFNA1/IFNA13, IFNK
OSM
PRL, CXCL12, IL18, C5,
IK, EBI3
IL1A, IL33, CCL11, IL17A, EDN1, CX3CL1
IL15, IL2, CD40LG
IL3
IL4, CSF3
IL5
IL10
IFNG
Inflammatory cytokines
IL6, TNF

[FIG.4A]

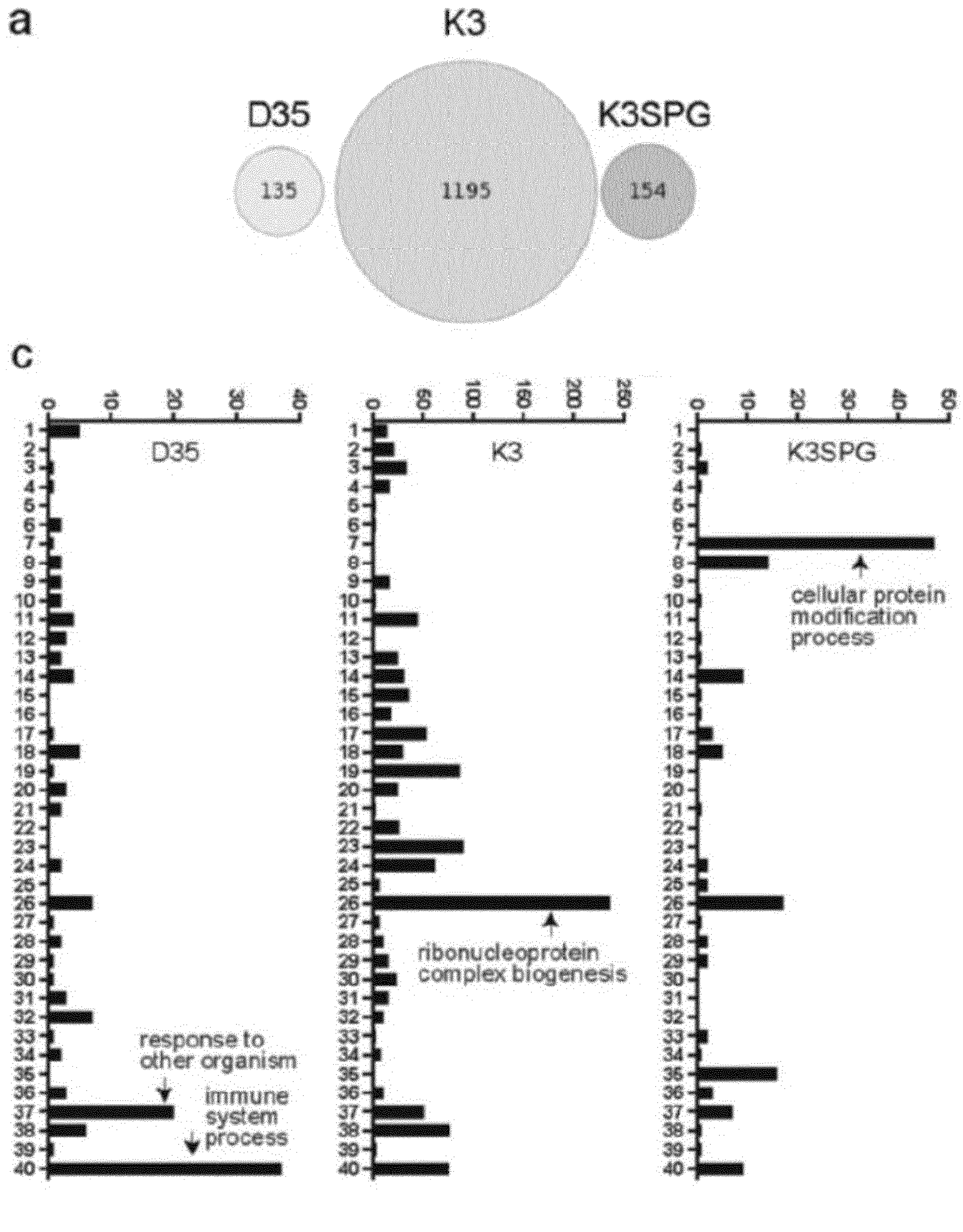
a
K3
D35
K3SPG
135
1195
154
c
D35
K3
K3SPG
cellular protein
modification
process
ribonucleoprotein
complex biogenesis
response to
other organism
immune
system
process

[FIG.4B]

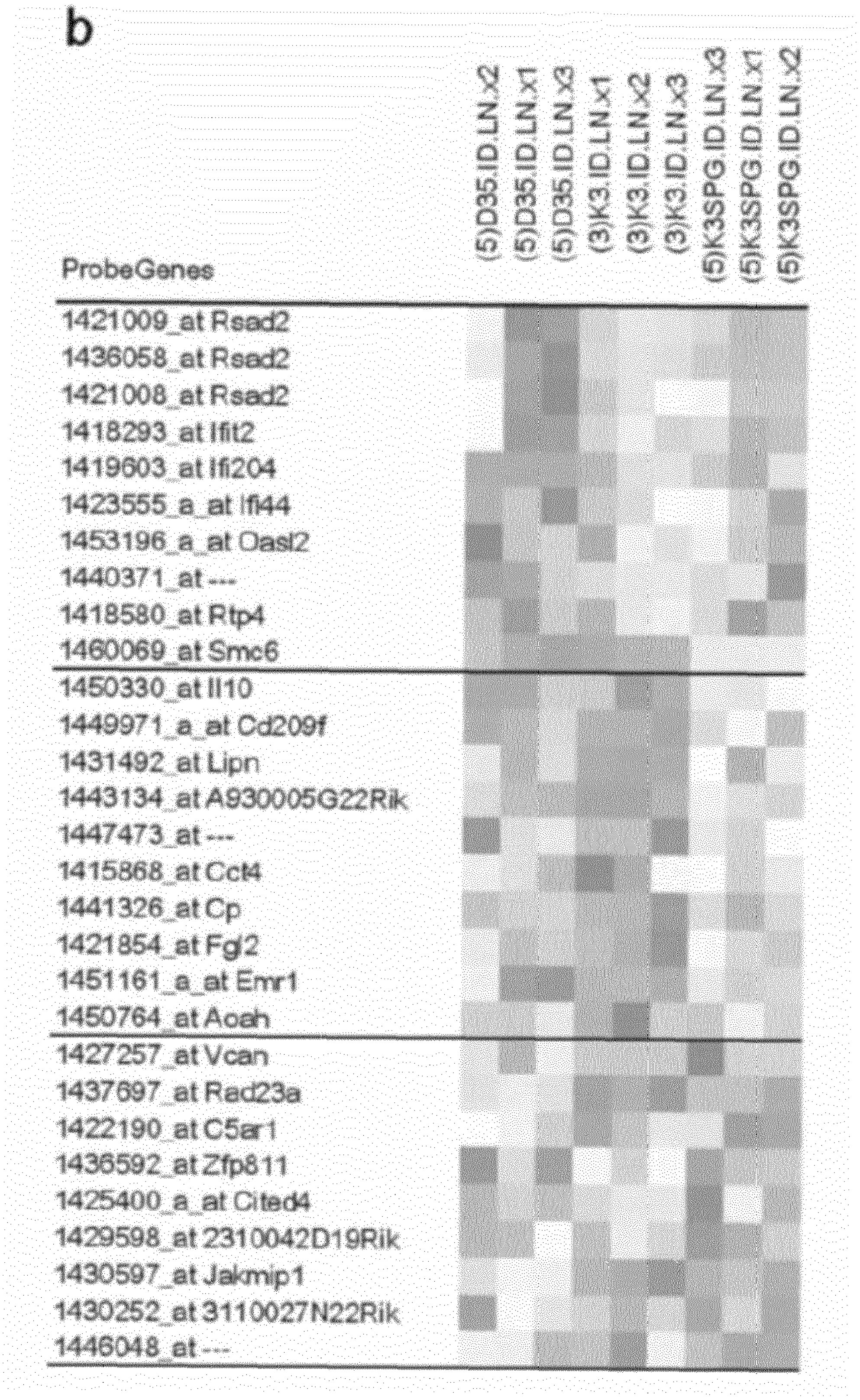
b
ProbeGenes
(5)D35.ID.LN.x2
(5)D35.ID.LN.x1
(5)D35.ID.LN.x3
(3)K3.ID.LN.x1
(3)K3.ID.LN.x2
(3)K3.ID.LN.x3
(5)K3SPG.ID.LN.x3
(5)K3SPG.ID.LN.x1
(5)K3SPG.ID.LN.x2
1421009_at Rsad2
1436058_at Rsad2
1421008_at Rsad2
1418293_at Ifit2
1419603_at Ifi204
1423555_a_at Ifi44
1453196_a_at Oasl2
1440371_at ---
1418580_at Rtp4
1460069_at Smc6
1450330_at Il10
1449971_a_at Cd209f
1431492_at Lipn
1443134_at A930005G22Rik
1447473_at ---
1415868_at Cct4
1441326_at Cp
1421854_at Fgl2
1451161_a_at Emr1
1450764_at Aoah
1427257_at Vcan
1437697_at Rad23a
1422190_at C5ar1
1436592_at Zfp811
1425400_a_at Cited4
1429598_at 2310042D19Rik
1430597_at Jakmip1
1430252_at 3110027N22Rik
1446048_at ---

[FIG.4C]

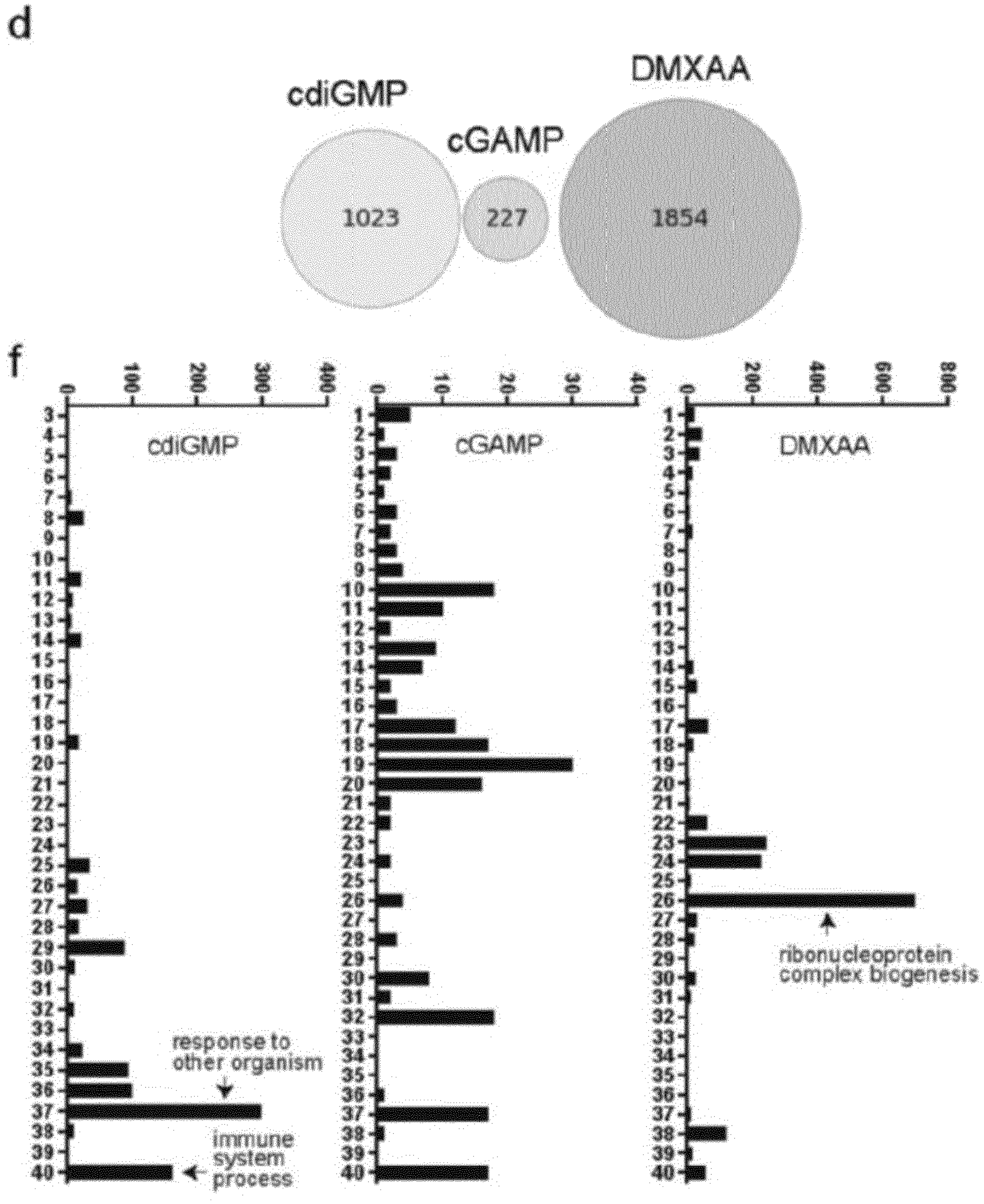
d
cdiGMP
cGAMP
DMXAA
1023
227
1854
f
cdiGMP
cGAMP
DMXAA
response to other organism
immune system process
ribonucleoprotein complex biogenesis

[FIG.4D]

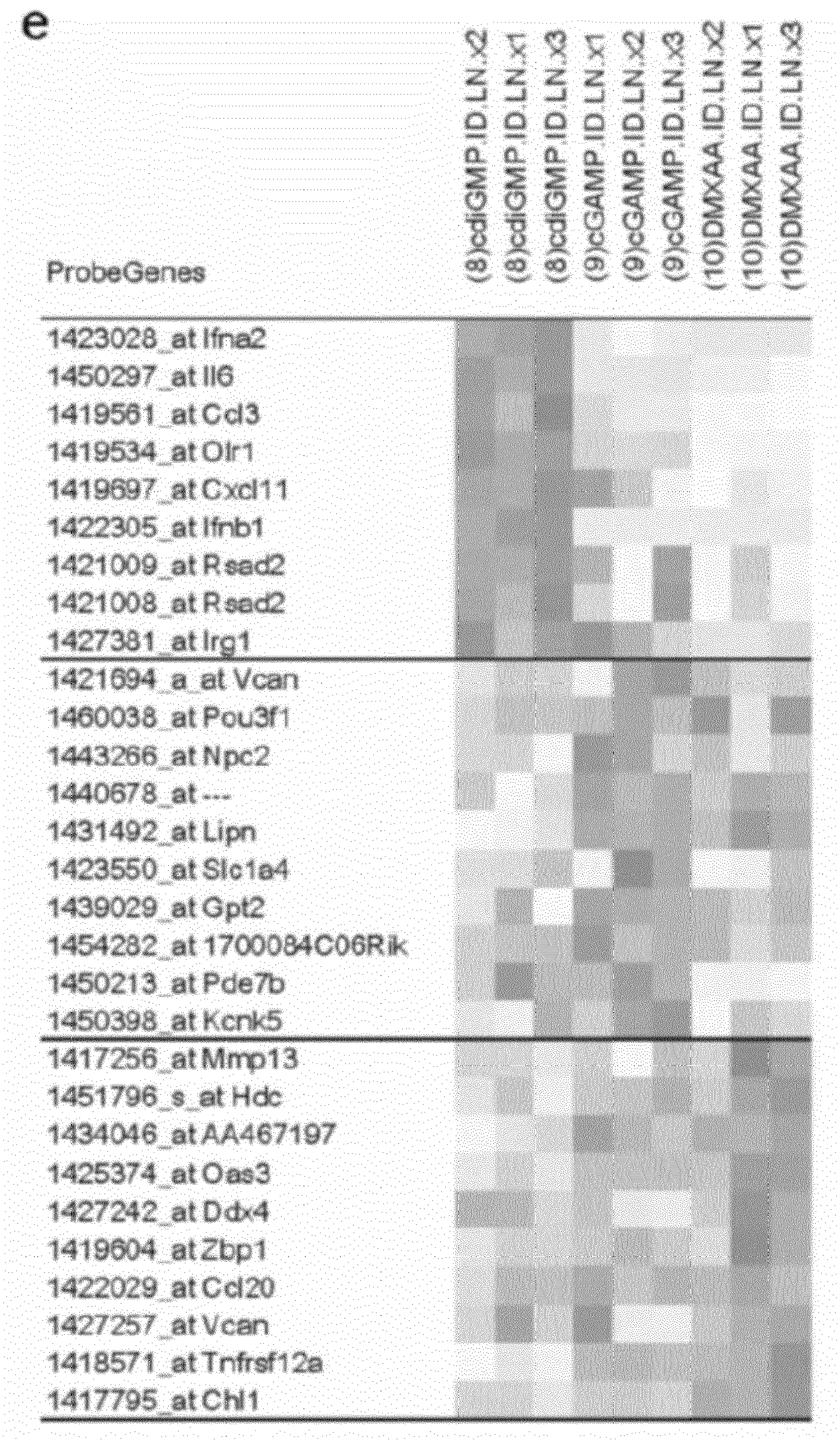
e
(8)cdiGMP.ID.LN.x2
(8)cdiGMP.ID.LN.x1
(8)cdiGMP.ID.LN.x3
(9)cGAMP.ID.LN.x1
(9)cGAMP.ID.LN.x2
(9)cGAMP.ID.LN.x3
(10)DMXAA.ID.LN.x2
(10)DMXAA.ID.LN.x1
(10)DMXAA.ID.LN.x3
ProbeGenes
1423028_at Ifna2
1450297_at Il6
1419561_at Ccl3
1419534_at Olr1
1419697_at Cxcl11
1422305_at Ifnb1
1421009_at Rsad2
1421008_at Rsad2
1427381_at Irg1
1421694_a_at Vcan
1460038_at Pou3f1
1443266_at Npc2
1440678_at ---
1431492_at Lipn
1423550_at Slc1a4
1439029_at Gpt2
1454282_at 1700084C06Rik
1450213_at Pde7b
1450398_at Kcnk5
1417256_at Mmp13
1451796_s_at Hdc
1434046_at AA467197
1425374_at Oas3
1427242_at Ddx4
1419604_at Zbp1
1422029_at Ccl20
1427257_at Vcan
1418571_at Tnfrsf12a
1417795_at Chl1

[FIG.5A]

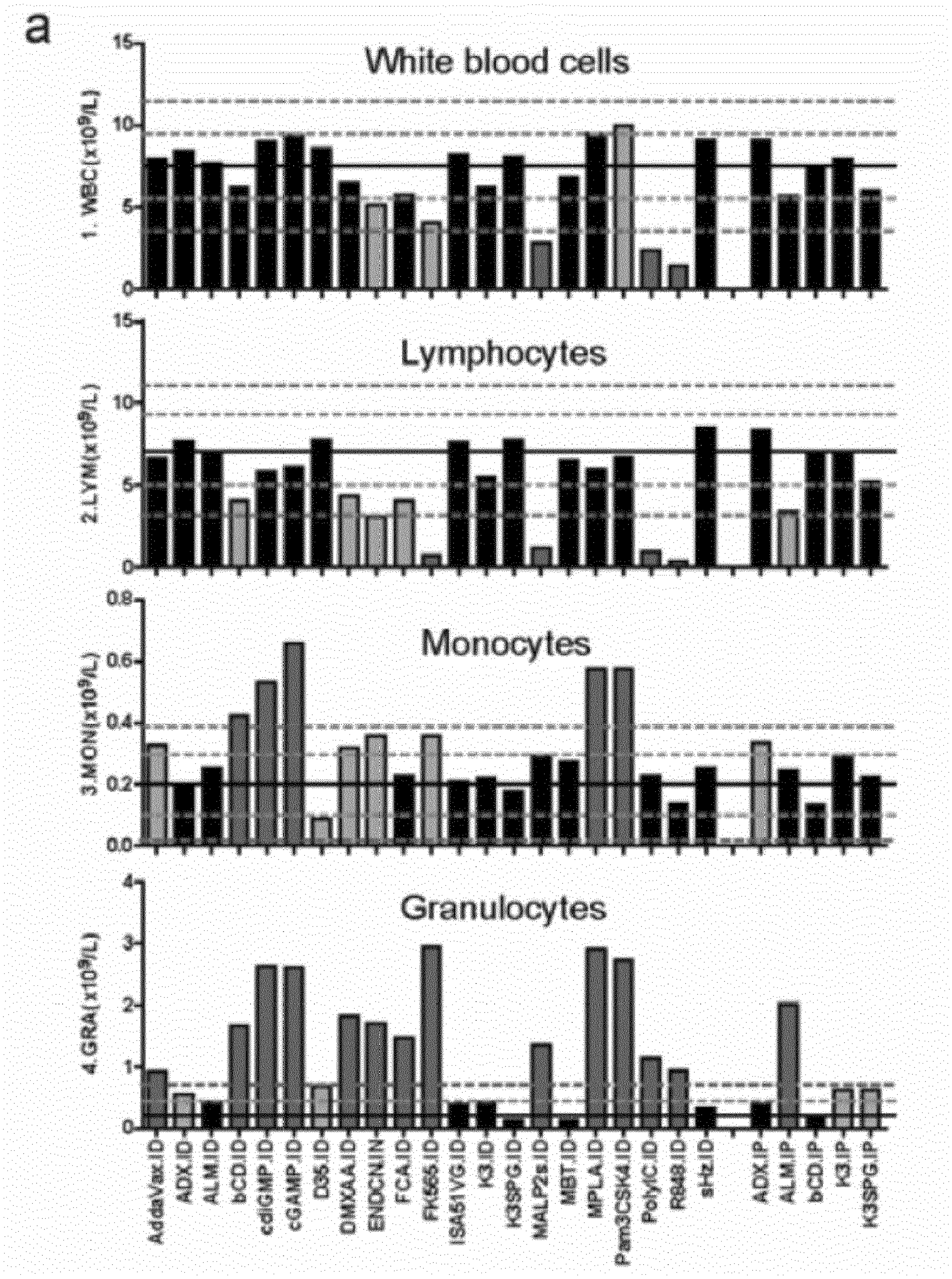
a
White blood cells
1. WBC(x10$^9$/L)
Lymphocytes
2.LYM(x10$^9$/L)
Monocytes
3.MON(x10$^9$/L)
Granulocytes
4.GRA(x10$^9$/L)
AddaVax.ID
ADX.ID
ALM.ID
bCD.ID
cdiGMP.ID
cGAMP.ID
D35.ID
DMXAA.ID
ENDCN.IN
FCA.ID
FK565.ID
ISA51VG.ID
K3.ID
K3SPG.ID
MALP2s.ID
MBT.ID
MPLA.ID
Pam3CSK4.ID
PolyIC.ID
R848.ID
sHz.ID
ADX.IP
ALM.IP
bCD.IP
K3.IP
K3SPG.IP

[FIG.5B]

b

| | LV (357 gene probes) | | SP (78 gene probes) | | LN (40 gene probes) | |
|---|---|---|---|---|---|---|
| | WBC | LYM | WBC | LYM | WBC | LYM |
| Up | Gbp2 | D17H6S56 | Slfn3 /// | Slfn3 /// | Mt1 | Serpina3c |
| | Fam26f | D17H6S56 | Ms4a6d | Slfn4 | Ubd | Wnt5a |
| | Ccl5 | Saa3 | Slfn4 | Gbp2 | Serpina3n | --- |
| | Cxcl9 | Ms4a6d | Ms4a6d | Lipg | Mt2 | |
| | Gbp2 | Adh7 | Lipg | Ms4a6d | | |
| Down | Klf12 | Ahnak | Crisp3 | S1pr5 | Cxadr | Stfa2l1 |
| | Hsd3b5 | Ank3 | Arhgap18 | Crisp3 | Gad1 | Clec4d |
| | Abcd2 | Klf12 | Edn3 | Edn3 | Ano1 | Cd14 |
| | Klf12 | Abcd2 | Cd209a | Igfbp4 | Limch1 | Ccr1 |
| | Ddc | Rnf152 | Adam22 | Il7 | 5930435M( | Socs3 |

c

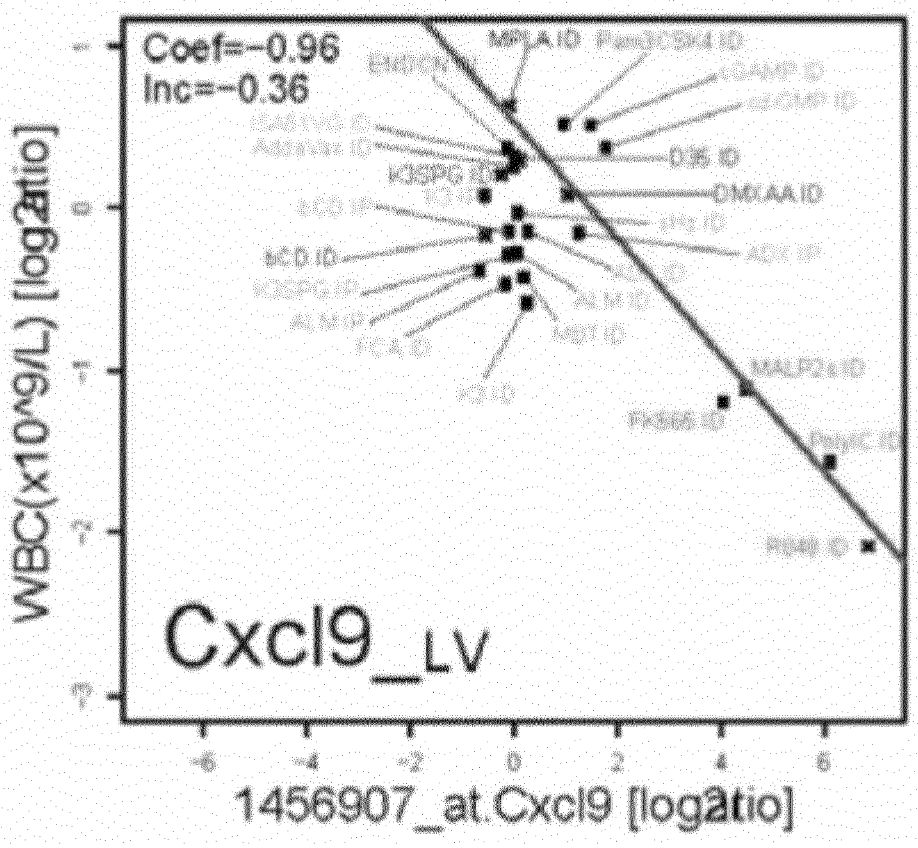

[FIG.6]

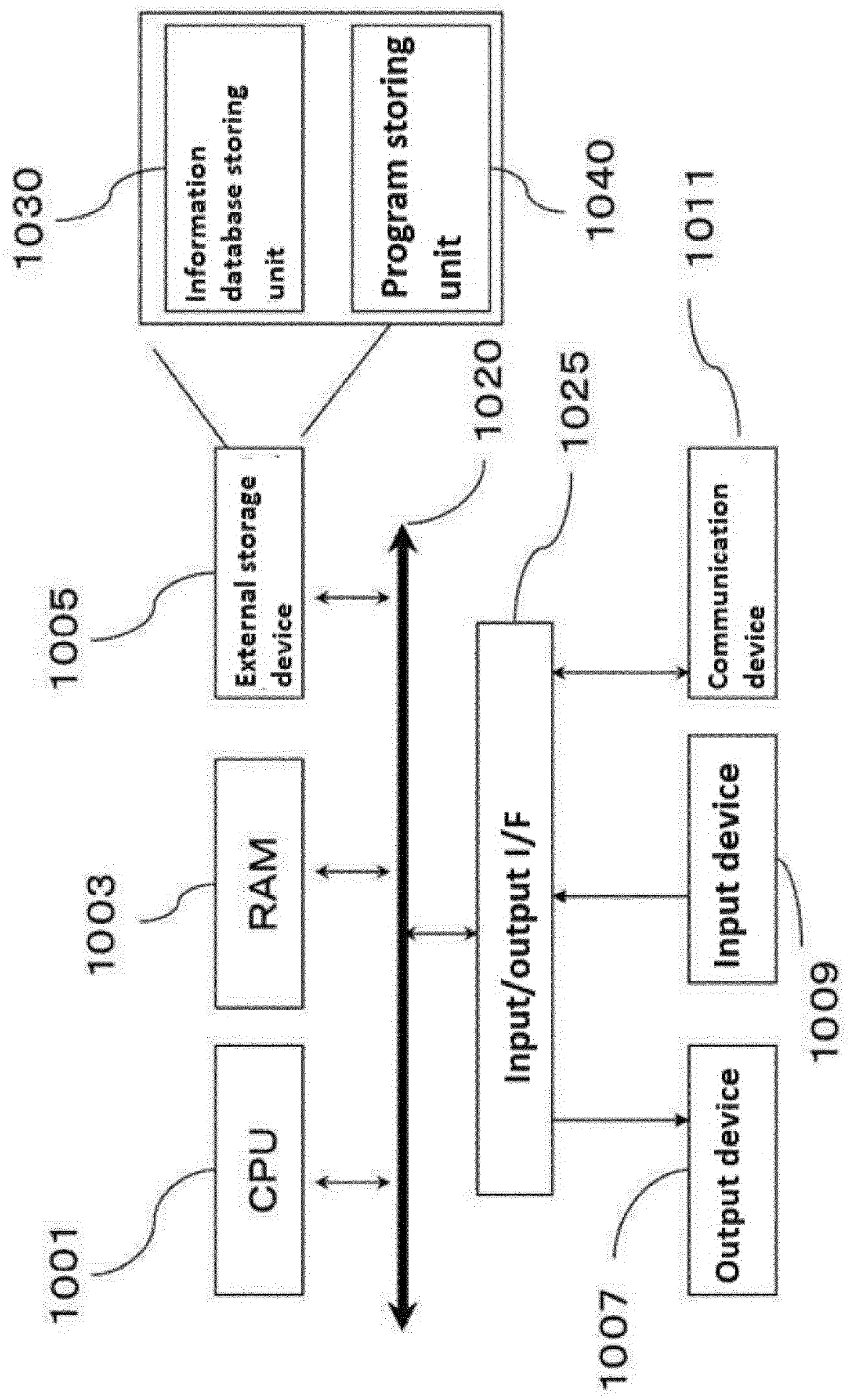
1030
Information database storing unit
Program storing unit
1040
1005
External storage device
1020
1025
1011
Communication device
1003
RAM
Input/output I/F
Input device
1009
1001
CPU
Output device
1007

[FIG.7]

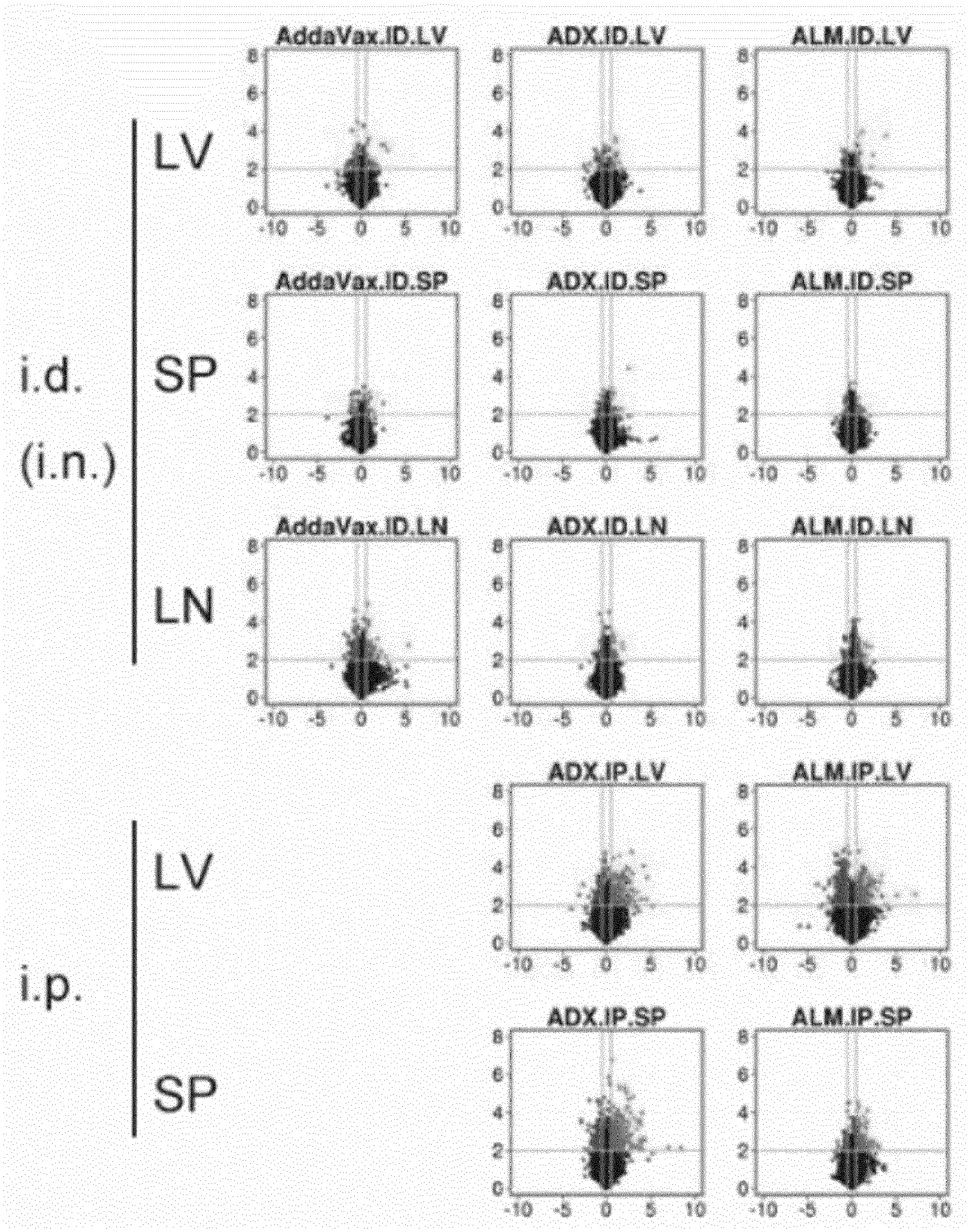
i.d.
(i.n.)
LV
SP
LN
i.p.
LV
SP
AddaVax.ID.LV
ADX.ID.LV
ALM.ID.LV
AddaVax.ID.SP
ADX.ID.SP
ALM.ID.SP
AddaVax.ID.LN
ADX.ID.LN
ALM.ID.LN
ADX.IP.LV
ALM.IP.LV
ADX.IP.SP
ALM.IP.SP

[FIG.7](**Continue**)

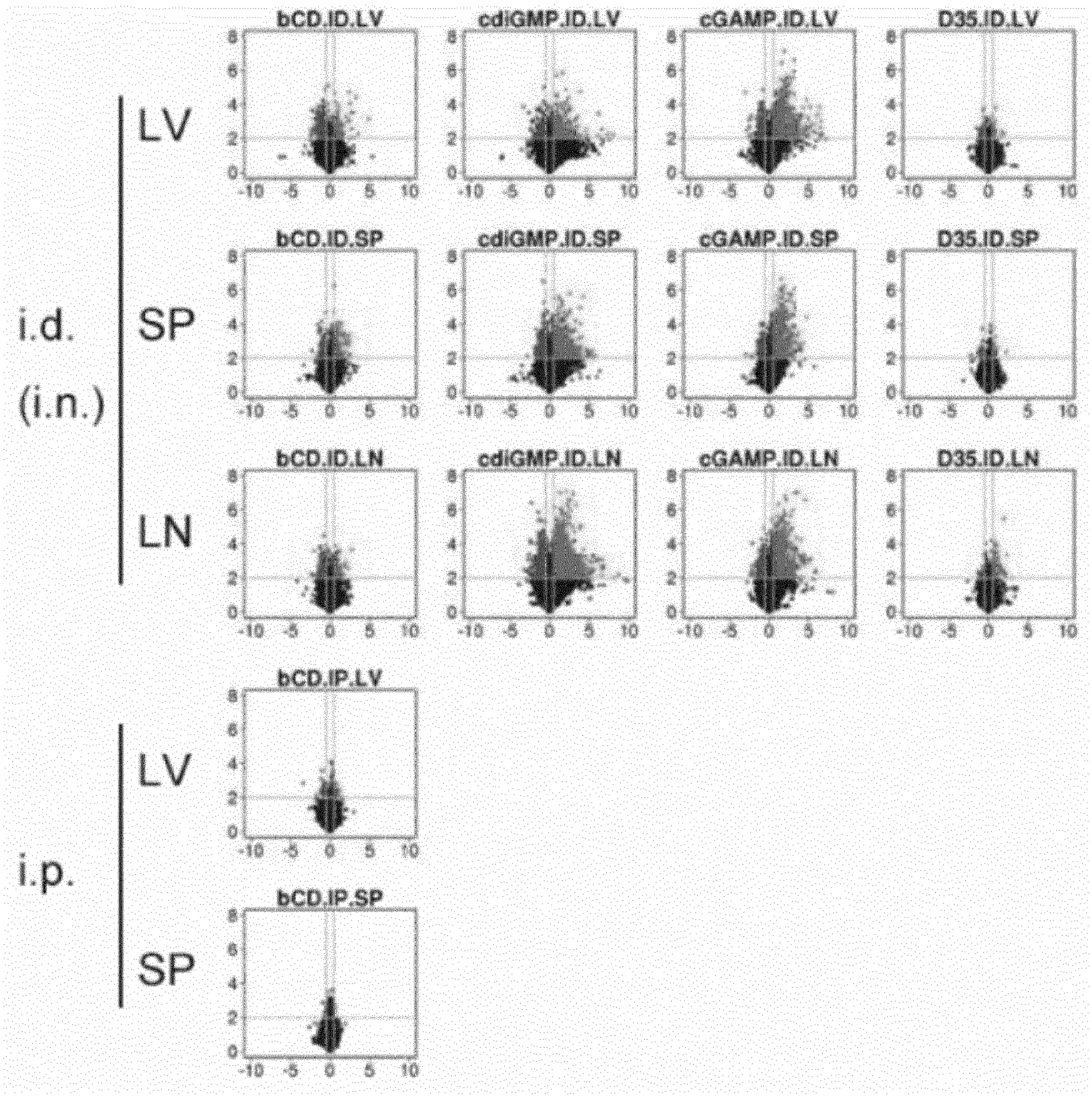

[FIG.7](**Continue**)

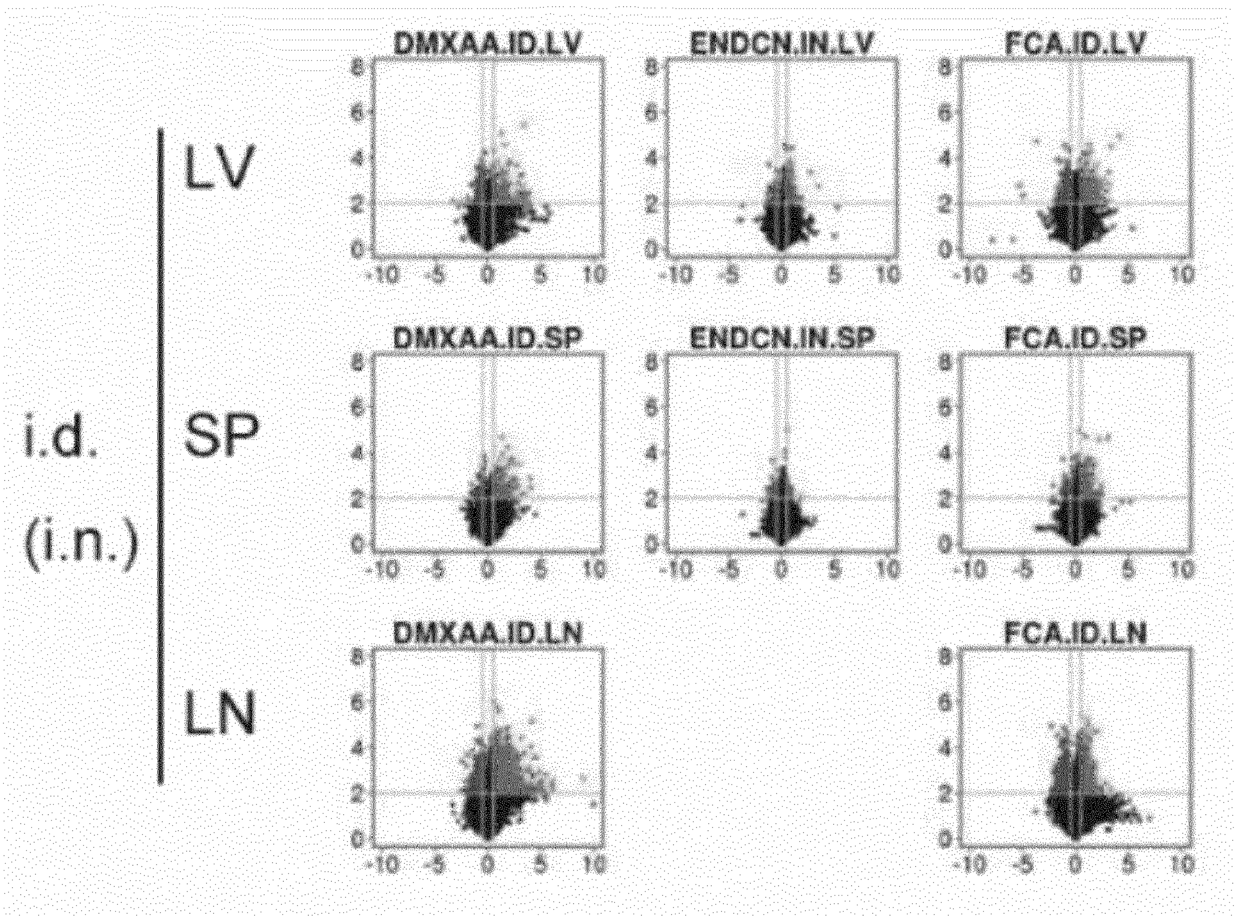

[FIG.7](**Continue**)

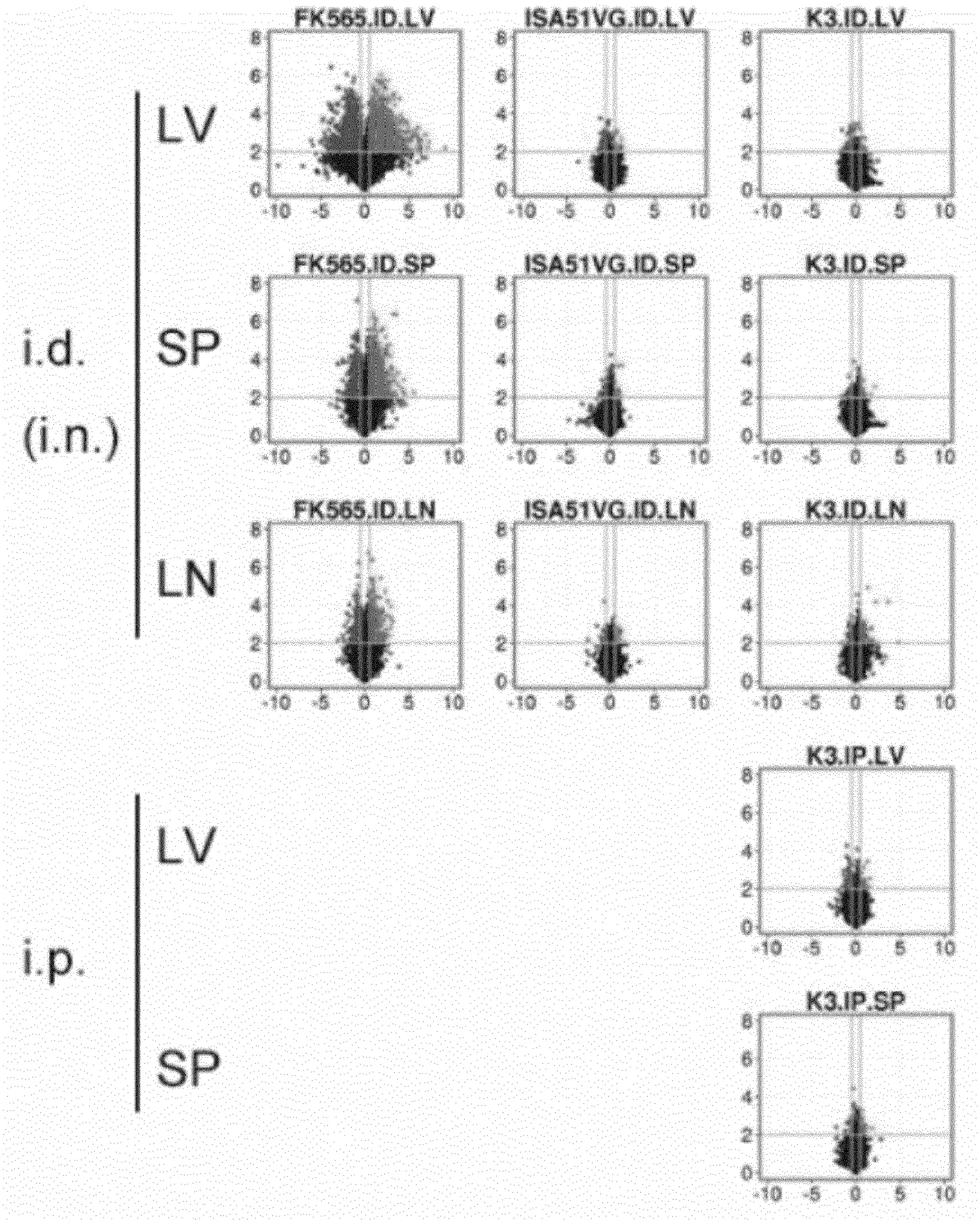

[FIG.7](**Continue**)

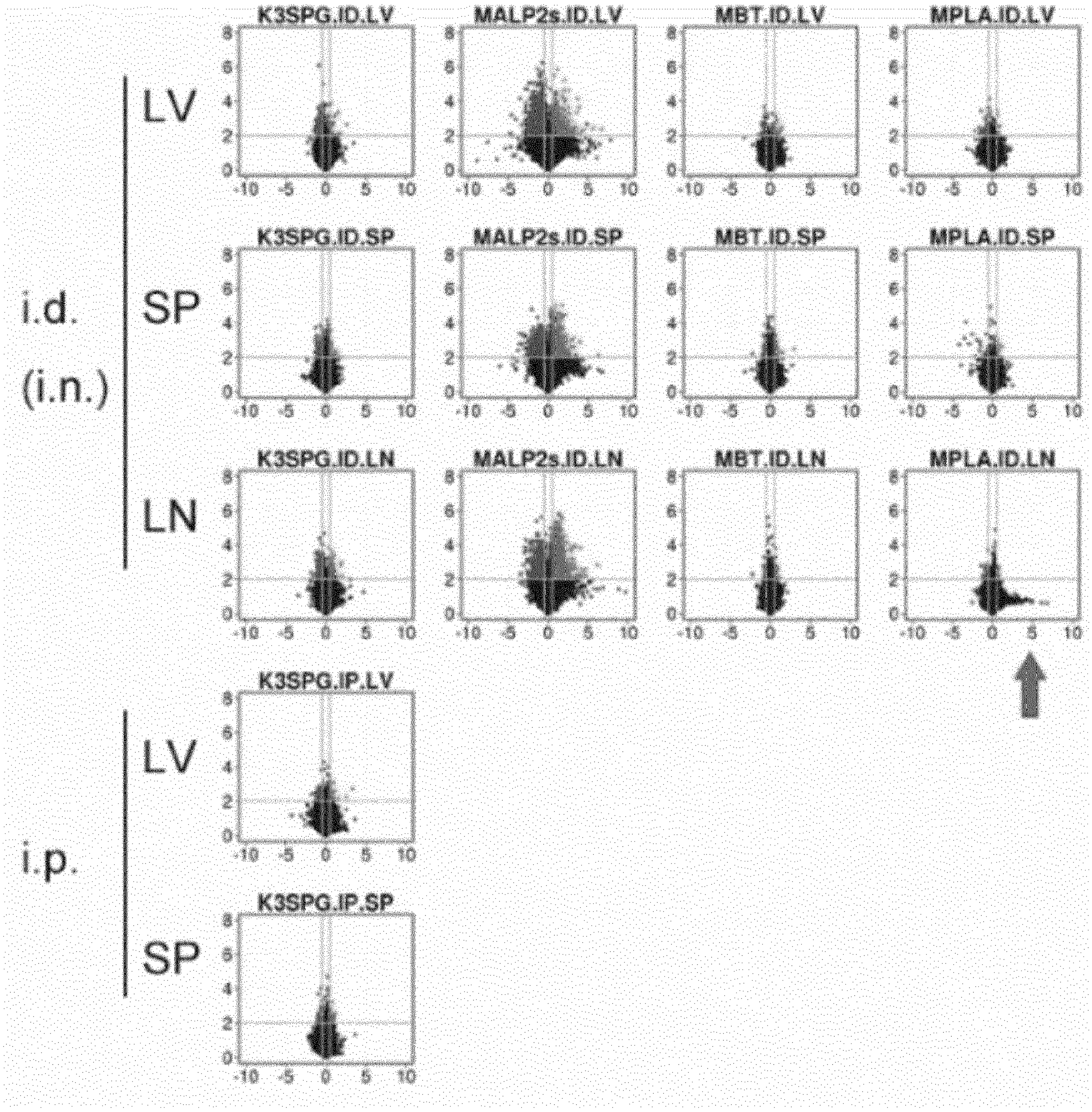

[FIG.7](**Continue**)

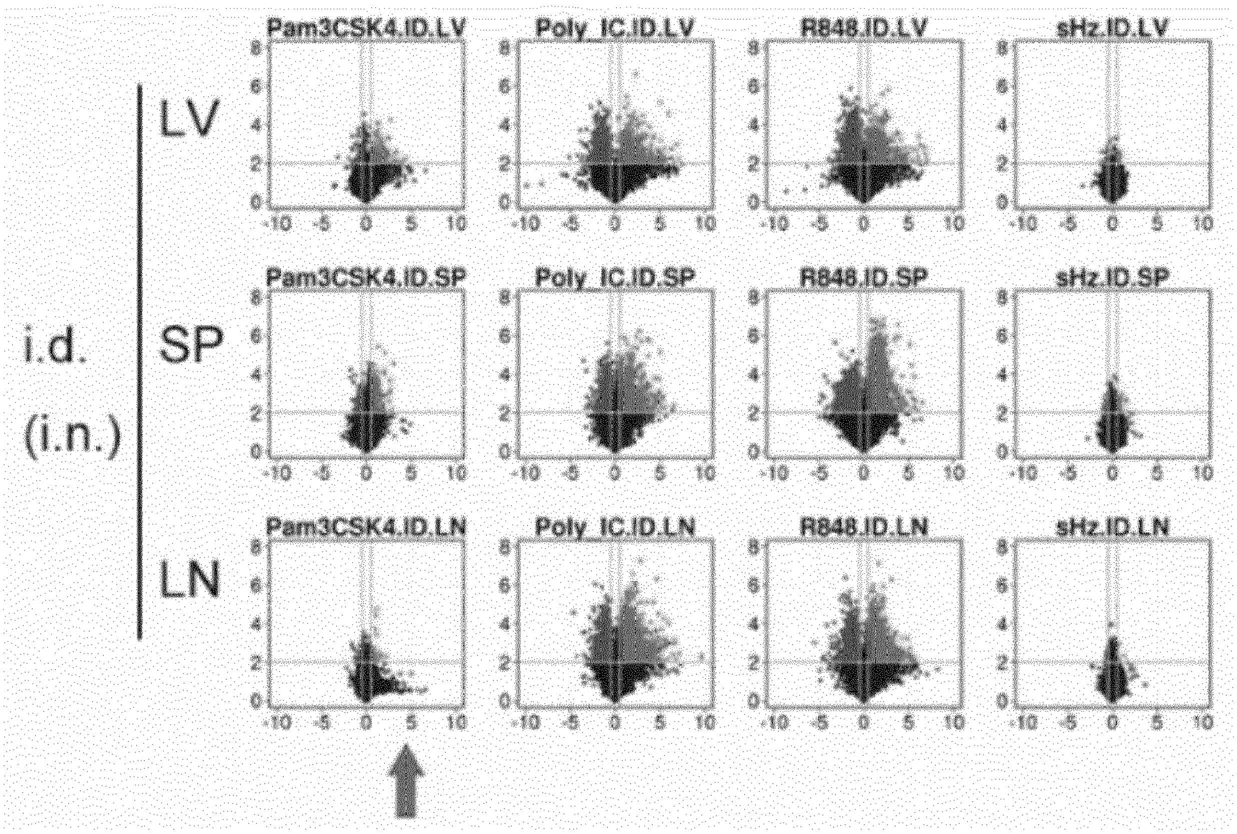

[FIG.8]

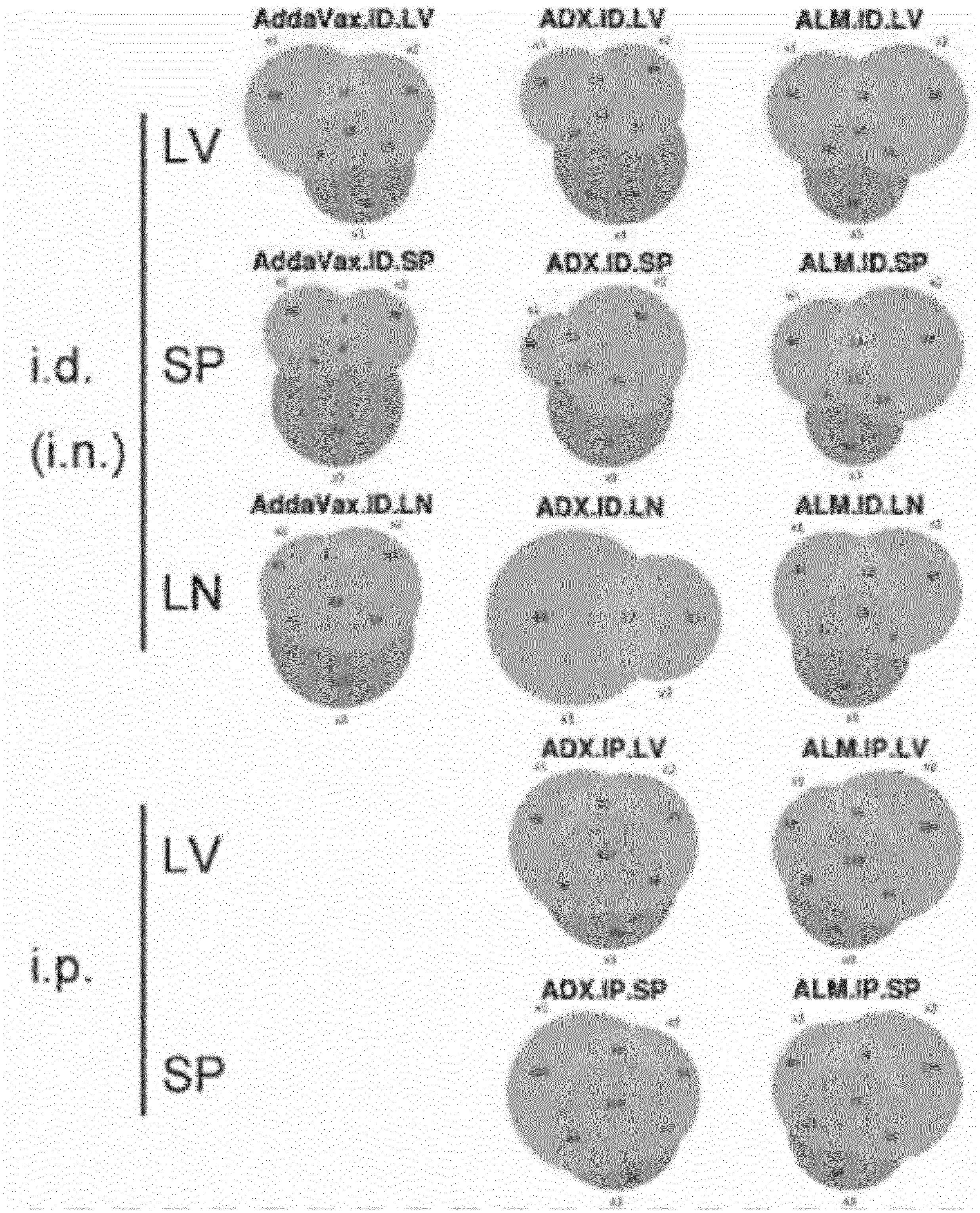
AddaVax.ID.LV
ADX.ID.LV
ALM.ID.LV
AddaVax.ID.SP
ADX.ID.SP
ALM.ID.SP
AddaVax.ID.LN
ADX.ID.LN
ALM.ID.LN
ADX.IP.LV
ALM.IP.LV
ADX.IP.SP
ALM.IP.SP
i.d.
(i.n.)
i.p.
LV
SP
LN
LV
SP

[FIG.8](**Continue**)

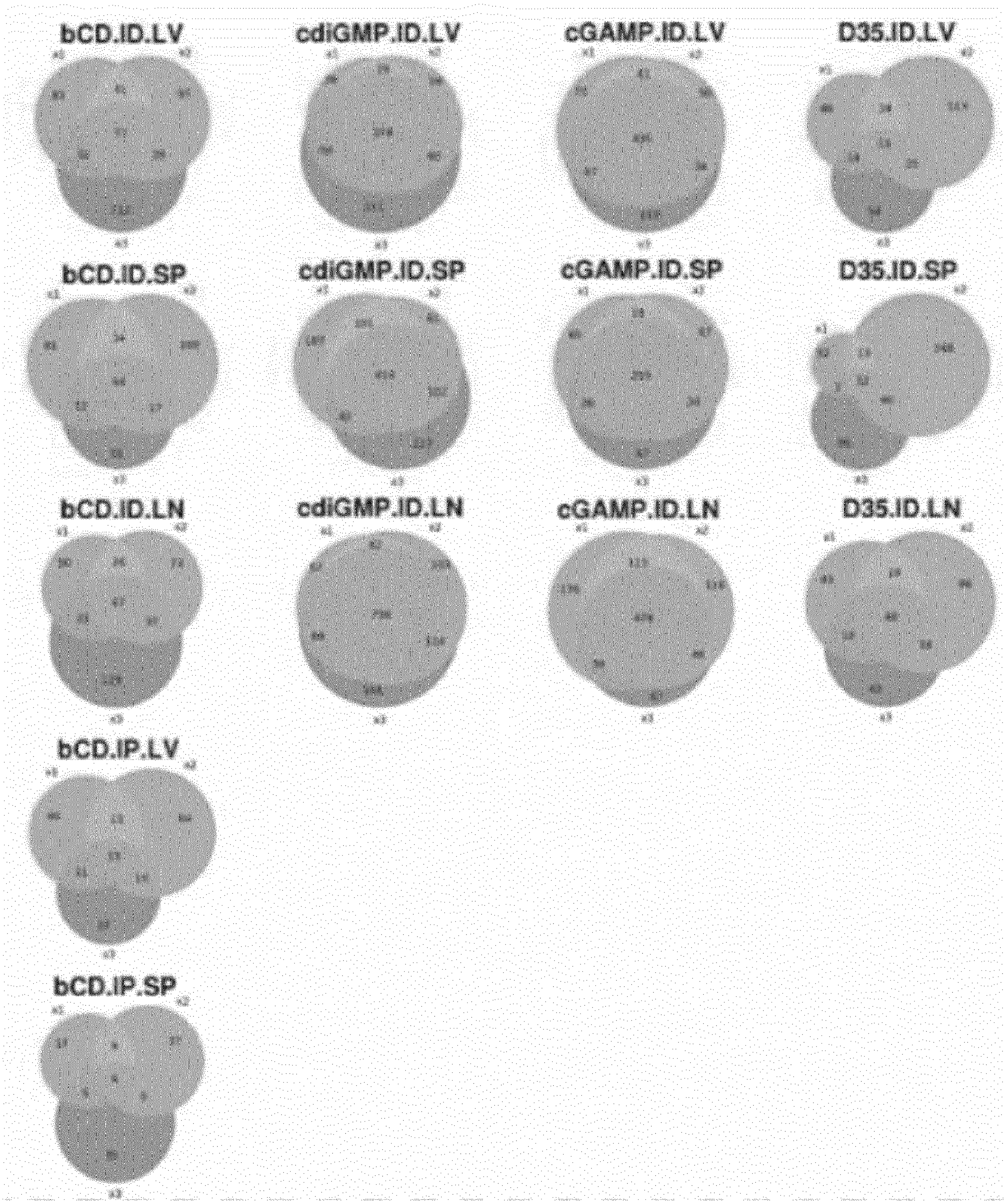

[FIG.8](**Continue**)

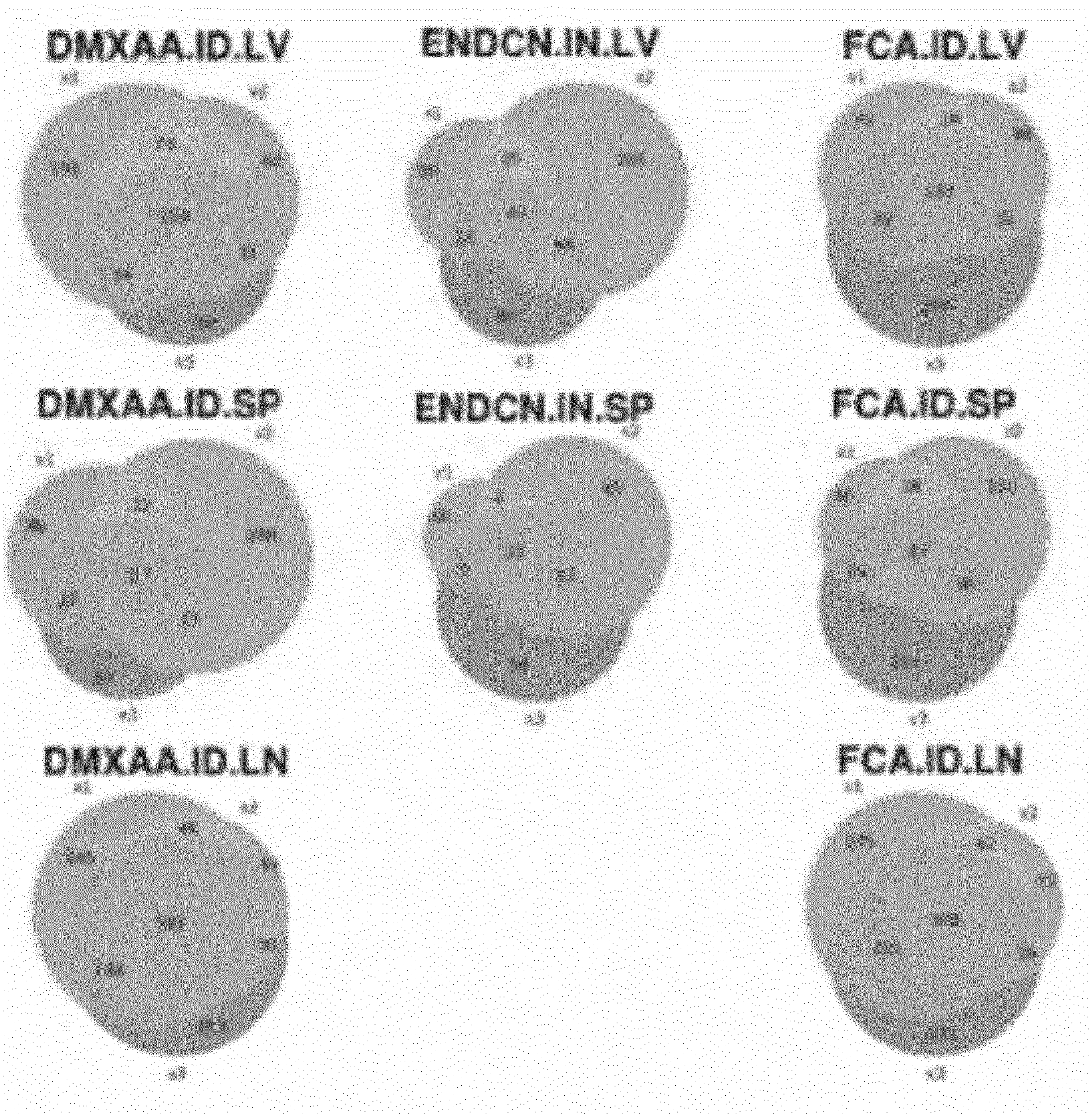

[FIG.8](**Continue**)

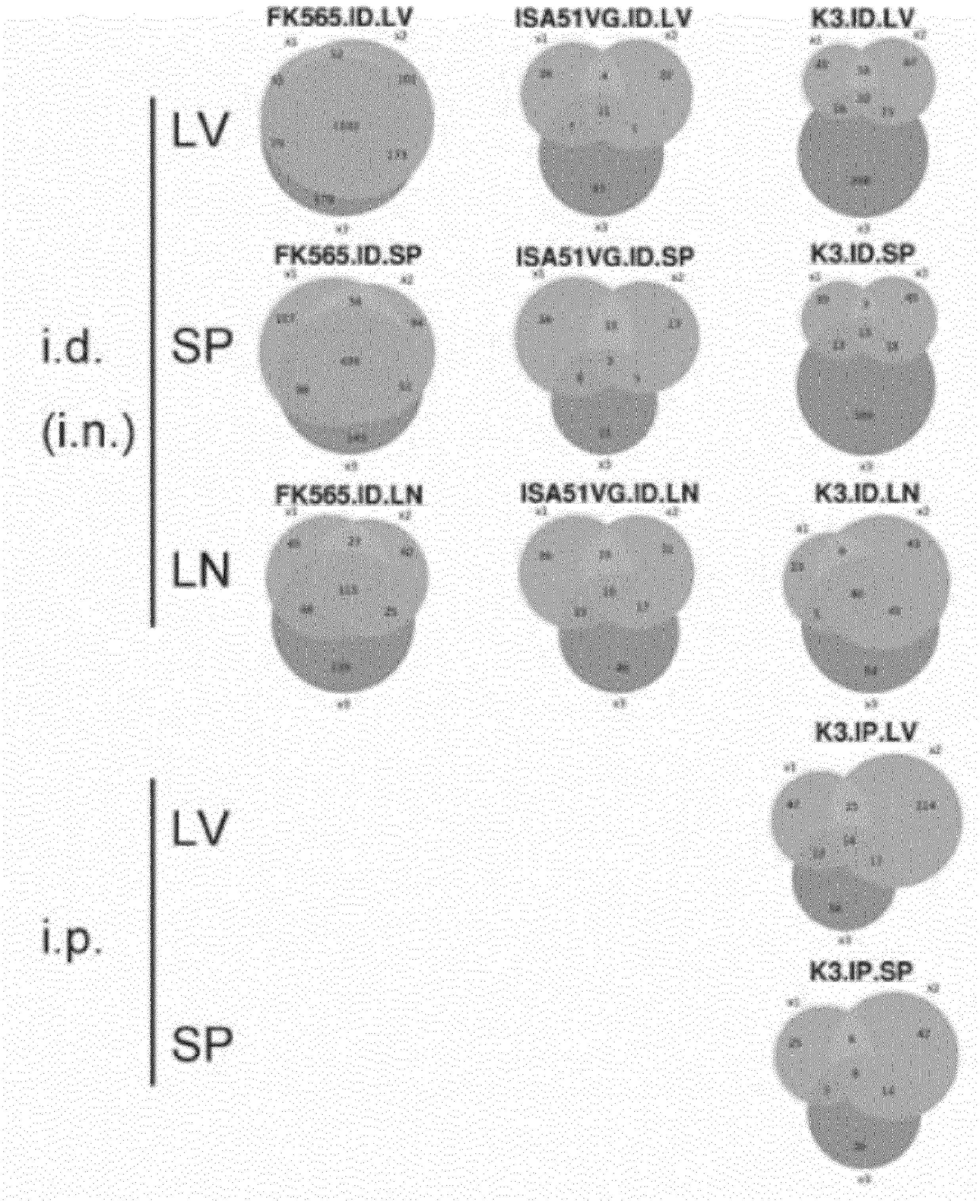

[FIG.8](**Continue**)

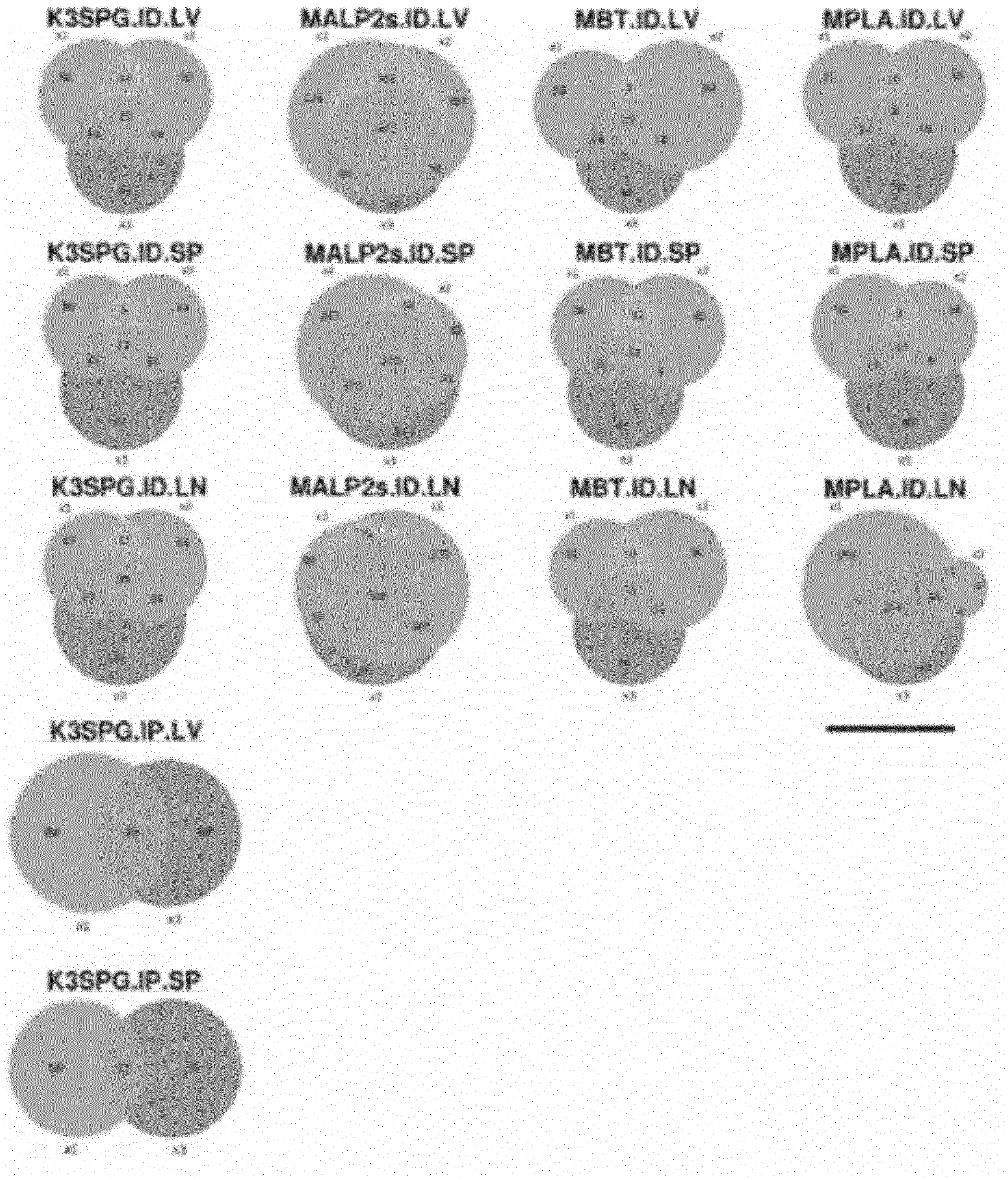

[FIG.8](**Continue**)

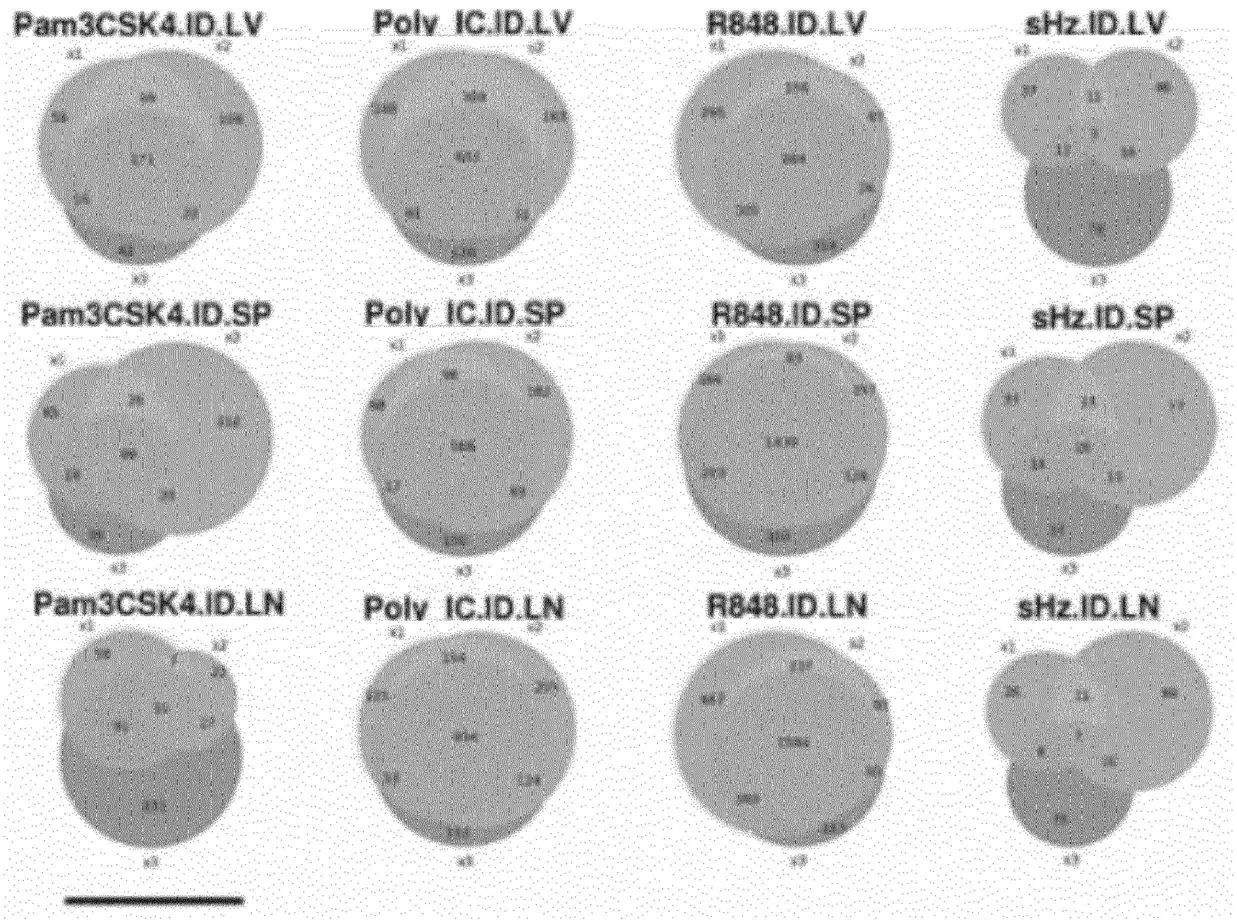

[FIG.9]

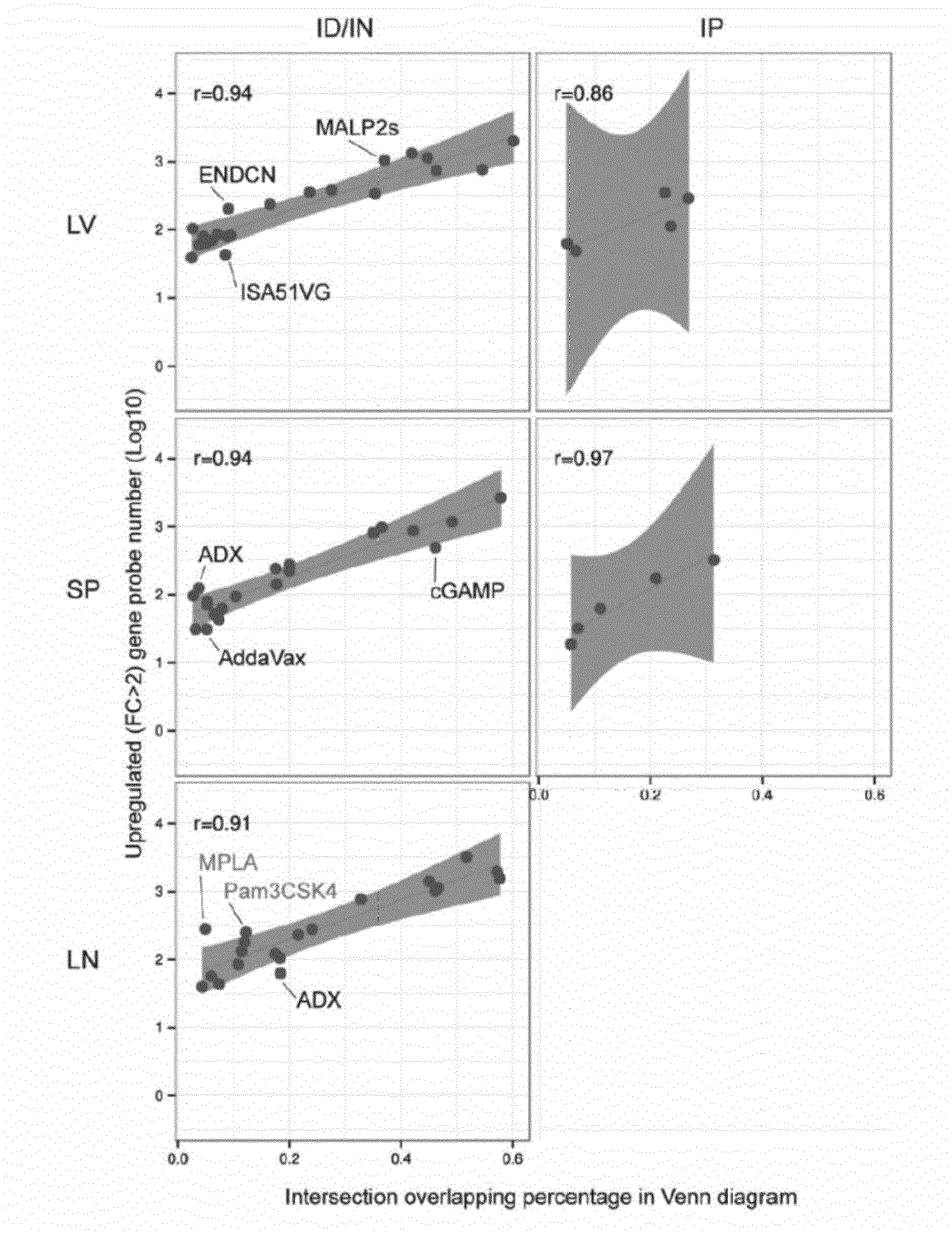
ID/IN
IP
LV
SP
LN
r=0.94
MALP2s
ENDCN
ISA51VG
r=0.86
r=0.94
ADX
cGAMP
AddaVax
r=0.97
r=0.91
MPLA
Pam3CSK4
ADX
Upregulated (FC>2) gene probe number (Log10)
Intersection overlapping percentage in Venn diagram
0.0
0.2
0.4
0.6
0
1
2
3
4

[FIG.10A]

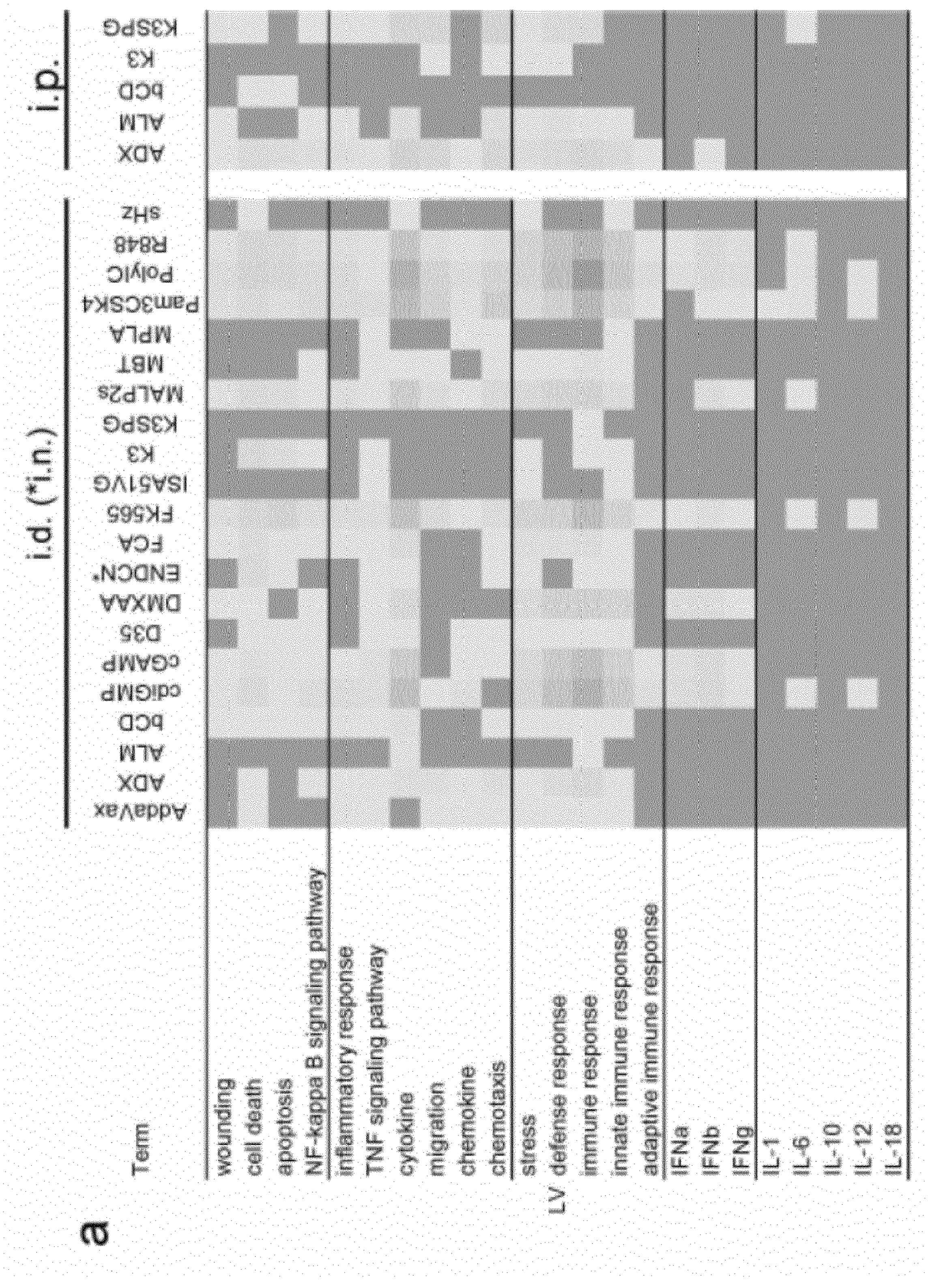
a
Term
i.d. (*i.n.)
i.p.
Addavax
ADX
ALM
bCD
cdiGMP
cGAMP
D35
DMXAA
ENDCN*
FCA
FK565
ISA51VG
K3
K3SPG
MALP2s
MBT
MPLA
Pam3CSK4
PolyIC
R848
sHz
ADX
ALM
bCD
K3
K3SPG
wounding
cell death
apoptosis
NF-kappa B signaling pathway
inflammatory response
TNF signaling pathway
cytokine
migration
chemokine
chemotaxis
stress
LV
defense response
immune response
innate immune response
adaptive immune response
IFNa
IFNb
IFNg
IL-1
IL-6
IL-10
IL-12
IL-18

[FIG.10B]

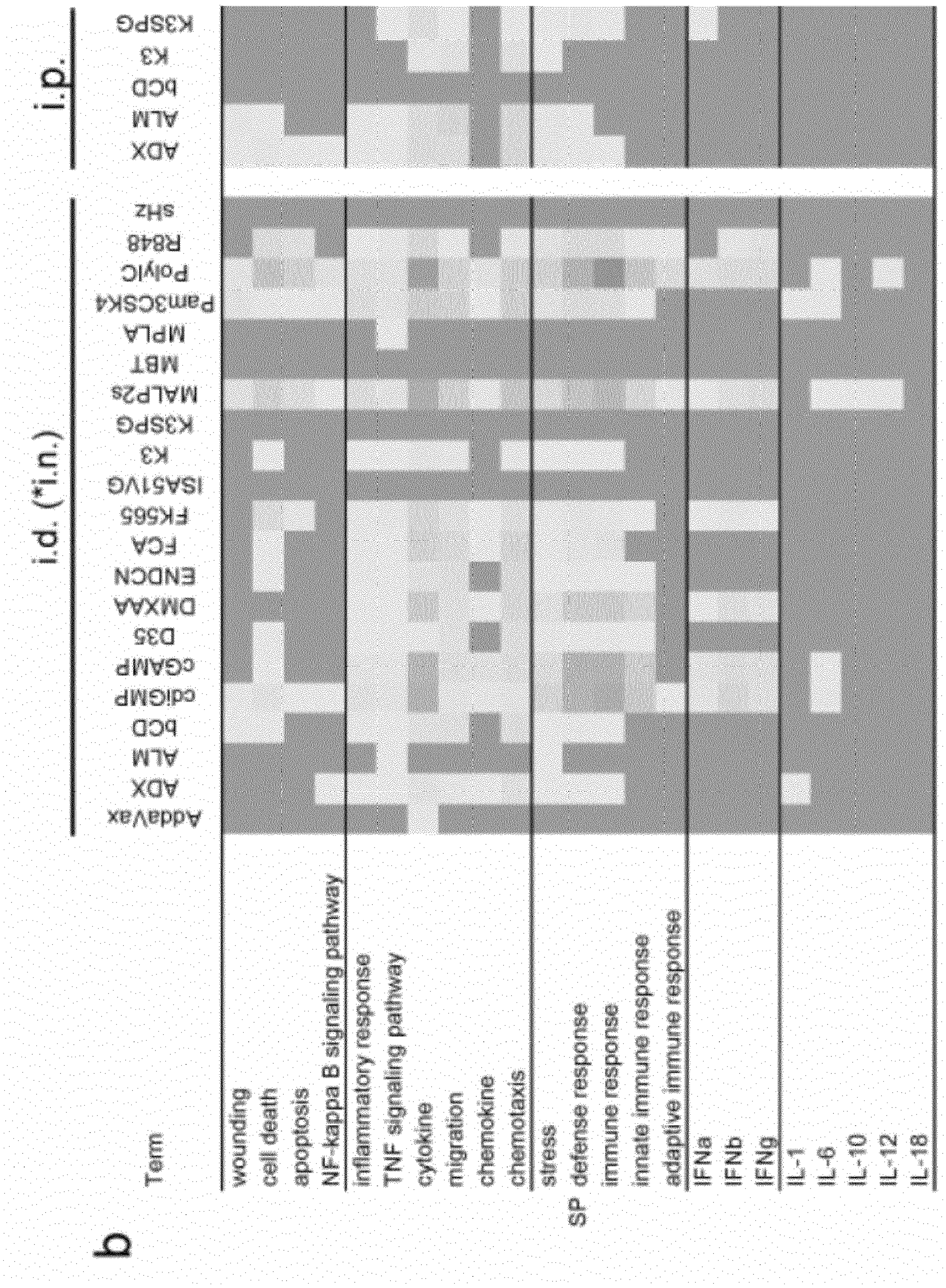
b
Term
wounding
cell death
apoptosis
NF-kappa B signaling pathway
inflammatory response
TNF signaling pathway
cytokine
migration
chemokine
chemotaxis
stress
SP
defense response
immune response
innate immune response
adaptive immune response
IFNa
IFNb
IFNg
IL-1
IL-6
IL-10
IL-12
IL-18
i.d. (*i.n.)
AddaVax
ADX
ALM
bCD
cdiGMP
cGAMP
D35
DMXAA
ENDCN
FCA
FK565
ISA51VG
K3
K3SPG
MALP2s
MBT
MPLA
Pam3CSK4
PolyIC
R848
sHz
i.p.
ADX
ALM
bCD
K3
K3SPG

[FIG.10C]

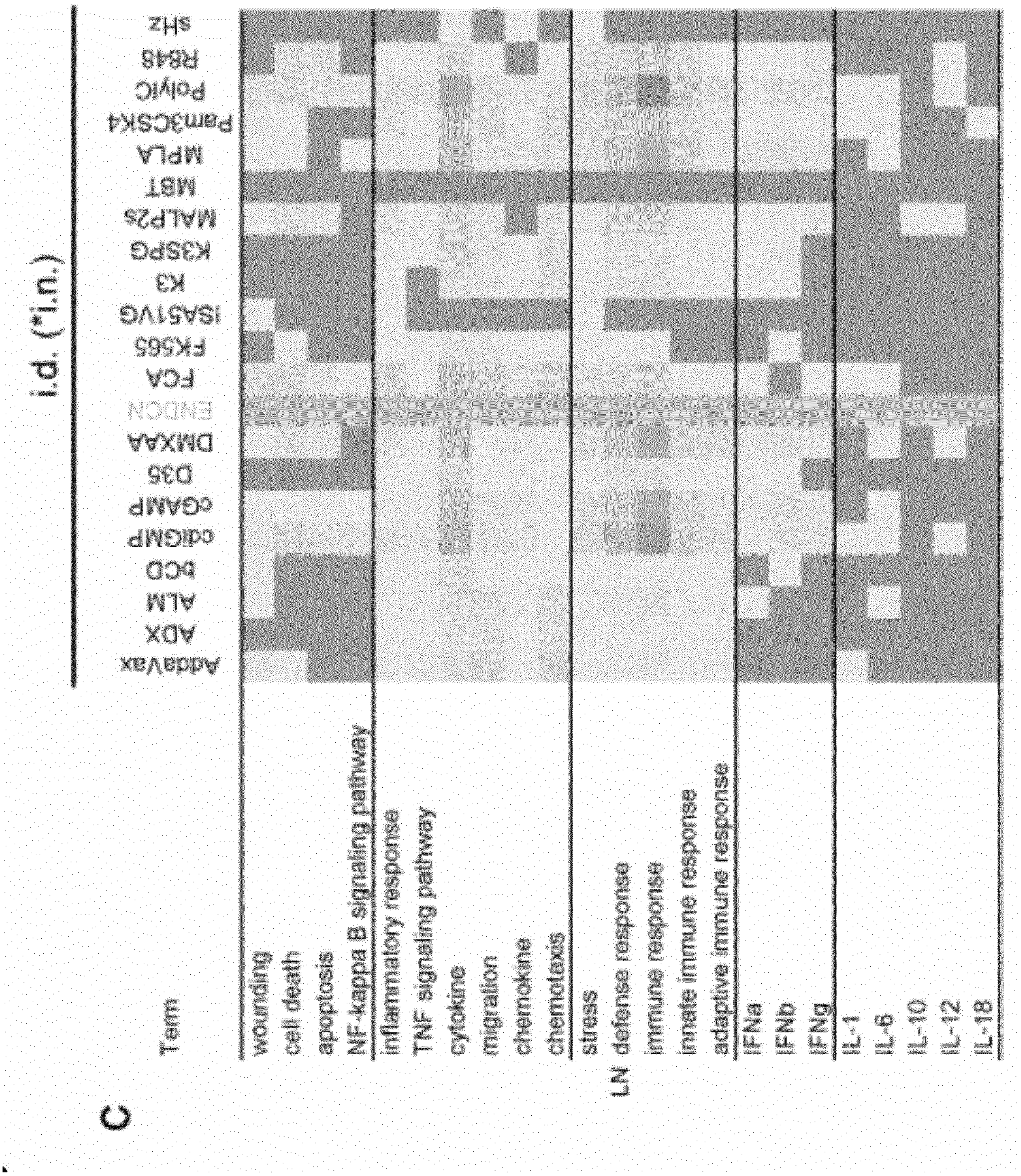
C
i.d. (*i.n.)
Term
AddaVax
ADX
ALM
bCD
cdiGMP
cGAMP
D35
DMXAA
ENDCN
FCA
FK565
ISA51VG
K3
K3SPG
MALP2s
MBT
MPLA
Pam3CSK4
PolyIC
R848
sHz
wounding
cell death
apoptosis
NF-kappa B signaling pathway
inflammatory response
TNF signaling pathway
cytokine
migration
chemokine
chemotaxis
stress
LN
defense response
immune response
innate immune response
adaptive immune response
IFNa
IFNb
IFNg
IL-1
IL-6
IL-10
IL-12
IL-18

[FIG.11A]

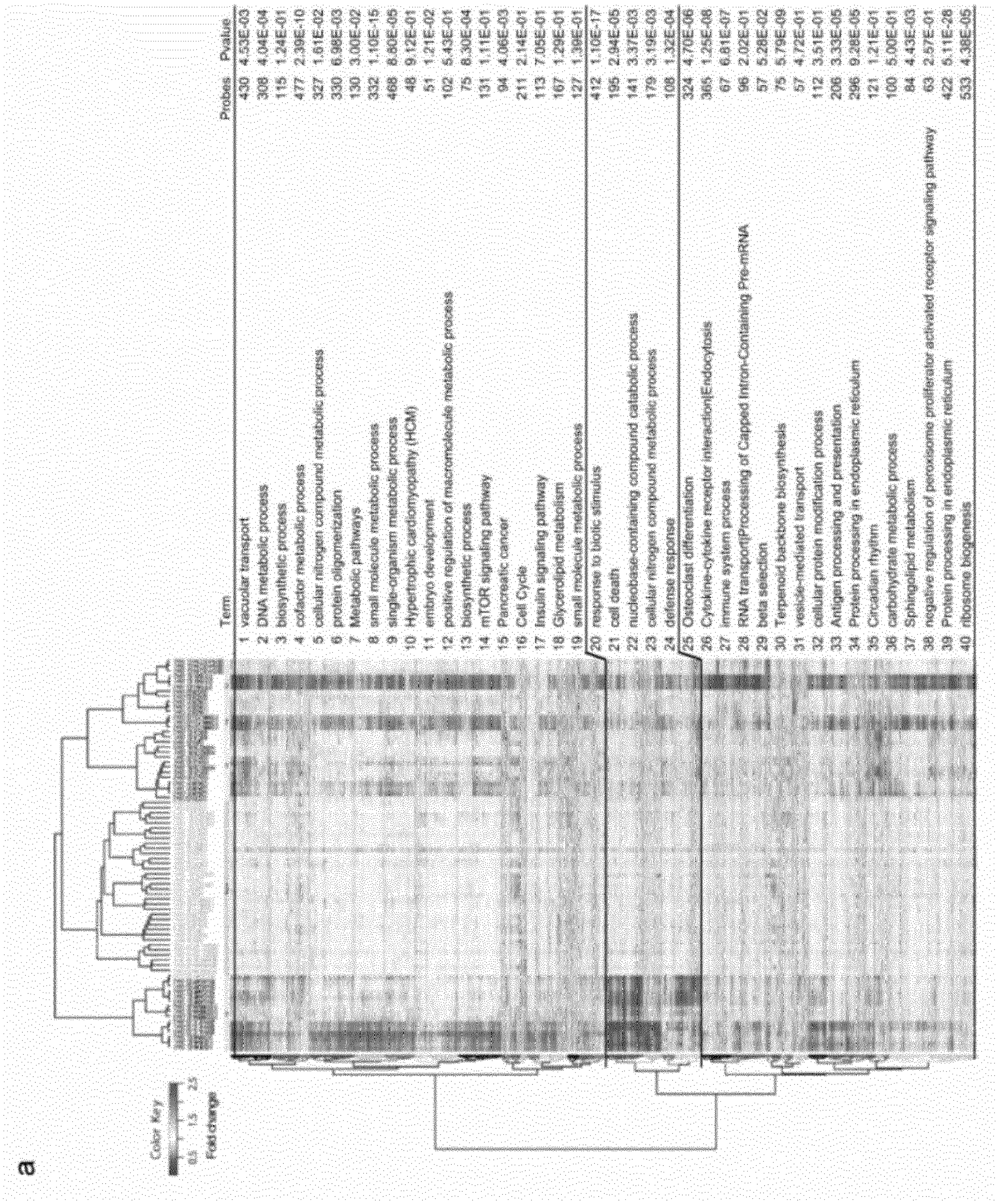
a
Color Key
0.5 1.5 2.5
Fold change
Term
Probes
Pvalue
1 vacuolar transport 430 4.53E-03
2 DNA metabolic process 308 4.04E-04
3 biosynthetic process 115 1.24E-01
4 cofactor metabolic process 477 2.39E-10
5 cellular nitrogen compound metabolic process 327 1.61E-02
6 protein oligomerization 330 6.98E-03
7 Metabolic pathways 130 3.00E-02
8 small molecule metabolic process 332 1.10E-15
9 single-organism metabolic process 468 8.80E-05
10 Hypertrophic cardiomyopathy (HCM) 48 9.12E-01
11 embryo development 51 1.21E-02
12 positive regulation of macromolecule metabolic process 102 5.43E-01
13 biosynthetic process 75 8.30E-04
14 mTOR signaling pathway 131 1.11E-01
15 Pancreatic cancer 94 4.06E-03
16 Cell Cycle 211 2.14E-01
17 Insulin signaling pathway 113 7.05E-01
18 Glycerolipid metabolism 167 1.29E-01
19 small molecule metabolic process 127 1.38E-01
20 response to biotic stimulus 412 1.10E-17
21 cell death 195 2.94E-05
22 nucleobase-containing compound catabolic process 141 3.37E-03
23 cellular nitrogen compound metabolic process 179 3.19E-03
24 defense response 108 1.32E-04
25 Osteoclast differentiation 324 4.70E-06
26 Cytokine-cytokine receptor interaction|Endocytosis 365 1.25E-08
27 immune system process 67 6.81E-07
28 RNA transport|Processing of Capped Intron-Containing Pre-mRNA 96 2.02E-01
29 beta selection 57 5.28E-02
30 Terpenoid backbone biosynthesis 75 5.79E-09
31 vesicle-mediated transport 57 4.72E-01
32 cellular protein modification process 112 3.51E-01
33 Antigen processing and presentation 206 3.33E-05
34 Protein processing in endoplasmic reticulum 296 9.28E-05
35 Circadian rhythm 121 1.21E-01
36 carbohydrate metabolic process 100 5.00E-01
37 Sphingolipid metabolism 84 4.43E-03
38 negative regulation of peroxisome proliferator activated receptor signaling pathway 63 2.57E-01
39 Protein processing in endoplasmic reticulum 422 5.11E-28
40 ribosome biogenesis 533 4.38E-05

[FIG.11B]

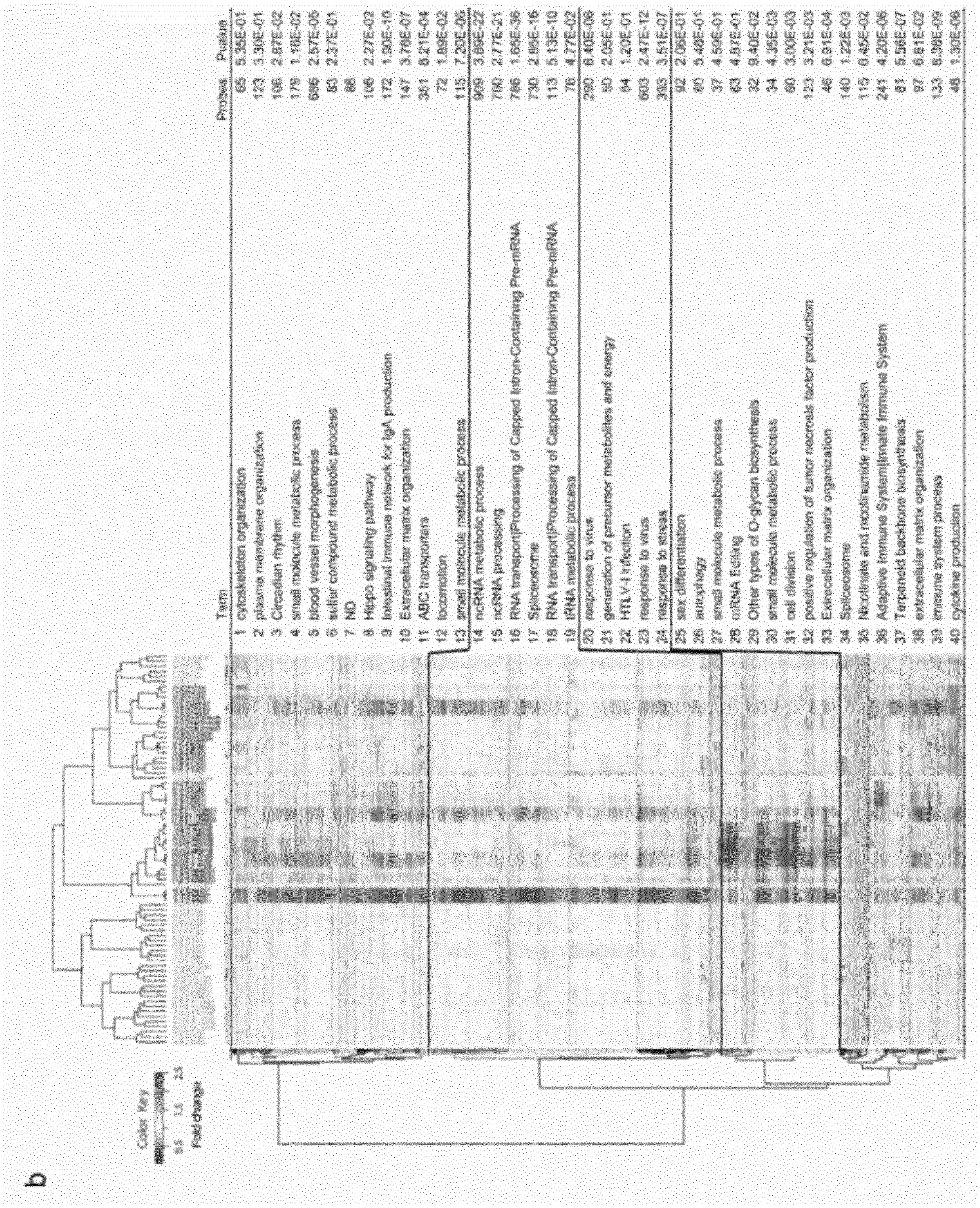
b
Color Key
0.5 1 1.5 2 2.5
Fold change
Term Probes Pvalue
1 cytoskeleton organization 65 5.35E-01
2 plasma membrane organization 123 3.30E-01
3 Circadian rhythm 106 2.87E-02
4 small molecule metabolic process 179 1.16E-02
5 blood vessel morphogenesis 686 2.57E-05
6 sulfur compound metabolic process 83 2.37E-01
7 ND 88
8 Hippo signaling pathway 106 2.27E-02
9 Intestinal immune network for IgA production 172 1.90E-10
10 Extracellular matrix organization 147 3.76E-07
11 ABC transporters 351 8.21E-04
12 locomotion 72 1.89E-02
13 small molecule metabolic process 115 7.20E-06
14 ncRNA metabolic process 909 3.69E-22
15 ncRNA processing 700 2.77E-21
16 RNA transport|Processing of Capped Intron-Containing Pre-mRNA 786 1.65E-36
17 Spliceosome 730 2.85E-16
18 RNA transport|Processing of Capped Intron-Containing Pre-mRNA 113 5.13E-10
19 tRNA metabolic process 76 4.77E-02
20 response to virus 290 6.40E-06
21 generation of precursor metabolites and energy 50 2.05E-01
22 HTLV-I infection 84 1.20E-01
23 response to virus 603 2.47E-12
24 response to stress 393 3.51E-07
25 sex differentiation 92 2.06E-01
26 autophagy 80 5.48E-01
27 small molecule metabolic process 37 4.59E-01
28 mRNA Editing 63 4.87E-01
29 Other types of O-glycan biosynthesis 32 9.40E-02
30 small molecule metabolic process 34 4.35E-03
31 cell division 60 3.00E-03
32 positive regulation of tumor necrosis factor production 123 3.21E-03
33 Extracellular matrix organization 46 6.91E-04
34 Spliceosome 140 1.22E-03
35 Nicotinate and nicotinamide metabolism 115 6.45E-02
36 Adaptive Immune System|Innate Immune System 241 4.20E-06
37 Terpenoid backbone biosynthesis 81 5.56E-07
38 extracellular matrix organization 97 6.81E-02
39 immune system process 133 8.38E-09
40 cytokine production 48 1.30E-06

[FIG.11C]

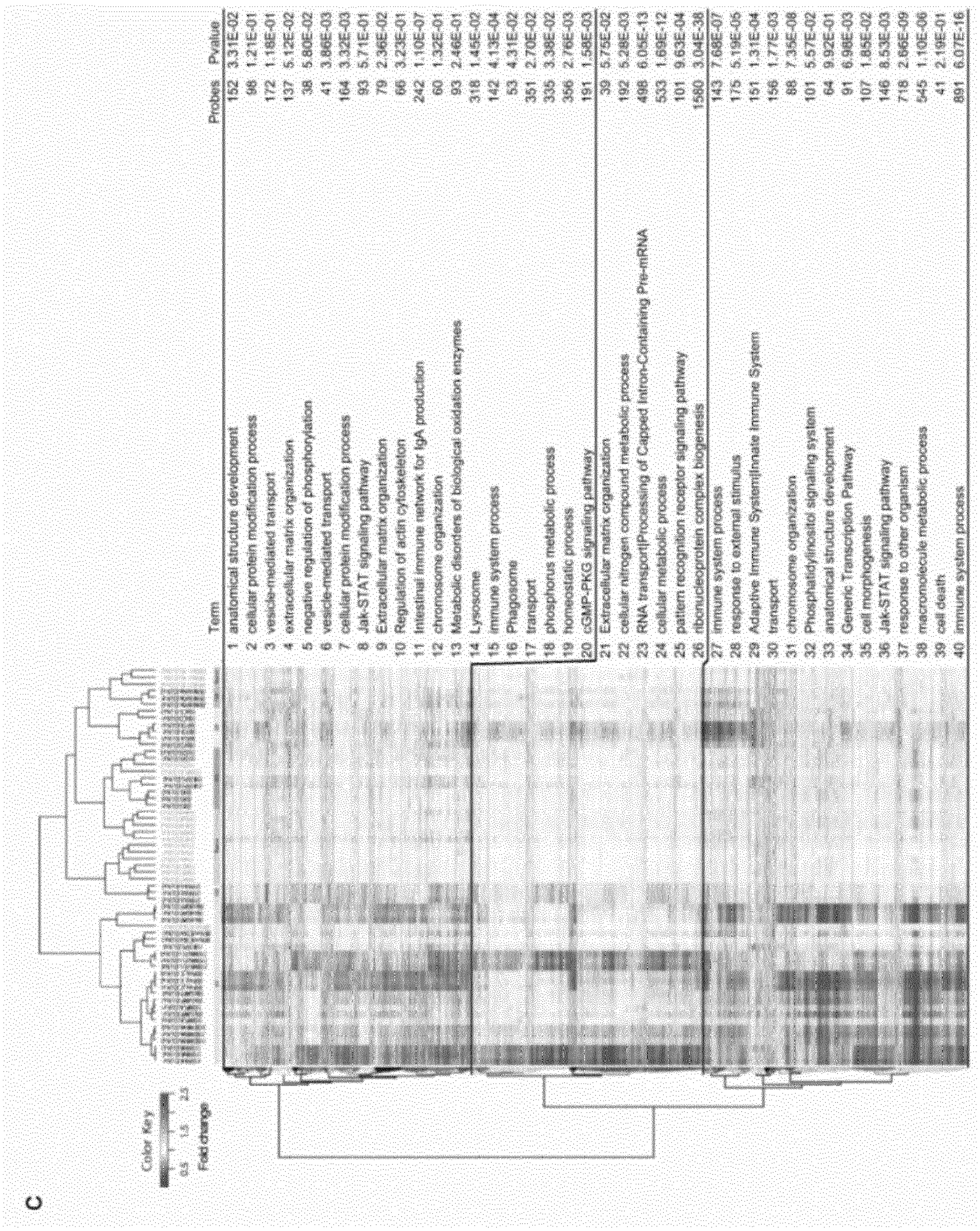
C
Color Key
0.5 1 1.5 2.5
Fold change
Term
Probes
Pvalue
1 anatomical structure development 152 3.31E-02
2 cellular protein modification process 98 1.21E-01
3 vesicle-mediated transport 172 1.18E-01
4 extracellular matrix organization 137 5.12E-02
5 negative regulation of phosphorylation 38 5.80E-02
6 vesicle-mediated transport 41 3.86E-03
7 cellular protein modification process 164 3.32E-03
8 Jak-STAT signaling pathway 93 5.71E-01
9 Extracellular matrix organization 79 2.36E-02
10 Regulation of actin cytoskeleton 66 3.23E-01
11 Intestinal immune network for IgA production 242 1.10E-07
12 chromosome organization 60 1.32E-01
13 Metabolic disorders of biological oxidation enzymes 93 2.46E-01
14 Lysosome 318 1.45E-02
15 immune system process 142 4.13E-04
16 Phagosome 53 4.31E-02
17 transport 351 2.70E-02
18 phosphorus metabolic process 335 3.38E-02
19 homeostatic process 356 2.76E-03
20 cGMP-PKG signaling pathway 191 1.58E-03
21 Extracellular matrix organization 39 5.75E-02
22 cellular nitrogen compound metabolic process 192 5.28E-03
23 RNA transport|Processing of Capped Intron-Containing Pre-mRNA 498 6.05E-13
24 cellular metabolic process 533 1.69E-12
25 pattern recognition receptor signaling pathway 101 9.63E-04
26 ribonucleoprotein complex biogenesis 1580 3.04E-38
27 immune system process 143 7.68E-07
28 response to external stimulus 175 5.19E-05
29 Adaptive Immune System|Innate Immune System 151 1.31E-04
30 transport 156 1.77E-03
31 chromosome organization 88 7.35E-08
32 Phosphatidylinositol signaling system 101 5.57E-02
33 anatomical structure development 64 9.92E-01
34 Generic Transcription Pathway 91 6.98E-03
35 cell morphogenesis 107 1.85E-02
36 Jak-STAT signaling pathway 146 8.53E-03
37 response to other organism 718 2.66E-09
38 macromolecule metabolic process 545 1.10E-06
39 cell death 41 2.19E-01
40 immune system process 891 6.07E-16

[FIG.12A]

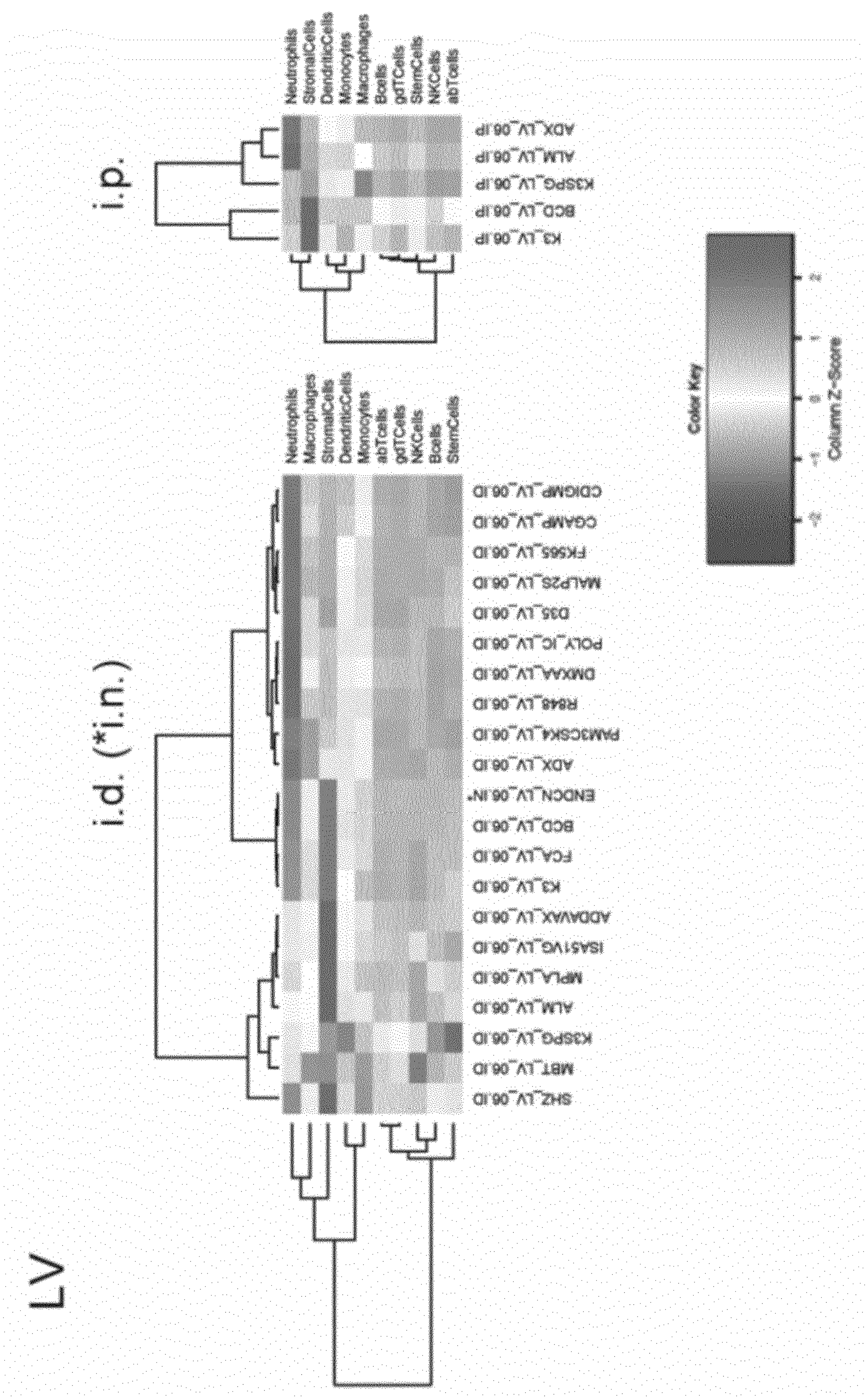
LV
i.d. (*i.n.)
i.p.
Color Key
Column Z-Score
Neutrophils
Macrophages
StromalCells
DendriticCells
Monocytes
abTcells
gdTCells
NKCells
Bcells
StemCells
CDIGMP_LV_06.ID
CGAMP_LV_06.ID
FK565_LV_06.ID
MALP2S_LV_06.ID
D35_LV_06.ID
POLY_IC_LV_06.ID
DMXAA_LV_06.ID
R848_LV_06.ID
PAM3CSK4_LV_06.ID
ADX_LV_06.ID
ENDCN_LV_06.IN*
BCD_LV_06.ID
FCA_LV_06.ID
K3_LV_06.ID
ADDAVAX_LV_06.ID
ISA51VG_LV_06.ID
MPLA_LV_06.ID
ALM_LV_06.ID
K3SPG_LV_06.ID
MBT_LV_06.ID
SHZ_LV_06.ID
ADX_LV_06.IP
ALM_LV_06.IP
K3SPG_LV_06.IP
BCD_LV_06.IP
K3_LV_06.IP

[FIG.12B]

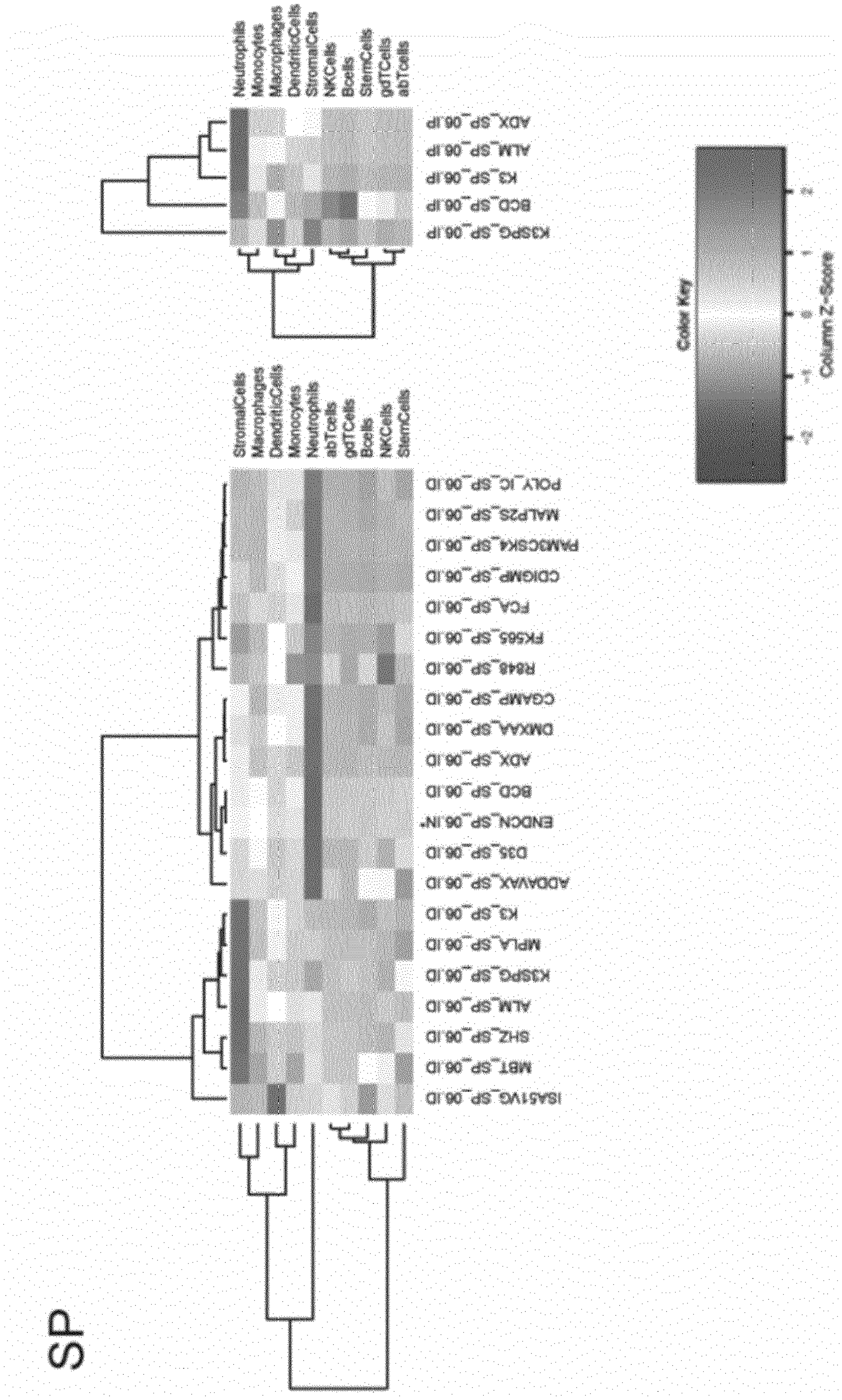
SP
Neutrophils
Monocytes
Macrophages
DendriticCells
StromalCells
NKCells
Bcells
StemCells
gdTCells
abTCells
ADX_SP_06.IP
ALM_SP_06.IP
K3_SP_06.IP
BCD_SP_06.IP
K3SPG_SP_06.IP
StromalCells
Macrophages
DendriticCells
Monocytes
Neutrophils
abTCells
gdTCells
Bcells
NKCells
StemCells
POLY_IC_SP_06.ID
MALP2S_SP_06.ID
PAM3CSK4_SP_06.ID
CDIGMP_SP_06.ID
FCA_SP_06.ID
FK565_SP_06.ID
R848_SP_06.ID
CGAMP_SP_06.ID
DMXAA_SP_06.ID
ADX_SP_06.ID
BCD_SP_06.ID
ENDCN_SP_06.IN*
D35_SP_06.ID
ADDAVAX_SP_06.ID
K3_SP_06.ID
MPLA_SP_06.ID
K3SPG_SP_06.ID
ALM_SP_06.ID
SHZ_SP_06.ID
MBT_SP_06.ID
ISA51VG_SP_06.ID
Color Key
-2 -1 0 1 2
Column Z-Score

[FIG.12C]

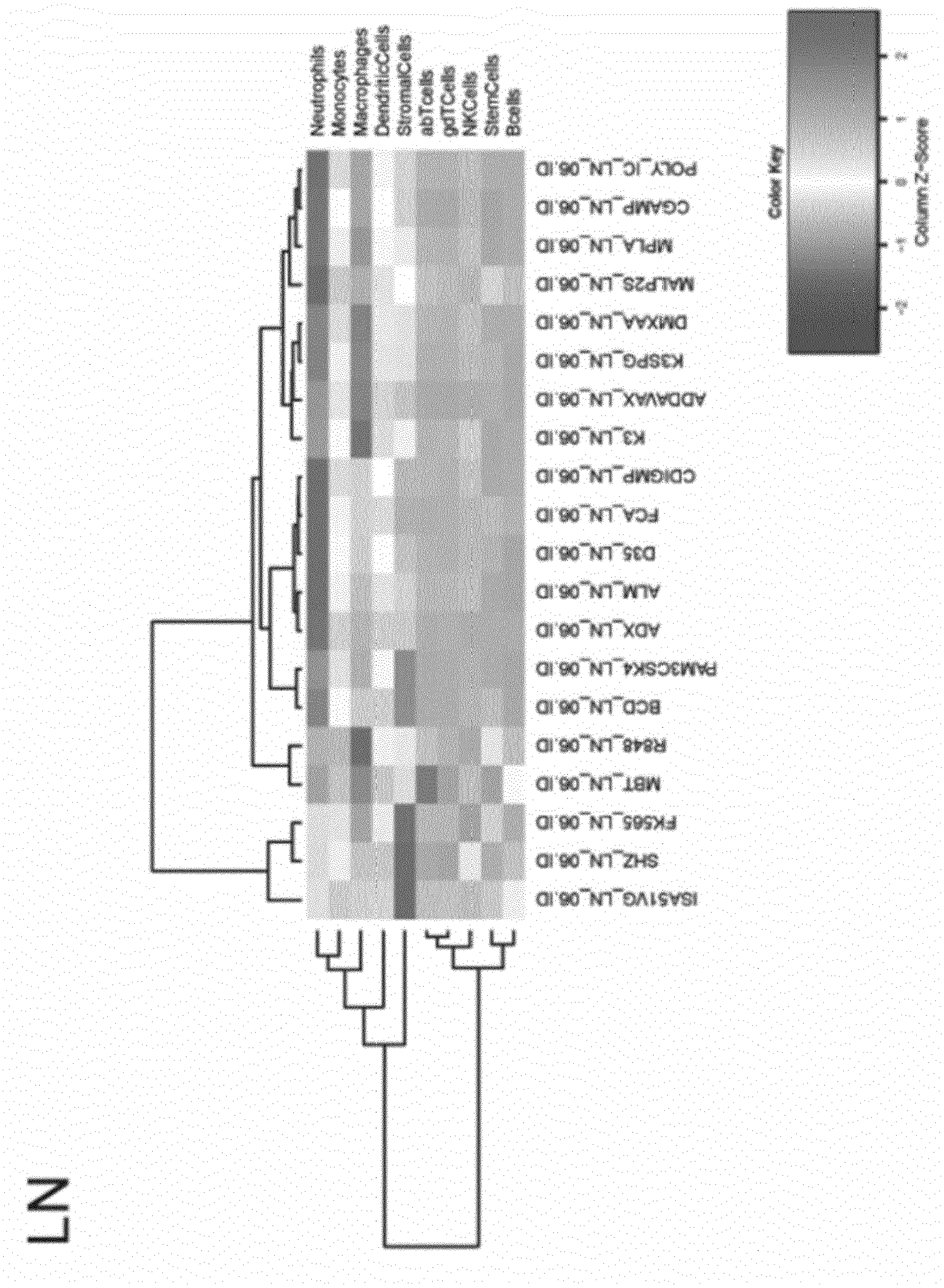
LN
Neutrophils
Monocytes
Macrophages
DendriticCells
StromalCells
abTcells
gdTCells
NKCells
StemCells
Bcells
Color Key
Column Z-Score
-2
-1
0
1
2
POLY_IC_LN_06.ID
CGAMP_LN_06.ID
MPLA_LN_06.ID
MALP2S_LN_06.ID
DMXAA_LN_06.ID
K3SPG_LN_06.ID
ADDAVAX_LN_06.ID
K3_LN_06.ID
CDIGMP_LN_06.ID
FCA_LN_06.ID
D35_LN_06.ID
ALM_LN_06.ID
ADX_LN_06.ID
PAM3CSK4_LN_06.ID
BCD_LN_06.ID
R848_LN_06.ID
MBT_LN_06.ID
FK565_LN_06.ID
SHZ_LN_06.ID
ISA51VG_LN_06.ID

[FIG.13A]

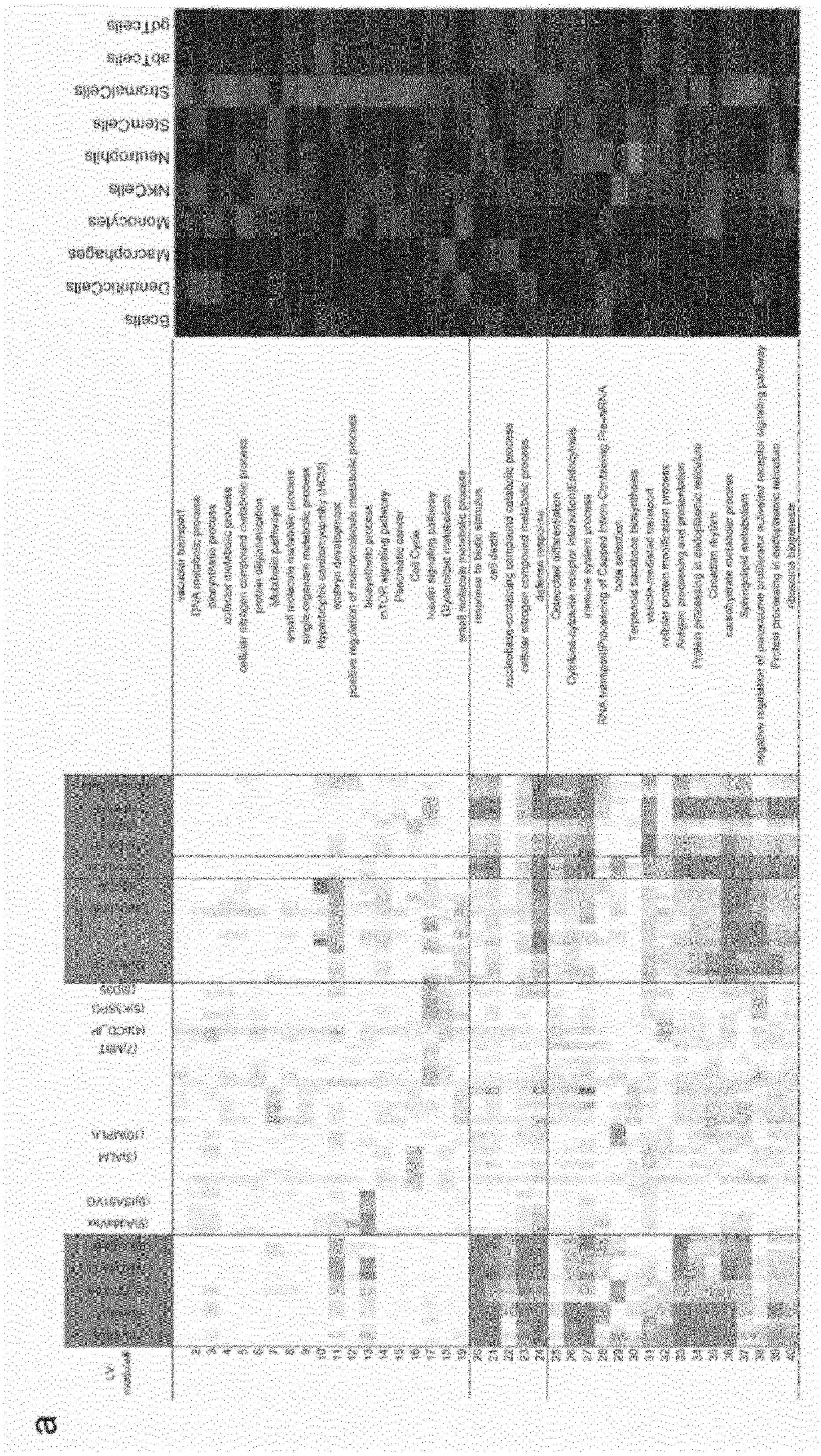

[FIG.13B]

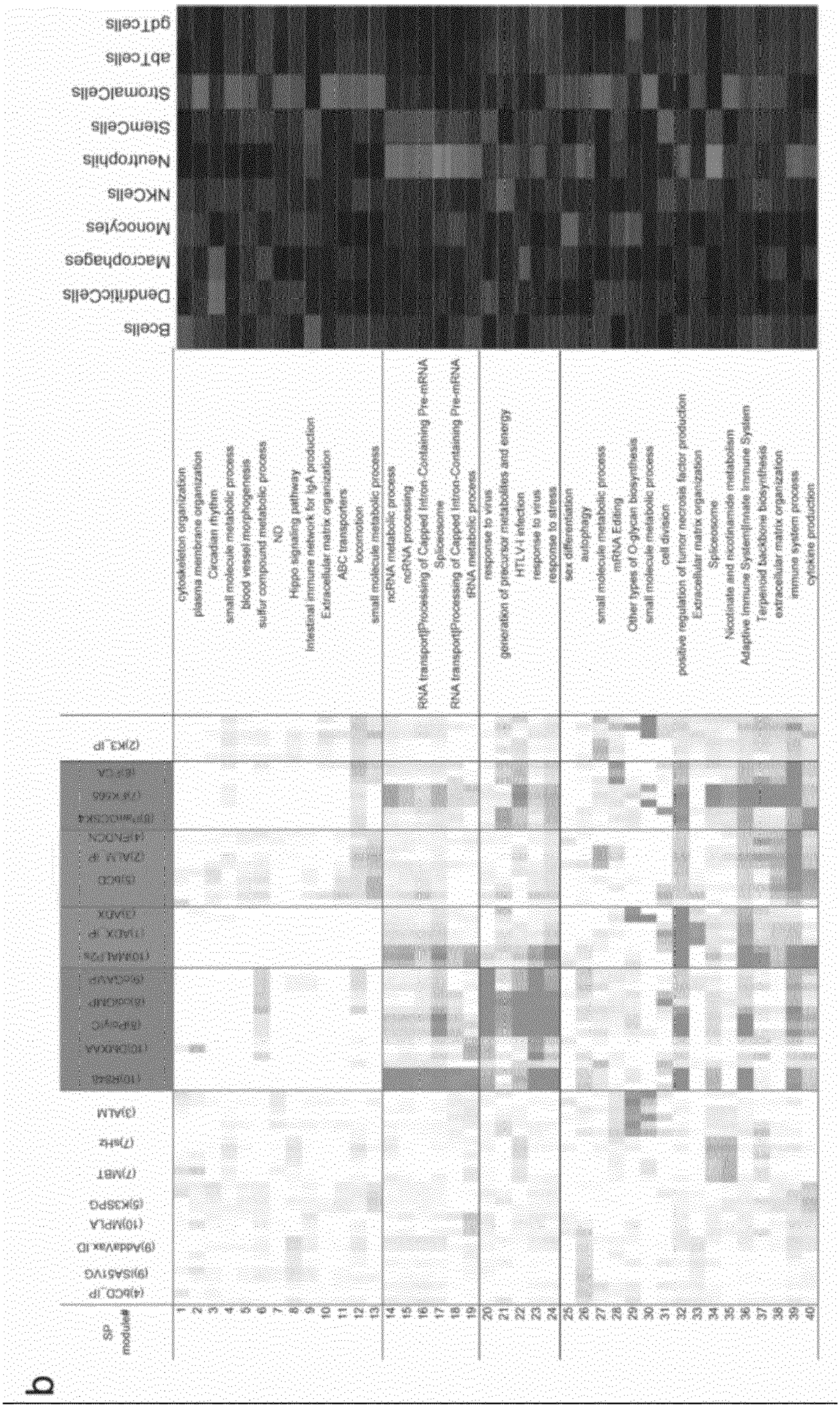
b
SP module#
Bcells
DendriticCells
Macrophages
Monocytes
NKCells
Neutrophils
StemCells
StromalCells
abTcells
gdTcells
cytoskeleton organization
plasma membrane organization
Circadian rhythm
small molecule metabolic process
blood vessel morphogenesis
sulfur compound metabolic process
ND
Hippo signaling pathway
Intestinal immune network for IgA production
Extracellular matrix organization
ABC transporters
locomotion
small molecule metabolic process
ncRNA metabolic process
ncRNA processing
RNA transport|Processing of Capped Intron-Containing Pre-mRNA
Spliceosome
RNA transport|Processing of Capped Intron-Containing Pre-mRNA
tRNA metabolic process
response to virus
generation of precursor metabolites and energy
HTLV-I infection
response to virus
response to stress
sex differentiation
autophagy
small molecule metabolic process
mRNA Editing
Other types of O-glycan biosynthesis
small molecule metabolic process
cell division
positive regulation of tumor necrosis factor production
Extracellular matrix organization
Spliceosome
Nicotinate and nicotinamide metabolism
Adaptive Immune System|Innate Immune System
Terpenoid backbone biosynthesis
extracellular matrix organization
immune system process
cytokine production
1
2
3
4
5
6
7
8
9
10
11
12
13
14
15
16
17
18
19
20
21
22
23
24
25
26
27
28
29
30
31
32
33
34
35
36
37
38
39
40

[FIG.13C]

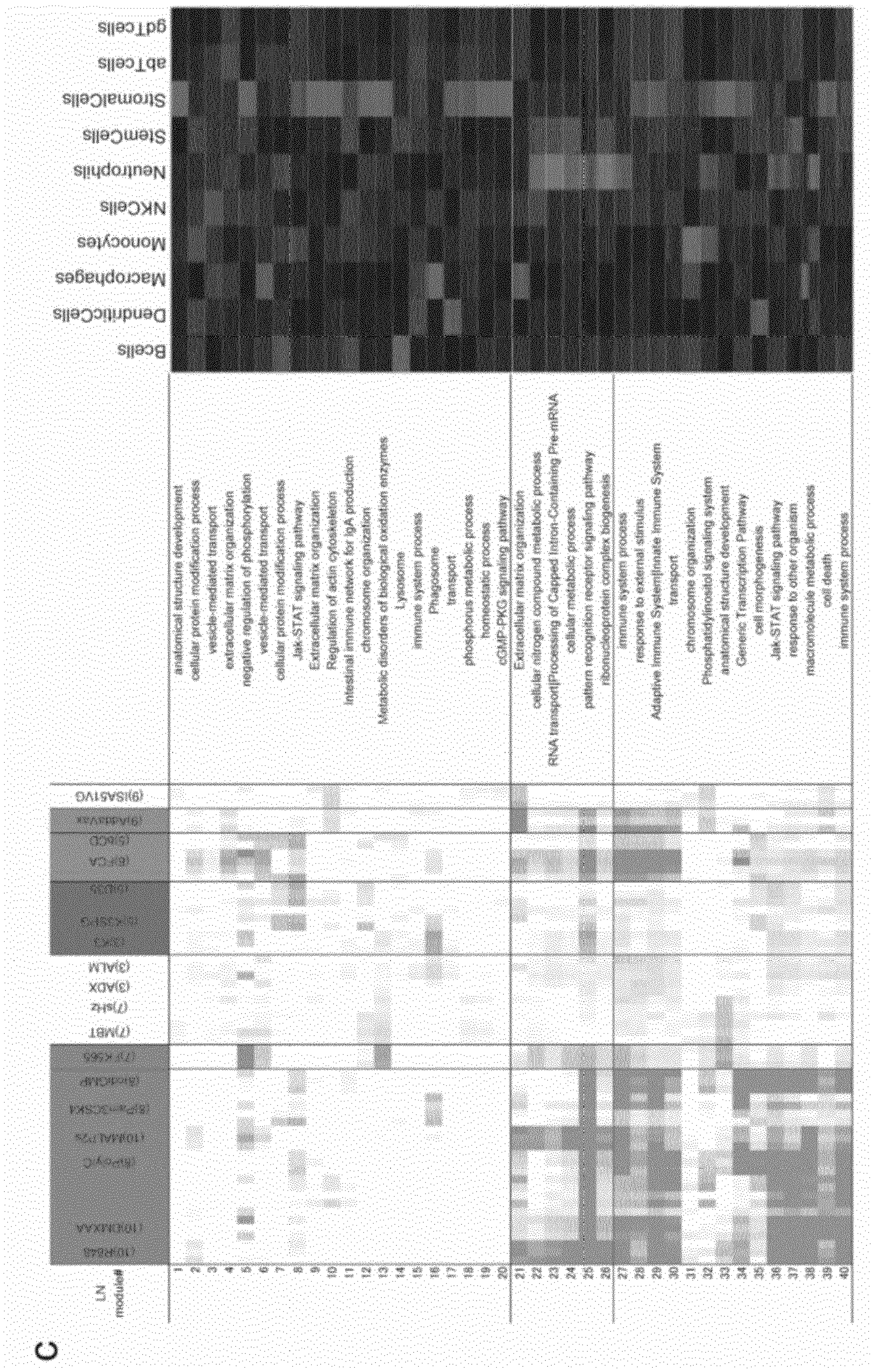
C
LN module#
gdTcells
abTcells
StromalCells
StemCells
Neutrophils
NKCells
Monocytes
Macrophages
DendriticCells
Bcells
anatomical structure development
cellular protein modification process
vesicle-mediated transport
extracellular matrix organization
negative regulation of phosphorylation
vesicle-mediated transport
cellular protein modification process
Jak-STAT signaling pathway
Extracellular matrix organization
Regulation of actin cytoskeleton
Intestinal immune network for IgA production
chromosome organization
Metabolic disorders of biological oxidation enzymes
Lysosome
immune system process
Phagosome
transport
phosphorus metabolic process
homeostatic process
cGMP-PKG signaling pathway
Extracellular matrix organization
cellular nitrogen compound metabolic process
RNA transport|Processing of Capped Intron-Containing Pre-mRNA
cellular metabolic process
pattern recognition receptor signaling pathway
ribonucleoprotein complex biogenesis
immune system process
response to external stimulus
Adaptive Immune System|Innate Immune System
transport
chromosome organization
Phosphatidylinositol signaling system
anatomical structure development
Generic Transcription Pathway
cell morphogenesis
Jak-STAT signaling pathway
response to other organism
macromolecule metabolic process
cell death
immune system process

[FIG.14A]

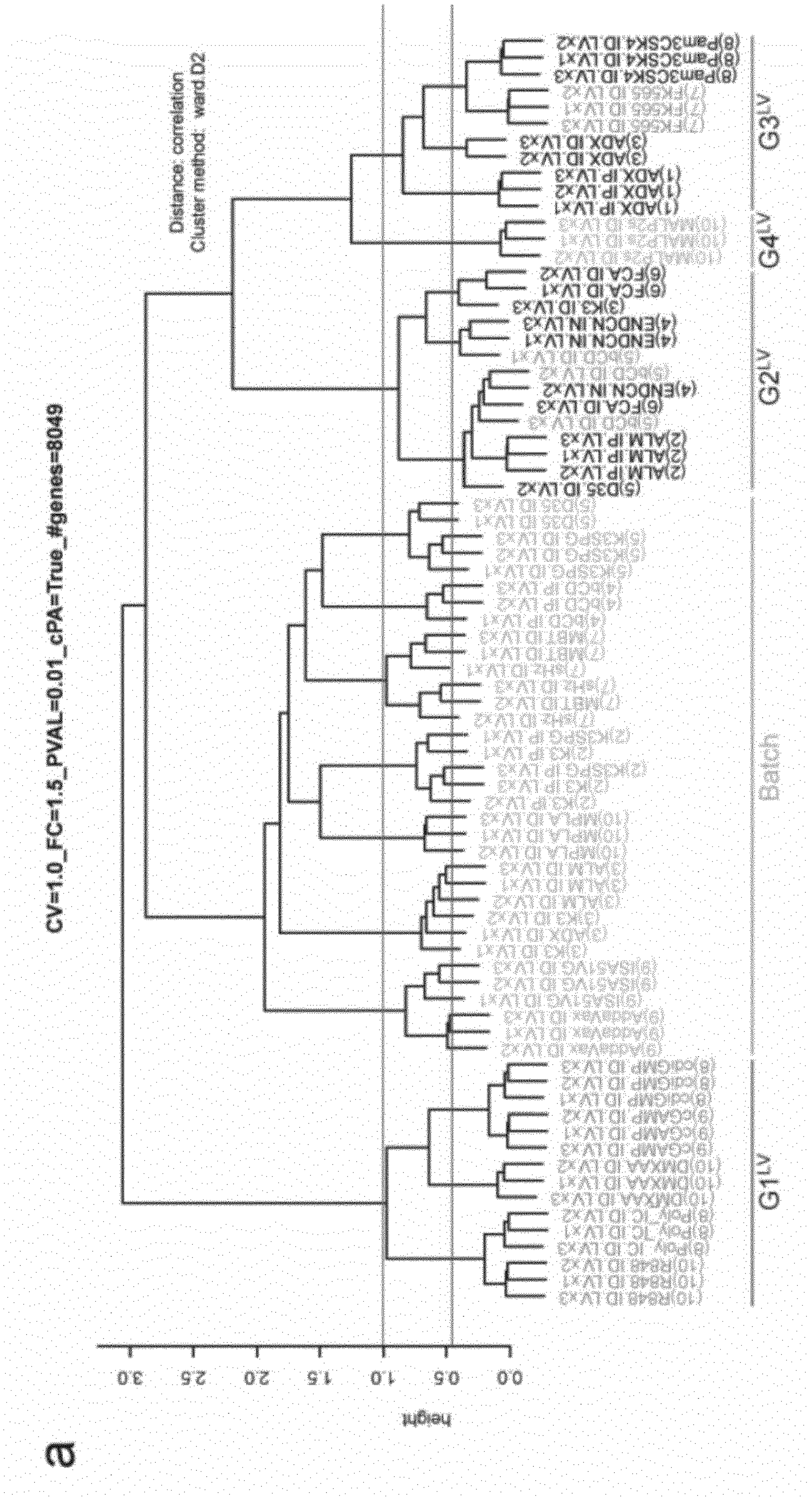
a
CV=1.0_FC=1.5_PVAL=0.01_cPA=True_#genes=8049
Distance: correlation
Cluster method: ward.D2
height
0.0
0.5
1.0
1.5
2.0
2.5
3.0
G1LV
Batch
G2LV
G4LV
G3LV

[FIG.14B]

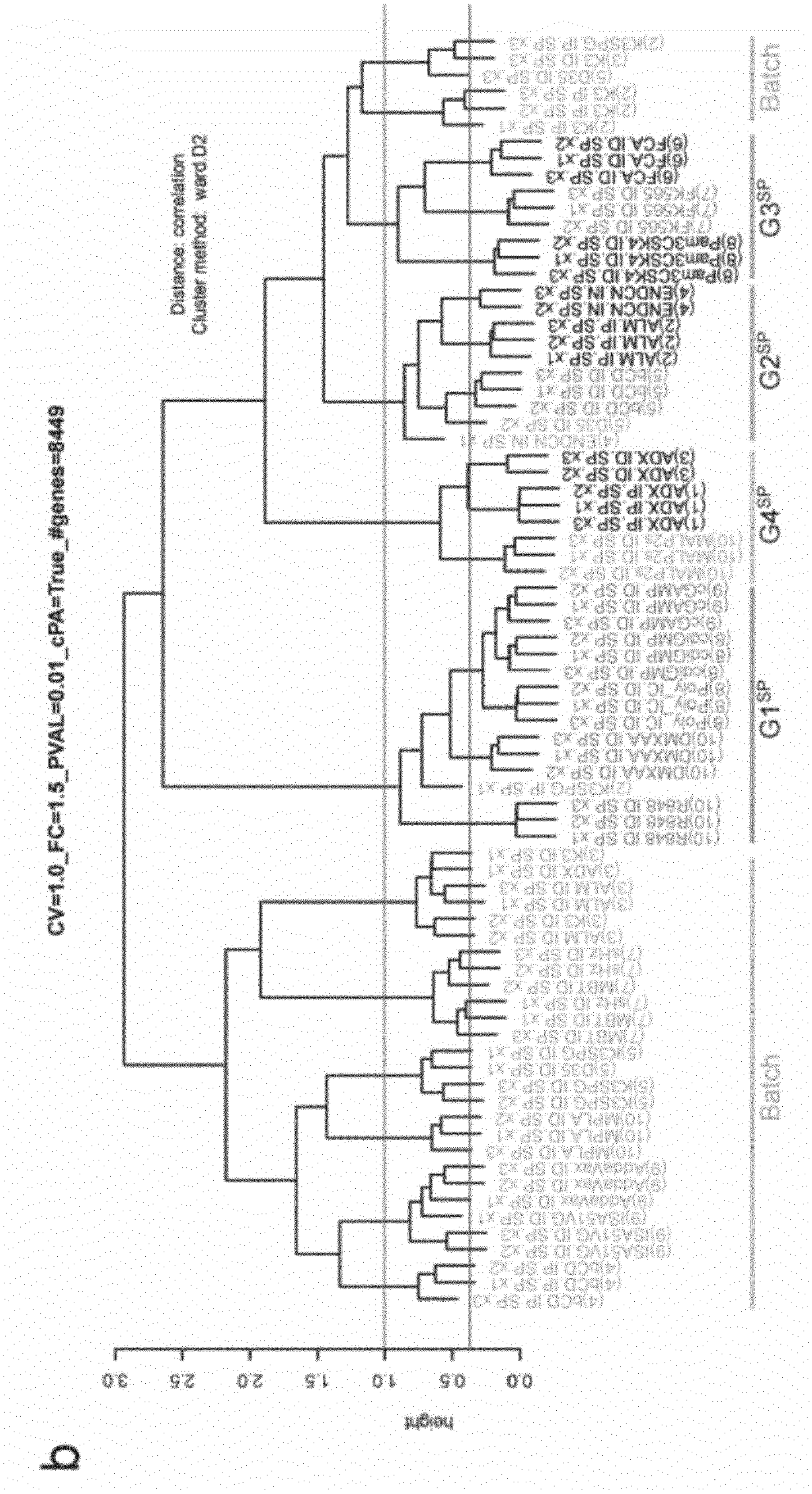
b
CV=1.0_FC=1.5_PVAL=0.01_cPA=True_#genes=8449
Distance: correlation
Cluster method: ward.D2
height
0.0
0.5
1.0
1.5
2.0
2.5
3.0
Batch
G3SP
G2SP
G4SP
G1SP
Batch

[FIG.14C]

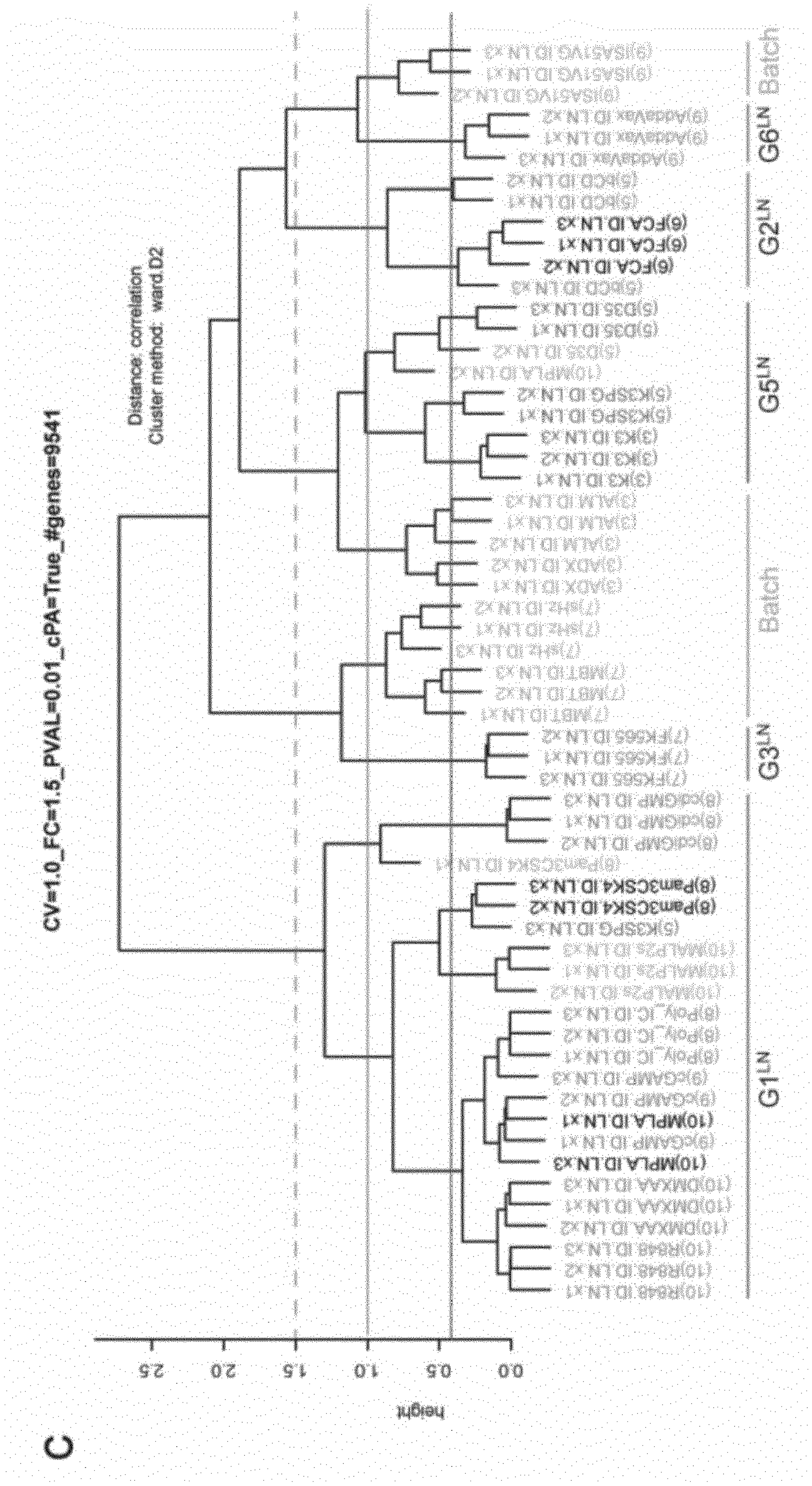
C
CV=1.0_FC=1.5_PVAL=0.01_cPA=True_#genes=9541
Distance: correlation
Cluster method: ward.D2
height
0.0
0.5
1.0
1.5
2.0
2.5
Batch
G6LN
G2LN
G5LN
Batch
G3LN
G1LN

[FIG.14D]

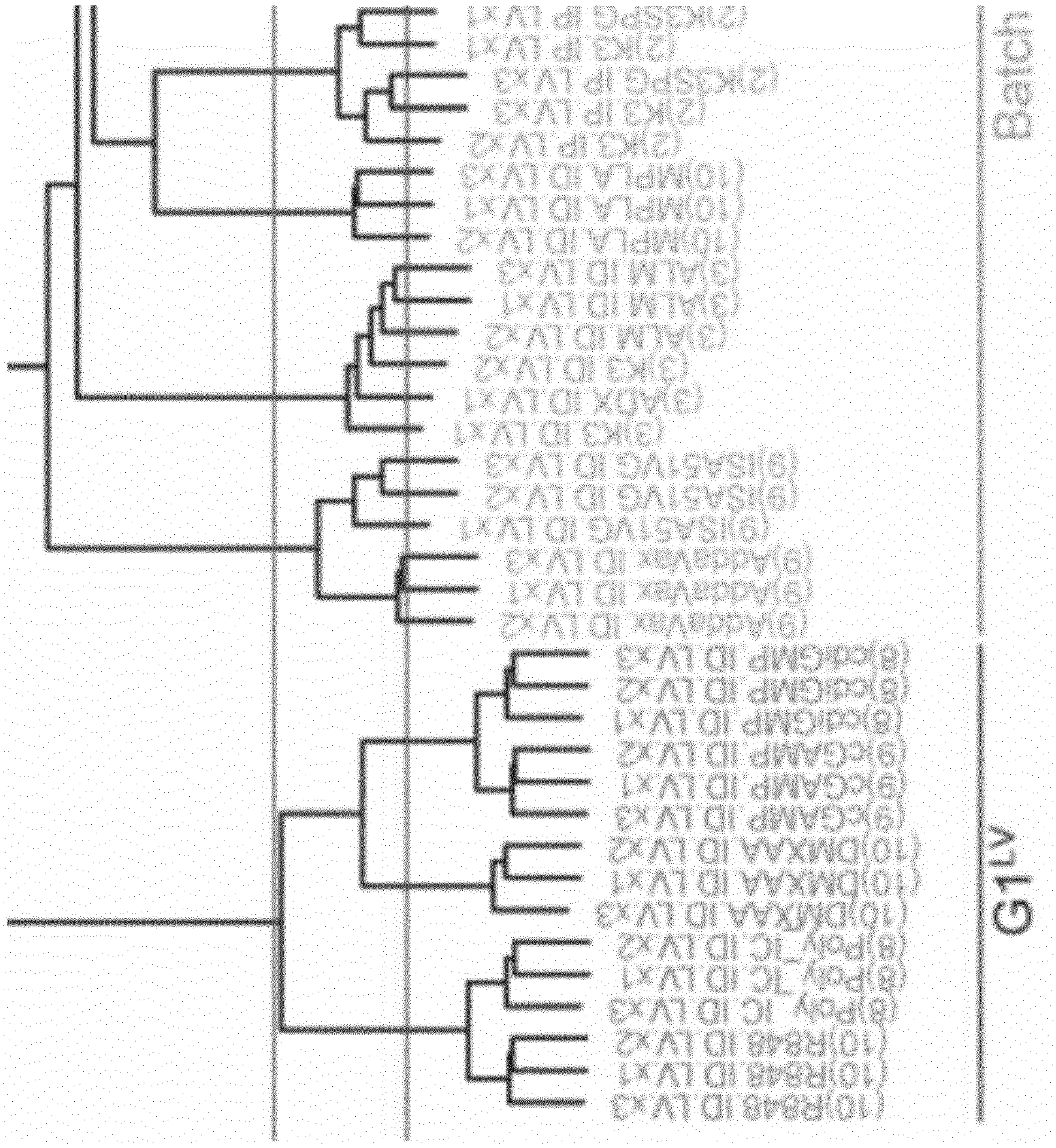
(2)K3SPG IP LVx1
(2)K3 IP LVx1
(2)K3SPG IP LVx3
(2)K3 IP LVx3
(2)K3 IP LVx2
(10)MPLA ID LVx3
(10)MPLA ID LVx1
(10)MPLA ID LVx2
(3)ALM ID LVx3
(3)ALM ID LVx1
(3)ALM ID LVx2
(3)K3 ID LVx2
(3)ADX ID LVx1
(3)K3 ID LVx1
(9)ISA51VG ID LVx3
(9)ISA51VG ID LVx2
(9)ISA51VG ID LVx1
(9)AddaVax ID LVx3
(9)AddaVax ID LVx1
(9)AddaVax ID LVx2
(8)cdiGMP ID LVx3
(8)cdiGMP ID LVx2
(8)cdiGMP ID LVx1
(9)cGAMP ID LVx2
(9)cGAMP ID LVx1
(9)cGAMP ID LVx3
(10)DMXAA ID LVx2
(10)DMXAA ID LVx1
(10)DMXAA ID LVx3
(8)Poly_IC ID LVx2
(8)Poly_IC ID LVx1
(8)Poly_IC ID LVx3
(10)R848 ID LVx2
(10)R848 ID LVx1
(10)R848 ID LVx3
Batch
G1LV

[FIG.14E]

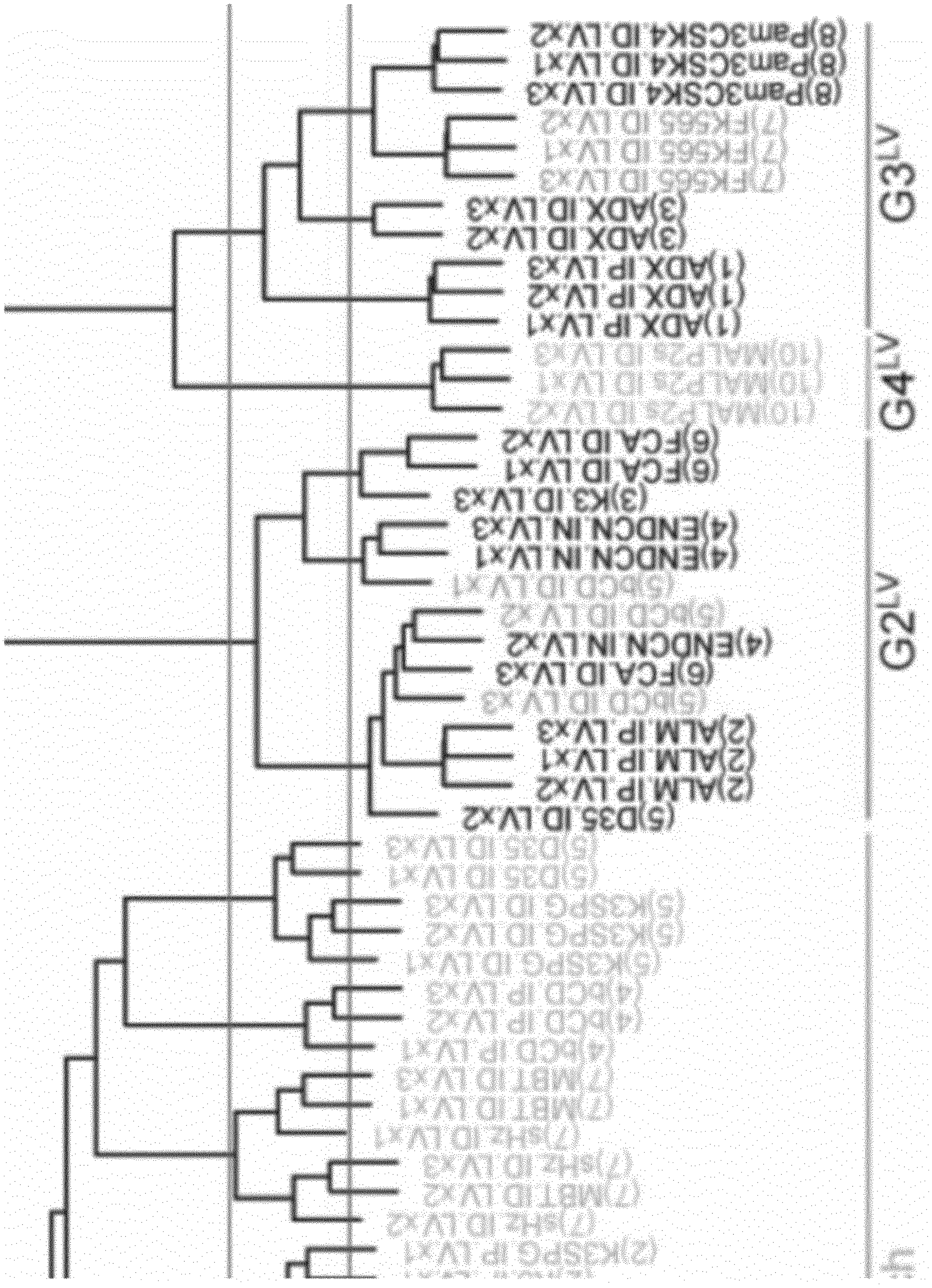
(8)Pam3CSK4.ID.LV.x2
(8)Pam3CSK4.ID.LV.x1
(8)Pam3CSK4.ID.LV.x3
(7)FK565.ID.LV.x2
(7)FK565.ID.LV.x1
(7)FK565.ID.LV.x3
(3)ADX.ID.LV.x3
(3)ADX.ID.LV.x2
(1)ADX.IP.LV.x3
(1)ADX.IP.LV.x2
(1)ADX.IP.LV.x1
(10)MALP2s.ID.LV.x3
(10)MALP2s.ID.LV.x1
(10)MALP2s.ID.LV.x2
(6)FCA.ID.LV.x2
(6)FCA.ID.LV.x1
(3)K3.ID.LV.x3
(4)ENDCN.IN.LV.x3
(4)ENDCN.IN.LV.x1
(5)bCD.ID.LV.x1
(5)bCD.ID.LV.x2
(4)ENDCN.IN.LV.x2
(6)FCA.ID.LV.x3
(5)bCD.ID.LV.x3
(2)ALM.IP.LV.x3
(2)ALM.IP.LV.x1
(2)ALM.IP.LV.x2
(5)D35.ID.LV.x2
(5)D35.ID.LV.x3
(5)D35.ID.LV.x1
(5)K3SPG.ID.LV.x3
(5)K3SPG.ID.LV.x2
(5)K3SPG.ID.LV.x1
(4)bCD.IP.LV.x3
(4)bCD.IP.LV.x2
(4)bCD.IP.LV.x1
(7)MBT.ID.LV.x3
(7)MBT.ID.LV.x1
(7)sHz.ID.LV.x1
(7)sHz.ID.LV.x3
(7)MBT.ID.LV.x2
(7)sHz.ID.LV.x2
(2)K3SPG.IP.LV.x1
G3LV
G4LV
G2LV

[FIG.14F]

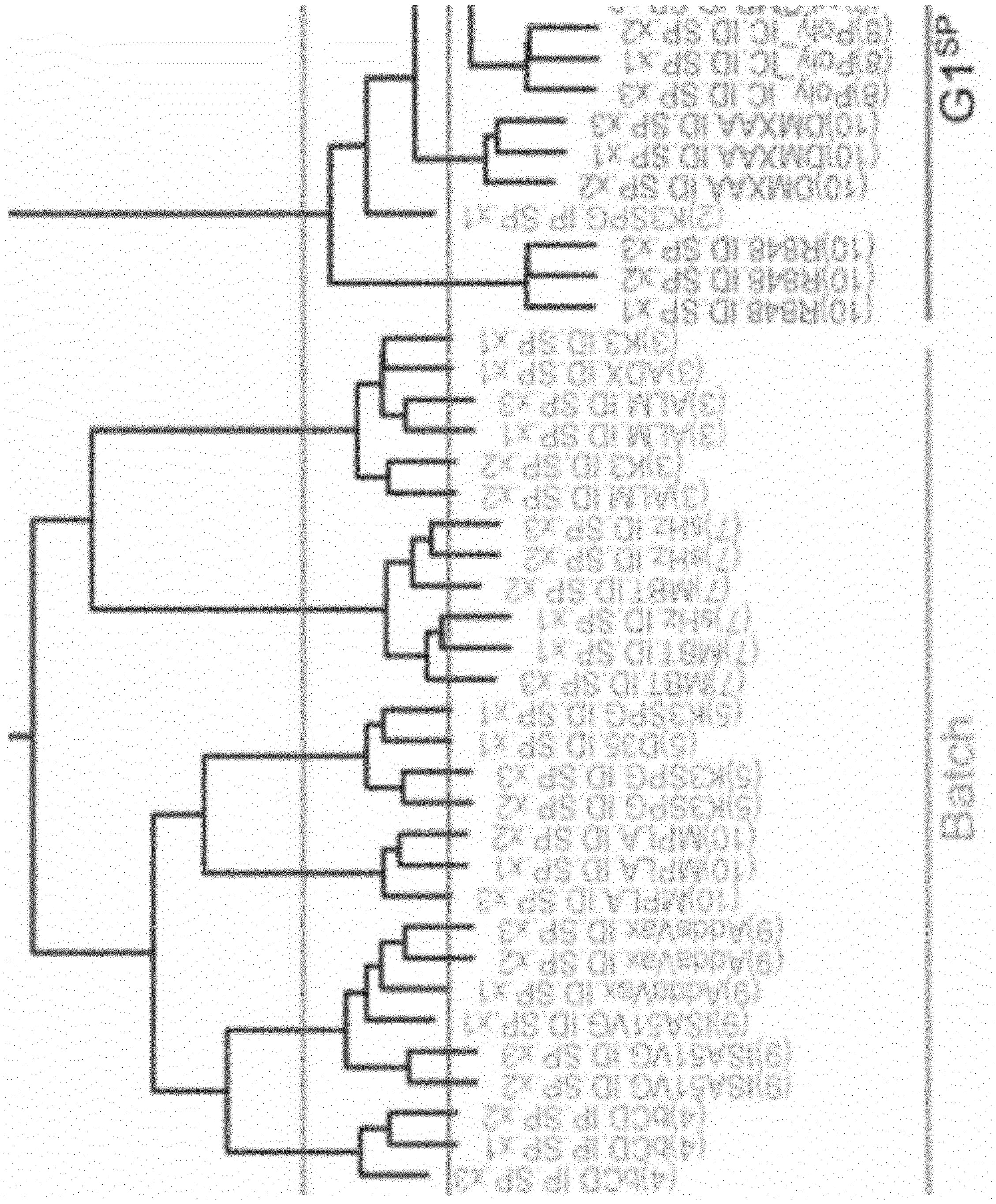
(8)Poly_IC ID SP x2
(8)Poly_IC ID SP x1
(8)Poly_IC ID SP x3
(10)DMXAA ID SP x3
(10)DMXAA ID SP x1
(10)DMXAA ID SP x2
(2)K3SPG IP SP x1
(10)R848 ID SP x3
(10)R848 ID SP x2
(10)R848 ID SP x1
(3)K3 ID SP x1
(3)ADX ID SP x1
(3)ALM ID SP x3
(3)ALM ID SP x1
(3)K3 ID SP x2
(3)ALM ID SP x2
(7)sHz ID SP x3
(7)sHz ID SP x2
(7)MBT ID SP x2
(7)sHz ID SP x1
(7)MBT ID SP x1
(7)MBT ID SP x3
(5)K3SPG ID SP x1
(5)D35 ID SP x1
(5)K3SPG ID SP x3
(5)K3SPG ID SP x2
(10)MPLA ID SP x2
(10)MPLA ID SP x1
(10)MPLA ID SP x3
(9)AddaVax ID SP x3
(9)AddaVax ID SP x2
(9)AddaVax ID SP x1
(6)ISA51VG ID SP x1
(6)ISA51VG ID SP x3
(6)ISA51VG ID SP x2
(4)bCD IP SP x2
(4)bCD IP SP x1
(4)bCD IP SP x3
G1SP
Batch

[FIG.14G]

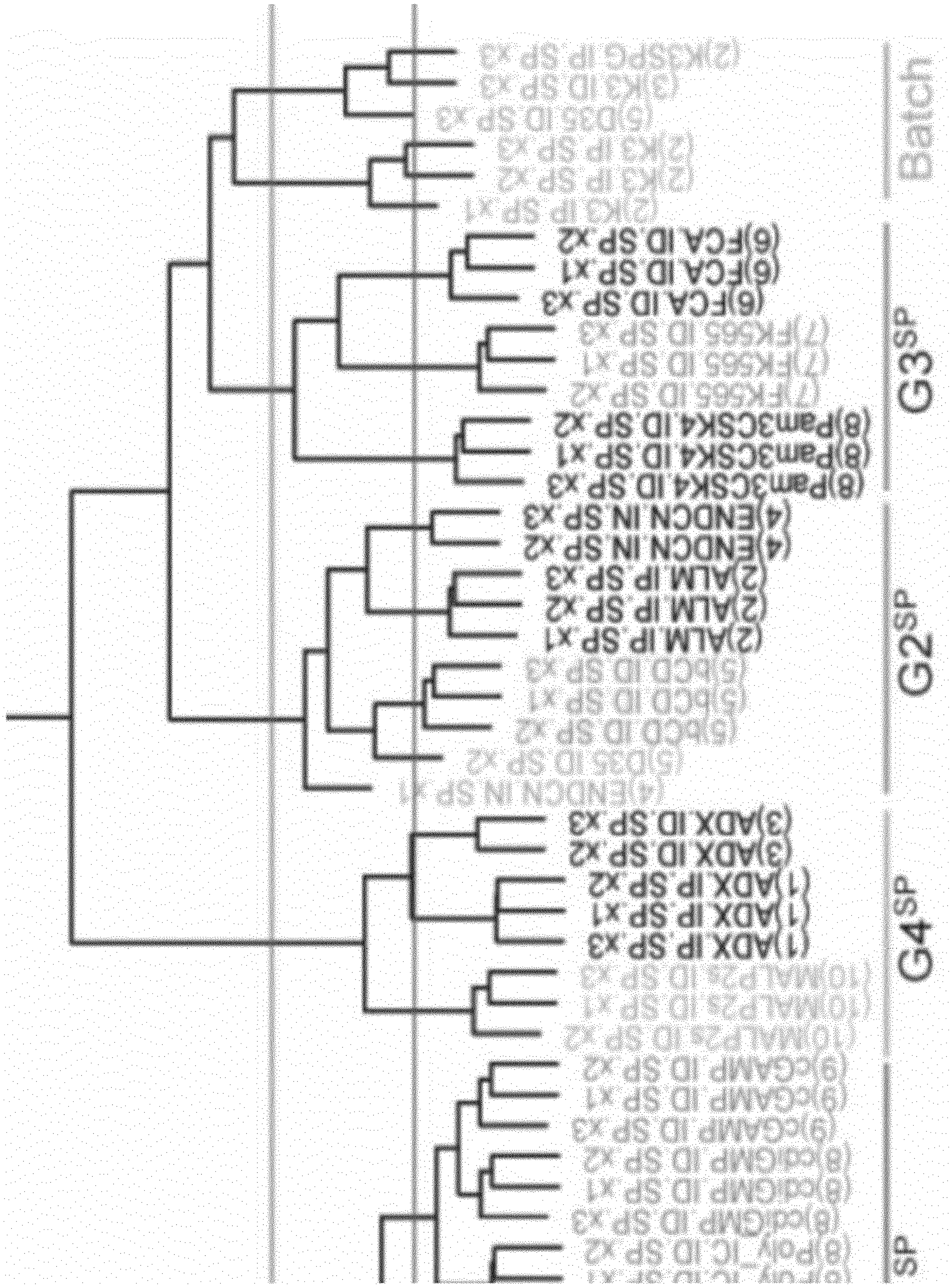
Batch
G3SP
G2SP
G4SP
SP
(6)FCA.ID.SP.x2
(6)FCA.ID.SP.x1
(6)FCA.ID.SP.x3
(8)Pam3CSK4.ID.SP.x2
(8)Pam3CSK4.ID.SP.x1
(8)Pam3CSK4.ID.SP.x3
(4)ENDCN.IN.SP.x3
(4)ENDCN.IN.SP.x2
(2)ALM.IP.SP.x3
(2)ALM.IP.SP.x2
(2)ALM.IP.SP.x1
(3)ADX.ID.SP.x3
(3)ADX.ID.SP.x2
(1)ADX.IP.SP.x2
(1)ADX.IP.SP.x1
(1)ADX.IP.SP.x3

[FIG.14H]

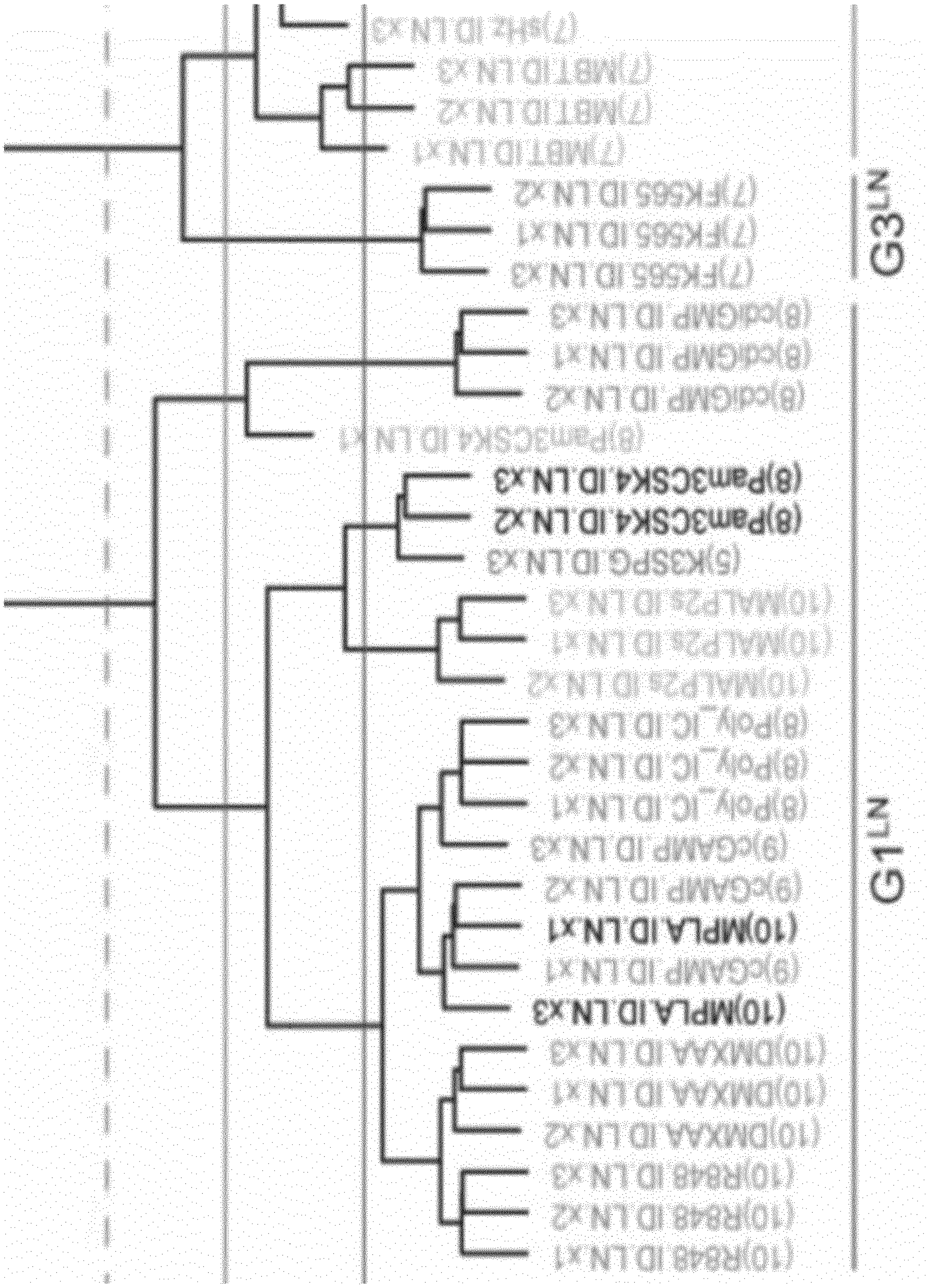
(7)MBT.ID.LN.x3
(7)MBT.ID.LN.x2
(7)MBT.ID.LN.x1
(7)FK565.ID.LN.x2
(7)FK565.ID.LN.x1
(7)FK565.ID.LN.x3
(8)cdiGMP.ID.LN.x3
(8)cdiGMP.ID.LN.x1
(8)cdiGMP.ID.LN.x2
(8)Pam3CSK4.ID.LN.x1
(8)Pam3CSK4.ID.LN.x3
(8)Pam3CSK4.ID.LN.x2
(5)K3SPG.ID.LN.x3
(10)MALP2s.ID.LN.x3
(10)MALP2s.ID.LN.x1
(10)MALP2s.ID.LN.x2
(8)Poly_IC.ID.LN.x3
(8)Poly_IC.ID.LN.x2
(8)Poly_IC.ID.LN.x1
(9)cGAMP.ID.LN.x3
(9)cGAMP.ID.LN.x2
(10)MPLA.ID.LN.x1
(9)cGAMP.ID.LN.x1
(10)MPLA.ID.LN.x3
(10)DMXAA.ID.LN.x3
(10)DMXAA.ID.LN.x1
(10)DMXAA.ID.LN.x2
(10)R848.ID.LN.x3
(10)R848.ID.LN.x2
(10)R848.ID.LN.x1
G3LN
G1LN

[FIG.14I]

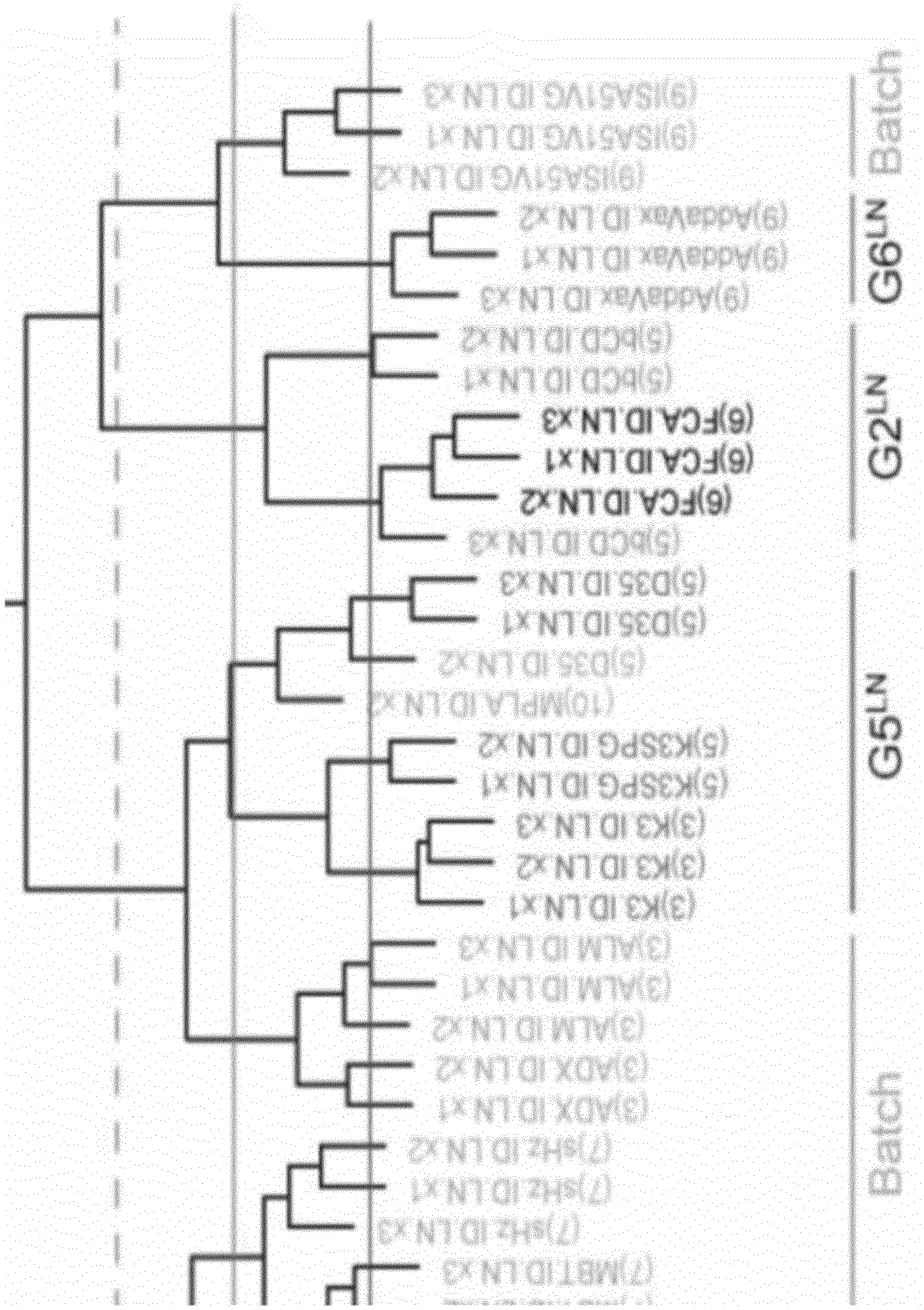
(9)ISA51VG ID LN x3
(9)ISA51VG ID LN x1
(9)ISA51VG ID LN x2
(9)AddaVax ID LN x2
(9)AddaVax ID LN x1
(9)AddaVax ID LN x3
(5)bCD ID LN x2
(5)bCD ID LN x1
(9)FCA ID LN x3
(9)FCA ID LN x1
(9)FCA ID LN x2
(5)bCD ID LN x3
(5)D35 ID LN x3
(5)D35 ID LN x1
(5)D35 ID LN x2
(10)MPLA ID LN x2
(5)K3SPG ID LN x2
(5)K3SPG ID LN x1
(3)K3 ID LN x3
(3)K3 ID LN x2
(3)K3 ID LN x1
(3)ALM ID LN x3
(3)ALM ID LN x1
(3)ALM ID LN x2
(3)ADX ID LN x2
(3)ADX ID LN x1
(7)sHz ID LN x2
(7)sHz ID LN x1
(7)sHz ID LN x3
(7)MBT ID LN x3
Batch
G6LN
G2LN
G5LN
Batch

[FIG.15A]

a

| Probe Index | Gene | Module# |
|---|---|---|
| 1449025_at | Ifit3 | 20 |
| 1450783_at | Ifit1 | 20 |
| 1431591_s_at | Gm9706// | 20 |
| 1436058_at | Rsad2 | 20 |
| 1437432_a_at | Trim12a | 20 |
| 1450033_a_at | Stat1 | 20 |
| 1418825_at | Irgm1 | 20 |
| 1450034_at | Stat1 | 20 |
| 1455500_at | Rnf213 | 20 |
| 1426276_at | Ifih1 | 20 |
| 1449326_x_at | Saa2 | 36 |
| 1425745_a_at | Tacc2 | 37 |
| 1419075_s_at | Saa1 | 36 |
| 1450788_at | Saa1 | 36 |
| 1448950_at | Il1r1 | 37 |
| 1441396_at | B3galt1 | 37 |
| 1439615_at | Gan | 37 |
| 1429185_at | Arhgef26 | 37 |
| 1434845_at | --- | 37 |
| 1457374_at | Nedd4l | 37 |
| 1420330_at | Clec4e | 31 |
| 1424542_at | S100a4 | 31 |
| 1420331_at | Clec4e | 31 |
| 1420394_s_at | Gp49a///l | 27 |
| 1417523_at | Plek | 27 |
| 1448749_at | Plek | 27 |
| 1448748_at | Plek | 27 |
| 1460271_at | Trem3 | 40 |
| 1456046_at | Cd93 | 26 |
| 1452233_at | Abcc1 | 40 |
| 1434719_at | A2m | 35 |
| 1451204_at | Scara5 | 35 |
| 1419005_at | Crybb3 | 35 |
| 1435936_at | Slc13a5 | 35 |
| 1441975_at | Acpp | 35 |
| 1434092_at | Atg9b | 39 |
| 1423072_at | 6720475J1 | 39 |
| 1428261_at | Ccdc134 | 39 |
| 1455721_at | Gspt2 | 35 |
| 1424529_s_at | Cgref1 | 39 |

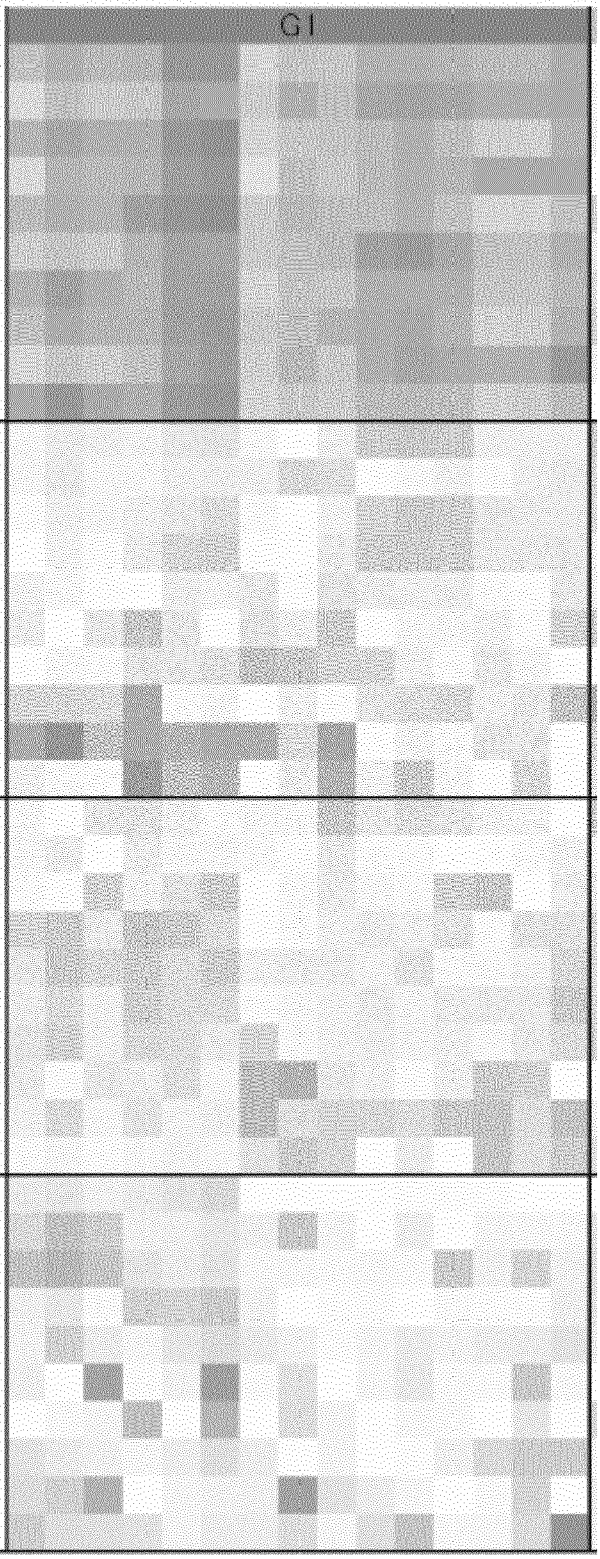

[FIG.15A](**Continue**)

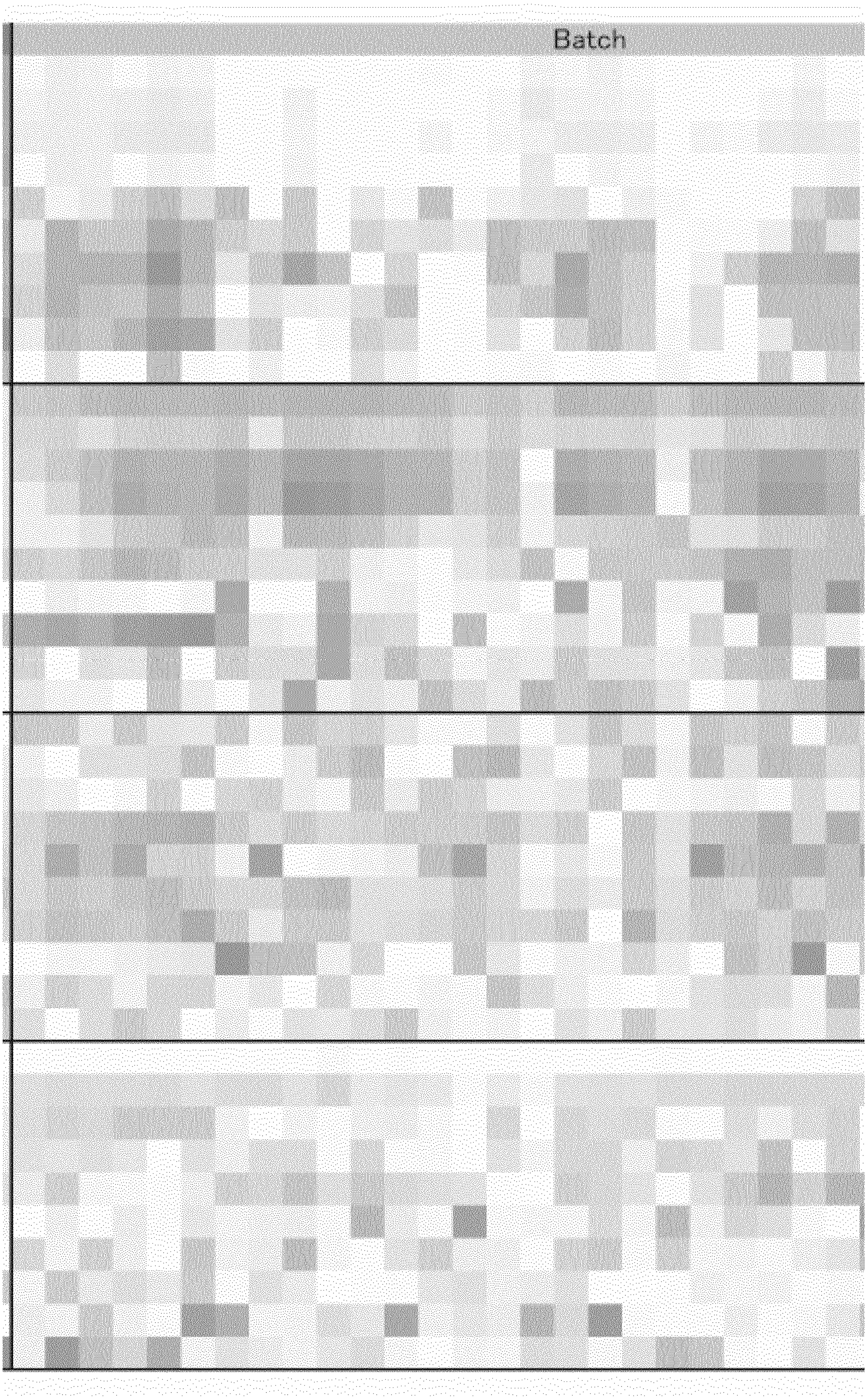

[FIG.15A](**Continue**)

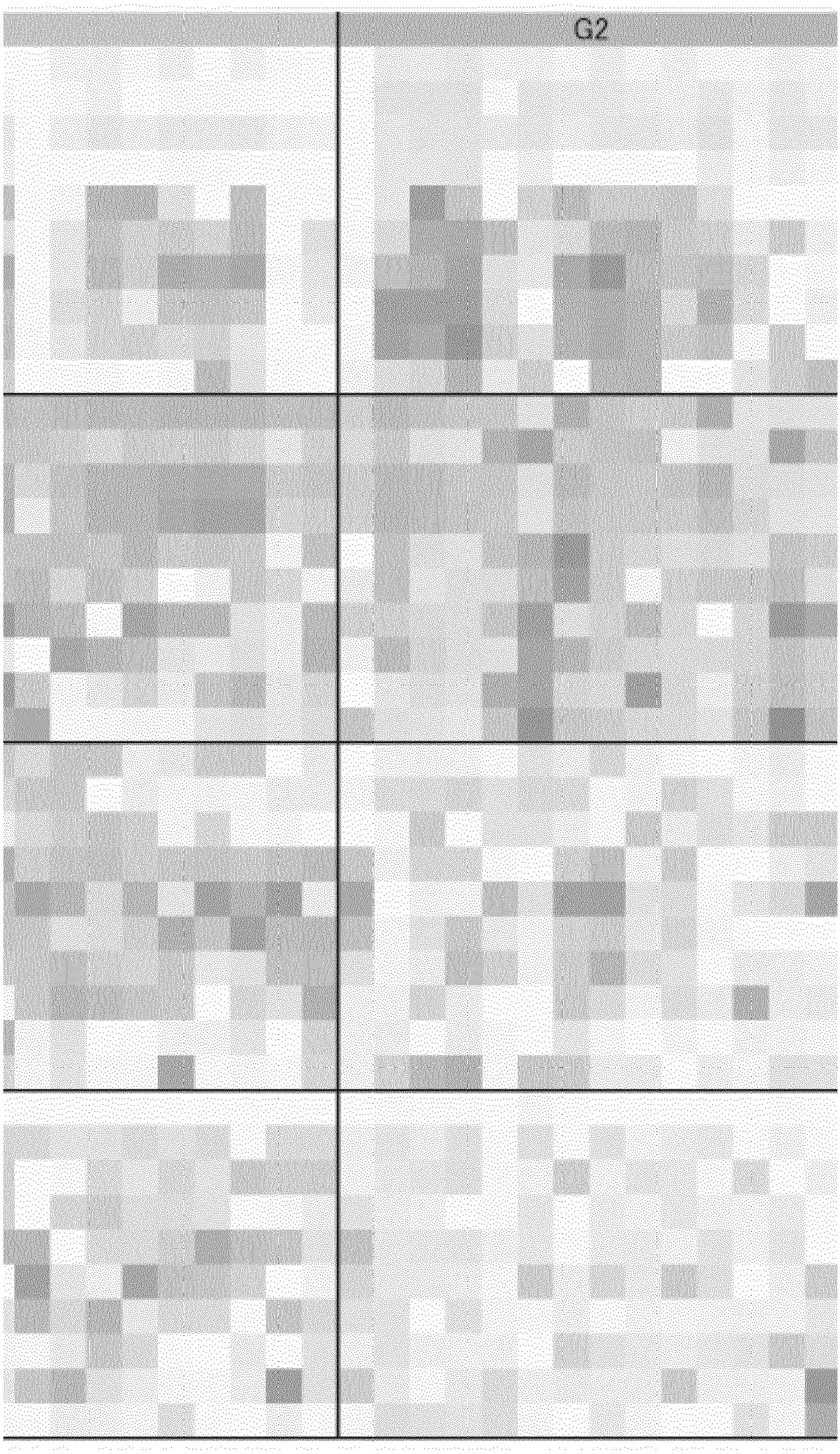

[FIG.15A](**Continue**)

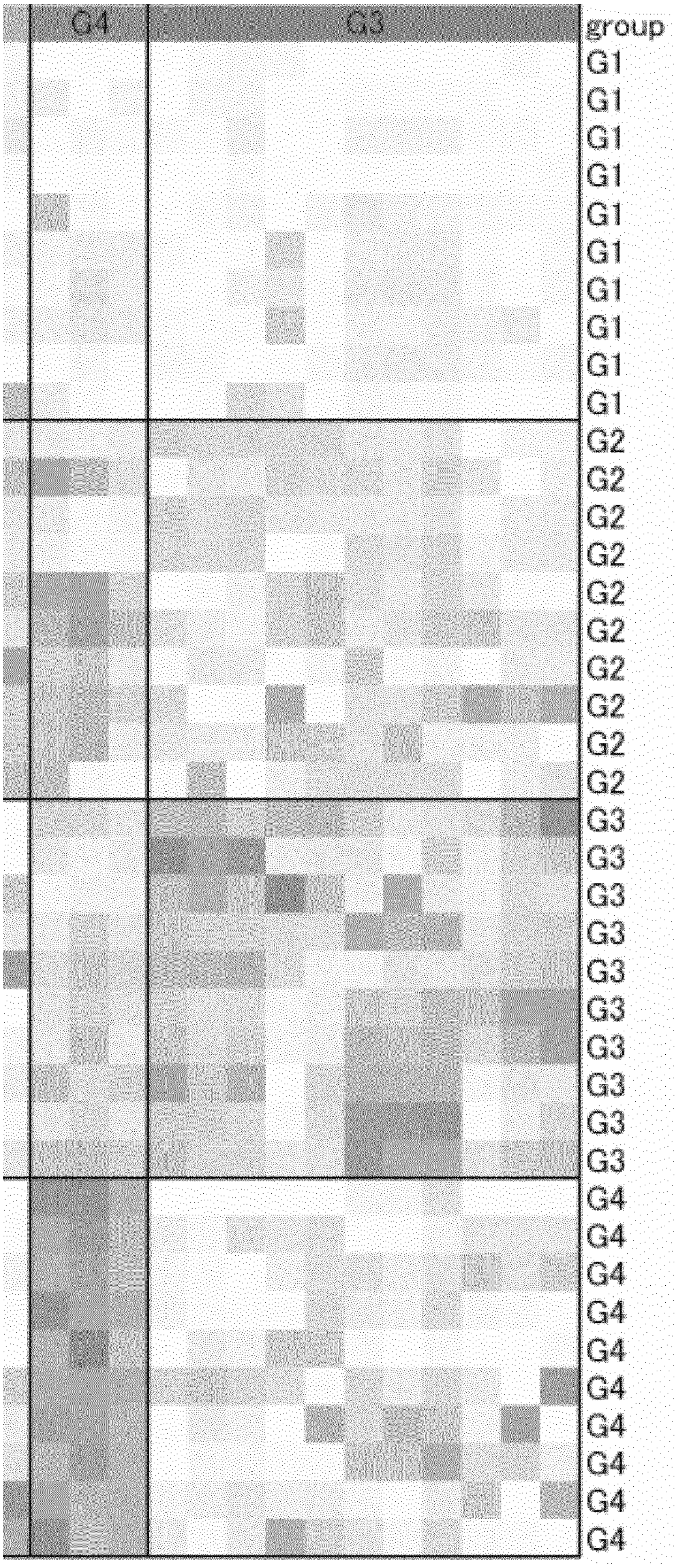

[FIG.15B]

b

| Probe Index | Gene | Module# |
|---|---|---|
| 1427102_at | Slfn4 | 23 |
| 1425065_at | Oas2 | 23 |
| 1424775_at | Oas1a | 23 |
| 1437636_at | Pydc4 | 23 |
| 1417876_at | Fcgr1 | 23 |
| 1455500_at | Rnf213 | 23 |
| 1422160_at | H2-T24 | 23 |
| 1451567_a_at | Gm16340/ | 23 |
| 1438027_at | --- | 23 |
| 1417172_at | Ube2l6 | 23 |
| 1440921_at | Nlrp12 | 39 |
| 1419610_at | Ccr1 | 39 |
| 1420764_at | Scrg1 | 39 |
| 1434049_at | Entpd3 | 39 |
| 1421734_at | Cxcr2 | 39 |
| 1452922_at | Ppp1r3d | 39 |
| 1425503_at | Gcnt2 | 39 |
| 1456893_at | --- | 38 |
| 1421917_at | Pdgfra | 39 |
| 1456295_at | B230114P | 38 |
| 1439902_at | C5ar1 | 36 |
| 1419647_a_at | Ier3 | 39 |
| 1442339_at | Stfa2l1 | 39 |
| 1423233_at | Cebpd | 39 |
| 1434848_at | --- | 39 |
| 1453678_at | Mbd1 | 36 |
| 1447284_at | Trem1 | 36 |
| 1425951_a_at | Clec4n | 38 |
| 1449991_at | Cd244 | 39 |
| 1437247_at | Fosl2 | 39 |
| 1449984_at | Cxcl2 | 32 |
| 1419561_at | Ccl3 | 32 |
| 1417925_at | Ccl22 | 36 |
| 1418449_at | Lad1 | 32 |
| 1418992_at | F10 | 32 |
| 1420413_at | Slc7a11 | 32 |
| 1431577_at | 5430437J1 | 36 |
| 1416022_at | Fabp5 | 36 |
| 1448656_at | Cacnb3 | 36 |
| 1416021_a_at | Fabp5///( | 36 |

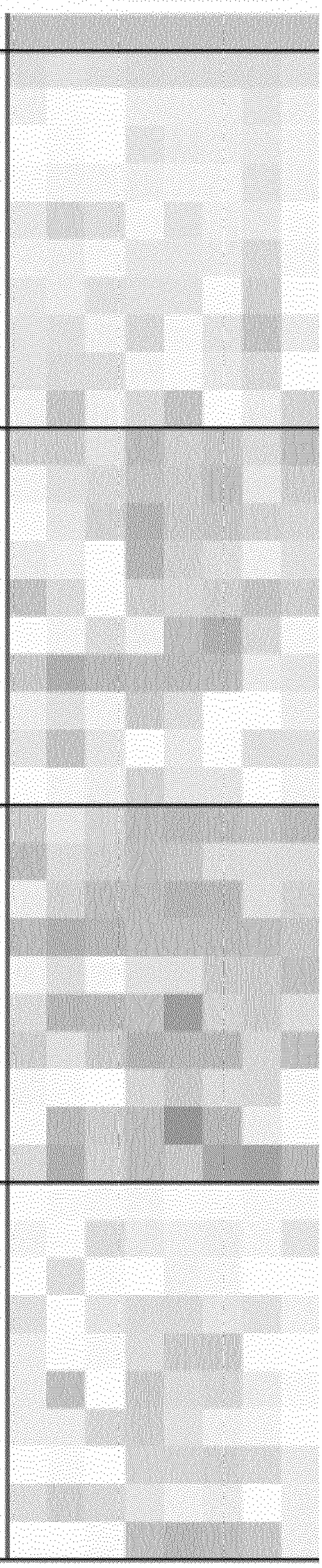

[FIG.15B](**Continue**)

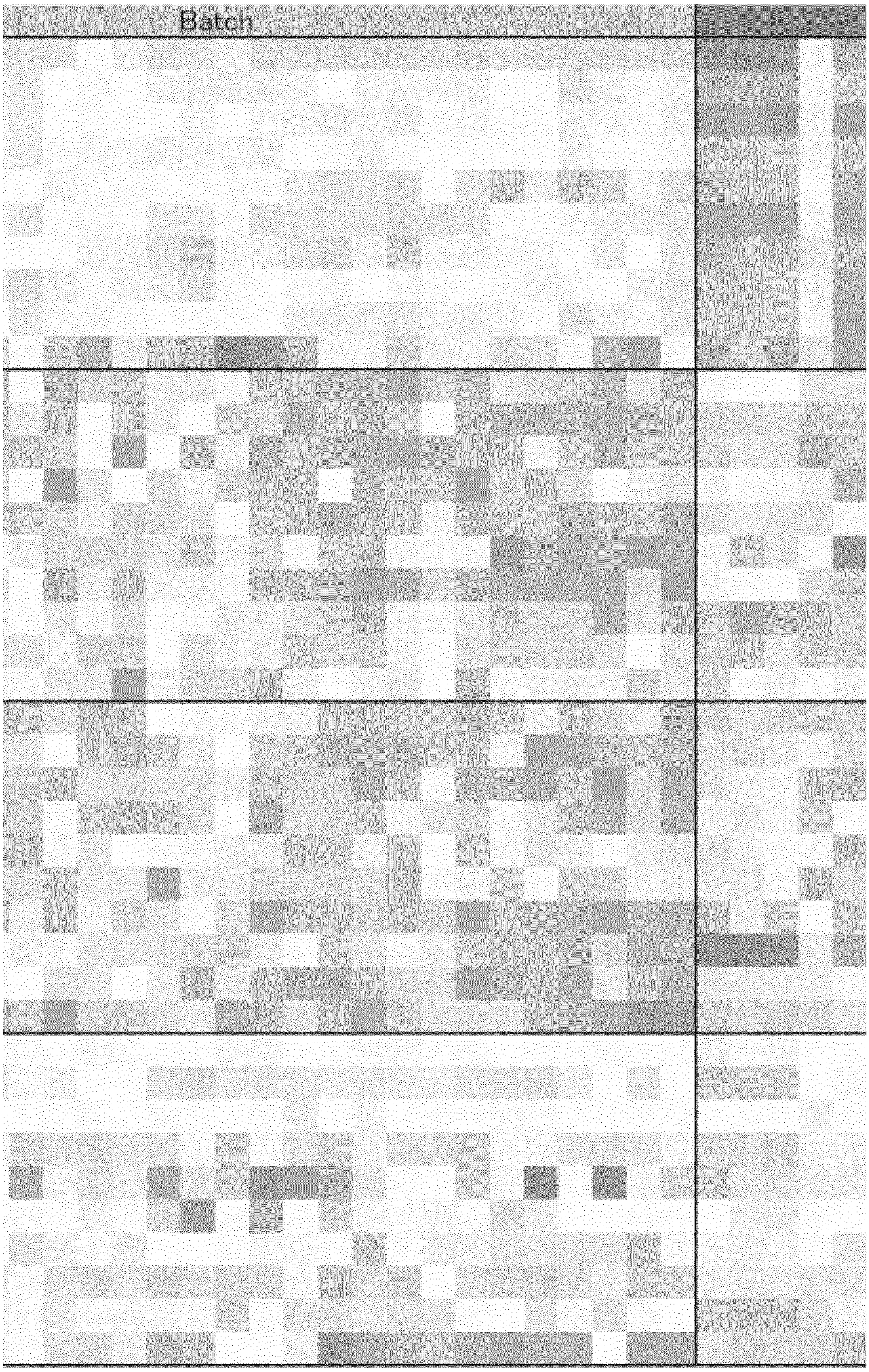

[FIG.15B](**Continue**)

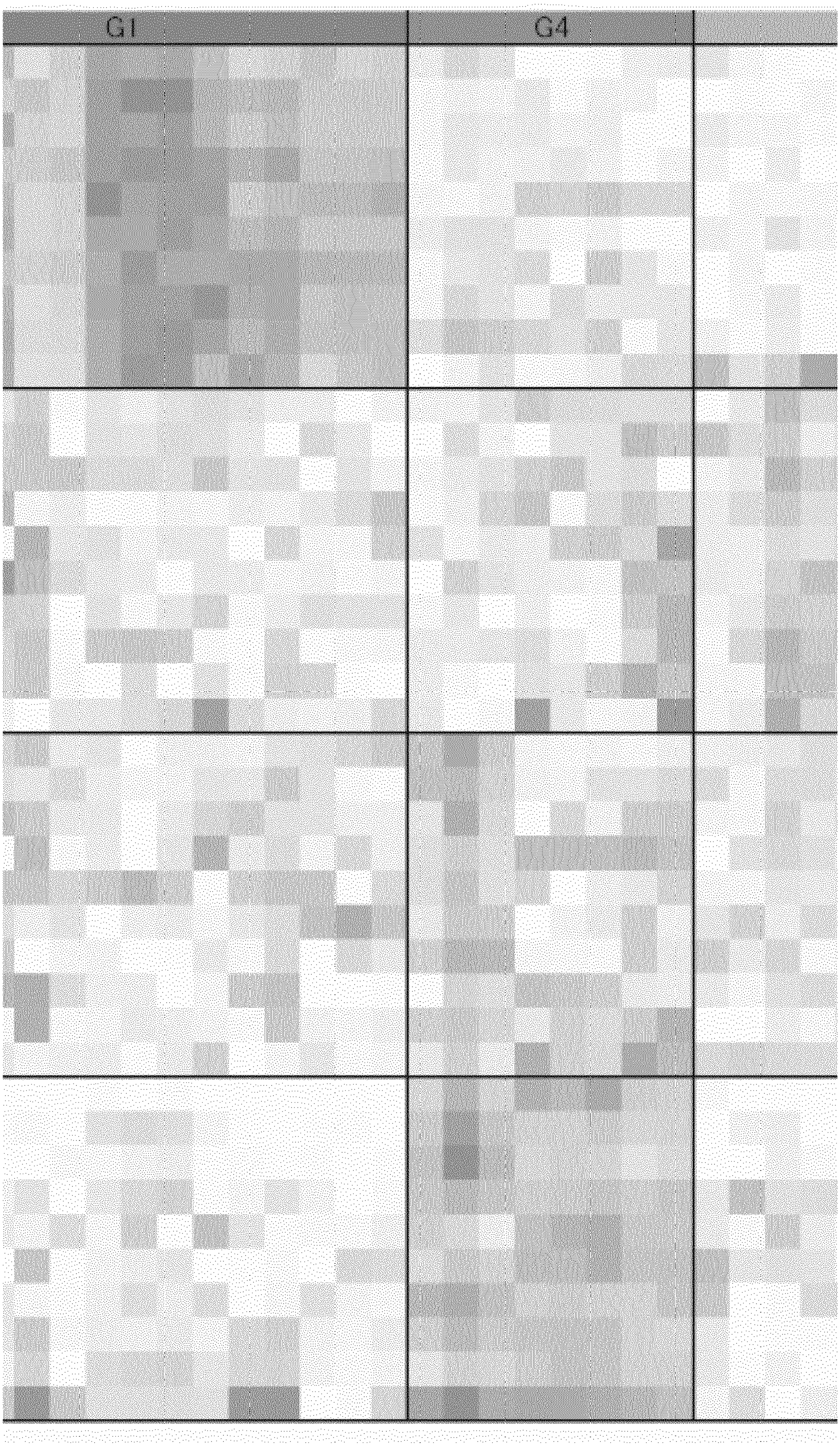

[FIG.15B](**Continue**)

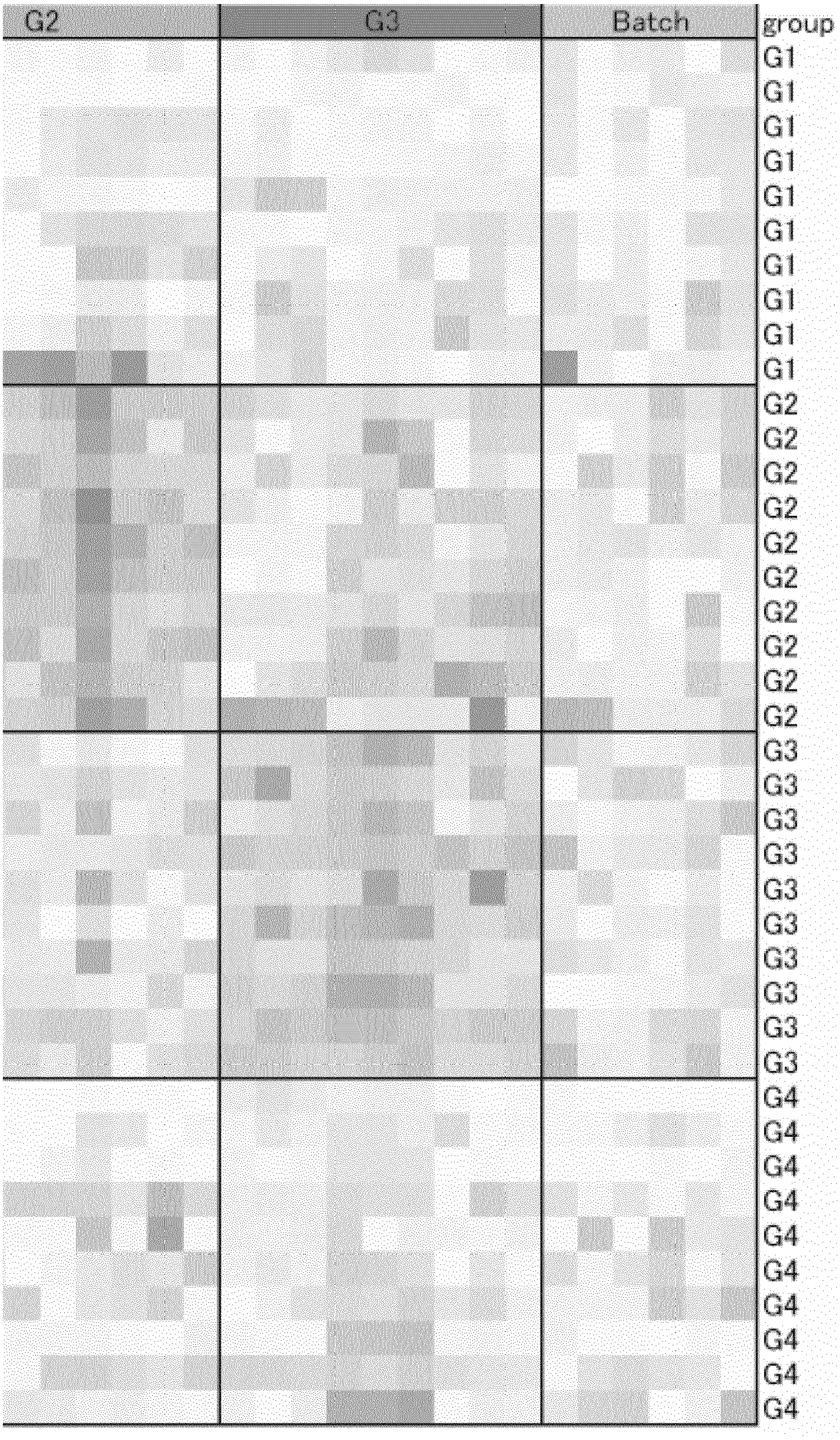

[FIG.15C]

C

| Probe Index | Gene | Module# |
|---|---|---|
| 1424775_at | Oas1a | 40 |
| 1435529_at | Gm14446 | 40 |
| 1438868_at | Phf11d | 40 |
| 1443698_at | Xaf1 | 40 |
| 1419026_at | Daxx | 40 |
| 1451655_at | Slfn8 | 40 |
| 1451050_at | Nt5c3 | 40 |
| 1448757_at | Pml | 40 |
| 1459151_x_at | Ifi35 | 40 |
| 1421304_at | Klra2 | 29 |
| 1420697_at | Slc15a3 | 40 |
| 1449455_at | Hck | 40 |
| 1444399_at | Nabp1 | 38 |
| 1455263_at | Lacc1 | 40 |
| 1460661_at | S1pr3 | 40 |
| 1422511_a_at | Ogfr | 40 |
| 1437516_at | ---- | 40 |
| 1416593_at | Glrx | 40 |
| 1441287_at | Mbtd1 | 40 |
| 1436100_at | Sh2d5 | 28 |
| 1419537_at | Tfec | 28 |
| 1417403_at | Elovl6 | 28 |
| 1448550_at | Lbp | 28 |
| 1444992_at | AI120166 | 6 |
| 1422823_at | Eps8 | 28 |
| 1416286_at | Rgs4 | 8 |
| 1447502_at | ---- | 14 |
| 1416287_at | Rgs4 | 8 |
| 1416253_at | Cdkn2d | 28 |

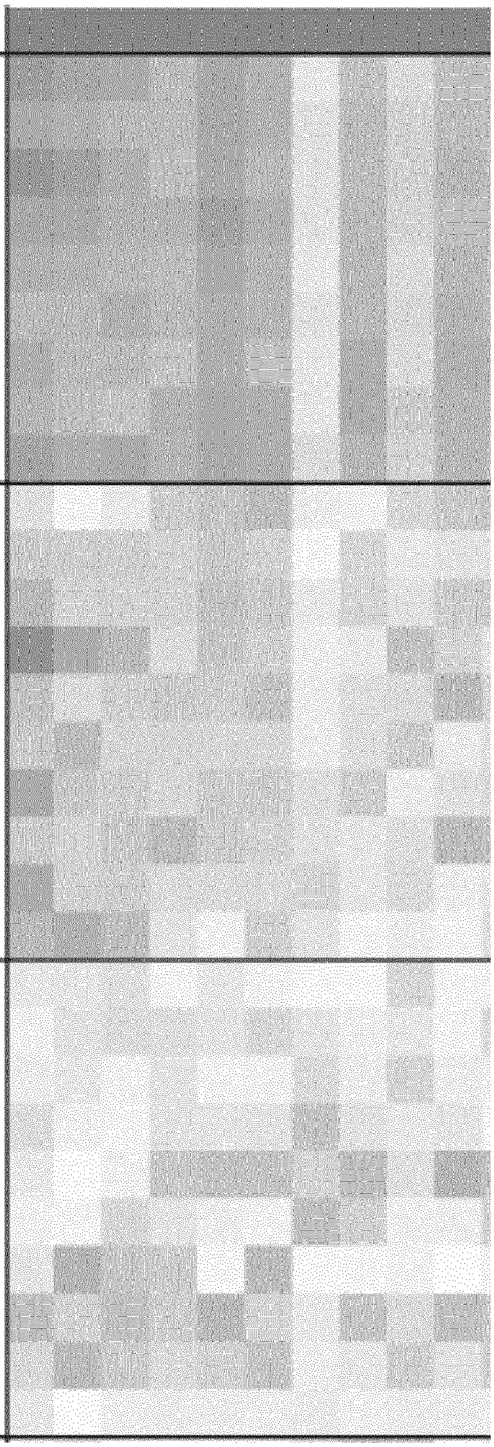

[FIG.15C](**Continue**)

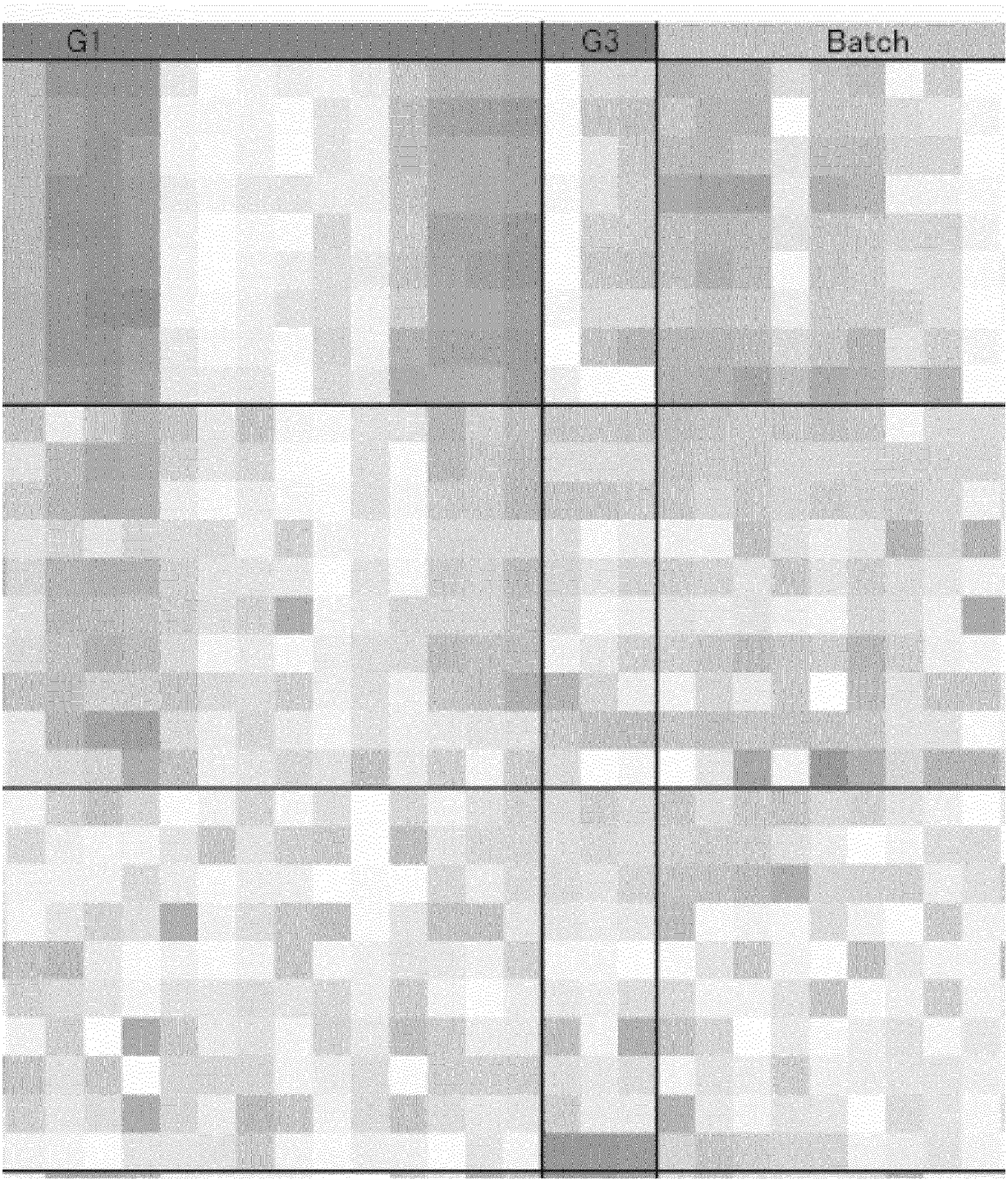

[FIG.15C](**Continue**)

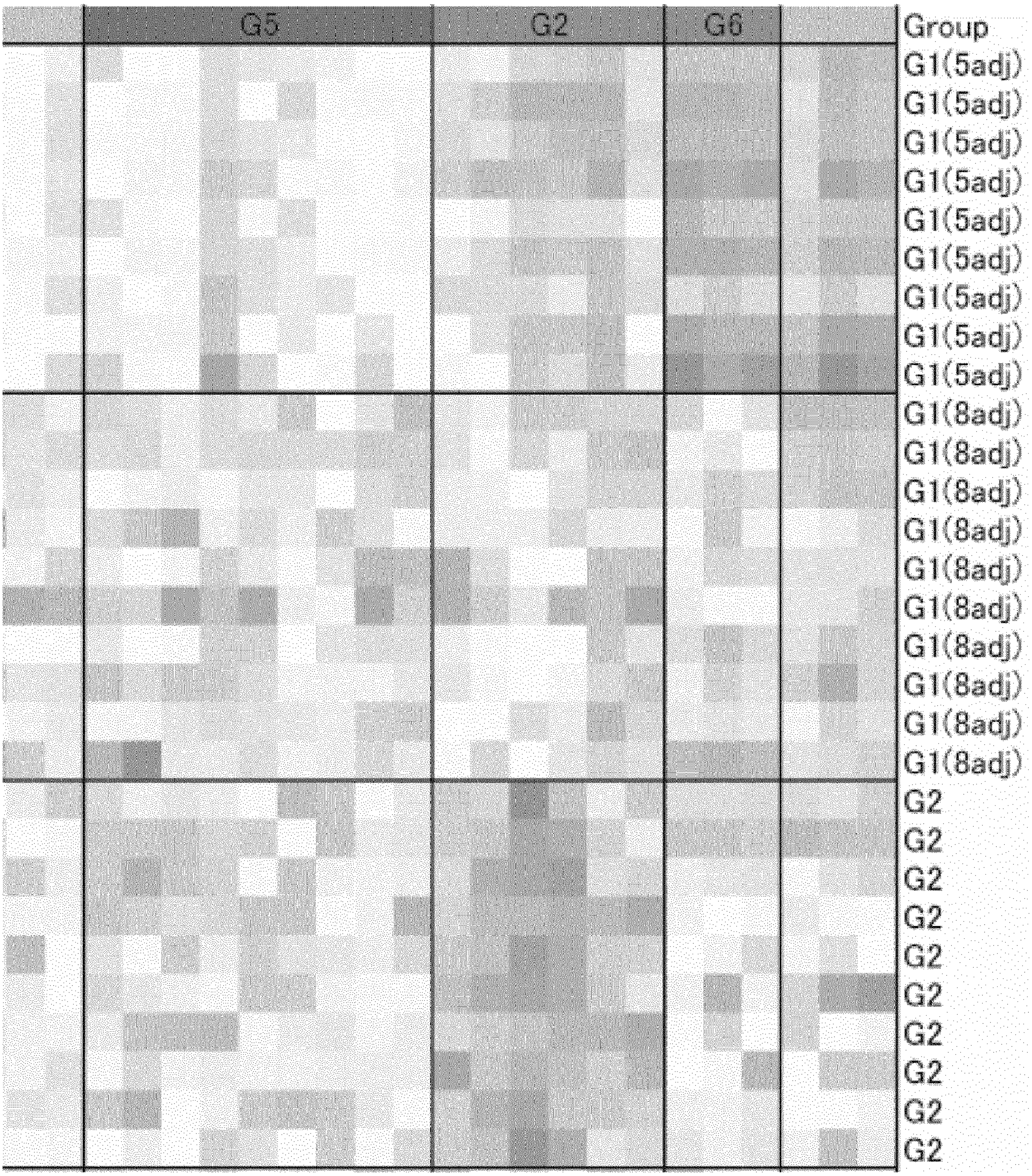

[FIG.15C](**Continue**)

| | | |
|---|---|---|
| 1433837_at | 8430408G: | 13 |
| 1434202_a_at | Fam107a | 13 |
| 1434203_at | Fam107a | 13 |
| 1442025_a_at | --- | 13 |
| 1448231_at | Fkbp5 | 5 |
| 1420582_at | Cd209e | 6 |
| 1453008_at | Trnp1 | 13 |
| 1439163_at | Zbtb16 | 13 |
| 1417273_at | Pdk4 | 5 |
| 1419874_x_at | Zbtb16 | 13 |
| 1434685_at | D3Bwg056 | 16 |
| 1436280_at | Zscan20 | 17 |
| 1456799_at | E130110O | 8 |
| 1446307_at | --- | 12 |
| 1447895_x_at | Pan3 | 20 |
| 1438592_at | Nek1 | 7 |
| 1440862_at | --- | 11 |
| 1444375_at | --- | 14 |
| 1427446_s_at | Ttn | 14 |
| 1439233_at | --- | 7 |
| 1444176_at | Atp6v0d2 | 21 |
| 1449305_at | F10 | 28 |
| 1422190_at | C5ar1 | 21 |
| 1449153_at | Mmp12 | 21 |
| 1420699_at | Clec7a | 28 |
| 1420102_at | --- | 32 |
| 1427005_at | Plk2 | 28 |
| 1446150_at | --- | 32 |
| 1427931_s_at | Pdxk | 21 |
| 1433864_at | Lrp12 | 18 |

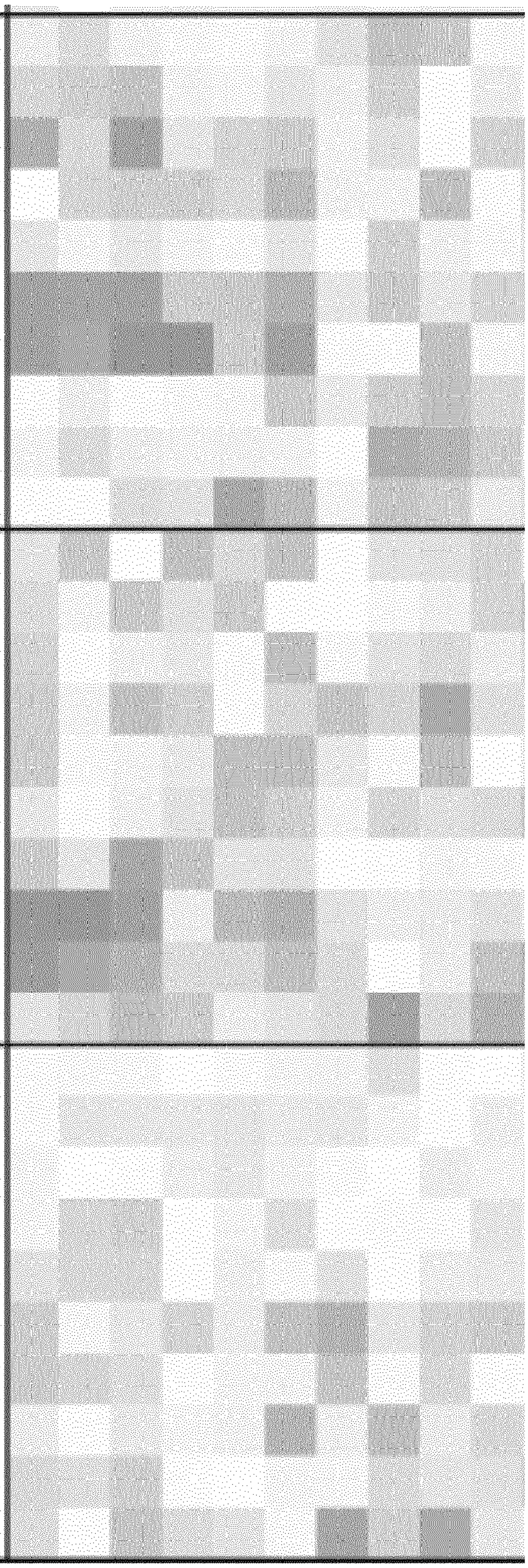

[FIG.15C](**Continue**)

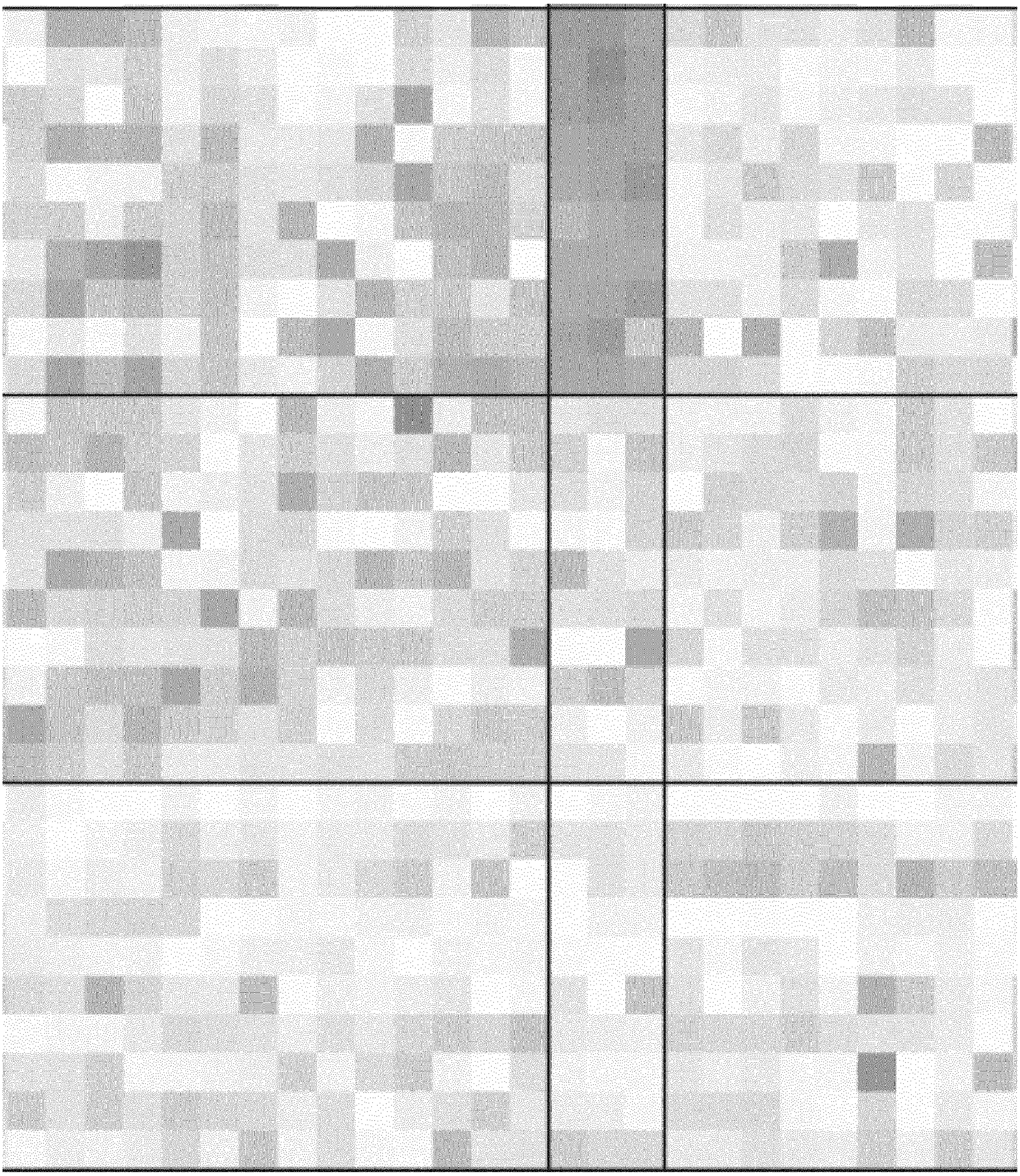

[FIG.15C](**Continue**)

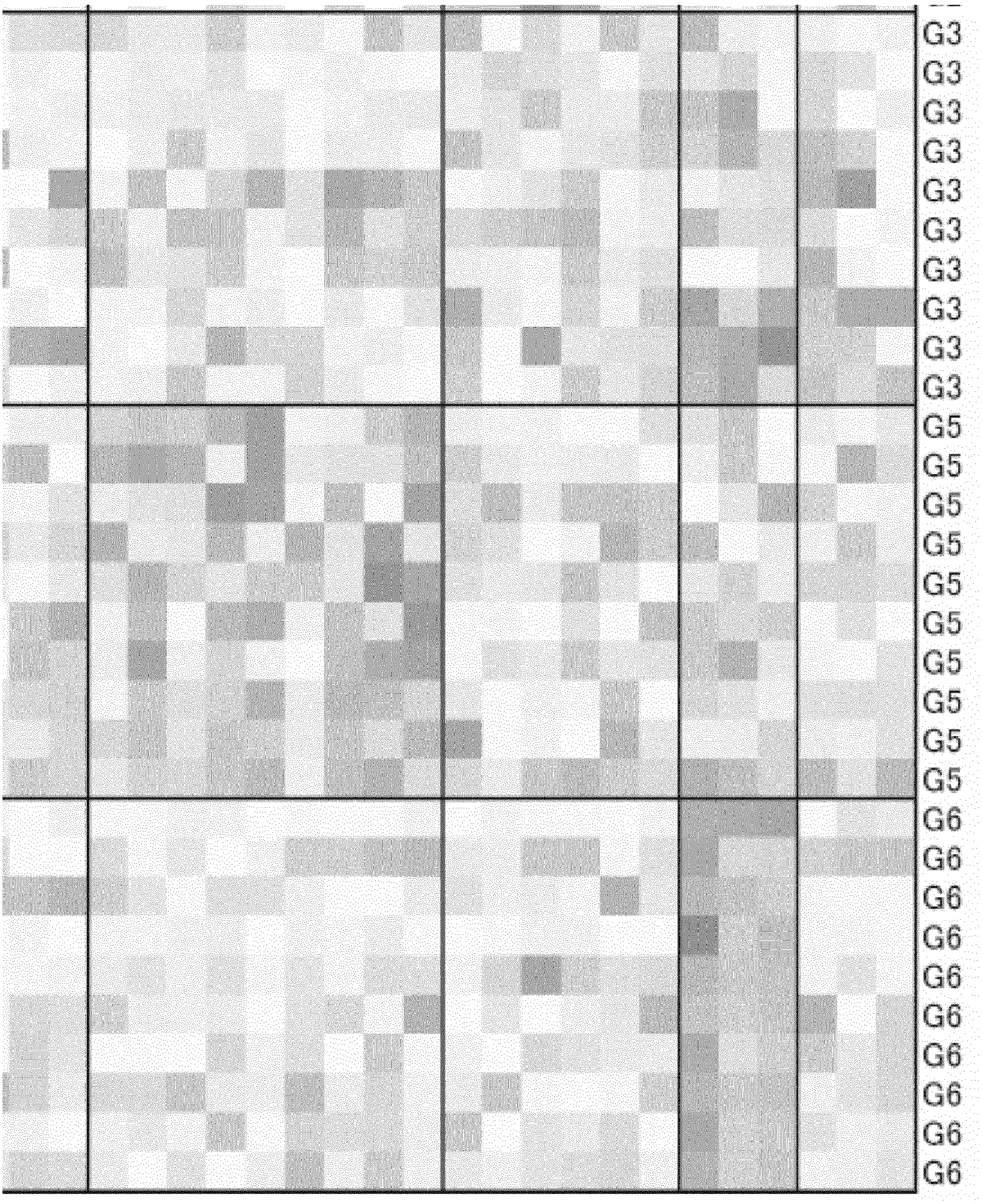

[FIG.16A]

a

| LV | Term | G1 | G2 | G3 | G4* |
|---|---|---|---|---|---|
| 1 | vacuolar transport | 0.0 | 0.0 | 1.9 | 0.2 |
| 2 | DNA metabolic process | 0.0 | 0.0 | 0.0 | 0.0 |
| 3 | biosynthetic process | 0.0 | 0.0 | 0.0 | 0.0 |
| 4 | cofactor metabolic process | 0.0 | 16.2 | 0.0 | 0.4 |
| 5 | cellular nitrogen compound metabolic process | 0.0 | 0.6 | 0.0 | 0.2 |
| 6 | protein oligomerization | 0.0 | 0.0 | 0.0 | 0.1 |
| 7 | Metabolic pathways | 0.0 | 0.0 | 0.0 | 0.1 |
| 8 | small molecule metabolic process | 0.0 | 0.0 | 0.0 | 0.0 |
| 9 | single-organism metabolic process | 0.0 | 0.6 | 0.0 | 0.0 |
| 10 | Hypertrophic cardiomyopathy (HCM) | 0.0 | 0.0 | 0.0 | 0.2 |
| 11 | embryo development | 0.0 | 4.6 | 0.0 | 0.2 |
| 12 | positive regulation of macromolecule metabolic process | 0.0 | 0.0 | 7.7 | 0.0 |
| 13 | biosynthetic process | 0.0 | 0.0 | 0.0 | 0.5 |
| 14 | mTOR signaling pathway | 0.0 | 4.6 | 0.0 | 0.9 |
| 15 | Pancreatic cancer | 0.0 | 0.0 | 9.6 | 0.0 |
| 16 | Cell Cycle | 0.0 | 0.0 | 5.8 | 0.1 |
| 17 | Insulin signaling pathway | 0.0 | 0.0 | 0.0 | 0.3 |
| 18 | Glycerolipid metabolism | 0.0 | 1.7 | 0.0 | 0.1 |
| 19 | small molecule metabolic process | 0.0 | 5.8 | 0.0 | 0.1 |
| 20 | response to biotic stimulus | 95.9 | 0.0 | 0.0 | 1.4 |
| 21 | cell death | 0.0 | 0.6 | 0.0 | 1.9 |
| 22 | nucleobase-containing compound catabolic process | 4.1 | 0.0 | 0.0 | 0.0 |
| 23 | cellular nitrogen compound metabolic process | 0.0 | 0.0 | 0.0 | 0.1 |
| 24 | defense response | 0.0 | 0.6 | 0.0 | 1.9 |
| 25 | Osteoclast differentiation | 0.0 | 0.0 | 19.2 | 1.4 |
| 26 | Cytokine-cytokine receptor interaction\|Endocytosis | 0.0 | 0.0 | 9.6 | 5.3 |
| 27 | immune system process | 0.0 | 0.0 | 21.2 | 2.2 |
| 28 | RNA transport\|Processing of Capped Intron-Containing Pre-mRNA | 0.0 | 0.6 | 0.0 | 0.6 |
| 29 | beta selection | 0.0 | 0.0 | 0.0 | 1.4 |
| 30 | Terpenoid backbone biosynthesis | 0.0 | 0.0 | 1.9 | 0.7 |
| 31 | vesicle-mediated transport | 0.0 | 0.0 | 11.5 | 0.4 |
| 32 | cellular protein modification process | 0.0 | 0.0 | 0.0 | 2.3 |
| 33 | Antigen processing and presentation | 0.0 | 0.0 | 0.0 | 3.6 |
| 34 | Protein processing in endoplasmic reticulum | 0.0 | 0.6 | 0.0 | 14.3 |
| 35 | Circadian rhythm | 0.0 | 1.2 | 0.0 | 5.0 |
| 36 | carbohydrate metabolic process | 0.0 | 15.0 | 1.9 | 4.1 |
| 37 | Sphingolipid metabolism | 0.0 | 15.0 | 0.0 | 2.7 |
| 38 | negative regulation of peroxisome proliferator activated receptor signaling path | 0.0 | 15.6 | 0.0 | 1.9 |
| 39 | Protein processing in endoplasmic reticulum | 0.0 | 4.0 | 0.0 | 24.0 |
| 40 | ribosome biogenesis | 0.0 | 12.7 | 9.6 | 21.3 |

[FIG.16B]

b

| SP | Term | G1 | G2 | G3 | G4 |
|---|---|---|---|---|---|
| 1 | cytoskeleton organization | 0.0 | 0.0 | 0.0 | 0.0 |
| 2 | plasma membrane organization | 0.0 | 0.0 | 0.0 | 0.0 |
| 3 | Circadian rhythm | 0.0 | 1.2 | 0.0 | 3.8 |
| 4 | small molecule metabolic process | 0.0 | 3.5 | 1.9 | 0.0 |
| 5 | blood vessel morphogenesis | 0.0 | 19.8 | 0.0 | 0.0 |
| 6 | sulfur compound metabolic process | 0.0 | 2.3 | 0.0 | 0.0 |
| 7 | ND | 0.0 | 0.0 | 0.0 | 0.0 |
| 8 | Hippo signaling pathway | 0.0 | 0.0 | 0.0 | 0.0 |
| 9 | Intestinal immune network for IgA production | 0.0 | 0.0 | 0.0 | 0.0 |
| 10 | Extracellular matrix organization | 0.0 | 0.0 | 0.0 | 0.0 |
| 11 | ABC transporters | 0.0 | 5.8 | 0.0 | 0.0 |
| 12 | locomotion | 0.0 | 1.2 | 9.3 | 0.0 |
| 13 | small molecule metabolic process | 0.0 | 26.7 | 1.9 | 0.0 |
| 14 | ncRNA metabolic process | 0.0 | 0.0 | 0.0 | 1.9 |
| 15 | ncRNA processing | 0.0 | 0.0 | 0.0 | 0.0 |
| 16 | RNA transport\|Processing of Capped Intron-Containing Pre-mRNA | 0.0 | 0.0 | 0.0 | 0.0 |
| 17 | Spliceosome | 0.0 | 0.0 | 0.0 | 3.8 |
| 18 | RNA transport\|Processing of Capped Intron-Containing Pre-mRNA | 0.0 | 0.0 | 0.0 | 0.0 |
| 19 | tRNA metabolic process | 0.0 | 0.0 | 0.0 | 0.0 |
| 20 | response to virus | 3.2 | 0.0 | 0.0 | 0.0 |
| 21 | generation of precursor metabolites and energy | 0.0 | 0.0 | 9.3 | 0.0 |
| 22 | HTLV-I infection | 0.0 | 0.0 | 1.9 | 0.0 |
| 23 | response to virus | 96.8 | 0.0 | 0.0 | 0.0 |
| 24 | response to stress | 0.0 | 0.0 | 3.7 | 1.9 |
| 25 | sex differentiation | 0.0 | 0.0 | 0.0 | 0.0 |
| 26 | autophagy | 0.0 | 0.0 | 0.0 | 0.0 |
| 27 | small molecule metabolic process | 0.0 | 0.0 | 0.0 | 0.0 |
| 28 | mRNA Editing | 0.0 | 1.2 | 1.9 | 0.0 |
| 29 | Other types of O-glycan biosynthesis | 0.0 | 0.0 | 0.0 | 0.0 |
| 30 | small molecule metabolic process | 0.0 | 0.0 | 0.0 | 0.0 |
| 31 | cell division | 0.0 | 0.0 | 0.0 | 0.0 |
| 32 | positive regulation of tumor necrosis factor production | 0.0 | 0.0 | 0.0 | 52.8 |
| 33 | Extracellular matrix organization | 0.0 | 0.0 | 0.0 | 0.0 |
| 34 | Spliceosome | 0.0 | 0.0 | 0.0 | 0.0 |
| 35 | Nicotinate and nicotinamide metabolism | 0.0 | 0.0 | 5.6 | 0.0 |
| 36 | Adaptive Immune System\|Innate Immune System | 0.0 | 0.0 | 18.5 | 26.4 |
| 37 | Terpenoid backbone biosynthesis | 0.0 | 0.0 | 0.0 | 7.5 |
| 38 | extracellular matrix organization | 0.0 | 12.8 | 11.1 | 0.0 |
| 39 | immune system process | 0.0 | 24.4 | 35.2 | 0.0 |
| 40 | cytokine production | 0.0 | 1.2 | 0.0 | 1.9 |

[FIG.16C]

C

| LN | Term | G1(5 adj) | G1(8 adj) | G2 | G3* | G5 | G6* |
|---|---|---|---|---|---|---|---|
| 1 | anatomical structure development | 0.0 | 0.0 | 0.5 | 3.2 | 1.7 | 2.7 |
| 2 | cellular protein modification process | 0.0 | 0.0 | 1.1 | 2.2 | 0.4 | 1.6 |
| 3 | vesicle-mediated transport | 0.0 | 0.0 | 3.8 | 5.0 | 1.3 | 4.5 |
| 4 | extracellular matrix organization | 0.0 | 0.0 | 10.4 | 0.4 | 0.4 | 7.9 |
| 5 | negative regulation of phosphorylation | 0.0 | 0.0 | 0.0 | 7.0 | 0.0 | 0.0 |
| 6 | vesicle-mediated transport | 0.0 | 0.0 | 8.2 | 3.0 | 0.0 | 0.0 |
| 7 | cellular protein modification process | 0.0 | 0.0 | 13.1 | 0.2 | 8.2 | 0.4 |
| 8 | Jak-STAT signaling pathway | 0.0 | 0.0 | 8.7 | 0.4 | 0.4 | 0.9 |
| 9 | Extracellular matrix organization | 0.0 | 0.0 | 0.0 | 0.0 | 0.4 | 0.4 |
| 10 | Regulation of actin cytoskeleton | 0.0 | 0.0 | 0.0 | 0.2 | 0.4 | 5.8 |
| 11 | Intestinal immune network for IgA production | 0.0 | 0.0 | 4.4 | 1.6 | 2.1 | 4.7 |
| 12 | chromosome organization | 0.0 | 0.0 | 0.0 | 3.4 | 3.0 | 0.2 |
| 13 | Metabolic disorders of biological oxidation enzymes | 0.0 | 0.0 | 0.0 | 12.2 | 0.0 | 1.6 |
| 14 | Lysosome | 0.0 | 0.0 | 21.3 | 0.0 | 26.6 | 4.0 |
| 15 | immune system process | 0.0 | 0.0 | 0.0 | 0.0 | 3.0 | 9.2 |
| 16 | Phagosome | 0.0 | 0.0 | 0.0 | 0.0 | 1.7 | 1.3 |
| 17 | transport | 0.0 | 0.0 | 2.2 | 0.8 | 22.7 | 8.5 |
| 18 | phosphorus metabolic process | 0.0 | 0.0 | 12.0 | 14.4 | 6.4 | 11.7 |
| 19 | homeostatic process | 0.0 | 0.0 | 2.2 | 0.4 | 3.9 | 4.5 |
| 20 | cGMP-PKG signaling pathway | 0.0 | 0.0 | 0.0 | 2.4 | 14.6 | 2.9 |
| 21 | Extracellular matrix organization | 0.0 | 0.0 | 0.0 | 0.2 | 0.0 | 7.2 |
| 22 | cellular nitrogen compound metabolic process | 0.0 | 0.0 | 0.0 | 10.8 | 0.0 | 0.0 |
| 23 | RNA transport\|Processing of Capped Intron-Containing Pre-mRNA | 0.0 | 0.0 | 0.5 | 2.4 | 0.0 | 0.2 |
| 24 | cellular metabolic process | 0.0 | 0.0 | 0.0 | 5.4 | 0.0 | 0.4 |
| 25 | pattern recognition receptor signaling pathway | 0.0 | 1.9 | 0.0 | 1.6 | 0.0 | 0.0 |
| 26 | ribonucleoprotein complex biogenesis | 0.0 | 7.4 | 0.5 | 5.8 | 0.0 | 1.3 |
| 27 | immune system process | 0.0 | 1.9 | 1.1 | 1.4 | 0.0 | 2.2 |
| 28 | response to external stimulus | 0.0 | 0.0 | 7.1 | 1.2 | 0.0 | 4.0 |
| 29 | Adaptive Immune System\|Innate Immune System | 0.0 | 1.9 | 1.6 | 0.0 | 0.0 | 0.4 |
| 30 | transport | 0.8 | 5.6 | 0.0 | 0.2 | 0.0 | 2.7 |
| 31 | chromosome organization | 0.0 | 3.7 | 0.0 | 2.6 | 0.0 | 1.6 |
| 32 | Phosphatidylinositol signaling system | 0.0 | 0.0 | 0.0 | 2.4 | 0.0 | 4.3 |
| 33 | anatomical structure development | 0.0 | 0.0 | 0.0 | 5.6 | 0.4 | 0.7 |
| 34 | Generic Transcription Pathway | 0.0 | 0.0 | 0.0 | 1.4 | 0.0 | 0.4 |
| 35 | cell morphogenesis | 0.0 | 0.0 | 0.5 | 0.0 | 0.0 | 0.0 |
| 36 | Jak-STAT signaling pathway | 0.0 | 5.6 | 0.0 | 0.0 | 0.0 | 0.0 |
| 37 | response to other organism | 10.2 | 5.6 | 0.5 | 0.2 | 0.0 | 0.7 |
| 38 | macromolecule metabolic process | 5.7 | 20.4 | 0.0 | 0.4 | 0.0 | 0.2 |
| 39 | cell death | 0.0 | 3.7 | 0.0 | 0.0 | 0.0 | 0.0 |
| 40 | immune system process | 83.2 | 42.6 | 0.0 | 1.8 | 0.0 | 0.4 |

[FIG.17]

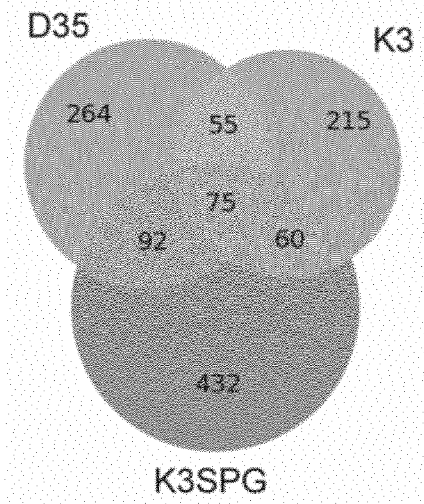


| | Annotation | P value | Genes (only first 5 genes are shown) |
|---|---|---|---|
| D35 | atrioventricular valve morphogenesis | 7.01E-02 | Cyr61, Hey2, Heyl, Olfm1 |
| | Proteoglycans in cancer | 2.15E-01 | Thbs1, Fn1, Col1a2, Fasl, Cdkn1a |
| | Oxytocin signaling pathway | 3.06E-01 | Plcb4, Prkag2, Fos, Cdkn1a, Itpr2 |
| | Amoebiasis | 5.09E-01 | Plcb4, Serpinb9g, Fn1, Col1a2, Prkacb |
| | Extracellular matrix organization | 5.77E-01 | Thbs1, Fn1, Col1a2, Timp1, Mmp3 |
| K3 | N.D. | | |
| K3SPG | cell death | 5.27E-01 | Ier3, Mtch2, Pak2, Fndc1, Chd8 |
| | cell differentiation | 5.78E-01 | Myo16, Drd3, Ednra, Nab1, Kdm6b |
| | Phenylalanine, tyrosine and tryptophan biosynthesis | 7.87E-01 | Got2, 4930438A08Rik |
| D35_K3 | Influenza A | 2.60E-05 | Pml, Irf9, Trim25, Tlr7, Ddx58 |
| | Herpes simplex infection | 2.65E-03 | Daxx, Pml, Irf9, Ddx58, Stat2 |
| | immune system process | 8.09E-03 | Pml, Samhd1, Hsh2d, Parp9, Irgm2 |
| | Osteoclast differentiation | 8.71E-03 | Irf9, Sirpb1a, Fcgr1, Stat2 |
| | Measles | 1.21E-02 | Irf9, Tlr7, Ddx58, Stat2 |
| K3_K3SPG | positive regulation of cytokine secretion | 4.30E-06 | Ccl3, Il1a, Clec4n, Ifng, Tlr2 |
| | Cytokine-cytokine receptor interaction | 9.10E-06 | Cd209f, Cd209g, Il1a, C5ar1, Cd14 |
| | positive regulation of cytokine production | 1.13E-05 | C3ar1, Ccl3, C5ar1, Il1a, Clec4n |
| | Tuberculosis | 2.77E-05 | Cd209f, Il1a, Cd209g, Cd14, Ifng |
| | Toll-like receptor signaling pathway | 2.96E-04 | Ccl3, Cd14, Tlr2, Cxcl9, Tnf |
| D35_K3SPG | TNF signaling pathway | 1.54E-03 | Ptgs2, Cxcl1, Ikbkg, Il1b, Socs3 |
| | Herpes simplex infection | 3.34E-02 | Hnrnpk, Ikbkg, Il1b, H2-T24, Socs3 |
| | Metabolism of water-soluble vitamins and cofactors | 9.59E-02 | Ptgs2, Nmnat1, Slc23a1 |
| | NOD-like receptor signaling pathway | 1.03E-01 | Cxcl1, Ikbkg, Il1b |
| | response to stress | 4.23E-01 | Ptgs2, Hnrnpk, Cxcl1, Ikbkg, Il1b |
| D35_K3_K3SPG | response to other organism | 2.16E-15 | Ifit3, Ifit2, Ifit1, Cxcl10, Il6 |
| | response to cytokine | 1.26E-10 | Ifit3, Ifit2, Ifit1, Cxcl10, Irf7 |
| | response to external stimulus | 2.18E-09 | Ifit3, Ifit2, Ifit1, Cxcl10, Psma1 |
| | Influenza A | 2.48E-08 | Eif2ak2, Rsad2, Ifih1, Ccl2, Cxcl10 |
| | defense response | 1.39E-07 | Cxcl10, Psma1, Irf7, Il6, Dhx58 |
| | Herpes simplex infection | 1.71E-07 | Eif2ak2, Gm14446, Ifih1, Ifit1, Ccl2 |
| | Cytokine-cytokine receptor interaction | 3.14E-07 | Ifit1, Cxcl10, Irf7, Il6, Vcan |
| | response to interferon-beta | 2.90E-06 | Ifit3, Igtp, Ifit1, Xaf1, Stat1 |
| | Innate/Adaptive Immune System | 3.60E-06 | Clec4d, Ifit1, Psma1, Il6, Gzmb |
| | immune system process | 4.50E-06 | Cxcl10, Psma1, Irf7, Il6, Gzmb |

[FIG.18]

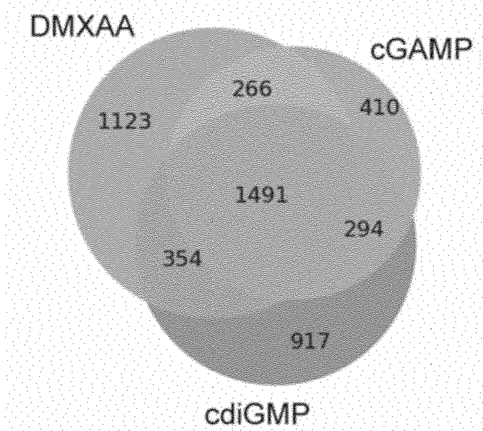


| | Annotation | P value | Genes (only first 5 genes are shown) |
|---|---|---|---|
| cdiGMP | Cytokine-cytokine receptor interaction | 2.15E-05 | Csf2ra, Ifna2, Ifnb1, Ifna1, Csf3r |
| | PI3K-Akt signaling pathway | 3.30E-05 | Nfkb1, Ccne1, Ifna2, Ifnb1, Pik3r5 |
| | Toll-like receptor signaling pathway | 8.58E-05 | Nfkb1, Ifna2, Ifnb1, Pik3r5, Ifna1 |
| | RIG-I-like receptor signaling pathway | 1.16E-04 | Nfkb1, Ifna2, Ifnb1, Ifna1, Ifnab |
| | Hepatitis B | 1.37E-04 | Nfkb1, Ccne1, Ifna2, Ifnb1, Prkca |
| cGAMP | positive regulation of biological process | 5.15E-02 | Dlx2, P2rx7, Cenpe, Rif1, Fuz |
| | cellular nitrogen compound metabolic process | 4.07E-01 | Dlx2, P2rx7, Rif1, Fabp4, Rbmx |
| | HIF-1 signaling pathway | 8.14E-01 | Arnt, Angpt1, Mapk1, Cul2, Igf1r |
| | biosynthetic process | 8.15E-01 | Dlx2, P2rx7, Pik3c2a, Fabp4, Oxsm |
| | Cytokine-cytokine receptor interaction | 9.17E-01 | Aicda, Prg2, Ikbkg, Atp6v0c, Mvb12b |
| DMXAA | ribosome biogenesis | 2.87E-14 | Xpo1, Bysl, Mphosph10, Srfbp1, Wdr46 |
| | ribonucleoprotein complex biogenesis | 4.56E-13 | Xpo1, Prmt7, Prmt5, Bysl, Mphosph10 |
| | ncRNA metabolic process | 1.01E-10 | Gars, Mars, Bysl, Mphosph10, Mettl2 |
| | ncRNA processing | 2.14E-09 | Bysl, Mphosph10, Mettl2, Srfbp1, Nop14 |
| | RNA processing | 2.16E-08 | Prmt7, Rbm25, Prmt5, Bysl, Mphosph10 |
| cdiGMP_cGAMP | protein maturation | 9.54E-02 | Pthlh, Birc3, Senp1, Tnf, Asph |
| | secondary metabolic process | 1.67E-01 | Kmo, Zeb2, Rapgef2,, |
| | anatomical structure development | 2.30E-01 | Rho, Lta, Srgap2, Pdgfc, Snx2 |
| | plasma membrane organization | 3.05E-01 | Flot1, Fat4, Optn, Whamm, Ehd3 |
| | cell death | 3.62E-01 | Casp12, Fem1b, Lta, Rbck1, Eda2r |
| cGAMP_DMXAA | ribosome biogenesis | 4.90E-06 | Lsm6, Dkc1, Nol9, Rps24, Tsr1 |
| | ribonucleoprotein complex biogenesis | 6.50E-04 | Snrpd3, Dkc1, Nufip1, Heatr1, Utp6 |
| | ncRNA metabolic process | 8.34E-03 | Dkc1, Heatr1, Utp6, Aars, Shq1 |
| | cellular metabolic process | 8.82E-03 | Prmt3, Card11, Aars, Kdm5c, Srm |
| | RNA processing | 9.49E-03 | Snrpd3, Dkc1, Papd4, Heatr1, Utp6 |
| cdiGMP_DMXAA | NF-kappa B signaling pathway | 3.35E-04 | Tnfaip3, Prkcq, Nfkbia, Birc2, Cxcl2 |
| | immune system process | 1.29E-03 | Rbm47, Nfkbia, Spta1, Il18bp, Cd83 |
| | cell death | 3.45E-03 | Ier3, Siah2, Bag3, Sav1, Tnfrsf1a |
| | regulation of response to stress | 5.34E-03 | Ier3, Nfkbia, Tnfrsf1a, Zfp36, Tlr13 |
| | Cytokine-cytokine receptor interaction | 6.64E-03 | Il1a, Tnfsf9, Cxcl2, Amhr2, Il12b |
| cdiGMP_cGAMP_DMXAA | response to other organism | 3.51E-31 | Samhd1, Cd86, 2010002M12Rik, Oas1b, Oas1a |
| | response to stress | 4.29E-25 | Mcl1, Samhd1, Ercc8, 2010002M12Rik, Cd86 |
| | defense response | 9.42E-25 | Samhd1, Ccrl2, 2010002M12Rik, Cd86, Klrk1 |
| | immune system process | 9.98E-25 | Themis2, Samhd1, Bcl11a, 2010002M12Rik, Gzmb |
| | response to virus | 2.35E-20 | Ifit3, Ifit2, Ifit1, Nlrc5, Samhd1 |
| | response to external stimulus | 2.81E-19 | Mycbp2, Samhd1, 2010002M12Rik, Cd86, Oas1b |
| | response to cytokine | 3.91E-19 | Pyhin1, Nlrc5, Mcl1, Ccrl2, Il6 |
| | multi-organism process | 5.93E-16 | Samhd1, 2010002M12Rik, Cd86, Oas1b, Oas1a |
| | Innate\|Adaptive Immune System\| | 9.73E-16 | Lpar1, Clec4d, Il13ra1, Pcgf5, Mcl1 |
| | defense response to other organism | 1.09E-15 | Nlrc5, Samhd1, Il6, 2010002M12Rik, Cd86 |

[FIG.19A]

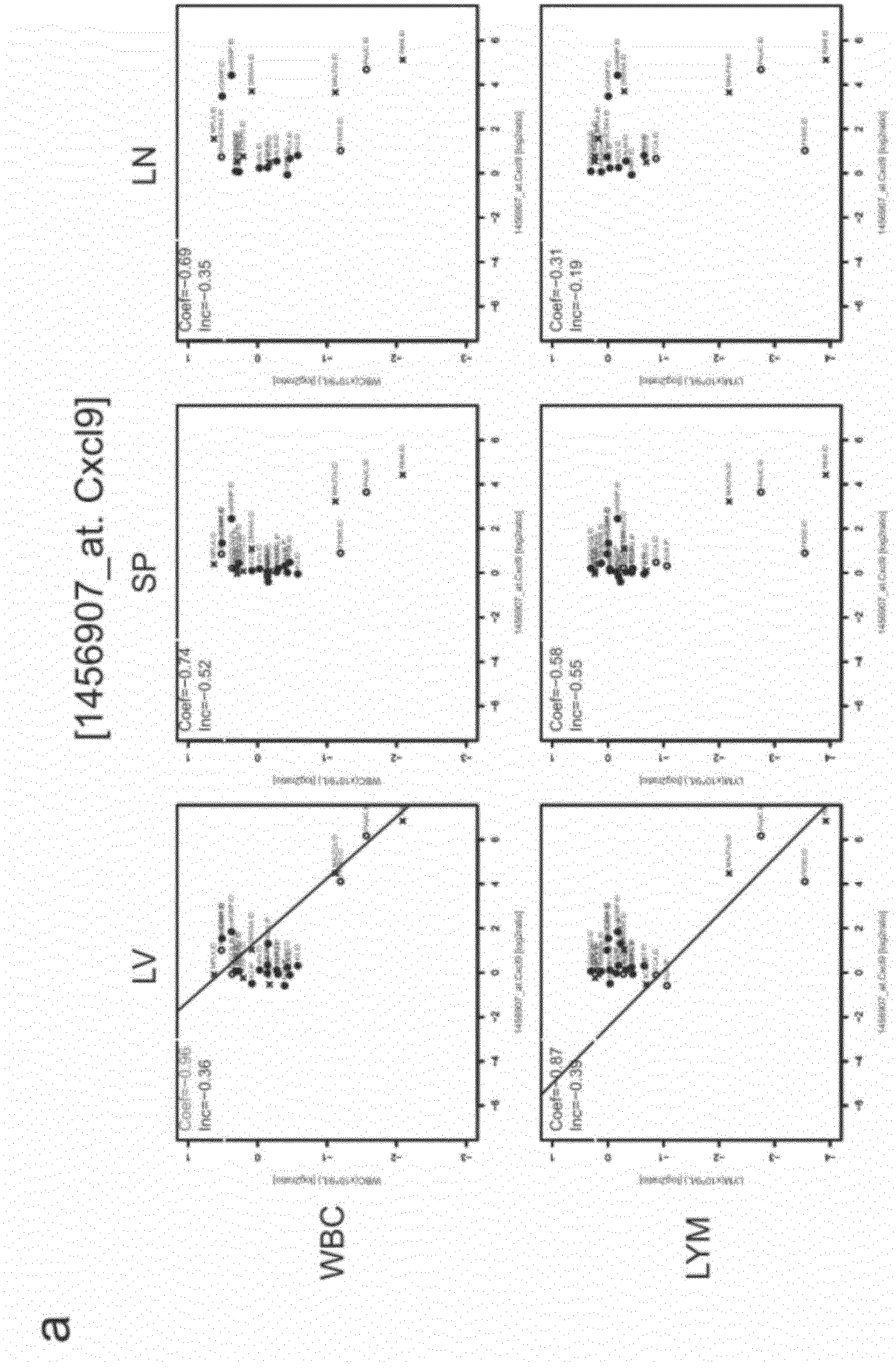
a
[1456907_at. Cxcl9]
LV
SP
LN
WBC
LYM
Coef=-0.69
Inc=-0.35
Coef=-0.74
Inc=-0.52
Coef=-0.31
Inc=-0.19
Coef=-0.58
Inc=-0.55
Inc=-0.36

[FIG.19B]

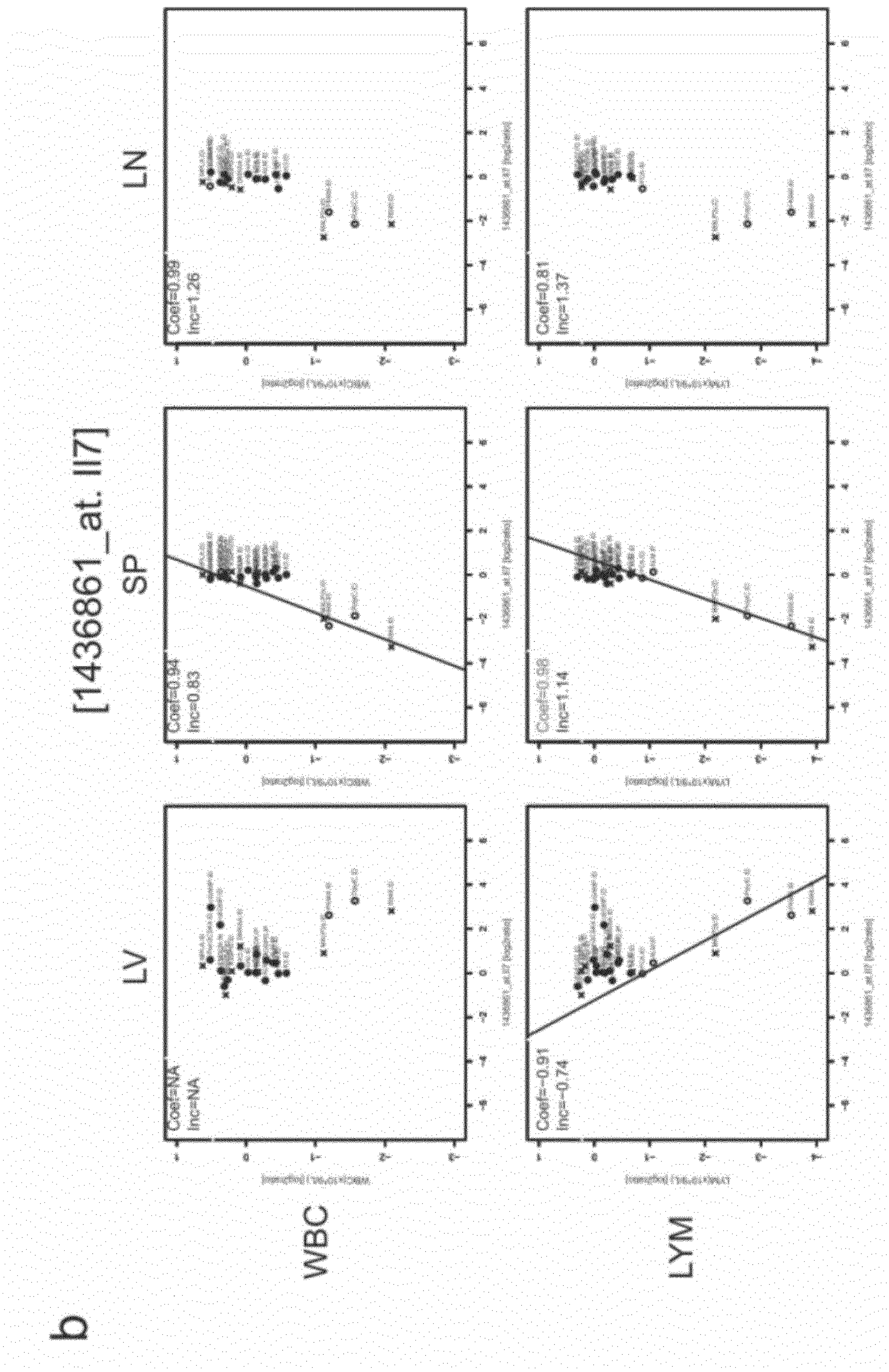
b
[1436861_at. Il7]
LV
SP
LN
WBC
LYM
Coef=NA
Inc=NA
Coef=0.94
Inc=0.83
Coef=0.99
Inc=1.26
Coef=-0.91
Inc=-0.74
Coef=0.98
Inc=1.14
Coef=0.81
Inc=1.37

[FIG.19C]

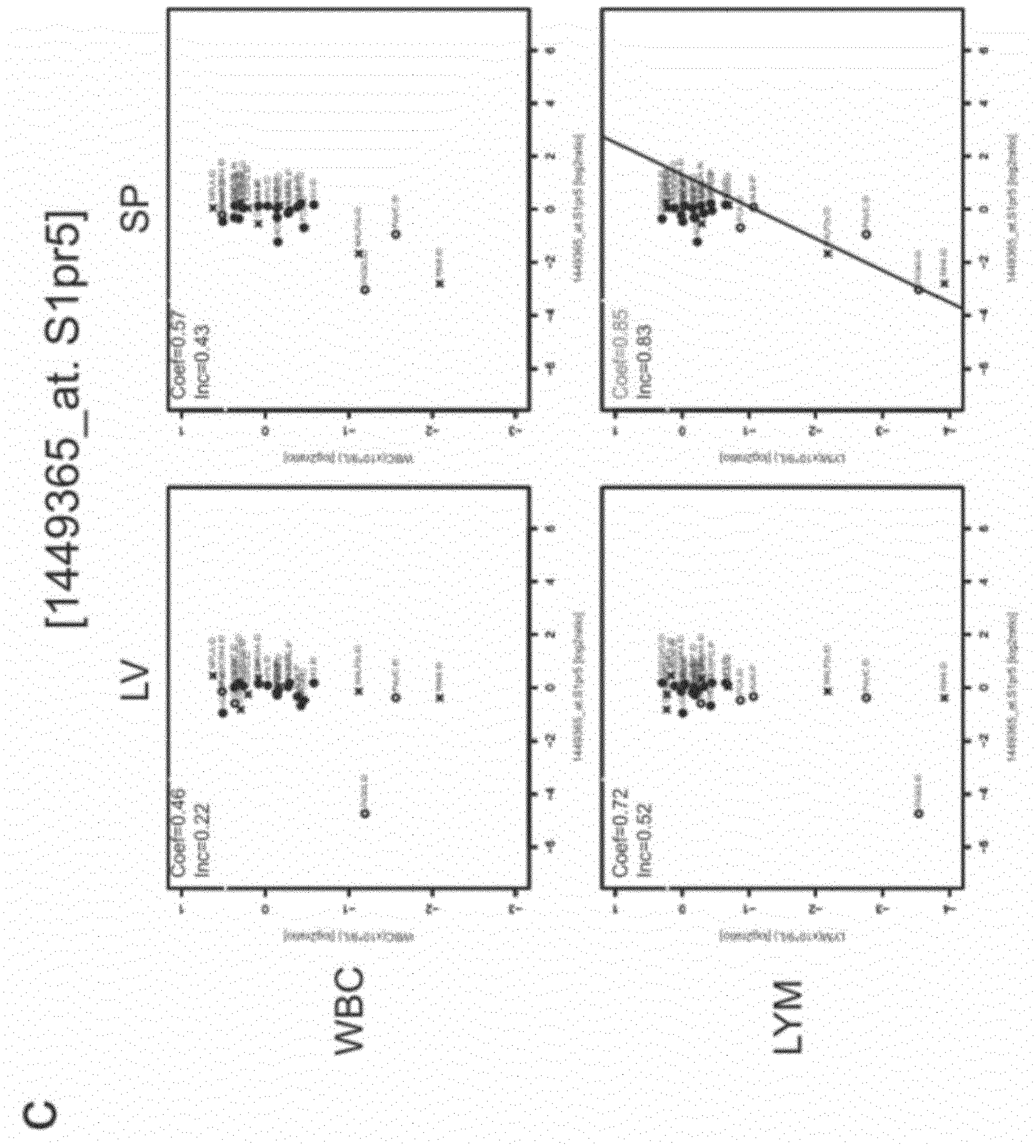
C
[1449365_at. S1pr5]
LV
SP
WBC
LYM
Coef=0.46
Inc=0.22
Coef=0.57
Inc=0.43
Coef=0.72
Inc=0.52
Coef=0.85
Inc=0.83

[FIG.20A]

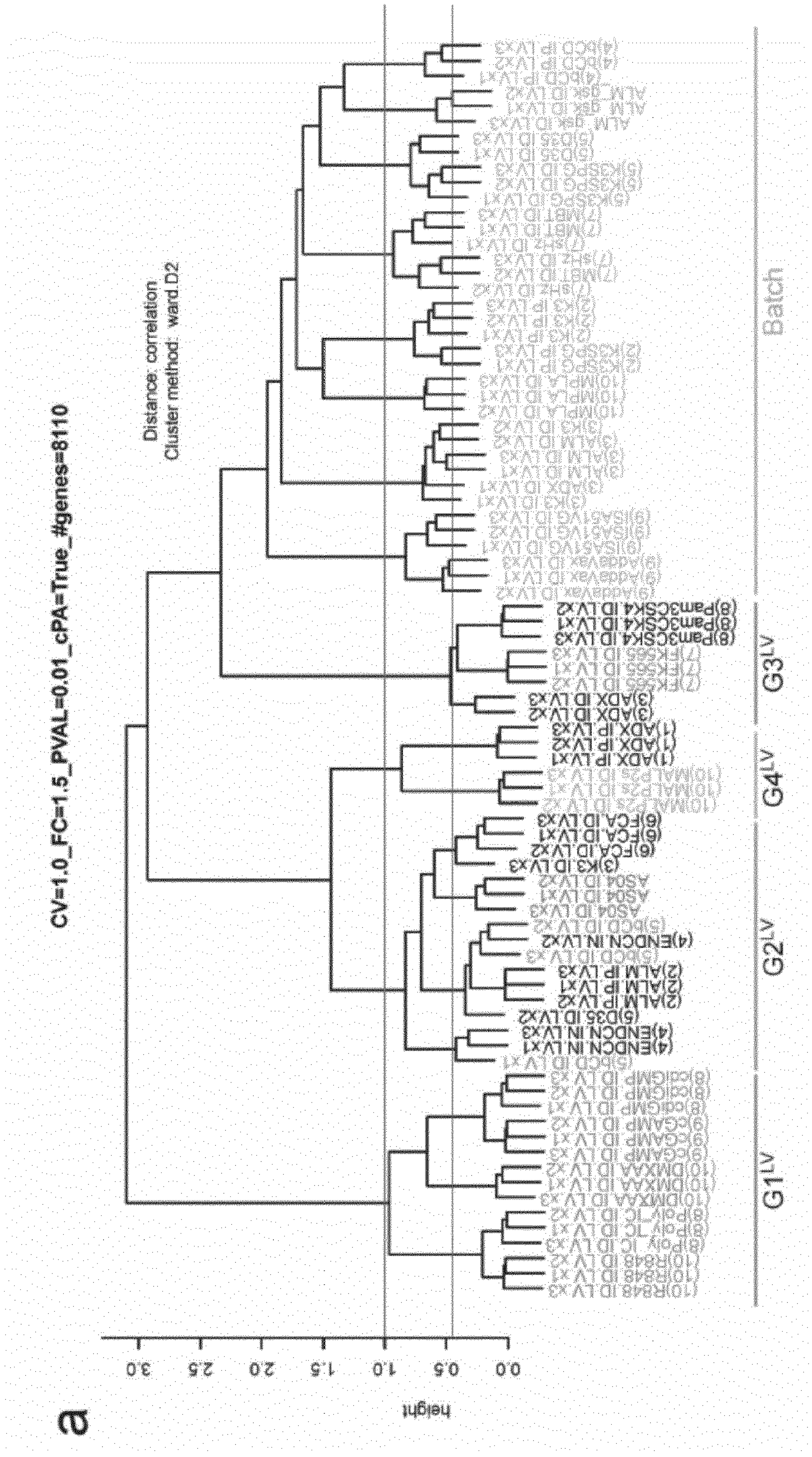
a
CV=1.0_FC=1.5_PVAL=0.01_cPA=True_#genes=8110
Distance: correlation
Cluster method: ward.D2
height
G1LV
G2LV
G4LV
G3LV
Batch

[FIG.20B]

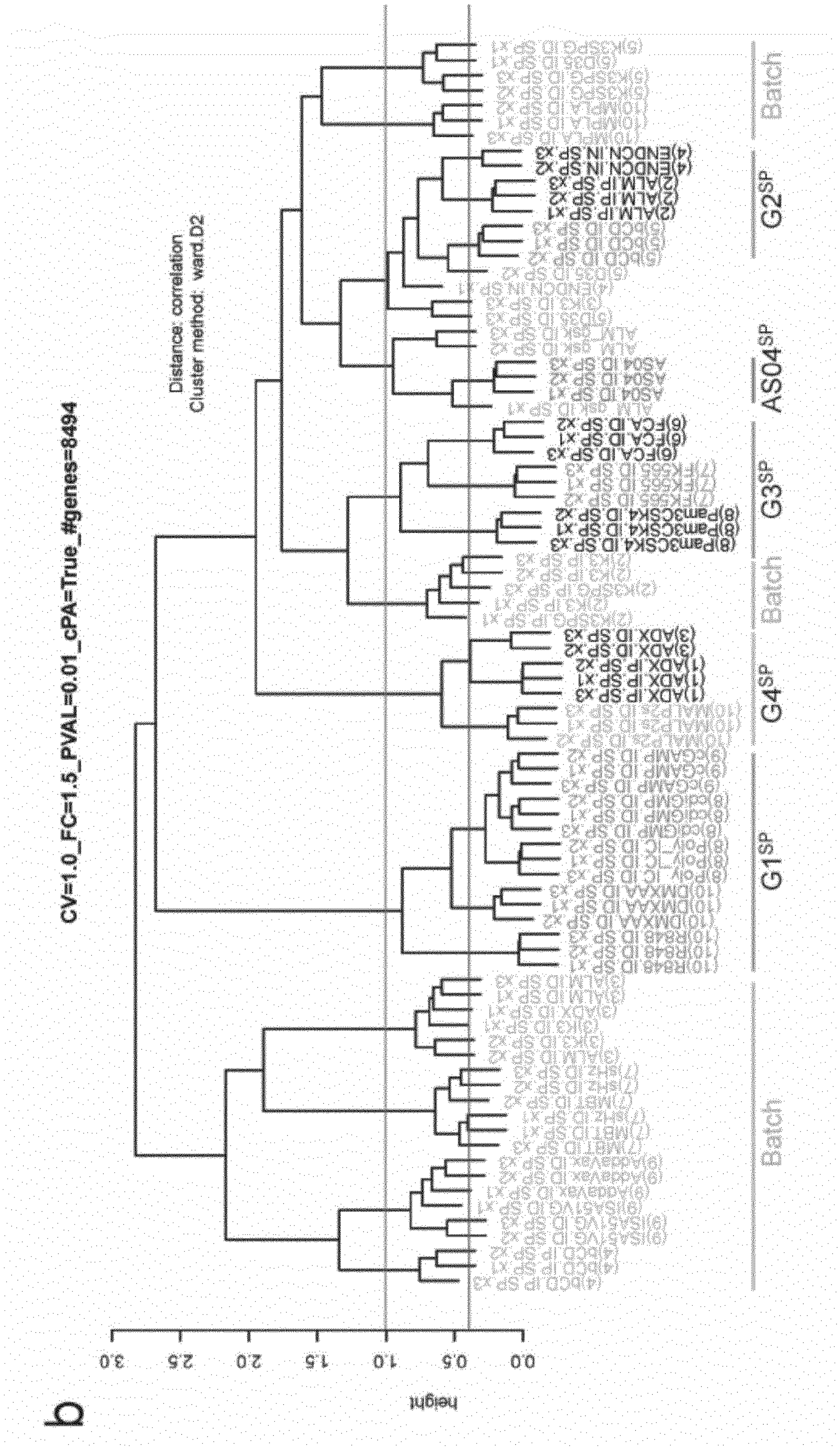
b
CV=1.0_FC=1.5_PVAL=0.01_cPA=True_#genes=8494
Distance: correlation
Cluster method: ward.D2
height
3.0
2.5
2.0
1.5
1.0
0.5
0.0
Batch
G1SP
G4SP
Batch
G3SP
AS04SP
G2SP
Batch

[FIG.20C]

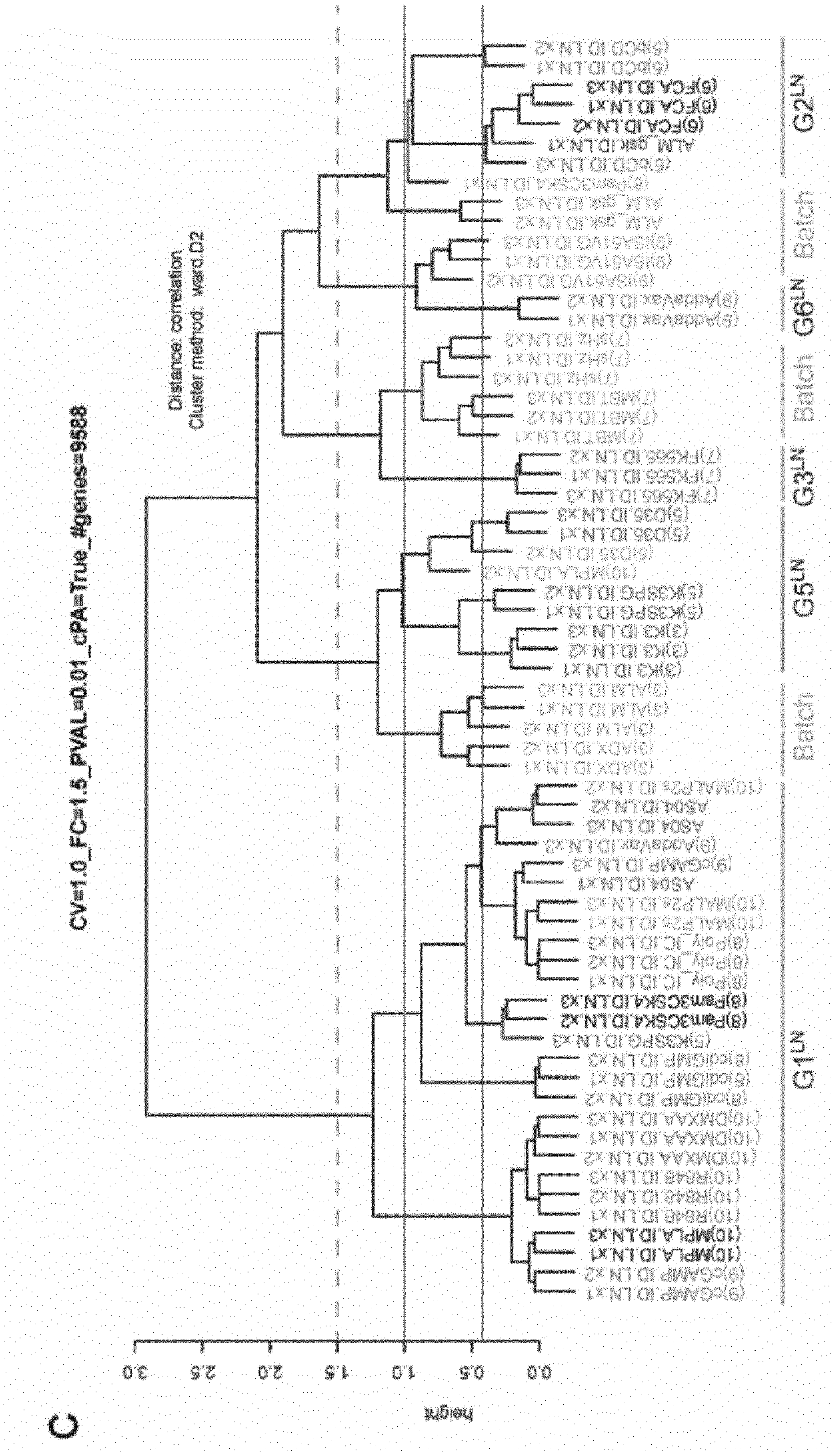
C
CV=1.0_FC=1.5_PVAL=0.01_cPA=True_#genes=9588
Distance: correlation
Cluster method: ward.D2
height
3.0
2.5
2.0
1.5
1.0
0.5
0.0
(5)bCD.ID.LN.x2
(5)bCD.ID.LN.x1
(6)FCA.ID.LN.x3
(6)FCA.ID.LN.x1
(6)FCA.ID.LN.x2
ALM_gsk.ID.LN.x1
(5)bCD.ID.LN.x3
(8)Pam3CSK4.ID.LN.x1
ALM_gsk.ID.LN.x3
ALM_gsk.ID.LN.x2
(9)ISA51VG.ID.LN.x3
(9)ISA51VG.ID.LN.x1
(9)ISA51VG.ID.LN.x2
(9)AddaVax.ID.LN.x2
(9)AddaVax.ID.LN.x1
(7)sHz.ID.LN.x2
(7)sHz.ID.LN.x1
(7)sHz.ID.LN.x3
(7)MBT.ID.LN.x3
(7)MBT.ID.LN.x2
(7)MBT.ID.LN.x1
(7)FK565.ID.LN.x2
(7)FK565.ID.LN.x1
(7)FK565.ID.LN.x3
(5)D35.ID.LN.x3
(5)D35.ID.LN.x1
(6)D35.ID.LN.x2
(10)MPLA.ID.LN.x2
(5)K3SPG.ID.LN.x2
(5)K3SPG.ID.LN.x1
(3)K3.ID.LN.x3
(3)K3.ID.LN.x2
(3)K3.ID.LN.x1
(3)ALM.ID.LN.x3
(3)ALM.ID.LN.x1
(3)ALM.ID.LN.x2
(3)ADX.ID.LN.x2
(3)ADX.ID.LN.x1
(10)MALP2s.ID.LN.x2
AS04.ID.LN.x2
AS04.ID.LN.x3
(9)AddaVax.ID.LN.x3
(9)cGAMP.ID.LN.x3
AS04.ID.LN.x1
(10)MALP2s.ID.LN.x3
(10)MALP2s.ID.LN.x1
(8)Poly_IC.ID.LN.x3
(8)Poly_IC.ID.LN.x2
(8)Poly_IC.ID.LN.x1
(8)Pam3CSK4.ID.LN.x3
(8)Pam3CSK4.ID.LN.x2
(5)K3SPG.ID.LN.x3
(8)cdiGMP.ID.LN.x3
(8)cdiGMP.ID.LN.x1
(8)cdiGMP.ID.LN.x2
(10)DMXAA.ID.LN.x3
(10)DMXAA.ID.LN.x1
(10)DMXAA.ID.LN.x2
(10)R848.ID.LN.x3
(10)R848.ID.LN.x2
(10)R848.ID.LN.x1
(10)MPLA.ID.LN.x3
(10)MPLA.ID.LN.x1
(9)cGAMP.ID.LN.x2
(9)cGAMP.ID.LN.x1
G2LN
Batch
G6LN
Batch
G3LN
G5LN
Batch
G1LN

[FIG.20D]

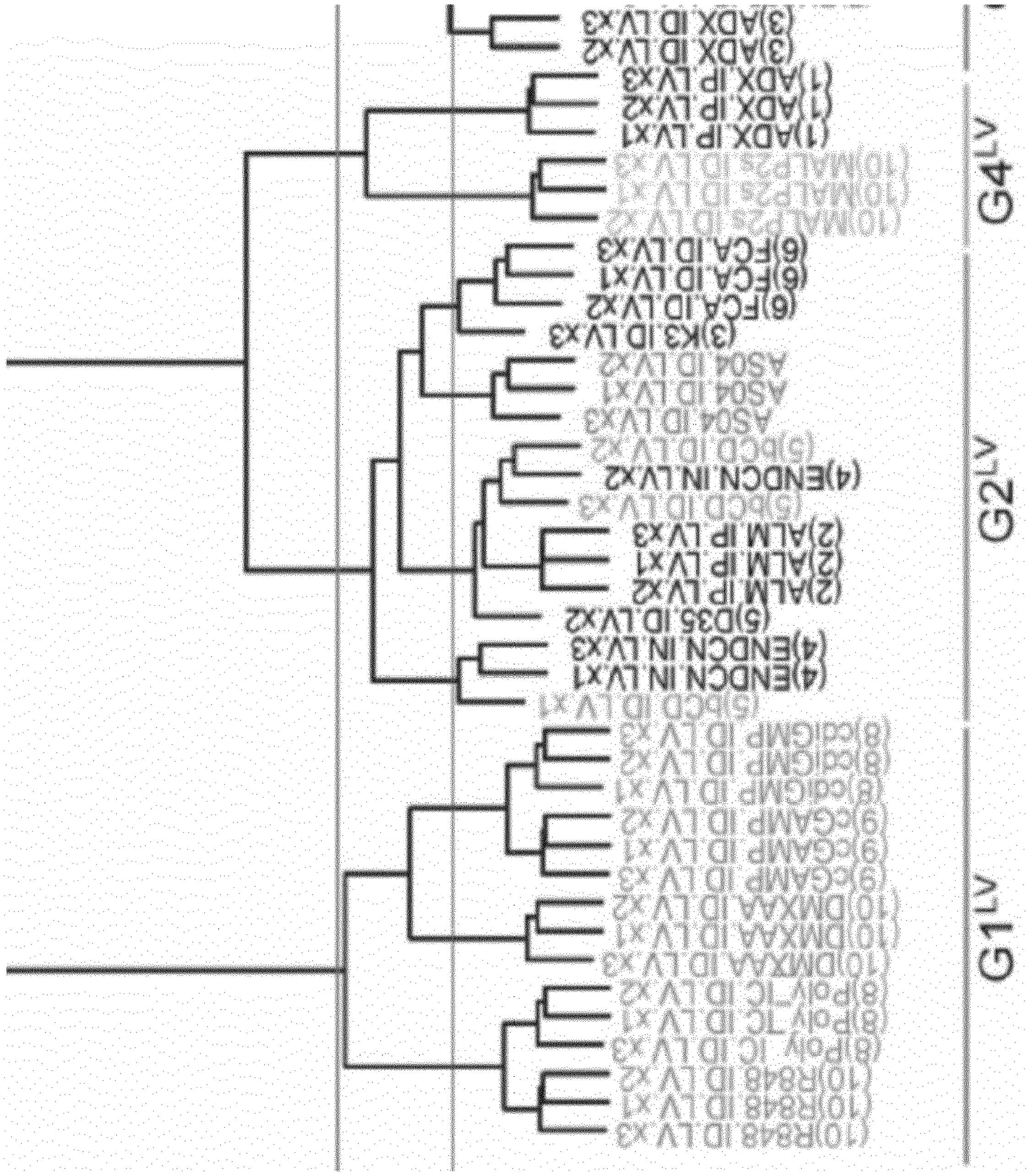
(3)ADX.ID.LV.x3
(3)ADX.ID.LV.x2
(1)ADX.IP.LV.x3
(1)ADX.IP.LV.x2
(1)ADX.IP.LV.x1
(10)MALP2s.ID.LV.x3
(10)MALP2s.ID.LV.x1
(10)MALP2s.ID.LV.x2
(6)FCA.ID.LV.x3
(6)FCA.ID.LV.x1
(6)FCA.ID.LV.x2
(3)K3.ID.LV.x3
AS04.ID.LV.x2
AS04.ID.LV.x1
AS04.ID.LV.x3
(5)bCD.ID.LV.x2
(4)ENDCN.IN.LV.x2
(5)bCD.ID.LV.x3
(2)ALM.IP.LV.x3
(2)ALM.IP.LV.x1
(2)ALM.IP.LV.x2
(5)D35.ID.LV.x2
(4)ENDCN.IN.LV.x3
(4)ENDCN.IN.LV.x1
(5)bCD.ID.LV.x1
(8)cdiGMP.ID.LV.x3
(8)cdiGMP.ID.LV.x2
(8)cdiGMP.ID.LV.x1
(9)cGAMP.ID.LV.x2
(9)cGAMP.ID.LV.x1
(9)cGAMP.ID.LV.x3
(10)DMXAA.ID.LV.x2
(10)DMXAA.ID.LV.x1
(10)DMXAA.ID.LV.x3
(8)PolyIC.ID.LV.x2
(8)PolyIC.ID.LV.x1
(8)Poly IC.ID.LV.x3
(10)R848.ID.LV.x2
(10)R848.ID.LV.x1
(10)R848.ID.LV.x3
G4LV
G2LV
G1LV

[FIG.20E]

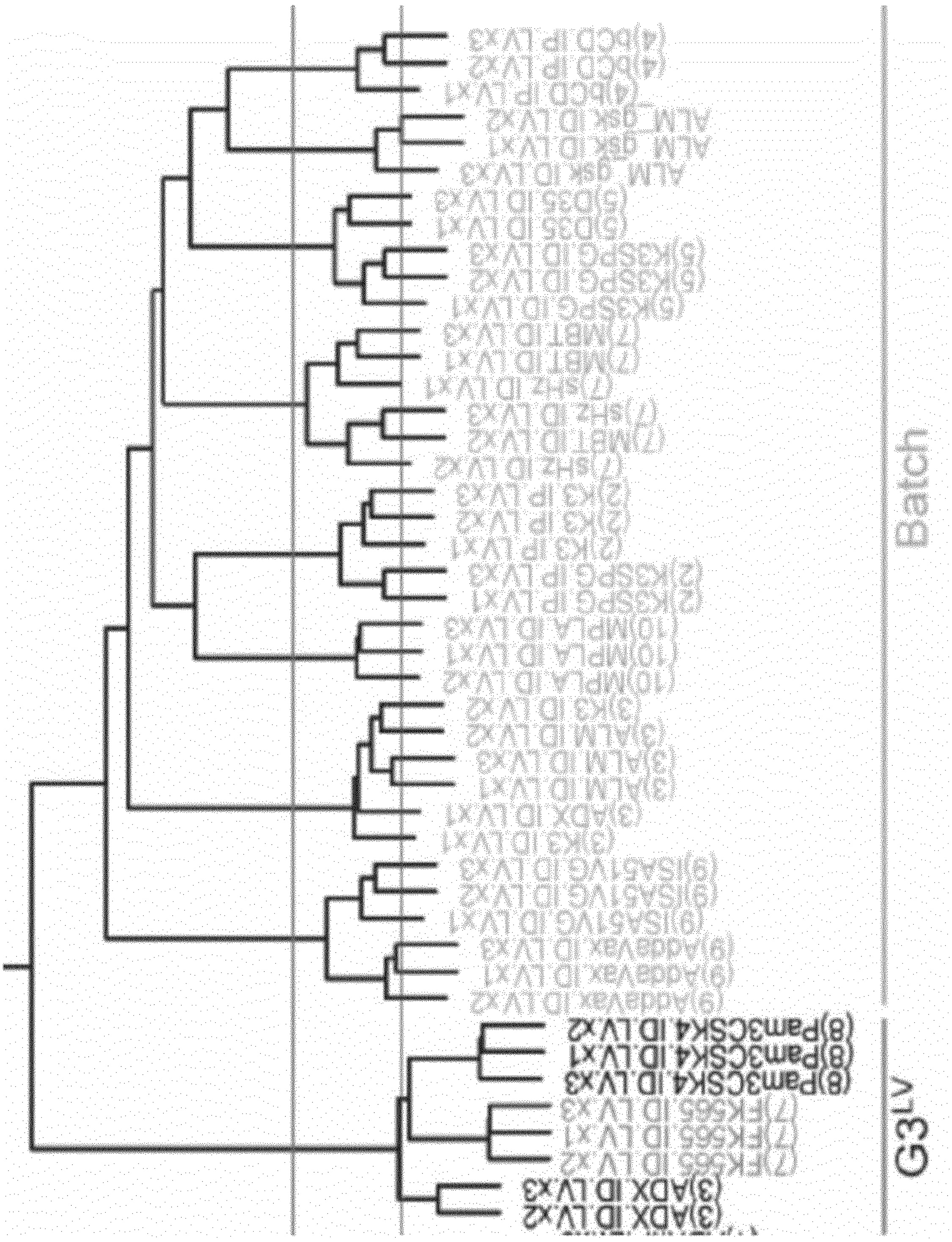
(4)bCD.IP.LV.x3
(4)bCD.IP.LV.x2
(4)bCD.IP.LV.x1
ALM_gsk.ID.LV.x2
ALM_gsk.ID.LV.x1
ALM_gsk.ID.LV.x3
(5)D35.ID.LV.x3
(5)D35.ID.LV.x1
(5)K3SPG.ID.LV.x3
(5)K3SPG.ID.LV.x2
(5)K3SPG.ID.LV.x1
(7)MBT.ID.LV.x3
(7)MBT.ID.LV.x1
(7)sHz.ID.LV.x1
(7)sHz.ID.LV.x3
(7)MBT.ID.LV.x2
(7)sHz.ID.LV.x2
(2)K3.IP.LV.x3
(2)K3.IP.LV.x2
(2)K3.IP.LV.x1
(2)K3SPG.IP.LV.x3
(2)K3SPG.IP.LV.x1
(10)MPLA.ID.LV.x3
(10)MPLA.ID.LV.x1
(10)MPLA.ID.LV.x2
(3)K3.ID.LV.x2
(3)ALM.ID.LV.x2
(3)ALM.ID.LV.x3
(3)ALM.ID.LV.x1
(3)ADX.ID.LV.x1
(3)K3.ID.LV.x1
(9)ISA51VG.ID.LV.x3
(9)ISA51VG.ID.LV.x2
(9)ISA51VG.ID.LV.x1
(9)Addavax.ID.LV.x3
(9)Addavax.ID.LV.x1
(9)Addavax.ID.LV.x2
(8)Pam3CSK4.ID.LV.x2
(8)Pam3CSK4.ID.LV.x1
(8)Pam3CSK4.ID.LV.x3
(7)FK565.ID.LV.x3
(7)FK565.ID.LV.x1
(7)FK565.ID.LV.x2
(3)ADX.ID.LV.x3
(3)ADX.ID.LV.x2
Batch
G3LV

[FIG.20F]

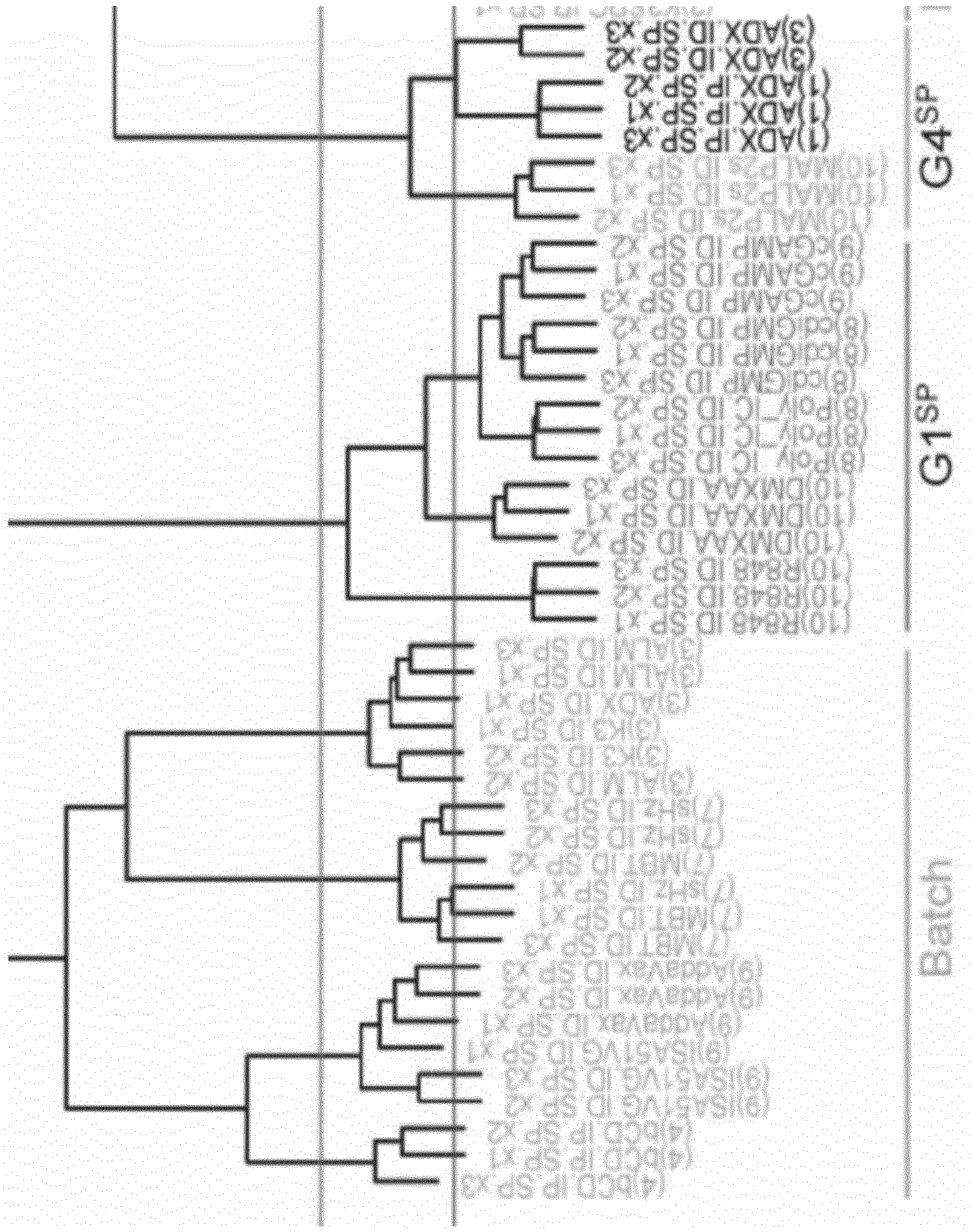
(3)ADX.ID.SP.x3
(3)ADX.ID.SP.x2
(1)ADX.IP.SP.x2
(1)ADX.IP.SP.x1
(1)ADX.IP.SP.x3
(10)MALP2s.ID.SP.x3
(10)MALP2s.ID.SP.x1
(10)MALP2s.ID.SP.x2
(9)cGAMP.ID.SP.x2
(9)cGAMP.ID.SP.x1
(9)cGAMP.ID.SP.x3
(8)cdiGMP.ID.SP.x2
(8)cdiGMP.ID.SP.x1
(8)cdiGMP.ID.SP.x3
(8)Poly_IC.ID.SP.x2
(8)Poly_IC.ID.SP.x1
(8)Poly_IC.ID.SP.x3
(10)DMXAA.ID.SP.x3
(10)DMXAA.ID.SP.x1
(10)DMXAA.ID.SP.x2
(10)R848.ID.SP.x3
(10)R848.ID.SP.x2
(10)R848.ID.SP.x1
(3)ALM.ID.SP.x3
(3)ALM.ID.SP.x1
(3)ADX.ID.SP.x1
(3)K3.ID.SP.x1
(3)K3.ID.SP.x2
(3)ALM.ID.SP.x2
(7)sHz.ID.SP.x3
(7)sHz.ID.SP.x2
(7)MBT.ID.SP.x2
(7)sHz.ID.SP.x1
(7)MBT.ID.SP.x1
(7)MBT.ID.SP.x3
(6)AddaVax.ID.SP.x3
(6)AddaVax.ID.SP.x2
(6)AddaVax.ID.SP.x1
(6)ISA51VG.ID.SP.x1
(6)ISA51VG.ID.SP.x3
(6)ISA51VG.ID.SP.x2
(4)bCD.IP.SP.x2
(4)bCD.IP.SP.x1
(4)bCD.IP.SP.x3
G4$^{SP}$
G1$^{SP}$
Batch

[FIG.20G]

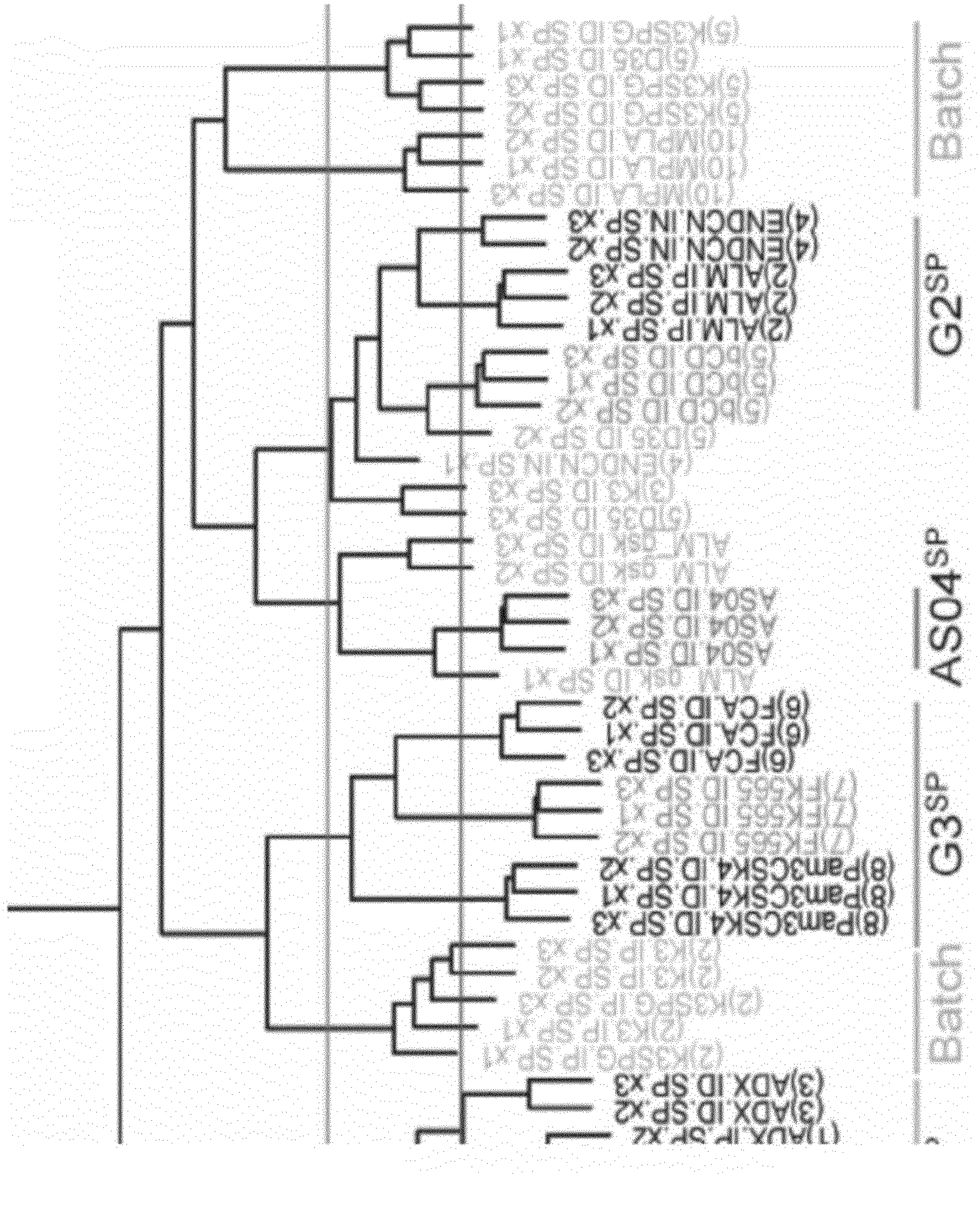
Batch
G2SP
AS04SP
G3SP
Batch
(5)K3SPG.ID.SP.x1
(5)D35.ID.SP.x1
(5)K3SPG.ID.SP.x3
(5)K3SPG.ID.SP.x2
(10)MPLA.ID.SP.x2
(10)MPLA.ID.SP.x1
(10)MPLA.ID.SP.x3
(4)ENDCN.IN.SP.x3
(4)ENDCN.IN.SP.x2
(2)ALM.IP.SP.x3
(2)ALM.IP.SP.x2
(2)ALM.IP.SP.x1
(5)bCD.ID.SP.x3
(5)bCD.ID.SP.x1
(5)bCD.ID.SP.x2
(5)D35.ID.SP.x2
(4)ENDCN.IN.SP.x1
(3)K3.ID.SP.x3
(5)D35.ID.SP.x3
ALM_gsk.ID.SP.x3
ALM_gsk.ID.SP.x2
AS04.ID.SP.x3
AS04.ID.SP.x2
AS04.ID.SP.x1
ALM_gsk.ID.SP.x1
(6)FCA.ID.SP.x2
(6)FCA.ID.SP.x1
(6)FCA.ID.SP.x3
(7)FK565.ID.SP.x3
(7)FK565.ID.SP.x1
(7)FK565.ID.SP.x2
(8)Pam3CSK4.ID.SP.x2
(8)Pam3CSK4.ID.SP.x1
(8)Pam3CSK4.ID.SP.x3
(2)K3.IP.SP.x3
(2)K3.IP.SP.x2
(2)K3SPG.IP.SP.x3
(2)K3.IP.SP.x1
(2)K3SPG.IP.SP.x1
(3)ADX.ID.SP.x3
(3)ADX.ID.SP.x2
(1)ADX.IP.SP.x2

[FIG.20H]

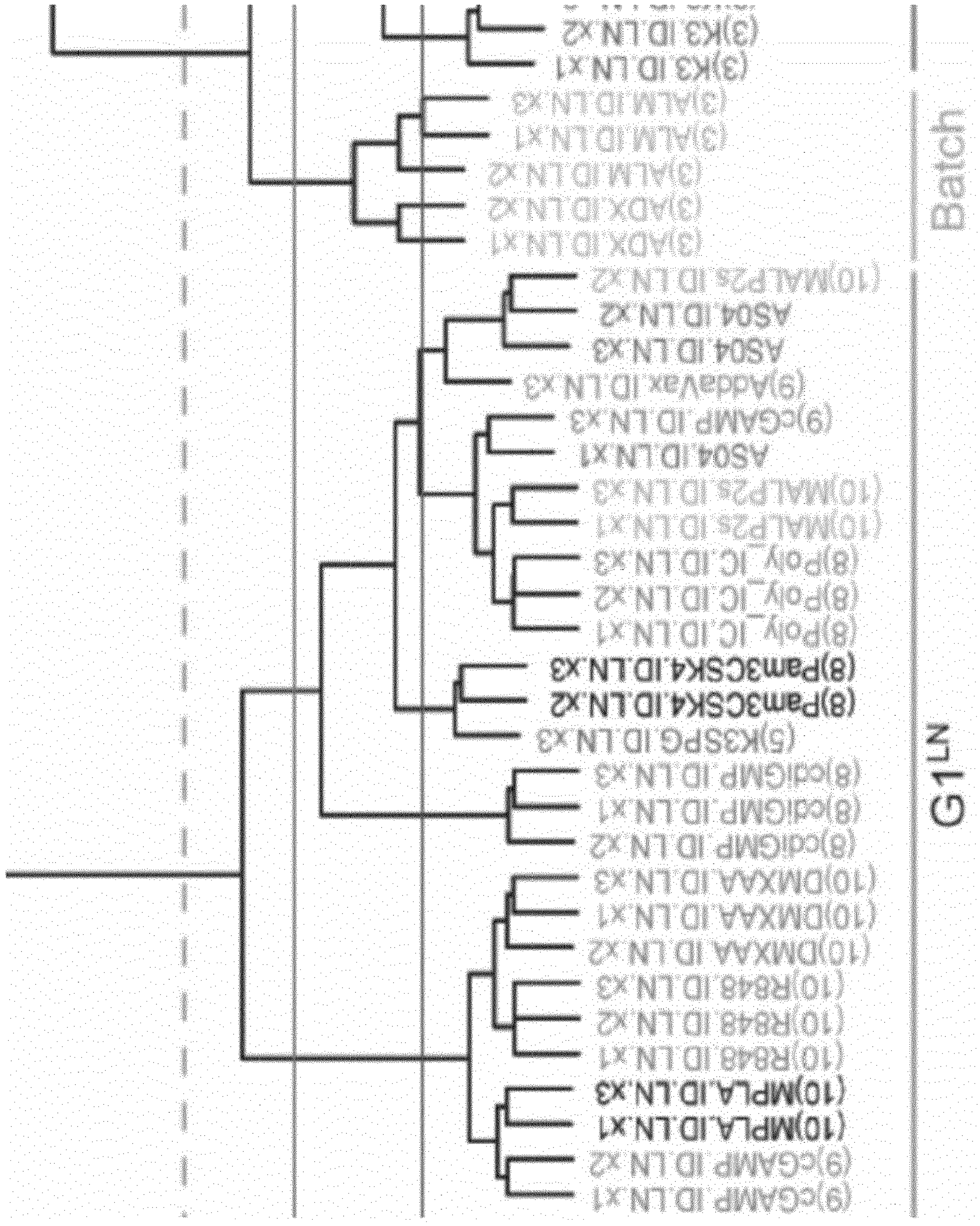
(3)K3.ID.LN.x2
(3)K3.ID.LN.x1
(3)ALM.ID.LN.x3
(3)ALM.ID.LN.x1
(3)ALM.ID.LN.x2
(3)ADX.ID.LN.x2
(3)ADX.ID.LN.x1
(10)MALP2s.ID.LN.x2
AS04.ID.LN.x2
AS04.ID.LN.x3
(9)AddaVax.ID.LN.x3
(9)cGAMP.ID.LN.x3
AS04.ID.LN.x1
(10)MALP2s.ID.LN.x3
(10)MALP2s.ID.LN.x1
(8)Poly_IC.ID.LN.x3
(8)Poly_IC.ID.LN.x2
(8)Poly_IC.ID.LN.x1
(8)Pam3CSK4.ID.LN.x3
(8)Pam3CSK4.ID.LN.x2
(5)K3SPG.ID.LN.x3
(8)cdiGMP.ID.LN.x3
(8)cdiGMP.ID.LN.x1
(8)cdiGMP.ID.LN.x2
(10)DMXAA.ID.LN.x3
(10)DMXAA.ID.LN.x1
(10)DMXAA.ID.LN.x2
(10)R848.ID.LN.x3
(10)R848.ID.LN.x2
(10)R848.ID.LN.x1
(10)MPLA.ID.LN.x3
(10)MPLA.ID.LN.x1
(9)cGAMP.ID.LN.x2
(9)cGAMP.ID.LN.x1
Batch
G1$^{LN}$

[FIG.20I]

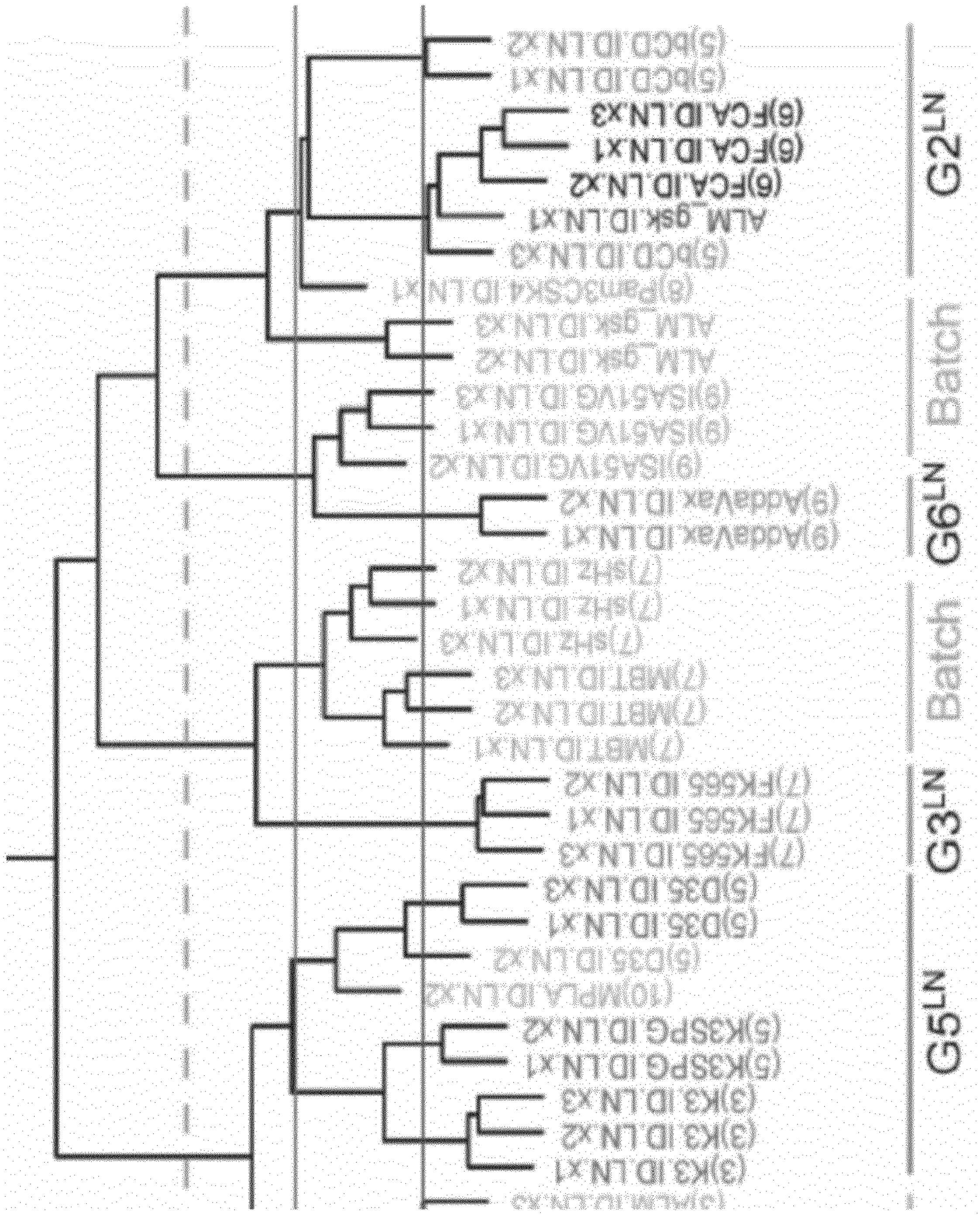
(5)bCD.ID.LN.x2
(5)bCD.ID.LN.x1
(6)FCA.ID.LN.x3
(6)FCA.ID.LN.x1
(6)FCA.ID.LN.x2
ALM_gsk.ID.LN.x1
(5)bCD.ID.LN.x3
(8)Pam3CSK4.ID.LN.x1
ALM_gsk.ID.LN.x3
ALM_gsk.ID.LN.x2
(9)ISA51VG.ID.LN.x3
(9)ISA51VG.ID.LN.x1
(9)ISA51VG.ID.LN.x2
(9)AddaVax.ID.LN.x2
(9)AddaVax.ID.LN.x1
(7)sHz.ID.LN.x2
(7)sHz.ID.LN.x1
(7)sHz.ID.LN.x3
(7)MBT.ID.LN.x3
(7)MBT.ID.LN.x2
(7)MBT.ID.LN.x1
(7)FK565.ID.LN.x2
(7)FK565.ID.LN.x1
(7)FK565.ID.LN.x3
(5)D35.ID.LN.x3
(5)D35.ID.LN.x1
(5)D35.ID.LN.x2
(10)MPLA.ID.LN.x2
(5)K3SPG.ID.LN.x2
(5)K3SPG.ID.LN.x1
(3)K3.ID.LN.x3
(3)K3.ID.LN.x2
(3)K3.ID.LN.x1
G2LN
Batch
G6LN
Batch
G3LN
G5LN

[FIG.21]

| i.d. (i.n.) | (local) LN | (systemic) LV | SP |
|---|---|---|---|
| ALM | Batch | Batch | Batch |
| ISA51VG | Batch | Batch | Batch |
| MBT | Batch | Batch | Batch |
| sHz | Batch | Batch | Batch |
| MPLA | G1 | Batch | Batch |
| D35 | G5 | Batch | Batch |
| K3 | G5 | Batch | Batch |
| K3SPG | G5 | Batch | Batch |
| AddaVax | G6 | Batch | Batch |
| cdiGMP | G1 | G1 | G1 |
| cGAMP | G1 | G1 | G1 |
| DMXAA | G1 | G1 | G1 |
| Poly_IC | G1 | G1 | G1 |
| R848 | G1 | G1 | G1 |
| bCD | G2 | G2 | G2 |
| ENDCN(i.n.) | | G2 | G2 |
| FCA | G2 | G2 | G3 |
| ADX | Batch | G3 | G4 |
| Pam3CSK4 | G1 | G3 | G3 |
| FK565 | G3 | G3 | G3 |
| MALP2s | G1 | G4 | G4 |

| | | | |
|---|---|---|---|
| ALM_gsk | Batch(G2) | Batch | Batch |
| AS04 | G1 | G2 | AS04 |

| i.p. | (systemic) LV | SP |
|---|---|---|
| ALM | G2 | G2 |
| | | |
| | | |
| | | |
| | | |
| | | |
| K3 | Batch | Batch |
| K3SPG | Batch | Batch |
| | | |
| | | |
| | | |
| | | |
| | | |
| | | |
| bCD | Batch | Batch |
| | | |
| | | |
| ADX | G3 | G4 |

[FIG.22A]

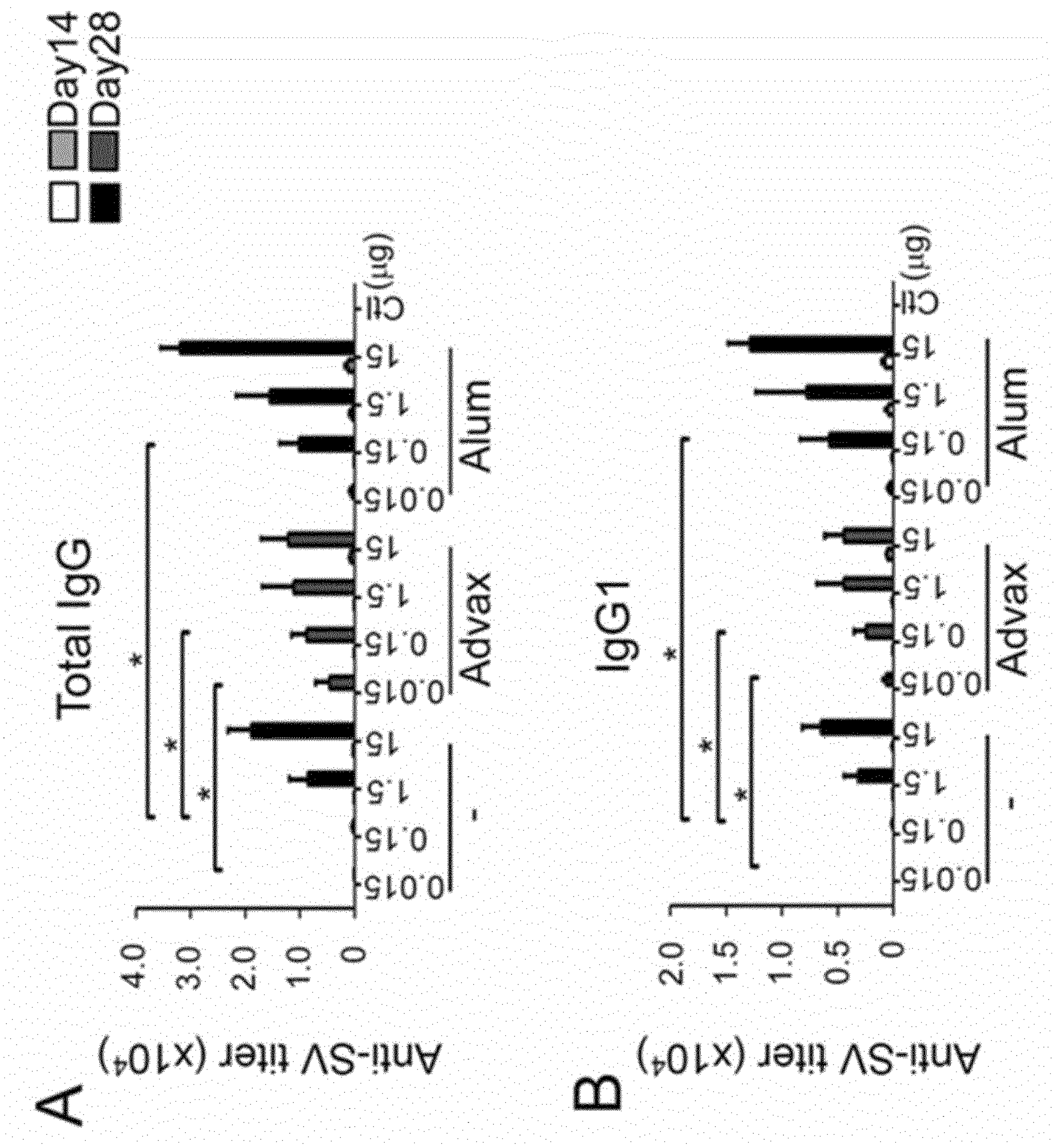
A
Total IgG
Anti-SV titer (x10⁴)
4.0
3.0
2.0
1.0
0
0.015
0.15
1.5
15
-
Advax
Alum
Ctl
(μg)
Day14
Day28
B
IgG1
2.0
1.5
1.0
0.5
*

[FIG.22B]

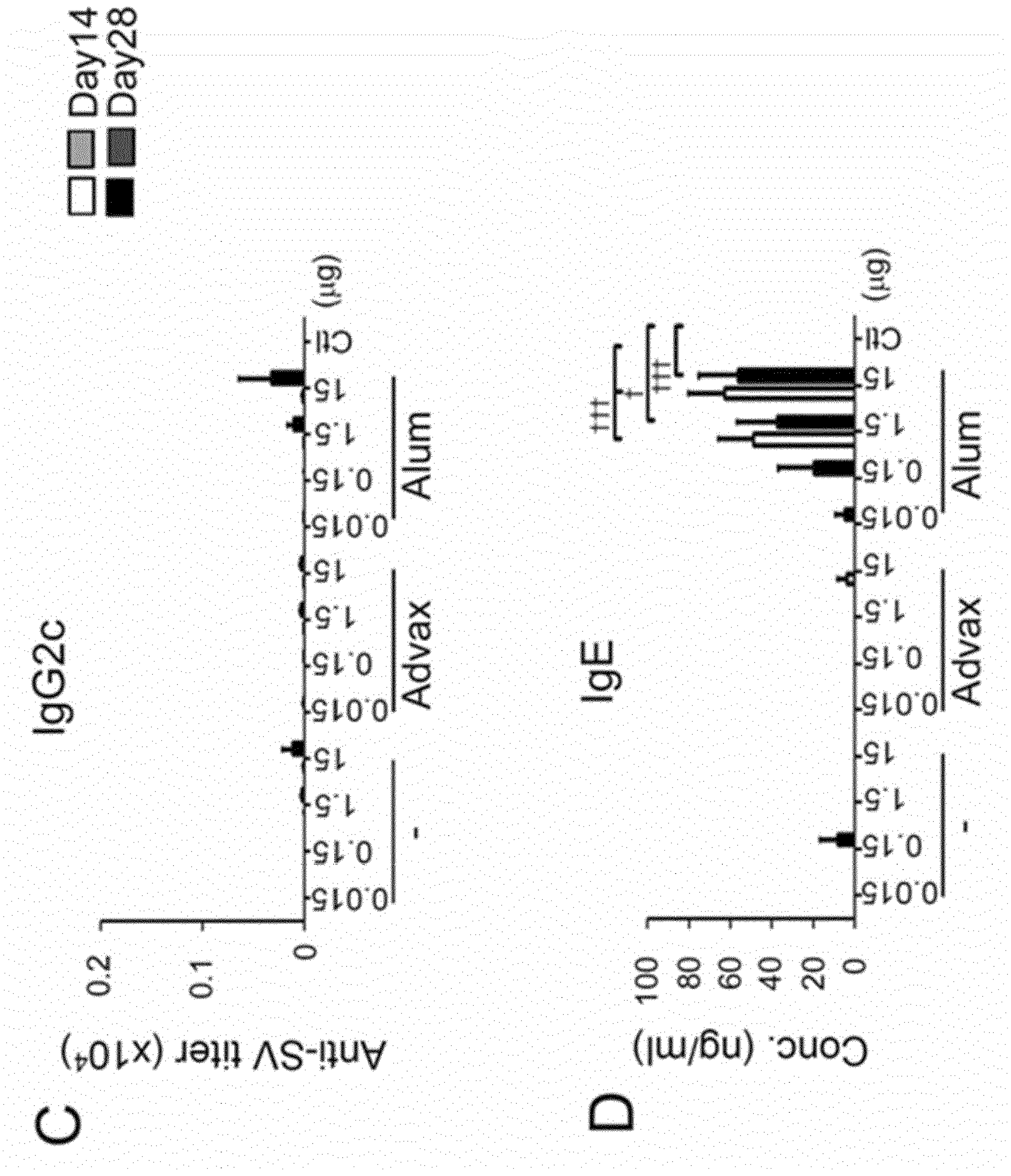
C
IgG2c
Anti-SV titer ($x10^4$)
0.2
0.1
0
0.015
0.15
1.5
15
Ctl
(µg)
-
Advax
Alum
Day14
Day28
D
IgE
Conc. (ng/ml)
100
80
60
40
20
0

[FIG.22C]

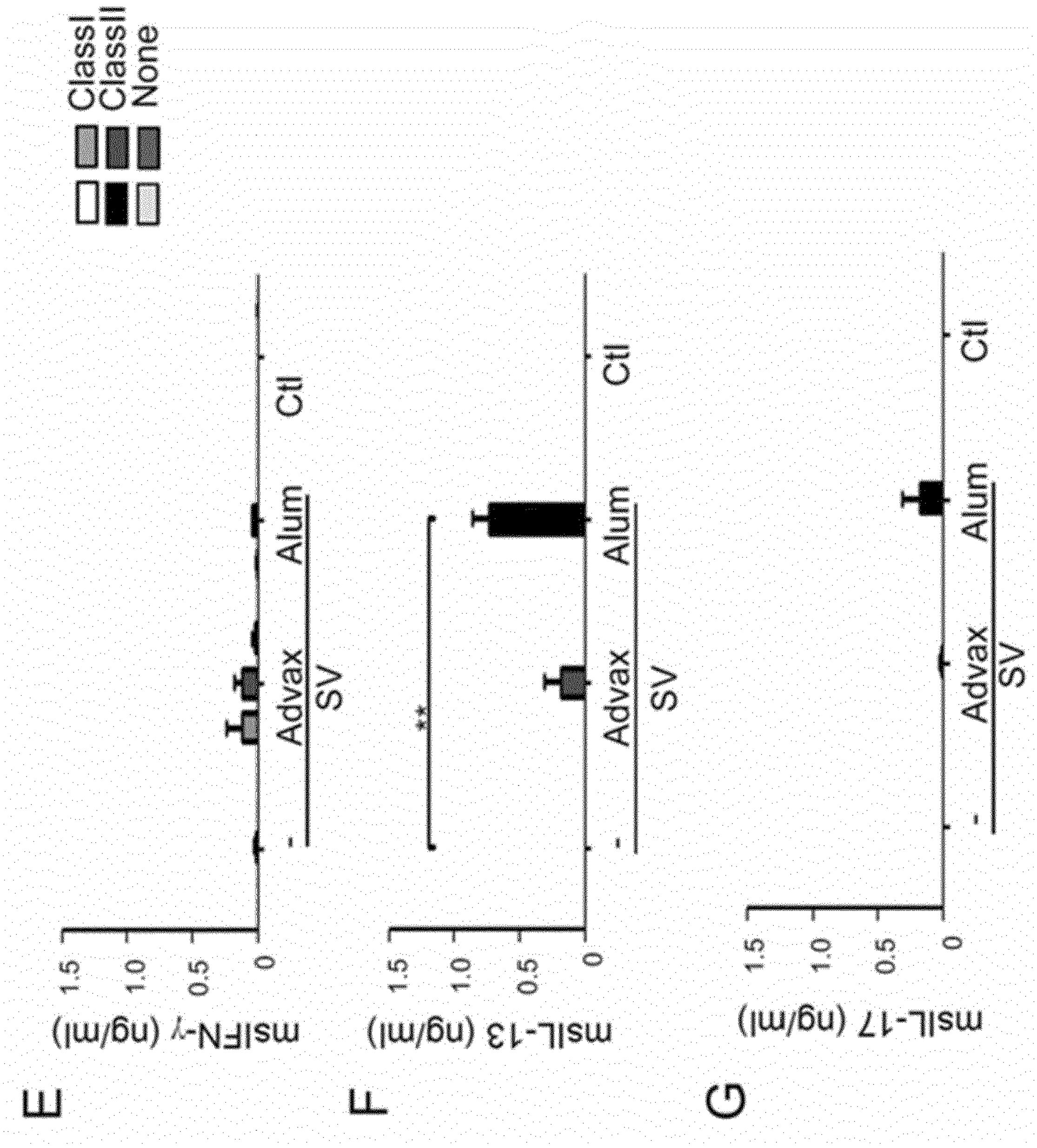
E
msIFN-γ (ng/ml)
F
msIL-13 (ng/ml)
G
msIL-17 (ng/ml)
0
0.5
1.0
1.5
-
Advax
Alum
Ctl
SV
**
ClassI
ClassII
None

[FIG.23A]

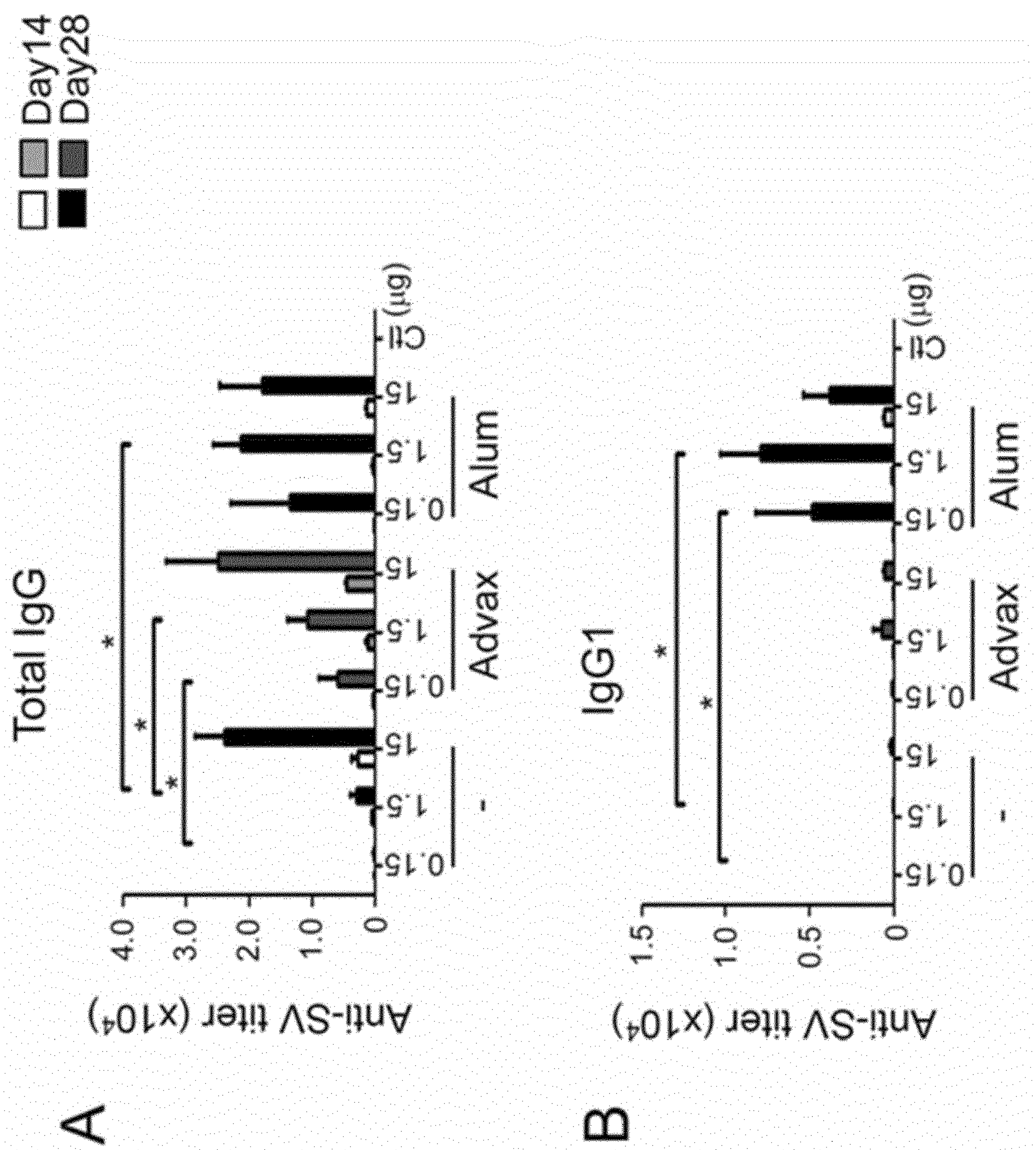
A
Total IgG
Anti-SV titer (x10^4)
4.0
3.0
2.0
1.0
0
0.15
1.5
15
-
Advax
Alum
Ctl
(μg)
*
Day14
Day28
B
IgG1
1.5
1.0
0.5

[FIG.23B]

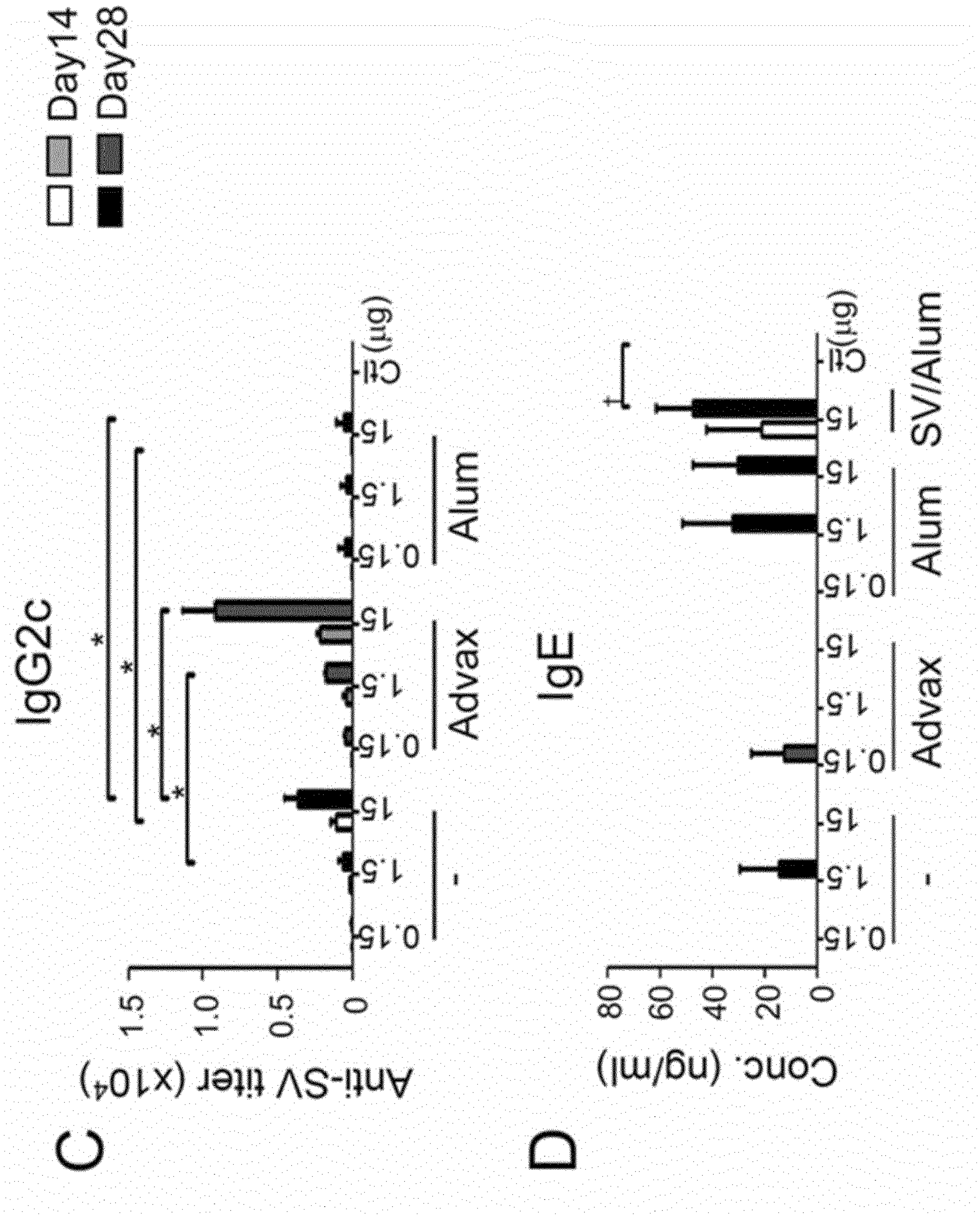
C
IgG2c
Anti-SV titer (x10⁴)
1.5
1.0
0.5
0
0.15
1.5
15
0.15
1.5
15
0.15
1.5
15
Ctl
(μg)
-
Advax
Alum
*
Day14
Day28
D
IgE
Conc. (ng/ml)
80
60
40
20
0
SV/Alum

[FIG.23C]

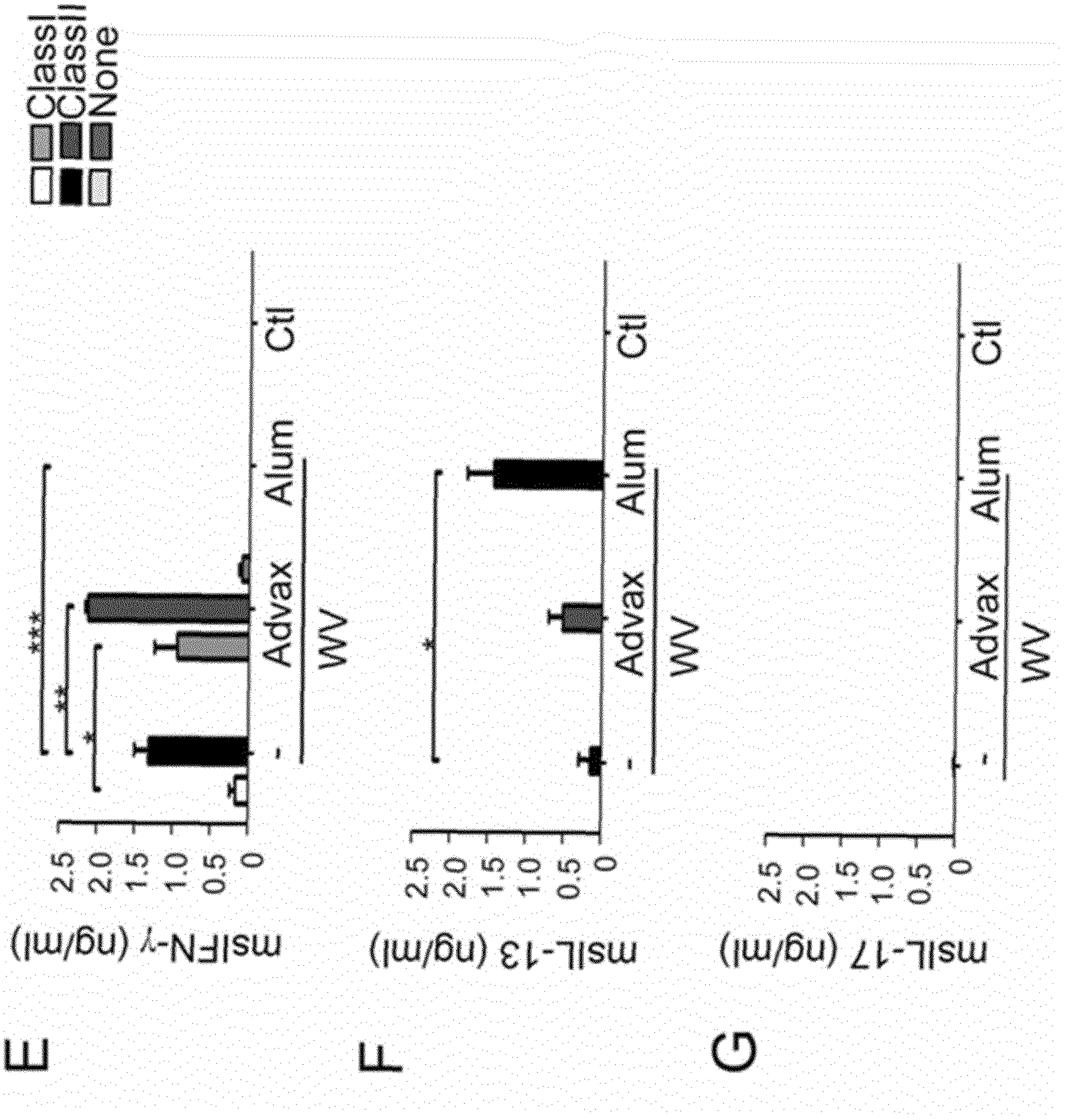
E
msIFN-γ (ng/ml)
2.5
2.0
1.5
1.0
0.5
0
*
**
***
-
Advax
Alum
Ctl
WV
ClassI
ClassII
None
F
msIL-13 (ng/ml)
2.5
2.0
1.5
1.0
0.5
0
*
-
Advax
Alum
Ctl
WV
G
msIL-17 (ng/ml)
2.5
2.0
1.5
1.0
0.5
0
-
Advax
Alum
Ctl
WV

[FIG.24]

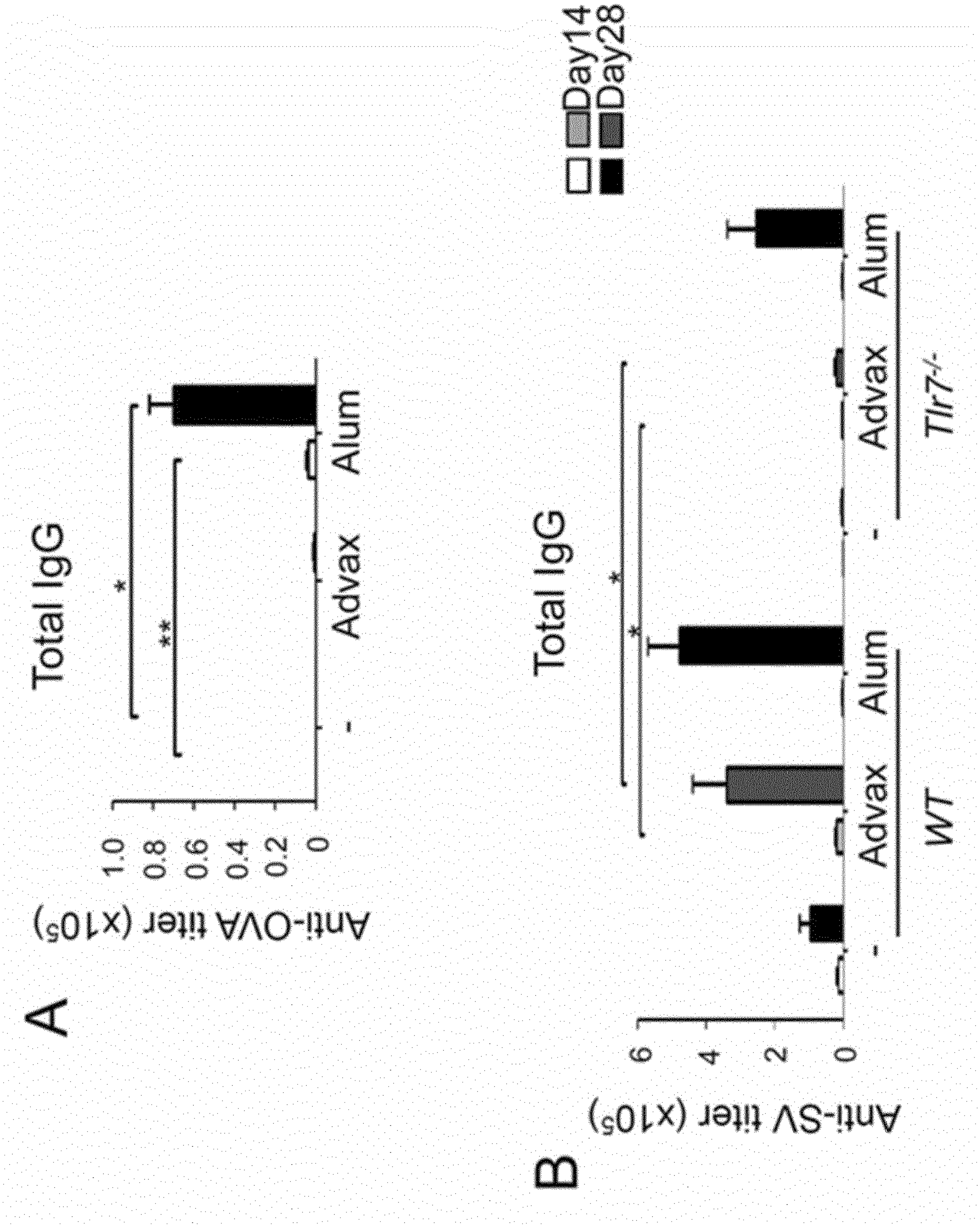
A
Total IgG
Anti-OVA titer (x10⁵)
1.0
0.8
0.6
0.4
0.2
0
*
**
-
Advax
Alum
B
Total IgG
Anti-SV titer (x10⁵)
6
4
2
0
*
*
Day14
Day28
-
Advax
Alum
WT
-
Advax
Alum
Tlr7-/-

[FIG.25]

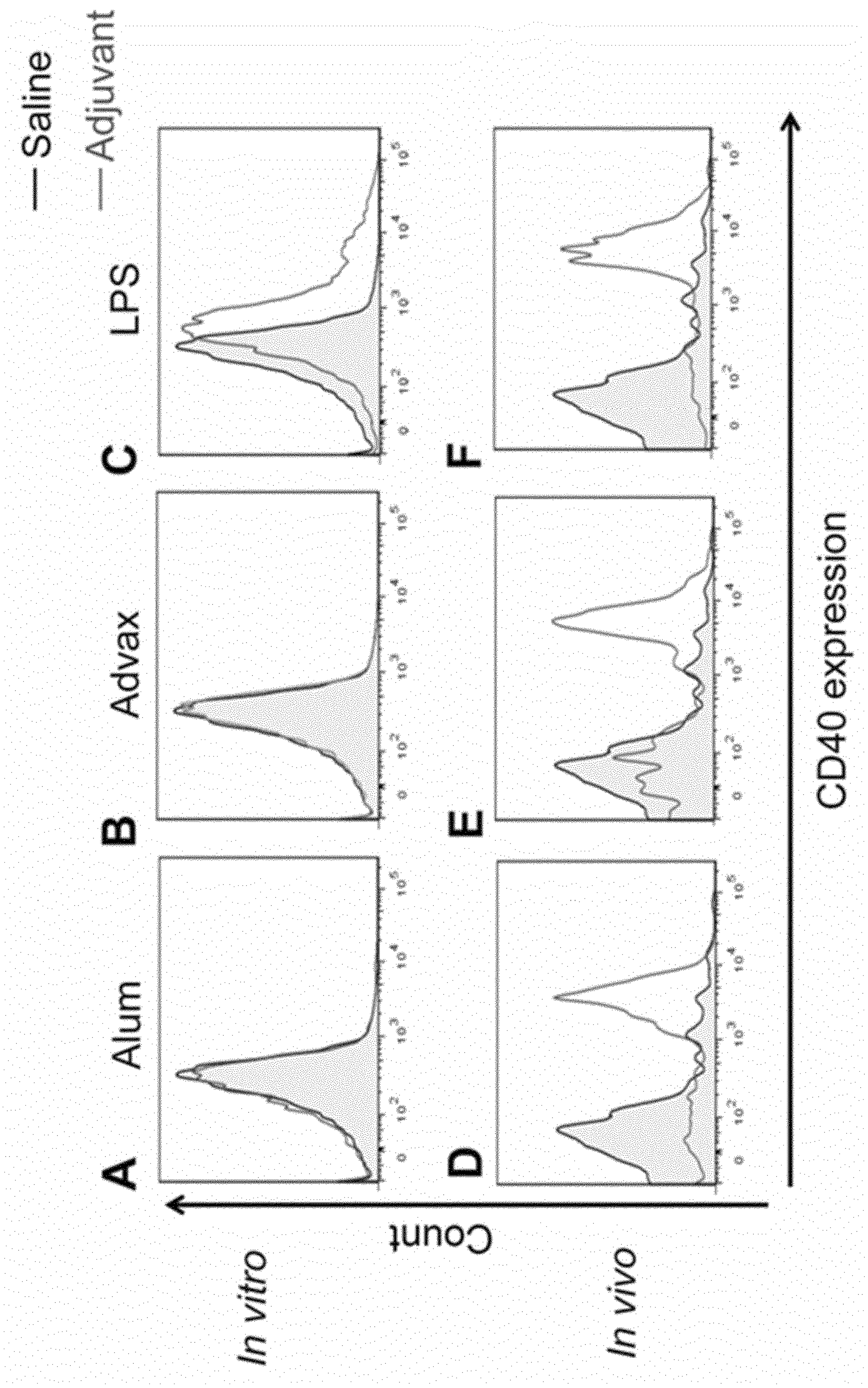
Saline
Adjuvant
A
Alum
B
Advax
C
LPS
D
E
F
Count
In vitro
In vivo
CD40 expression

[FIG.26A]

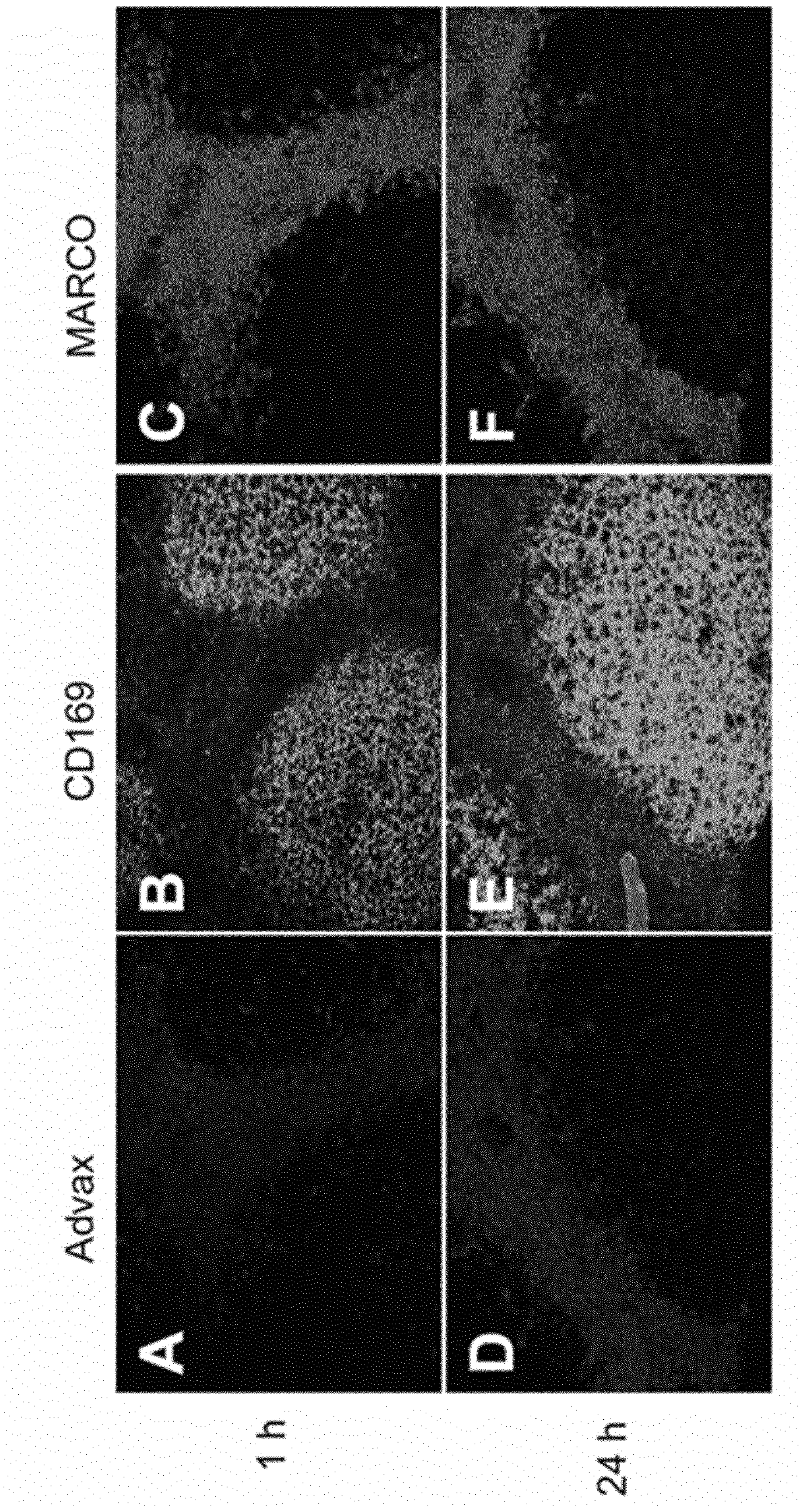

[FIG.26B]

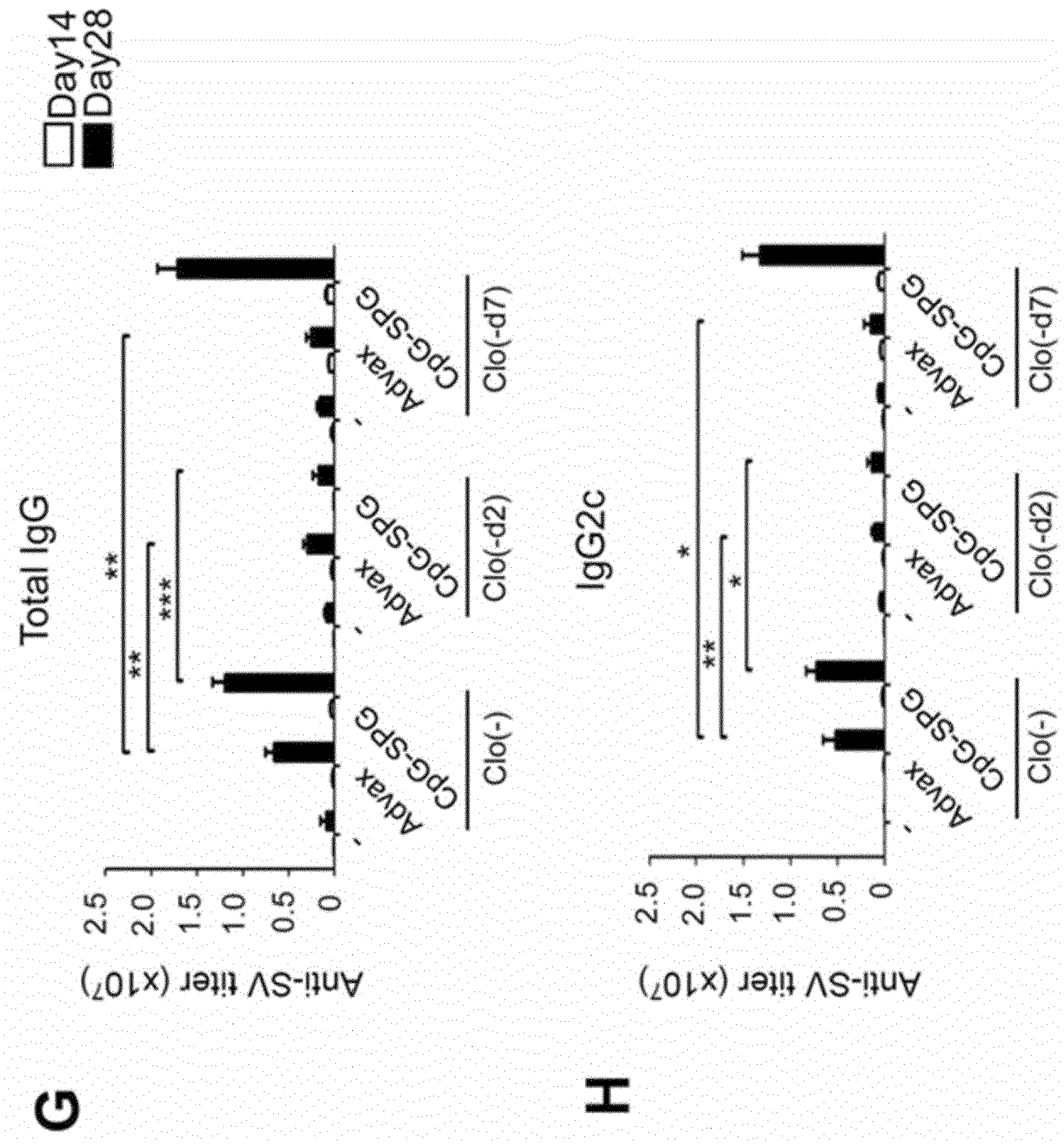
G
Total IgG
Anti-SV titer (x10⁷)
2.5
2.0
1.5
1.0
0.5
0
-
Advax
CpG-SPG
Clo(-)
Clo(-d2)
Clo(-d7)
**
**
***
Day14
Day28
H
IgG2c
*
**
*

[FIG.27A]

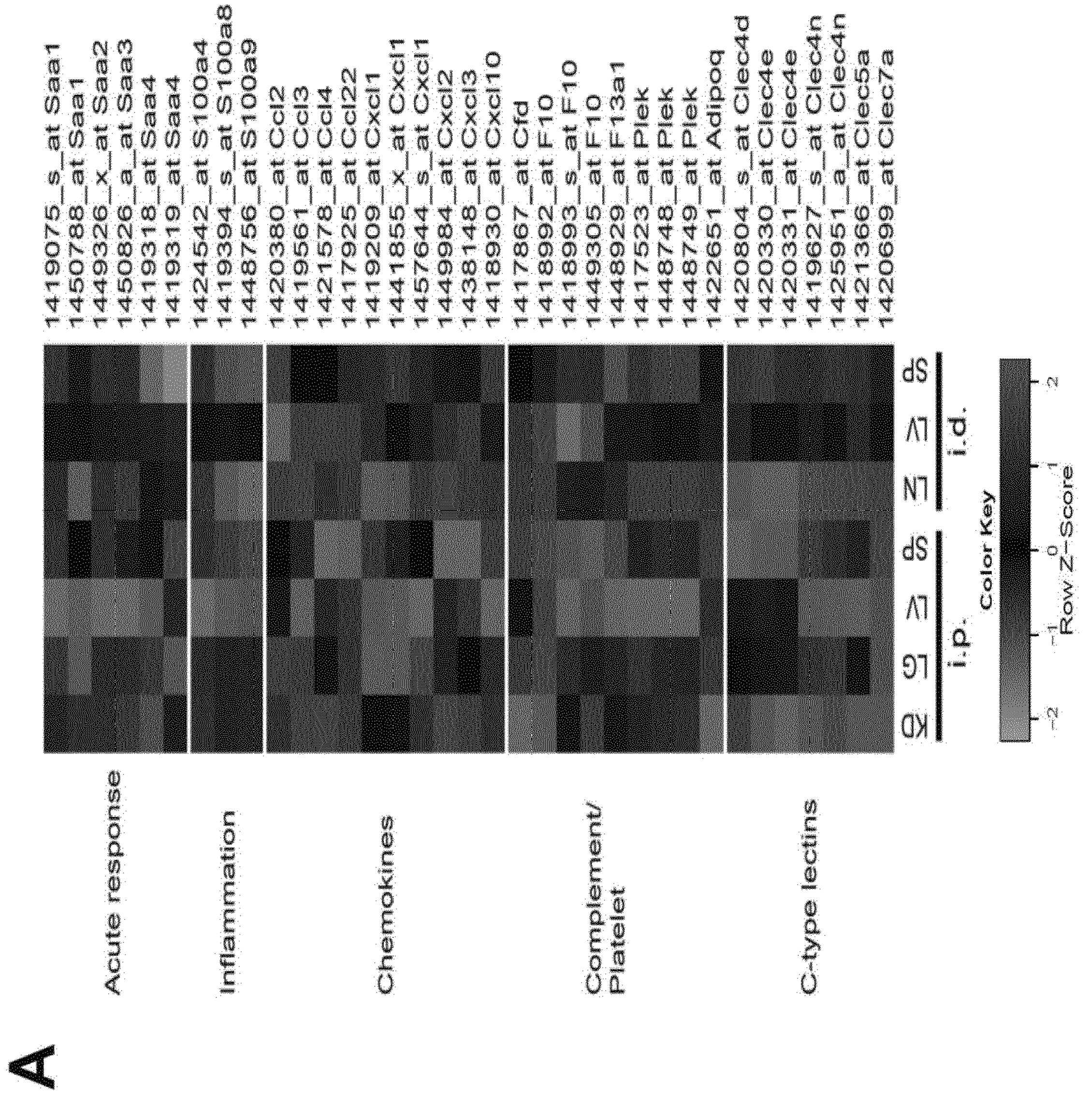
A
Acute response
Inflammation
Chemokines
Complement/
Platelet
C-type lectins
1419075_s_at Saa1
1450788_at Saa1
1449326_x_at Saa2
1450826_a_at Saa3
1419318_at Saa4
1419319_at Saa4
1424542_at S100a4
1419394_s_at S100a8
1448756_at S100a9
1420380_at Ccl2
1419561_at Ccl3
1421578_at Ccl4
1417925_at Ccl22
1419209_at Cxcl1
1441855_x_at Cxcl1
1457644_s_at Cxcl1
1449984_at Cxcl2
1438148_at Cxcl3
1418930_at Cxcl10
1417867_at Cfd
1418992_at F10
1418993_s_at F10
1449305_at F10
1448929_at F13a1
1417523_at Plek
1448748_at Plek
1448749_at Plek
1422651_at Adipoq
1420804_s_at Clec4d
1420330_at Clec4e
1420331_at Clec4e
1419627_s_at Clec4n
1425951_a_at Clec4n
1421366_at Clec5a
1420699_at Clec7a
KD
LG
LV
SP
LN
LV
SP
i.p.
i.d.
Color Key
Row Z-Score
−2
−1
0
1
2

[FIG.27B]

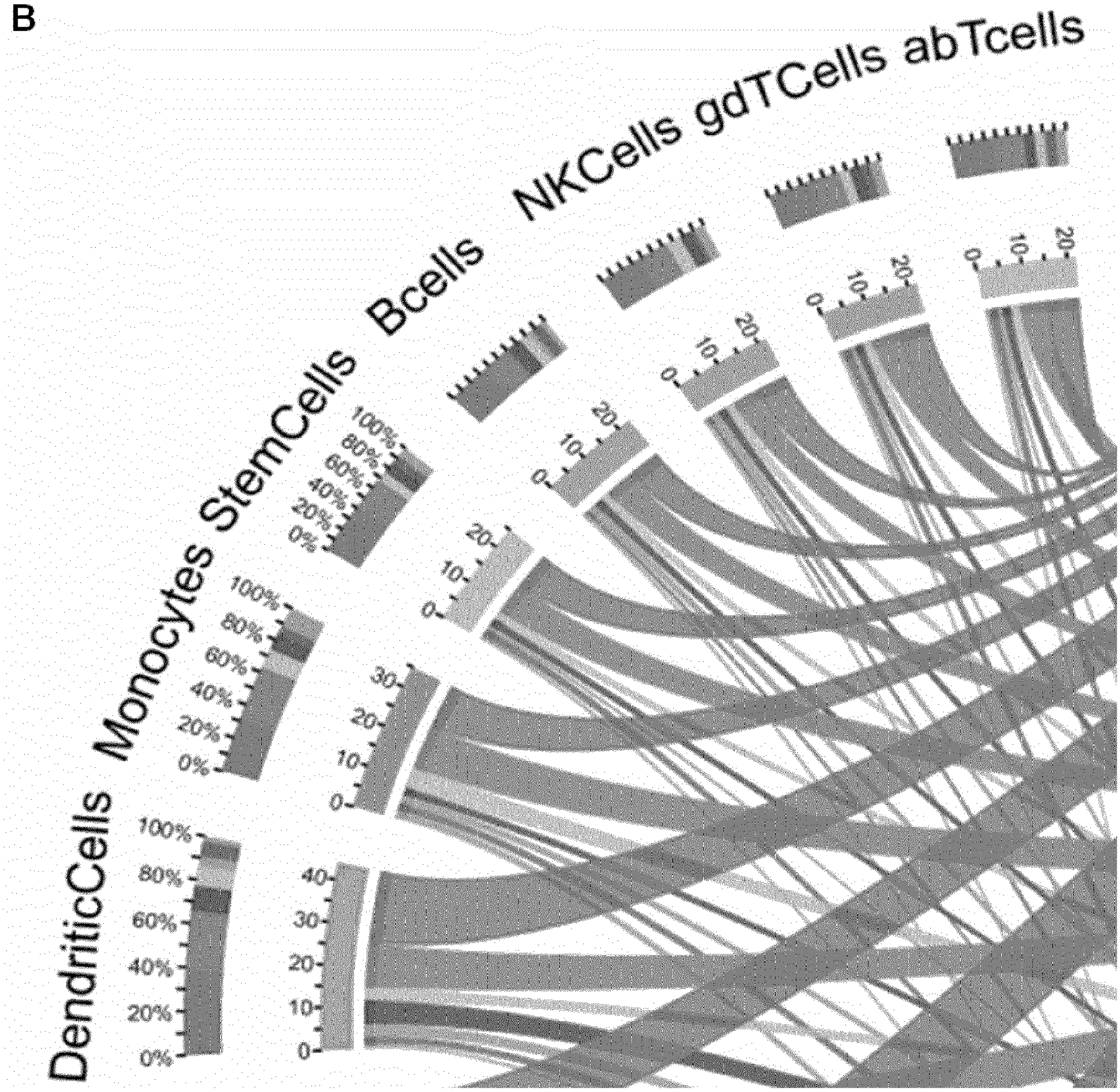
B
DendriticCells
Monocytes
StemCells
Bcells
NKCells
gdTCells
abTcells
100%
80%
60%
40%
20%
0%
0
10
20
30
40

[FIG.27B](**Continue**)

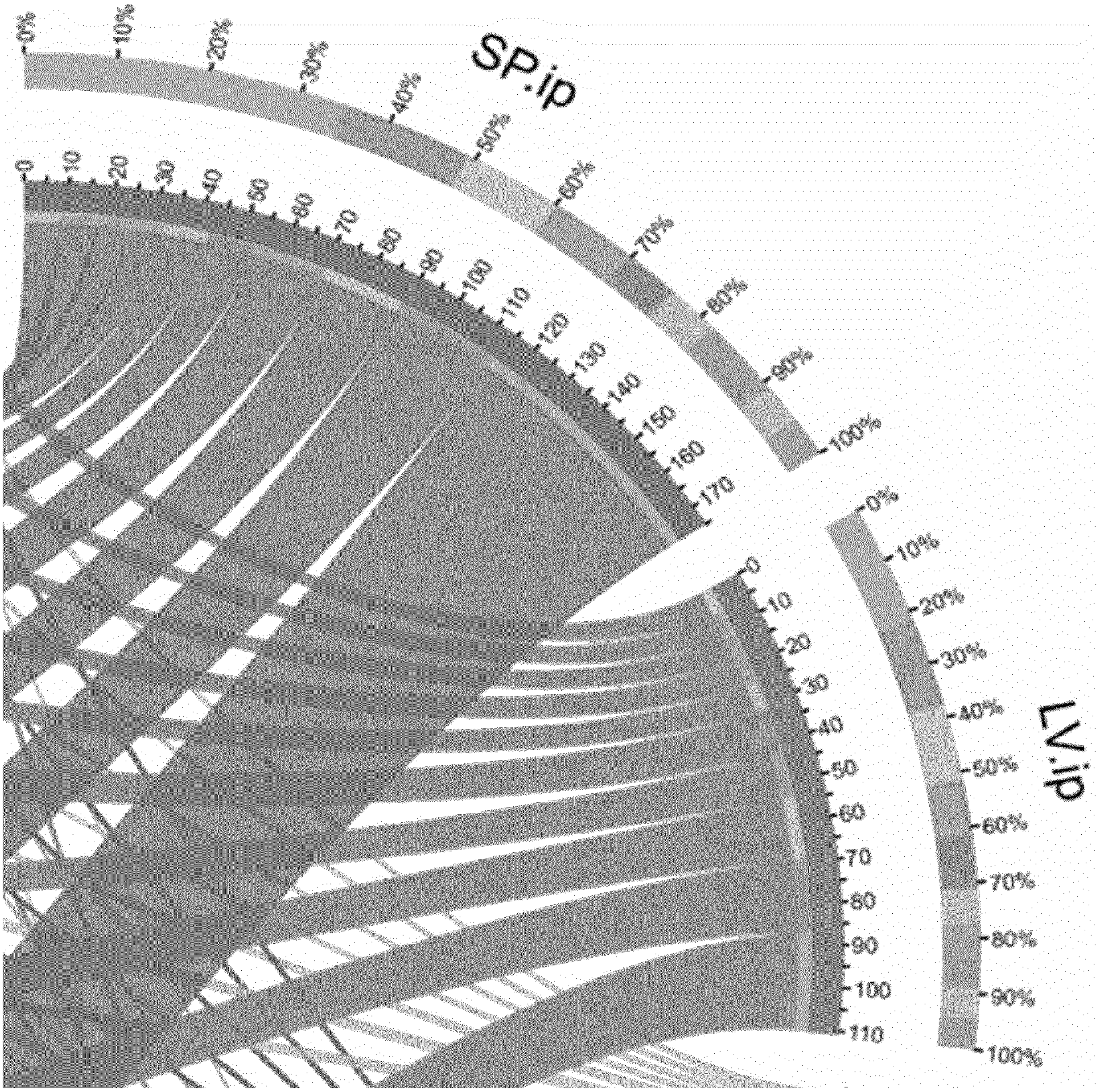

[FIG.27B](**Continue**)

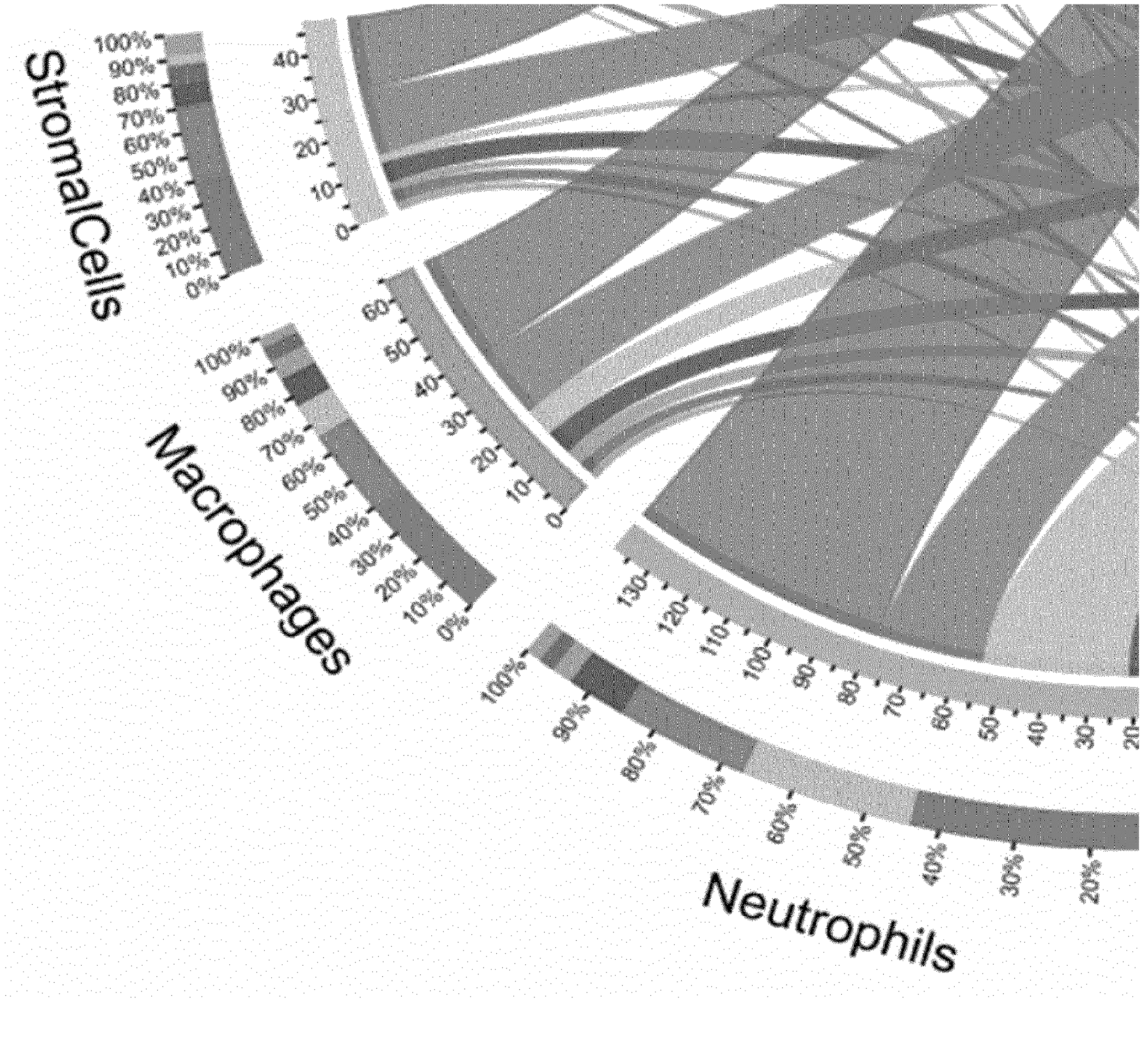

[FIG.27B](**Continue**)

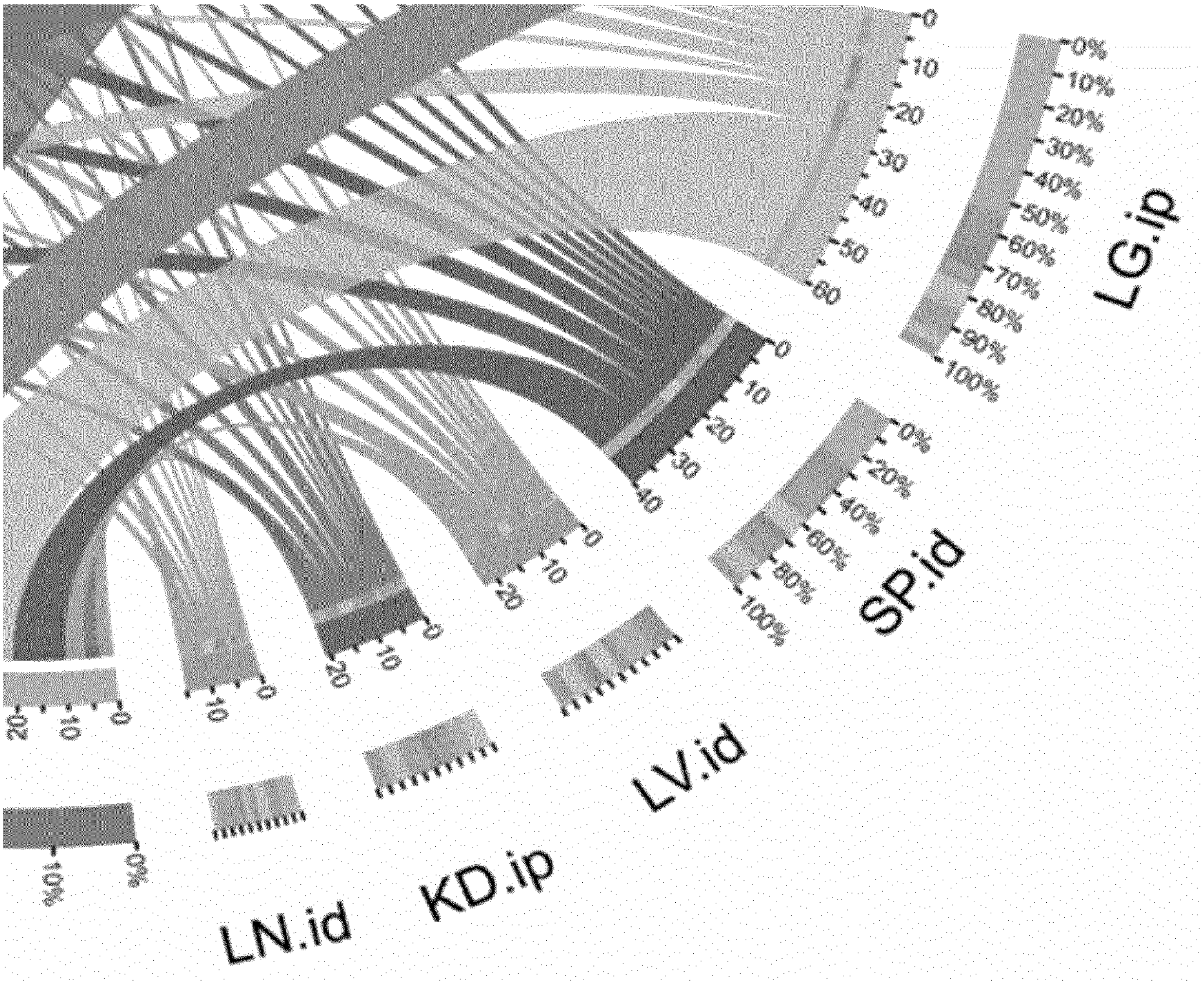

[FIG.27C]

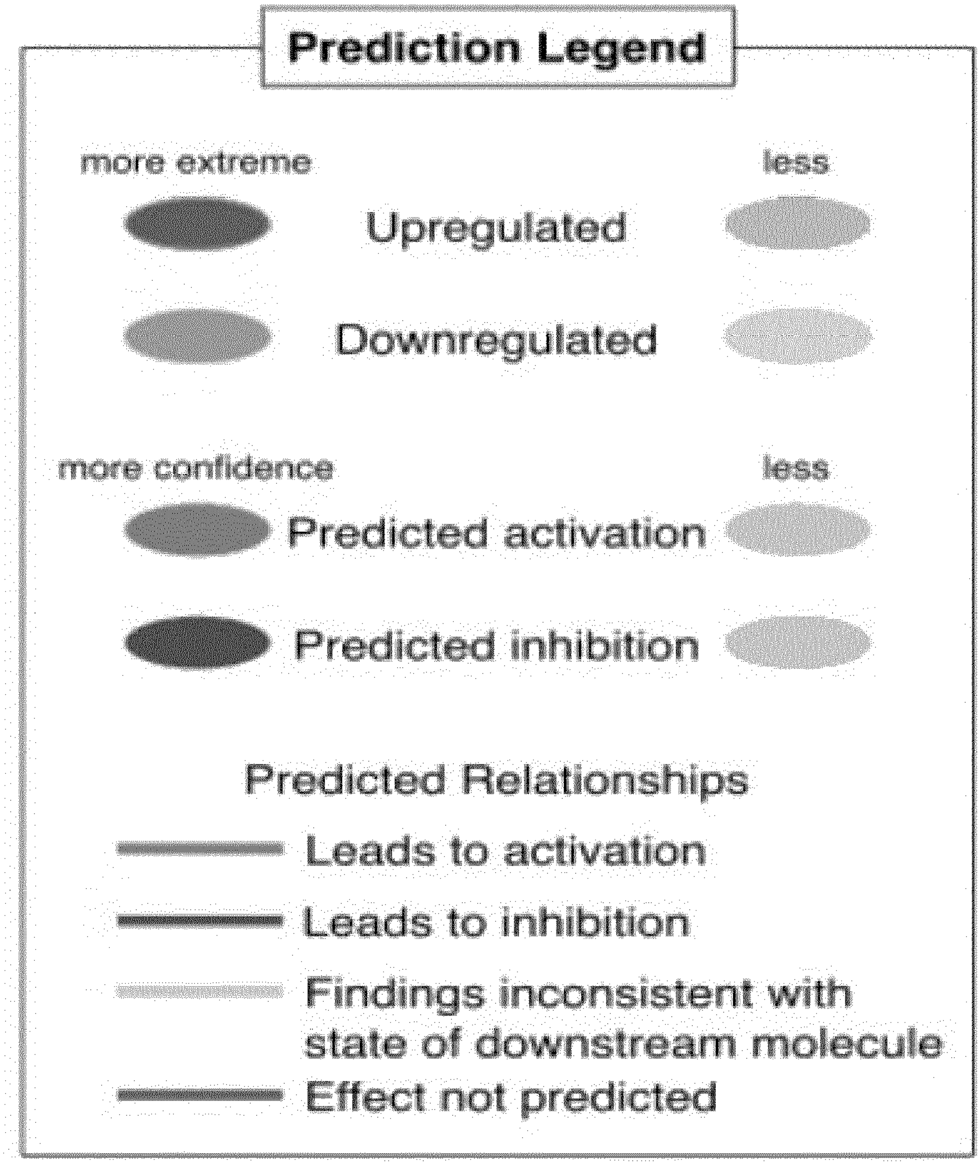
Prediction Legend
more extreme
less
Upregulated
Downregulated
more confidence
less
Predicted activation
Predicted inhibition
Predicted Relationships
Leads to activation
Leads to inhibition
Findings inconsistent with
state of downstream molecule
Effect not predicted

[FIG.27C](**Continue**)

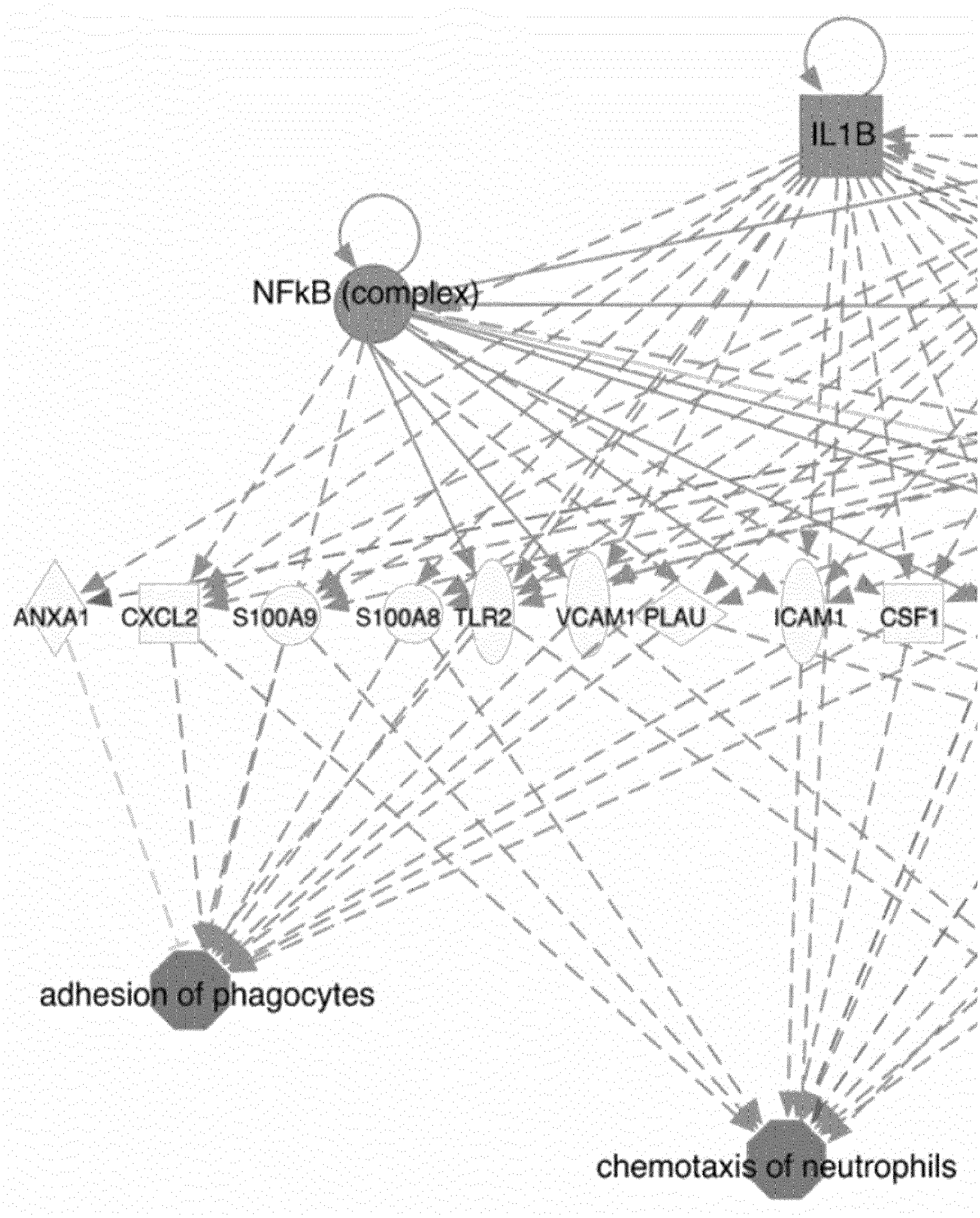

[FIG.27C](**Continue**)

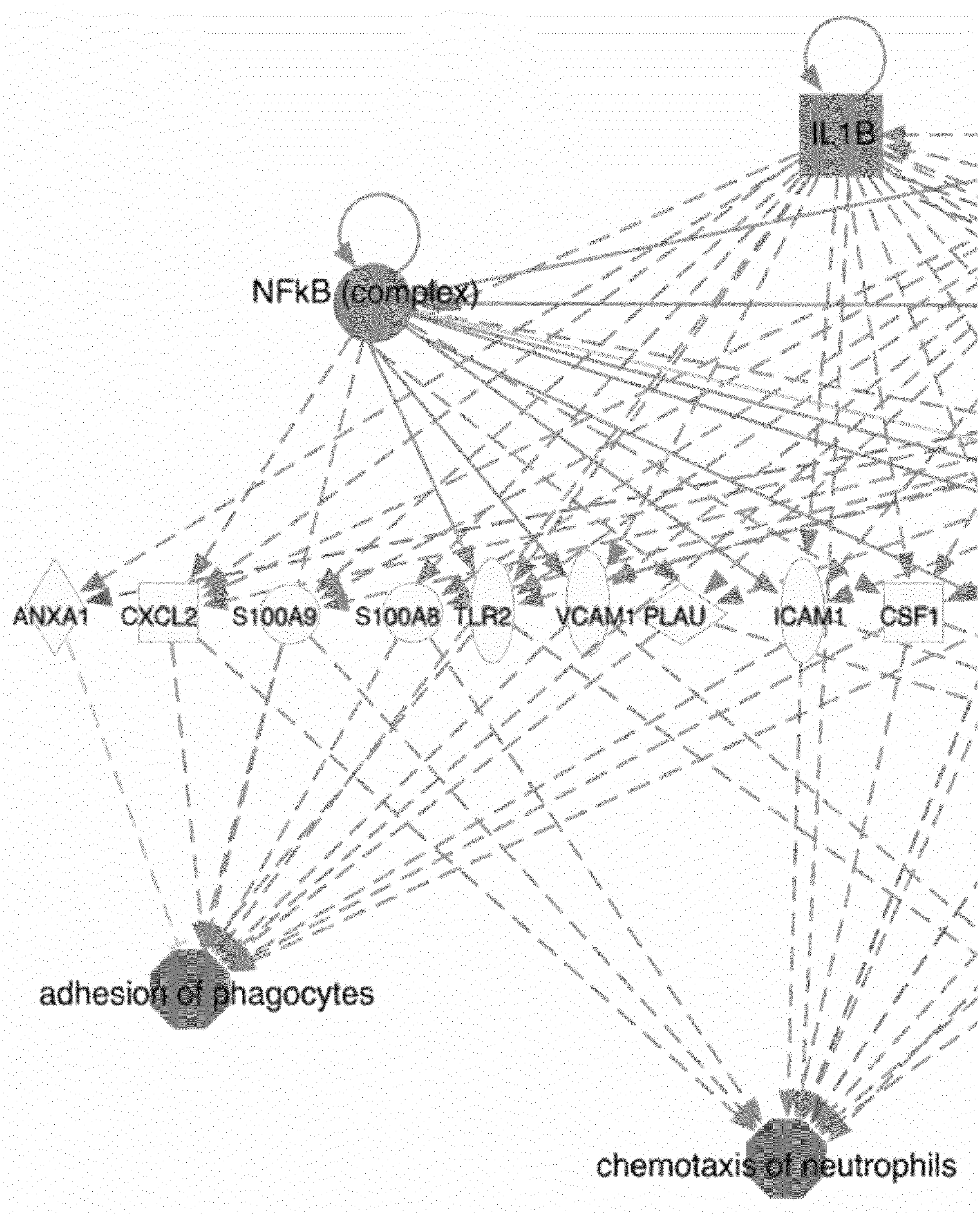

[FIG.27D]

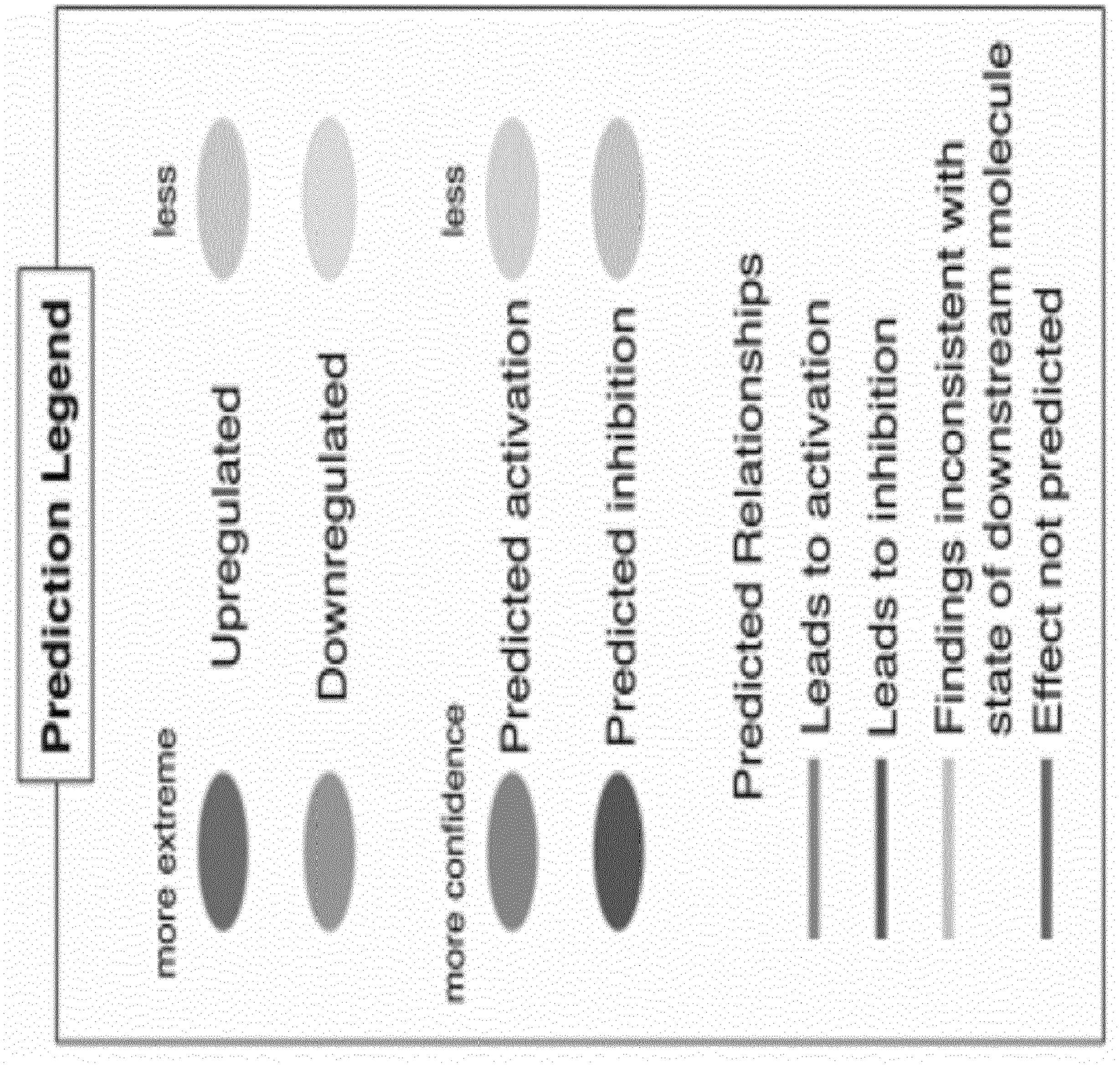
Prediction Legend
more extreme
less
Upregulated
Downregulated
more confidence
less
Predicted activation
Predicted inhibition
Predicted Relationships
Leads to activation
Leads to inhibition
Findings inconsistent with state of downstream molecule
Effect not predicted

[FIG.27E]

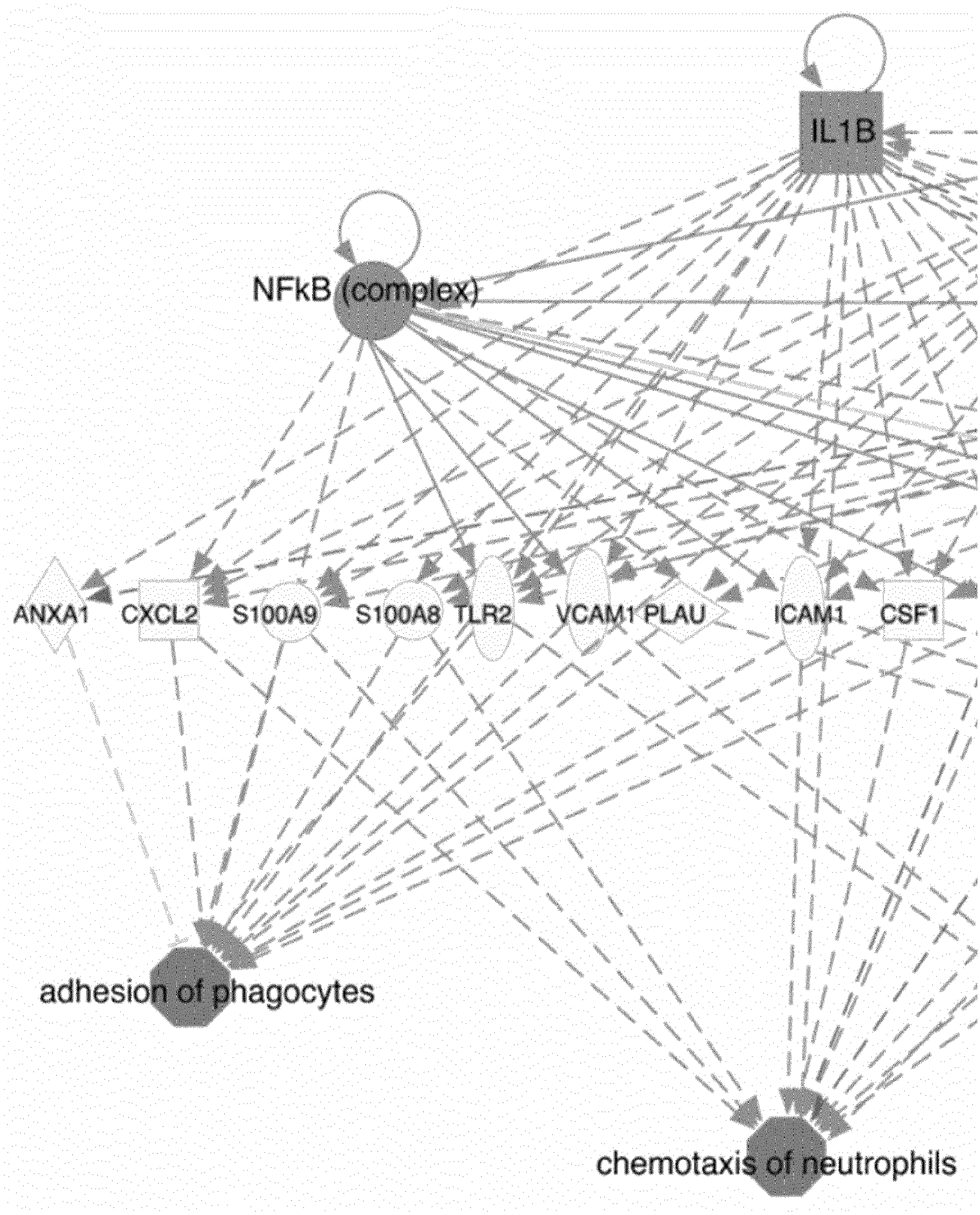
IL1B
NFkB (complex)
ANXA1
CXCL2
S100A9
S100A8
TLR2
VCAM1
PLAU
ICAM1
CSF1
adhesion of phagocytes
chemotaxis of neutrophils

[FIG.27E](**Continue**)

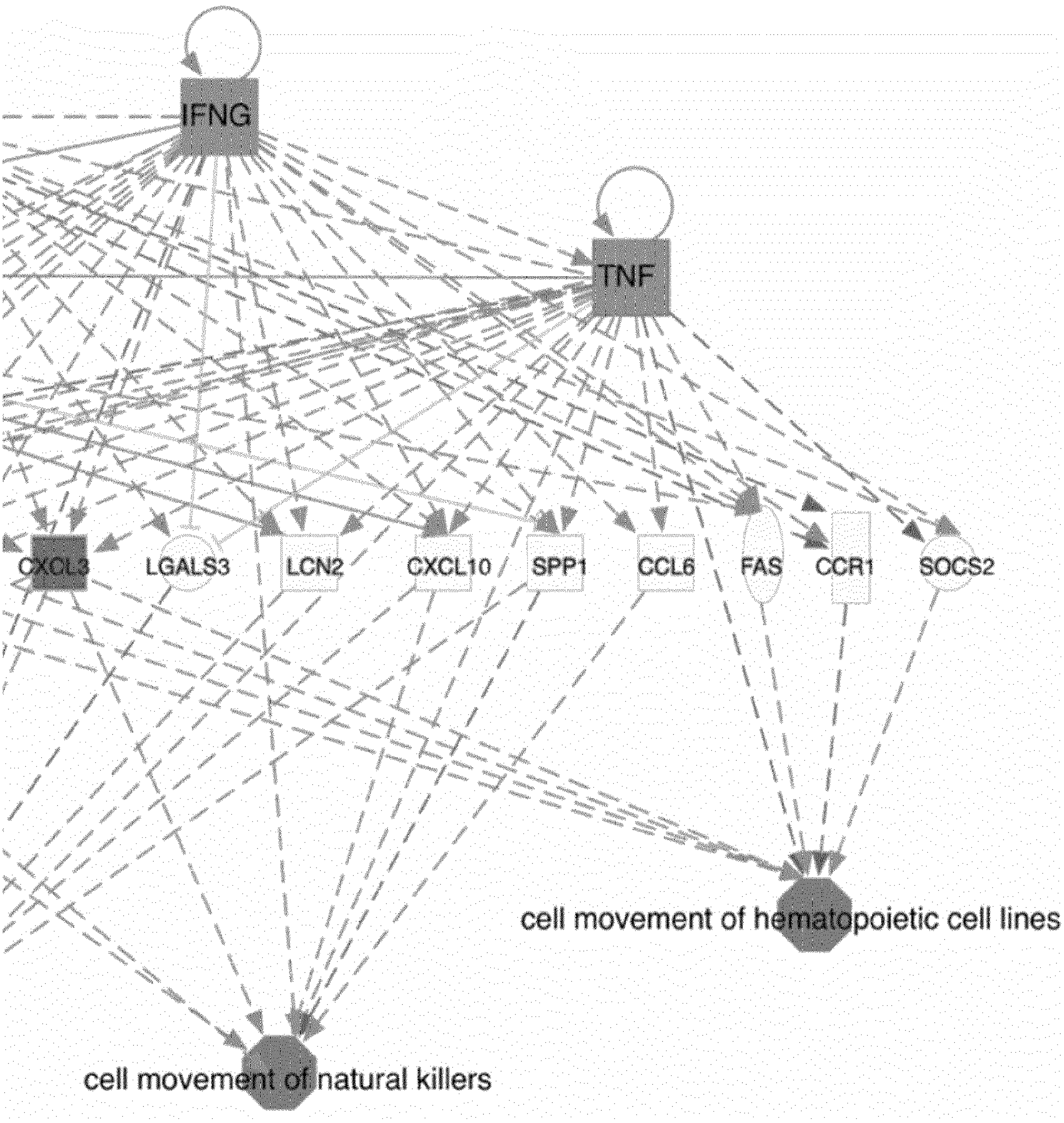

[FIG.28A]

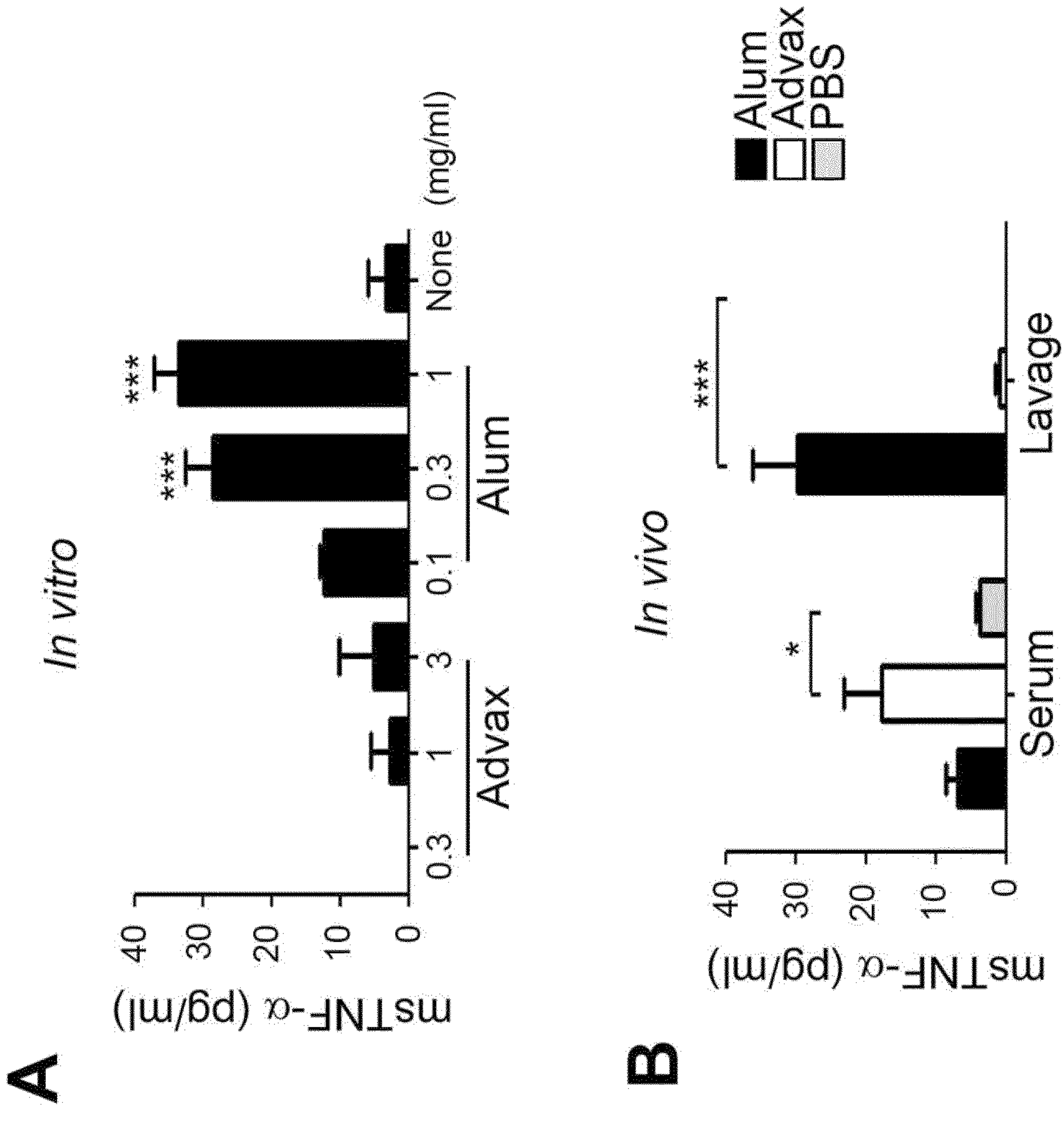
A
In vitro
msTNF-α (pg/ml)
40
30
20
10
0
***
***
0.3
1
3
0.1
0.3
1
None
(mg/ml)
Advax
Alum
B
In vivo
msTNF-α (pg/ml)
40
30
20
10
0
*
***
Serum
Lavage
Alum
Advax
PBS

[FIG.28B]

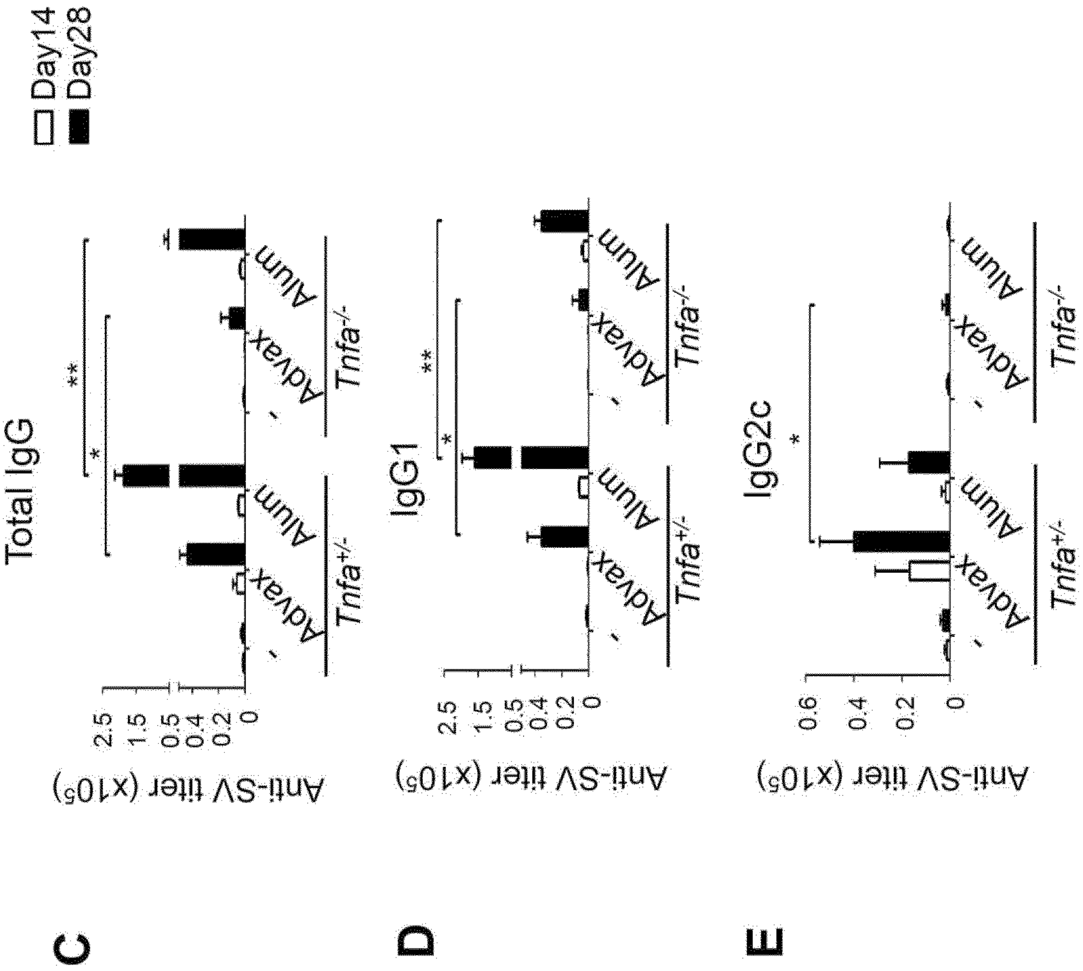
C
Total IgG
D
IgG1
E
IgG2c
Anti-SV titer (x10⁵)
Day14
Day28
Advax
Alum
Tnfa+/-
Tnfa-/-
*
**

[FIG.29]

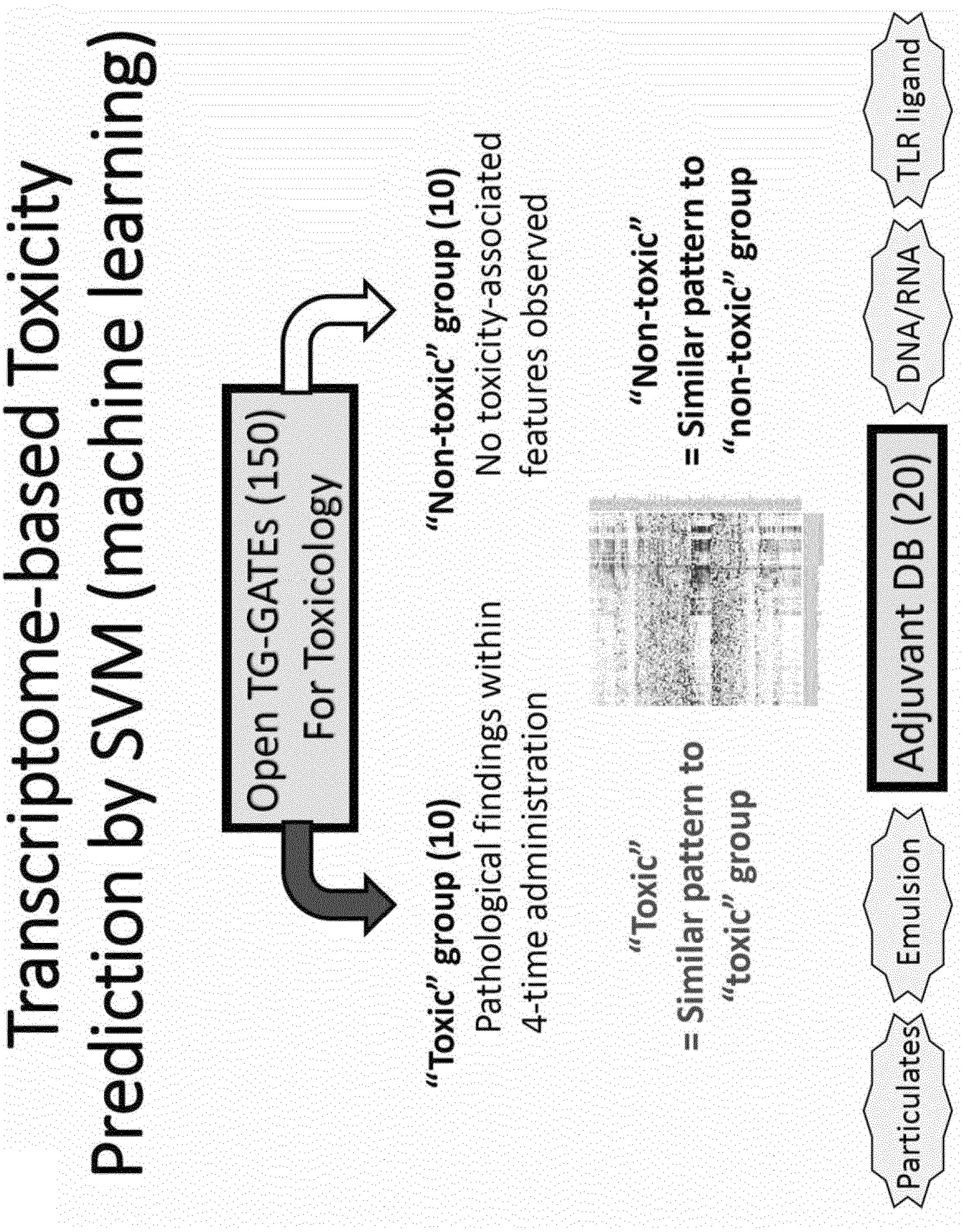
Transcriptome-based Toxicity
Prediction by SVM (machine learning)
Open TG-GATEs (150)
For Toxicology
"Toxic" group (10)
Pathological findings within
4-time administration
"Non-toxic" group (10)
No toxicity-associated
features observed
"Toxic"
= Similar pattern to
"toxic" group
"Non-toxic"
= Similar pattern to
"non-toxic" group
Particulates
Emulsion
Adjuvant DB (20)
DNA/RNA
TLR ligand

[FIG.30A]

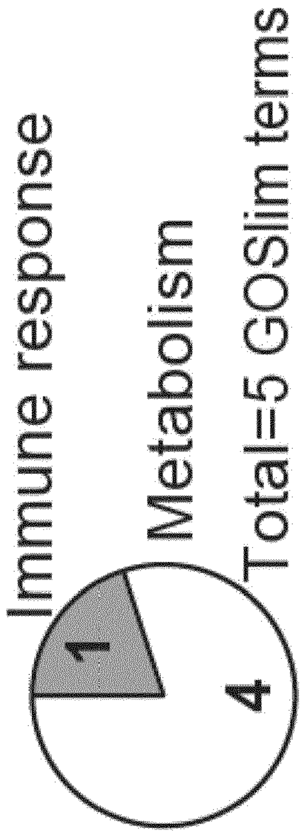

[FIG.30B]

**Transcriptome at 6h**

| Score | Adjuvant |
|---|---|
| 1.000 | Adjuvant X (NOD1 ligand) |
| 1.000 | Adjuvant A |
| 1.000 | Adjuvant B |
| 1.000 | Pam3CSK4 |
| 0.585 | R848 |

**Transcriptome at 24h**

| Score | Adjuvant |
|---|---|
| 0.835 | Adjuvant X (NOD1 ligand) |
| 0.538 | K3 |
| 0.532 | ISA51VG |
| 0.521 | FCA |
| 0.469 | Alum |

A

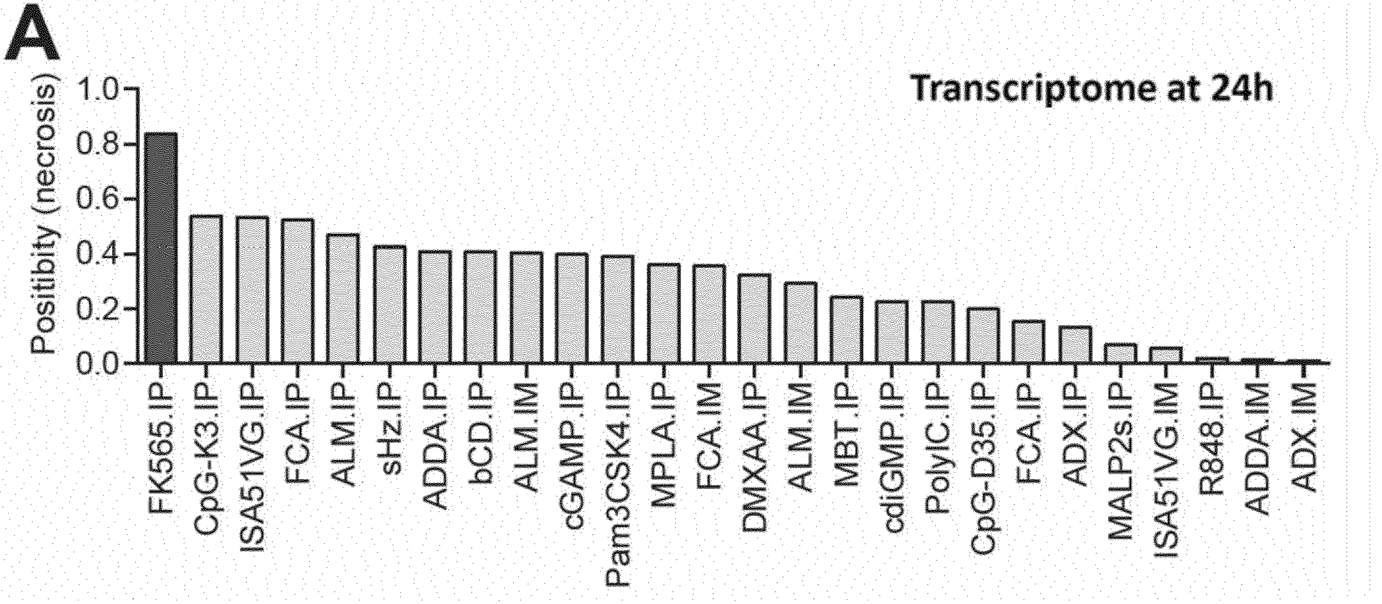

[FIG.31]

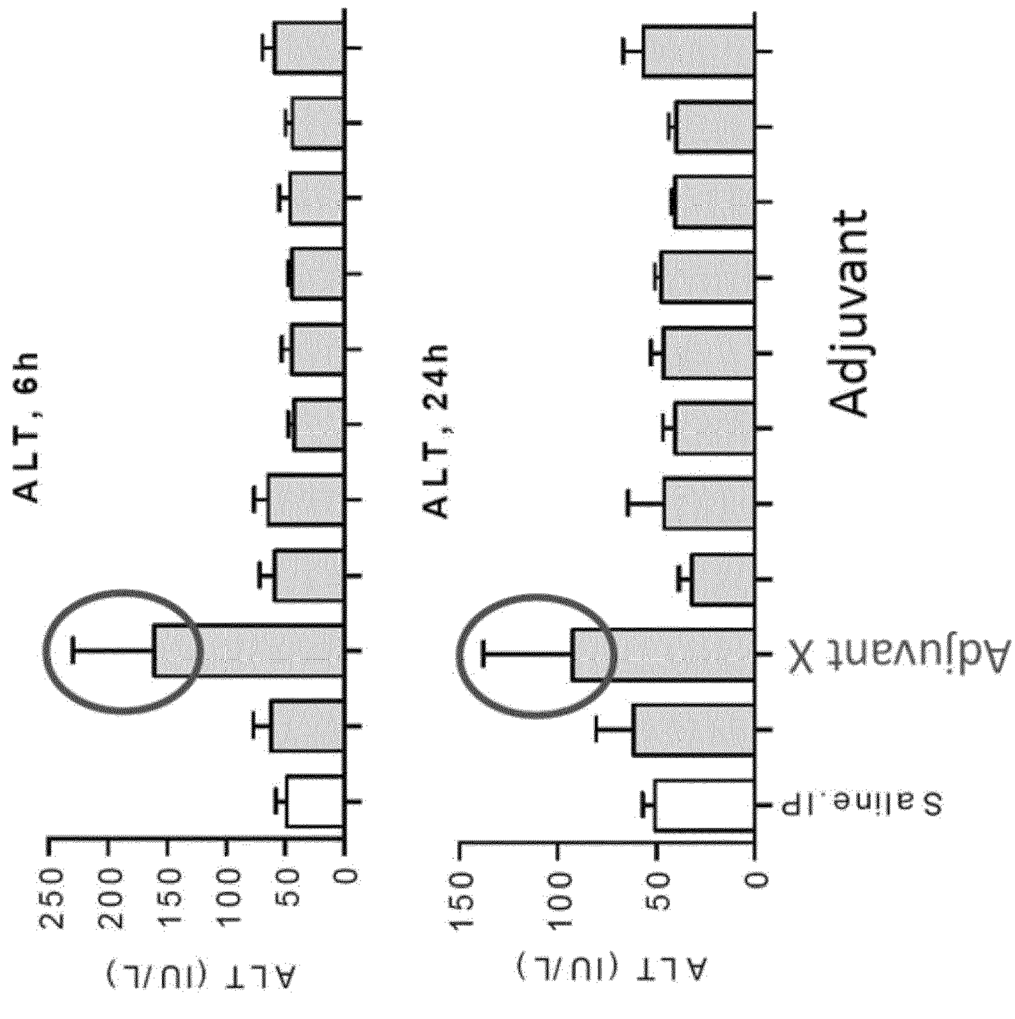
ALT, 6h
ALT (IU/L)
250
200
150
100
50
0
ALT, 24h
ALT (IU/L)
150
100
50
0
Saline.IP
Adjuvant X
Adjuvant

[FIG.32]

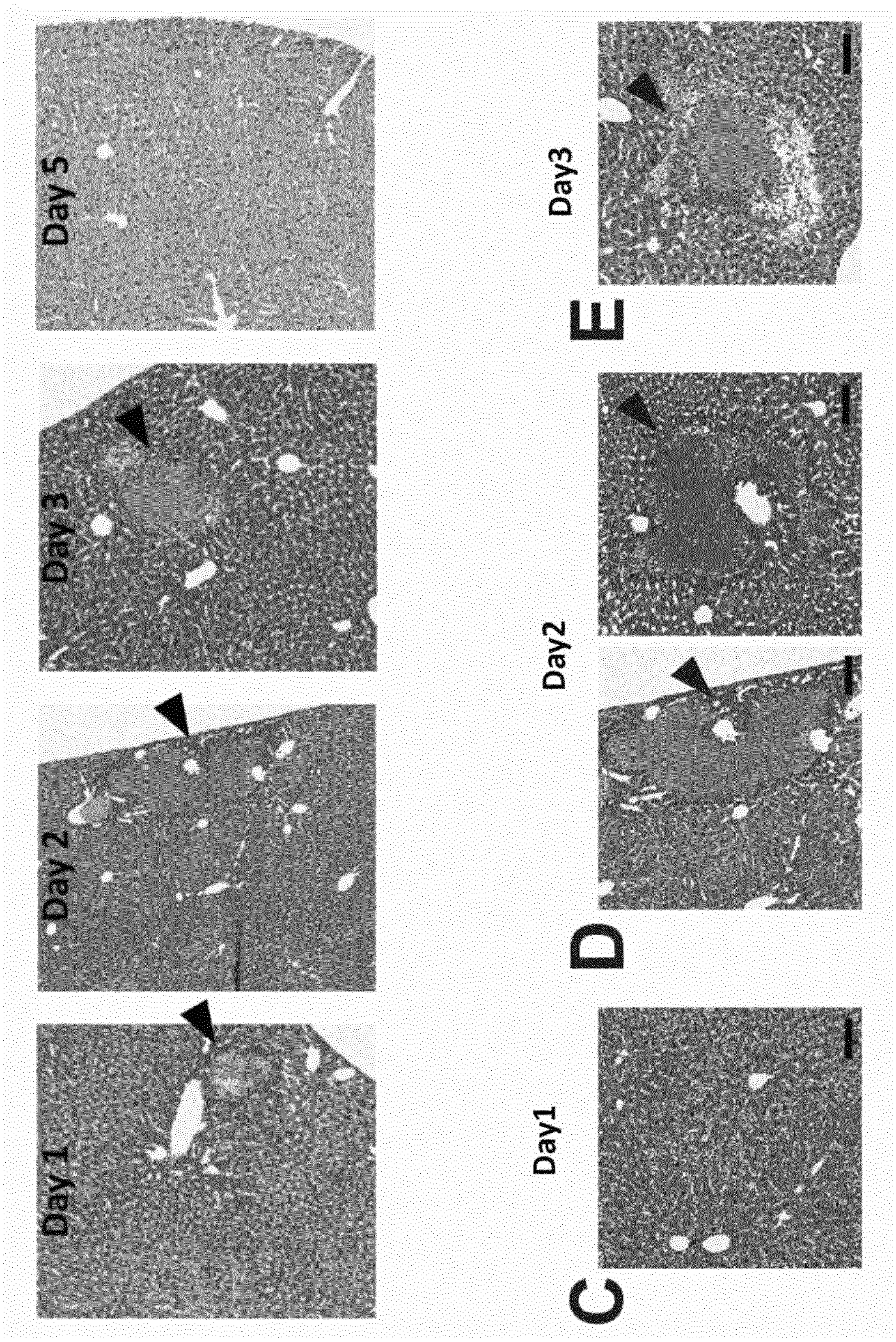

[FIG.33]

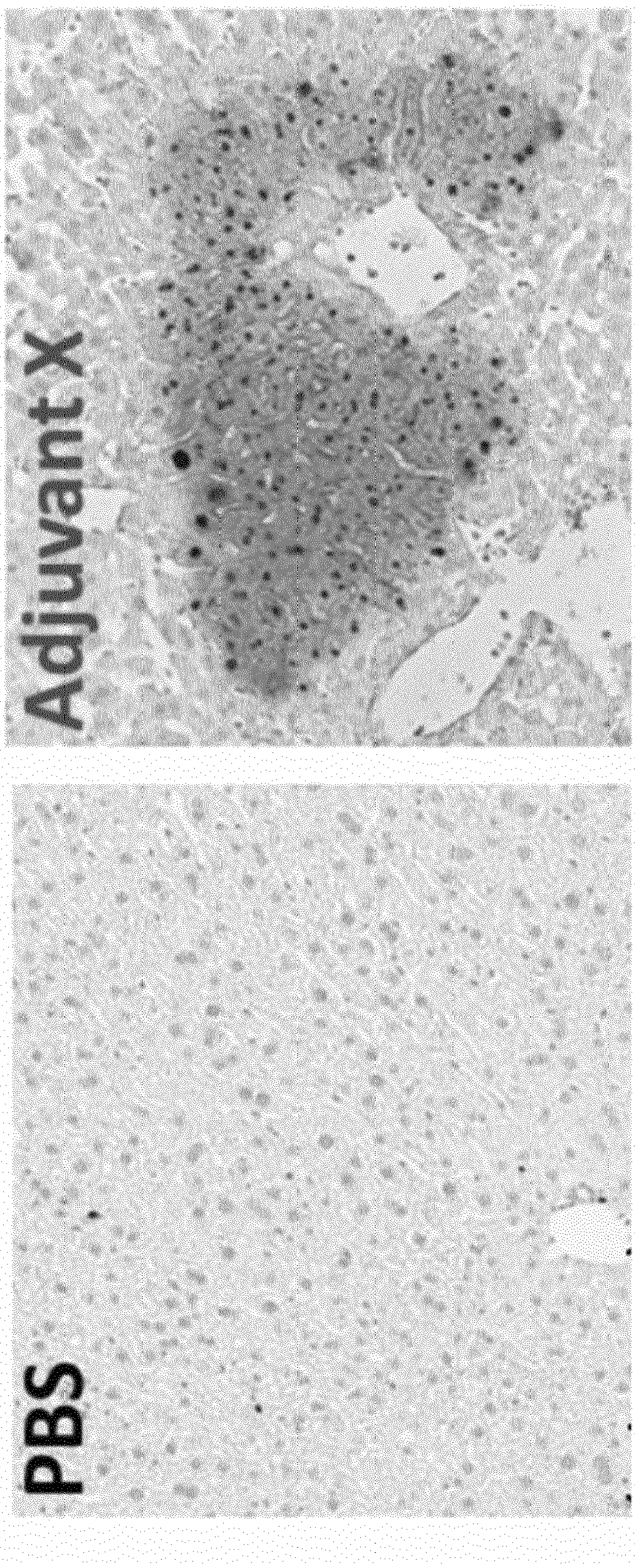

[FIG.34]

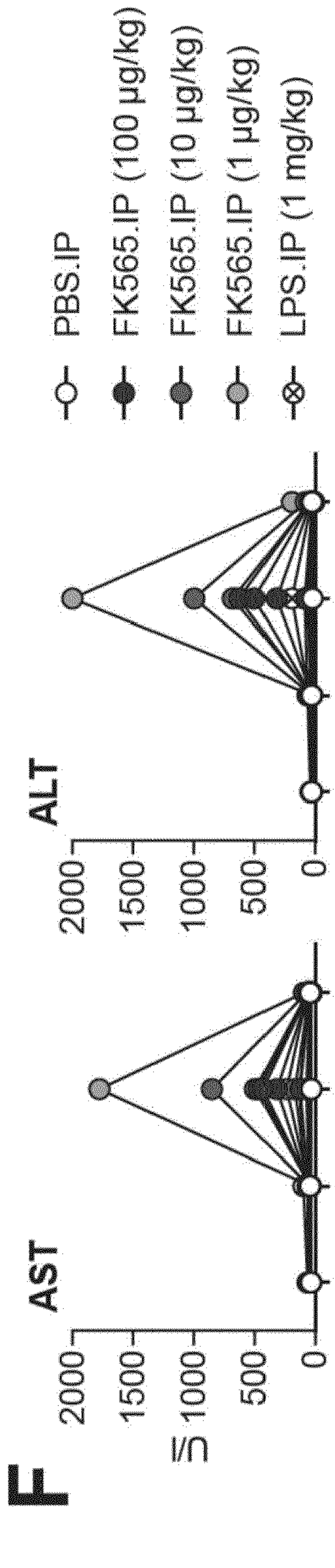
F
U/l
AST
ALT
2000
1500
1000
500
0
Pre
3h
1d
2d
PBS.IP
FK565.IP (100 μg/kg)
FK565.IP (10 μg/kg)
FK565.IP (1 μg/kg)
LPS.IP (1 mg/kg)

[FIG.35]

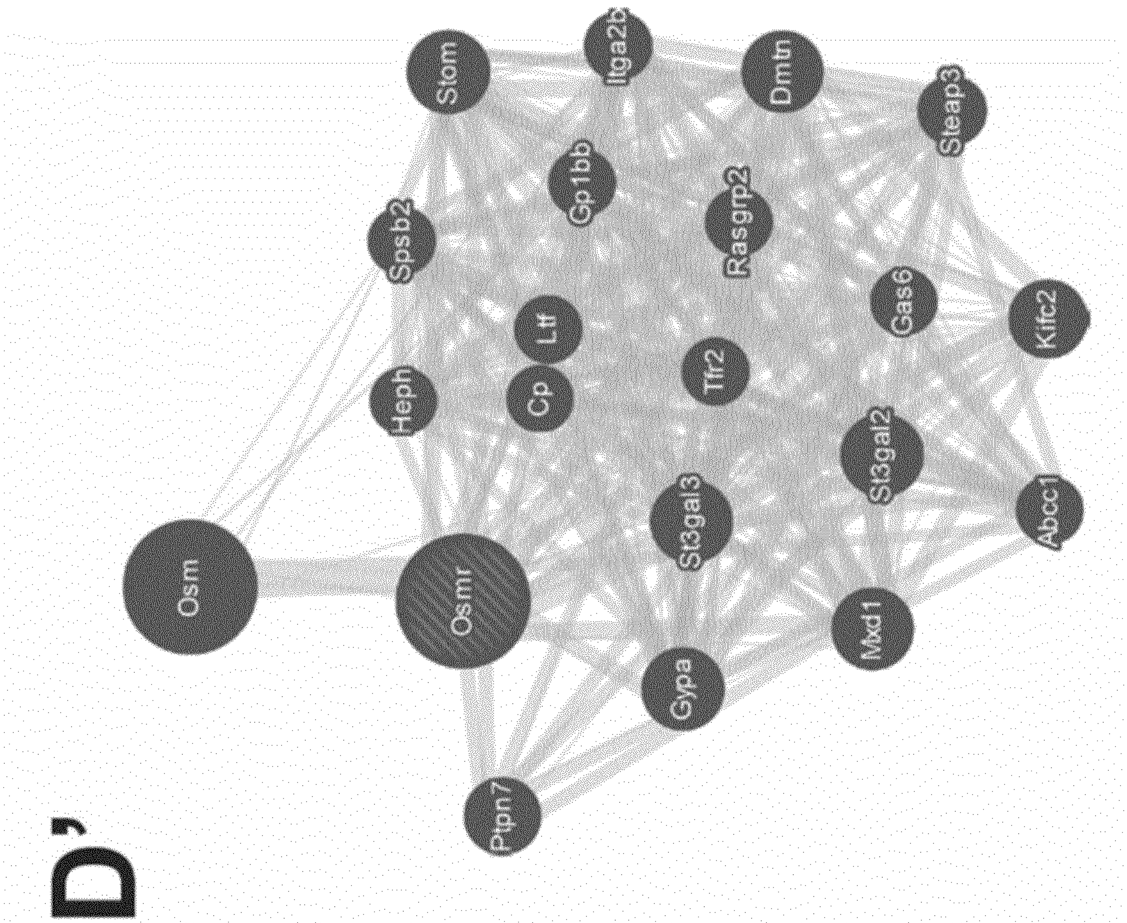
D'
Osm
Osmr
Ptpn7
Gypa
Mxd1
St3gal3
St3gal2
Abcc1
Heph
Cp
Ltf
Tfr2
Gas6
Kifc2
Spsb2
Gp1bb
Rasgrp2
Stom
Itga2b
Dmtn
Steap3

[FIG.36]

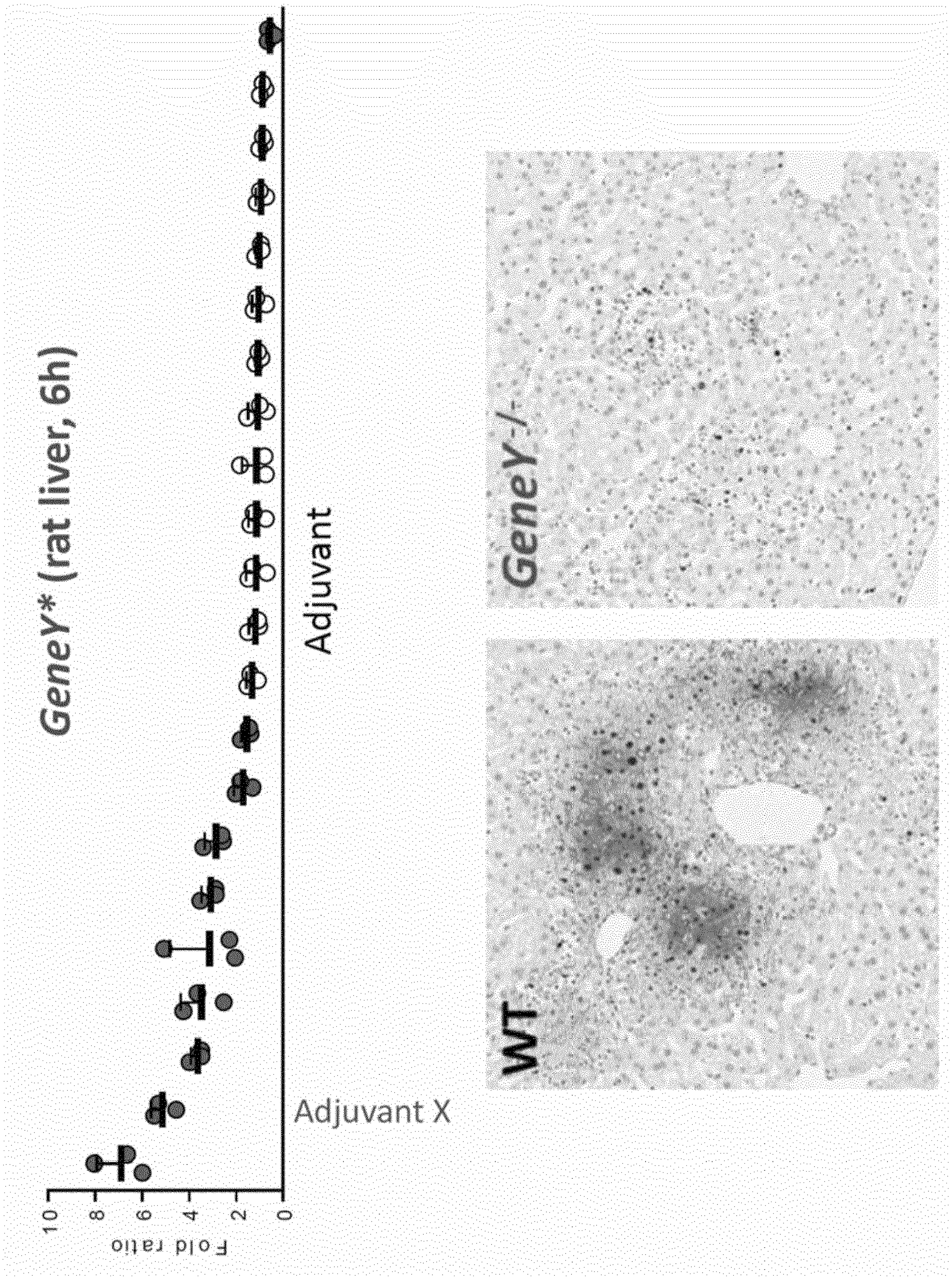
GeneY* (rat liver, 6h)
Fold ratio
10
8
6
4
2
0
Adjuvant X
Adjuvant
WT
GeneY-/-

[FIG.37]

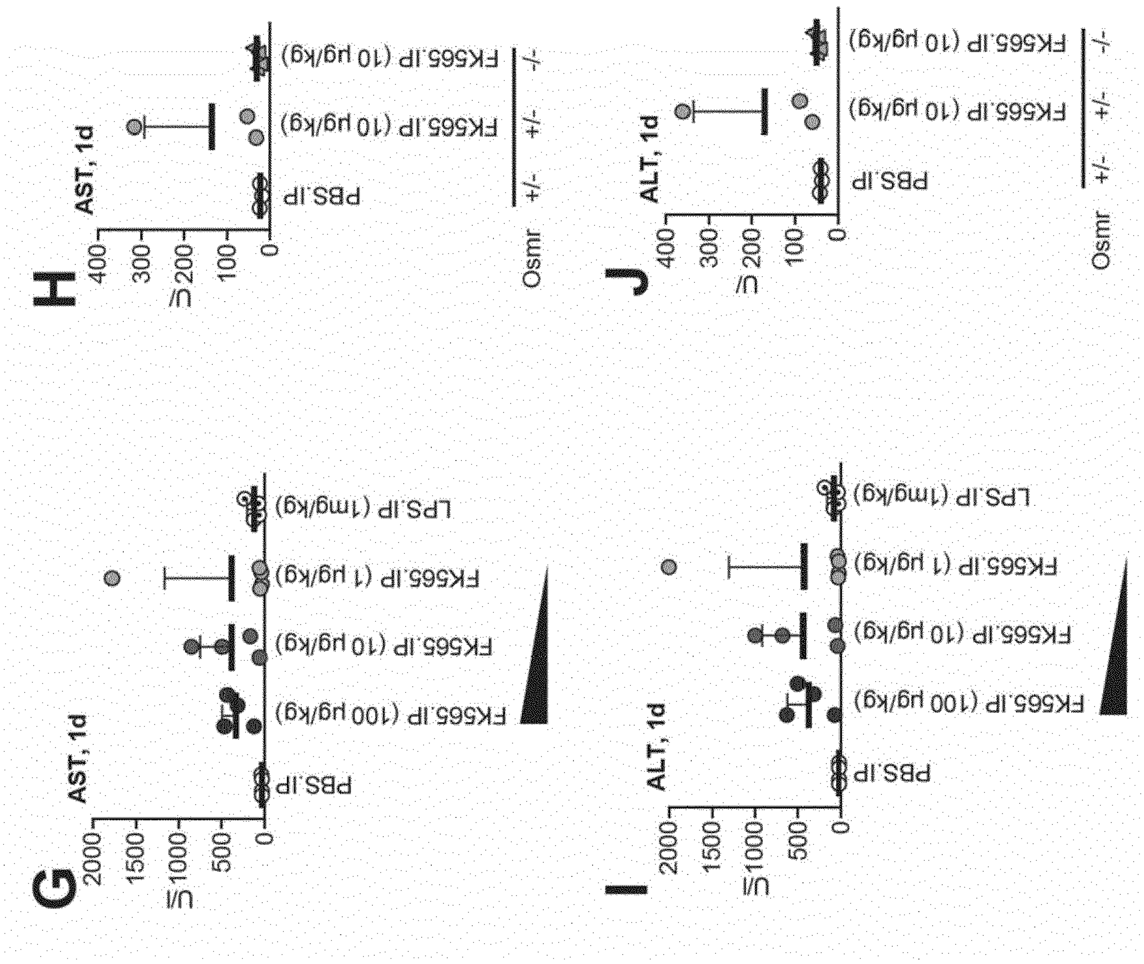
G
AST, 1d
U/l
PBS.IP
FK565.IP (100 μg/kg)
FK565.IP (10 μg/kg)
FK565.IP (1 μg/kg)
LPS.IP (1mg/kg)
H
AST, 1d
U/l
PBS.IP
FK565.IP (10 μg/kg)
FK565.IP (10 μg/kg)
Osmr
+/-
+/-
-/-
I
ALT, 1d
U/l
PBS.IP
FK565.IP (100 μg/kg)
FK565.IP (10 μg/kg)
FK565.IP (1 μg/kg)
LPS.IP (1mg/kg)
J
ALT, 1d
U/l
PBS.IP
FK565.IP (10 μg/kg)
FK565.IP (10 μg/kg)
Osmr
+/-
+/-
-/-

[FIG.38]

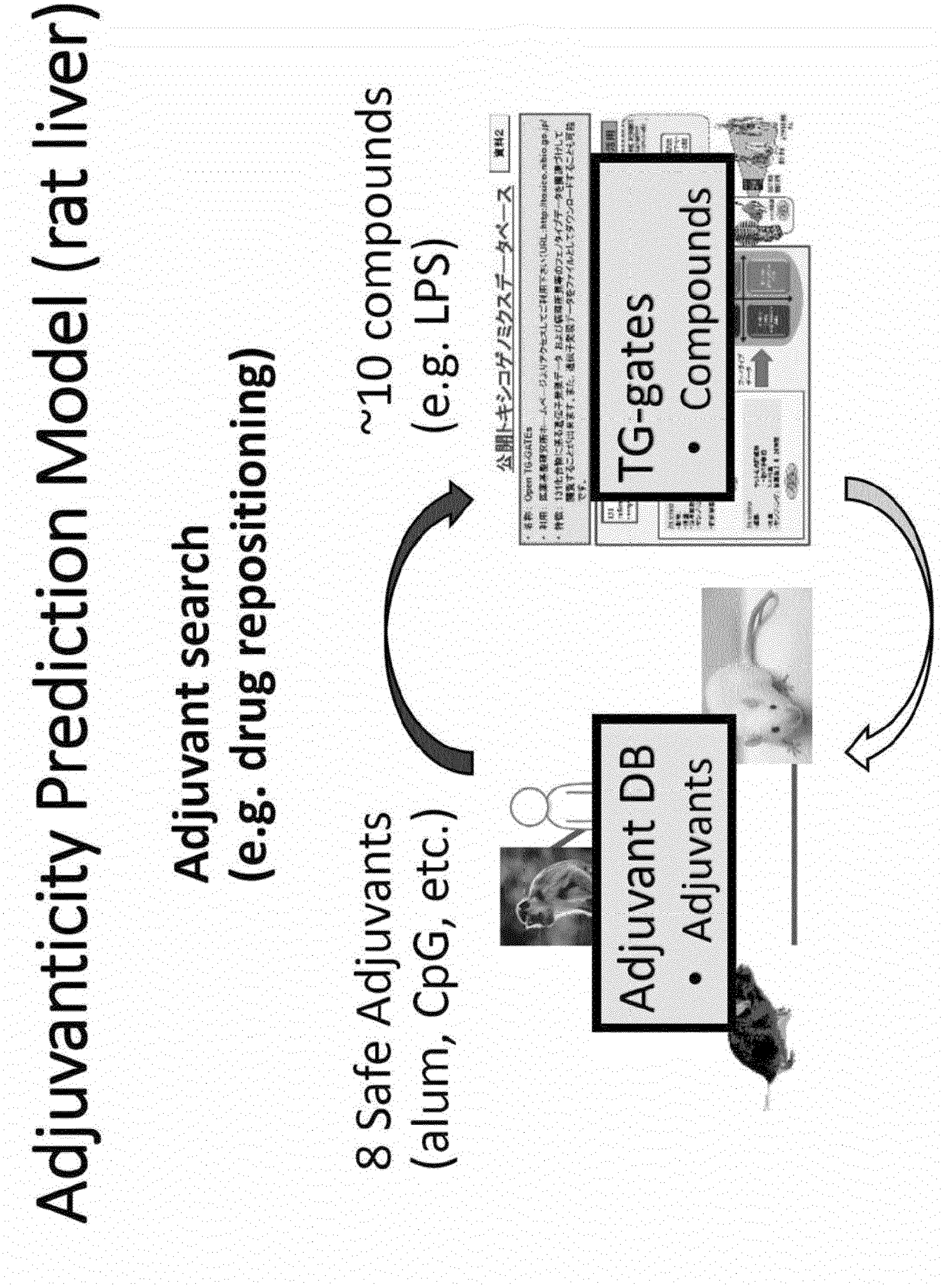
Adjuvanticity Prediction Model (rat liver)
Adjuvant search
(e.g. drug repositioning)
8 Safe Adjuvants
(alum, CpG, etc.)
~10 compounds
(e.g. LPS)
公開トキシコゲノミクスデータベース
資料2
Open TG-GATEs
Adjuvant DB
• Adjuvants
TG-gates
• Compounds

[FIG.39]

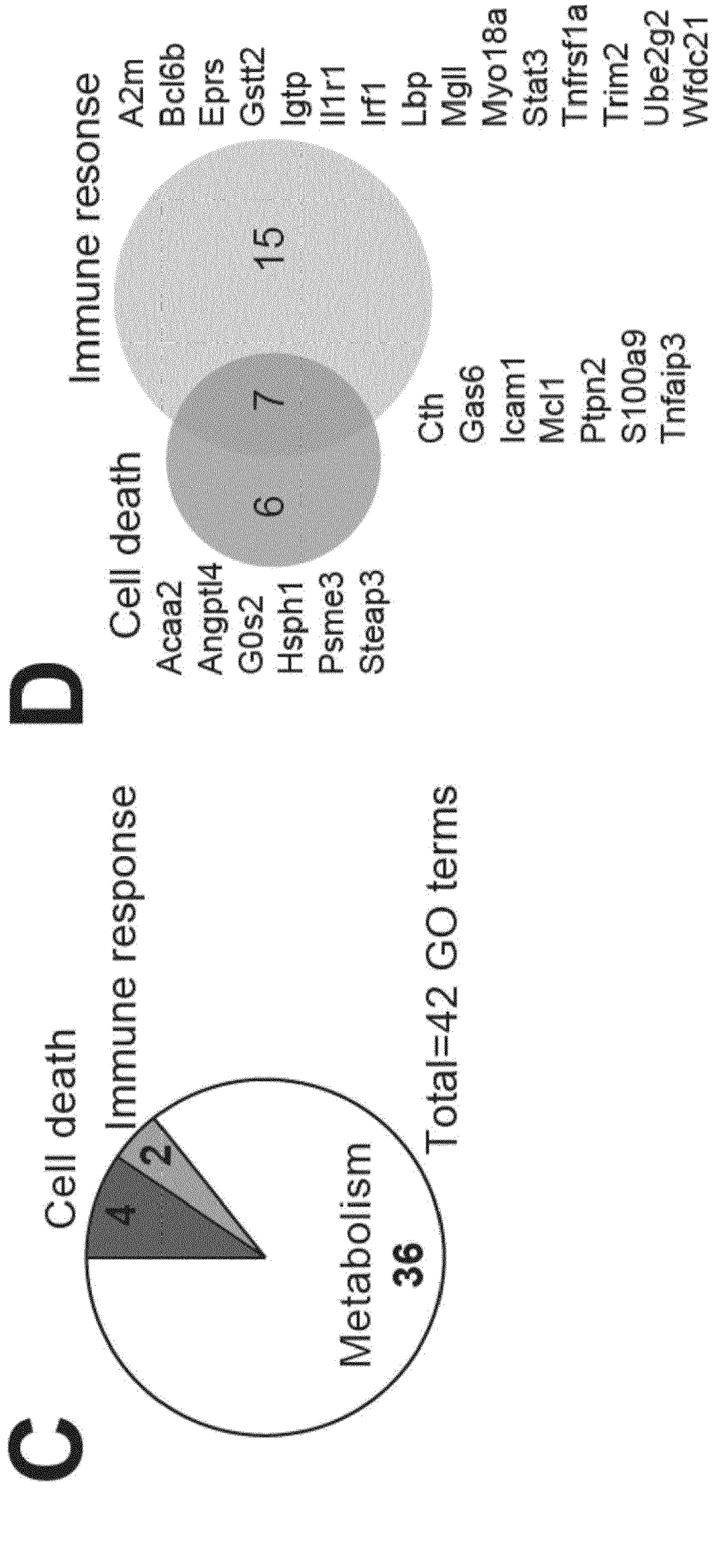
C
Cell death
Immune response
4
2
Metabolism
36
Total=42 GO terms
D
Cell death
Immune resonse
6
7
15
Acaa2
Angptl4
G0s2
Hsph1
Psme3
Steap3
Cth
Gas6
Icam1
Mcl1
Ptpn2
S100a9
Tnfaip3
A2m
Bcl6b
Eprs
Gstt2
Igtp
Il1r1
Irf1
Lbp
Mgll
Myo18a
Stat3
Tnfrsf1a
Trim2
Ube2g2
Wfdc21

[FIG.40]

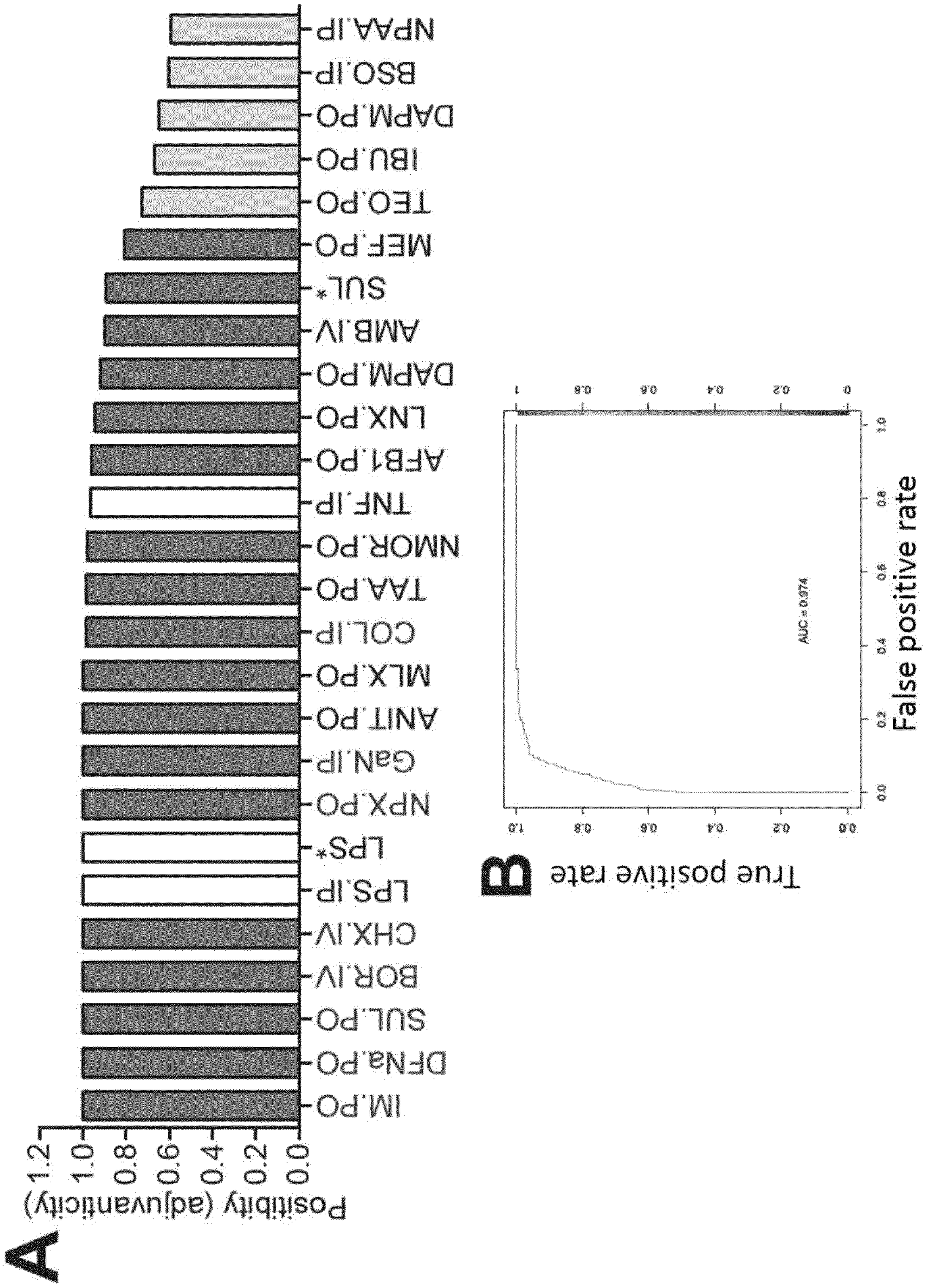
A
Positibity (adjuvanticity)
1.2
1.0
0.8
0.6
0.4
0.2
0.0
IM.PO
DFNa.PO
SUL.PO
BOR.IV
CHX.IV
LPS.IP
LPS*
NPX.PO
GaN.IP
ANIT.PO
MLX.PO
COL.IP
TAA.PO
NMOR.PO
TNF.IP
AFB1.PO
LNX.PO
DAPM.PO
AMB.IV
SUL*
MEF.PO
TEO.PO
IBU.PO
DAPM.PO
BSO.IP
NPAA.IP
B
True positive rate
False positive rate
AUC = 0.974

[FIG.41]

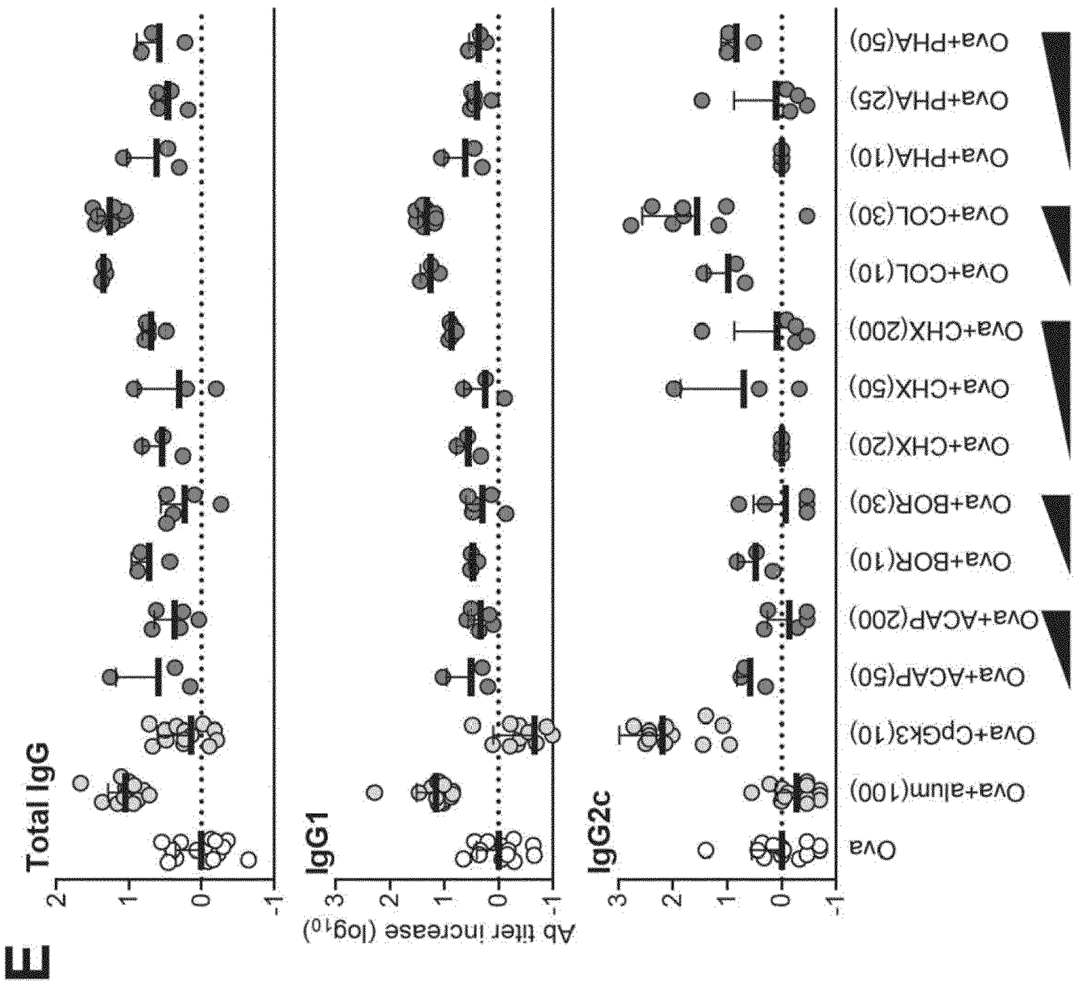
E
Total IgG
IgG1
IgG2c
Ab titer increase ($log_{10}$)
Ova
Ova+alum(100)
Ova+CpGK3(10)
Ova+ACAP(50)
Ova+ACAP(200)
Ova+BOR(10)
Ova+BOR(30)
Ova+CHX(20)
Ova+CHX(50)
Ova+CHX(200)
Ova+COL(10)
Ova+COL(30)
Ova+PHA(10)
Ova+PHA(25)
Ova+PHA(50)

[FIG.42]

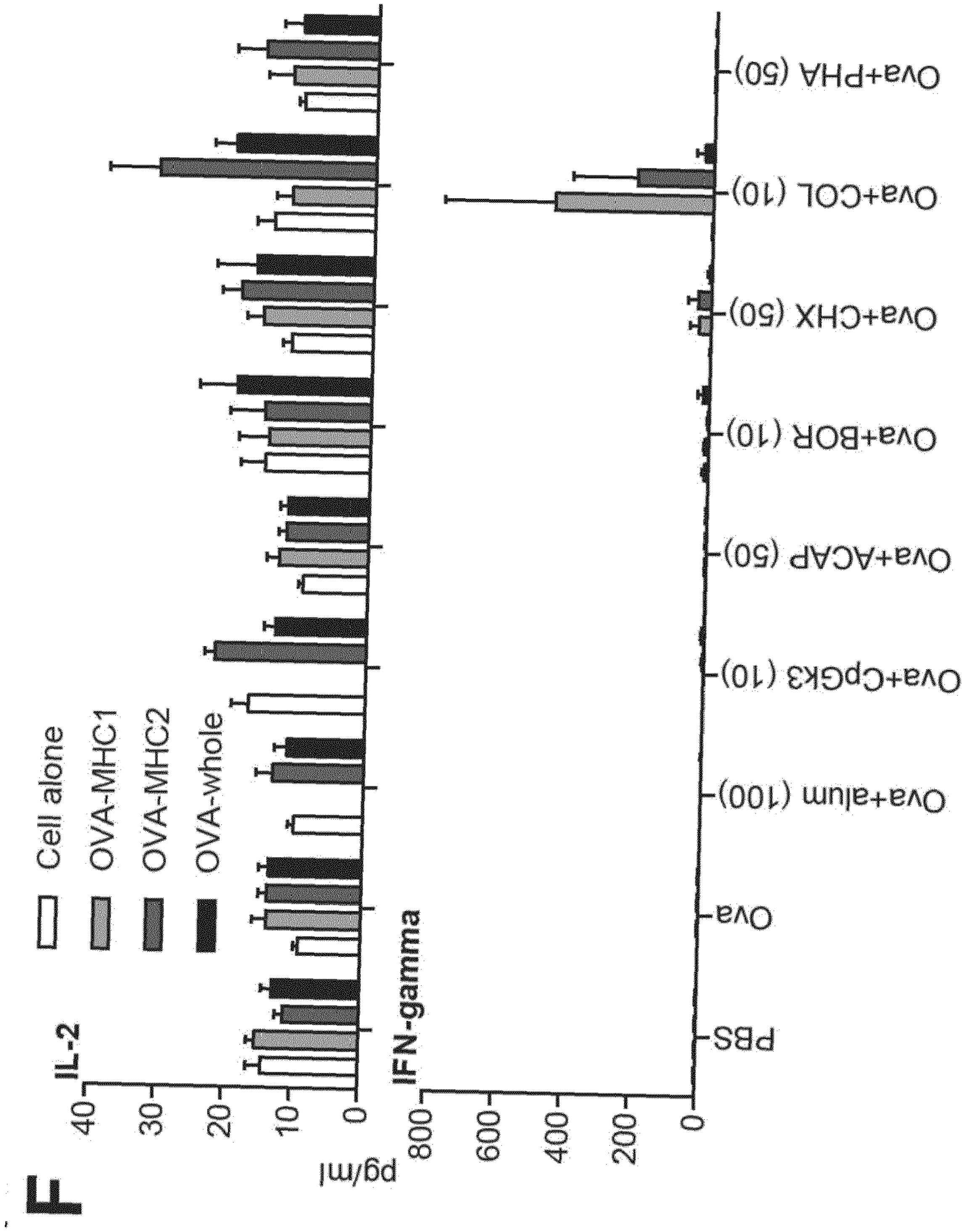
F
IL-2
IFN-gamma
pg/ml
40
30
20
10
0
800
600
400
200
0
Cell alone
OVA-MHC1
OVA-MHC2
OVA-whole
PBS
Ova
Ova+alum (100)
Ova+CpGK3 (10)
Ova+ACAP (50)
Ova+BOR (10)
Ova+CHX (50)
Ova+COL (10)
Ova+PHA (50)

[FIG.43]

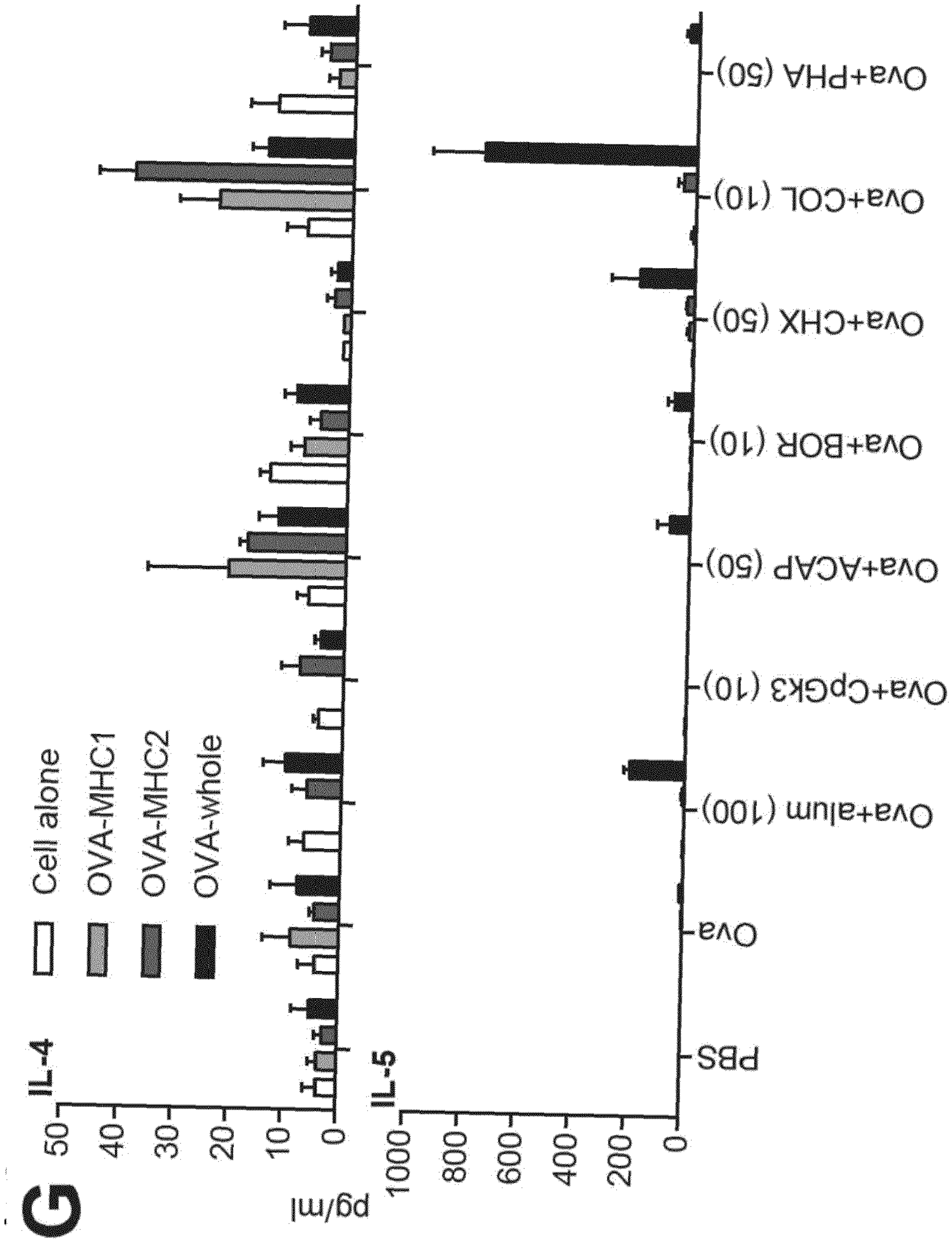
G
IL-4
IL-5
pg/ml
50
40
30
20
10
0
1000
800
600
400
200
0
Cell alone
OVA-MHC1
OVA-MHC2
OVA-whole
PBS
Ova
Ova+alum (100)
Ova+CpGK3 (10)
Ova+ACAP (50)
Ova+BOR (10)
Ova+CHX (50)
Ova+COL (10)
Ova+PHA (50)

[FIG.44]

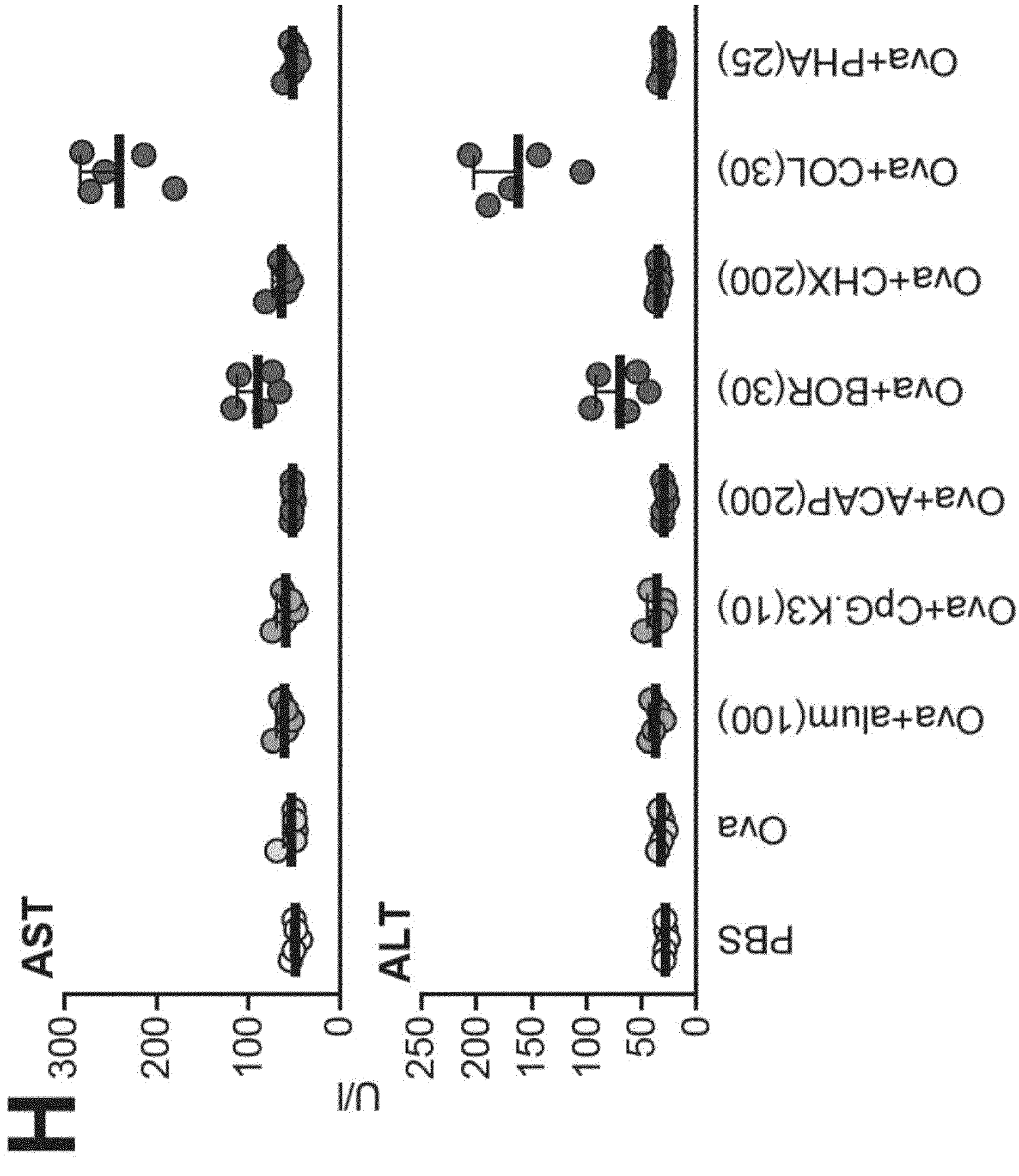
H
AST
ALT
U/l
300
200
100
0
250
200
150
100
50
0
PBS
Ova
Ova+alum(100)
Ova+CpG.K3(10)
Ova+ACAP(200)
Ova+BOR(30)
Ova+CHX(200)
Ova+COL(30)
Ova+PHA(25)

[FIG.45]

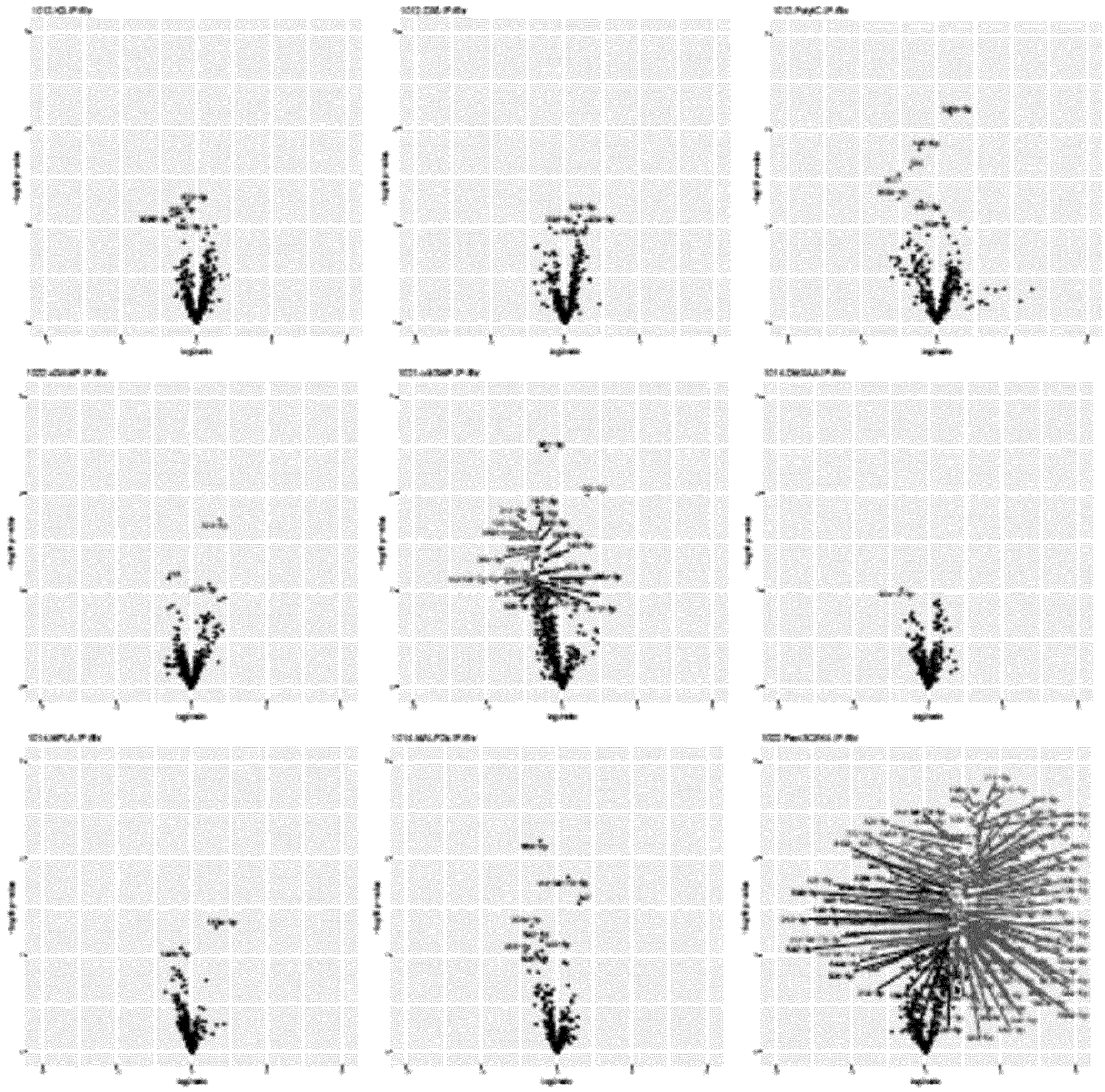

[FIG.45](**Continue**)

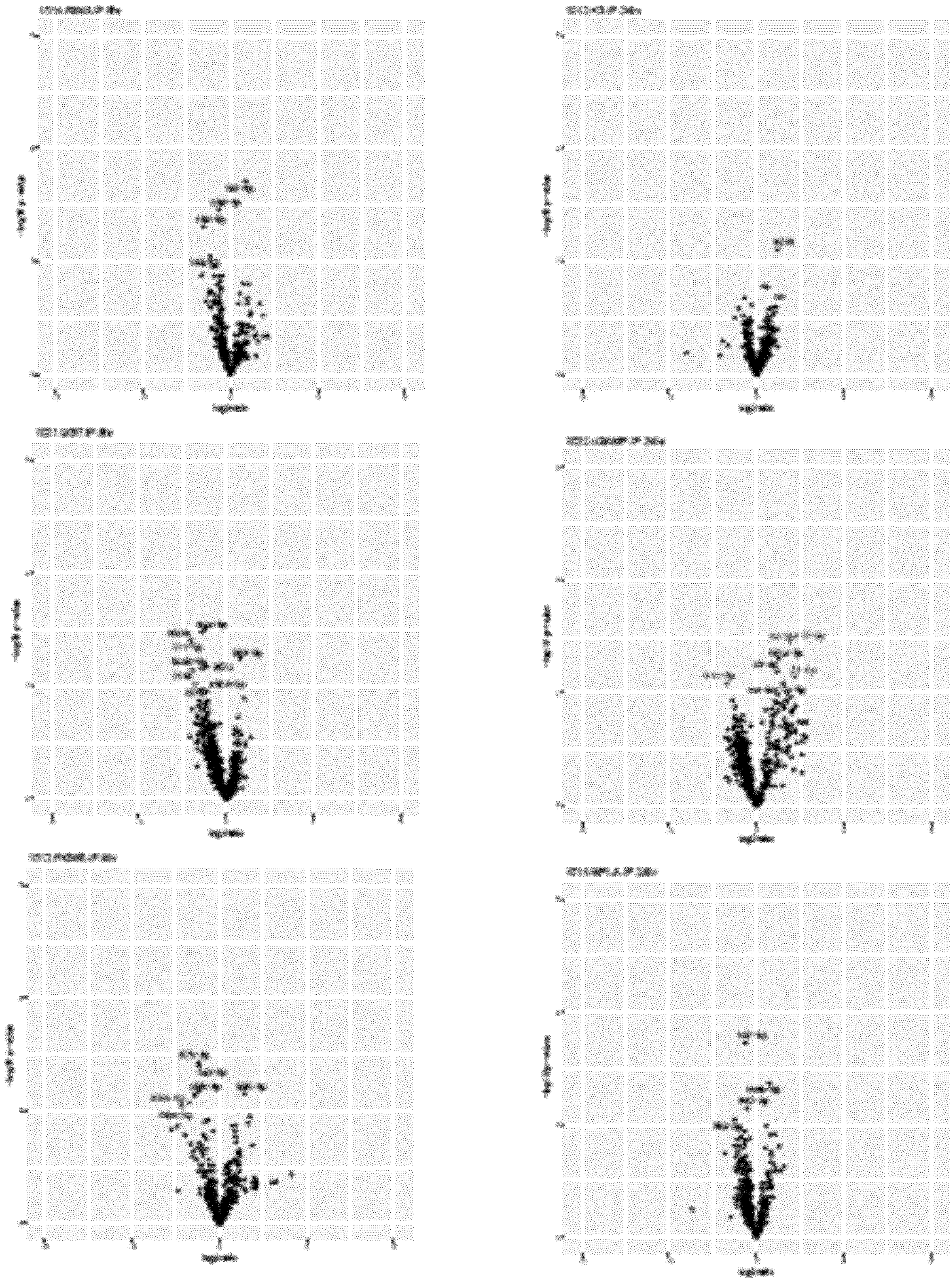

[FIG.45](**Continue**)

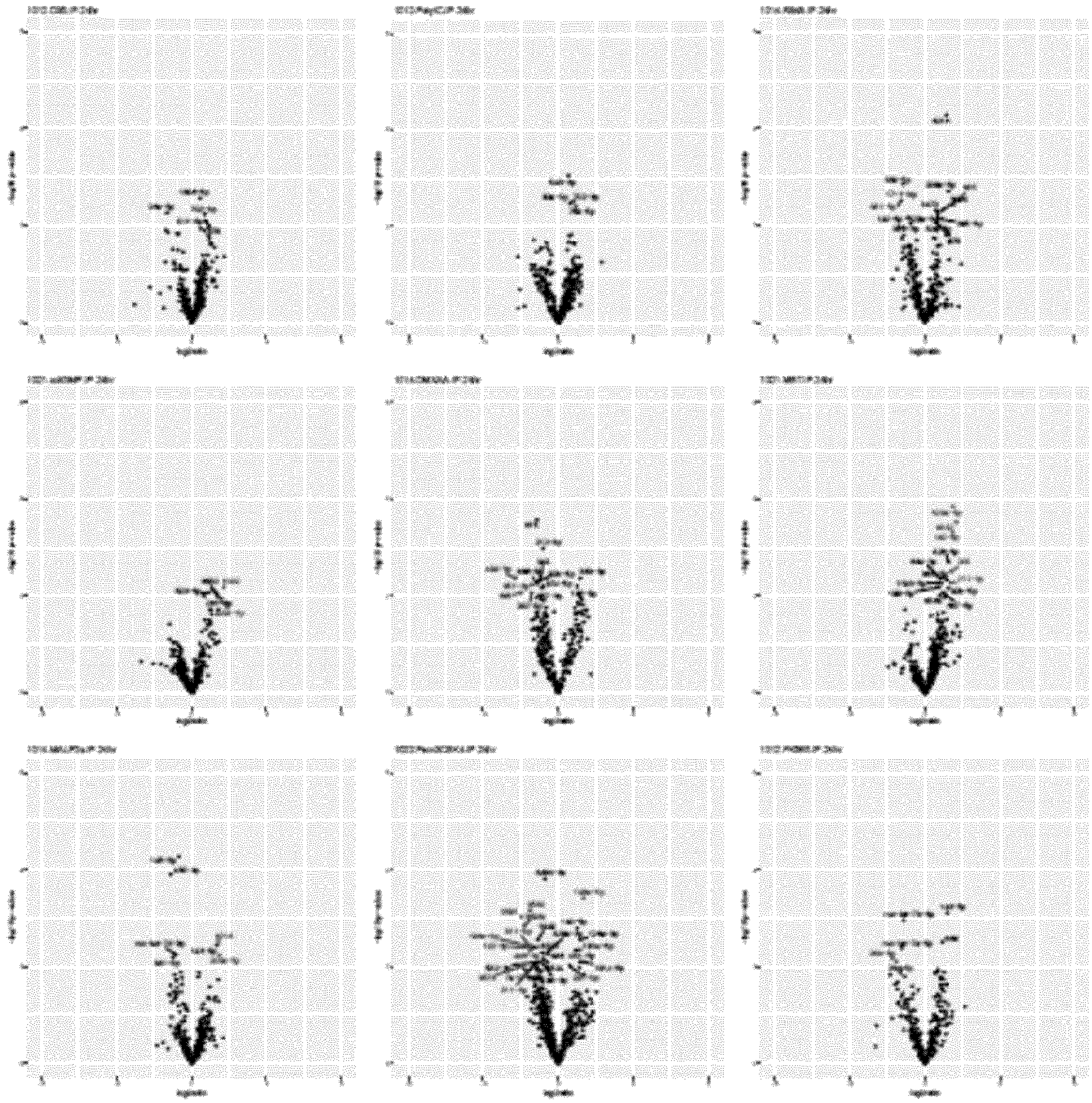

[FIG.45](**Continue**)

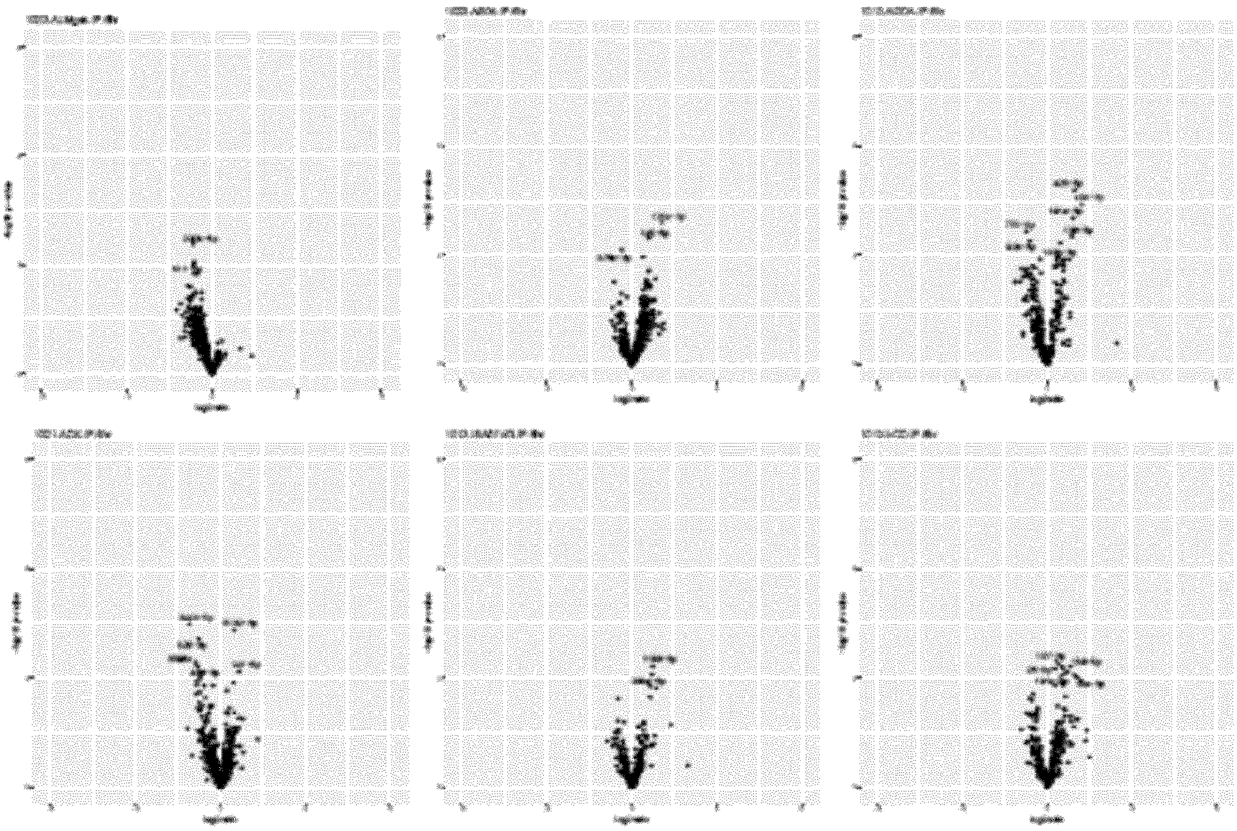

[FIG.45](**Continue**)

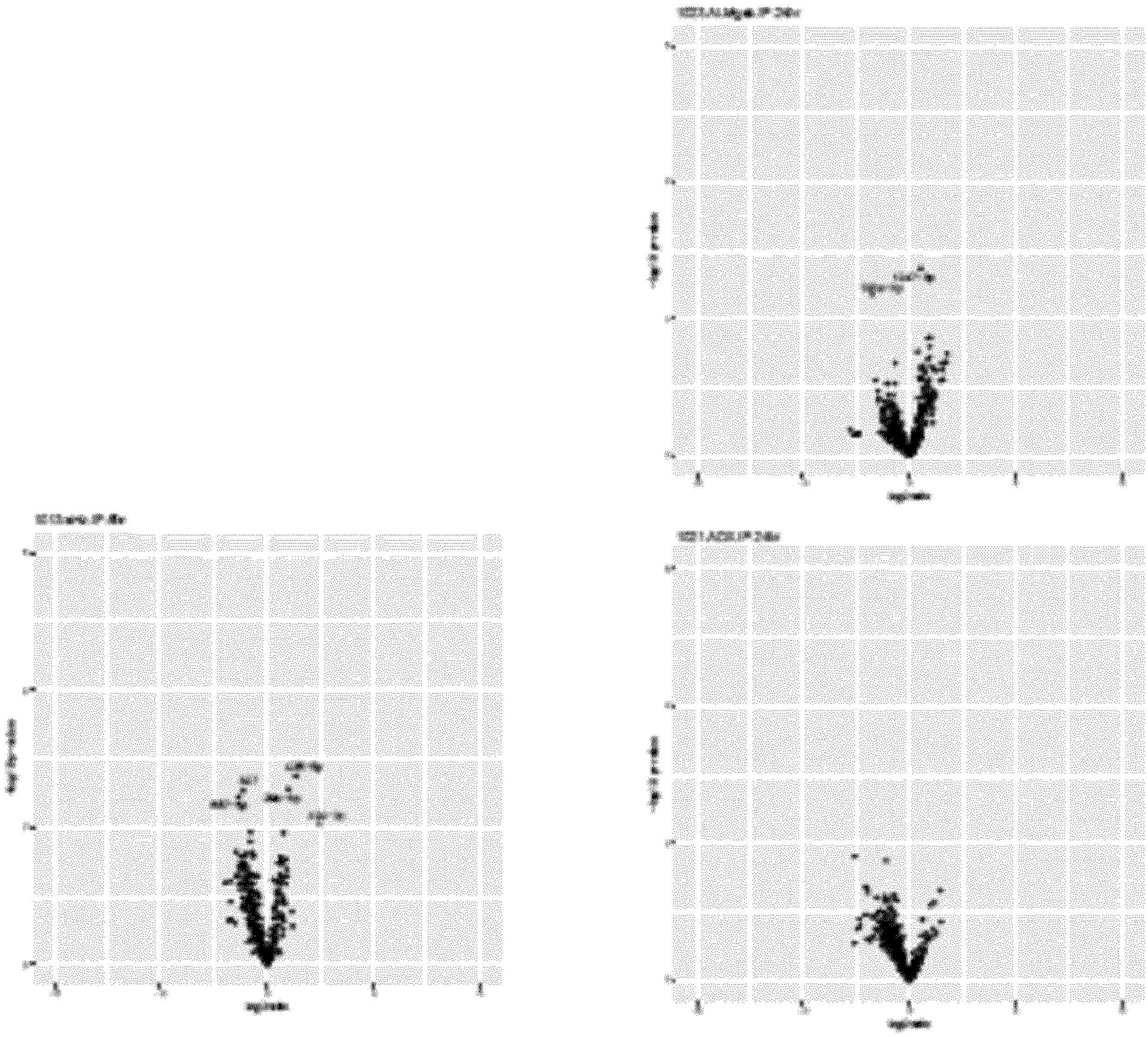

[FIG.45](**Continue**)

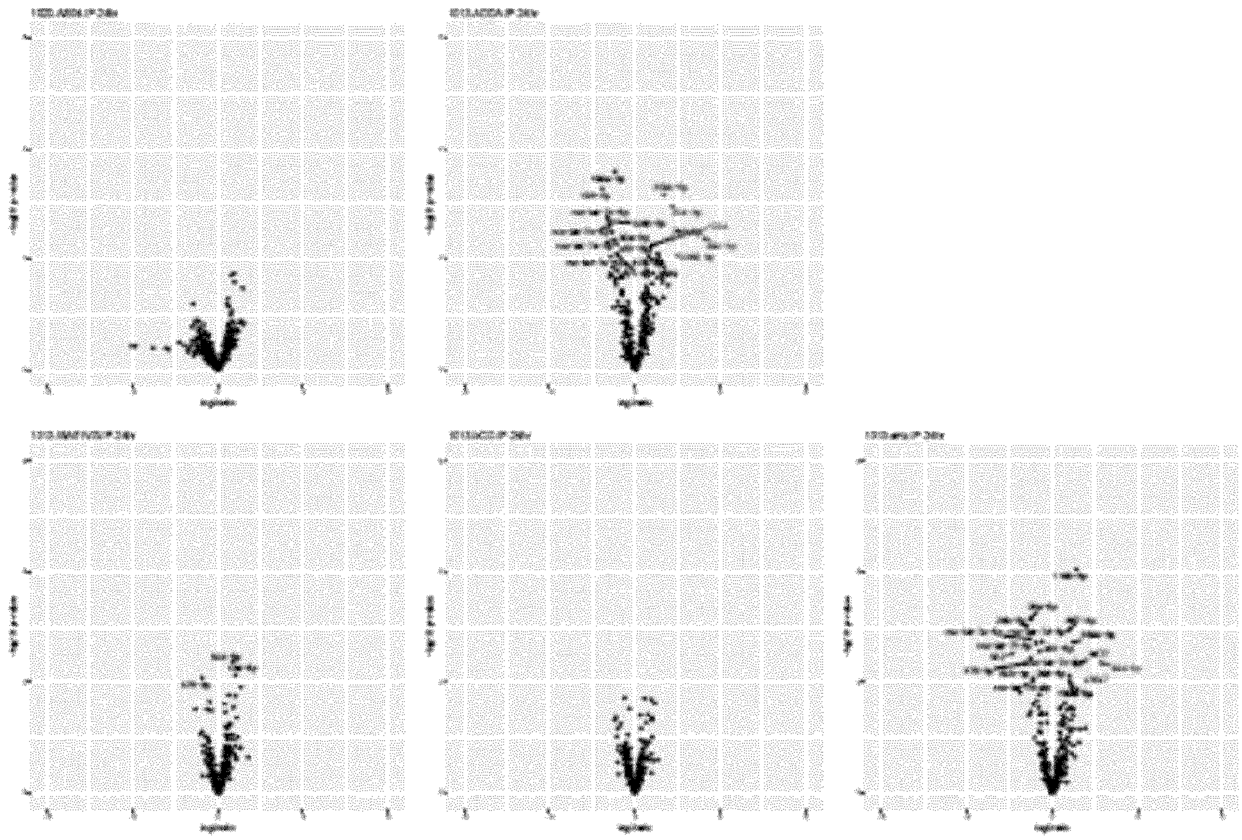

[FIG.46]

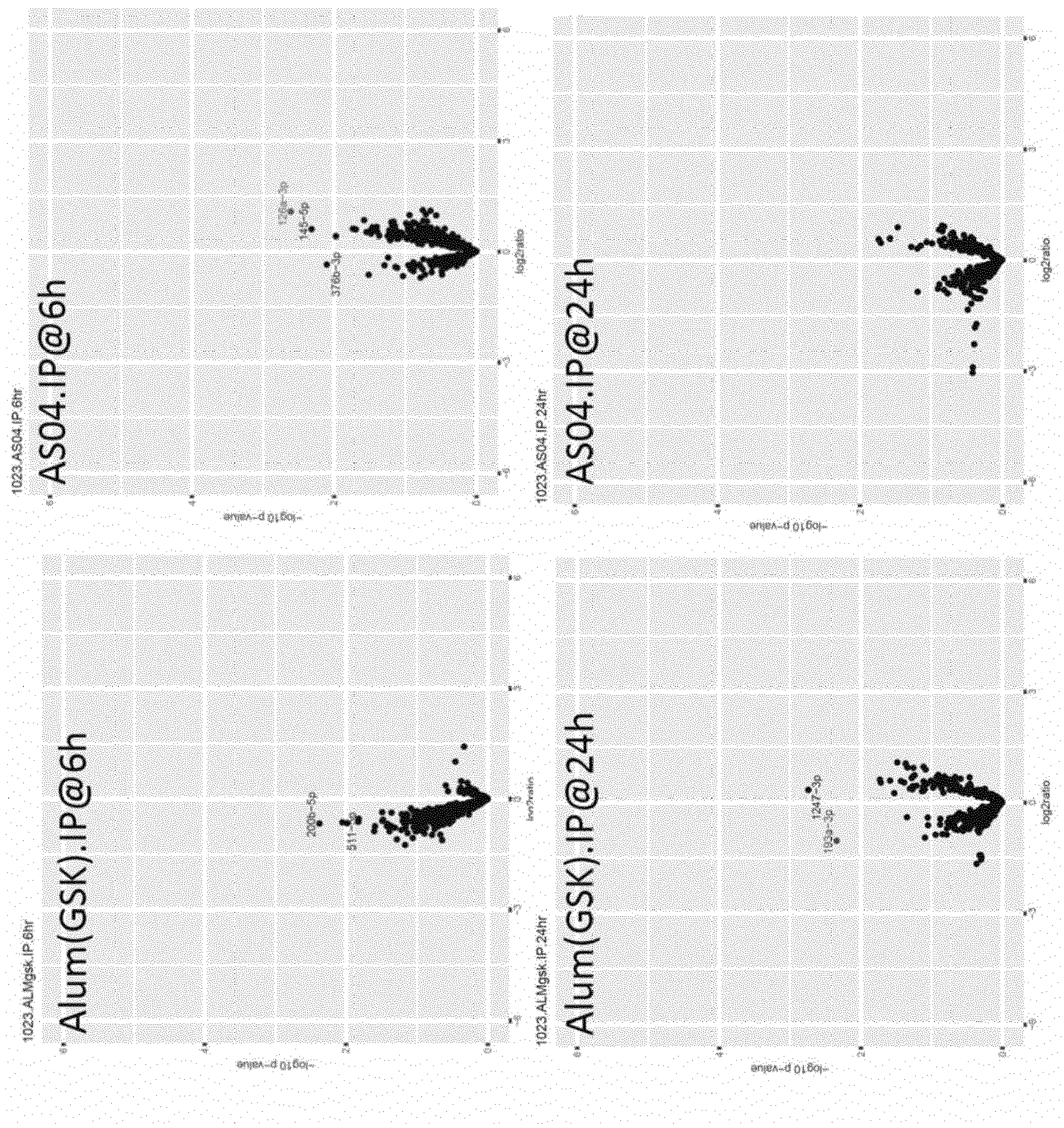
1023.AS04.IP.6hr
AS04.IP@6h
126a-3p
145-5p
376b-3p
log2ratio
-log10 p-value
1023.AS04.IP.24hr
AS04.IP@24h
1023.ALMgsk.IP.6hr
Alum(GSK).IP@6h
200b-5p
1023.ALMgsk.IP.24hr
Alum(GSK).IP@24h
1247-3p
193a-3p

European Patent Office

# EUROPEAN SEARCH REPORT

Application Number
EP 22 20 4376

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | YOSHINOBU IGARASHI ET AL: "Abstract", NUCLEIC ACIDS RESEARCH, vol. 43, no. D1, 13 October 2014 (2014-10-13), pages D921-D927, XP055749991, ISSN: 0305-1048, DOI: 10.1093/nar/gku955 * the whole document * ----- | 1-15 | INV. G01N33/50 C12M1/00 C12Q1/68 G01N33/49 G01N33/68 C12Q1/6876 |
| X | TAKEKI UEHARA ET AL: "Prediction model of potential hepatocarcinogenicity of rat hepatocarcinogens using a large-scale toxicogenomics database", TOXICOLOGY AND APPLIED PHARMACOLOGY, ACADEMIC PRESS, AMSTERDAM, NL, vol. 255, no. 3, 6 July 2011 (2011-07-06), pages 297-306, XP028275723, ISSN: 0041-008X, DOI: 10.1016/J.TAAP.2011.07.001 [retrieved on 2011-07-19] * see whole doc. esp. Figure 3 * ----- | 1-15 | |
| | | | TECHNICAL FIELDS SEARCHED (IPC) |
| X | US 2012/258125 A1 (COWING CAROL O [US] ET AL) 11 October 2012 (2012-10-11) * see whole doc., esp. claims and Tables * ----- | 1-15 | C12Q G16B |
| X | WO 2015/183173 A1 (GRAFSTRÖM ROLAND [SE]; KOHONEN PEKKA [SE] ET AL.) 3 December 2015 (2015-12-03) * see whole doc. esp. claims * ----- -/-- | 1-15 | |

The present search report has been drawn up for all claims

| Place of search | Date of completion of the search | Examiner |
|---|---|---|
| Munich | 8 May 2023 | Mueller, Frank |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

page 1 of 3

Europäisches Patentamt
European Patent Office
Office européen des brevets

# EUROPEAN SEARCH REPORT

Application Number
EP 22 20 4376

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | YANG Y ET AL: "Toxicogenomics in drug discovery: from preclinical studies to clinical trials", CHEMICO-BIOLOGICAL INTERACTIONS, ELSEVIER SCIENCE IRLAND, IR, vol. 150, no. 1, 1 November 2004 (2004-11-01), pages 71-85, XP004619546, ISSN: 0009-2797, DOI: 10.1016/J.CBI.2004.09.013 * the whole document * ----- | 1-15 | |
| X | US 2014/037685 A1 (BROJATSCH JURGEN [US] ET AL) 6 February 2014 (2014-02-06) * see whole doc. esp. claims * ----- | 1-15 | |
| X | WO 2011/160767 A1 (MERCK PATENT GMBH [DE]; MUELLER STEFAN [DE] ET AL.) 29 December 2011 (2011-12-29) * see whole doc., esp. claims * ----- | 1-15 | TECHNICAL FIELDS SEARCHED (IPC) |
| X | WO 2006/050124 A2 (NOVARTIS AG [CH]; NOVARTIS PHARMA GMBH [AT] ET AL.) 11 May 2006 (2006-05-11) * see whole doc. esp. claims * ----- | 1-15 | |
| X | G. NATSOULIS ET AL: "Classification of a large microarray data set: Algorithm comparison and analysis of drug signatures", GENOME RESEARCH, vol. 15, no. 5, 1 May 2005 (2005-05-01), pages 724-736, XP055750407, US ISSN: 1088-9051, DOI: 10.1101/gr.2807605 * the whole document * ----- -/-- | 1-15 | |

The present search report has been drawn up for all claims

2

| Place of search | Date of completion of the search | Examiner |
|---|---|---|
| Munich | 8 May 2023 | Mueller, Frank |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 20 4376

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ENGELBERG ALAN: "Iconix Pharmaceuticals, Inc.--removing barriers to efficient drug discovery through chemogenomics", PHARMACOGENOMICS, FUTURE MEDICINE, UK, vol. 5, no. 6, 31 August 2004 (2004-08-31), pages 741-744, XP009524060, ISSN: 1462-2416, DOI: 10.1517/14622416.5.6.741 [retrieved on 2004-11-04] * the whole document * ----- | 1-15 | |
| | | | TECHNICAL FIELDS SEARCHED (IPC) |

The present search report has been drawn up for all claims

| Place of search | Date of completion of the search | Examiner |
|---|---|---|
| Munich | 8 May 2023 | Mueller, Frank |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 3 of 3

## ANNEX TO THE EUROPEAN SEARCH REPORT ON EUROPEAN PATENT APPLICATION NO.

EP 22 20 4376

This annex lists the patent family members relating to the patent documents cited in the above-mentioned European search report. The members are as contained in the European Patent Office EDP file on
The European Patent Office is in no way liable for these particulars which are merely given for the purpose of information.

08-05-2023

| Patent document cited in search report | Publication date | Patent family member(s) | Publication date |
|---|---|---|---|
| US 2012258125 A1 | 11-10-2012 | US 2012258125 A1 | 11-10-2012 |
| | | US 2015307938 A1 | 29-10-2015 |
| | | WO 2012138928 A1 | 11-10-2012 |
| WO 2015183173 A1 | 03-12-2015 | DK 3149204 T3 | 25-10-2021 |
| | | EP 3149204 A1 | 05-04-2017 |
| | | US 2017270239 A1 | 21-09-2017 |
| | | WO 2015183173 A1 | 03-12-2015 |
| US 2014037685 A1 | 06-02-2014 | NONE | |
| WO 2011160767 A1 | 29-12-2011 | CA 2803677 A1 | 29-12-2011 |
| | | CN 102959090 A | 06-03-2013 |
| | | DE 102010024898 A1 | 29-12-2011 |
| | | EP 2585612 A1 | 01-05-2013 |
| | | ES 2526977 T3 | 19-01-2015 |
| | | IL 223816 A | 31-07-2016 |
| | | JP 5421501 B2 | 19-02-2014 |
| | | JP 2013534820 A | 09-09-2013 |
| | | US 2013102493 A1 | 25-04-2013 |
| | | WO 2011160767 A1 | 29-12-2011 |
| WO 2006050124 A2 | 11-05-2006 | EP 1807539 A2 | 18-07-2007 |
| | | JP 2008518598 A | 05-06-2008 |
| | | US 2008096770 A1 | 24-04-2008 |
| | | WO 2006050124 A2 | 11-05-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 8702679 A
- WO 2006024100 A
- WO 9001949 A
- JP 2016256270 A
- JP 2016256278 A

### Non-patent literature cited in the description


- **COFFMAN, R.L. ; SHER, A. ; SEDER, R.A.** *Immunity,* 2010, vol. 33, 492-503
- **REED, S.G. ; ORR, M.T. ; FOX, C.B.** *Nat Med,* 2013, vol. 19, 1597-1608
- **DESMET, C.J. ; ISHII, K.J.** *Nature reviews. Immunology,* 2012, vol. 12, 479-491
- **JANEWAY, C.A., JR.** *Cold Spring Harbor symposia on quantitative biology 54,* 1989, vol. 1, 1-13
- **MATZINGER, P.** *Annu Rev Immunol,* 1994, vol. 12, 991-1045
- **KAWAI, T. ; AKIRA, S.** *Nature immunology,* 2010, vol. 11, 373-384
- **MATZINGER, P.** *Annu RevImmunol,* 1994, vol. 12, 991-1045
- **SHOENFELD, Y. ; AGMON-LEVIN, N.** *Journal of autoimmunity,* 2011, vol. 36, 4-8
- **BATISTA-DUHARTE, A. ; LINDBLAD, E.B. ; OVIEDO-ORTA, E.** *Toxicology letters,* 2011, vol. 203, 97-105
- **ZAITSEVA, M. et al.** *Vaccine,* 2012, vol. 30, 4859-4865
- **STASSIJNS, J. ; BOLLAERTS, K. ; BAAY, M. ; VERSTRAETEN, T.** *Vaccine,* 2016, vol. 34, 714-722
- **BATISTA-DUHARTE, A. ; LINDBLAD, E.B. ; OVIEDO-ORTA.** *Toxicology letters,* 2011, vol. 203, 97-105
- **SUN, Y. ; GRUBER, M. ; MATSUMOTO, M.** *Journal of pharmacological and toxicological methods,* 2012, vol. 65, 49-57
- **MASTELIC, B. et al.** *Biologicals,* 2013, vol. 41, 115-124
- **PULENDRAN, B.** *Proc Natl Acad Sci USA,* 2014, vol. 111, 12300-12306
- **RAVINDRAN, R. et al.** *Science,* 2014, vol. 343, 313-317
- **TSANG, J.S. et al.** *Cell,* 2014, vol. 157, 499-513
- **NAKAYA, H.I. et al.** *Immunity,* 2015, vol. 43, 1186-1198
- **SOBOLEV, O. et al.** *Nature immunology,* 2016, vol. 17, 204-213
- **OLAFSDOTTIR, T. ; LINDQVIST, M. ; HARANDI, A.M.** *Vaccine,* 2015, vol. 33, 5302-5307
- **N.LANCE ; W.T.WILLIAMS.** *The Computer Journal,* 1967, vol. 9, 373-380
- **F.MURTAGH.** *The Computer Journal,* 1983, vol. 26, 354-359
- **C.F.OLSON.** *Parallel Computing,* 1995, vol. 21, 1313-1325
- *Nat Genet.,* December 2002, vol. 32 (526-32
- **CALABRO S et al.** *Vaccine,* 18 July 2013, vol. 31 (33), 3363-9
- **CAPRONI E et al.** *J Immunol.,* 01 April 2012, vol. 188 (7), 3088-98
- **HONDA-OKUBO Y et al.** *Vaccine,* 03 August 2012, vol. 30 (36), 5373-81
- **SAADE F et al.** *Vaccine,* 08 April 2013, vol. 31 (15), 1999-2007
- **VERTHELYI D ; ISHII KJ ; GURSEL M ; TAKESHITA F ; KLINMAN DM.** *J Immunol.,* 15 February 2001, vol. 166 (4), 2372-7
- **AOSHI T et al.** *J Immunol Res. 2015,* 2015, 316364
- **FALKEBORN T et al.** *PLoS One.,* 08 August 2013, vol. 8 (8), e70527
- **MALTAIS AK et al.** *Vaccine.,* 30 May 2014, vol. 32 (26), 3307-15
- **KOBIYAMA K et al.** *Proc Natl Acad Sci USA.,* 25 February 2014, vol. 111 (8), 3086-91
- **SAWAHATA R et al.** *Microbes Infect.,* April 2011, vol. 13 (4), 350-8
- **COBAN C et al.** *Cell Host Microbe.,* 21 January 2010, vol. 7 (1), 50-61
- **ONISHI M et al.** *Vaccine.,* 23 May 2014, vol. 32 (25), 3004-9
- *J Antibiot (Tokyo).,* August 1983, vol. 36 (8), 1045-50
- **AKAZAWA T. et al.** *CancerSci,* 2010, vol. 101, 1596-1603
- **HEMMI, H. et al.** *Nature,* 2000, vol. 408, 740-745
- **KRIEG, A.M.** *Nature reviews. Drug discovery,* 2006, vol. 5, 471-484

- **BRAZOLOT MILLAN, C.L. ; WEERATNA, R. ; KRIEG, A.M. ; SIEGRIST, C.A. ; DAVIS, H.L.** *Proceedings of the National Academy of Sciences of the United States of America,* 1998, vol. 95, 15553-15558
- **CHU, R.S. ; TARGONI, O.S. ; KRIEG, A.M. ; LEHMANN, P.V. ; HARDING, C.V.** *The Journal of experimental medicine,* 1997, vol. 186, 1623-1631
- **KRIEG, A.M.** Nature reviews. *Drug discovery,* 2006, vol. 5, 471-484
- **KLINMAN, D.M.** Nature reviews. *Immunology,* 2004, vol. 4, 249-258
- *Advanced drug delivery reviews,* 2009, vol. 61, 195-204
- **HARTMANN, G. et al.** *European journal of immunology,* 2003, vol. 33, 1633-1641
- **MARSHALL, J.D. et al.** *Journal of leukocyte biology,* 2003, vol. 73, 781-792
- **SAMULOWITZ, U. et al.** *Oligonucleotides,* 2010, vol. 20, 93-101
- **OLIVE.** *Expert review of vaccines,* 2012, vol. 11, 237-256
- **DESMET, C.J. ; ISHII, K.J.** *Nature reviews Immunology,* 2012, vol. 12, 479-491
- **KOYAMA, S. ; AOSHI, T. ; TANIMOTO, T. ; KUMAGAI, Y. ; KOBIYAMA, K. ; TOUGAN, T. ; SAKURAI, K. ; COBAN, C. ; HORII, T. ; AKIRA, S. et al.** *Science translational medicine,* 2010, vol. 2, 25ra24
- **IGARASHI, Y. et al.** Open TG-GATEs: a large-scale toxicogenomics database. *Nucleic acids research,* 2015, vol. 43, D921-927
- **CHEN, Y.A. ; TRIPATHI, L.P. ; MIZUGUCHI, K.** *PloS one,* 2011, vol. 6, e17844, http://targetmine.mizuguchilab.org
- **CHEN, Y.A. et al.** *PloS one,* 2011, vol. 6, e17844, http://targetmine.mizuguchilab.org
- **HENG, T.S. et al.** *Nature immunology,* 2008, vol. 9, 1091-1094, http://www.immgen.org
- **CHEN, Y.A. et al.** *PloS one,* 2011, vol. 6, e17844
- **HENG, T.S. et al.** *Nature immunology,* 2008, vol. 9, 1091-1094, https://www.immgen.org
- **LEEK, J.T. et al.** *Nat Rev Genet,* 2010, vol. 11, 733-739
- **MARICHAL, T. et al.** *Nat Med,* 2011, vol. 17, 996-1002
- **ONISHI, M. et al.** *Journal of immunology,* 2015, vol. 194, 2673-2682
- **VONO.** *Proceedings of the National Academy of Sciences of the United States of America,* 2013, vol. 110, 21095-21100
- **FALKEBORN, T. et al.** *PloS one,* 2013, vol. 8, e70527
- **MALTAIS, A.K. et al.** *Vaccine,* 2014, vol. 32, 3307-3315
- **HAYASHI, M. et al.** *Scientific Reports,* 2016
- **HONDA-OKUBO, Y. et al.** *Vaccine,* 2012, vol. 30, 5373-5381
- **SAADE, F. et al.** *Vaccine,* 2013, vol. 31, 1999-2007
- **HAYASHI et al.** *Scientific Reports,* 2016, vol. 6
- **GAO, P. et al.** *Cell,* 2013, vol. 154, 748-762
- **STEINHAGEN, F. et al.** *Journal of Leukocyte Biology,* 2012, vol. 92, 775-785
- **MADHUN, A.S. et al.** *Vaccine,* 2011, vol. 29, 4973-4982
- **TANG, C.K. et al.** *PloS one,* 2013, vol. 8, e60038
- **WOODWARD, J.J. et al.** *Science,* 2010, vol. 328, 1703-1705
- **TANAKA, M. et al.** *Bioscience, Biotechnology, and Biochemistry,* 1993, vol. 57, 1602-1603
- **MIZUKAMI, T. et al.** *Vaccine,* 2008, vol. 26, 2270-2283
- **MIZUKAMI, T. et al.** *PloS one,* 2014, vol. 9, e101835
- **DIDIERLAURENT, A.M. et al.** *Journal of immunology,* 2009, vol. 183, 6186-6197
- **KNUDSEN, N.P. et al.** *Scientific reports,* 2016, vol. 6, 19570
- **IGARASHI, Y. et al.** *Nucleic acids research,* 2015, vol. 43, D921-927
- **HARA et al.** *Nature immunology,* vol. 8, 619-629
- **ARASE et al.** *J Exp Med,* 1997, vol. 186, 1957-1963
- **TAKAI et al.** *Cell,* 1994, vol. 76, 519-529
- **MARICHAL et al.** *Nature medicine,* 2011, vol. 17, 996-1002
- **KOBIYAMA et al.** *Proceedings of the National Academy of Sciences of the United States of America,* 2014, vol. 111, 3086
- **ONISHI et al.** *Journal of immunology,* 2015, vol. 194, 2673-2682
- **NORDVALL.** *Allergy,* 1982, vol. 37, 259-264
- **GONZALEZ et al.** *Nature immunology,* 2010, vol. 11, 427-434
- **SUZUKI et al.** *J Exp Med,* 2009, vol. 206, 1485-1493
- **AOSHI et al.** *European journal of immunology,* 2009, vol. 39, 417-425
- **AOSHI et al.** *Immunity,* 2008, vol. 29, 476-486
- **EISENBARTH et al.** *Nature,* 2008, vol. 453, 1122-1126
- **KURODA et al.** *Int Rev Immunol,* 2013, vol. 32, 209-220
- **UEHARA, T. et al.** *Toxicol Appl Pharmacol,* 15 September 2011, vol. 255 (3), 297-306